# EUROPEAN PATENT SPECIFICATION

(11) **EP 2 058 394 B1**
(45) Date of publication and mention of the grant of the patent: **12.11.2014**
(21) Application number: 07767790.4
(22) Date of filing: 28.06.2007
(51) Int. Cl.: C12N 15/09, C12N 1/15, C12N 1/19, C12N 1/21, C12N 5/10, C12N 9/44

(54) **METHOD FOR DESIGNING MUTATED ENZYME, METHOD FOR PREPARING THE SAME AND MUTATED ENZYME**
VERFAHREN ZUR KONSTRUKTION EINES MUTIERTEN ENZYMS, VERFAHREN ZUR HERSTELLUNG DAVON UND MUTIERTES ENZYM
PROCEDE DE CONCEPTION D'ENZYME MUTEE, SON PROCEDE DE PREPARATION ET ENZYME MUTEE

(30) Priority: 04.08.2006 JP 2006213490
(43) Date of publication of application: 13.05.2009
(60) Divisional application: 13179288.9
(73) Proprietor: Amano Enzyme Inc., Nagoya-shi, Aichi 460-0003 (JP)
(72) Inventor: MIKAMI, Bunzo, Uji-shi, Kyoto 611-0011 (JP); IWAMOTO, Hiroyuki, Fukuyama-shi Hiroshima 729-0292 (JP); YAMAGUCHI, Shotaro, Kakamigahara-shi Gifu 509-0109 (JP)
(74) Representative: Müller Fottner Steinecke
(86) International application number: PCT/JP2007/062996
(87) International publication number: WO 2008/015861

(56) References cited:
- WO-A-01/51620
- WO-A-99/45124
- JP-A- 2006 174 841
- US-B1- 6 265 197
- DATABASE UniProt [Online] 1 January 1998 (1998-01-01), "SubName: Full=AmyX protein;" XP002540760 retrieved from EBI accession no. UNIPROT:O34587 Database accession no. O34587
- MATSUMOTO D. ET AL.: 'Pullalanase no Kishitsu Ketsugo Bui no Dotei' NIPPON NOGEI KAGAKU KAISHI vol. 67, no. 3, 1993, page 127 + ABSTR. NO. 2WP3, XP003020912
- MIKAMI E. ET AL.: 'Crystal structure of pullalanase: evidence for parallel binding of oligosaccharides in the active site' J. MOL. BIOL. vol. 359, no. 3, June 2006, pages 690 - 707, XP005455212
- HIROMI K. ET AL.: 'Starch no Edakiri Koso no Kozo.Kino Oyobi Oyo ni Kansuru Kisoteki Kenkyu' ANNUAL REPORT / THE IIJIMA MEMORIAL FOUNDATION FOR PROMOTION OF FOOD SCIENCE AND TECHNOLOGY 1993, pages 211 - 213, XP003020913
- IWAMOTO H.: 'Shokuhin Kano ni okeru Starch Edakiri Koso no Kino Kaiseki to Kino Kaihatsu' ANNUAL REPORT / THE IIJIMA MEMORIAL FOUNDATION FOR PROMOTION OF FOOD SCIENCE AND TECHNOLOGY 2003, pages 138 - 143, XP003020914
- IWAMOTO H. ET AL.: 'Denpun Edakiri Koso pullulanase to Cyclodextrin tono Sogo Sayo' THE MEMOIRS OF THE FACULTY OF ENGINEERING, FAKUSHIMA UNIVERSITY vol. 19, no. 2, 1996, pages 71 - 80, XP003020915
- MALLE D. ET AL.: 'Overexpression, purification and preliminary X-ray analysis of pullulanase from Bacillus subtilis strain 168' ACTA CRYSTALLOGRAPH. SECT. F STRUCT. BIOL. CRYST. COMMUN. vol. 62, no. PART 4, April 2006, pages 381 - 384, XP003020916

## Description

### TECHNICAL FIELD

The present invention relates to a method for designing a mutated enzyme hydrolyzing an α-1, 6-glycosidic linkage and a method for preparing the same.

### BACKGROUND ART

Pullulanase (EC 3.2.1.41) is an enzyme hydrolyzing an α-1,6 linkage of, for example, amylopectin in starch. Pullulanase is an enzyme having a high industrial applicability in the fields of sugar, for example, production of maltooligosaccharides such as glucose, maltose, maltotriose, maltotetraose, maltopentaose and maltohexaose (OLIGOSACCHARIDES, Gordon and Breach Science Publishers, p3), improvement of rice cooking (patent document 1), and the like.

Pullulanase derived from microorganism includes Bacillus sp. APC-9603 (patent document 2), and ones derived from Klebsiella pneumonia (AMANO ENZYME INC.), Bacillus deramificans, Bacillus acidpullulyticus, Bacillus stearothermophilus, Bacillus sectorramus, Bacillus circulans, Bacillus cereus, and Bacillus sectorramus.

Similar to the other enzymes, when pullulanase is used, concentration of substrate and enzyme, reaction temperatures, reaction time, and the like are adjusted depending upon the applications of use. However, with adjustment of such enzyme reaction conditions alone, it may not be possible to produce intended products or to obtain an expected yield. Thus, it has been necessary to modify the properties themselves of pullulanase.

In order to modify the properties of pullulanase, it is necessary that mutants of pullulanase should be produced, and the activity, substrate specificity, and the like, should be evaluated so as to search for an excellent mutant. However, such processes have required much labor. Patent document 3 discloses one example of a mutant of pullulanase.
[Patent document 1] JP H7-289186 A
[Patent document 2] JP H5-292962 A
[Patent document 3] JP 2002-505108 A
[Non-patent document 1] J.Mol Biol. 2006 Jun 9; 359 (3): 690-707

In addition, WO 99/45124 A describes the recombinant expression of pullulanase obtained from Bacillus deramificans and modifications of the pullulanase at its amino terminus. JP 2006 174841 A discloses pullulan variants having different pH optima or better durability to oxidants. US 6 265 197 B1 relates to pullulanase variants with improved thermostability and WO 01/51620 A describes pullulanase variants, where certain amino acids of the pullulanase have been modified in order to provide an enzyme having altered properties.

### [DISCLOSURE OF THE INVENTION]

### [Problems to be Solved by the Invention]

One of objects of the present invention is to provide a novel method for improving an enzyme hydrolyzing an α-1,6-glycosidic linkage. This method is useful when providing a mutated enzyme whose action properties have been improved. With the change in action property, it is possible to reduce the amount of enzyme to be used, to shorten a reaction time, to increase applications of use, and the like.

### [Means to Solve the Problems]

In order to solve the above-mentioned problems, the present inventors have keenly investigated further. As a result, the present inventors have obtained an important finding regarding the recognition of a substrate in pullulanase derived from Bacillus subtilis strain 168 by making good use of an X-ray analysis technology for a crystalline structure. That is to say, regarding the pullulanase, the present inventors have succeeded in crystallizing it into a state containing a substrate analog (α-cyclodextrin), and in obtaining information about the three-dimensional structure thereof. Thereby, they have clarified a site to which a substrate analog is bound. Thus, amino acid that is thought to be involved in recognition of a substrate has been specified. Furthermore, as a result of comparison between the three-dimensional structure of the pullulanase and the three-dimensional structure of the same kinds of enzymes derived from the other microorganisms, high similarity is recognized as a whole. In particular, it has been determined that the similarity is extremely high in the site relating to the recognition of a substrate. Since such a high similarity is recognized, it is predicted that an amino acid corresponding to the amino acid specified in the above-mentioned pullulanase plays an important role in the recognition of a substrate in each enzyme.

By the way, as to pullulanase of Klebsiella pneumoniae that is one of the enzymes used in the investigation at this time, an active site is searched for by using G4 (maltotetraose) (see, non-patent document 1). The binding site of the substrate indicated therein is located in the vicinity of the binding site of a substrate analog predicted by the above-mentioned method (a method by comparing with pullulanase derived from Bacillus subtilis strain 168). This fact supports the involvement of the binding site of the substrate analog successfully found by the present inventors in the recognition of actual substrate.

The present invention is mainly based on the above-mentioned results and provides a designing method of an enzyme mentioned below.
[1] A method for designing a mutated enzyme, wherein the activity and/or substrate specificity for hydrolyzing an α-1,6-glycosidic linkage with respect to pullulan or amylopectin is improved as compared with the enzyme to be mutated, the method including the following steps:
   (1) specifying one or two or more amino acids selected from the group shown below in an amino acid sequence of an enzyme (enzyme to be mutated) that hydrolyzes an α-1,6-glycosidic linkage, the group consisting of an amino acid corresponding to an amino acid at the 292 position, an amino acid corresponding to an amino acid at the 371 position, an amino acid corresponding to an amino acid at the 406 position, an amino acid corresponding to an amino acid at the 407 position, an amino acid corresponding to an amino acid at the 437 position, an amino acid corresponding to an amino acid at the 465 position, an amino acid corresponding to an amino acid at the 475 position, an amino acid corresponding to an amino acid at the 476 position; an amino acid corresponding to an amino acid at the 525 position, an amino acid corresponding to an amino acid at the 526 position, an amino acid corresponding to an amino acid at the 580 position and an amino acid corresponding to an amino acid at the 582 position of the amino acid sequence set forth in SEQ ID NO: 2;
   (2) constructing an amino acid sequence in which the amino acid specified in the step (1) is substituted with another amino acid or deleted based on the amino acid sequence of the enzyme to be mutated;
   (3) wherein the amino acid sequence of the enzyme to be mutated is an amino acid sequence set forth in any of SEQ ID NOs: 2, 13 to 16; and
   (4) evaluating the activity and/or substrate specificity with respect to pullulan or amylopectin of the mutated enzyme obtained in step (2).
[2] The method for designing a mutated enzyme according to [1], wherein in the step (1), one or two or more amino acids selected from the group consisting of an amino acid corresponding to an amino acid at the 292 position, an amino acid corresponding to an amino acid at the 371 position, an amino acid corresponding to an amino acid at the 407 position, an amino acid corresponding to an amino acid at the 475 position, an amino acid corresponding to an amino acid at the 476 position, and an amino acid corresponding to an amino acid at the 582 position of the amino acid sequence set forth in SEQ ID NO: 2 is specified.
[3] The method for designing a mutated enzyme according to [1], wherein in the step (1), an amino acid corresponding to an amino acid at the 476 position of an amino acid sequence set forth in SEQ ID NO: 2 is specified.
[4] The method for designing a mutated enzyme according to any one of [1] to [3], wherein the specifying of an amino acid in step (1) is carried out by comparing between an amino acid sequence of the enzyme to be mutated and the amino acid sequence set forth in SEQ ID NO: 2 and/or between a three-dimensional structure of the enzyme to be mutated and a three-dimensional structure of the amino acid sequence set forth in SEQ ID NO: 2.
[5] The method for designing a mutated enzyme according to any one of [1] to [4], wherein the enzyme to be mutated is a wild type enzyme.
[6] The method for designing a mutated enzyme according to any one of [1] to [4], wherein the enzyme to be mutated is pullulanase or isoamylase derived from a microorganism.
[7] The method for designing a mutated enzyme according to [6], wherein the microorganism is a microorganism of genus bacillus, a microorganism of genus Klebsiella, or a microorganism of genus pseudomonas.
[8] The method for designing a mutated enzyme according to any one of [1] to [4], wherein the amino acid sequence of the enzyme to be mutated is an amino acid sequence having a 70% or more identity to the amino acid sequence set forth in SEQ ID NO: 2.
[9] The method for designing a mutated enzyme according to any one of [1] to [4], wherein the amino acid sequence of the enzyme to be mutated is an amino acid sequence set forth in any of SEQ ID NOs :2, 13 to 16.
[10] A method for preparing a mutated enzyme, the method including the following steps:
   (1) preparing a nucleic acid encoding an amino acid sequence constructed by the method described in any of [1] to [9];
   (2) expressing the nucleic acid; and
   (3) collecting expression products.

The method of the invention is useful in obtaining as an example a mutated enzyme including an amino acid sequence in which one or two or more amino acids selected from the group shown below in an amino acid sequence of an enzyme (enzyme to be mutated) that hydrolyzes an α-1,6-glycosidic linkage, the group consisting of an amino acid corresponding to an amino acid at the 292 position, an amino acid corresponding to an amino acid at the 371 position, an amino acid corresponding to an amino acid at the 406 position, an amino acid corresponding to an amino acid at the 407 position, an amino acid corresponding to an amino acid at the 437 position, an amino acid corresponding to an amino acid at the 465 position, an amino acid corresponding to an amino acid at the 475 position, an amino acid corresponding to an amino acid at the 476 position; an amino acid corresponding to an amino acid at the 525 position, an amino acid corresponding to an amino acid at the 526 position, an amino acid corresponding to an amino acid at the 580 position and an amino acid corresponding to an amino acid at the 582 position of the amino acid sequence set forth in SEQ ID NO: 2 is/are substituted with another amino acid or deleted.

In this exemplary mutated enzyme the substituted or deleted amino acid may be one or two or more amino acids selected from the group consisting of an amino acid corresponding to an amino acid at the 292 position, an amino acid corresponding to an amino acid at the 371 position, an amino acid corresponding to an amino acid at the 407 position, an amino acid corresponding to an amino acid at the 475 position, an amino acid corresponding to an amino acid at the 476 position, and an amino acid corresponding to an amino acid at the 582 position of the amino acid sequence set forth in SEQ ID NO: 2.

In the exemplary mutated enzyme the substituted or deleted amino acid may be an amino acid corresponding to an amino acid at the 476 position of the amino acid sequence set forth in SEQ ID NO: 2.

In the above-mentioned exemplary mutated enzyme the enzyme to be mutated may be a wild type enzyme.

In the above-mentioned exemplary mutated enzyme, the enzyme to be mutated may be pullulanase or isoamylase derived from a microorganism.

In the above-mentioned exemplary mutated enzyme, the microorganism may be a microorganism of genus bacillus, a microorganism of genus Klebsiella, or a microorganism of genus pseudomonas.

In the exemplary mutated enzyme, the amino acid sequence of the enzyme to be mutated may be an amino acid sequence having a 70% or more homology with respect to the amino acid sequence set forth in SEQ ID NO: 2.

In the mutated enzyme, the amino acid sequence of the enzyme to be mutated may be an amino acid sequence set forth in any of SEQ ID NOs: 2, 13 to 16.

In the exemplary mutated enzyme, an action property with respect to pullulan or an action property with respect to amylopectin may be improved as compared with the enzyme to be mutated..

As an example a gene encoding the mutated enzyme mentioned above is disclosed.

As a further example a recombinant DNA including the gene as mentioned above is herewith described.

In addition, a microorganism carrying the above-mentioned recombinant DNA is described herein as an example.

### [BRIEF DESCRIPTION OF THE DRAWINGS]

Fig. 1 is a view showing a three-dimensional structure of Bacillus subtilis pullulanase having α-cyclodextrin as a ligand, which is shown by the use of a ribbon model. CD: α-cyclodextrin.
Fig. 2 is a view shown by superimposing Bacillus subtilis pullulanase (BSP) having α-cyclodextrin as a ligand and α carbon of pullulanase (KPP) Klebsiella pneumonia onto each other. CD: α-cyclodextrin.
Fig. 3 is an enlarged view showing a substrate binding region of Fig. 2. CD: α-cyclodextrin, G4: maltotetraose. An amino acid of Bacillus subtilis pullulanase (upper stage) and an amino acid of pullulanase of Klebsiella pneumonia (lower stage) corresponding to the amino acid of the upper stage are shown.
Fig. 4 is a view shown by superimposing Bacillus subtilis pullulanase (BSP) having α-cyclodextrin as a ligand and α carbon of isoamylase of Pseudomonas amyloderamosa (PIA) onto each other. CD: α-cyclodextrin.
Fig. 5 is an enlarged view showing a substrate binding region of Fig. 4. CD: α-cyclodextrin, G4: maltotetraose. An amino acid of Bacillus subtilis pullulanase (upper stage) and an amino acid of isoamylase of Pseudomonas amyloderamosa (lower stage) corresponding to the amino acid of the upper stage are shown.

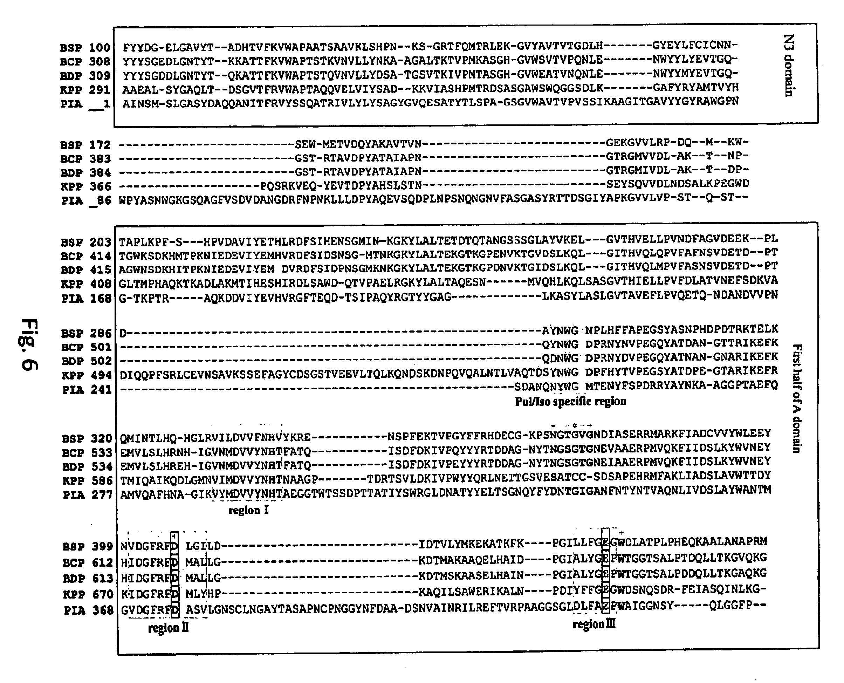

Fig. 6 shows a multiple alignment of amino acid sequences of five kinds of pullulanase and isoamylase. BSP: pullulanase of Bacillus subtilis, BCP: pullulanase of Bacillus sp. APC-9603, BDP: pullulanase of Bacillus deramificans, KPP: pullulanase of Klebsiella pneumoniae, PIA: isoamylase of Pseudomonas amyloderamosa (Amemura, A., Chakraborty, R., Fujita, M., Noumi, T. and Futai, M., Cloning and nucleotide sequence of the isoamylase gene from Pseudomonas amyloderamosa SB-15, JOURNAL J. Biol. Chem. 263 (19), 9271-9275 (1988)). *: position of an amino acid that has been deduced to be involved in binding to α-cyclodextrin, +: amino acid that has been deduced to be involved in binding to G4 altotetraose) among amino acids that have been deduced to be involved in binding to α-cyclodextrin in the above-mentioned report, -: position of the amino acid that has been deduced to be involved in binding to α-cyclodextrin in the above-mentioned report (excluding the position of amino acid that has been deduced to be involved in binding to α-cyclodextrin). Pul / Iso (pullulanase /isoamylase) specific region, region I, region II, region III and region IV are shown by a shaded area. Furthermore, an amino acid of the active site is surrounded by square.
Fig. 7 is the continuation of Fig. 6.
Fig. 8 is an enlarged view showing an α-cyclodextrin biding site of Ifg. 1.

### [BEST MODE FOR CARRYING OUT THE INVENTION]

### 1. Designing method of mutated enzyme

A first aspect of the present invention provides a designing method of a mutated enzyme based on an enzyme hydrolyzing an α-1, 6-glycosidic linkage. With the designing method of the present invention, it is possible to obtain an enzyme that is different from the enzyme before mutation in terms of action properties. In other words, the designing method of the present invention is used as a technique for changing the action properties of an enzyme. Specifically, for example, the designing method of the present invention can be used for the purpose of improving the activity and/or substrate specificity of pullulanase with respect to pullulan, or the activity and/or substrate specificity of pullulanase with respect to amylopectin. It can be expected that the improvement of the activity enables obtaining of a sufficient effect with less amount. That is to say, reduction of the amount to be used can be expected. On the other hand, the improvement of the substrate specificity facilitates the use thereof and reduces the amount to be used. Furthermore, if different substrate specificities are provided, a novel application of use can be achieved.

Pullulanase that is one of the enzymes hydrolyzing an α-1, 6-glycosidic linkage can act on amylopectin in starch so as to form straight chain amylase. By the use of this characteristic, pullulanase has been widely used for processing starch, production of glucose, maltose, oligosaccharide, or the like, or brewing. Furthermore, pullulanase is an enzyme that is used for various industrial purposes of, for example, manufacturing a material of thermally stable microcapsule, a carrier of an immobilized enzyme, and the like. If the reactivity with respect to α-1,6 binding can be freely changed, for example, it is possible to increase the yield in products, to reduce the amount of enzyme to be used (an amount to be added). At the same time, this enzyme can be applied to new fields.

In the present specification, unless otherwise noted, the term "action property" is used as a term including properties (including activity and substrate specificity with respect to pullulan and activity and substrate specificity with respect to amylopectin) relating to the actions for hydrolyzing an α-1,6-glycosidic linkage. The evaluation of the "action property" can be carried out by using the Km value, Kcat value, and the like, obtained by the test system using pullulan, amylopectin, and starch as a substrate. Km value, Kcat value can be determined by the following method.
(1) Substrates (for example, pullulan or amylopectin) with various concentrations are dissolved in 50 mM acetate buffer (pH 5.6) and reacted at 25°C.
(2) The concentration of a reducing sugar contained in a regularly sampled reaction solution is determined by a Park-Johnson method, and the reaction rate is measured from the increasing rate of the reducing sugar.
(3) Km value and Kcat value are obtained by curve fitting into Michaelis-Menten equation by the non-linear minimum square method.

Note here that although depending upon the experiment conditions, by comparing the concentrations of the reducing sugar contained in the reaction solution at certain points, the action properties of two enzymes can be compared and evaluated.

The designing method of the mutated enzyme of the present invention includes roughly two steps, that is, a step of specifying an amino acid to be mutated (step (1)) and a step of constructing an amino acid sequence of the mutated amino acid (step (2)). Hereinafter, the respective steps are described in detail. Note here that in this specification, an enzyme as a base in designing a mutated enzyme (an enzyme to which mutation is carried out) is referred to as "enzyme to be mutated."

### Step (1)

In step (1), in an amino acid sequence of an enzyme (enzyme to be mutated) hydrolyzing an α-1,6-glycosidic linkage, one or two or more of amino acid(s) to which mutation is carried out (hereinafter, which is also referred to as "amino acid to be mutated") is specified. The amino acid to be mutated of the present invention is selected from the group shown below in an amino acid sequence of an enzyme that hydrolyzes an α-1,6-glycosidic linkage, that is, the group consisting of an amino acid corresponding to an amino acid at the 292 position, an amino acid corresponding to an amino acid at the 371 position, an amino acid corresponding to an amino acid at the 406 position, an amino acid corresponding to an amino acid at the 407 position, an amino acid corresponding to an amino acid at the 437 position, an amino acid corresponding to an amino acid at the 465 position, an amino acid corresponding to an amino acid at the 475 position, an amino acid corresponding to an amino acid at the 476 position; an amino acid corresponding to an amino acid at the 525 position, an amino acid corresponding to an amino acid at the 526 position, an amino acid corresponding to an amino acid at the 580 position and an amino acid corresponding to an amino acid at the 582 position of the amino acid sequence set forth in SEQ ID NO: 2. Note here that these amino acids to be mutated are amino acids that have been suggested to be involved in the recognition of the substrate as a result of analysis of the three-dimensional structure at the time of binding of a substrate analog (α-cyclodextrin, hereinafter, referred to as "CD") regarding pullulanase derived from Bacillus subtilis strain 168 (including the amino acid sequence set force in SEQ ID NO: 2), and from the comparison results between this three-dimensional structure and the three-dimensional structure of an enzyme derived from a microorganism. By mutating these amino acids, it is expected that the action property (in particular, substrate specificity) of the enzyme is changed.

Herein, the term "corresponding" to be used for amino acid residues in the specification means the equal contribution to exhibition of the function between proteins (enzymes) to be compared. In particular, it means that the contribution to the substrate specificity is equivalent. For example, when an amino acid sequence to be compared is arranged with respect to the reference amino acid sequence (that is to say, amino acid sequence set forth in SEQ ID NO: 2) so that suitable comparison can be carried out while considering the partial homology of the primary structure (that is to say, an amino acid sequence) (at this time, a gap may be introduced so as to optimize the alignment if necessary), an amino acid in a position corresponding to a certain amino acid in the reference to amino acid sequence can be defined as "corresponding amino acid." Instead of comparison between primary structures, or in addition thereto, by comparison between the stereostructures (three-dimensional structures), "corresponding amino acid" can be specified. By using the three-dimensional structure information, it is possible to comparison results with high reliability. In this case, atomic coordinates of the three-dimensional structures of a plurality of enzymes can be compared with each other so as to carry out alignment. The three-dimensional structure information on the enzyme to be mutated can be obtained from, for example, Protein Data Bank (http://www.pdbj.org/index_j.html).

An example of the method of determining the three-dimensional structure of protein by an X-ray analysis of crystalline structure is described below.
(1) Protein is crystallized. The crystallization is indispensable for determination of the three-dimensional structure. Besides, the crystallization is industrially useful as a purification of protein with high purity and a preservation method of protein with high density. In this case, protein to which a substrate or an analog compound thereof is bound as a ligand may be crystallized.
(2) The prepared crystal is irradiated with X ray and analysis data are collected. Note here that protein crystal may be damaged by X ray irradiation and may be deteriorated in its diffraction ability so often. In such a case, a low-temperature measurement method for rapidly cooling a crystal to about -173°C and collecting diffraction data in this state has been recently widespread. Note here that finally, in order to collect high resolution data used for determining a structure, synchrotron radiation light with high intensity is used.
(3) For carrying out analysis of a crystalline structure, phase information is necessary in addition to the diffraction data. When the crystalline structure of a related protein with respect to the intended protein is not known, it is impossible to determine the structure by a molecule substitution method. Problem as to the phase must be resolved by the heavy atom isomorphous replacement method. The heavy atom isomorphous replacement method is a method of introducing a metal atom having a larger atomic number such as mercury and platinum into a crystal and using the contribution of the metal atom to X-ray diffraction data of X-ray scattering power, thereby obtaining phase information. The determined phase can be improved by smoothing the electron density in the solvent region in the crystal. Since the water molecule in the solvent region is largely fluctuated, electrical density is hardly observed. Therefore, by approximating the electron density in this region to 0, it can approach to the real electron density. Consequently, the phase is improved. Furthermore, when a plurality of molecules are included in an asymmetrical unit, by averaging the electron densities of these molecules, the phase is further radically improved. A protein model is fitted to the view of the electron density calculated by using the thus improved phase. This process is carried out by using a program such as QUANTA (MSI, America) on a computer graphics. Thereafter, by using a program such as X-PLOR (MSI), refinement of the structure is carried out. Thus, the structure analysis is completed.

When the crystalline structure of a related protein with respect to the intended protein is known, the structure can be determined by a molecule substitution method by using an atomic coordinate of the known protein. The molecule substitution and structure refinement can be carried out by using a program such as CNS_SOLVE ver.11.

The present inventors have tried to crystallize the recombinant pullulanase purified from Bacillus subtilis strain 168 and to crystallize the pullulanase in a state in which it contains CD as a substrate analog and have succeeded in obtaining three-dimensional structure of both types of pullulanase. Note here that atomic coordinates of the three-dimensional structure of the pullulanase containing CD are shown in the last part of this specification. Furthermore, the amino acid sequence of pullulanase and the base sequence of a gene encoding thereof are shown in SEQ ID NO: 2 and SEQ ID NO: 1 in the sequence listing, respectively.

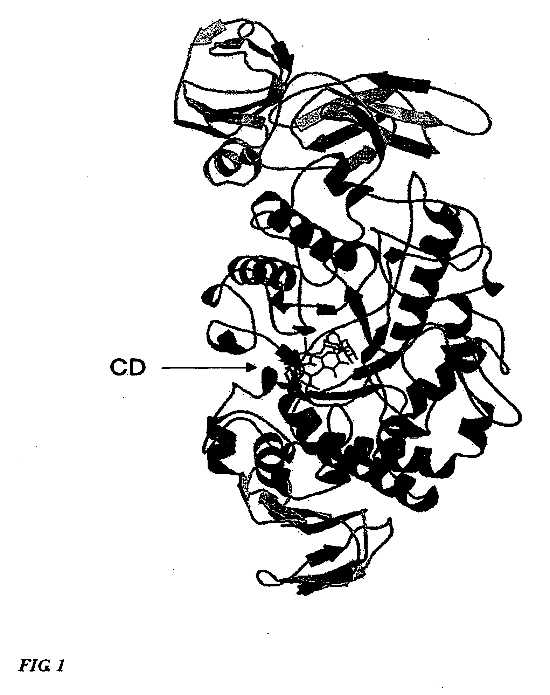

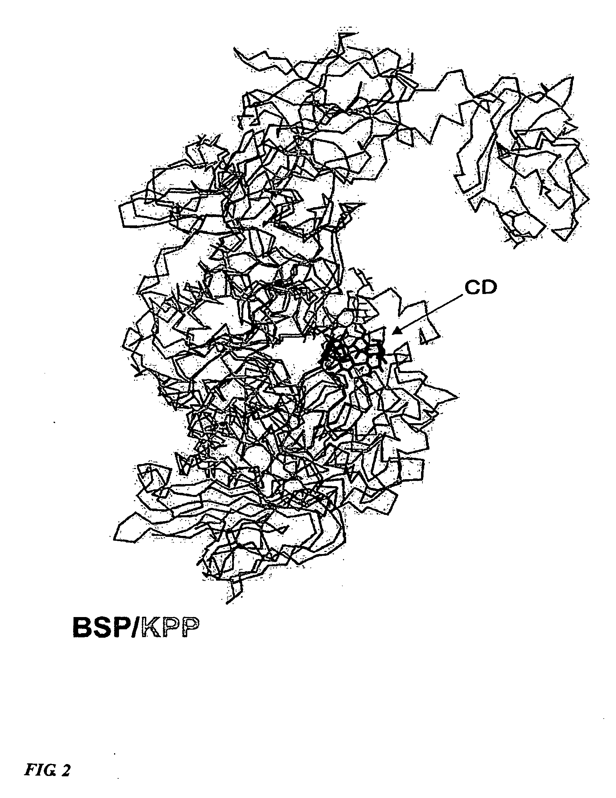

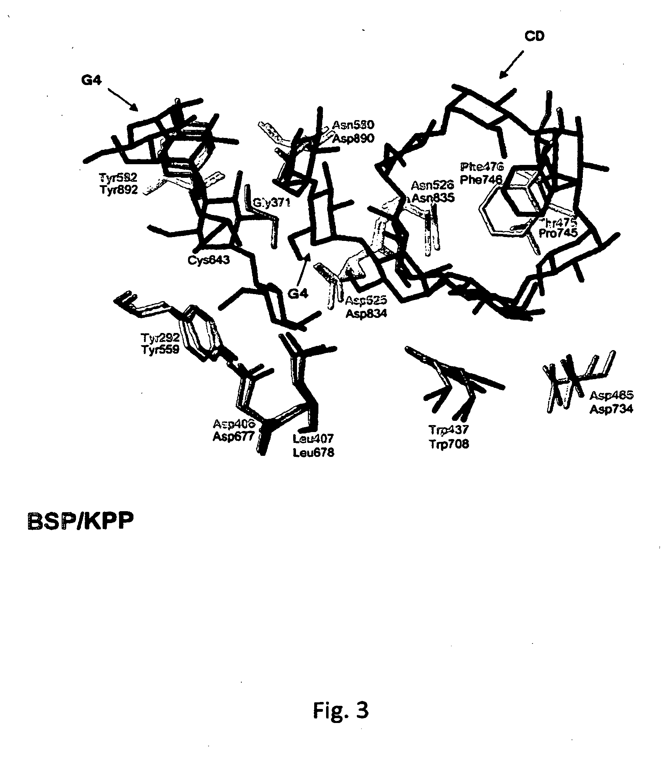

As shown in the below-mentioned Examples, it has been determined that the pullulanase molecule derived from Bacillus subtilis strain 168 has rhombic system P2(1)2(1)2(1) having 70.568 × 127.68 × 189.25 Å (see, Figs. 1 to 3). Fig. 1 is a view showing a crystalline structure of pullulanase by a ribbon model. α-helix and β-sheet are shown in a helix shape and an arrow shape, respectively (Fig. 1), and a substrate analog (CD) is shown by an arrow CD (Figs. 1 to 3). Fig. 2 is a view shown by superimposing Bacillus subtilis pullulanase (BSP) having α-cyclodextrin as a ligand and α carbon of pullulanase (KPP) Klebsiella pneumonia onto each other. Fig. 3 is an enlarged view of a substrate binding region of Fig. 2.

In one preferable embodiment of the present invention, the amino acid to be mutated is selected from the group consisting of an amino acid corresponding to an amino acid at the 292 position, an amino acid corresponding to an amino acid at the 371 position, an amino acid corresponding to an amino acid at the 407 position, an amino acid corresponding to an amino acid at the 475 position, an amino acid corresponding to an amino acid at the 476 position and an amino acid corresponding to an amino acid at the 582 position of the amino acid sequence set forth in SEQ ID NO: 2. Note here that amino acids to be mutated are an amino acid corresponding to an amino acid that has been determined to be directly involved in binding between pullulanase derived from Bacillus subtilis strain 168 and a substrate analog (CD).

By the way, it has clarified that an amino acid at the 476 position of the amino acid sequence set forth in SEQ ID NO: 2 is arranged so that it enters a ring structure of CD that is a substrate analog in the three-dimensional structure analysis about pullulanase derived from Bacillus subtilis strain 168, and this amino acid is thought to play an important role in the recognition of a substrate. Therefore, in the further preferable embodiment of the present invention, an amino acid corresponding to the amino acid is to be an amino acid to be mutated.

The kinds, origins and the like of enzymes to be mutated in accordance with the present invention are not particularly limited as long as the enzymes hydrolyze an α-1,6-glycosidic linkage. Preferably, the enzyme to be mutated is pullulanase or isoamylase derived from microorganisms. An example of the microorganism herein can include a microorganism of genus bacillus, a microorganism of genus Klebsiella, or a microorganism of genus pseudomonas. As the pullulanase derived from microorganism, Bacillus sp. APC-9603 (Japanese Patent Unexamined Publication No. H5-292962), and ones derived from derived from Klebsiella pneumonia (AMANO ENZYME INC.), Bacillus deramificans, Bacillus acidpullulyticus, Bacillus stearothermophilus, Bacillus sectorramus, Bacillus circulans, Bacillus cereus, and Bacillus subtilis strain 168 are well known. For example, any of them can be employed as the enzyme to be mutated in the present invention. Furthermore, isoamylase of Pseudomonas amyloderamosa can be employed as the enzyme to be mutated in the present invention. A specific example of the enzyme to be mutated includes an enzyme (pullulanase of Bacillus subtilis strain 168) consisting of an amino acid sequence set forth in SEQ ID NO: 2, an enzyme (pullulanase of Klebsiella pneumoniae ATCC9621) consisting of an amino acid sequence set forth in SEQ ID NO: 13, an enzyme (pullulanase of Bacillus sp. APC-9603) consisting of an amino acid sequence set forth in SEQ ID NO: 14, an enzyme (pullulanase of Bacillus deramificans) consisting of an amino acid sequence set forth in SEQ ID NO: 15, and an enzyme (isoamylase of Pseudomonas amyloderamosa) consisting of an amino acid sequence set forth in SEQ ID NO: 16.

It is preferable that an enzyme consisting of an amino acid sequence having a high identity with the amino acid sequence set forth in SEQ ID NO: 2 is an enzyme to be mutated. It is preferable because effective improvement is expected to be achieved and the specification of the amino acid to be mutated is facilitated.

Specifically, it is preferable that the enzyme to be mutated is an enzyme consisting of an amino acid sequence having 70% or more identity with the amino acid sequence set forth in SEQ ID NO: 2. Herein, the higher identity is generally more preferable. For example, the enzyme to be mutated is an enzyme consisting of an amino acid sequence having the identity of preferably 80% or more, further preferably 90% or more, and further preferably 95% or more.

Herein, the identity (%) between two amino acid sequences can be determined by the following procedure. Firstly, two sequences are aligned for optimum comparison of the two sequences (for example, a gap may be introduced in the first sequence so as to obtain an optimum alignment with the second sequence). When a molecule (amino acid residue) at the specific position in the first sequence and a molecule in the corresponding position in the second sequence are the same, the molecules in the positions are defined as being identical. The identity between two sequences is an action property of the number of identical positions shared by the sequences (i.e., identity (%) = number of identical positions / total number of positions x 100), preferably taking into account the number of gaps, and the length of each gap, which need to be introduced for optimal alignment of the two sequences.

The comparison and determination of identity between two sequences can be carried out by using a mathematical algorithm. A specific example of mathematical algorithm that can be used for comparing sequences include an algorithm described in Karlin and Altschul (1990) Proc. Natl. Acad. Sci. USA 87:2264-68 and modified by Karlin and Altschul (1993) Proc. Natl. Acad. Sci. USA 90:5873-77 but the algorithm is not limited to this. Such an algorithm is incorporated in NBLAST and XBLAST programs (version 2.0) of Altschul et al. (1990) J. Mol. Biol. 215: 403-10. BLAST polypeptide searches may be carried out by, for example, the NBLAST program, score = 50, wordlength = 3 to obtain amino acid sequence homologous to a certain amino acid sequence. To obtain gapped alignments for comparison purposes, Gapped BLAST as described in Altschul et al., (1997) Amino Acids Research 25(17): 3389-3402 can be utilized. When utilizing BLAST and Gapped BLAST programs, the default parameters of the respective programs (e.g., XBLAST and NBLAST) can be used. In detail, see http://www.ncbi.nlm.nih.gov. Another example of mathematical algorithm that can be used for comparing sequences includes an algorithm of Meyers and Miller (Comput. Appl. Biosci. 4: 11-17 (1988)) which has been incorporated into the ALIGN program that can be used for, for example, GENESTREAM network server (IGH Montpellier, France) or ISREC server. When the ALIGN program is used for comparison of the amino acid sequences, for example, a PAM120 weight residue table can be used with a gap length penalty of 12 and a gap penalty of 4.

The identity between two amino acid sequences can be determined using the GAP program in the GCG software package, using a Blossom 62 matrix or PAM250 matrix and a gap weight of 12, 10, 8, 6, or 4, and a gap length weight of 2, 3, or 4.
Furthermore, the homology between two nucleic acid sequences can be determined using the GAP program in the GCG software package (available at http://www.gcg.com) with a gap weight of 50 and a gap length weight of 3.

The enzyme to be mutated is typically a wild type enzyme (naturally occurring enzyme). However, an enzyme to which some mutation or modification has already been given is not excluded. Thus, the present invention can be used for the purpose of further improving the property of an enzyme.

### Step (2)

In the step (2), an amino acid sequence, in which an amino acid specified in the step (1) has been substituted with another amino acid or the amino acid has been deleted, is constructed based on an amino acid sequence of the enzyme to be mutated. The kinds of substituted amino acids are not particularly limited and therefore may include conservative substitution of amino acid or non-conservative substitution of amino acid. Herein, a "conservative amino acid substitution" is one in which the amino acid residue is substituted with an amino acid residue having a side chain with similar feature. The amino acid residues are divided into some families including basic side chains (e. g. , lysine, arginine, histidine), acidic side chains (e.g., aspartic acid, glutamic acid), uncharged polar side chains (e. g., asparagine, glutamine, serine, threonine, tyrosine, cysteine), nonpolar side chains (e. g, glycine, alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan). Preferably, the conservative amino acid substitution is a substitution between preferably an amino acid residue of the same family.

### 2. Preparation method of mutated enzyme

A second aspect of the present invention relates to a preparation method of a mutated enzyme. The preparation method in accordance with the present invention includes the following steps:
(1) preparing nucleic acid encoding an amino acid sequence constructed by the designing method in accordance with the present invention;
(2) expressing the nucleic acid; and
(3) collecting expression products.

In the step (1), necessary mutation (that is, substitution or deletion of amino acids in a certain position in protein as an expression product) is applied to a gene encoding the enzyme to be mutated based on an amino acid sequence constructed by the designing method of the present invention, and thereby nucleic acid (gene) encoding a mutated enzyme is obtained. A large number of methods for the position specific substitution of base sequence have been known (see, for example, Molecular Cloning, Third Edition, Cold Spring Harbor Laboratory Press, New York). Among them, appropriate methods can be selected and used.

As the position specific mutation introduction method, a position specific amino acid saturated mutation method can be employed. The position specific amino acid saturated mutation method is a "Semi-rational, semi-random" technique in which a position relating to the intended function is deduced based on the three-dimensional structure of protein, and the amino acid saturated mutation is introduced (J. Mol. Biol. 331, 585-592 (2003)). For example, a position specific amino acid saturated mutation can be introduced by using a kit such as Quick change (Stratagene), Overlap extension PCR (Nucleic Acid Res. 16, 7351-7367 (1988)). As DNA polymerase used for PCR, Taq polymerase can be used. However, it is preferable that DNA polymerase with high purify, for example, KOD-PLUS- (TOYOBO), Pfu turbo (Stratagene) are used.

On the other hand, a gene encoding a mutated enzyme can be prepared by inserting random mutation into an enzyme gene, comparing the substrate specificities of expression product by mutants each other, and selecting a gene having preferable substrate specificity. When such a random mutation is introduced, firstly, for example, error-prone PCR is used and mutation is introduced into a targeted gene region randomly so as to construct a mutated enzyme gene library. Then, a clone is selected from the resultant library using the enzymatic activity or the substrate specificity as an index.

In the step (2), a gene prepared in the step (1) is expressed. Then, in the subsequent step (3), mutated enzymes as expression products are collected.

In general, from the step of expressing a gene to the step of collecting the expression products (mutated enzymes) are carried out by using an appropriate host-vector system. However, a cell-free synthesis system may be used. As to the detail of the preparation method of the mutated enzyme by using a host-vector system, the below-mentioned description may be employed (see, the column of 4. Nucleic acid encoding mutated enzyme).

Herein, the "cell-free synthesis system (cell-free transcription system, cell-free transcription / translation system)" denotes that living cells are not used but a ribosome derived from living cells (or cells obtained by genetically engineering technique) or by using a transcription / translation factor and the like, mRNA or protein encoded by nucleic acid (DNA or mRNA) as a template are synthesized from them in vitro. In general, in the cell-free synthesis system, a cell extract obtained by purifying a cell homogenized solution if necessary is used. In general, a cell extract includes ribosome necessary to synthesis of protein, various factors such an initiation factor, various enzymes such as tRNA. When synthesis of protein is carried out, various amino acids, energy sources such as ATP and GTP, creatine phosphate, and the like, are added to the cell extract solution. Needless to say, at the time of synthesis of protein, additionally prepared ribosome or various factors, and/or various enzymes may be replenished if necessary.

Development of a transcription / translation system in which each molecule (factor) necessary to synthesis of protein is reconstructed has been reported (Shimizu, Y. et al.: Nature Biotech., 19, 751-755, 2001). In this system, a gene of 31 kinds of factors consisting of three kinds of initiation factors constituting a protein synthesis system of bacteria, three kinds of elongation factors, four kinds of factors involved in termination, 20 kinds of aminoacyl tRNA synthases for binding each amino acid to tRNA, and methionyl tRNA formyl transferase is amplified from Escherichia coli genome. They are used so as to reconstruct a protein synthesis system in vitro. In the present invention, such a re-constructed synthesis system may be used.

The term "cell-free transcription /translation system" can be used interchangeably with the term cell-free protein synthesis system, in vitro translation system or in vitro transcription / translation system. In the in vitro translation system, protein is synthesized by using RNA as a template. As the template RNA, total RNA, mRNA, in vitro transcription product, and the like, are used. On the other hand, in the in vitro transcription / translation system, DNA is used as a template. The template DNA should include a ribosome-binding region and preferably includes an appropriate terminator sequence. Note here that the in vitro transcription / translation system sets a condition in which factors necessary to reaction are added so that the transcription reaction and translation reaction proceed consecutively.

### 3. Mutated enzyme

According to the above-mentioned preparation method, it is possible to obtain a mutated enzyme in which the action property with respect to α-1, 6-glycosidic linkage has been changed. As an example a mutated enzyme has been described herein. In the mutated enzyme, an action property with respect to pullulan or action property with respect to amylopectin are improved over the enzyme to be mutated.

The mutated enzyme is an amino acid sequence wherein in the amino acid sequence of enzyme (enzyme to be mutated) hydrolyzing an α-1,6-glycosidic linkage, one or two or more amino acids selected from the group consisting of an amino acid corresponding to an amino acid at the 292 position, an amino acid corresponding to an amino acid at the 371 position, an amino acid corresponding to an amino acid at the 406 position, an amino acid corresponding to an amino acid at the 407 position, an amino acid corresponding to an amino acid at the 437 position, an amino acid corresponding to an amino acid at the 465 position, an amino acid corresponding to an amino acid at the 475 position, an amino acid corresponding to an amino acid at the 476 position; an amino acid corresponding to an amino acid at the 525 position, an amino acid corresponding to an amino acid at the 526 position, an amino acid corresponding to an amino acid at the 580 position and an amino acid corresponding to an amino acid at the 582 position of the amino acid sequence set forth in SEQ ID NO: 2 is/are substituted with another amino acid or deleted.

Preferably, the substituted or deleted amino acid is one or two or more amino acid selected from the group consisting of an amino acid corresponding to an amino acid at the 292 position, an amino acid corresponding to an amino acid at the 371 position, an amino acid corresponding to an amino acid at the 407 position, an amino acid corresponding to an amino acid at the 475 position, an amino acid corresponding to an amino acid at the 476 position, and an amino acid corresponding to an amino acid at the 582 position of the amino acid sequence set forth in SEQ ID NO: 2.

Further preferably, the substituted or deleted amino acid is an amino acid corresponding to an amino acid at the 476 position of the amino acid sequence set forth in SEQ ID NO: 2.

The kinds, origins and the like of the enzymes to be mutated are the same as those in the above-mentioned first aspect, and therefore the same description is omitted herein. A specific example of the enzyme to be mutated includes an enzyme (pullulanase of Bacillus subtilis strain 168) consisting of an amino acid sequence set forth in SEQ ID NO:2, an enzyme (pullulanase of Klebsiella pneumoniae ATCC9621) consisting of an amino acid sequence set forth in SEQ ID NO: 13, an enzyme (pullulanase of Bacillus sp. APC-9603) consisting of an amino acid sequence set forth in SEQ ID NO: 14, an enzyme (pullulanase of Bacillus deramificans) consisting of an amino acid sequence set forth in SEQ ID NO: 15, and an enzyme (isoamylase of Pseudomonas amyloderamosa) consisting of an amino acid sequence set forth in SEQ ID NO:16.

The mutated enzyme is characterized by having an amino acid sequence in which a certain position of the amino acid sequence of the enzyme before mutation (enzyme to be mutated) has been mutated. In a position other than the position related to the mutation, a part of the amino acid may be mutated or modified. Thus, it is described herewith for exemplary purposes a protein having the same function as compared with the mutated enzyme having the amino acid sequence to which the above-mentioned mutation has been given but having a difference in a part of the amino acid sequence (hereinafter, also referred to as "homologous protein"). The term "having a difference in a part of the amino acid sequence" typically means that the amino acid sequence is mutated (changed) by the deletion and substitution of one to several amino acids constituting the amino acid sequence, or addition of one to several amino acids, or the combination thereof. The difference in the amino acid sequence herein is acceptable as long as the properties related to hydrolysis of an α-1,6-glycosidic linkage are not radically reduced (preferably, in the limit substantially held). As long as this condition is satisfied, the position of difference in the amino acid sequence is not particularly limited and the difference may occur in a plurality of positions. The plurality herein signifies a numerical value corresponding to less than about 30%, preferably less than about 20%, further preferably less than about

10%, yet further preferably less than about 5%, and most preferably less than about 1% with respect to the entire amino acid. That is to say, homologous protein has, for example, about 70% or more, preferably about 80% or more, further preferably about 90% or more, yet further preferably about 95% or more and most preferably about 99% or more of similarly to the amino acid sequence of the mutated enzyme.

The mutated enzyme can be used for arbitrary applications that need hydrolyzing an α-1,6-glycosidic linkage. For example, the mutated enzyme can be used for production of maltooligosaccharide such as glucose, maltose, maltotriose, maltotetraose, maltopentaose, and maltohexaose, and for the improvement of rice cooking, and the like. The amount of the mutated enzyme to be used can be appropriately set so that the intended effect can be exhibited. For example, the enzyme reaction can be carried out under conditions so that the enzyme concentration in the reaction solution becomes about 10 nM to about 100 µM.

### 4. Nucleic acid encoding mutated enzyme

A nucleic acid related to the exemplary mutated enzyme is described herewith. That is to say, a gene encoding a mutated enzyme, nucleic acid that can be used as a probe for identifying a nucleic acid encoding a mutated enzyme, and nucleic acid that can be used as a primer for amplifying or mutating a nucleic acid encoding the mutated enzyme is disclosed herewith as an example.

A gene encoding a mutated enzyme is typically used for preparing a mutated enzyme. A genetically engineering preparation method using a gene encoding a mutated enzyme makes it possible to obtain a mutated enzyme in a more homogeneous state. Furthermore, the method is a suitable for preparing a large amount of mutated enzymes. Note here that the application of the gene encoding a mutated enzyme is not limited to preparation of a mutated enzyme. For example, the nucleic acid can be used as an research tool for the purpose of elucidating the mechanism of action of a mutated enzyme, or as a tool for designing or preparing a further mutant of an enzyme.

In the present invention, the "gene encoding a mutated enzyme" refers to nucleic acid capable of obtaining the mutated enzyme when it is expressed, and includes not only a nucleic acid having a base sequence corresponding to an amino acid sequence of the mutated enzyme but also a nucleic acid obtained by adding a sequence that does not encode the amino acid sequence to the nucleic acid. Furthermore, degeneracy of codon is also considered.

The nucleic acid can be prepared in an isolated state by standard genetic engineering technique, molecular biological technique, biochemical technique, and the like, with reference to sequence information disclosed in this specification or attached sequence list.

It is herewith described as an example a nucleic acid that has a base sequence having the same function as the base sequences of a gene encoding a mutated enzyme of the present invention but having a difference in a part of the base sequence (hereinafter, which is also referred to as "homologous nucleic acid." Furthermore, a base sequence specifying the homologous nucleic acid is also referred to as a "homologous base sequence"). An example of the homologous nucleic acid can include DNA encoding a protein including a base sequence in which one or a plurality of bases are substituted, deleted, inserted, added or inverted relative to the base sequences of a gene encoding a mutated enzyme of the present invention and having an activity capable of hydrolyzing an α-1,6-glycosidic linkage. Such substitution, deletion, or the like, may be occurred in a plurality of sites. The "plurality" herein differs depending upon the position or kinds of amino acid residues in a three-dimensional structure of a protein encoded by the nucleic acid codes, but the "plurality" of bases includes, for example, 2 to 40 bases, preferably 2 to 20 bases and more preferably 2 to 10 bases.

The above-mentioned homologous nucleic acid can be obtained by introduction of mutation by, for example, a treatment with a restriction enzyme; treatment with exonuclease, DNA ligase, etc; introduction of mutation by a site-directed mutagenesis (Molecular Cloning, Third Edition, Chapter 13, Cold Spring Harbor Laboratory Press, New York); random mutagenesis (Molecular Cloning, Third Edition, Chapter 13, Cold Spring Harbor Laboratory Press, New York), and the like. Furthermore, homologous nucleic acid can be obtained by other methods such as irradiation with ultraviolet ray.

A nucleic acid having a base sequence complementary to the base sequence of the gene encoding a mutated enzyme and a nucleic acid having a base sequence at least 60%, 70%, 80%, 90%, 95%, 99% or 99.9% identical to the base sequences of the gene encoding a mutated enzyme are also described herein as examples.

A further example relates to nucleic acid having a base sequence that hybridizes, under stringent conditions, to the base sequence complementary to the base sequences of the gene encoding a mutated enzyme or the homologous base sequence thereof. The "stringent conditions" herein denote a condition in which a so-called specific hybrid is formed and a non-specific hybrid is not formed. Such stringent conditions are well known to the person skilled in the art and can be set with reference to Molecular Cloning (Third Edition, Cold Spring Harbor Laboratory press, New York) or Current protocols in molecular biology (edited by Frederick M. Ausubel et al., 1987). An example of the stringent conditions includes a condition in which a DNA is incubated in a hybridization solution (50% formamide, 10 x SSC (0.15 M NaCl, 15 mM sodium citrate, pH 7.0), 5 × Denhardt solution, 1% SDS, 10% dextran sulfate, 10 µg/ml denatured salmon sperm DNA, 50 mM phosphate buffer (pH7.5)) at about 42°C to about 50°C, followed by washing with 0.1 × SSC and 0.1% SDS at about 65°C to about 70°C. A more preferable example of the stringent conditions can include a condition using a hybridization solution (50% formamide, 5 × SSC (0.15 M NaCl, 15 mM sodium citrate, pH 7.0), 1 × Denhardt solution, 1% SDS, 10% dextran sulfate, 10 µg/ml denatured salmon sperm DNA, 50 mM phosphate buffer (pH 7.5)).

A further example describes a base sequence of the gene encoding a mutated enzyme of the present invention or a nucleic acid (a nucleic acid fragment) having a part of a base sequence complementary to the base sequence. Such a nucleic acid fragment can be used for detecting, identifying and/or amplifying the nucleic acid having a base sequence of a gene encoding a mutated enzyme. The nucleic acid fragment is designed to include at least a part for hybridizing a continuous nucleotide part (for example, about 10 to about 100 base length, preferably about 20 to about 100 base length, and further preferably about 30 to about 100 base length) in the base sequence of the gene encoding a mutated enzyme.

When the nucleic acid fragment is used as a probe, it can be labeled. Labeling can be carried out by using a fluorescence material, enzyme, and radioactive isotope.

A yet further example relates to recombinant DNA including a gene (a gene encoding a mutated enzyme). The recombinant DNA is provided in a form of, for example, a vector. The term "vector", in this specification refers to a nucleic acid molecule that can transport a nucleic acid inserted therein to the inside of a target such as a cell.

In accordance with the purpose of use (cloning, protein expression), and by considering the kinds of host cells, an appropriate vector is selected. Specific examples of a vector include a vector using Escherichia coli as a host (M13 phage or the modified body thereof, λ phage or the modified body thereof, pBR322 or the modified body thereof (pB325, pAT153, pUC8, etc.) and the like), a vector using yeast as a host (pYepSec1, pMFa, pYES2, etc.), a vector using insect cells as a host (pAc, pVL, etc.), a vector using mammalian cells as a host (pCDM8, pMT2PC, etc.).

The vector may be an expression vector. The term "expression vector" is a vector capable of introducing the nucleic acid inserted therein into the target cells (host cells) and expressing in the cells. The expression vector usually includes a promoter sequence necessary for expression of the inserted nucleic acid and an enhancer sequence for promoting the expression, and the like. An expression vector including a selection marker can be used. When such an expression vector is used, by using the selection marker, the presence or absence of the introduction of an expression vector (and the degree thereof) can be confirmed.

Insertion of the nucleic acid into a vector, insertion of the selection marker gene (if necessary), and insertion of a promoter (if necessary), and the like, can be carried out by a standard recombination DNA technology (see, for example, Molecular Cloning, Third Edition, 1.84, Cold Spring Harbor Laboratory Press, New York, a well-known method using restriction enzyme and DNA ligase).

As the host cell, from the viewpoint of ease in handling, it is preferable that a bacterial cell such as Escherichia coli is used. However, the host cell is not limited to the bacterial cell as long as it can reproduce the recombinant DNA and express a gene of the mutated enzyme. As a preferable example of the host includes T7 system promoter, Escherichia coli BL21 (DE3) pLysS can be used. In other case, Escherichia coli JM109 can be used..

A further example relates to a microorganism (that is, transformant) carrying a recombinant DNA. The microorganism can be obtained by the transfection or transformation using the above-mentioned vector. For example, a calcium chloride method (J. Mol. Biol., Vol. 53, page 159 (1970)), a Hanahan method (J. Mol. Biol., Vol. 166, page 557 (1983)), a SEM method (Gene, Vol. 96, page 23 (1990)), a method by Chung et al. (Proceeding of the national Academy of Sciences of the USA, Vol.86, page 2172 (1989)), a calcium phosphate coprecipitation method, electroporation (Potter, H. et al., Proc. Natl. Acad. Sci. U.S.A. 81, 7161-7165 (1984)), lipofection (Felgner, P.L. et al., Proc. Natl. Acad. Sci. U.S.A. 84,7413-7417 (1984)), and the like.

The above described microorganism can be used for producing a mutated enzyme. That is to say, it is herewith described a method for producing a mutated enzyme by using the above-mentioned microorganism. The production method of the present invention includes at least a step of culturing the above-mentioned microorganism under the conditions in which the mutated enzyme of the present invention is produced. In general, in addition to this step, a step of collecting (separating and purifying) the produced protein is carried out.

The microorganism (transformant) is cultured in a usual manner. As a carbon source to be used for a medium, a carbon compound that can be assimilated may be used. An example may include glucose, sucrose, lactose, maltose, syrup, pyruvic acid, and the like. Furthermore, as a nitrogen source, any available nitrogen compounds may be used. An example may include peptone, meat extract, yeast extract, casein hydrolysate, soybean cake alkali extract, and the like. Besides, salts of phosphate, carbonate, sulfate, magnesium, calcium, potassium, iron, manganese, zinc, and the like, certain amino acids, certain vitamins, can be used if necessary.

On the other hand, the culture temperature can be set 30 to 40°C (preferably about 37°C). Culture time can be set considering the growing property of transformant to be cultured or production property of mutated enzyme, and the like. The pH of the medium is adjusted in a range in which the transformant is grown and an enzyme is produced. Preferably, pH of the medium is adjusted to about 6.0 to 9.0 (preferably, around pH 7.0).

A culture solution containing a bacterial body producing a mutated enzyme can be used as an enzyme solution as it is or after concentration or removing of impurities are carried out. Generally, however, a mutated enzyme is once collected from a culture solution or a bacterial body. When the produced mutated enzyme is secreting type protein, the mutated enzyme can be collected from the culture solution, and in other case, the mutated enzyme can be collected from the bacterial body. When the mutated enzyme is collected from the culture solution, purified products of the mutated enzyme can be obtained by, for example, subjecting the culture supernatant to filtering or centrifugation so as to remove insoluble matters, followed by carrying out separation and purification by the combination of vacuum concentration, membrane concentration, salting out using ammonium sulfate and sodium sulfate, fractional precipitation by methanol, ethanol or acetone, dialysis, heat treatment, isoelectric point treatment, various chromatography such as gel filtration chromatography, adsorption chromatography, ion exchange chromatography and affinity chromatography (for example, gel filtration by Sephadex gel (Pharmacia Biotech) and the like, DEAE sepharose CL-6B (Pharmacia Biotech), octyl sepharose CL-6B (Pharmacia Biotech), CM sepharose CL-6B (Pharmacia Biotech)), and the like. On the other hand, when the mutated enzyme is collected from a bacterial body, purified products of the mutated enzyme can be obtained by subjecting a culture solution to filtration and centrifugation to collect a bacterial body, followed by destructing the bacterial body by mechanical method such as pressure treatment and ultrasonication, or by an enzymatic method using lysozyme, and then carrying out separation and purification as mentioned above.

Purified enzyme obtained as mentioned above can be provided in a state of powder by, for example, freeze drying, vacuum drying or spray drying. At this time, a purified enzyme may be solved in phosphate buffer, triethanolamine buffer, Tris hydrochloric acid buffer, GOOD buffer in advance. Preferably, phosphate buffer and triethanolamine buffer can be used. An example of the GOOD buffer may include PIPES, MES or MOPS.

Hereinafter, the present invention is described further specifically.

### [Example]

### 1. Preparation of Bacillus subtilis recombinant pullulanase

### (1) Cloning of Bacillus subtilis pullulanase gene

A gene *AmyX* (GenBank Accesion No. NC 000964) encoding pullulanase, which is found in the genome sequence of Bacillus subtilus, was amplified by PCR as follows. A chromosome DNA of Bacillus subtilis strain 168 isolated by the method by Sambrook et al. (Molecular Cloning: a laboratory manual, 2nd Edition, Cold Spring harbor Laboratory Press, 1989) was used as a template of PCR, and oligonucleotides of SEQ ID NO: 3 and SEQ ID NO: 4 were synthesized to form a primer. In the PCR reaction, 30 cycles of reactions of 94°C / 2 minutes, 94°C / 15 seconds - 60°C / 30 seconds and amplification of 68°C / 4 minutes were carried out by using KOD plus system (TOYOBO). The obtained PCR fragment was treated with restriction enzymes *NcoI* and *XhoI,* and then linked to plasmid pET21d (Novagen), which had been cleaved with the both restriction enzymes. Thus, an expression plasmid pEBSP was obtained. It was confirmed by determining the base sequence that the obtained PCR fragment encoded pullulanase correctly.
SEQ ID NO: 3
   5'-GGCCATGGTCAGCATCCGCCGCAGCTTCGA-3'
   (underlined part represents a restriction enzyme *NcoI* recognition site)
SEQ ID NO: 4
   5'-GGCTCGAGTCAAGCAAAACTCTTAAGATCT-3'
   (underlined part represents a restriction enzyme *XhoI* recognition site)

### (2) Expression of Bacillus subtilis recombinant pullulanase

The above-mentioned expression plasmid was introduced into Escherichia coli HMS174 (DE3) (Novagen) by transformation. The obtained transformant was inoculated on LB medium (500 ml) containing 100 µ/ml ampicillin and the medium was shaken at 37°C. At the time when the turbidity at 600 nm reached 0.6 to 0.8, isopropylthiogalactoside (IPTG) was added so that the final concentration became 0.5 mM and further cultured at 18°C for 60 hours. The bacterial bodies were collected from the culture solution by centrifugation and suspended in a buffer solution (20 mM tris-hydrochloric acid (pH 8.1) / 5 mM EDTA / 20 mM β-mercaptoethanol / 0.2 mM PMSF).

### (3) Purification of Bacillus subtilis recombinant pullulanase

The suspension obtained in (2) was subjected to ultrasonication (4°C, 30 minutes, 200 µA) and then subjected to centrifugation (14000 rpm, 4°C, 30 minutes) to obtain a crude solution. To this solution, 30% saturated ammonium sulfate was added. Then, impurities were removed by centrifugation. To this centrifuged supernatant, 60% saturated ammonium sulfate was added. Then, precipitates were collected by centrifugation. The precipitates were dissolved in the buffer solution of (2) containing 20% saturated ammonium sulfate, and subjected to Butyl-Toyopearl 650M column (TOSOH CORPORATION) that had been equilibrated by the buffer solution containing 20% saturated ammonium sulfate, and eluted at the concentration gradient of 20% to 0% of ammonium sulfate. The obtained pullulanase active fraction was dialyzed to buffer solution of (2), and subjected to HiLoad Q Sepharose Column (Amersham) that had been equilibrated by the same buffer solution, and eluted at the concentration gradient of 0 to 0.5 M of NaCl to obtain active fraction. This fraction was dialyzed to the buffer solution of (2), and subjected to Mono Q Column (Amersham) that had been equilibrated by the same buffer solution, and eluted at the concentration gradient of 0 to 0.5 M of NaCl to obtain purified Bacillus subtilis recombinant pullulanase. The analysis result of N-terminal amino acid sequence of this purified enzyme was MVSIRRSFEA and completely identity to gene sequence.

### 2. X-ray analysis of Bacillus subtilis pullulanase

### (1) Crystallization

Crystallization of Bacillus subtilis recombinant pullulanase was carried out by the following procedure. Firstly, screening was carried out by a sitting drop vapor diffusion method by using a 96-well plate (product by Emerald Biostructure and Hampton Research). After 24 hours at 20°C, small and thin crystal was observed in Wizard II No.8 well. Next, by changing the molecular weight and concentration of polyethylene glycol (PEG) or the concentration of buffer solution or salts, conditions were optimized. Finally, a crystal was obtained by a hanging drop vapor diffusion method by using a 24-well plate. The hanging drop (6 µl) consisting of 3 µl of enzyme solution (10 mg/ml) and 3 µl of reservoir solution (10% PEG4000, 0.1 M acetate buffer, pH 5.2, 0.2 M Mg CH₃COO)₂) was used and incubated for one to two days. Thus, diamond shaped crystal was obtained. Prior to the X-ray analysis, treatment with a reservoir solution containing 30% glycerol was carried out and then cooled instantly by using liquid nitrogen (-173°C).

### (2) X-ray analysis

X-ray diffraction data were collected at a temperature of liquid nitrogen by using Synchrotron radiation BL-38B1 (SPring-8, Hyogo, Japan) and processed by using HKL2000 program. X-ray diffraction data of 2.1 Ǻ resolution were collected so as to determine the crystallographic parameter. Space group was P2₁2₁2₁ and lattice constant was a = 70.57 Ǻ, b =127.68 Ǻ, and c = 189.25 Ǻ.

### (3) Determination of three-dimensional structure

A three-dimensional structure was determined at the resolution of 2.1 Ǻ by a molecule substitution method using an atomic coordinate (PDB accession code 2FGZ) of pullulanase of Klebsiella pneumonia. The substitution of molecules and the refinement of structure were carried out by using program CNS_SOLVE ver.11. Data related to statistics of data collection and refinement are shown in Table 1.

**[Table 1]**

| Crystal | Bacillus subtilis pullulanase |
|---|---|
| Data collection | Spring-8 BL38B1 |
| wavelength (Ǻ) | 0.8 |
| detector | Jupiter 210 CCD |
| space group | *P*2₁2₁2₁ |
| lattice constant (Ǻ) | A=70.57, b=127.68, c=189.25 |
| resolution (Ǻ) | 46.3 - 2.1 (2.18 - 2.10) |
| measured reflection | 590,744(38,618) |
| unique reflection | 99,003 (9,775) |
| integrity (%) | 99.6 (99.6) |
| *R*merge (%) | 7.8 (31.4) |
| determination of structure | molecule substitution method |
| Refinement | |
| Residues/Water/Ca2+/α-CD | 712 x 2, 673, 2, 0 |
| Resolution (Ǻ) | 15.0 - 2.1 (2.17 - 2.1) |
| used reflection | 98,636 (8,725) |
| r.m.s. bond (Ǻ), | 0.005, |
| angle (°) | 1.25 |
| R and Rfree | 0.201, 0.238 (0.224, 0.252) |

### 3. X-ray analysis of Bacillus subtilis pullulanase (in a state of containing α-cyclodextrin (CD))

Since α-cyclodextrin (CD) is cyclic oligosaccharide and is an analog having a structure of amylose helix consisting of six glucose residues, it has often been used for studying as an inhibitor of an amylolytic enzyme such as amylase and pullulanase. In order to determine the substrate binding site of Bacillus subtilis pullulanase, an analysis of the three-dimensional structure of enzyme containing CD as a ligand (hereinafter, referred to as "CD-containing Bacillus subtilis pullulanase") was carried out. The analysis of the three-dimensional structure was carried out basically by the same method as 2. except that purified Bacillus subtilis recombinant pullulanase was prepared in 1. and 20 mM CD was added in the hanging drop at the time of crystallization. Data related to statistics about data collection and data are shown in Table 2.

**[Table 2]**

| Crystal | Bacillus subtilis pullulanase containing α-cyclodextrin |
|---|---|
| Data collection | Spring-8 BL38B1 |
| wavelength (Ǻ) | 1.0 |
| detector | Jupiter 210 CCD |
| space group | *P*2₁2₁2₁ |
| lattice constant (Ǻ) | A=70.36, b=127.86, c=189.29 |
| resolution (Ǻ) | 40.0 - 2.2 (2.28 - 2.20) |
| measured reflection | 4.08,687 (35,910) |
| unique reflection | 87,392 (8,550) |
| integrity (%) | 99.8 (99.3) |
| *R*merge (%) | 9.2 (42.4) |
| determination of structure | molecule substitution method |
| Refinement | |
| Residues/Water/Ca2+/α-CD | 712 x 2, 673, 2, 2 |
| Resolution (Ǻ) | 15.0 - 2.2 (2.28 -2.2) |
| used reflection | 87,004 (8,495) |
| r.m.s. bond (Ǻ), | 0.005, |
| angle (°) | 1.25 |
| R and Rfree | 0.197, 0.238 (0.249, 0.282) |

A model of the three-dimensional structure of the obtained CD-containing Bacillus subtilis pullulanase is shown in Fig. 1. Note here that data of atomic coordinate are shown in the last part of the specification.

### 4. Comparison analysis between three-dimensional structure of CD-containing Bacillus subtilis pullulanase and three-dimensional structure of Klebsiella pneumoniae pullulanase

The three-dimensional structure of CD-containing Bacillus subtilis pullulanase obtained in 3. and the three-dimensional structure of Klebsiella pneumoniae pullulanase (J. Mol. Biol., 359 (3): 690-707 (2006), RCSB Protein Data Bank code 2FHF) were superimposed onto each other by Least Square method by using program TURBO-FRODO, α carbons of amino acids of 499 residues were common within 2 Ǻ with the root means square deviation (r. m. s. d.) of 1.09 Ǻ. Fig. 2 shows the superimposed results. Fig. 3 is an enlarged view showing a CD binding site. Fig. 3 shows a CD binding site with respect to Bacillus subtilis pullulanase and the binding site of maltotetraose with respect to Klebsiella pneumoniae pullulanase (G4) (see, non-patent document 1). An amino acid residue (upper stage) in a position capable of forming CD and hydrogen binding in Bacillus subtilis pullulanase is shown together with the corresponding amino acid residue (lower stage) in Klebsiella pneumoniae pullulanase. Furthermore, amino acid residue (lower stage) that has been deduced to be involved in binding to G4 in Klebsiella pneumoniae pullulanase and the corresponding amino acid residue (upper stage) in Bacillus subtilis pullulanase are also shown together.

Although a domain (N1 domain) in the N terminal region observed in Klebsiella pneumoniae pullulanase was not observed in Bacillus subtilis pullulanase, both enzymes were very similar to each other in a catalytic region and a domain (A domain) as a nucleus including a substrate binding region. Furthermore, a CD binding site of Bacillus subtilis pullulanase is in the vicinity of G4 binding site of Klebsiella pneumonia.

As shown in Fig. 3, in Bacillus subtilis pullulanase, twelve in total of amino acid residues that are thought to be involved in binding to the substrate were successfully identified as an amino acid position in the binding site of CD: Y292 (tyrosine the 292 position), G371 (glycine at the 371 position), D406 (aspartic acid at the 406 position), L407 (leucine at the 407 position), W437 (tryptophane at the 437 position), D465 (aspartic acid at the 465 position), T475 (threonine at the 475 position), F476 (phenyl alanine at the 476 position), D525 (aspartic acid at the 525 position), N526 (asparagine at the 526 position), N580 (asparagine at the 580 position), and Y582 (tyrosine at the 582 position). Furthermore, in Klebsiella pneumoniae pullulanase, amino acid corresponding to these amino acid residues, that is, Y559 (tyrosine at the 559 position), C643 (cysteine at the 643 position), D677 (aspartic acid at the 677 position), L678 (leucine at the 678 position), W708 (tryptophane at the 708 position), D734 (aspartic acid at the 734 position), P745 (proline at the 745 position), F746 (phenyl alanine at the 746 position), D834 (aspartic acid at the 834 position), N835 (asparagine at the 835 position), D890 (aspartic acid at the 890 position), and Y892 (tyrosine at the 892 position) were successfully determined. Among the thus determined amino acids, D677, W708, D734, D834, N835 and D890 were amino acids deduced to be involved in binding to G4 in the above-mentioned report.

### 5. Comparison analysis between three-dimensional structure of CD-containing Bacillus subtilis pullulanase and three-dimensional structure of Pseudomonas amyloderamosa isoamylase

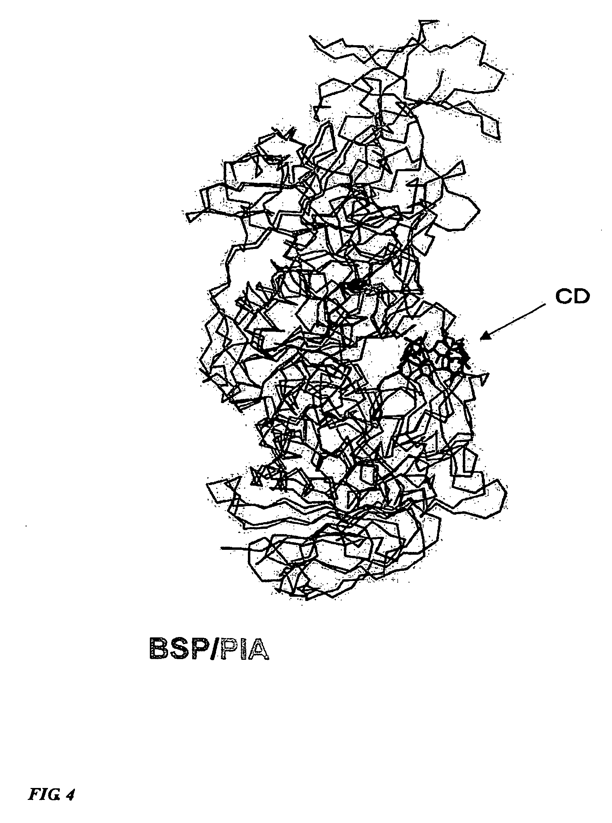

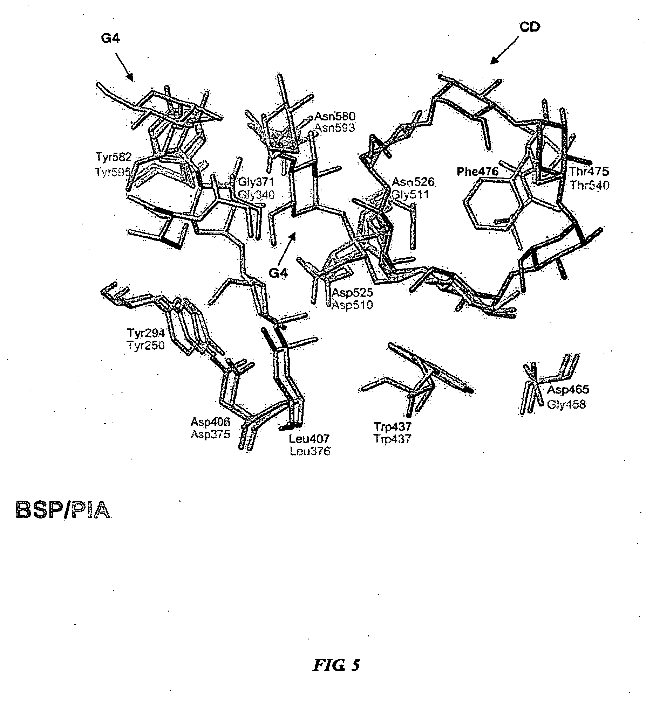

The three-dimensional structure of CD-containing Bacillus subtilis pullulanase obtained in 3. and the three-dimensional structure of Pseudomonas amyloderamosa isoamylase (J. Mol Biol., 281 (5): 885-97 (1998), RCSB Protein Data Bank code 1BF2) were superimposed onto each other by Least Square method by using program TURBO-FRODO, α carbons of amino acids of 421 residues were common within 2 Ǻ with the root means square deviation (r. m. s. d.) of 1.13 Ǻ. Fig. 4 shows the superimposed results. Fig. 5 is an enlarged view showing a CD binding site.

The both enzymes do not have a domain (N1 domain) in the N terminal region observed in Klebsiella pneumoniae pullulanase and is very similar in domain (A domain) in terms of, for example, a nucleus including a catalytic region or a substrate binding region. The amino acid residue (upper stage) in the position capable of forming hydrogen binding to CD in Bacillus subtilis pullulanase is shown together with the corresponding amino acid residue (lower stage) in Pseudomonas amyloderamosa isoamylase. Furthermore, amino acid residues corresponding to the amino acids deduced to be involved in binding to G4 in Klebsiella pneumoniae pullulanase (upper stage: Bacillus subtilis pullulanase, and lower stage: Pseudomonas amyloderamosa).

### 6. Formation of alignment of amino acid sequence based on three-dimensional structure

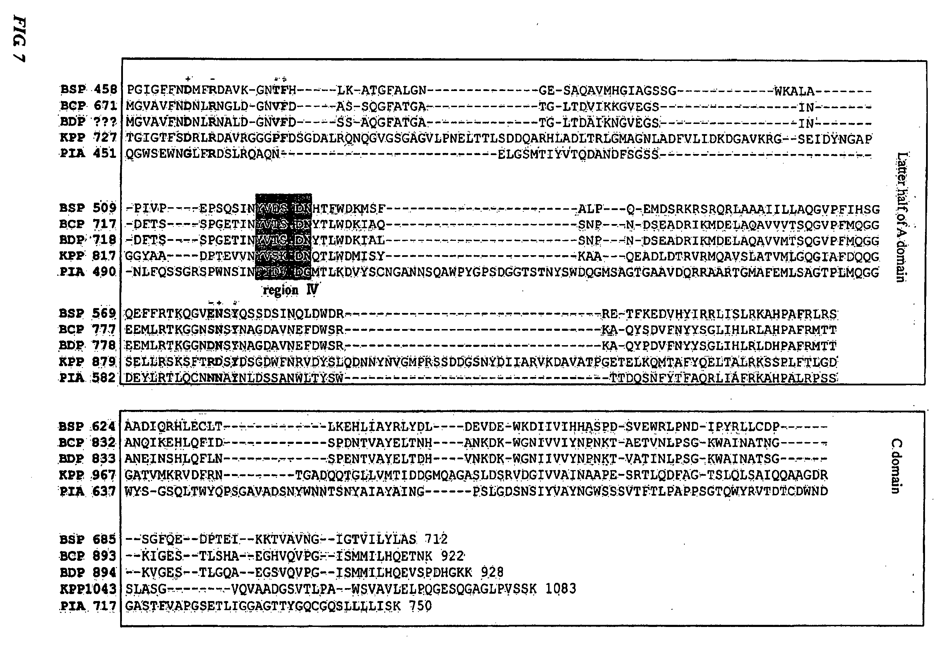

With the use of the result of comparison of the three-dimensional structures of CD-containing Bacillus subtilis pullulanase, Klebsiella pneumoniae pullulanase and Pseudomonas amyloderamosa isoamylase obtained in 4. and 5., alignments of amino acid sequences with respect to these three enzymes were formed based on the three-dimensional structures. Furthermore, alignments of amino acid sequences with respect to two kinds of pullulanases (pullulanase from Bacillus sp. APC-9603 and pullulanase from Bacillus deramificans) being derived from strains related to Bacillus subtilis and having a high homology in primary structure were formed by using ClustalW program. Alignments were formed with respect to five kinds of enzymes with these two alignments added (see Figs. 6 and 7). As shown in Figs. 6 and 7, these five kinds of enzymes have extremely high partial similarity.

### 7. Production of Bacillus subtilis recombinant pullulanase mutant

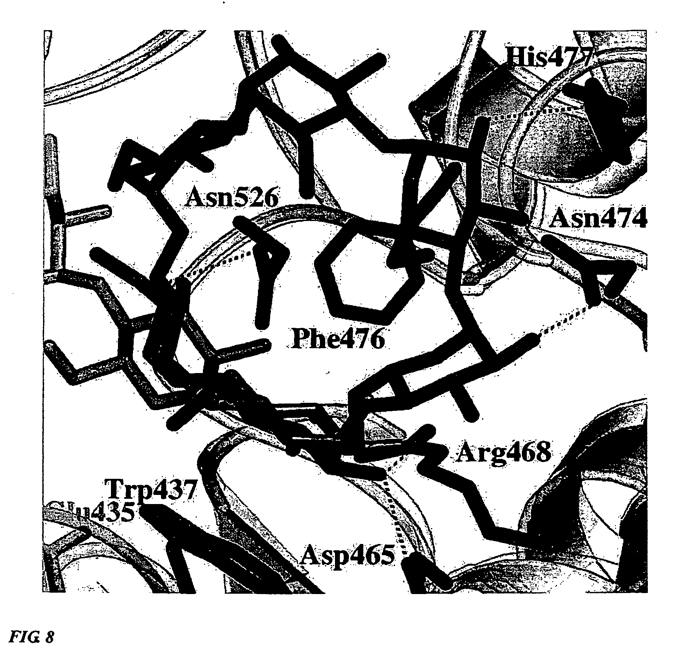

From the three-dimensional structure of Bacillus subtilis pullulanase to which CD obtained in 3. was bonded as a ligand, it is assumed that the side chain of Phe476 of Bacillus subtilis pullulanase is incorporated in the cyclic structure of the CD as a substrate analog (Fig. 8) and has something to do with the recognition of a substrate. Under this assumption, Phe476 was substituted with another amino acid by the following procedure and the effect thereof was verified.

Based on the sequence of gene *AmyX* encoding Bacillus subtilis pullulanase, a primer for substituting Phe476 with Gly or Ser was synthesized. In order to substitute Phe476 with Gly or Ser, forward primers and the corresponding reverse primers set forth in SEQ ID NOs: 5 and 6 were synthesized, respectively, and the concentration was adjusted to 100 ng/µl. By using an expression plasmid pEBSP of Bacillus subtilis recombinant pullulanase as a template, the following PCR reaction was carried out: 1.5 µl (30ng/µl) of pEBSP, 5 µl of 10 x PCR buffer solution (one attached to the below-mentioned DNA polymerase), 1.0 µl (2.5 mM each) of dNTP, 1.25 µl each of mutated primer sets (forward and reverse primers), 39 µl of sterile water and 1 µl (2.5 U) of Pfu Turbo Hotstart DNA polymerase (Stratagene) were prepared; 30 cycles of reactions at 95°C for 30 seconds (denaturation) - at 55°C for 60 seconds (annealing) - at 68°C for 12 minutes (elongation) were carried out; and finally amplification at 68°C for 5 minutes was carried out. The obtained PCR product was confirmed by 1% agarose gel electrophoresis, and then the rest of the PCR product was treated with restriction enzyme Dpn I to degrade a methylated template plasmid and transformed to Escherichia coli competent cell DH5α strain. Plasmid DNA was isolated from the obtained ampicillin resistance transformant, the base sequence was confirmed and the intended mutated gene was obtained. A plasmid having the intended mutated gene was introduced into an expression host Escherichia coli HMS174 (DE3) strain to express and purify the mutated pullulanase of Bacillus subtilis similar to 1. (2) and (3).

SEQ ID NO: 5
   5'-GTAAAAGGGAACACCGGTCACCTTAAGGCAATA-3'
SEQ NO: 6
   5'-GTAAAAGGGAACACCTCTCACCTTAAGGCAATA-3'

### 8. Substrate specificity of Bacillus subtilis recombinant pullulanase mutant

The kinetic parameters with respect to pullulan and amylopectin of Bacillus subtilis recombinant pullulanase mutant prepared in 4. were measure according to the method described in J Biochem (Tokyo), 116(6): 1264-8 (1994). Pullulan or amylopectin having various concentrations were dissolved in 50 mM acetate buffer (pH 5.6) and reacted at 25°C. The concentration of a reducing sugar contained in the regularly sampled reaction solution was determined by a Park-Johnson method, and the reaction rate was measured from the increasing rate of the reducing sugar. Km value and Kcat value were obtained by curve fitting into Michaelis-Menten equation by the non-linear minimum square method. Results are shown in Table 3.

**[Table 3]**

| | pullulan | | amylopectin | |
|---|---|---|---|---|
| | *K*m (mg/ml) | *k* cat (s⁻¹) | *K*m (mg/ml) | *k* cat (s⁻¹) |
| wild type pullulanase | 14.93 | 4317 | 199.20 | 4121 |
| mutant type (F476G) | 20.45 | 5362 | 53.19 | 1198 |
| mutant type (F476S) | 645.16 | 10365 | 156.25 | 2146 |

When the wild type and the mutant types are compared with each other, they are shown to be different in the Km value and Kcat value. For example, in the mutant type (F476G: mutated enzyme in which an amino acid residue at the 476 position is changed from phenyl alanine to glycine), the Km value when amylopectin is used as a substrate is considerably lower than that of the wild type, which shows that the affinity to amylopectin is radically improved. Furthermore, in the mutant type (F476S: mutated enzyme in which an amino acid residue at the 476 position is changed from phenyl alanine to serine), the Km value when amylopectin is used as a substrate is lower than that of the wild type while the Km value when pullulan is used as a substrate is radically higher than that of the wild type, which shows that the substrate specificity has radically changed. Thus, introduction of mutation enabled the action property with respect to pullulan and amylopectin to be changed.

### 9. Cloning of Klebsiella pneumoniae pullulanase gene

### (1) Cloning of Klebsiella pneumoniae pullulanase gene

Two primers (SEQ ID NOs: 7 and 8) were synthesized with reference to the base sequence of pullulanase of Klebsiella aerogence W70 strain (J. Bacteriol. 169 (5), 2301-2306 (1987) and J Bacteriol. 174(9): 3095.(1992), GenBank accession No. M16187). The PCR reaction was carried out under the following conditions by using chromosome DNA of Klebsiella pneumoniae ATCC9621 strain that had been isolated by the method by Sambrook et al. (Molecular Cloning: a laboratory manual, 2nd Edition, Cold Spring harbor Laboratory Press, 1989) as a template. The PCR reaction was carried out by using Accu TaqTM LA DNA polymerase system (Sigma), the reaction included one cycle of reaction at 98°C for 30 seconds - at 59°C for 20 seconds - at 68°C for 3 minutes; 29 cycles of reaction at 98°C for 15 seconds - at 59°C for 20 seconds - at 68°C for 3 minutes; and reaction at 68°C for 10 minutes of amplification was carried out. The obtained PCR fragment was subjected to TA cloning by using a pGEM-T vector (Promega). It was confirmed that the obtained PCR fragment encodes pullulanase correctly by determining the base sequence. The base sequence of the obtained PCR fragment and the amino acid sequence encoded thereby are shown in SEQ ID NO: 12 and SEQ ID NO: 13, respectively.

SEQ ID NO: 7
   5'-TTATTGCCGGAGAGTGGCGA-3'
SEQ ID NO: 8
   5'-CCAGACTGCTGACAAAGTGC-3'

### (2) Expression of Klebsiella pneumoniae pullulanase gene

In order to construct an expression vector, primers shown in SEQ ID NOs: 9 and 10 were synthesized, and PCR reaction was carried out by using a pullulanase gene of Klebsiella pneumoniae as a template. The obtained PCR product was treated with restriction enzymes *N*de I and *Xba* I, and linked to a plasmid pCold-I (TAKARA) to obtain an expression plasmid pCold-KPP of Klebsiella pneumoniae pullulanase. The plasmid was introduced into Escherichia coli JM109 (TAKARA) by transformation. Culture of the obtained transformant and purification of the expressed recombinant pullulanase were carried out according to the method described in 1.

SEQ ID NO: 9
   5'-GGAATTCCATATGGATGTCGTCGTCCGCTTACCG-3'(34 mer)
SEQ ID NO:10
   5'-GCTCTAGATTATTTAACTGCTCACCGGCAG-3' (29 mer)

### 10. Production of Klebsiella pneumoniae pullulanase mutant

The results obtained by analysis by superimposing the three-dimensional structure of CD-containing Bacillus subtilis pullulanase obtained in 3. and the three-dimensional structure of Klebsiella pneumoniae pullulanase (RCSB Protein Data Bank code 2FHF) onto each other (Figs. 2 and 3) and the results of amino acid sequence alignments (Figs. 6 and 7), an amino acid residue of pullulanase of Klebsiella pneumonia corresponding to Phe 476 residue that was thought to be involved in recognition of a substrate in Bacillus subtilis pullulanase was thought to be a Phe746 residue. Then, the Phe746 residue of pullulanase of Klebsiella pneumonia was substituted with Ala (or other amino acid).

Forward primers and the corresponding reverse primers set forth in SEQ ID NO: 11 were synthesized respectively in order to replace Phe746 with Ala in Klebsiella pneumoniae, and the concentration was adjusted to 100 ng/µl. By using an expression plasmid pCold-KPP of Klebsiella pneumoniae pullulanase as a template, the following PCR reaction was carried out: 1.5 µl (30 ng/µl) of pEBSP, 5 µl of 10 x PCR buffer solution (one attached to the below-mentioned DNA polymerase), 1.0 µl (2.5 mM each) of dNTP, 1.25 µl each of mutated primer sets (forward and reverse primers), 39 µl of sterile water and 1 µl (2.5 U) of Pfu Turbo Hotstart DNA polymerase (Stratagene) were prepared; 30 cycles of reactions at 95°C for 30 seconds (denaturation) - at 55°C for 60 seconds (annealing) - at 68°C for 12 minutes (elongation) were carried out; and finally amplification at 68°C for 5 minutes was carried out. The obtained PCR product was confirmed by 1% agarose gel electrophoresis, and then the rest of the PCR product was treated with restriction enzyme *Dpn I* to degrade a methylated template plasmid and transformed to Escherichia coli competent cell DH5α strain. Plasmid DNA was isolated from the obtained ampicillin resistance transformant, the base sequence was confirmed and the intended mutated gene was obtained. A plasmid having the intended mutated gene was introduced into an expression host Escherichia coli HMS174 (DE3) strain to express and purify the mutated pullulanase of Klebsiella pneumoniae similar to 1. (2) and (3).
SEQ ID NO: 11
5'-GCCGGCCGACTCCGGTGAC-3'

### 11. Substrate specificity of Klebsiella pneumoniae pullulanase mutant

The kinetic parameters with respect to pullulan and amylopectin of mutated pullulanase (F746A) and wild type pullulanase (WT) of Klebsiella pneumoniae prepared in 10. were measure according to the method described in J Biochem (Tokyo), 116(6): 1264-8 (1994). Pullulan or amylopectin having various concentrations were dissolved in 50 mM acetate buffer (pH 5.6) and reacted at 25°C. The concentration of a reducing sugar contained in the regularly sampled reaction solution was determined by a Park-Johnson method, and the reaction rate was measured from the increasing rate of the reducing sugar. Km value and Kcat value were obtained by curve fitting into Michaelis-Menten equation by the non-linear minimum square method. Results are shown in Table 4.

**[Table 4]**

| | pullulan | | amylopectin | |
|---|---|---|---|---|
| | *K*m (%) | *K*cat (s⁻¹) | *K*m (%) | *K*cat (s⁻¹) |
| wild type pullulanase | 0.00091 | 52.0 | 0.0063 | 7.0 |
| mutant type (F746A) | 0.00045 | 18.2 | 0.0083 | 5.2 |

Km value when pullulan was used for a substrate was lowered in the case of mutated enzyme as compared with the case of the wild type enzyme. On the other hand, Km value when amylopectin was used for a substrate was increased. From the results, it can be evaluated that in the mutated enzyme, the affinity to pullulan is improved (the affinity to amylopectin is lowered). Furthermore, Kcat value is different between the wild type and the mutant type. Thus, introduction of mutation enabled the action property with respect to pullulan and amylopectin to be changed.

Note here that the enzyme in which the affinity to a certain substrate is improved can act on a smaller amount of substrate. Such a property is advantageous in that the presence of the small amount of substrates is required to be measured.

### [Industrial Applicability]

A designing method and a preparation method of the present invention are used for improving an enzyme hydrolyzing an α-1,6-glycosidic linkage. With a mutated enzyme whose affinity and specificity to a substrate are improved, the increase in the yield of products and reduction of the amount of enzyme to be used (additive amount) can be achieved. On the other hand, it can be expected that the use of the designing method and the preparation method of the present invention provide a mutated enzyme usable to a novel application that has not been assumed with the use of wild type enzymes. That is to say, the present invention is capable of contributing to expansion of the applications of use of enzymes hydrolyzing an α-1,6-glycosidic linkage.

The atomic coordinates of three-dimensional structure of CD-containing Bacillus subtilis (Bacillus subtilis strain 168) are shown below.
REMARK coordinates from restrained individual B-factor refinement
REMARK refinement resolution: 15.0 - 2.2 A
REMARK starting r= 0.1970 free_r= 0.2378
REMARK final r= 0.1966 free_r= 0.2377
REMARK B rmsd for bonded mainchain atoms= 1.246 target= 1.5
REMARK B rmsd for bonded sidechain atoms= 1.964 target= 2.0
REMARK B rmsd for angle mainchain atoms= 1.982 target= 2.0
REMARK B rmsd for angle sidechain atoms= 2.784 target= 2.5
REMARK rweight= 0.1000 (with wa= 2.63874)
REMARK target= mlf steps= 25
REMARK sg= P2(1)2(1)2(1) a= 70.327 b= 127.780 c= 189.274 alpha= 90 beta= 90 gamma= 90
REMARK parameter file 1 : CNS_TOPPAR:protein rep.param
REMARK parameter file 2 : CNS_TOPPAR:carbohydrate.param
REMARK parameter file 3 : CNS_TOPPAR:ion.param
REMARK parameter file 4 : CNS_TOPPAR:water_rep.param
REMARK parameter file 5 : act.par
REMARK molecular structure file: generate.mtf
REMARK input coordinates: minimize2.pdb
REMARK reflection file= 050715b138_bdpulcd.cv
REMARK ncs= none
REMARK B-correction resolution: 6.0 - 2.2
REMARK initial B-factor correction applied to fobs :
REMARK B1= -4.731 B22= -0.623 B33= 5.354
REMARK B12= 0.000 B13= 0.000 B23= 0.000
REMARK B-factor correction applied to coordinate array B: -0.013
REMARK bulk solvent: density level= 0.380879 e/A^3, B-factor= 43.8838 A^2
REMARK reflections with |Fobs|/sigma_F < 0.0 rejected
REMARK reflections with |Fobs| > 10000 * rms(Fobs) rejected
REMARK theoretical total number of refl. in resol. range: 87004 ( 100.0 %)
REMARK number of unobserved reflections (no entry or |F|=0): 142 ( 0.2 %)
REMARK number of reflections rejected: 0 ( 0.0 % )
REMARK total number of reflections used: 86862 ( 99.8 % )
REMARK number of reflections in working set: 78156 ( 89.8 % )
REMARK number of reflections in test set: 8706 ( 10.0 % )
CRYST1 70.327 127.780 189.274 90.00 90.00 90.00 P 21 21 21
REMARK FILENAME="/draco/usr1/people/sad/xplor/bspul/060312_pulcd/bindividual2"
REMARK DATE:12-Mar-06 02:57:02 created by user: sad
REMARK VERSION:1.1
ATOM 1 CB META 1 115.939 61.329 91.386 1.00 54.74 A
ATOM 2 CG MET A 1 115.017 62.526 91.604 1.00 57.11 A
ATOM 3 SD META 1 114.480 62.687 93.331 1.00 62.36 A
ATOM 4 CE MET A 1 113.026 61.597 93.364 1.0059.84 A
ATOM 5 C MET A 1 117.210 62.339 89.488 1.0050.15 A
ATOM 6 O MET A 1 117.743 63.089 90.312 1.0052.01 A
ATOM 7 N META 1 117.191 59.881 89.799 1.0053.16 A
ATOM 8 CA MET A 1 116.388 61.135 89.934 1.00 52.15 A
ATOM 9 N VALA 2 117.316 62.507 88.175 1.0046.07 A
ATOM 10 CA VALA 2 118.057 63.611 87.587 1.0040.26 A
ATOM 11 CB VALA 2 118.254 63.368 86.089 1.0041.24 A
ATOM 12 CG1 VAL A 2 119.120 62.141 85.881 1.0040.69 A
ATOM 13 CG2 VAL A 2 116.902 63.167 85.418 1.00 38.76 A
ATOM 14 C VALA 2 117.269 64.899 87.781 1.00 37.46 A
ATOM 15 O VAL A 2 116.205 64.890 88.396 1.0036.99 A
ATOM 16 N SERA 3 117.781 66.005 87.256 1.0034.46 A
ATOM 17 CA SERA 3 117.083 67.281 87.386 1.00 31.37 A
ATOM 18 CB SERA 3 117.122 67.752 88.840 1.0032.19 A
ATOM 19 OG SER A 3 118.454 68.011 89.243 1.0031.27 A
ATOM 20 C SER A 3 117.679 68.366 86.497 1.00 29.38 A
ATOM 21 O SERA 3 118.743 68.188 85.906 1.0028.14 A
ATOM 22 N ILEA 4 116.979 69.491 86.404 1.0027.94 A
ATOM 23 CA ILE A 4 117.452 70.612 85.611 1.00 27.40 A
ATOM 24 CB ILEA 4 116.338 71.641 85.369 1.00 26.57 A
ATOM 25 CG2 ILE A 4 116.900 72.844 84.626 1.00 26.14 A
ATOM 26 CG1 ILEA 4 115.193 71.002 84.583 1.0026.77 A
ATOM 27 CD1 ILE A 4 114.028 71.945 84.326 1.0024.52 A
ATOM 28 C ILEA 4 118.569 71.292 86.393 1.0028.09 A
ATOM 29 O ILE A 4 118.421 71.562 87.584 1.00 26.62 A
ATOM 30 N ARGA 5 119.685 71.565 85.728 1.0028.45 A
ATOM 31 CA ARG A 5 120.808 72.218 86.390 1.00 30.40 A
ATOM 32 CB ARG A 5 122.074 72.075 85.548 1.00 32.11 A
ATOM 33 CG ARG A 5 123.358 72.414 86.290 1.00 36.55 A
ATOM 34 CD ARG A 5 124.532 72.471 85.331 1.00 39.10 A
ATOM 35 NE ARG A 5 125.815 72.281 86.000 1.04 43.48 A
ATOM 36 CZ ARG A 5 126.993 72.403 85.395 1.0044.98 A
ATOM 37 NH1 ARG A 5 127.047 72.720 84.108 1.00 44.19 A
ATOM 38 NH2 ARG A 5 128.118 72.197 86.070 1.00 46.35 A
ATOM 39 C ARG A 5 120.496 73.698 86.592 1.00 29.37 A
ATOM 40 O ARG A 5 120.210 74.415 85.632 1.00 28.39 A
ATOM 41 N ARG A 6 120.553 74.149 87.841 1.0029.48 A
ATOM 42 CA ARG A 6 120.272 75.545 88.178 1.00 29.47 A
ATOM 43 CB ARG A 6 118.873 75.661 88.811 1.0028.91 A
ATOM 44 CG ARG A 6 117.734 75.603 87.791 1.00 30.26 A
ATOM 45 CD ARG A 6 116.335 75.799 88.402 1.00 30.62 A
ATOM 46 NE ARG A 6 115.720 74.542 88.820 1.00 30.26 A
ATOM 47 CZ ARG A 6 114.495 74.146 88.477 1.00 27.11 A
ATOM 48 NH1 ARG A 6 113.726 74.902 87.706 1.00 25.84 A
ATOM 49 NH2 ARG A 6 114.040 72.979 88.902 1.00 28.51 A
ATOM 50 C ARG A 6 121.327 76.137 89.116 1.00 29.29 A
ATOM 51 O ARG A 6 121.798 75.464 90.028 1.0029.14 A
ATOM 52 N SERA 7 121.694 77.396 88.877 1.00 29.53 A
ATOM 53 CA SERA 7 122.687 78.103 89.691 1.00 30.61 A
ATOM 54 CB SERA 7 122.845 79.545 89.201 1.00 28.86 A
ATOM 55 OG SERA 7 123.294 79.582 87.863 1.0027.61 A
ATOM 56 C SERA 7 122.281 78.127 91.160 1.00 31.29 A
ATOM 57 O SERA 7 123.083 77.832 92.049 1.0031.69 A
ATOM 58 N PHE A 8 121.030 78.513 91.396 1.00 31.53 A
ATOM 59 CA PHEA 8 120.458 78.582 92.734 1.0031.67 A
ATOM 60 CB PHEA 8 120.849 79.902 93.419 1.0028.57 A
ATOM 61 CG PHEA 8 120.331 81.130 92.729 1.0029.33 A
ATOM 62 CD1 PHE A 8 119.065 81.628 93.022 1.00 28.22 A
ATOM 63 CD2 PHE A 8 121.108 81.788 91.780 1.00 30.44 A
ATOM 64 CE1 PHE A 8 118.577 82.762 92.381 1.0028.93 A
ATOM 65 CE2 PHE A 8 120.629 82.928 91.129 1.0030.65 A
ATOM 66 CZ PHEA 8 119.360 83.415 91.431 1.00 30.94 A
ATOM 67 C PHE A 8 118.938 78.442 92.601 1.00 31.32 A
ATOM 68 O PHE A 8 118.427 78.272 91.491 1.0031.74 A
ATOM 69 N GLU A 9 118.220 78.509 93.717 1.00 30.64 A
ATOM 70 CA GLU A 9 116.770 78.342 93.691 1.00 31.21 A
ATOM 71 CB GLU A 9 116.378 77.179 94.606 1.0032.64 A
ATOM 72 CG GLU A 9 117.070 75.873 94.271 1.00 34.49 A
ATOM 73 CD GLU A 9 116.903 74.828 95.358 1.00 38.18 A
ATOM 74 OE1 GLU A 9 117.336 75.079 96.503 1.0038.47 A
ATOM 75 OE2 GLU A 9 116.339 73.751 95.067 1.0041.59 A
ATOM 76 C GLU A 9 115.985 79.586 94.096 1.00 30.31 A
ATOM 77 O GLU A 9 116.473 80.423 94.848 1.00 29.92 A
ATOM 78 N ALAA 10 114.761 79.688 93.584 1.0029.73 A
ATOM 79 CA ALAA 10 113.868 80.803 93.890 1.0029.34 A
ATOM 80 CB ALAA 10 114.124 81.962 92.948 1.0026.09 A
ATOM 81 C ALAA 10 112.427 80.322 93.751 1.0030.21 A
ATOM 82 O ALAA 10 112.050 79.765 92.718 1.0030.72 A
ATOM 83 N TYRA 11 111.626 80.536 94.791 1.0029.57 A
ATOM 84 CA TYR A 11 110.231 80.113 94.776 1.00 29.43 A
ATOM 85 CB TYRA 11 110.021 78.932 95.725 1.00 30.44 A
ATOM 86 CG TYRA 11 110.991 77.797 95.533 1.00 33.12 A
ATOM 87 CD1 TYR A 11 112.299 77.891 96.000 1.00 33.76 A
ATOM 88 CE1 TYR A 11 113.198 76.850 95.830 1.00 34.94 A
ATOM 89 CD2 TYR A 11 110.605 76.624 94.884 1.00 33.69 A
ATOM 90 CE2 TYRA 11 111.498 75.576 94.707 1.0034.24 A
ATOM 91 CZ TYRA 11 112.794 75.697 95.184 1.00 34.91 A
ATOM 92 OH TYRA 11 113.690 74.669 95.017 1.0036.40 A
ATOM 93 C TYRA 11 109.258 81.216 95.170 1.00 29.54 A
ATOM 94 O TYRA 11 109.588 82.109 95.955 1.00 28.15 A
ATOM 95 N VALA 12 108.053 81.150 94.613 1.0029.47 A
ATOM 96 CA VAL A 12 107.010 82.113 94.940 1.00 29.46 A
ATOM 97 CB VALA 12 106.078 82.366 93.743 1.0030.10 A
ATOM 98 CG1 VAL A 12 104.992 83.361 94.124 1.0029.98 A
ATOM 99 CG2 VAL A 12 106.876 82.890 92.579 1.0032.24 A
ATOM 100 C VALA 12 106.201 81.477 96.070 1.00 28.46 A
ATOM 101 O VALA 12 105.343 80.631 95.824 1.0025.62 A
ATOM 102 N ASP A 13 106.491 81.866 97.307 1.0029.17 A
ATOM 103 CA ASP A 13 105.784 81.303 98.450 1.00 30.71 A
ATOM 104 CB ASPA 13 106.729 81.161 99.644 1.0032.53 A
ATOM 105 CG ASP A 13 107.718 80.018 99.463 1.0034.63 A
ATOM 106 OD1 ASP A 13 107.279 78.892 99.144 1.00 34.80 A
ATOM 107 OD2 ASP A 13 108.932 80.236 99.641 1.00 37.70 A
ATOM 108 C ASP A 13 104.532 82.078 98.841 1.00 31.18 A
ATOM 109 O ASPA 13 103.755 81.629 99.683 1.00 30.97 A
ATOM 110 N ASP A 14 104.336 83.238 98.225 1.00 30.87 A
ATOM 111 CA ASP A 14 103.150 84.039 98.485 1.0031.50 A
ATOM 112 CB ASP A 14 103.269 84.779 99.821 1.00 32.46 A
ATOM 113 CG ASPA 14 101.909 85.116 100.424 1.00 33.62 A
ATOM 114 OD1 ASP A 14 101.843 85.347 101.649 1.00 36.34 A
ATOM 115 OD2 ASP A 14 100.906 85.154 99.678 1.00 32.18 A
ATOM 116 C ASP A 14 102.980 85.013 97.329 1.00 31.67 A
ATOM 117 O ASPA 14 103.902 85.208 96.543 1.0029.85 A
ATOM 118 N MET A 15 101.799 85.611 97.219 1.00 32.37 A
ATOM 119 CA MET A 15 101.502 86.533 96.133 1.00 33.73 A
ATOM 120 CB META 15 100.164 87.234 96.390 1.00 33.56 A
ATOM 121 CG MET A 15 98.973 86.280 96.448 1.00 34.69 A
ATOM 122 SD MET A 15 98.875 85.188 95.009 1.0034.24 A
ATOM 123 CE MET A 15 97.854 86.164 93.916 1.00 35.15 A
ATOM 124 C META 15 102.581 87.573 95.836 1.00 35.41 A
ATOM 125 O META 15 102.766 87.960 94.679 1.0036.24 A
ATOM 126 N ASN A 16 103.300 88.024 96.859 1.0035.22 A
ATOM 127 CA ASN A 16 104.337 89.022 96.629 1.00 36.25 A
ATOM 128 CB ASN A 16 103.822 90.411 97.020 1.00 39.08 A
ATOM 129 CG ASN A 16 103.778 90.615 98.523 1.0042.46 A
ATOM 130 OD1 ASN A 16 103.350 89.732 99.272 1.00 43.85 A
ATOM 131 ND2 ASN A 16 104.212 91.788 98.972 1.00 44.44 A
ATOM 132 C ASN A 16 105.644 88.736 97.359 1.00 35.90 A
ATOM 133 O ASN A 16 106.409 89.657 97.645 1.00 36.52 A
ATOM 134 N ILE A 17 105.907 87.469 97.668 1.00 34.00 A
ATOM 135 CA ILE A 17 107.147 87.129 98.347 1.00 34.16 A
ATOM 136 CB ILEA 17 106.909 86.810 99.864 1.00 36.34 A
ATOM 137 CG2 ILEA 17 105.691 87.566 100.371 1.00 36.74 A
ATOM 138 CG1 ILE A 17 106.741 85.312 100.097 1.00 36.64 A
ATOM 139 CD1 ILEA 17 108.046 84.618 100.447 1.00 38.52 A
ATOM 140 C ILEA 17 107.860 85.975 97.643 1.00 33.44 A
ATOM 141 O ILEA 17 107.282 84.919 97.386 1.00 33.60 A
ATOM 142 N ILEA 18 109.125 86.207 97.312 1.00 31.77 A
ATOM 143 CA ILEA 18 109.948 85.225 96.626 1.00 29.08 A
ATOM 144 CB ILEA 18 110.532 85.830 95.329 1.00 29.42 A
ATOM 145 CG2 ILE A 18 111.555 84.881 94.710 1.00 28.45 A
ATOM 146 CG1 ILE A 18 109.392 86.136 94.354 1.00 30.22 A
ATOM 147 CD1 ILE A 18 109.813 86.934 93.139 1.00 30.93 A
ATOM 148 C ILE A 18 111.090 84.758 97.523 1.00 28.86 A
ATOM 149 O ILE A 18 111.914 85.563 97.963 1.00 28.83 A
ATOM 150 N THR A 19 111.132 83.457 97.790 1.00 26.54 A
ATOM 151 CA THR A 19 112.175 82.873 98.625 1.0026.82 A
ATOM 152 CB THRA 19 111.622 81.676 99.423 1.0026.28 A
ATOM 153 OG1 THRA 19 110.676 82.152 100.387 1.00 27.58 A
ATOM 154 CG2 THRA 19 112.730 80.931 100.129 1.00 23.40 A
ATOM 155 C THRA 19 113.353 82.421 97.762 1.00 28.70 A
ATOM 156 O THRA 19 113.213 81.546 96.902 1.00 28.92 A
ATOM 157 N VALA 20 114.511 83.035 97.986 1.00 28.43 A
ATOM 158 CA VALA 20 115.711 82.704 97.229 1.0027.99 A
ATOM 159 CB VALA 20 116.455 83.981 96.784 1.0027.52 A
ATOM 160 CG1 VAL A 20 117.790 83.621 96.140 1.00 25.24 A
ATOM 161 CG2 VALA 20 115.596 84.757 95.808 1.00 28.39 A
ATOM 162 C VAL A 20 116.660 81.842 98.046 1.00 28.73 A
ATOM 163 O VAL A 20 117.109 82.239 99.120 1.0029.24 A
ATOM 164 N LEU A 21 116.957 80.654 97.537 1.0029.19 A
ATOM 165 CA LEU A 21 117.869 79.743 98.215 1.0030.56 A
ATOM 166 CB LEU A 21 117.255 78.347 98.324 1.0029.87 A
ATOM 167 CG LEU A 21 115.966 78.237 99.138 1.0032.42 A
ATOM 168 CD1 LEU A 21 115.502 76.777 99.154 1.00 30.46 A
ATOM 169 CD2 LEU A 21 116.204 78.746 100.561 1.00 30.26 A
ATOM 170 C LEU A 21 119.166 79.674 97.426 1.00 31.70 A
ATOM 171 O LEU A 21 119.170 79.363 96.234 1.00 31.30 A
ATOM 172 N ILE A 22 120.270 79.974 98.095 1.00 34.34 A
ATOM 173 CA ILEA 22 121.575 79.958 97.449 1.00 36.70 A
ATOM 174 CB ILEA 22 122.243 81.339 97.552 1.00 36.85 A
ATOM 175 CG2 ILE A 22 123.696 81.262 97.113 1.00 37.70 A
ATOM 176 CG1 ILEA 22 121.463 82.333 96.694 1.00 38.16 A
ATOM 177 CD1 ILE A 22 121.823 83.761 96.946 1.0040.57 A
ATOM 178 C ILEA 22 122.468 78.909 98.082 1.0037.28 A
ATOM 179 O ILEA 22 122.652 78.893 99.298 1.00 38.17 A
ATOM 180 N PRO A 23 123.033 78.010 97.262 1.00 38.36 A
ATOM 181 CD PRO A 23 123.073 78.019 95.790 1.00 38.44 A
ATOM 182 CA PRO A 23 123.906 76.969 97.803 1.00 39.36 A
ATOM 183 CB PRO A 23 124.468 76.299 96.547 1.00 39.32 A
ATOM 184 CG PRO A 23 124.404 77.386 95.515 1.00 39.88 A
ATOM 185 C PROA 23 124.981 77.561 98.709 1.0039.94 A
ATOM 186 O PROA 23 125.581 78.589 98.395 1.0039.14 A
ATOM 187 N ALAA 24 125.201 76.905 99.843 1.0041.93 A
ATOM 188 CA ALAA 24 126.176 77.348 100.831 1.00 43.24 A
ATOM 189 CB ALAA 24 126.438 76.223 101.837 1.00 42.48 A
ATOM 190 C ALAA 24 127.496 77.845 100.245 1.00 44.18 A
ATOM 191 O ALAA 24 127.894 78.982 100.496 1.00 43.85 A
ATOM 192 N GLU A 25 128.166 77.009 99.455 1.0045.52 A
ATOM 193 CA GLU A 25 129.453 77.392 98.879 1.0047.10 A
ATOM 194 CB GLU A 25 130.176 76.160 98.317 1.0049.60 A
ATOM 195 CG GLU A 25 129.652 75.631 96.989 1.0053.94 A
ATOM 196 CD GLU A 25 128.198 75.212 97.047 1.00 57.10 A
ATOM 197 OE1 GLU A 25 127.813 74.507 98.008 1.00 57.48 A
ATOM 198 OE2 GLU A 25 127.442 75.580 96.120 1.0058.98 A
ATOM 199 C GLU A 25 129.391 78.483 97.815 1.00 46.28 A
ATOM 200 O GLU A 25 130.383 78.750 97.139 1.00 46.20 A
ATOM 201 N GLN A 26 128.234 79.123 97.677 1.00 45.65 A
ATOM 202 CA GLN A 26 128.066 80.189 96.693 1.00 44.22 A
ATOM 203 CB GLN A 26 127.069 79.759 95.616 1.00 44.41 A
ATOM 204 CG GLN A 26 127.517 78.551 94.818 1.0043.33 A
ATOM 205 CD GLN A 26 128.664 78.862 93.874 1.0042.34 A
ATOM 206 OE1 GLN A 26 129.344 77.957 93.391 1.00 41.19 A
ATOM 207 NE2 GLN A 26 128.876 80.145 93.597 1.00 39.81 A
ATOM 208 C GLN A 26 127.568 81.454 97.373 1.00 43.92 A
ATOM 209 O GLN A 26 127.354 82.478 96.726 1.0042.91 A
ATOM 210 N LYS A 27 127.397 81.372 98.687 1.00 43.44 A
ATOM 211 CA LYS A 27 126.908 82.490 99.483 1.00 44.97 A
ATOM 212 CB LYSA 27 127.027 82.148 100.970 1.0045.24 A
ATOM 213 CG LYS A 27 126.563 83.248 101.911 1.00 45.64 A
ATOM 214 CD LYS A 27 126.689 82.798 103.359 1.00 46.23 A
ATOM 215 CE LYS A 27 126.283 83.897 104.327 1.00 45.42 A
ATOM 216 NZ LYS A 27 126.359 83.427 105.738 1.00 44.95 A
ATOM 217 C LYS A 27 127.591 83.833 99.212 1.00 45.58 A
ATOM 218 O LYS A 27 126.928 84.871 99.162 1.00 44.73 A
ATOM 219 N GLU A 28 128.907 83.821 99.031 1.00 46.60 A
ATOM 220 CA GLU A 28 129.632 85.065 98.795 1.00 48.13 A
ATOM 221 CB GLU A 28 131.054 84.959 99.348 1.00 49.39 A
ATOM 222 CG GLU A 28 131.121 84.597 100.824 1.00 51.77 A
ATOM 223 CD GLU A 28 130.187 85.438 101.681 1.00 52.63 A
ATOM 224 OE1 GLU A 28 130.208 86.682 101.552 1.00 53.42 A
ATOM 225 OE2 GLU A 28 129.437 84.849 102.489 1.00 52.92 A
ATOM 226 C GLU A 28 129.685 85.483 97.331 1.00 48.59 A
ATOM 227 O GLU A 28 130.199 86.551 97.003 1.00 48.76 A
ATOM 228 N ILE A 29 129.152 84.643 96.452 1.00 49.24 A
ATOM 229 CA ILE A 29 129.143 84.945 95.027 1.00 48.83 A
ATOM 230 CB ILEA 29 129.587 83.717 94.210 1.00 49.24 A
ATOM 231 CG2 ILE A 29 129.491 84.014 92.718 1.00 47.59 A
ATOM 232 CG1 ILE A 29 131.019 83.337 94.609 1.00 48.82 A
ATOM 233 CD1 ILEA 29 131.516 82.038 94.006 1.0049.38 A
ATOM 234 C ILE A 29 127.747 85.387 94.593 1.00 48.94 A
ATOM 235 O ILEA 29 127.531 86.554 94.262 1.00 49.51 A
ATOM 236 N META 30 126.802 84.453 94.597 1.0048.66 A
ATOM 237 CA META 30 125.426 84.759 94.224 1.00 47.60 A
ATOM 238 CB META 30 124.668 83.454 93.974 1.0047.66 A
ATOM 239 CG MET A 30 125.233 82.672 92.794 1.00 48.77 A
ATOM 240 SD META 30 124.559 81.017 92.574 1.0049.76 A
ATOM 241 CE META 30 126.043 80.098 92.197 1.0049.42 A
ATOM 242 C META 30 124.827 85.551 95.385 1.0046.73 A
ATOM 243 O MET A 30 124.281 84.987 96.332 1.0045.70 A
ATOM 244 N THRA 31 124.941 86.871 95.297 1.0046.64 A
ATOM 245 CA THR A 31 124.476 87.761 96.354 1.00 45.85 A
ATOM 246 CB THRA 31 125.673 88.455 97.013 1.00 45.56 A
ATOM 247 OG1 THR A 31 126.330 89.277 96.037 1.00 44.15 A
ATOM 248 CG2 THRA 31 126.658 87.427 97.552 1.00 44.57 A
ATOM 249 C THRA 31 123.522 88.855 95.888 1.00 45.20 A
ATOM 250 O THR A 31 123.359 89.082 94.690 1.0045.13 A
ATOM 251 N PRO A 32 122.879 89.549 96.845 1.00 44.68 A
ATOM 252 CD PRO A 32 122.846 89.220 98.282 1.0044.60 A
ATOM 253 CA PRO A 32 121.939 90.634 96.543 1.00 44.42 A
ATOM 254 CB PROA 32 121.373 90.998 97.916 1.0044.75 A
ATOM 255 CG PROA 32 121.492 89.731 98.692 1.00 44.71 A
ATOM 256 C PRO A 32 122.687 91.809 95.918 1.00 44.28 A
ATOM 257 O PRO A 32 123.914 91.882 95.990 1.00 45.55 A
ATOM 258 N PROA 33 121.956 92.752 95.306 1.0044.46 A
ATOM 259 CD PRO A 33 122.505 94.066 94.938 1.00 44.06 A
ATOM 260 CA PROA 33 120.493 92.756 95.182 1.00 43.69 A
ATOM 261 CB PROA 33 120.181 94.197 94.774 1.0044.18 A
ATOM 262 CG PRO A 33 121.362 94.977 95.283 1.0044.22 A
ATOM 263 C PROA 33 119.993 91.761 94.139 1.0042.68 A
ATOM 264 O PROA 33 120.666 91.510 93.136 1.00 42.14 A
ATOM 265 N PHE A 34 118.815 91.193 94.385 1.0041.60 A
ATOM 266 CA PHE A 34 118.207 90.256 93.449 1.00 40.47 A
ATOM 267 CB PHEA 34 117.544 89.088 94.179 1.00 38.35 A
ATOM 268 CG PHEA 34 118.495 88.242 94.968 1.0035.79 A
ATOM 269 CD1 PHE A 34 118.628 88.420 96.341 1.00 35.56 A
ATOM 270 CD2 PHE A 34 119.254 87.263 94.343 1.00 34.24 A
ATOM 271 CE1 PHE A 34 119.504 87.630 97.081 1.00 34.91 A
ATOM 272 CE2 PHE A 34 120.136 86.467 95.073 1.00 35.66 A
ATOM 273 CZ PHEA 34 120.260 86.652 96.446 1.00 34.69 A
ATOM 274 C PHE A 34 117.141 91.020 92.680 1.00 41.22 A
ATOM 275 O PHEA 34 116.614 92.018 93.173 1.0041.07 A
ATOM 276 N ARGA 35 116.824 90.559 91.476 1.0041.76 A
ATOM 277 CA ARG A 35 115.806 91.219 90.666 1.00 42.95 A
ATOM 278 CB ARG A 35 116.457 92.155 89.643 1.0042.63 A
ATOM 279 CG ARG A 35 117.326 93.242 90.252 1.0043.53 A
ATOM 280 CD ARG A 35 117.880 94.167 89.180 1.00 44.00 A
ATOM 281 NE ARG A 35 116.837 94.987 88.570 1.0045.22 A
ATOM 282 CZ ARG A 35 116.198 95.973 89.194 1.0045.07 A
ATOM 283 NH1 ARG A 35 116.497 96.268 90.454 1.0045.99 A
ATOM 284 NH2ARG A 35 115.264 96.668 88.559 1.0043.06 A
ATOM 285 C ARG A 35 114.950 90.194 89.940 1.00 42.60 A
ATOM 286 O ARG A 35 115.440 89.141 89.534 1.0042.68 A
ATOM 287 N LEU A 36 113.668 90.506 89.788 1.0043.04 A
ATOM 288 CA LEU A 36 112.734 89.625 89.099 1.0044.77 A
ATOM 289 CB LEU A 36 111.347 89.722 89.737 1.00 45.16 A
ATOM 290 CG LEU A 36 110.261 88.804 89.172 1.0044.99 A
ATOM 291 CD1 LEU A 36 110.536 87.368 89.586 1.0044.69 A
ATOM 292 CD2 LEU A 36 108.903 89.250 89.685 1.00 45.48 A
ATOM 293 C LEU A 36 112.666 90.084 87.650 1.00 45.76 A
ATOM 294 O LEU A 36 112.517 91.272 87.385 1.00 46.60 A
ATOM 295 N GLU A 37 112.776 89.150 86.714 1.0047.94 A
ATOM 296 CA GLU A 37 112.740 89.499 85.298 1.00 49.94 A
ATOM 297 CB GLU A 37 114.125 89.292 84.676 1.00 49.25 A
ATOM 298 CG GLU A 37 115.228 90.113 85.322 1.00 50.16 A
ATOM 299 CD GLU A 37 116.616 89.725 84.835 1.0050.85 A
ATOM 300 OE1 GLU A 37 117.598 90.371 85.265 1.00 52.21 A
ATOM 301 OE2 GLU A 37 116.729 88.777 84.028 1.00 49.90 A
ATOM 302 C GLU A 37 111.714 88.695 84.509 1.00 51.61 A
ATOM 303 O GLU A 37 111.407 87.550 84.847 1.00 50.85 A
ATOM 304 N THR A 38 111.190 89.311 83.453 1.0054.61 A
ATOM 305 CA THRA 38 110.214 88.672 82.578 1.00 57.52 A
ATOM 306 CB THRA 38 108.849 88.485 83.309 1.00 58.18 A
ATOM 307 OG1 THRA 38 108.467 87.104 83.251 1.00 58.77 A
ATOM 308 CG2 THR A 38 107.754 89.321 82.673 1.00 58.05 A
ATOM 309 C THR A 38 110.053 89.522 81.314 1.00 59.04 A
ATOM 310 O THRA 38 110.619 90.610 81.231 1.00 58.72 A
ATOM 311 N GLU A 39 109.303 89.009 80.338 1.00 61.78 A
ATOM 312 CA GLU A 39 109.049 89.688 79.061 1.00 63.71 A
ATOM 313 CB GLU A 39 107.542 89.702 78.774 1.00 64.30 A
ATOM 314 CG GLU A 39 106.697 90.407 79.825 1.00 64.97 A
ATOM 315 CD GLU A 39 105.504 89.583 80.264 1.0065.10 A
ATOM 316 OE1 GLU A 39 105.713 88.488 80.827 1.00 65.23 A
ATOM 317 OE2 GLU A 39 104.358 90.027 80.046 1.0065.73 A
ATOM 318 C GLU A 39 109.612 91.108 78.991 1.00 64.83 A
ATOM 319 O GLU A 39 110.629 91.348 78.341 1.00 65.93 A
ATOM 320 N ILE A 40 108.943 92.045 79.652 1.00 65_47 A
ATOM 321 CA ILEA 40 109.387 93.433 79.696 1.00 66.35 A
ATOM 322 CB ILE A 40 108.268 94.401 79.247 1.00 66.17 A
ATOM 323 CG2 ILEA 40 108.328 94.602 77.743 1.00 66.73 A
ATOM 324 CG1 ILE A 40 106.896 93.861 79.667 1.00 65.74 A
ATOM 325 CD1 ILEA 40 106.671 93.804 81.161 1.00 64.36 A
ATOM 326 C ILEA 40 109.758 93.733 81.142 1.00 67.16 A
ATOM 327 O ILEA 40 110.375 94.755 81.452 1.0067.66 A
ATOM 328 N THRA 41 109.380 92.803 82.013 1.00 67.15 A
ATOM 329 CA THRA 41 109.612 92.894 83.447 1.0066.42 A
ATOM 330 CB THR A 41 108.866 91.760 84.183 1.00 66.31 A
ATOM 331 OG1 THR A 41 107.461 91.889 83.948 1.00 67.74 A
ATOM 332 CG2 THRA 41 109.134 91.805 85.678 1.0067.02 A
ATOM 333 C THRA 41 111.074 92.846 83.875 1.0065.60 A
ATOM 334 O THR A 41 111.892 92.139 83.287 1.0065.87 A
ATOM 335 N ASPA 42 111.373 93.609 84.921 1.00 64.52 A
ATOM 336 CA ASP A 42 112.701 93.682 85.518 1.00 62.03 A
ATOM 337 CB ASP A 42 113.734 94.194 84.519 1.0063.58 A
ATOM 338 CG ASP A 42 115.139 94.178 85.089 1.0065.32 A
ATOM 339 OD1 ASP A 42 115.406 94.938 86.043 1.0065.65 A
ATOM 340 OD2 ASP A 42 115.975 93.396 84.589 1.00 67.19 A
ATOM 341 C ASPA 42 112.602 94.635 86.696 1.00 59.23 A
ATOM 342 O ASPA 42 112.914 95.817 86.582 1.00 58.83 A
ATOM 343 N PHEA 43 112.154 94.108 87.828 1.00 56.72 A
ATOM 344 CA PHEA 43 111.984 94.910 89.028 1.00 54.68 A
ATOM 345 CB PHE A 43 110.538 94.805 89.522 1.00 54.97 A
ATOM 346 CG PHE A 43 109.520 95.309 88.539 1.0056.22 A
ATOM 347 CD1 PHE A 43 109.390 94.722 87.286 1.00 57.40 A
ATOM 348 CD2 PHE A 43 108.681 96.367 88.872 1.00 57.59 A
ATOM 349 CE1 PHEA 43 108.438 95.181 86.377 1.00 58.18 A
ATOM 350 CE2 PHEA 43 107.725 96.835 87.971 1.00 57.80 A
ATOM 351 CZ PHEA 43 107.604 96.240 86.721 1.0057.75 A
ATOM 352 C PHE A 43 112.930 94.488 90.145 1.00 52.78 A
ATOM 353 O PHE A 43 113.519 93.405 90.102 1.00 53.25 A
ATOM 354 N PRO A 44 113.093 95.352 91.161 1.00 50.32 A
ATOM 355 CD PRO A 44 112.616 96.747 91.217 1.0049.06 A
ATOM 356 CA PRO A 44 113.970 95.064 92.298 1.0048.46 A
ATOM 357 CB PRO A 44 114.284 96.451 92.837 1.0048.40 A
ATOM 358 CG PRO A 44 112.985 97.161 92.629 1.0048.00 A
ATOM 359 C PRO A 44 113.260 94.199 93.332 1.00 46.64 A
ATOM 360 O PRO A 44 112.032 94.125 93.349 1.00 45.99 A
ATOM 361 N LEU A 45 114.039 93.552 94.191 1.00 44.56 A
ATOM 362 CA LEU A 45 113.486 92.706 95.239 1.0042.86 A
ATOM 363 CB LEU A 45 113.804 91.233 94.966 1.00 41.22 A
ATOM 364 CG LEU A 45 113.167 90.589 93.733 1.0040.07 A
ATOM 365 CD1 LEU A 45 113.623 89.142 93.626 1.0038.96 A
ATOM 366 CD2 LEU A 45 111.655 90.666 93.836 1.00 39.02 A
ATOM 367 C LEU A 45 114.067 93.109 96.586 1.00 42.81 A
ATOM 368 O LEU A 45 115.284 93.113 96.771 1.00 42.89 A
ATOM 369 N ALA A 46 113.193 93.448 97.525 1.00 42.14 A
ATOM 370 CA ALA A 46 113.623 93.854 98.855 1.00 40.81 A
ATOM 371 CB ALA A 46 112.614 94.828 99.453 1.00 39.84 A
ATOM 372 C ALA A 46 113.774 92.636 99.754 1.00 40.61 A
ATOM 373 O ALA A 46 112.908 91.762 99.778 1.0040.86 A
ATOM 374 N VALA 47 114.880 92.581 100.487 1.0040.37 A
ATOM 375 CA VALA 47 115.147 91.474 101.394 1.00 40.30 A
ATOM 376 CB VAL A 47 116.665 91.239 101.551 1.00 40.27 A
ATOM 377 CG1 VAL A 47 116.927 90.137 102.578 1.00 39.03 A
ATOM 378 CG2 VAL A 47 117.273 90.871 100.205 1.00 39.84 A
ATOM 379 C VALA 47 114.544 91.753 102.766 1.00 41.25 A
ATOM 380 O VAL A 47 115.034 92.601 103.508 1.00 42.46 A
ATOM 381 N ARG A 48 113.471 91.042 103.095 1.00 41.56 A
ATOM 382 CA ARG A 48 112.810 91.211 104.382 1.00 43.02 A
ATOM 383 CB ARG A 48 111.405 90.613 104.336 1.00 43.66 A
ATOM 384 CG ARG A 48 110.755 90.464 105.705 1.00 44.35 A
ATOM 385 CD ARG A 48 109.463 89.684 105.599 1.00 44.37 A
ATOM 386 NE ARG A 48 108.483 90.389 104.781 1.00 44.46 A
ATOM 387 CZ ARG A 48 107.371 1 89.836 104.310 1.00 44.86 A
ATOM 388 NH1 ARG A 48 107.100 88.562 104.572 1.00 45.62 A
ATOM 389 NH2 ARG A 48 106.525 90.562 103.592 1.00 43.96 A
ATOM 390 C ARG A 48 113.619 90.531 105.482 1.00 43.40 A
ATOM 391 O ARG A 48 113.658 90.998 106.619 1.00 44.15 A
ATOM 392 N GLU A 49 114.250 89.416 105.141 1.00 43.34 A
ATOM 393 CA GLU A 49 115.066 88.690 106.098 1.00 43.61 A
ATOM 394 CB GLU A 49 114.190 88.033 107.167 1.00 45.48 A
ATOM 395 CG GLU A 49 113.265 86.947 106.662 1.0047.79 A
ATOM 396 CD GLU A 49 112.373 86.395 107.763 1.00 51.49 A
ATOM 397 OE1 GLU A 49 112.907 85.988 108.823 1.00 50.16 A
ATOM 398 OE2 GLU A 49 111.137 86.368 107.566 1.00 52.91 A
ATOM 399 C GLU A 49 115.897 87.639 105.382 1.00 43.59 A
ATOM 400 O GLU A 49 115.591 87.251 104.252 1.00 42.77 A
ATOM 401 N GLU A 50 116.953 87.187 106.049 1.00 42.31 A
ATOM 402 CA GLU A 50 117.856 86.194 105.491 1.00 41.53 A
ATOM 403 CB GLU A 50 118.950 86.891 104.677 1.00 41.50 A
ATOM 404 CG GLU A 50 119.837 87.825 105.497 1.00 40.46 A
ATOM 405 CD GLU A 50 120.856 88.569 104.651 1.0039.23 A
ATOM 406 OEI GLU A 50 120.485 89.550 103.970 1.00 38.19 A
ATOM 407 OE2 GLU A 50 122.035 88.164 104.663 1.00 40.01 A
ATOM 408 C GLU A 50 118.485 85.382 106.615 1.00 41.78 A
ATOM 409 O GLU A 50 118.803 85.918 107.674 1.00 40.43 A
ATOM 410 N TYR A 51 118.658 84.087 106.383 1.00 43.05 A
ATOM 411 CA TYRA 51 119.252 83.209107.380 1.00 44.63 A
ATOM 412 CB TYRA 51 118.259 82.944 108.518 1.00 42.81 A
ATOM 413 CG TYRA 51 116.910 82.403 108.095 1.00 42.40 A
ATOM 414 CD1 TYR A 51 116.731 81.046 107.831 1.00 43.28 A
ATOM 415 CE1 TYR A 51 115.476 80.539 107.476 1.00 44.10 A
ATOM 416 CD2 TYR A 51 115.803 83.247 107.989 1.00 42.06 A
ATOM 417 CE2 TYR A 51 114.548 82.754 107.637 1.0043.06 A
ATOM 418 CZ TYR A 51 114.391 81.400 107.384 1.00 43.81 A
ATOM 419 OH TYRA 51 113.148 80.903 107.056 1.0045.73 A
ATOM 420 C TYRA 51 119.730 81.900 106.763 1.00 46.83 A
ATOM 421 O TYRA 51 119.276 81.499 105.690 1.0045.89 A
ATOM 422 N SERA 52 120.649 81.238 107.456 1.00 48.97 A
ATOM 423 CA SERA 52 121.235 79.997 106.978 1.00 51.68 A
ATOM 424 CB SERA 52 122.640 79.844107.565 1.00 52.76 A
ATOM 425 OG SERA 52 123.214 78.600 107.209 1.00 5.13 A
ATOM 426 C SERA 52 120.439 78.725 107.246 1.00 53.34 A
ATOM 427 O SERA 52 119.793 78.578 108.285 1.00 54.02 A
ATOM 428 N LEU A 53 120.503 77.810 106.284 1.0054.71 A
ATOM 429 CA LEU A 53 119.840 76.514 106.367 1.00 55.74 A
ATOM 430 CB LEU A 53 118.836 76.340 105.226 1.00 55.23 A
ATOM 431 CG LEU A 53 117.533 77.140 105.311 1.00 55.28 A
ATOM 432 CD1 LEU A 53 117.833 78.626 105.356 1.00 55.55 A
ATOM 433 CD2 LEU A 53 116.657 76.808 104.113 1.00 54.71 A
ATOM 434 C LEU A 53 120.934 75.463 106.252 1.00 56.68 A
ATOM 435 O LEU A 53 122.078 75.793 105.946 1.00 57.60 A
ATOM 436 N GLU A 54 120.587 74.203 106.489 1.00 57.49 A
ATOM 437 CA GLU A 54 121.562 73.120 106.426 1.00 58.37 A
ATOM 438 CB GLU A 54 120.868 71.795 106.108 1.0061.35 A
ATOM 439 CG GLU A 54 121.817 70.602 106.089 1.00 64.78 A
ATOM 440 CD GLU A 54 122.579 70.439 107.397 1.0067.45 A
ATOM 441 OE1 GLU A 54 121.929 70.214 108.443 1.0068.66 A
ATOM 442 OE2 GLU A 54 123.827 70.538 107.382 1.0067.89 A
ATOM 443 C GLU A 54 122.693 73.348 105.423 1.00 57.65 A
ATOM 444 O GLU A 54 123.855 73.484 105.809 1.0058.27 A
ATOM 445 N ALAA 55 122.358 73.395 104.139 1.00 55.87 A
ATOM 446 CA ALAA 55 123.376 73.586 103.116 1.00 54.00 A
ATOM 447 CB ALAA 55 123.544 72.301 102.310 1.00 53.78 A
ATOM 448 C ALAA 55 123.077 74.750 102.182 1.00 52.30 A
ATOM 449 O ALAA 55 123.599 74.807 101.068 1.00 52.77 A
ATOM 450 N LYS A 56 122.247 75.682 102.631 1.00 49.35 A
ATOM 451 CA LYS A 56 121.903 76.825 101.799 1.00 47.24 A
ATOM 452 CB LYS A 56 120.617 76.541 101.018 1.00 46.55 A
ATOM 453 CG LYS A 56 120.648 75.277 100.178 1.00 45.96 A
ATOM 454 CD LYS A 56 119.303 75.034 99.522 1.0045.42 A
ATOM 455 CE LYS A 56 119.255 73.679 98.846 1.0045.31 A
ATOM 456 NZ LYS A 56 117.898 73.384 98.315 1.00 45.32 A
ATOM 457 C LYS A 56 121.702 78.083 102.625 1.00 45.65 A
ATOM 458 O LYS A 56 121.478 78.015 103.832 1.00 46.18 A
ATOM 459 N TYRA 57 121.803 79.233 101.970 1.00 43.01 A
ATOM 460 CA TYR A 57 121.575 80.500 102.642 1.00 42.42 A
ATOM 461 CB TYR A 57 122.722 81.483 102.413 1.00 42.23 A
ATOM 462 CG TYR A 57 122.617 82.705 103.297 1.00 42.14 A
ATOM 463 CD1 TYR A 57 122.763 82.603 104.684 1.00 42.50 A
ATOM 464 CE1 TYR A 57 122.627 83.723 105.508 1.0041.46 A
ATOM 465 CD2 TYR A 57 122.334 83.958 102.756 1.0042.83 A
ATOM 466 CE2 TYR A 57 122.196 85.082 103.569 1.00 42.35 A
ATOM 467 CZ TYR A 57 122.342 84.958 104.941 1.00 41.84 A
ATOM 468 OH TYR A 57 122.198 86.070 105.737 1.00 39.76 A
ATOM 469 C TYR A 57 120.301 81.025 102.007 1.00 41.54 A
ATOM 470 O TYRA 57 120.207 81.134 100.785 1.00 42.53 A
ATOM 471 N LYS A 58 119.322 81.344 102.844 1.00 40.57 A
ATOM 472 CA LYS A 58 118.023 81.804 102.377 1.00 38.34 A
ATOM 473 CB LYSA 58 116.944 81.028 103.136 1.0037.04 A
ATOM 474 CG LYSA 58 115.510 81.487 102.921 1.00 38.62 A
ATOM 475 CD LYSA 58 114.560 80.611 103.740 1.00 37.22 A
ATOM 476 CE LYSA 58 113.134 81.121 103.692 1.00 38.41 A
ATOM 477 NZ LYS A 58 112.219 80.271 104.501 1.0037.40 A
ATOM 478 C LYS A 58 117.760 83.300 102.488 1.00 38.84 A
ATOM 479 O LYS A 58 118.208 83.957 103.429 1.00 39.56 A
ATOM 480 N TYR A 59 117.033 83.826 101.505 1.00 38.28 A
ATOM 481 CA TYR A 59 116.638 85.230 101.472 1.00 38.39 A
ATOM 482 CB TYR A 59 117.288 85.994 100.313 1.0038.31 A
ATOM 483 CG TYR A 59 118.789 86.130 100.366 1.0039.75 A
ATOM 484 CD1 TYR A 59 119.614 85.138 99.849 1.00 39.55 A
ATOM 485 CD1 TYR A 59 120.996 85.279 99.853 1.00 41.96 A
ATOM 486 CD2 TYR A 59 119.386 87.274 100.901 1.00 40.29 A
ATOM 487 CE2 TYR A 59 120.769 87.427 100.914 1.00 41.27 A
ATOM 488 CZ TYRA 59 121.570 86.425 100.385 1.00 43.03 A
ATOM 489 OH TYRA 59 122.942 86.567 100.369 1.0043.16 A
ATOM 490 C TYRA 59 115.128 85.279 101.257 1.00 38.88 A
ATOM 491 O TYR A 59 114.614 84.680 100.308 1.00 38.60 A
ATOM 492 N VAL A 60 114.420 85.974 102.141 1.00 37.61 A
ATOM 493 CA VAL A 60 112.979 86.119 102.004 1.00 35.06 A
ATOM 494 CB VALA 60 112.261 86.061 103.373 1.00 33.72 A
ATOM 495 CG1 VAL A 60 110.752 86.224 103.184 1.00 30.67 A
ATOM 496 CG2 VAL A 60 112.562 84.736 104.059 1.00 30.00 A
ATOM 497 C VALA 60 112.801 87.494 101.377 1.0036.04 A
ATOM 498 O VALA 60 112.894 88.513 102.058 1.00 36.33 A
ATOM 499 N CYS A 61 112.566 87.514 100.068 1.00 36.80 A
ATOM 500 CA CYS A 61 112.416 88.766 99.338 1.00 37.47 A
ATOM 501 CB CYSA 61 113.119 88.649 97.988 1.0037.28 A
ATOM 502 SG CYSA 61 114.823 88.071 98.131 1.0040.32 A
ATOM 503 C CYS A 61 110.975 89.216 99.134 1.0038.27 A
ATOM 504 O CYSA 61 110.039 88.422 99.212 1.00 38.68 A
ATOM 505 N VALA 62 110.811 90.506 98.864 1.00 38.89 A
ATOM 506 CA VALA 62 109.498 91.088 98.650 1.00 39.43 A
ATOM 507 CB VALA 62 109.145 92.064 99.795 1.00 38.16 A
ATOM 508 CG1 VAL A 62 107.742 92.612 99.605 1.00 36.50 A
ATOM 509 CG2 VAL A 62 109.260 91.354 101.132 1.00 38.06 A
ATOM 510 C VALA 62 109.464 91.841 97.324 1.00 41.49 A
ATOM 511 O VAL A 62 110.407 92.553 96.981 1.00 42.14 A
ATOM 512 N SERA 63 108.380 91.673 96.573 1.00 43.70 A
ATOM 513 CA SERA 63 108.228 92.361 95.297 1.00 46.67 A
ATOM 514 CB SERA 63 107.868 91.374 94.181 1.00 46.64 A
ATOM 515 OG SERA 63 106.559 90.857 94.354 1.00 50.52 A
ATOM 516 C SERA 63 107.120 93.392 95.444 1.00 47.72 A
ATOM 517 O SERA 63 106.181 93.198 96.215 1.00 48.71 A
ATOM 518 N ASP A 64 107.235 94.492 94.711 1.00 49.10 A
ATOM 519 CA ASP A 64 106.240 95.554 94.772 1.00 50.27 A
ATOM 520 CB ASP A 64 106.798 96.822 94.127 1.00 51.61 A
ATOM 521 CG ASP A 64 107.965 97.398 94.901 1.00 52.12 A
ATOM 522 OD1 ASP A 64 108.645 98.303 94.372 1.00 53.00 A
ATOM 523 OD2 ASP A 64 108.195 96.947 96.044 1.00 52.26 A
ATOM 524 C ASPA 64 104.965 95.129 94.066 1.0050.49 A
ATOM 525 O ASP A 64 103.863 95.294 94.588 1.00 50.52 A
ATOM 526 N ALAA 65 105.123 94.578 92.871 1.00 51.19 A
ATOM 527 CA ALA A 65 103.984 94.122 92.094 1.00 50.75 A
ATOM 528 CB ALAA 65 104.223 94.401 90.615 1.00 51.84 A
ATOM 529 C ALAA 65 103.770 92.628 92.323 1.0050.15 A
ATOM 530 O ALAA 65 104.726 91.849 92.337 1.0049.39 A
ATOM 531 N PROA 66 102.511 92.212 92.532 1.0049.22 A
ATOM 532 CD PRO A 66 101.304 93.030 92.753 1.00 48.82 A
ATOM 533 CA PRO A 66 102.225 90.791 92.752 1.00 47.29 A
ATOM 534 CB PRO A 66 100.710 90.770 92.924 1.00 48.04 A
ATOM 535 CG PRO A 66 100.434 92.100 93.569 1.00 48.20 A
ATOM 536 C PRO A 66 102.684 89.987 91.538 1.00 45.53 A
ATOM 537 O PRO A 66 102.544 90.439 90.401 1.00 45.24 A
ATOM 538 N VALA 67 103.241 88.806 91.781 1.0043.11 A
ATOM 539 CA VALA 67 103.723 87.956 90.700 1.0040.06 A
ATOM 540 CB VAL A 67 104.346 86.659 91.259 1.00 40.00 A
ATOM 541 CG1 VALA 67 104.911 85.819 90.126 1.00 40.59 A
ATOM 542 CG2 VAL A 67 105.433 86.999 92.258 1.00 39.79 A
ATOM 543 C VALA 67 102.594 87.588 89.742 1.00 38.83 A
ATOM 544 O VALA 67 101.466 87.335 90.163 1.0039.12 A
ATOM 545 N THR A 68 102.899 87.570 88.450 1.00 36.61 A
ATOM 546 CA THR A 68 101.911 87.222 87.438 1.00 35.06 A
ATOM 547 CB THRA 68 101.936 88.210 86.267 1.0035.97 A
ATOM 548 OG1 THR A 68 103.274 88.315 85.763 1.00 36.22 A
ATOM 549 CG2 THR A 68 101.438 89.578 86.721 1.00 36.19 A
ATOM 550 C THRA 68 102.201 85.826 86.908 1.0033.60 A
ATOM 551 O THRA 68 103.230 85.590 86.276 1.00 35.05 A
ATOM 552 N PHEA 69 101.276 84.909 87.159 1.0030.89 A
ATOM 553 CA PHEA 69 101.432 83.524 86.751 1.00 28.63 A
ATOM 554 CB PHEA 69 100.558 82.642 87.640 1.0027.08 A
ATOM 555 CG PHE A 69 100.882 82.759 89.103 1.00 25.99 A
ATOM 556 CD1 PHE A 69 100.402 83.828 89.852 1.00 23.80 A
ATOM 557 CD2 PHE A 69 101.693 81.812 89.727 1.00 24.43 A
ATOM 558 CE1 PHE A 69 100.723 83.954 91.200 1.00 24.23 A
ATOM 559 CE2 PHE A 69 102.022 81.929 91.076 1.00 24.23 A
ATOM 560 CZ PHEA 69 101.536 83.001 91.813 1.0023.93 A
ATOM 561 C PHEA 69 101.153 83.222 85.284 1.00 28.19 A
ATOM 562 O PHEA 69 100.403 83.931 84.612 1.0028.28 A
ATOM 563 N GLY A 70 101.773 82.155 84.794 1.00 27.66 A
ATOM 564 CA GLY A 70 101.582 81.763 83.411 1.00 29.14 A
ATOM 565 C GLY A 70 102.768 82.114 82.539 1.0028.84 A
ATOM 566 O GLY A 70 102.896 81.621 81.418 1.0029.13 A
ATOM 567 N LYS A 71 103.638 82.973 83.057 1.00 29.40 A
ATOM 568 CA LYS A 71 104.824 83.399 82.328 1.00 31.04 A
ATOM 569 CB LYS A 71 104.834 84.924 82.172 1.00 33.80 A
ATOM 570 CG LYS A 71 103.724 85.475 81.290 1.0036.24 A
ATOM 571 CD LYS A 71 103.939 85.101 79.833 1.00 37.57 A
ATOM 572 CE LYS A 71 102.787 85.584 78.964 1.00 39.11 A
ATOM 573 NZ LYS A 71 102.599 87.057 79.063 1.00 40.41 A
ATOM 574 C LYS A 71 106.070 82.962 83.078 1.0031.42 A
ATOM 575 O LYS A 71 106.098 82.976 84.308 1.0031.35 A
ATOM 576 N ILE A 72 107.097 82.560 82.336 1.00 32.04 A
ATOM 577 CA ILEA 72 108.342 82.134 82.952 1.00 33.26 A
ATOM 578 CB ILEA 72 109.247 81.410 81.931 1.00 33.42 A
ATOM 579 CG2 ILE A 72 110.638 81.197 82.515 1.00 32.27 A
ATOM 580 CG1 ILE A 72 108.615 80.067 81.553 1.00 34.17 A
ATOM 581 CD1 ILE A 72 109.388 79.280 80.520 1.0035.59 A
ATOM 582 C ILEA 72 109.067 83.355 83.513 1.00 34.96 A
ATOM 583 O ILEA 72 109.299 84.331 82.805 1.00 35.13 A
ATOM 584 N HIS A 73 109.407 83.299 84.794 1.0036.96 A
ATOM 585 CA HIS A 73 110.105 84.400 85.447 1.0038.95 A
ATOM 586 CB HIS A 73 109.397 84.802 86.747 1.0041.62 A
ATOM 587 CG HIS A 73 108.086 85.493 86.543 1.0043.82 A
ATOM 588 CD2 HIS A 73 106.891 85.336 87.160 1.00 43.83 A
ATOM 589 ND1 HIS A 73 107.921 86.528 85.648 1.00 45.26 A
ATOM 590 CE1 HIS A 73 106.683 86.980 85.723 1.00 45.00 A
ATOM 591 NE2 HIS A 73 106.037 86.275 86.634 1.0044.45 A
ATOM 592 C HIS A 73 111.539 84.017 85.781 1.00 38.32 A
ATOM 593 O HIS A 73 111.847 82.850 86.014 1.00 38.29 A
ATOM 594 N CYS A 74 112.412 85.015 85.805 1.00 38.26 A
ATOM 595 CA CYS A 74 113.809 84.793 86.138 1.00 38.92 A
ATOM 596 CB CYS A 74 114.707 85.056 84.919 1.00 38.99 A
ATOM 597 SG CYS A 74 114.560 83.861 83.554 1.00 39.93 A
ATOM 598 C CYS A 74 114.214 85.729 87.277 1.0039.23 A
ATOM 599 O CYS A 74 113.844 86.904 87.289 1.0037.39 A
ATOM 600 N VALA 75 114.944 85.195 88.251 1.00 39.61 A
ATOM 601 CA VALA 75 115.439 86.008 89.353 1.0040.12 A
ATOM 602 CB VALA 75 115.101 85.403 90.738 1.00 39.52 A
ATOM 603 CG1 VAL A 75 113.597 85.383 90.941 1.00 39.02 A
ATOM 604 CG2 VAL A 75 115.659 84.012 90.848 1.00 42.29 A
ATOM 605 C VAL A 75 116.952 86.078 89.152 1.0040.60 A
ATOM 606 O VALA 75 117.632 85.057 89.013 1.0039.51 A
ATOM 607 N ARG A 76 117.466 87.298 89.114 1.0042.29 A
ATOM 608 CA ARG A 76 118.881 87.529 88.884 1.0043.64 A
ATOM 609 CB ARG A 76 119.036 88.554 87.759 1.0045.09 A
ATOM 610 CG ARG A 76 120.458 88.980 87.492 1.00 50.21 A
ATOM 611 CD ARG A 76 120.514 89.989 86.361 1.00 53.73 A
ATOM 612 NE ARG A 76 121.857 90.534 86.196 1.00 56.98 A
ATOM 613 CZ ARG A 76 122.495 91.236 87.126 1.00 58.18 A
ATOM 614 NH1 ARG A 76 121.911 91.481 88.293 1.00 59.11 A
ATOM 615 NH2 ARG A 76 123.718 91.693 86.890 1.00 58.95 A
ATOM 616 C ARG A 76 119.625 88.004 90.124 1.00 42.66 A
ATOM 617 O ARGA 76 119.151 88.879 90.849 1.00 43.59 A
ATOM 618 N ALA A 77 120.794 87.416 90.360 1.00 41.50 A
ATOM 619 CA ALAA 77 121.628 87.784 91.497 1.0040.78 A
ATOM 620 CB ALAA 77 122.390 86.566 92.004 1.00 39.04 A
ATOM 621 C ALAA 77 122.607 88.868 91.047 1.0040.92 A
ATOM 622 O ALAA 77 122.844 89.038 89.849 1.0039.87 A
ATOM 623 N SER A 78 123.169 89.602 92.004 1.0040.85 A
ATOM 624 CA SERA 78 124.121 90.658 91.680 1.00 41.40 A
ATOM 625 CB SERA 78 124.651 91.320 92.962 1.0040.60 A
ATOM 626 OG SERA 78 125.422 90.421 93.747 1.00 40.76 A
ATOM 627 C SER A 78 125.285 90.083 90.876 1.00 41.49 A
ATOM 628 O SERA 78 125.875 90.769 90.045 1.00 41.60 A
ATOM 629 N SERA 79 125.600 88.814 91.129 1.00 42.28 A
ATOM 630 CA SERA 79 126.689 88.126 90.444 1.00 42.83 A
ATOM 631 CB SERA 79 126.979 86.791 91.129 1.00 43.59 A
ATOM 632 OG SER A 79 125.863 85.923 91.036 1.00 44.81 A
ATOM 633 C SERA 79 126.381 87.874 88.973 1.00 42.83 A
ATOM 634 O SERA 79 127.264 87.502 88.201 1.0043.50 A
ATOM 635 N GLY A 80 125.127 88.069 88.586 1.0042.75 A
ATOM 636 CA GLY A 80 124.757 87.852 87.201 1.0042.53 A
ATOM 637 C GLY A 80 124.089 86.512 86.959 1.0042.15 A
ATOM 638 O GLYA 80 123.495 86.296 85.904 1.0042.50 A
ATOM 639 N HIS A 81 124.186 85.603 87.922 1.00 41.85 A
ATOM 640 CA HIS A 81 123.558 84.299 87.767 1.0041.97 A
ATOM 641 CB HIS A 81 123.966 83.357 88.902 1.0043.31 A
ATOM 642 CG HIS A 81 125.420 83.004 88.902 1.0046.14 A
ATOM 643 CD2 HIS A 81 126.058 81.857 88.567 1.0047.85 A
ATOM 644 ND1 HIS A 81 126.404 83.895 89.271 1.00 46.87 A
ATOM 645 CE1 HIS A 81 127.586 83.314 89.164 1.0047.04 A
ATOM 646 NE2 HIS A 81 127.404 82.077 88.739 1.0049.03 A
ATOM 647 C HIS A 81 122.042 84.463 87.756 1.00 40.59 A
ATOM 648 O HIS A 81 121.510 85.471 88.223 1.00 40.52 A
ATOM 649 N LYS A 82 121.350 83.471 87.211 1.00 38.88 A
ATOM 650 CA LYS A 82 119.897 83.507 87.143 1.00 37.83 A
ATOM 651 CB LYS A 82 119.432 84.026 85.779 1.00 37.66 A
ATOM 652 CG LYSA 82 119.845 85.447 85.483 1.0039.64 A
ATOM 653 CD LYSA 82 119.299 85.904 84.143 1.0041.73 A
ATOM 654 CE LYSA 82 119.764 87.316 83.821 1.0042.18 A
ATOM 655 NZ LYS A 82 119.147 87.828 82.566 1.0043.56 A
ATOM 656 C LYS A 82 119.322 82.123 87.360 1.00 35.64 A
ATOM 657 O LYS A 82 120.042 81.125 87.339 1.0034.49 A
ATOM 658 N THRA 83 118.013 82.080 87.572 1.00 34.25 A
ATOM 659 CA THRA 83 117.295 80.833 87.771 1.00 33.00 A
ATOM 660 CB THRA 83 117.462 80.299 89.217 1.0033.28 A
ATOM 661 OG1 THR A 83 116.980 78.951 89.288 1.00 34.63 A
ATOM 662 CG2 THR A 83 116.686 81.143 90.198 1.00 32.68 A
ATOM 663 C THRA 83 115.827 81.127 87.477 1.00 32.51 A
ATOM 664 O THRA 83 115.385 82.278 87.571 1.00 31.89 A
ATOM 665 N ASP A 84 115.082 80.098 87.090 1.0030.76 A
ATOM 666 CA ASP A 84 113.671 80.268 86.777 1.00 30.18 A
ATOM 667 CB ASPA 84 113.174 79.121 85.900 1.0028.81 A
ATOM 668 CG ASP A 84 113.373 77.764 86.548 1.0029.34 A
ATOM 669 OD1 ASP A 84 112.413 76.965 86.577 1.00 30.95 A
ATOM 670 OD2 ASP A 84 114.492 77.490 87.025 1.0028.42 A
ATOM 671 C ASP A 84 112.858 80.303 88.061 1.0029.78 A
ATOM 672 O ASP A 84 113.080 79.496 88.966 1.0029.68 A
ATOM 673 N LEU A 85 111.917 81.235 88.134 1.00 28.78 A
ATOM 674 CA LEU A 85 111.070 81.357 89.311 1.00 29.93 A
ATOM 675 CB LEU A 85 110.235 82.631 89.239 1.00 30.53 A
ATOM 676 CG LEU A 85 110.085 83.444 90.525 1.00 33.79 A
ATOM 677 CD1 LEU A 85 108.802 84.260 90.414 1.00 33.62 A
ATOM 678 CD2 LEU A 85 110.049 82.539 91.751 1.00 29.98 A
ATOM 679 C LEU A 85 110.133 80.161 89.366 1.00 29.42 A
ATOM 680 O LEU A 85 109.252 80.020 88.519 1.00 29.56 A
ATOM 681 N GLN A 86 110.332 79.302 90.360 1.0029.16 A
ATOM 682 CA GLN A 86 109.504 78.118 90.536 1.00 29.33 A
ATOM 683 CB GLN A 86 110.346 76.973 91.103 1.00 28.89 A
ATOM 684 CG GLN A 86 111.298 76.342 90.103 1.00 28.39 A
ATOM 685 CD GLN A 86 112.231 75.343 90.753 1.00 28.31 A
ATOM 686 OE1 GLN A 86 113.396 75.644 91.021 1.00 26.61 A

ATOM 687 NE2 GLN A 86 111.718 74.149 91.027 1.00 29.36 A
ATOM 688 C GLN A 86 108.312 78.390 91.459 1.00 31.17 A
ATOM 689 O GLN A 86 108.221 79.451 92.080 1.00 31.38 A
ATOM 690 N ILE A 87 107.411 77.415 91.540 1.00 31.26 A
ATOM 691 CA ILEA 87 106.211 77.503 92.364 1.00 31.49 A
ATOM 692 CB ILE A 87 105.130 76.505 91.874 1.00 32.58 A
ATOM 693 CG2 ILE A 87 103.783 76.838 92.514 1.00 31.64 A
ATOM 694 CG1 ILEA 87 105.007 76.572 90.345 1.0031.86 A
ATOM 695 CD1 ILE A 87 104.224 75.427 89.735 1.00 27.73 A
ATOM 696 C ILEA 87 106.516 77.190 93.828 1.00 31.66 A
ATOM 697 O ILEA 87 107.175 76.197 94.140 1.00 33.04 A
ATOM 698 N GLY A 88 106.035 78.046 94.722 1.0029.98 A
ATOM 699 CA GLY A 88 106.254 77.831 96.139 1.00 28.97 A
ATOM 700 C GLY A 88 104.960 77.427 96.821 1.00 28.26 A
ATOM 701 O GLY A 88 104.138 76.724 96.233 1.00 28.80 A
ATOM 702 N ALAA 89 104.768 77.874 98.057 1.0026.21 A
ATOM 703 CA ALAA 89 103.557 77.542 98.797 1.0024.87 A
ATOM 704 CB ALA A 89 103.840 77.570 100.297 1.00 24.93 A
ATOM 705 C ALAA 89 102.436 78.516 98.456 1.00 23.75 A
ATOM 706 O ALAA 89 101.327 78.404 98.975 1.0023.96 A
ATOM 707 N VALA 90 102.727 79.465 97.572 1.0023.58 A
ATOM 708 CA VAL A 90 101.753 80.478 97.172 1.0022.81 A
ATOM 709 CB VAL A 90 102.305 81.332 96.009 1.00 22.73 A
ATOM 710 CG1 VAL A 90 102.423 80.473 94.735 1.00 19.42 A
ATOM 711 CG2 VAL A 90 101.409 82.544 95.780 1.00 20.49 A
ATOM 712 C VALA 90 100.411 79.874 96.750 1.00 23.83 A
ATOM 713 O VALA 90 99.358 80.480 96.931 1.00 24.29 A
ATOM 714 N ILE A 91 100.468 78.674 96.186 1.00 23.92 A
ATOM 715 CA ILEA 91 99.294 77.946 95.706 1.00 24.54 A
ATOM 716 CB ILEA 91 99.718 76.599 95.132 1.00 24.87 A
ATOM 717 CG2 ILE A 91 100.423 76.797 93.794 1.00 24.01 A
ATOM 718 CG1 ILE A 91 100.614 75.907 96.168 1.00 27.14 A
ATOM 719 CD1 ILE A 91 101.029 74.520 95.835 1.00 30.80 A
ATOM 720 C ILE A 91 98.238 77.660 96.766 1.00 23.37 A
ATOM 721 O ILEA 91 97.054 77.564 96.458 1.00 22.93 A
ATOM 722 N ARG A 92 98.674 77.505 98.009 1.00 24.62 A
ATOM 723 CA ARG A 92 97.767 77.196 99.107 1.00 26.53 A
ATOM 724 CB ARG A 92 98.463 76.254 100.097 1.00 27.20 A
ATOM 725 CG ARG A 92 98.753 74.867 99.533 1.00 29.06 A
ATOM 726 CD ARG A 92 99.233 73.911 100.618 1.00 31.93 A
ATOM 727 NE ARG A 92 100.628 74.133 100.992 1.00 33.57 A
ATOM 728 CZ ARG A 92 101.669 73.794 100.237 1.00 33.46 A
ATOM 729 NH1 ARG A 92 102.904 74.033 100.655 1.00 34.45 A
ATOM 730 NH2 ARG A 92 101.475 73.211 99.064 1.00 31.91 A
ATOM 731 C ARG A 92 97.218 78.409 99.857 1.00 27.58 A
ATOM 732 O ARG A 92 96.557 78.251 100.886 1.00 29.59 A
ATOM 733 N THRA 93 97.480 79.611 99.346 1.00 27.10 A
ATOM 734 CA THRA 93 97.010 80.837 99.995 1.00 25.81 A
ATOM 735 CB THRA 93 97.927 82.026 99.691 1.00 24.50 A
ATOM 736 OG1 THR A 93 97.868 82.316 98.290 1.0023.82 A
ATOM 737 CG2 THRA 93 99.359 81.720 100.097 1.00 22.99 A
ATOM 738 C THRA 93 95.610 81.234 99.550 1.00 26.21 A
ATOM 739 O THRA 93 95.196 80.947 98.425 1.0027.36 A
ATOM 740 N ALAA 94 94.894 81.918 100.437 1.00 24.60 A
ATOM 741 CA ALA A 94 93.539 82.371 100.150 1.0022.54 A
ATOM 742 CB ALAA 94 92.934 83.027 101.392 1.00 22.77 A
ATOM 743 C ALAA 94 93.554 83.359 98.996 1.0021.67 A
ATOM 744 O ALAA 94 92.635 83.392 98.179 1.0021.39 A
ATOM 745 N ALAA 95 94.608 84.167 98.933 1.0020.79 A
ATOM 746 CA ALAA 95 94.732 85.165 97.878 1.00 20.19 A
ATOM 747 CB ALA A 95 95.940 86.047 98.143 1.00 18.24 A
ATOM 748 C ALA A 95 94.856 84.497 96.511 1.00 20.85 A
ATOM 749 O ALA A 95 94.257 84.942 95.528 1.0021.10 A
ATOM 750 N PHEA 96 95.645 83.430 96.450 1.0020.91 A
ATOM 751 CA PHE A 96 95.836 82.708 95.206 1.00 19.94 A
ATOM 752 CB PHEA 96 96.889 81.615 95.397 1.0022.42 A
ATOM 753 CG PHEA 96 97.234 80.879 94.133 1.00 22.55 A
ATOM 754 CD1 PHE A 96 98.168 81.398 93.244 1.00 22.69 A
ATOM 755 CD2 PHEA 96 96.609 79.676 93.821 1.00 22.13 A
ATOM 756 CE1 PHE A 96 98.476 80.725 92.058 1.0024.90 A
ATOM 757 CE2 PHE A 96 96.91 78.997 92.636 1.0022.04 A
ATOM 758 CZ PHEA 96 97.843 79.522 91.757 1.0022.51 A
ATOM 759 C PHE A 96 94.504 82.078 94.783 1.0020.04 A
ATOM 760 O PHEA 96 94.120 82.131 93.615 1.0017.39 A
ATOM 761 N ASP A 97 93.801 81.491 95.748 1.0020.36 A
ATOM 762 CA ASP A 97 92.518 80.847 95.484 1.00 21.31 A
ATOM 763 CB ASP A 97 91.968 80.222 96.773 1.00 21.73 A
ATOM 764 CG ASP A 97 91.578 78.757 96.604 1.00 21.70 A
ATOM 765 OD1 ASP A 97 91.557 78.252 95.456 1.00 21.72 A
ATOM 766 OD2 ASP A 97 91.282 78.109 97.628 1.00 21.86 A
ATOM 767 C ASP A 97 91.491 81.829 94.923 1.00 21.94 A
ATOM 768 O ASPA 97 90.819 81.538 93.937 1.0021.62 A
ATOM 769 N ASP A 98 91.367 82.994 95.551 1.00 24.51 A
ATOM 770 CA ASPA 98 90.397 83.988 95.100 1.0026.34 A
ATOM 771 CB ASPA 98 90.237 85.084 96.160 1.00 30.39 A
ATOM 772 CG ASP A 98 89.466 84.601 97.387 1.00 33.78 A
ATOM 773 OD1 ASP A 98 89.370 85.357 98.379 1.00 33.77 A
ATOM 774 OD2 ASP A 98 88.950 83.460 97.354 1.00 35.82 A
ATOM 775 C ASP A 98 90.756 84.595 93.751 1.00 26.15 A
ATOM 776 O ASP A 98 89.881 85.031 93.005 1.00 26.97 A
ATOM 777 N GLU A 99 92.043 84.613 93.430 1.00 26.31 A
ATOM 778 CA GLU A 99 92.496 85.162 92.156 1.00 27.29 A
ATOM 779 CB GLU A 99 93.996 85.482 92.225 1.00 29.58 A
ATOM 780 CG GLU A 99 94.675 85.738 90.869 1.0036.43 A
ATOM 781 CD GLU A 99 94.216 87.023 90.185 1.00 40.64 A
ATOM 782 OE1 GLU A 99 94.732 87.326 89.086 1.0043.48 A
ATOM 783 OE2 GLU A 99 93.345 87.731 90.737 1.00 43.74 A
ATOM 784 C GLU A 99 92.233 84.210 90.985 1.00 26.16 A
ATOM 785 O GLU A 99 91.858 84.643 89.899 1.00 25.50 A
ATOM 786 N PHE A 100 92.408 82.912 91.209 1.00 24.43 A
ATOM 787 CA PHE A 100 92.230 81.954 90.128 1.00 24.07 A
ATOM 788 CB PHE A 100 93.501 81.117 89.984 1.00 23.41 A
ATOM 789 CG PHE A 100 94.708 81.920 89.604 1.0021.35 A
ATOM 790 CD1 PHE A 100 95.657 82.274 90.559 1.00 21.43 A
ATOM 791 CD2 PHE A 100 94.894 82.331 88.286 1.00 20.27 A
ATOM 792 CE1 PHE A 100 96.782 83.030 90.201 1.00 22.94 A
ATOM 793 CE2 PHE A 100 96.010 83.086 87.916 1.00 18.21 A
ATOM 794 CZ PHE A 100 96.956 83.435 88.873 1.0021.17 A
ATOM 795 C PHE A 100 91.022 81.026 90.161 1.00 22.79 A
ATOM 796 O PHE A 100 90.871 80.188 89.274 1.0021.16 A
ATOM 797 N TYRA 101 90.160 81.159 91.161 1.00 22.86 A
ATOM 798 CA TYR A 101 88.991 80.290 91.213 1.00 23.01 A
ATOM 799 CB TYR A 101 88.068 80.660 92.372 1.00 21.38 A
ATOM 800 CG TYR A 101 86.768 79.897 92.306 1.0023.85 A
ATOM 801 CD1 TYR A 101 86.725 78.535 92.598 1.0023.47 A
ATOM 802 CE1 TYR A 101 85.552 77.800 92.450 1.00 23.95 A
ATOM 803 CD2 TYR A 101 85.594 80.515 91.866 1.00 23.68 A
ATOM 804 CE2 TYR A 101 84.413 79.789 91.713 1.00 25.67 A
ATOM 805 CZ TYR A 101 84.404 78.428 92.007 1.00 25.81 A
ATOM 806 OH TYR A 101 83.255 77.692 91.844 1.00 27.38 A
ATOM 807 C THR A 101 88.204 80.401 89.911 1.00 22.67 A
ATOM 808 O TYR A 101 87.875 81.503 89.475 1.0022.63 A
ATOM 809 N TYR A 102 87.905 79.258 89.300 1.0023.02 A
ATOM 810 CA TYR A 102 87.154 79.214 88.046 1.00 21.50 A
ATOM 811 CB TYR A 102 87.971 78.498 86.967 1.0020.67 A
ATOM 812 CG TYR A 102 87.249 78.312 85.651 1.00 20.49 A
ATOM 813 CD1 TYR A 102 86.855 79.410 84.881 1.00 20.44 A
ATOM 814 CE1 TYR A 102 86.215 79.238 83.649 1.0020.13 A
ATOM 815 CD2 TYR A 102 86.982 77.035 85.161 1.00 21.83 A
ATOM 816 CE2 TYR A 102 86.344 76.851 83.933 1.0022.48 A
ATOM 817 CZ TYR A 102 85.967 77.956 83.184 1.00 21.43 A
ATOM 818 OH TYR A 102 85.369 77.766 81.964 1.00 24.58 A
ATOM 819 C TYR A 102 85.834 78.484 88.268 1.00 22.33 A
ATOM 820 O TYR A 102 85.809 77.345 88.740 1.00 23.05 A
ATOM 821 N ASP A 103 84.738 79.137 87.901 1.0023.29 A
ATOM 822 CA ASP A 103 83.411 78.571 88.092 1.00 23.76 A
ATOM 823 CB ASP A 103 82.467 79.665 88.590 1.00 28.08 A
ATOM 824 CG ASP A 163 81.229 79.105 89.260 1.00 32.52 A
ATOM 825 OD1 ASP A 103 81.377 78.312 90.216 1.00 35.06 A
ATOM 826 OD2 ASP A 103 80.109 79.462 88.837 1.00 37.22 A
ATOM 827 C ASP A 103 82.793 77.885 86.876 1.00 21.88 A
ATOM 828 O ASP A 103 81.664 77.407 86.956 1.00 20.91 A
ATOM 829 N GLY A 104 83.524 77.823 85.763 1.00 19.92 A
ATOM 830 CA GLY A 104 82.996 77.191 84.564 1.00 19.07 A
ATOM 831 C GLY A 104 83.211 75.688 84.543 1.00 21.38 A
ATOM 832 O GLY A 104 83.587 75.099 85.561 1.00 20.21 A
ATOM 833 N GLU A 105 82.963 75.048 83.402 1.00 20.58 A
ATOM 834 CA GLU A 105 83.173 73.608 83.332 1.00 20.82 A
ATOM 835 CB GLU A 105 82.115 72.907 82.463 1.00 21.89 A
ATOM 836 CG GLU A 105 81.974 73.382 81.039 1.00 28.49 A
ATOM 837 CD GLU A 105 81.038 72.481 80.226 1.00 31.55 A
ATOM 838 OE1 GLU A 105 80.447 72.967 79.238 1.00 31.48 A
ATOM 839 OE2 GLU A 105 80.903 71.282 80.569 1.00 31.47 A
ATOM 840 C GLU A 105 84.569 73.267 82.839 1.00 18.34 A
ATOM 841 O GLU A 105 85.156 73.970 82.009 1.00 18.85 A
ATOM 842 N LEU A 106 85.099 72.182 83.381 1.00 16.11 A
ATOM 843 CA LEU A 106 86.430 71.721 83.036 1.00 16.23 A
ATOM 844 CB LEU A 106 87.298 71.691 84.299 1.00 16.52 A
ATOM 845 CG LEU A 106 87.510 73.065 84.953 1.00 17.21 A
ATOM 846 CD1 LEU A 106 88.077 72.907 86.362 1.00 17.28 A
ATOM 847 CD2 LEU A 106 88.437 73.895 84.078 1.00 16.89 A
ATOM 848 C LEU A 106 86.329 70.336 82.428 1.00 16.05 A
ATOM 849 O LEU A 106 85.318 69.652 82.597 1.00 15.88 A
ATOM 850 N GLY A 107 87.377 69.930 81.717 1.00 18.28 A
ATOM 851 CA GLY A 107 87.394 68.617 81.094 1.00 18.36 A
ATOM 852 C GLY A 107 87.218 68.686 79.590 1.00 19.52 A
ATOM 853 O GLY A 107 87.538 69.698 78.962 1.0019.16 A
ATOM 854 N ALA A 108 86.719 67.602 79.009 1.00 19.11 A
ATOM 855 CA ALA A 108 86.482 67.544 77.575 1.00 20.28 A
ATOM 856 CB ALA A 108 86.866 66.160 77.036 1.00 16.64 A
ATOM 857 C ALA A 108 85.003 67.837 77.297 1.00 20.68 A
ATOM 858 O ALA A 108 84.122 67.058 77.671 1.00 20.44 A
ATOM 859 N VAL A 109 84.736 68.973 76.659 1.00 21.31 A
ATOM 860 CA VAL A 109 83.370 69.366 76.332 1.00 22.71 A
ATOM 861 CB VAL A 109 83.174 70.881 76.514 1.00 25.86 A
ATOM 862 CG1 VAL A 109 81.702 71.253 76.334 1.00 26.32 A
ATOM 863 CG2 VAL A 109 83.667 71.300 77.888 1.00 24.42 A
ATOM 864 C VAL A 109 83.157 68.980 74.875 1.00 23.92 A
ATOM 865 O VAL A 109 83.609 69.672 73.966 1.00 23.61 A
ATOM 866 N TYR A 110 82.462 67.865 74.673 1.00 23.34 A
ATOM 867 CA THR A 110 82.213 67.312 73.353 1.00 24.37 A
ATOM 868 CB TYR A 110 82.058 65.797 73.468 1.0022.58 A
ATOM 869 CG THR A 110 81.721 65.105 72.166 1.0021.36 A
ATOM 870 CD1 TYR A 110 82.728 64.683 71.297 1.00 19.47 A
ATOM 871 CE1 TYR A 110 82.423 64.034 70.102 1.0020.02 A
ATOM 872 CD2 TYR A 110 80.393 64.866 71.805 1.00 20.26 A
ATOM 873 CE2 TYR A 110 80.076 64.220 70.610 1.00 20.71 A
ATOM 874 CZ TYR A 110 81.095 63.805 69.768 1.0020.62 A
ATOM 875 OH TYR A 110 80.789 63.147 68.607 1.00 21.02 A
ATOM 876 C TYR A 110 81.031 67.841 72.555 1.0027.13 A
ATOM 877 O TYR A 110 79.997 68.211 73.102 1.00 29.62 A
ATOM 878 N THR A 111 81.220 67.833 71.240 1.00 28.21 A
ATOM 879 CA THRA 111 80.234 68.228 70.244 1.00 28.32 A
ATOM 880 CB THR A 111 80.358 69.723 69.841 1.0030.25 A
ATOM 881 OG1 THR A 111 79.679 70.549 70.798 1.0031.34 A
ATOM 882 CG2 THR A 111 79.765 69.953 68.474 1.0030.59 A
ATOM 883 C THR A 111 80.653 67.363 69.061 1.00 28.37 A
ATOM 884 O THR A 111 81.843 67.102 68.882 1.00 26.37 A
ATOM 885 N ALA A 112 79.697 66.898 68.268 1.00 28.43 A
ATOM 886 CA ALA A 112 80.043 66.071 67.114 1.00 29.90 A
ATOM 887 CB ALA A 112 78.777 65.633 66.384 1.00 30.09 A
ATOM 888 C ALA A 112 80.949 66.855 66.164 1.00 29.75 A
ATOM 889 O ALA A 112 81.795 66.289 65.465 1.00 27.74 A
ATOM 890 N ASP A 113 80.767 68.168 66.164 1.00 30.43 A
ATOM 891 CA ASP A 113 81.524 69.061 65.299 1.00 31.92 A
ATOM 892 CB ASP A 113 80.740 70.374 65.138 1.00 36.28 A
ATOM 893 CG ASP A 113 81.331 71.287 64.083 1.00 40.61 A
ATOM 894 OD1 ASPA 113 80.773 72.384 63.873 1.00 45.45 A
ATOM 895 OD2 ASP A 113 82.346 70.916 63.460 1.00 44.57 A
ATOM 896 C ASPA 113 82.926 69.355 65.825 1.00 29.65 A
ATOM 897 O ASP A 113 83.886 69.441 65.056 1.0028.88 A
ATOM 898 N HIS A 114 83.041 69.498 67.141 1.00 29.06 A
ATOM 899 CA HIS A 114 84.318 69.818 67.761 1.00 25.28 A
ATOM 900 CB HIS A 114 84.637 71.296 67.527 1.00 23.96 A
ATOM 901 CG HIS A 114 83.647 72.227 68.161 1.0024.37 A
ATOM 902 CD2 HIS A 114 83.577 72.734 69.416 1.00 23.30 A
ATOM 903 ND1 HIS A 114 82.526 72.686 67.502 1.00 23.88 A
ATOM 904 CE1 HIS A 114 81.810 73.435 68.322 1.0023.12 A
ATOM 905 NE2 HIS A 114 82.425 73.479 69.490 1.00 22.81 A
ATOM 906 C HIS A 114 84.291 69.553 69.267 1.0023.66 A
ATOM 907 O HIS A 114 83.227 69.409 69.867 1.00 22.88 A
ATOM 908 N THR A 115 85.473 69.518 69.872 1.00 22.34 A
ATOM 909 CA THR A 115 85.611 69.286 71.308 1.00 21.14 A
ATOM 910 CB THR A 115 86.259 67.910 71.603 1.00 18.57 A
ATOM 911 OG1 THR A 115 85.357 66.866 71.223 1.00 22.15 A
ATOM 912 CG2 THR A 115 86.575 67.769 73.080 1.00 21.35 A
ATOM 913 C THR A 115 86.485 70.368 71.929 1.00 20.53 A
ATOM 914 O THR A 115 87.553 70.690 71.41.0 1.00 22.15 A
ATOM 915 N VAL A 116 86.022 70.940 73.031 1.00 19.58 A
ATOM 916 CA VAL A 116 86.793 71.959 73.722 1.00 19.22 A
ATOM 917 CB VAL A 116 85.892 73.115 74.229 1.00 20.08 A
ATOM 918 CG1 VAL A 116 86.752 74.186 74.895 1.00 16.14 A
ATOM 919 CG2 VAL A 116 85.096 73.720 73.064 1.00 18.56 A
ATOM 920 C VAL A 116 87.449 71.281 74.922 1.00 20.72 A
ATOM 921 O VAL A 116 86.775 70.597 75.692 1.00 21.13 A
ATOM 922 N PHE A 117 88.762 71.446 75.062 1.00 18.74 A
ATOM 923 CA PHE A 117 89.485 70.863 76.183 1.00 17.87 A
ATOM 924 CB PHE A 117 90.755 70.145 75.715 1.00 15.85 A
ATOM 925 CG PHE A 117 90.495 68.918 74.897 1.0014.71 A
ATOM 926 CD1 PHE A 117 90.468 68.982 73.508 1.00 15.14 A
ATOM 927 CD2 PHE A 117 90.274 67.694 75.517 1.00 13.73 A
ATOM 928 CE1 PHE A 117 90.226 67.837 72.745 1.00 17.41 A
ATOM 929 CE2 PHE A 117 90.030 66.544 74.768 1.00 15.85 A
ATOM 930 CZ PHE A 117 90.006 66.611 73.379 1.00 3.38 A
ATOM 931 C PHE A 117 89.865 71.967 77.152 1.00 17.73 A
ATOM 932 O PHE A 117 90.449 72.970 76.756 1.00 18.46 A
ATOM 933 N LYS A 118 89.528 71.783 78.422 1.0018.02 A
ATOM 934 CA LYS A 118 89.846 72.776 79.438 1.00 18.32 A
ATOM 935 CB LYS A 118 88.599 73.599 79.794 1.0019.50 A
ATOM 936 CG LYS A 118 88.218 74.639 78.746 1.0020.86 A
ATOM 937 CD LYS A 118 87.057 75.513 79.213 1.00 21.23 A
ATOM 938 CE LYS A 118 85.743 74.762 79.150 1.00 23.53 A
ATOM 939 NZ LYS A 118 84.613 75.561 79.700 1.00 24.59 A
ATOM 940 C LYS A 118 90.418 72.156 80.702 1.00 17.97 A
ATOM 941 O LYS A 118 89.902 71.155 81.211 1.00 17.57 A
ATOM 942 N VALA 119 91.496 72.754 81.198 1.00 16.68 A
ATOM 943 CA VAL A 119 92.134 72.291 82.418 1.00 15.97 A
ATOM 944 CB VALA 119 93.327 71.351 82.124 1.00 17.38 A
ATOM 945 CG1 VAL A 119 94.332 72.042 81.216 1.00 16.22 A
ATOM 946 CG2 VAL A 119 93.982 70.925 83.441 1.00 13.98 A
ATOM 947 C VAL A 119 92.616 73.487 83.233 1.00 16.83 A
ATOM 948 O VAL A 119 93.126 74.469 82.689 1.00 17.25 A
ATOM 949 N TRP A 120 92.437 73.396 84.543 1.00 16.58 A
ATOM 950 CA TRP A 120 92.828 74.453 85.461 1.00 16.37 A
ATOM 951 CB TRP A 120 91.882 74.431 86.666 1.00 16.09 A
ATOM 952 CG TRP A 120 91.984 75.607 87.582 1.00 17.02 A
ATOM 953 CD2 TRP A 120 92.508 75.612 88.919 1.00 15.56 A
ATOM 954 CE2 TRP A 120 92.401 76.934 89.403 1.00 15.42 A
ATOM 955 CE3 TRP A 120 93.058 74.628 89.752 1.00 17.34 A
ATOM 956 CD1 TRP A 120 91.593 76.887 87.319 1.00 15.09 A
ATOM 957 NE1 TRP A 120 91.840 77.690 88.407 1.00 16.25 A
ATOM 958 CZ2 TRP A 120 92.825 77.302 90.686 1.00 16.00 A
ATOM 959 CZ3 TRP A 120 93.483 74.991 91.034 1.00 17.14 A
ATOM 960 CH2 TRP A 120 93.362 76.320 91.485 1.00 19.44 A
ATOM 961 C TRP A 120 94.276 74.224 85.902 1.00 16.84 A
ATOM 962 O TRP A 120 94.572 73.271 86.625 1.00 19.45 A
ATOM 963 N ALA A 121 95.178 75.092 85.454 1.00 17.70 A
ATOM 964 CA ALA A 121 96.596 74.984 85.801 1.00 18.79 A
ATOM 965 CB ALA A 121 97.347 74.198 84.719 1.00 18.20 A
ATOM 966 C ALA A 121 97.185 76.384 85.944 1.00 19.20 A
ATOM 967 O ALA A 121 98.060 76.784 85.177 1.00 19.22 A
ATOM 968 N PRO A 122 96.721 77.139 86.953 1.00 18.36 A
ATOM 969 CD PRO A 122 95.805 76.653 88.001 1.00 18.82 A
ATOM 970 CA PRO A 122 97.160 78.507 87.239 1.00 18.73 A
ATOM 971 CB PRO A 122 96.333 78.879 88.476 1.00 19.96 A
ATOM 972 CG PRO A 122 96.141 77.551 89.167 1.0019.63 A
ATOM 973 C PRO A 122 98.659 78.731 87.451 1.00 19.67 A
ATOM 974 O PRO A 122 99.199 79.761 87.037 1.00 19.95 A
ATOM 975 N ALA A 123 99.333 77.777 88.088 1.00 20.14 A
ATOM 976 CA ALAA 123 100.762 77.920 88.367 1.00 19.76 A
ATOM 977 CB ALAA 123 101.130 77.140 89.636 1.00 19.74 A
ATOM 978 C ALAA 123 101.647 77.476 87.214 1.00 19.78 A
ATOM 979 O ALAA 123 102.853 77.703 87.230 1.00 19.92 A
ATOM 980 N ALA A 124 101.045 76.842 86.215 1.00 20.36 A
ATOM 981 CA ALA A 124 101.788 76.369 85.058 1.00 19.51 A
ATOM 982 CB ALAA 124 100.965 75.320 84.311 1.00 18.32 A
ATOM 983 C ALA A 124 102.141 77.529 84.126 1.00 18.99 A
ATOM 984 O ALA A 124 101.615 78.641 84.273 1.00 18.41 A
ATOM 985 N THR A 125 103.041 77.266 83.179 1.00 16.29 A
ATOM 986 CA THR A 125 103.457 78.280 82.205 1.00 17.53 A
ATOM 987 CB THR A 125 104.984 78.539 82.236 1.00 16.64 A
ATOM 988 OG1 THR A 125 105.687 77.298 82.084 1.00 16.36 A
ATOM 989 CG2 THR A 125 105.388 79.213 83.548 1.00 15.04 A
ATOM 990 C THR A 125 103.095 77.832 80.796 1.00 18.80 A
ATOM 991 O THR A 125 103.259 78.579 79.828 1.00 17.99 A
ATOM 992 N SERA 126 102.597 76.606 80.680 1.00 17.39 A
ATOM 993 CA SERA 126 102.227 76.094 79.375 1.00 18.81 A
ATOM 994 CB SERA 126 103.482 75.903 78.522 1.00 18.31 A
ATOM 995 OG SERA 126 103.153 75.897 77.153 1.00 23.44 A
ATOM 996 C SERA 126 101.479 74.778 79.526 1.00 17.88 A
ATOM 997 O SERA 126 101.631 74.075 80.522 1.00 17.38 A
ATOM 998 N ALA A 127 100.659 74.459 78.534 1.00 16.91 A
ATOM 999 CA ALA A 127 99.881 73.238 78.568 1.00 18.10 A
ATOM 1000 CB ALA A 127 98.606 73.450 79.376 1.00 16.60 A
ATOM 1001 C ALA A 127 99.531 72.819 77.156 1.00 17.36 A
ATOM 1002 O ALA A 127 99.497 73.642 76.244 1.00 19.18 A
ATOM 1003 N ALAA 128 99.269 71.529 76.990 1.00 17.64 A
ATOM 1004 CA ALA A 128 98.909 70.977 75.695 1.00 18.62 A
ATOM 1005 CB ALA A 128 100.171 70.603 74.910 1.00 16.86 A
ATOM 1006 C ALA A 128 98.046 69.746 75.900 1.00 18.40 A
ATOM 1007 O ALA A 128 98.068 69.125 76.971 1.00 17.84 A
ATOM 1008 N VALA 129 97.275 69.402 74.876 1.0018.22 A
ATOM 1009 CA VAL A 129 96.446 68.217 74.949 1.00 18.22 A
ATOM 1010 CB VALA 129 94.985 68.525 74.566 1.00 17.92 A
ATOM 101 CG1 VAL A 129 94.896 68.986 73.112 1.00 14.81 A
ATOM 1012 CG2 VAL A 129 94.133 67.297 74.804 1.00 16.09 A
ATOM 1013 C VALA 129 97.044 67.186 73.995 1.00 18.98 A
ATOM 1014 O VAL A 129 97.393 67.510 72.858 1.00 20.74 A
ATOM 1015 N LYS A 130 97.191 65.952 74.469 1.00 19.33 A
ATOM 1016 CA LYS A 130 97.758 64.892 73.647 1.00 18.97 A
ATOM 1017 CB LYS A 130 98.889 64.168 74.383 1.0019.55 A
ATOM 1018 CG LYS A 130 99.489 63.046 73.546 1.00 23.33 A
ATOM 1019 CD LYS A 130 100.528 62.222 74.290 1.00 28.71 A
ATOM 1020 CE LYS A 130 101.813 62.984 74.524 1.00 30.15 A
ATOM 1021 NZ LYS A 130 102.913 62.033 74.864 1.00 32.58 A
ATOM 1022 C LYS A 130 96.686 63.889 73.246 1.00 18.97 A
ATOM 1023 O LYS A 130 96.145 63.170 74.090 1.00 16.29 A
ATOM 1024 N LEU A 131 96.403 63.848 71.946 1.00 16.61 A
ATOM 1025 CA LEU A 131 95.394 62.967 71.376 1.00 18.79 A
ATOM 1026 CB LEU A 131 94.559 63.742 70.351 1.00 18.64 A
ATOM 1027 CG LEU A 131 93.899 65.029 70.860 1.00 19.28 A
ATOM 1028 CD1 LEU A 131 93.420 65.860 69.685 1.00 19.96 A
ATOM 1029 CD2 LEU A 131 92.745 64.689 71.786 1.00 18.89 A
ATOM 1030 C LEU A 131 95.988 61.730 70.701 1.00 20.14 A
ATOM 1031 O LEU A 131 97.007 61.804 70.013 1.0020.44 A
ATOM 1032 N SERA 132 95.334 60.593 70.902 1.00 21.37 A
ATOM 1033 CA SERA 132 95.764 59.334 70.309 1.00 23.20 A
ATOM 1034 CB SERA 132 96.756 58.614 71.230 1.00 19.52 A
ATOM 1035 OG SERA 132 96.169 58.321 72.484 1.0020.63 A
ATOM 1036 C SERA 132 94.523 58.474 70.073 1.0026.72 A
ATOM 1037 O SERA 132 93.487 58.665 70.716 1.0025.53 A
ATOM 1038 N HIS A 133 94.627 57.525 69.151 1.00 31.78 A
ATOM 1039 CA HIS A 133 93.494 56.672 68.821 1.00 36.51 A
ATOM 1040 CB HIS A 133 92.646 57.358 67.745 1.00 37.84 A
ATOM 1041 CG HIS A 133 91.388 56.625 67.399 1.0039.99 A
ATOM 1042 CD2 HIS A 133 91.183 55.494 66.682 1.00 39.89 A
ATOM 1043 ND1 HIS A 133 90.143 57.045 67.817 1.00 41.47 A
ATOM 1044 CE1 HIS A 133 89.225 56.205 67.373 1.00 40.63 A
ATOM 1045 NE2 HIS A 133 89.830 55.254 66.682 1.00 40.31 A
ATOM 1046 C HIS A 133 93.987 55.331 68.303 1.00 39.87 A
ATOM 1047 O HIS A 133 94.980 55.271 67.577 1.0039.94 A
ATOM 1048 N PRO A 134 93.302 54.234 68.671 1.00 43.06 A
ATOM 1049 CD PRO A 134 92.112 54.135 69.538 1.0043.46 A
ATOM 1050 CA PRO A 134 93.718 52.909 68.204 1.00 45.50 A
ATOM 1051 CB PRO A 134 92.533 52.029 68.589 1.00 45.85 A
ATOM 1052 CG PRO A 134 92.073 52.656 69.871 1.00 44.19 A
ATOM 1053 C PRO A 134 93.981 52.925 66.697 1.00 47.40 A
ATOM 1054 O PRO A 134 93.085 53.218 65.902 1.00 46.32 A
ATOM 1055 N ASN A 135 95.220 52.622 66.317 1.00 51.08 A
ATOM 1056 CA ASN A 135 95.623 52.612 64.914 1.00 53.22 A
ATOM 1057 CB ASN A 135 95.226 51.286 64.257 1.00 57.00 A
ATOM 1058 CG ASN A 135 96.163 50.150 64.632 1.00 59.42 A
ATOM 1059 OD1 ASN A 135 95.961 49.001 64.231 1.00 61.06 A
ATOM 1060 ND2 ASN A 135 97.201 50.470 65.400 1.00 60.56 A
ATOM 1061 C ASN A 135 95.050 53.789 64.123 1.00 52.96 A
ATOM 1062 O ASN A 135 94.231 53.617 63.217 1.00 52.72 A
ATOM 1063 N LYS A 136 95.489 54.987 64.493 1.00 51.96 A
ATOM 1064 CA LYS A 136 95.070 56.220 63.841 1.00 50.04 A
ATOM 1065 CB LYS A 136 93.713 56.691 64.376 1.00 49.40 A
ATOM 1066 CG LYS A 136 93.163 57.912 63.651 1.00 48.33 A
ATOM 1067 CD LYS A 136 91.751 58.261 64.096 1.00 48.64 A
ATOM 1068 CE LYS A 136 90.741 57.241 63.609 1.00 49.32 A
ATOM 1069 NZ LYS A 136 89.348 57.618 63.992 1.00 50.45 A
ATOM 1070 C LYS A 136 96.150 57.263 64.119 1.00 48.83 A
ATOM 1071 O LYS A 136 97.133 56.975 64.805 1.0049.80 A
ATOM 1072 N SERA 137 95.970 58.469 63.593 1.00 45.58 A
ATOM 1073 CA SERA 137 96.956 59.529 63.761 1.0042.81 A
ATOM 1074 CB SERA 137 96.808 60.545 62.624 1.0044.92 A
ATOM 1075 OG SERA 137 96.710 59.891 61.368 1.00 48.12 A
ATOM 1076 C SERA 137 96.883 60.257 65.105 1.00 39.41 A
ATOM 1077 O SERA 137 95.832 60.781 65.484 1.00 38.19 A
ATOM 1078 N GLY A 138 98.009 60.284 65.817 1.0035.20 A
ATOM 1079 CA GLY A 138 98.071 60.968 67.096 1.00 31.79 A
ATOM 1080 C GLY A 138 98.521 62.401 66.874 1.00 29.59 A
ATOM 1081 O GLY A 138 99.320 62.665 65.977 1.0030.48 A
ATOM 1082 N ARG A 139 98.014 63.331 67.677 1.0025.51 A
ATOM 1083 CA ARG A 139 98.372 64.739 67.534 1.00 22.57 A
ATOM 1084 CB ARG A 139 97.342 65.462 66.656 1.0020.88 A
ATOM 1085 CG ARG A 139 97.043 64.803 65.307 1.0025.04 A
ATOM 1086 CD ARG A 139 98.114 65.096 64.256 1.0022.87 A
ATOM 1087 NE ARG A 139 98.167 66.512 63.910 1.00 22.59 A
ATOM 1088 CZ ARG A 139 97.204 67.174 63.273 1.00 22.73 A
ATOM 1089 NH1 ARG A 139 96.095 66.551 62.895 1.00 21.60 A
ATOM 1090 NH2 ARG A 139 97.343 68.469 63.028 1.00 21.12 A
ATOM 1091 C ARG A 139 98.412 65.415 68.901 1.00 22.14 A
ATOM 1092 O ARG A 139 97.624 65.085 69.787 1.00 20.24 A
ATOM 1093 N THR A 140 99.332 66.360 69.074 1.00 21.17 A
ATOM 1094 CA THR A 140 99.432 67.092 70.336 1.00 20.49 A
ATOM 1095 CB THR A 140 100.733 66.761 71.094 1.0020.60 A
ATOM 1096 OG1 THR A 140 100.726 65.377 71.459 1.00 21.45 A
ATOM 1097 CG2 THR A 140 100.846 67.605 72.363 1.00 18.59 A
ATOM 1098 C THR A 140 99.398 68.570 70.012 1.00 19.38 A
ATOM 1099 O THR A 140 100.271 69.082 69.307 1.00 20.15 A
ATOM 1100 N PHE A 141 98.380 69.247 70.528 1.00 17.43 A
ATOM 1101 CA PHEA 141 98.181 70.669 70.283 1.00 17.21 A
ATOM 1102 CB PHE A 141 96.754 70.899 69.785 1.00 14.89 A
ATOM 1103 CG PHE A 141 96.425 70.156 68.523 1.00 17.06 A
ATOM 1104 CD1 PHE A 141 95.239 69.433 68.415 1.00 15.19 A
ATOM 1105 CD2 PHE A 141 97.290 70.191 67.434 1.00 16.39 A
ATOM 1106 CE1 PHE A 141 94.920 68.757 67.240 1.00 16.55 A
ATOM 1107 CE2 PHE A 141 96.977 69.516 66.250 1.00 16.87 A
ATOM 1108 CZ PHE A 141 95.791 68.799 66.155 1.00 16.20 A
ATOM 1109 C PHE A 141 98.418 71.532 71.518 1.00 19.11 A
ATOM 1110 O PHE A 141 97.989 71.189 72.626 1.00 18.90 A
ATOM 111 N GLN A 142 99.093 72.658 71.325 1.00 19.57 A
ATOM 1112 CA GLN A 142 99.341 73.562 72.433 1.00 20.59 A
ATOM 1113 CB GLN A 142 100.277 74.696 72.021 1.00 23.1 A
ATOM 1114 CG GLN A 142 100.630 75.601 73.189 1.0029.08 A
ATOM 1115 CD GLN A 142 101.492 76.778 72.805 1.00 33.00 A
ATOM 1116 OE1 GLN A 142 102.041 77.457 73.671 1.00 39.31 A
ATOM 1117 NE2 GLN A 142 101.608 77.038 71.508 1.00 35.20 A
ATOM 1118 C GLN A 142 98.002 74.157 72.851 1.00 19.90 A
ATOM 1119 O GLN A 142 97.124 74.364 72.017 1.00 18.19 A
ATOM 1120 N MET A 143 97.847 74.420 74.142 1.00 18.96 A
ATOM 1121 CA MET A 143 96.615 75.006 74.655 1.00 19.94 A
ATOM 1122 CB META 143 96.146 74.246 75.906 1.00 17.87 A
ATOM 1123 CG META 143 95.732 72.801 75.610 1.00 19.17 A
ATOM 1124 SD MET A 143 95.366 71.780 77.058 1.0021.35 A
ATOM 1125 CE MET A 143 93.707 72.320 77.469 1.00 17.43 A
ATOM 1126 C META 143 96.872 76.474 74.979 1.00 20.18 A
ATOM 1127 O META 143 98.016 76.877 75.219 1.0019.89 A
ATOM 1128 N THR A 144 95.815 77.275 74.979 1.0019.31 A
ATOM 1129 CA THR A 144 95.952 78.697 75.264 1.00 21.23 A
ATOM 1130 CB THR A 144 95.134 79.541 74.266 1.00 21.90 A
ATOM 1131 OG1 THR A 144 95.547 79.229 72.929 1.00 22.68 A
ATOM 1132 CG2 THR A 144 95.348 81.032 74.520 1.00 21.21 A
ATOM 1133 C THR A 144 95.481 79.023 76.675 1.00 21.58 A
ATOM 1134 O THR A 144 94.475 78.489 77.139 1.00 22.49 A
ATOM 1135 N ARG A 145 96.205 79.905 77.353 1.0021.27 A
ATOM 1136 CA ARG A 145 95.830 80.294 78.706 1.00 22.16 A
ATOM 1137 CB ARG A 145 97.054 80.755 79.507 1.0020.70 A
ATOM 1138 CG ARG A 145 96.714 81.119 80.954 1.00 18.15 A
ATOM 1139 CD ARG A 145 97.941 81.525 81.759 1.0019.19 A
ATOM 1140 NE ARG A 145 97.593 81.912 83.126 1.00 18.04 A
ATOM 1141 CZ ARG A 145 98.034 81.294 84.216 1.00 17.93 A
ATOM 1142 NH1 ARG A 145 98.841 80.248 84.107 1.00 18.86 A
ATOM 1143 NH2 ARG A 145 97.684 81.733 85.417 1.00 17.79 A
ATOM 1144 C ARG A 145 94.799 81.418 78.681 1.00 22.11 A
ATOM 1145 O ARG A 145 95.035 82.470 78.100 1.0021.50 A
ATOM 1146 N LEU A 146 93.658 81.180 79.316 1.00 22.33 A
ATOM 1147 CA LEU A 146 92.590 82.166 79.391 1.0022.95 A
ATOM 1148 CB LEU A 146 91.244 81.503 79.105 1.00 22.25 A
ATOM 1149 CG LEU A 146 91.154 80.645 77.838 1.00 24.65 A
ATOM 1150 CD1 LEU A 146 89.754 80.052 77.736 1.00 21.10 A
ATOM 1151 CD2 LEU A 146 91.472 81.488 76.613 1.00 19.93 A
ATOM 1152 C LEU A 146 92.593 82.730 80.808 1.00 24.61 A
ATOM 1153 O LEU A 146 93.604 82.651 81.513 1.0026.21 A
ATOM 1154 N GLU A 147 91.465 83.286 81.233 1.0025.28 A
ATOM 1155 CA GLU A 147 91.362 83.846 82.577 1.00 26.61 A
ATOM 1156 CB GLU A 147 90.132 84.755 82.681 1.00 29.29 A
ATOM 1157 CG GLU A 147 90.190 85.953 81.758 1.00 35.59 A
ATOM 1158 CD GLU A 147 91.397 86.829 82.037 1.00 40.51 A
ATOM 1159 OE1 GLU A 147 91.699 87.709 81.204 1.00 44.44 A
ATOM 1160 OE2 GLU A 147 92.044 86.640 83.092 1.00 43.00 A
ATOM 1161 C GLU A 147 91.258 82.738 83.612 1.00 25.61 A
ATOM 1162 O GLU A 147 90.972 81.591 83.276 1.00 23.46 A
ATOM 1163 N LYS A 148 91.502 83.090 84.871 1.00 25.38 A
ATOM 1164 CA LYS A 148 91.415 82.143 85.978 1.00 23.98 A
ATOM 1165 CB LYS A 148 89.972 81.654 86.129 1.0025.57 A
ATOM 1166 CG LYS A 148 88.931 82.768 86.125 1.00 26.35 A
ATOM 1167 CD LYS A 148 89.178 83.755 87.247 1.00 28.03 A
ATOM 1168 CE LYS A 148 88.131 84.854 87.272 1.00 27.41 A
ATOM 1169 NZ LYS A 148 88.321 85.706 88.477 1.0027.48 A
ATOM 1170 C LYS A 148 92.341 80.941 85.821 1.0023.89 A
ATOM 1171 O LYS A 148 92.005 79.834 86.246 1.00 23.62 A
ATOM 1172 N GLY A 149 93.502 81.164 85.212 1.0021.55 A
ATOM 1173 CA GLY A 149 94.467 80.098 85.027 1.00 19.09 A
ATOM 1174 C GLY A 149 93.962 78.878 84.278 1.00 18.97 A
ATOM 1175 O GLY A 149 94.495 77.770 84.438 1.0017.47 A
ATOM 1176 N VAL A 150 92.939 79.070 83.455 1.00 17.85 A
ATOM 1177 CA VAL A 150 92.386 77.970 82.673 1.00 16.35 A
ATOM 1178 CB VAL A 150 90.844 78.112 82.520 1.00 17.78 A
ATOM 1179 CG1 VAL A 150 90.316 77.134 81.465 1.00 14.08 A
ATOM 1180 CG2 VAL A 150 90.165 77.848 83.865 1.00 13.60 A
ATOM 1181 C VAL A 150 93.011 77.884 81.280 1.00 17.12 A
ATOM 1182 O VAL A 150 93.063 78.875 80.558 1.00 19.24 A
ATOM 1183 N TYR A 151 93.491 76.700 80.913 1.00 17.09 A
ATOM 1184 CA TYR A 151 94.068 76.478 79.587 1.00 17.10 A
ATOM 1185 CB TYR A 151 95.339 75.623 79.672 1.00 14.98 A
ATOM 1186 CG TYR A 151 96.536 76.385 80.172 1.00 16.11 A
ATOM 1187 CD1 TYR A 151 97.415 77.013 79.284 1.00 15.75 A
ATOM 1188 CE1 TYR A 151 98.484 77.772 79.754 1.00 15.20 A
ATOM 1189 CD2 TYR A 151 96.759 76.535 81.537 1.00 14.80 A
ATOM 1190 CE2 TYR A 151 97.814 77.291 82.014 1.00 15.23 A
ATOM 1191 CZ TYR A 151 98.672 77.909 81.123 1.00 16.51 A
ATOM 1192 OH TYR A 151 99.704 78.672 81.620 1.0017.89 A
ATOM 1193 C TYR A 151 93.025 75.757 78.741 1.00 17.04 A
ATOM 1194 O TYR A 151 92.353 74.840 79.213 1.00 16.81 A
ATOM 1195 N ALA A 152 92.891 76.166 77.487 1.00 16.81 A
ATOM 1196 CA ALA A 152 91.916 75.537 76.612 1.00 16.67 A
ATOM 1197 CB ALAA 152 90.590 76.285 76.704 1.00 17.71 A
ATOM 1198 C ALA A 152 92.376 75.490 75.168 1.00 16.90 A
ATOM 1199 O ALA A 152 93.290 76.209 74.771 1.00 18.15 A
ATOM 1200 N VAL A 153 91.735 74.624 74.391 1.00 17.29 A
ATOM 1201 CA VALA 153 92.010 74.492 72.968 1.00 16.64 A
ATOM 1202 CB VALA 153 93.300 73.666 72.669 1.00 17.27 A
ATOM 1203 CG1 VALA 153 93.122 72.209 73.053 1.00 12.35 A
ATOM 1204 CG2 VAL A 153 93.643 73.780 71.189 1.00 16.43 A
ATOM 1205 C VALA 153 90.805 73.790 72.371 1.00 18.13 A
ATOM 1206 O VAL A 153 90.252 72.870 72.972 1.00 19.58 A
ATOM 1207 N THR A 154 90.373 74.243 71.203 1.00 17.35 A
ATOM 1208 CA THR A 154 89.234 73.633 70.547 1.00 18.05 A
ATOM 1209 CB THR A 154 88.211 74.694 70.117 1.00 18.26 A
ATOM 1210 OG1 THRA 154 87.804 75.437 71.269 1.00 17.88 A
ATOM 1211 CG2 THR A 154 86.982 74.042 69.493 1.00 19.66 A
ATOM 1212 C THR A 154 89.759 72.886 69.336 1.00 19.27 A
ATOM 1213 O THR A 154 90.481 73.453 68.518 1.00 18.46 A
ATOM 1214 N VALA 155 89.410 71.607 69.245 1.00 19.50 A
ATOM 1215 CA VALA 155 89.857 70.755 68.148 1.00 21.32 A
ATOM 1216 CB VALA 155 90.495 69.449 68.703 1.00 20.19 A
ATOM 1217 CG1 VALA 155 91.052 68.608 67.572 1.00 19.19 A
ATOM 1218 CG2 VALA 155 91.596 69.793 69.705 1.00 17.15 A
ATOM 1219 C VAL A 155 88.648 70.415 67.279 1.00 23.50 A
ATOM 1220 O VAL A 155 87.677 69.836 67.766 1.00 24.72 A
ATOM 1221 N THR A 156 88.691 70.785 65.999 1.00 24.96 A
ATOM 1222 CA THR A 156 87.561 70.500 65.123 1.00 24.38 A
ATOM 1223 CB THR A 156 87.562 71.357 63.837 1.00 25.83 A
ATOM 1224 OG1 THR A 156 88.096 70.583 62.759 1.0028.92 A
ATOM 1225 CG2 THR A 156 88.394 72.608 64.013 1.00 23.23 A
ATOM 1226 C THR A 156 87.588 69.040 64.706 1.00 25.30 A
ATOM 1227 O THR A 156 88.655 68.439 64.561 1.0024.58 A
ATOM 1228 N GLY A 157 86.399 68.484 64.507 1.00 26.05 A
ATOM 1229 CA GLY A 157 86.277 67.095 64.117 1.00 25.39 A
ATOM 1230 C GLY A 157 85.508 66.360 65.194 1.00 25.37 A
ATOM 1231 O GLY A 157 85.256 66.913 66.264 1.00 23.39 A
ATOM 1232 N ASP A 158 85.116 65.124 64.907 1.00 25.33 A
ATOM 1233 CA ASP A 158 84.388 64.313 65.872 1.00 26.00 A
ATOM 1234 CB ASP A 158 83.460 63.329 65.156 1.00 28.34 A
ATOM 1235 CG ASP A 158 82.498 62.644 66.107 1.0029.60 A
ATOM 1236 OD1 ASP A 158 82.832 62.517 67.307 1.00 28.79 A
ATOM 1237 OD2 ASP A 158 81.411 62.226 65.654 1.00 31.89 A
ATOM 1238 C ASP A 158 85.446 63.536 66.636 1.00 24.87 A
ATOM 1239 O ASP A 158 86.062 62.625 66.086 1.00 24.74 A
ATOM 1240 N LEU A 159 85.667 63.885 67.895 1.00 24.25 A
ATOM 1241 CA LEU A 159 86.691 63.192 68.659 1.00 24.19 A
ATOM 1242 CB LEU A 159 87.389 64.166 69.621 1.00 23.29 A
ATOM 1243 CG LEU A 159 88.160 65.351 69.011 1.00 22.99 A
ATOM 1244 CD1 LEU A 159 89.197 65.846 70.006 1.00 22.30 A
ATOM 1245 CD2 LEU A 159 88.861 64.930 67.732 1.00 24.14 A
ATOM 1246 C LEU A 159 86.214 61.963 69.423 1.00 24.45 A
ATOM 1247 O LEU A 159 86.908 61.492 70.325 1.00 25.62 A
ATOM 1248 N HIS A 160 85.048 61.433 69.064 1.0022.78 A
ATOM 1249 CA HIS A 160 84.531 60.253 69.755 1.00 23.00 A
ATOM 1250 CB HIS A 160 83.149 59.848 69.221 1.00 22.66 A
ATOM 1251 CG HIS A 160 82.610 58.596 69.845 1.0023.13 A
ATOM 1252 CD2 HIS A 160 81.800 58.410 70.914 1.00 24.71 A
ATOM 1253 ND1 HIS A 160 82.951 57.334 69.402 1.00 23.29 A
ATOM 1254 CE1 HIS A 160 82.376 56.427 70.171 1.00 22.43 A
ATOM 1255 NE2 HIS A 160 81.672 57.053 71.097 1.0024.97 A
ATOM 1256 C HIS A 160 85.481 59.071 69.622 1.00 22.62 A
ATOM 1257 O HIS A 160 85.899 58.717 68.517 1.00 22.36 A
ATOM 1258 N GLY A 161 85.816 58.465 70.758 1.00 21.49 A
ATOM 1259 CA GLY A 161 86.712 57.323 70.756 1.00 20.58 A
ATOM 1260 C GLY A 161 88.158 57.734 70.925 1.00 20.86 A
ATOM 1261 O GLY A 161 89.023 56.889 71.165 1.00 21.45 A
ATOM 1262 N TYR A 162 88.422 59.032 70.792 1.00 19.32 A
ATOM 1263 CA TYR A 162 89.774 59.562 70.931 1.00 19.44 A
ATOM 1264 CB TYRA 162 89.851 60.999 70.397 1.00 19.84 A
ATOM 1265 CG TYR A 162 90.224 61.097 68.933 1.0023.17 A
ATOM 1266 CD1 TYR A 162 89.282 60.856 67.930 1.00 23.70 A
ATOM 1267 CE1 TYR A 162 89.640 60.904 66.577 1.0025.48 A
ATOM 1268 CD2 TYRA 162 91.535 61.390 68.553 1.00 22.93 A
ATOM 1269 CE2 TYR A 162 91.906 61.435 67.213 1.00 25.60 A
ATOM 1270 CZ TYRA 162 90.955 61.191 66.230 1.0026.50 A
ATOM 1271 OH TYR A 162 91.330 61.221 64.908 1.00 28.51 A
ATOM 1272 C TYR A 162 90.281 59.543 72.371 1.00 18.98 A
ATOM 1273 O TYR A 162 89.568 59.930 73.303 1.0017.45 A
ATOM 1274 N GLU A 163 91.516 59.081 72.542 1.0017.29 A
ATOM 1275 CA GLU A 163 92.143 59.043 73.856 1.00 17. 10 A
ATOM 1276 CB GLU A 163 93.182 57.929 73.919 1.00 18.00 A
ATOM 1277 CG GLU A 163 92.659 56.599 73.412 1.00 22.64 A
ATOM 1278 CD GLU A 163 93.760 55.596 73.149 1.0024.59 A
ATOM 1279 OE1 GLU A 163 94.865 56.015 72.725 1.0023.85 A
ATOM 1280 OE2 GLU A 163 93.510 54.389 73.345 1.0026.35 A
ATOM 1281 C GLU A 163 92.832 60.391 74.005 1.00 16.94 A
ATOM 1282 O GLU A 163 93.267 60.984 73.013 1.00 17.04 A
ATOM 1283 N TYRA 164 92.916 60.890 75.230 1.00 14.45 A
ATOM 1284 CA TYRA 164 93.565 62.166 75.444 1.00 13.69 A
ATOM 1285 CB TYRA 164 92.552 63.322 75.330 1.00 12.99 A
ATOM 1286 CG TYRA 164 91.573 63.493 76.483 1.0013.11 A
ATOM 1287 CD1 TYRA 164 91.635 64.620 77.308 1.00 12.65 A
ATOM 1288 CE1 TYRA 164 90.681 64.842 78.302 1.00 11.84 A
ATOM 1289 CD2 TYRA 164 90.535 62.583 76.693 1.00 10.43 A
ATOM 1290 CE2 TYR A 164 89.580 62.795 77.681 1.00 11.29 A
ATOM 1291 CZ TYRA 164 89.655 63.928 78.483 1.00 13.96 A
ATOM 1292 OH TYRA 164 88.697 64.156 79.450 1.00 10.64 A
ATOM 1293 C TYRA 164 94.281 62.227 76.773 1.00 13.38 A
ATOM 1294 O TYR A 164 93.938 61.514 77.711 1.00 12.69 A
ATOM 1295 N LEU A 165 95.295 63.079 76.822 1.00 13.30 A
ATOM 1296 CA LEU A 165 96.096 63.297 78.013 1.00 13.49 A
ATOM 1297 CB LEU A 165 97.392 62.493 77.946 1.00 14.08 A
ATOM 1298 CG LEU A 165 97.351 60.969 78.049 1.00 15.54 A
ATOM 1299 CD1 LEU A 165 98.766 60.437 77.850 1.00 13.53 A
ATOM 1300 CD2 LEU A 165 96.808 60.538 79.413 1.00 9.61 A
ATOM 1301 C LEU A 165 96.446 64.767 78.008 1.00 14.83 A
ATOM 1302 O LEU A 165 96.434 65.402 76.955 1.00 15.53 A
ATOM 1303 N PHE A 166 96.740 65.317 79.177 1.00 15.09 A
ATOM 1304 CA PHE A 166 97.142 66.712 79.259 1.00 17.41 A
ATOM 1305 CB PHE A 166 96.363 67.466 80.352 1.00 14.80 A
ATOM 1306 CG PHE A 166 94.930 67.764 79.992 1.00 15.87 A
ATOM 1307 CD 1 PHE A 166 93.940 67.763 80.975 1.00 14.74 A
ATOM 1308 CD2 PHE A 166 94.566 68.046 78.675 1.00 16.72 A
ATOM 1309 CE1 PHE A 166 92.606 68.034 80.654 1.0014.88 A
ATOM 1310 CE2 PHE A 166 93.234 68.321 78.341 1.00 17.11 A
ATOM 1311 CZ PHE A 166 92.252 68.314 79.333 1.00 16.27 A
ATOM 1312 C PHE A 166 98.629 66.737 79.592 1.00 18.69 A
ATOM 1313 O PHE A 166 99.140 65.879 80.321 1.00 18.13 A
ATOM 1314 N CYS A 167 99.328 67.706 79.025 1.00 20.66 A
ATOM 1315 CA CYS A 167 100.742 67.875 79.292 1.00 22.05 A
ATOM 1316 CB CYS A 167 101.534 67.898 77.979 1.00 25.54 A
ATOM 1317 SG CYS A 167 101.503 66.327 77.047 1.00 34.53 A
ATOM 1318 C CYS A 167 100.789 69.227 80.002 1.00 21.20 A
ATOM 1319 O CYS A 167 100.432 70.252 79.427 1.00 21.25 A
ATOM 1320 N ILE A 168 101.199 69.220 81.263 1.00 19.78 A
ATOM 1321 CA ILE A 168 101.249 70.444 82.048 1.00 18.12 A
ATOM 1322 CB ILEA 168 100.432 70.290 83.354 1.00 17.63 A
ATOM 1323 CG2 ILEA 168 100.328 71.626 84.050 1.00 15.87 A
ATOM 1324 CG1 ILE A 168 99.041 69.711 83.052 1.00 16.22 A
ATOM 1325 CD1 ILE A 168 98.158 70.596 82.193 1.00 17.67 A
ATOM 1326 C ILE A 168 102.687 70.802 82.410 1.00 18.46 A
ATOM 1327 O ILE A 168 103.432 69.975 82.942 1.00 17.84 A
ATOM 1328 N CYS A 169 103.077 72.040 82.132 1.00 17.78 A
ATOM 1329 CA CYS A 169 104.437 72.466 82.440 1.00 18.13 A
ATOM 1330 CB CYS A 169 105.045 73.203 81.242 1.00 17.87 A
ATOM 1331 SG CYS A 169 106.765 73.698 81.488 1.00 15.89 A
ATOM 1332 C CYS A 169 104.516 73.351 83.679 1.00 17.64 A
ATOM 1333 O CYS A 169 104.162 74.532 83.628 1.00 16.45 A
ATOM 1334 N ASN A 170 104.975 72.762 84.785 1.00 16.52 A
ATOM 1335 CA ASN A 170 105.143 73.458 86.059 1.00 16.30 A
ATOM 1336 CB ASN A 170 104.347 72.758 87.177 1.00 14.76 A
ATOM 1337 CG ASN A 170 102.841 73.000 87.082 1.00 17.58 A
ATOM 1338 OD1 ASN A 170 102.371 74.138 87.196 1.00 17.69 A
ATOM 1339 ND2 ASN A 170 102.079 71.926 86.884 1.00 11.45 A
ATOM 1340 C ASN A 170 106.627 73.441 86.433 1.00 17.55 A
ATOM 1341 O ASN A 170 107.269 72.389 86.378 1.00 18.20 A
ATOM 1342 N ASN A 171 107.172 74.596 86.813 1.00 18.49 A
ATOM 1343 CA ASN A 171 108.577 74.676 87.206 1.00 18.39 A
ATOM 1344 CB ASN A 171 108.791 73.875 88.489 1.00 20.37 A
ATOM 1345 CG ASN A 171 108.017 74.446 89.669 1.00 24.13 A
ATOM 1346 OD1 ASN A 171 107.652 73.722 90.594 1.00 27.89 A
ATOM 1347 ND2 ASN A 171 107.773 75.748 89.643 1.00 24.07 A
ATOM 1348 C ASN A 171 109.501 74.147 86.110 1.0018.06 A
ATOM 1349 O ASN A 171 110.502 73.479 86.389 1.0017.13 A
ATOM 1350 N SERA 172 109.147 74.442 84.864 1.00 16.91 A
ATOM 1351 CA SERA 172 109.918 74.009 83.704 1.00 19.03 A
ATOM 1352 CB SERA 172 111.334 74.575 83.775 1.00 19.66 A
ATOM 1353 OG SERA 172 111.314 75.979 83.584 1.0023.18 A
ATOM 1354 C SERA 172 109.973 72.493 83.478 1.00 19.71 A
ATOM 1355 O SERA 172 110.804 72.005 82.706 1.00 20.95 A
ATOM 1356 N GLU A 173 109.098 71.751 84.150 1.00 18.44 A
ATOM 1357 CA GLU A 173 109.037 70.308 83.961 1.00 16.95 A
ATOM 1358 CB GLU A 173 109.283 69.553 85.268 1.00 8.55 A
ATOM 1359 CG GLU A 173 109.208 68.041 85.077 1.00 18.25 A
ATOM 1360 CD GLU A 173 109.636 67.240 86.306 1.0020.21 A
ATOM 1361 OE1 GLU A 173 110.106 67.837 87.300 1.0020.61 A
ATOM 1362 OE2 GLU A 173 109.508 66.001 86.263 1.00 19.29 A
ATOM 1363 C GLU A 173 107.657 69.950 83.426 1.00 17.05 A
ATOM 1364 O GLU A 173 106.636 70.330 84.008 1.00 14.92 A
ATOM 1365 N TRP A 174 107.631 69.235 82.305 1.00 16.39 A
ATOM 1366 CA TRP A 174 106.376 68.826 81.705 1.00 15.04 A
ATOM 1367 CB TRP A 174 106.506 68.614 80.191 1.00 16.73 A
ATOM 1368 CG TRP A 174 106.605 69.864 79.386 1.00 7.53 A
ATOM 1369 CD2 TRP A 174 105.522 70.579 78.783 1.00 18.82 A
ATOM 1370 CE2 TRP A 174 106.078 71.699 78.124 1.00 17.44 A
ATOM 1371 CE3 TRP A 174 104.133 70.384 78.735 1.00 18.01 A
ATOM 1372 CD1 TRP A 174 107.740 70.556 79.081 1.00 18.56 A
ATOM 1373 NE1 TRP A 174 107.433 71.658 78.323 1.00 19.16 A
ATOM 1374 CZ2 TRP A 174 105.296 72.624 77.424 1.00 15.50 A
ATOM 1375 CZ3 TRP A 174 103.353 71.305 78.039 1.00 16.47 A
ATOM 1376 CH2 TRP A 174 103.939 72.412 77.393 1.00 16.79 A
ATOM 1377 C TRP A 174 105.944 67.521 82.307 1.00 16.63 A
ATOM 1378 O TRP A 174 106.752 66.610 82.479 1.00 18.87 A
ATOM 1379 N META 175 104.667 67.427 82.641 1.00 17.19 A
ATOM 1380 CA META 175 104.150 66.187 83.165 1.00 19.82 A
ATOM 1381 CB META 175 103.853 66.305 84.658 1.00 22.99 A
ATOM 1382 CG META 175 105.085 65.932 85.484 1.00 24.86 A
ATOM 1383 SD META 175 104.870 66.018 87.242 1.00 34.31 A
ATOM 1384 CE META 175 106.534 66.392 87.781 1.00 29.75 A
ATOM 1385 C MET A 175 102.929 65.781 82.366 1.00 21.33 A
ATOM 1386 O MET A 175 102.135 66.619 81.936 1.00 22.93 A
ATOM 1387 N GLU A 176 102.819 64.483 82.132 1.00 21.36 A
ATOM 1388 CA GLU A 176 101.722 63.918 81.367 1.00 22.69 A
ATOM 1389 CB GLU A 176 102.255 62.782 80.517 1.00 24.09 A
ATOM 1390 CG GLU A 176 101.448 62.453 79.315 1.0028.75 A
ATOM 1391 CD GLU A 176 102.144 61.402 78.483 1.00 32.06 A
ATOM 1392 OE1 GLU A 176 102.168 60.228 78.918 1.00 33.42 A
ATOM 1393 OE2 GLU A 176 102.685 61.758 77.415 1.0031.96 A
ATOM 1394 C GLU A 176 100.699 63.385 82.347 1.00 20.61 A
ATOM 1395 O GLU A 176 101.051 62.671 83.275 1.00 23.13 A
ATOM 1396 N THR A 177 99.433 63.711 82.139 1.00 19.75 A
ATOM 1397 CA THR A 177 98.420 63.245 83.064 1.00 19.81 A
ATOM 1398 CB THR A 177 98.284 64.214 84.254 1.00 21.87 A
ATOM 1399 OG1 THR A 177 97.309 63.713 85.170 1.00 25.08 A
ATOM 1400 CG2 THRA 177 97.847 65.589 83.771 1.00 23.16 A
ATOM 1401 C THR A 177 97.054 63.086 82.442 1.00 18.00 A
ATOM 1402 O THR A 177 96.759 63.671 81.407 1.00 18.15 A
ATOM 1403 N VAL A 178 96.224 62.280 83.093 1.00 16.76 A
ATOM 1404 CA VAL A 178 94.860 62.049 82.657 1.00 15.51 A
ATOM 1405 CB VAL A 178 94.293 60.732 83.235 1.00 14.06 A
ATOM 1406 CG1 VAL A 178 92.797 60.635 82.958 1.00 13.99 A
ATOM 1407 CG2 VAL A 178 95.014 59.547 82.624 1.00 14.59 A
ATOM 1408 C VAL A 178 94.029 63.199 83.207 1.00 17.27 A
ATOM 1409 O VAL A 178 94.251 63.651 84.332 1.00 19.10 A
ATOM 1410 N ASP A 179 93.081 63.674 82.410 1.00 16.95 A
ATOM 1411 CA ASP A 179 92.189 64.754 82.817 1.00 15.29 A
ATOM 1412 CB ASP A 179 91.164 64.982 81.693 1.00 16.40 A
ATOM 1413 CG ASP A 179 90.038 65.915 82.088 1.00 18.44 A
ATOM 1414 OD1 ASP A 179 90.202 66.715 83.038 1.00 18.85 A
ATOM 1415 OD2 ASP A 179 88.982 65.853 81.423 1.00 19.09 A
ATOM 1416 C ASP A 179 91.500 64.362 84.126 1.00 15.85 A
ATOM 1417 O ASP A 179 90.987 63.248 84.246 1.00 14.56 A
ATOM 1418 N GLN A 180 91.518 65.262 85.111 1.00 15.14 A
ATOM 1419 CA GLN A 180 90.880 65.019 86.410 1.00 16.46 A
ATOM 1420 CB GLN A 180 91.063 66.228 87.340 1.0015.16 A
ATOM 1421 CG GLN A 180 92.504 66.606 87.609 1.0015.02 A
ATOM 1422 CD GLN A 180 92.648 67.981 88.234 1.00 16.64 A
ATOM 1423 OE1 GLN A 180 93.747 68.534 88.286 1.00 19.71 A
ATOM 1424 NE2 GLN A 180 91.544 68.539 88.713 1.00 10.34 A
ATOM 1425 C GLN A 180 89.381 64.785 86.213 1.00 18.39 A
ATOM 1426 O GLN A 180 88.741 64.062 86.982 1.0017.36 A
ATOM 1427 N TYR A 181 88.831 65.409 85.174 1.00 18.55 A
ATOM 1428 CA TYR A 181 87.411 65.295 84.860 1.00 18.23 A
ATOM 1429 CB TYR A 181 86.874 66.662 84.442 1.0017.50 A
ATOM 1430 CG TYR A 181 86.649 67.605 85.599 1.00 16.76 A
ATOM 1431 CD1 TYR A 181 85.394 67.702 86.200 1.00 15.01 A
ATOM 1432 CE1 TYR A 181 85.166 68.567 87.265 1.00 16.28 A
ATOM 1433 CD2 TYR A 181 87.689 68.402 86.099 1.00 15.83 A
ATOM 1434 CE2 TYR A 181 87.470 69.275 87.175 1.00 16.12 A
ATOM 1435 CZ TYRA 181 86.201 69.349 87.746 1.00 16.65 A
ATOM 1436 OH TYRA 181 85.943 70.211 88.782 1.00 16.11 A
ATOM 1437 C TYR A 181 87.122 64.271 83.766 1.00 19.11 A
ATOM 1438 O TYR A 181 86.086 64.343 83.107 1.0020.36 A
ATOM 1439 N ALA A 182 88.034 63.322 83.578 1.00 17.54 A
ATOM 1440 CA ALA A 182 87.867 62.280 82.567 1.0016.62 A
ATOM 1441 CB ALA A 182 89.092 61.373 82.555 1.00 11.71 A
ATOM 1442 C ALA A 182 86.611 61.455 82.854 1.00 17.26 A
ATOM 1443 O ALA A 182 86.490 60.859 83.928 1.0017.26 A
ATOM 1444 N LYS A 183 85.690 61.406 81.896 1.00 15.69 A
ATOM 1445 CA LYS A 183 84.447 60.657 82.082 1.00 17.65 A
ATOM 1446 CB LYS A 183 83.272 61.496 81.562 1.00 14.78 A

ATOM 1447 CG LYS A 183 83.039 62.690 82.492 1.00 17.73 A
ATOM 1448 CD LYS A 183 82.197 63.814 81.922 1.00 18.02 A
ATOM 1449 CE LYS A 183 82.117 64.949 82.953 1.00 21.75 A
ATOM 1450 NZ LYS A 183 81.349 66.140 82.487 1.00 27.10 A
ATOM 1451 C LYS A 183 84.482 59.261 81.469 1.00 17.33 A
ATOM 1452 O LYS A 183 83.525 58.491 81.551 1.00 17.78 A
ATOM 1453 N ALA A 184 85.625 58.935 80.885 1.00 17.60 A
ATOM 1454 CA ALA A 184 85.855 57.631 80.283 1.00 17.24 A
ATOM 1455 CB ALA A 184 85.274 57.578 78.873 1.00 16.25 A
ATOM 1456 C ALAA 184 87.363 57.467 80.243 1.00 16.33 A
ATOM 1457 O ALAA 184 88.092 58.451 80.132 1.00 15.79 A
ATOM 1458 N VALA 185 87.834 56.232 80.354 1.00 16.61 A
ATOM 1459 CA VALA 185 89.264 55.973 80.319 1.00 17.37 A
ATOM 1460 CB VALA 185 89.872 55.973 81.740 1.00 17.99 A
ATOM 1461 CG1 VAL A 185 89.687 57.344 82.384 1.00 17.68 A
ATOM 1462 CG2 VAL A 185 89.221 54.878 82.588 1.00 16.63 A
ATOM 1463 C VALA 185 89.566 54.639 79.665 1.00 17.33 A
ATOM 1464 O VAL A 185 88.680 53.802 79.498 1.00 20.28 A
ATOM 1465 N THR A 186 90.825 54.448 79.295 1.00 17.29 A
ATOM 1466 CA THR A 186 91.267 53.213 78.667 1.00 17.51 A
ATOM 1467 CB THR A 186 92.583 53.439 77.909 1.00 18.19 A
ATOM 1468 OG1 THR A 186 93.554 54.010 78.801 1.00 17.37 A
ATOM 1469 CG2 THRA 186 92.360 54.379 76.736 1.00 17.22 A
ATOM 1470 C THRA 186 91.490 52.153 79.749 1.00 18.90 A
ATOM 1471 O THR A 186 91.419 52.459 80.944 1.00 17.63 A
ATOM 1472 N VAL A 187 91.758 50.916 79.333 1.00 18.07 A
ATOM 1473 CA VAL A 187 91.990 49.829 80.282 1.00 19.82 A
ATOM 1474 CB VAL A 187 92.456 48.535 79.555 1.00 20.05 A
ATOM 1475 CG1 VAL A 187 93.784 48.771 78.853 1.00 19.96 A
ATOM 1476 CG2 VAL A 187 92.567 47.388 80.547 1.00 20.41 A
ATOM 1477 C VAL A 187 93.035 50.248 81.323 1.00 19.45 A
ATOM 1478 O VAL A 187 94.064 50.830 80.986 1.00 19.50 A
ATOM 1479 N ASN A 188 92.754 49.955 82.589 1.0019.02 A
ATOM 1480 CA ASN A 188 93.645 50.314 83.693 1.00 19.46 A
ATOM 1481 CB ASN A 188 94.991 49.586 83.575 1.00 19.20 A
ATOM 1482 CG ASN A 188 94.900 48.127 84.012 1.00 22.86 A
ATOM 1483 OD1 ASN A 188 94.170 47.796 84.953 1.00 23.80 A
ATOM 1484 ND2 ASN A 188 95.650 47.254 83.344 1.00 19.60 A
ATOM 1485 C ASN A 188 93.879 51.818 83.825 1.00 19.77 A
ATOM 1486 O ASN A 188 94.902 52.252 84.358 1.00 18.23 A
ATOM 1487 N GLY A 189 92.918 52.599 83.330 1.00 19.48 A
ATOM 1488 CA GLY A 189 92.972 54.052 83.413 1.00 18.51 A
ATOM 1489 C GLY A 189 94.235 54.791 83.010 1.00 19.13 A
ATOM 1490 O GLY A 189 94.574 55.812 83.610 1.00 18.83 A
ATOM 1491 N GLU A 190 94.925 54.307 81.987 1.0019.91 A
ATOM 1492 CA GLU A 190 96.152 54.957 81.538 1.00 22.01 A
ATOM 1493 CB GLU A 190 96.961 53.990 80.664 1.0023.89 A
ATOM 1494 CG GLU A 190 97.179 52.638 81.339 1.00 33.67 A
ATOM 1495 CD GLU A 190 98.264 51.799 80.680 1.0039.90 A
ATOM 1496 OE1 GLU A 190 99.443 52.213 80.722 1.0043.40 A
ATOM 1497 OE2 GLU A 190 97.941 50.726 80.123 1.00 42.19 A
ATOM 1498 C GLU A 190 95.878 56.262 80.790 1.00 20.01 A
ATOM 1499 O GLU A 190 96.662 57.204 80.862 1.00 20.02 A
ATOM 1500 N LYS A 191 94.762 56.323 80.073 1.00 19.47 A
ATOM 1501 CA LYS A 191 94.417 57.531 79.335 1.00 19.99 A
ATOM 1502 CB LYS A 191 94.764 57.377 77.851 1.00 20.21 A
ATOM 1503 CG LYS A 191 96.226 57.114 77.561 1.00 20.17 A
ATOM 1504 CD LYS A 191 96.446 56.900 76.074 1.00 21.50 A
ATOM 1505 CE LYS A 191 97.913 56.686 75.758 1.0021.14 A
ATOM 1506 NZ LYS A 191 98.117 56.564 74.295 1.00 26.39 A
ATOM 1507 C LYS A 191 92.936 57.847 79.447 1.00 19.45 A
ATOM 1508 O LYS A 191 92.116 56.965 79.703 1.00 19.17 A
ATOM 1509 N GLY A 192 92.602 59.116 79.256 1.00 18.67 A
ATOM 1510 CA GLY A 192 91.211 59.513 79.292 1.00 17.58 A
ATOM 1511 C GLY A 192 90.675 59.278 77.893 1.0017.86 A
ATOM 1512 O GLY A 192 91.455 59.098 76.949 1.0017.08 A
ATOM 1513 N VAL A 193 89.356 59.268 77.747 1.00 17.55 A
ATOM 1514 CA VAL A 193 88.746 59.048 76.440 1.00 16.68 A
ATOM 1515 CB VAL A 193 88.226 57.594 76.292 1.00 16.61 A
ATOM 1516 CG1 VAL A 193 87.622 57.400 74.919 1.00 17.12 A
ATOM 1517 CG2 VAL A 193 89.356 56.601 76.504 1.00 18.40 A
ATOM 1518 C VAL A 193 87.569 59.984 76.242 1.00 16.66 A
ATOM 1519 O VAL A 193 86.797 60.219 77.167 1.0017.66 A
ATOM 1520 N VAL A 194 87.442 60.522 75.035 1.00 17.64 A
ATOM 1521 CA VAL A 194 86.331 61.407 74.696 1.00 16.97 A
ATOM 1522 CB VAL A 194 86.736 62.457 73.644 1.00 17.21 A
ATOM 1523 CG1 VAL A 194 85.582 63.425 73.415 1.00 14.46 A
ATOM 1524 CG2 VALA 194 87.991 63.188 74.088 1.00 15.59 A
ATOM 1525 C VAL A 194 85.227 60.544 74.085 1.00 17.67 A
ATOM 1526 O VAL A 194 85.474 59.795 73.144 1.00 18.97 A
ATOM 1527 N LEU A 195 84.016 60.644 74.619 1.00 17.29 A
ATOM 1528 CA LEU A 195 82.892 59.864 74.107 1.00 18.24 A
ATOM 1529 CB LEU A 195 82.570 58.691 75.049 1.00 17.65 A
ATOM 1530 CG LEU A 195 83.608 57.569 75.195 1.00 19.18 A
ATOM 1531 CD1 LEU A 195 83.141 56.568 76.248 1.00 19.22 A
ATOM 1532 CD2 LEU A 195 83.819 56.879 73.854 1.00 18.52 A
ATOM 1533 C LEU A 195 81.669 60.751 73.996 1.00 18.89 A
ATOM 1534 O LEU A 195 81.440 61.602 74.854 1.00 18.04 A
ATOM 1535 N ARG A 196 80.879 60.560 72.944 1.00 19.73 A
ATOM 1536 CA ARG A 196 79.667 61.355 72.788 1.00 20.60 A
ATOM 1537 CB ARG A 196 79.097 61.202 71.378 1.0023.24 A
ATOM 1538 CG ARG A 196 78.691 59.784 71.036 1.00 27.04 A
ATOM 1539 CD ARG A 196 78.346 59.657 69.562 1.0029.07 A
ATOM 1540 NE ARG A 196 78.644 58.307 69.109 1.0034.53 A
ATOM 1541 CZ ARG A 196 79.381 58.022 68.044 1.0033.43 A
ATOM 1542 NH1 ARG A 196 79.901 58.994 67.305 1.00 34.50 A
ATOM 1543 NH2 ARG A 196 79.607 56.759 67.730 1.00 36.74 A
ATOM 1544 C ARG A 196 78.671 60.811 73.796 1.00 19.21 A
ATOM 1545 O ARG A 196 78.821 59.680 74.266 1.00 16.64 A
ATOM 1546 N PRO A 197 77.638 61.599 74.139 1.00 20.39 A
ATOM 1547 CD PRO A 197 77.278 62.924 73.600 1.00 21.23 A
ATOM 1548 CA PRO A 197 76.637 61.133 75.108 1.00 21.26 A
ATOM 1549 CB PRO A 197 75.579 62.234 75.072 1.00 21.64 A
ATOM 1550 CG PRO A 197 76.369 63.467 74.676 1.0021.83 A
ATOM 1551 C PRO A 197 76.094 59.790 74.634 1.00 22.32 A
ATOM 1552 O PRO A 197 75.750 59.644 73.462 1.00 23.02 A
ATOM 1553 N ASP A 198 76.030 58.811 75.530 1.0023.14 A
ATOM 1554 CA ASP A 198 75.550 57.485 75.158 1.00 23.88 A
ATOM 1555 CB ASP A 198 76.010 56.430 76.175 1.00 24.30 A
ATOM 1556 CG ASP A 198 75.601 56.762 77.603 1.0024.35 A
ATOM 1557 OD1 ASP A 198 74.550 57.409 77.806 1.00 23.84 A
ATOM 1558 OD2 ASP A 198 76.333 56.355 78.527 1.00 23.09 A
ATOM 1559 C ASP A 198 74.038 57.398 74.991 1.00 24.57 A
ATOM 1560 O ASP A 198 73.503 56.327 74.711 1.00 23.99 A
ATOM 1561 N GLN A 199 73.351 58.519 75.168 1.00 25.75 A
ATOM 1562 CA GLN A 199 71.900 58.546 75.014 1.00 28.42 A
ATOM 1563 CB GLN A 199 71.536 58.348 73.538 1.0029.47 A
ATOM 1564 CG GLN A I99 72.188 59.367 72.610 1.00 32.38 A
ATOM 1565 CD GLN A 199 71.755 60.795 72.905 1.0034.71 A
ATOM 1566 OE1 GLN A 199 72.414 61.755 72.495 1.00 36.67 A
ATOM 1567 NE2 GLN A 199 70.636 60.942 73.607 1.00 33.91 A
ATOM 1568 C GLN A 199 71.184 57.496 75.874 1.00 28.42 A
ATOM 1569 O GLN A 199 70.125 56.991 75.505 1.00 28.49 A
ATOM 1570 N META200 71.773 57.168 77.018 1.0027.48 A
ATOM 1571 CA MET A 200 71.181 56.204 77.936 1.00 26.52 A
ATOM 1572 CB MET A 200 72.097 56.018 79.152 1.00 28.00 A
ATOM 1573 CG META 200 71.442 55.340 80.349 1.00 28.01 A
ATOM 1574 SD META200 70.940 53.635 80.040 1.0028.86 A
ATOM 1575 CE META200 70.315 53.164 81.664 1.00 25.27 A
ATOM 1576 C META200 69.836 56.767 78.382 1.00 25.58 A
ATOM 1577 O META200 69.722 57.969 78.626 1.0026.16 A
ATOM 1578 N LYS A 201 68.821 55.912 78.478 1.0022.73 A
ATOM 1579 CA LYS A 201 67.502 56.367 78.912 1.00 24.68 A
ATOM 1580 CB LYS A 201 66.425 56.036 77.869 1.00 24.23 A
ATOM 1581 CG LYSA201 66.707 56.527 76.459 1.00 23.21 A
ATOM 1582 CD LYS A 201 66.920 58.025 76.414 1.0024.61 A
ATOM 1583 CE LYS A 201 67.262 58.482 75.006 1.0025.99 A
ATOM 1584 NZ LYS A 201 67.687 59.909 74.995 1.00 29.01 A
ATOM 1585 C LYS A 201 67.115 55.711 80.234 1.00 25.86 A
ATOM 1586 O LYS A 201 67.134 54.490 80.359 1.0026.50 A
ATOM 1587 N TRP A 202 66.773 56.526 81.224 1.0027.22 A
ATOM 1588 CA TRP A 202 66.357 55.998 82.515 1.00 28.69 A
ATOM 1589 CB TRP A 202 66.710 56.992 83.621 1.00 26.26 A
ATOM 1590 CG TRP A 202 68.176 57.304 83.613 1.00 26.84 A
ATOM 1591 CD2 TRP A 202 69.234 56.462 84.090 1.00 24.86 A
ATOM 1592 CE2 TRP A 202 70.454 57.120 83.804 1.00 24.99 A
ATOM 1593 CE3 TRP A 202 69.270 55.214 84.731 1.00 24.44 A
ATOM 1594 CD1 TRP A 202 68.779 58.407 83.074 1.00 26.74 A
ATOM 1595 NE1 TRP A 202 70.146 58.303 83.185 1.00 25.04 A
ATOM 1596 CZ2 TRP A 202 71.699 56.572 84.137 1.00 23.44 A
ATOM 1597 CZ3 TRP A 202 70.508 54.669 85.063 1.0024.50 A
ATOM 1598 CH2 TRP A 202 71.705 55.350 84.764 1.0024.56 A
ATOM 1599 C TRPA202 64.854 55.759 82.424 1.00 29.85 A
ATOM 1600 O TRP A 202 64.066 56.700 82.322 1.00 30.81 A
ATOM 1601 N THR A 203 64.472 54.487 82.448 1.00 30.75 A
ATOM 1602 CA THR A 203 63.078 54.097 82.313 1.00 31.93 A
ATOM 1603 CB THR A 203 62.941 53.019 81.219 1.00 31.67 A
ATOM 1604 OGI THR A 203 63.763 51.892 81.550 1.00 32.20 A
ATOM 1605 CG2 THRA203 63.385 53.572 79.873 1.00 31.69 A
ATOM 1606 C THR A 203 62.406 53.592 83.584 1.0032.29 A
ATOM 1607 O THRA203 61.424 52.857 83.522 1.00 32.39 A
ATOM 1608 N ALAA204 62.919 53.995 84.736 1.0034.30 A
ATOM 1609 CA ALAA204 62.344 53.556 86.000 1.00 35.34 A
ATOM 1610 CB ALA A 204 63.176 52.422 86.583 1.00 35.05 A
ATOM 1611 C ALA A 204 62.267 54.708 86.988 1.00 36.60 A
ATOM 1612 O ALA A 204 62.908 54.680 88.039 1.00 38.08 A
ATOM 1613 N PRO A 205 61.478 55.741 86.661 1.0037.60 A
ATOM 1614 CD PRO A 205 60.703 55.893 85.416 1.00 38.45 A
ATOM 1615 CA PRO A 205 61.312 56.917 87.520 1.0037.63 A
ATOM 1616 CB PRO A 205 60.187 57.683 86.829 1.0038.50 A
ATOM 1617 CG PRO A 205 60.428 57.380 85.388 1.0038.29 A
ATOM 1618 C PRO A 205 60.969 56.573 88.967 1.00 37.25 A
ATOM 1619 O PROA205 60.021 55.833 89.233 1.0038.01 A
ATOM 1620 N LEU A 206 61.751 57.107 89.898 1.0036.86 A
ATOM 1621 CA LEU A 206 61.511 56.876 91.317 1.00 36.02 A
ATOM 1622 CB LEU A 206 62.727 56.233 91.988 1.0035.42 A
ATOM 1623 CG LEU A 206 62.979 54.762 91.658 1.00 35.94 A
ATOM 1624 CD1 LEU A 206 64.124 54.234 92.515 1.00 35.41 A
ATOM 1625 CD2 LEU A 206 61.708 53.965 91.924 1.00 35.30 A
ATOM 1626 C LEU A 206 61.194 58.185 92.010 1.00 35.31 A
ATOM 1627 O LEU A 206 61.810 59.213 91.737 1.00 35.11 A
ATOM 1628 N LYS A 207 60.217 58.141 92.902 1.0035.12 A
ATOM 1629 CA LYS A 207 59.821 59.324 93.646 1.00 36.03 A
ATOM 1630 CB LYS A 207 58.490 59.071 94.362 1.00 38.65 A
ATOM 1631 CG LYS A 207 57.312 58.760 93.436 1.0042.37 A
ATOM 1632 CD LYS A 207 57.520 57.502 92.579 I .00 46.24 A
ATOM 1633 CE LYS A 207 57.647 56.219 93.406 1.00 47.07 A
ATOM 1634 NZ LYS A 207 58.908 56.133 94.198 1.0047.92 A
ATOM 1635 C LYS A 207 60.915 59.614 94.671 1.0033.93 A
ATOM 1636 O LYS A 207 61.645 58.711 95.086 1.0032.46 A
ATOM 1637 N PRO A 208 61.072 60.883 95.067 1.00 31.74 A
ATOM 1638 CD PRO A 208 60.422 62.123 94.617 1.00 31.59 A
ATOM 1639 CA PRO A 208 62.115 61.158 96.057 1.00 31.06 A
ATOM 1640 CB PRO A 208 61.948 62.658 96.340 1.00 31.23 A
ATOM 1641 CG PRO A 208 60.554 62.984 95.835 1.00 32.88 A
ATOM 1642 C PROA208 61.889 60.278 97.284 1.0029.49 A
ATOM 1643 O PRO A 208 60.750 60.082 97.709 1.0028.87 A
ATOM 1644 N PHEA209 62.963 59.730 97.842 1.0026.94 A
ATOM 1645 CA PHE A 209 62.812 58.859 98.995 1.00 25.72 A
ATOM 1646 CB PHE A 209 64.159 58.278 99.412 1.00 24.42 A
ATOM 1647 CG PHE A 209 64.034 57.061 100.274 1.00 26.12 A
ATOM 1648 CD1 PHE A 209 64.328 57.118 101.635 1.00 24.80 A
ATOM 1649 CD2 PHEA209 63.578 55.862 99.731 1.00 25.35 A
ATOM 1650 CE1 PHEA209 64.169 55.996 102.447 1.0026.35 A
ATOM 1651 CE2 PHEA209 63.413 54.730 100.534 1.00 27.98 A
ATOM 1652 CZ PHE A 209 63.710 54.797 101.898 1.00 26.46 A
ATOM 1653 C PHE A 209 62.161 59.617 100.144 1.00 24.10 A
ATOM 1654 O PHEA209 62.531 60.747 100.442 1.0025.54 A
ATOM 1655 N SER A 210 61.185 58.985 100.784 1.00 23.65 A
ATOM 1656 CA SER A 210 60.433 59.618 101.864 1.00 22.65 A
ATOM 1657 CB SERA 210 59.424 58.626 102.450 1.00 22.40 A
ATOM 1658 OG SERA210 60.071 57.478 102.968 1.0024.04 A
ATOM 1659 C SER A 210 61.233 60.245 102.995 1.00 21.86 A
ATOM 1660 O SER A 210 61.103 61.436 103.264 1.00 22.10 A
ATOM 1661 N HIS A 211 62.059 59.446 103.656 1.00 21.79 A
ATOM 1662 CA HIS A 211 62.837 59-935 104.785 1.00 21.54 A
ATOM 1663 CB HIS A 211 61.894 60.158 105.973 1.0022.62 A
ATOM 1664 CG HIS A 211 62.560 60.716 107.192 1.00 23.59 A
ATOM 1665 CD2 HIS A 211 63.318 60.122 108.145 1.00 21.09 A
ATOM 1666 ND1 HIS A 211 62.469 62.046 107.547 1.00 23.86 A
ATOM 1667 CE1 HIS A 211 63.140 62.246 108.669 1.00 24.31 A
ATOM 1668 NE2 HIS A 211 63.664 61.094 109.052 1.0025.43 A
ATOM 1669 C HIS A 211 63.886 58.883 105.135 1.00 21.67 A
ATOM 1670 O HIS A 211 63.652 57.684 104.959 1.00 22.26 A
ATOM 1671 N PRO A 212 65.058 59.318 105.631 1.00 20.97 A
ATOM 1672 CD PRO A 212 65.484 60.730 105.737 1.00 20.28 A
ATOM 1673 CA PRO A 212 66.152 58.414 106.008 1.00 19.43 A
ATOM 1674 CB PRO A 212 67.153 59.352 106.683 1.00 17.48 A
ATOM 1675 CG PRO A 212 66.996 60.616 105.887 1.00 18.86 A
ATOM 1676 C PRO A 212 65.777 57.226 106.901 1.00 18.96 A
ATOM 1677 O PRO A 212 66.169 56.095 106.619 1.00 18.95 A
ATOM 1678 N VALA 213 65.026 57.467 107.974 1.00 18.71 A
ATOM 1679 CA VAL A 213 64.659 56.371 108.869 1.00 18.30 A
ATOM 1680 CB VAL A 213 63.980 56.889 110.171 1.00 17.53 A
ATOM 1681 CG1 VAL A 213 64.859 57.944 110.811 1.0015.37 A
ATOM 1682 CG2 VAL A 213 62.584 57.437 109.884 1.00 14.51 A
ATOM 1683 C VAL A 213 63.765 55.335 108.199 1.00 18.95 A
ATOM 1684 O VAL A 213 63.492 54.287 108.779 1.00 20.52 A
ATOM 1685 N ASP A 214 63.321 55.633 106.976 1.0018.98 A
ATOM 1686 CA ASP A 214 62.475 54.725106.195 1.00 19.1 A
ATOM 1687 CB ASP A 214 61.490 55.511 105.316 1.00 19.80 A
ATOM 1688 CG ASP A 214 60.372 56.150 106.114 1.00 23.99 A
ATOM 1689 OD1 ASP A 214 59.654 57.008 105.550 1.00 21.94 A
ATOM 1690 OD2 ASP A 214 60.210 55.789 107.301 1.00 23.90 A
ATOM 1691 C ASP A 214 63.321 53.841 105.282 1.00 19.03 A
ATOM 1692 O ASP A 214 62.783 53.058 104.496 1.00 18.03 A
ATOM 1693 N ALA A 215 64.639 53.972 105.369 1.00 16.92 A
ATOM 1694 CA ALA A 215 65.516 53.170 104.523 1.0017.93 A
ATOM 1695 CB ALA A 215 66.626 54.045 103.937 1.00 16.87 A
ATOM 1696 C ALA A 215 66.138 51.972 105.219 1.00 17.09 A
ATOM 1697 O ALA A 215 66.204 51.907 106.449 1.00 17.40 A
ATOM 1698 N VALA216 66.582 51.017 104.409 1.00 16.32 A
ATOM 1699 CA VAL A 216 67.261 49.826 104.903 1.00 17.96 A
ATOM 1700 CB VAL A 216 66.358 48.568 104.857 1.0018.75 A
ATOM 1701 CG1 VAL A 216 67.115 47.367 105.423 1.00 17.69 A
ATOM 1702 CG2 VAL A 216 65.082 48.805 105.664 1.00 15.66 A
ATOM 1703 C VAL A 216 68.434 49.664 103.944 1.00 19.29 A
ATOM 1704 O VAL A 216 68.256 49.282 102.783 1.00 19.41 A
ATOM 1705 N ILE A 217 69.630 49.979 104.436 1.00 18.72 A
ATOM 1706 CA ILE A 217 70.848 49.929 103.637 1.00 17.96 A
ATOM 1707 CB ILE A 217 71.818 51.042 104.109 1.0018.09 A
ATOM 1708 CG2 ILE A 217 73.049 51.094 103.214 1.00 17.36 A
ATOM 1709 CG1 ILE A 217 71.084 52.389 104.085 1.00 17.89 A
ATOM 1710 CD1 ILE A 217 71.893 53.560 104.631 1.00 15.03 A
ATOM 1711 C ILE A 217 71.548 48.568 103.661 1.00 17.82 A
ATOM 1712 O ILE A 217 71.760 47.975 104.719 1.00 17.13 A
ATOM 1713 N TYR A 218 71.907 48.094 102.472 1.00 16.63 A
ATOM 1714 CA TYR A 218 72.562 46.803 102.286 1.00 17.27 A
ATOM 1715 CB TYR A 218 71.653 45.910 101.429 1.00 16.94 A
ATOM 1716 CG TYR A 218 72.01 44.445 101.423 1.04 17.86 A
ATOM 1717 CD1 TYR A 218 72.457 43.822 100.259 1.00 20.32 A
ATOM 1718 CE1 TYR A 218 72.783 42.465 100.243 1.00 20.21 A
ATOM 1719 CD2 TYR A 218 71.906 43.674 102.580 1.0021.56 A
ATOM 1720 CE2 TYR A 218 8 72.233 42.317 102.577 1.00 20.13 A
ATOM 1721 CZ TYR A 218 72.670 41.723 101.406 1.00 19.83 A
ATOM 1722 OH TYR A 218 73.012 40.39 101.400 1.00 19.80 A
ATOM 1723 C TYR A 218 73.910 47.037 101.589 1.00 17.29 A
ATOM 1724 O TYR A 218 73.961 47.353 t00.402 1.00 16.01 A
ATOM 1725 N GLU A 219 74.998 46.888 102.338 1.00 7.22 A
ATOM 1726 CA GLU A 219 76.333 47.123 101.804 1.00 18.13 A
ATOM 1727 CB GLU A 219 77.270 47.568 102.921 1.00 17.85 A
ATOM 1728 CG GLU A 219 78.537 48.193 102.406 1.00 20.16 A
ATOM 1729 CD GLU A 219 79.593 48.343 103.476 1.0021.66 A
ATOM 1730 OE1 GLU A 219 80.422 49.271 103.354 1.00 21.78 A
ATOM 1731 OE2 GLU A 219 79.600 47.529 104.425 1.00 19.88 A
ATOM 1732 C GLU A 219 76.945 45.919 101.101 1.00 19.63 A
ATOM 1733 O GLU A 219 77.159 44.869 101.709 1.00 17.79 A
ATOM 1734 N THR A 220 77.259 46.089 99.822 1.00 19.21 A
ATOM 1735 CA THR A 220 77.833 45.003 99.050 1.00 20.32 A
ATOM 1736 CB THR A 220 76.736 44.273 98.257 1.00 21.65 A
ATOM 1737 OG1 THR A 220 77.333 43.276 97.423 1.00 27.21 A
ATOM 1738 CG2 THR A 220 75.975 45.247 97.390 1.00 20.12 A
ATOM 1739 C THRA220 78.923 45.444 98.077 1.00 19.67 A
ATOM 1740 O THRA220 78.934 46.581 97.612 1.00 19.94 A
ATOM 1741 N HIS A 221 79.835 44.522 97.785 1.00 19.05 A
ATOM 1742 CA HIS A 221 80.944 44.740 96.863 1.00 18.21 A
ATOM 1743 CB HIS A 221 82.226 44.128 97.445 1.0018.55 A
ATOM 1744 CG HIS A 221 83.441 44.315 96.590 1.0017.09 A
ATOM 1745 CD2 HIS A 221 83.989 43.524 95.638 1.00 17.27 A
ATOM 1746 ND1 HIS A 221 84.264 45.416 96.691 1.00 18.40 A
ATOM 1747 CE1 HIS A 221 85.269 45.293 95.843 1.00 16.84 A
ATOM 1748 NE2 HIS A 221 85.126 44.154 95.191 1.00 16.76 A
ATOM 1749 C HIS A 221 80.542 44.008 95.582 1.00 18.08 A
ATOM 1750 O HIS A 221 80.094 42.861 95.640 1.00 18.51 A
ATOM 1751 N LEU A 222 80.703 44.651 94.428 1.00 18.08 A
ATOM 1752 CA LEU A 222 80.301 44.017 93.165 1.00 17.84 A
ATOM 1753 CB LEU A 222 80.506 44.979 91.993 1.00 15.73 A
ATOM 1754 CG LEU A 222 79.530 46.156 91.957 1.00 15.97 A
ATOM 1755 CD1 LEU222 79.810 47.031 90.743 1.00 14.64 A
ATOM 1756 CD2 LEU A 222 78.104 45.624 91.910 1.00 17.01 A
ATOM 1757 C LEU A 222 80.964 42.674 92.854 1.00 17.86 A
ATOM 1758 O LEU A 222 80.376 41.836 92.176 1.00 18.31 A
ATOM 1759 N ARG A 223 82.182 42.465 93.336 1.0018.01 A
ATOM 1760 CA ARG A 223 82.858 41.201 93.091 1.00 20.83 A
ATOM 1761 CB ARG A 223 84.368 41.376 93.243 1.00 24.09 A
ATOM 1762 CG ARG A 223 85.197 40.130 92.973 1.00 25.00 A
ATOM 1763 CD ARG A 223 86.581 40.569 92.540 1.0027.92 A
ATOM 1764 NE ARG A 223 87.563 39.493 92.489 1.0028.20 A
ATOM 1765 CZ ARGA223 88.764 39.628 91.933 1.00 31.43 A
ATOM 1766 NH1 ARG223 89.618 38.613 91.927 1.00 30.25 A
ATOM 1767 NH2 ARG A 223 89.102 40.783 91.361 1.00 29.89 A
ATOM 1768 C ARG A 223 82.341 40.159 94.075 1.00 20.13 A
ATOM 1769 O ARG A 223 81.905 39.079 93.676 1.00 21.55 A
ATOM 1770 N ASP A 224 82.380 40.501 95.358 1.00 19.26 A
ATOM 1771 CA ASP A 224 81.910 39.610 96.421 1.00 19.46 A
ATOM 1772 CB ASP A 224 81.843 40.345 97.769 1.00 18.40 A
ATOM 1773 CG ASP A 224 83.189 40.862 98.244 1.00 20.98 A
ATOM 1774 OD1 ASP A 224 83.240 41.401 99.370 1.00 20.03 A
ATOM 1775 OD2 ASP A 224 84.191 40.740 97.511 1.00 22.49 A
ATOM 1776 C ASPA224 80.513 39.064 96.141 1.00 19.37 A
ATOM 1777 O ASP A 224 80.287 37.863 96.184 1.00 20.77 A
ATOM 1778 N PHEA225 79.582 39.972 95.864 1.00 20.78 A
ATOM 1779 CA PHE A 225 78.179 39.645 95.630 1.00 19.99 A
ATOM 1780 CB PHE A 225 77.423 40.883 95.135 1.00 18.80 A
ATOM 1781 CG PHE A 225 75.938 40.686 95.067 1.00 16.59 A
ATOM 1782 CD1 PHE A 225 75.173 40.683 96.230 1.00 18.40 A
ATOM 1783 CD2 PHE A 225 75.309 40.443 93.853 1.00 17.69 A
ATOM 1784 CE1 PHEA225 73.798 40.437 96.185 1.00 18.52 A
ATOM 1785 CE2 PHE A 225 73.928 40.193 93.791 1.00 17.40 A
ATOM 1786 CZ PHE A 225 73.174 40.189 94.959 1.00 18.41 A
ATOM 1787 C PHEA225 77.812 38.488 94.712 1.00 21.05 A
ATOM 1788 O PHE A 225 76.817 37.805 94.955 1.0020.49 A
ATOM 1789 N SERA226 78.586 38.255 93.659 1.00 22.68 A
ATOM 1790 CA SERA 226 78.220 37.187 92.731 1.00 22.86 A
ATOM 1791 CB SERA 226 77.497 37.794 91.535 1.00 23.82 A
ATOM 1792 OG SERA 226 78.381 38.648 90.826 1.00 25.73 A
ATOM 1793 C SERA226 79.363 36.331 92.212 1.00 22.20 A
ATOM 1794 O SER A 226 79.163 35.516 91.313 1.00 20.10 A
ATOM 1795 N ILE A 227 80.553 36.510 92.767 1.00 23.78 A
ATOM 1796 CA ILE A 227 81.709 35.752 92.312 1.00 24.15 A
ATOM 1797 CB ILE A 227 83.017 36.355 92.885 1.0024.72 A
ATOM 1798 CG2 ILE A 227 83.066 36.167 94.387 1.00 23.34 A
ATOM 1799 CG1 ILE A 227 84.233 35.725 92.201 1.00 23.21 A
ATOM 1800 CD1 ILE A 227 84.430 36.199 90.774 1.00 24.71 A
ATOM 1801 C ILE A 227 81.631 34.266 92.677 1.00 25.44 A
ATOM 1802 O ILE A 227 82.232 33.429 92.005 1.00 26.10 A
ATOM 1803 N HIS A 228 80.884 33.940 93.730 1.00 25.11 A
ATOM 1804 CA HIS A 228 80.756 32.554 94.177 1.0027.23 A
ATOM 1805 CB HIS A 228 79.898 32.495 95.442 1.0027.59 A
ATOM 1806 CG HIS A 228 79.919 31.167 96.135 1.0030.55 A
ATOM 1807 CD2 HIS A 228 80.668 30.714 97.169 1.00 30.61 A
ATOM 1808 ND1 HIS A 228 79.069 30.135 95.798 1.00 30.50 A
ATOM 1809 CE1 HIS A228 79.290 29.106 96.598 1.00 30.49 A
ATOM 1810 NE2 HIS A 228 80.255 29.432 97.439 1.00 30.60 A
ATOM 1811 C HIS A 228 80.170 31.649 93.096 1.00 27.24 A
ATOM 1812 O HIS A 228 79.202 32.003 92.422 1.00 26.82 A
ATOM 1813 N GLU A 229 80.777 30.478 92.941 1.00 27.98 A
ATOM 1814 CA GLU A 229 80.362 29.496 91.941 1.00 30.05 A
ATOM 1815 CB GLU A 229 81.165 28.203 92.127 1.0032.31 A
ATOM 1816 CG GLU A 229 81.078 27.644 93.540 1.0039.08 A
ATOM 1817 CD GLU A 229 81.878 26.369 93.729 1.0043.13 A
ATOM 1818 OE1 GLU A 229 83.112 26.397 93.521 1.00 45.12 A
ATOM 1819 OE2 GLU A 229 81.268 25.338 94.092 1.00 45.42 A
ATOM 1820 C GLU A 229 78.874 29.167 91.976 1.00 28.68 A
ATOM 1821 O GLU A 229 78.256 28.940 90.938 1.00 26.85 A
ATOM 1822 N ASNA230 78.305 29.152 93.175 1.00 27.73 A
ATOM 1823 CA ASN A 230 76.896 28.823 93.359 1.00 28.38 A
ATOM 1824 CB ASN A 230 76.735 28.022 94.660 1.00 30.26 A
ATOM 1825 CG ASN A 230 75.383 27.339 94.774 1.00 33.68 A
ATOM 1826 OD1 ASN A 230 74.959 26.954 95.870 1.00 34.33 A
ATOM 1827 ND2 ASN A 230 74.704 27.169 93.642 1.00 32.24 A
ATOM 1828 C ASN A 230 76.004 30.065 93.411 1.00 27.07 A
ATOM 1829 O ASN A 230 74.853 29.982 93.848 1.00 24.22 A
ATOM 1830 N SER A 231 76.517 31.206 92.955 1.00 26.42 A
ATOM 1831 CA SER A 231 75.739 32.448 92.998 1.0026.26 A
ATOM 1832 CB SER A 231 76.607 33.648 92.606 1.00 26.12 A
ATOM 1833 OG SER A 231 76.763 33.736 91.203 1.0025.17 A
ATOM 1834 C SER A 231 74.484 32.435 92.131 z6 A
ATOM 1835 O SER A 231 73.528 33.152 92.416 1.00 27.74 A
ATOM 1836 N GLY A 232 74.484 31.629 91.074 1.00 26.83 A
ATOM 1837 CA GLYA232 73.317 31.557 90.210 1.00 25.14 A
ATOM 1838 C GLY A 232 73.254 32.662 89.173 1.00 25.85 A
ATOM 1839 O GLY A 232 72.276 32.772 88.423 1.00 24.98 A
ATOM 1840 N META233 74.298 33.484 89.130 1.0025.11 A
ATOM 1841 CA META233 74.372 34.584 88.176 1.0024.72 A
ATOM 1842 CB META 233 74.659 35.903 88.906 1.0024.75 A
ATOM 1843 CG MET A 233 73.529 36.359 89.819 1.0024.62 A
ATOM 1844 SD META233 73.975 37.765 90.853 1.0024.80 A
ATOM 1845 CE META233 74.413 36.925 92.398 1.00 23.05 A
ATOM 1846 C META233 75.468 34.301 87.158 1.00 25.16 A
ATOM 1847 O MET A 233 76.533 33.789 87.506 1.0023.38 A
ATOM 1848 N ILE A 234 75.195 34.641 85.902 1.0026.36 A
ATOM 1849 CA ILE A 234 76.135 34.428 84.804 1.0028.11 A
ATOM 1850 CB ILE A 234 75.427 34.612 83.437 1.00 30.02 A
ATOM 1851 CG2 ILE A 234 76.421 34.415 82.304 1.00 29.69 A
ATOM 1852 CG1 ILE A 234 74.267 33.622 83.309 1.00 33.37 A
ATOM 1853 CD1 LE A 234 73.425 33.828 82.056 1.00 34.27 A
ATOM 1854 C ILE A 234 77.348 35.368 84.834 1.00 26.51 A
ATOM 1855 O ILE A 234 78.491 34.927 84.699 1.00 24.14 A
ATOM 1856 N ASN A 235 77.079 36.659 85.003 1.00 25.02 A
ATOM 1857 CA ASN A 235 78.109 37.698 85.020 1.00 24.21 A
ATOM 1858 CB ASN A 235 77.475 39.027 84.600 1.00 24.24 A
ATOM 1859 CG ASN A 235 76.876 38.966 83.203 1.00 27.02 A
ATOM 1860 OD ASN A 235 77.598 39.016 82.207 1.00 28.64 A
ATOM 1861 ND2 ASN A 235 75.552 38.839 83.124 1.00 25.04 A
ATOM 1862 C ASNA235 78.763 37.835 86.388 1.00 21.86 A
ATOM 1863 O ASN A 235 78.674 38.878 87.031 1.00 22.03 A
ATOM 1864 N LYSA236 79.433 36.774 86.818 1.00 23.11 A
ATOM 1865 CA LYS A 236 80.088 36.742 88.120 1.0021.99 A
ATOM 1866 CB LYSA236 80.735 35.371 88.332 1.0022.75 A
ATOM 1867 CG LYS A 236 79.718 34.236 88.409 1.0022.17 A
ATOM 1868 CD LYS A 236 80.389 32.892 88.633 1.0024.66 A
ATOM 1869 CE LYSA236 79.419 31.889 89.256 1.00 25.20 A
ATOM 1870 NZ LYS A 236 78.180 31.700 88.456 1.00 23.49 A
ATOM 1871 C LYSA236 81.116 37.842 88.325 1.00 22.28 A
ATOM 1872 O LYSA236 82.058 37.978 87.546 1.0021.78 A
ATOM 1873 N GLYA237 80.920 38.629 89.381 1.0022.83 A
ATOM 1874 CA GLY A 237 81.837 39.711 89.695 1.00 20.63 A
ATOM 1875 C GLYA237 81.794 40.898 88.750 1.00 21.48 A
ATOM 1876 O GLY A 237 82.740 41.686 88.709 1.00 21.25 A
ATOM 1877 N LYS A 238 80.702 41.041 88.002 1.0020.90 A
ATOM 1878 CA LYS A 238 80.566 42.141 87.046 1.00 21.54 A
ATOM 1879 CB LYS A 238 80.525 41.590 85.620 1.0021.67 A
ATOM 1880 CG LYS A 238 81.676 40.665 85.249 1.0024.87 A
ATOM 1881 CD LYS A 238 83.020 41.384 85.252 1.0026.67 A
ATOM 1882 CE LYS A 238 84.096 40.535 84.584 1.0025.93 A
ATOM 1883 NZ LYS A 238 85.433 41.201 84.599 1.00 29.04 A
ATOM 1884 C LYS A 238 79.307 42.976 87.287 1.0020.50 A
ATOM 1885 O LYSA238 78.374 42.527 87.953 1.00 20.26 A
ATOM 1886 N TYR A 239 79.290 44.186 86.730 1.00 18.50 A
ATOM 1887 CA TYRA239 78.152 45.102 86.861 1.00 19.02 A
ATOM 1888 CB TYRA239 78.316 46.315 85.938 1.00 18.11 A
ATOM 1889 CG TYRA 239 79.442 47.250 86.284 1.00 19.04 A
ATOM 1890 CD1 TYR A 239 80.466 47.502 85.370 1.00 16.36 A
ATOM 1891 CE1 TYR A 239 81.482 48.407 85.665 1.00 16.26 A
ATOM 1892 CD2 TYRA239 79.463 47.922 87.507 1.00 15.46 A
ATOM 1893 CE2 TYR A 239 80.472 48.823 87.811 1.00 16.30 A
ATOM 1894 CZ TYRA239 81.476 49.062 86.889 1.00 16.01 A
ATOM 1895 OH TYRA239 82.470 49.955 87.196 1.00 17.37 A
ATOM 1896 C TYRA239 76.825 44.452 86.498 1.00 19.54 A
ATOM 1897 O TYRA239 75.824 44.642 87.183 1.0019.24 A
ATOM 1898 N LEU A 240 76.821 43.698 85.402 1.00 20.91 A
ATOM 1899 CA LEU A 240 75.604 43.055 84.916 1.00 22.34 A
ATOM 1900 CB LEU A 240 75.840 42.499 83.508 1.00 23.20 A
ATOM 1901 CG LEU A 240 76.016 43.557 82.413 1.0024.02 A
ATOM 1902 CD1 LEU A240 76.164 42.862 81.064 1.00 24.42 A
ATOM 1903 CD2 LEU A 240 74.816 44.502 82.400 1.00 22.52 A
ATOM 1904 C LEU A 240 74.965 41.971 85.783 1.00 22.09 A
ATOM 1905 O LEU A 240 73.792 41.650 85.589 1.0021.97 A
ATOM 1906 N ALA A 241 75.710 41.407 86.730 1.00 19.66 A
ATOM 1907 CA ALA A 241 75.141 40.361 87.580 1.00 20.89 A
ATOM 1908 CB ALA A 241 76.143 39.949 88.665 1.00 18.63 A
ATOM 1909 C ALAA241 73.828 40.822 88.226 1.0021.61 A
ATOM 1910 O ALA A 241 72.823 40.105 88. 191 1.00 20.00 A
ATOM 1911 N LEU A 242 73.846 42.024 88.805 1.00 20.97 A
ATOM 1912 CA LEU A 242 72.674 42.579 89.470 1.0022.75 A
ATOM 1913 CB LEU A 242 73.035 43.871 90.219 1.0023.99 A
ATOM 1914 CG LEU A 242 73.290 43.803 91.732 1.0026.64 A
ATOM 1915 CD1 LEU A 242 72.189 42.984 92.399 1.00 24.98 A
ATOM 1916 CD2 LEU A 242 74.640 43.175 92.012 1.00 30.1 A
ATOM 1917 C LEU A 242 71.485 42.856 88.549 1.00 23.91 A
ATOM 1918 O LEU A 242 70.423 43.249 89.033 1.00 23.89 A
ATOM 1919 N THR A 243 71.655 42.674 87.237 1.00 21.95 A
ATOM 1920 CA THR A 243 70.550 42.898 86.305 1.00 22.98 A
ATOM 1921 CB THR A 243 71.020 43.438 84.925 1.00 21.96 A
ATOM 1922 OG1 THRA 243 71.772 42.430 84.245 1.00 21.56 A
ATOM 1923 CG2 THRA243 71.877 44.691 85.088 1.00 20.95 A
ATOM 1924 C THR A 243 69.797 41.593 86.051 1.00 23.79 A
ATOM 1925 O THRA243 68.676 41.611 85.548 1.00 24.44 A
ATOM 1926 N GLU A 244 70.426 40.471 86.400 1.00 24.04 A
ATOM 1927 CA GLU A 244 69.842 39.145 86.202 1.00 24.78 A
ATOM 1928 CB GLU A 244 70.924 38.072 86.365 1.0025.04 A
ATOM 1929 CG GLU A 244 71.984 38.128 85.268 1.0024.59 A
ATOM 1930 CD GLU A 244 73.132 37.162 85.490 1.0024.13 A
ATOM 1931 OE1 GLU A 244 72.873 35.962 85.699 1.00 23.90 A
ATOM 1932 OE2 GLU A 244 74.300 37.604 85.446 1.00 24.05 A
ATOM 1933 C GLU A 244 68.681 38.871 87.149 1.00 26.56 A
ATOM 1934 O GLU A 244 68.840 38.843 88.370 1.00 27.25 A
ATOM 1935 N THR A 245 67.509 38.656 86.566 1.00 27.67 A
ATOM 1936 CA THR A 245 66.293 38.407 87.328 1.00 28.31 A
ATOM 1937 CB THR A 245 65.079 38.958 86.574 1.00 28.72 A
ATOM 1938 OG1 THR A 245 64.996 38.328 85.289 1.00 28.15 A
ATOM 1939 CG2 THR A 245 65.213 40.459 86.379 1.00 26.55 A
ATOM 1940 C THRA245 66.026 36.938 87.656 1.00 28.64 A
ATOM 1941 O THR A 245 66.452 36.034 86.936 1.00 28.54 A
ATOM 1942 N ASP A 246 65.322 36.724 88.765 1.00 28.38 A
ATOM 1943 CA ASP A 246 64.937 35.395 89.233 1.00 29.35 A
ATOM 1944 CB ASP A 246 63.806 34.852 88.350 1.00 30.08 A
ATOM 1945 CG ASP A 246 62.552 35.708 88.416 1.00 33.50 A
ATOM 1946 OD1 ASPA 246 61.860 35.824 87.384 1..00 38.11 A
ATOM 1947 OD2 ASP A 246 62.249 36.262 89.495 1.00 33.51 A
ATOM 1948 C ASP A 246 66.046 34.352 89.326 1.00 28.71 A
ATOM 1949 O ASP A 246 65.802 33.174 89.078 1.00 29.10 A
ATOM 1950 N THRA247 67.256 34.765 89.687 1.00 27.06 A
ATOM 1951 CA THRA247 68.353 33.807 89.805 1.0025.75 A
ATOM 1952 CB THR A 247 69.733 34.497 89.728 1.00 24.96 A
ATOM 1953 OG1 THRA247 69.852 35.448 90.791 1.00 25.87 A
ATOM 1954 CG2 THR A 247 69.900 35.203 88.386 1.00 24.03 A
ATOM 1955 C THRA247 68.242 33.066 91.132 1.00 25.95 A
ATOM 1956 O THR A 247 67.603 33.546 92.067 1.00 25.56 A
ATOM 1957 N GLN A 248 68.863 31.895 91.214 1.00 26.15 A
ATOM 1958 CA GLN A 248 68.799 31.101 92.435 1.00 28.77 A
ATOM 1959 CB GLN A 248 67.525 30.248 92.435 1.00 29.31 A
ATOM 1960 CG GLN A 248 67.573 29.087 91.458 1.00 32.55 A
ATOM 1961 CD GLN A 248 66.216 28.439 91.228 1.00 35.58 A
ATOM 1962 OE1 GLN A 248 66.131 27.346 90.671 1.00 37.30 A
ATOM 1963 NE2 GLN A 248 65.150 29.116 91.646 1.00 36.58 A
ATOM 1964 C GLN A 248 70.016 30.196 92.552 1.00 28.59 A
ATOM 1965 O GLN A 248 70.704 29.932 91.564 1.00 28.68 A
ATOM 1966 N THRA249 70.277 29.719 93.763 1.00 28.39 A
ATOM 1967 CA THR A 249 71.409 28.835 93.999 1.00 29.01 A
ATOM 1968 CB THR A 249 71.695 28.686 95.505 1.00 29.40 A
ATOM 1969 OG1 THR A 249 70.584 28.044 96.140 1.00 27.86 A
ATOM 1970 CG2 THR A 249 71.904 30.054 96.145 1.00 29.55 A
ATOM 1971 C THRA249 71.083 27.463 93.422 1.00 30.39 A
ATOM 1972 O THR A 249 69.977 27.233 92.930 1.0029.84 A
ATOM 1973 N ALA A 250 72.049 26.555 93.483 1.00 32.26 A
ATOM 1974 CA ALA A 250 71.860 25.206 92.966 1.0034.44 A
ATOM 1975 CB ALAA250 73.123 24.387 93.180 1.00 34.57 A
ATOM 1976 C ALA A 250 70.676 24.524 93.647 1.0036.60 A
ATOM 1977 O ALA A 250 69.837 23.915 92.979 1.0036.33 A
ATOM 1978 N ASN A 251 70.604 24.637 94.973 1.0037.76 A
ATOM 1979 CA ASN A 251 69.522 24.009 95.727 1.00 38.05 A
ATOM 1980 CB ASN A 251 69.866 23.959 97.230 1.00 39.97 A
ATOM 1981 CG ASN A 251 69.229 25.095 98.031 1.00 41.85 A
ATOM 1982 OD1 ASN A 251 69.441 26.274 97.749 1.00 42.07 A
ATOM 1983 ND2 ASN A 251 68.447 24.734 99.044 1.00 43.29 A
ATOM 1984 C ASN A 251 68.174 24.696 95.507 1.00 37.27 A
ATOM 1985 O ASN A 251 67.146 24.220 95.994 1.0037.86 A
ATOM 1986 N GLYA252 68.174 25.812 94.779 1.0034.29 A
ATOM 1987 CA GLYA252 66.921 26.501 94.509 1.0032.01 A
ATOM 1988 C GLY A 252 66.601 27.747 95.317 1.0030.79 A
ATOM 1989 O GLYA252 65.564 28.373 95.096 1.0030.00 A
ATOM 1990 N SERA253 67.470 28.116 96.253 1.00 29.77 A
ATOM 1991 CA SERA253 67.234 29.314 97.059 1.00 28.99 A
ATOM 1992 CB SER A 253 68.186 29.350 98.250 1.00 26.63 A
ATOM 1993 OG SERA253 67.891 28.302 99.147 1.0027.73 A
ATOM 1994 C SER A 253 67.430 30.567 96.209 1.0028.50 A
ATOM 1995 O SERA 253 68.225 30.567 95.273 1.0027.48 A
ATOM 1996 N SERA254 66.702 31.631 96.533 1.00 27.17 A
ATOM 1997 CA SER A 254 66.820 32.861 95.772 1.0026.83 A
ATOM 1998 CB SERA254 65.762 33.870 96.225 1.00 27.59 A
ATOM 1999 OG SER A 254 65.888 34.162 97.603 1.00 31.34 A
ATOM 2000 C SER A 254 68.217 33.450 95.938 1.00 25.88 A
ATOM 2001 O SERA254 68.857 33.264 96.975 1.00 24.99 A
ATOM 2002 N SERA255 68.685 34.143 94.902 1.00 23.35 A
ATOM 2003 CA SERA 255 70.004 34.775 94.914 1.0023.42 A
ATOM 2004 CB SERA 255 71.052 33.845 94.301 1.00 24.62 A
ATOM 2005 OG SERA 255 70.888 33.757 92.891 1.00 24.62 A
ATOM 2006 C SER A 255 69.966 36.074 94.109 1.0023.01 A
ATOM 2007 O SER A 255 68.929 36.444 93.559 1.00 23.68 A
ATOM 2008 N GLY A 256 71.101 36.761 94.049 1.0022.07 A
ATOM 2009 CA GLY A 256 71.190 38.002 93.296 1.00 20.64 A
ATOM 2010 C GLY A 256 70.084 39.022 93.503 1.0020.69 A
ATOM 2011 O GLY A 256 69.576 39.195 94.613 1.0022.01 A
ATOM 2012 N LEU A 257 69.715 39.699 92.417 1.00 19.92 A
ATOM 2013 CA LEU A 257 68.679 40.727 92.443 1.00 20.25 A
ATOM 2014 CB LEU A 257 68.341 41.179 91.019 1.00 21.56 A
ATOM 2015 CG LEU A 257 67.165 42.148 90.861 1.0022.11 A
ATOM 2016 CD1 LEU A 257 67.477 43.449 91.578 1.00 24.98 A
ATOM 2017 CD2 LEU A 257 66.909 42.412 89.382 1.00 23.34 A
ATOM 2018 C LEU A 257 67.408 40.274 93.139 1.00 18.71 A
ATOM 2019 O LEUA257 66.888 40.975 94.003 1.00 18.05 A
ATOM 2020 N ALA A 258 66.909 39.102 92.762 1.00 19.70 A
ATOM 2021 CA ALA A 258 65.688 38.562 93.355 1.00 19.49 A
ATOM 2022 CB ALA A 258 65.381 37.195 92.753 1.00 17.58 A
ATOM 2023 C ALAA258 65.835 38.448 94.876 1.00 21.45 A
ATOM 2024 O ALA A 258 64.899 38.741 95.628 1.00 21.66 A
ATOM 2025 N TYRA259 67.018 38.028 95.319 1.0020.91 A
ATOM 2026 CA TYRA259 67.308 37.867 96.740 1.00 20.03 A
ATOM 2027 CB TYR A 259 68.703 37.261 96.909 1.0019.43 A
ATOM 2028 CG TYR A 259 69.166 37.117 98.348 1.00 20.12 A
ATOM 2029 CD1 TYRA259 68.718 36.062 99.149 1.00 18.22 A
ATOM 2030 CE1 TYRA259 69.151 35.925 100.474 1.00 17.92 A
ATOM 2031 CD2 TYR A 259 70.059 38.036 98.906 1.00 18.19 A
ATOM 2032 CE2 TYR A 259 70.494 37.910 100.226 1.00 17.51 A
ATOM 2033 CZ TYRA259 70.036 36.854 101.001 1.00 17.14 A
ATOM 2034 OH TYRA259 70.462 36.734 102.298 1.00 14.77 A
ATOM 2035 C TYR A 259 67.235 39.216 97.461 1.0021.16 A
ATOM 2036 O TYRA 259 66.462 39.393 98.402 1.0020.73 A
ATOM 2037 N VAL A 260 68.043 40.167 97.006 1.0021.02 A
ATOM 2038 CA VAL A 260 68.076 41.492 97.605 1.0020.09 A
ATOM 2039 CB VAL A 260 69.042 42.415 96.826 1.0020.19 A
ATOM 2040 CG1 VAL A 260 69.030 43.817 97.417 1.00 20.24 A
ATOM 2041 CG2 VAL A 260 70.452 41.842 96.890 1.00 20.17 A
ATOM 2042 C VAL A 260 66.683 42.120 97.671 1.00 19.55 A
ATOM 2043 O VALA260 66.343 42.786 98.649 1.00 17.96 A
ATOM 2044 N LYS A 261 65.878 41.902 96.635 1.00 19.83 A
ATOM 2045 CA LYS A 261 64.522 42.440 96.601 1.00 22.87 A
ATOM 2046 CB LYS A 261 63.902 42.221 95.220 1.00 24.40 A
ATOM 2047 CG LYS A 261 62.445 42.649 95.125 1.00 27.56 A
ATOM 2048 CD LYS A 261 62.298 44.143 95.380 1.0029.42 A
ATOM 2049 CE LYS A 261 60.842 44.575 95.355 1.00 28.82 A
ATOM 2050 NZ LYS A 261 60.713 46.046 95.502 1.0029.87 A
ATOM 2051 C LYS A 261 63.658 41.756 97.663 1.00 23.80 A
ATOM 2052 O LYS A 261 62.849 42.394 98.345 1.0023.69 A
ATOM 2053 N GLU A 262 63.862 40.451 97.795 1.00 23.32 A
ATOM 2054 CA GLU A 262 63.145 39.606 98.741 1.00 24.80 A
ATOM 2055 CB GLU A 262 63.542 38.153 98.467 1.0027.89 A
ATOM 2056 CG GLU A 262 62.801 37.082 99.228 1.0032.42 A
ATOM 2057 CD GLU A 262 63.085 35.702 98.653 1.0034.72 A
ATOM 2058 OE1 GLU A 262 62.553 35.385 97.567 1.00 37.19 A
ATOM 2059 OE2 GLU A 262 63.855 34.943 99.272 1.00 36.10 A
ATOM 2060 C GLU A 262 63.448 39.993 100.194 1.0024.65 A
ATOM 2061 O GLU A 262 62.545 40.006 101.042 1.00 22.28 A
ATOM 2062 N LEU A 263 64.710 40.311 100.478 1.00 23.40 A
ATOM 2063 CA LEU A 263 65.112 40.702 101.829 1.00 24.61 A
ATOM 2064 CB LEU A 263 66.601 41.049 101.879 1.00 27.40 A
ATOM 2065 CG LEU A 263 67.582 39.941 102.244 1.00 29.31 A
ATOM 2066 CD1 LEU A 263 68.990 40.518 102.291 1.00 29.07 A
ATOM 2067 CD2 LEU A 263 67.204 39.347 103.595 1.00 31.49 A
ATOM 2068 C LEU A 263 64.317 41.906 102.316 1.00 23.01 A
ATOM 2069 O LEU A 263 64.067 42.049 103.507 1.00 22.56 A
ATOM 2070 N GLY A 264 63.930 42.772 101.386 1.00 22.61 A
ATOM 2071 CA GLY A 264 63.163 43.94 101.746 1.00 21.77 A
ATOM 2072 C GLY A 264 64.009 45.202 101.881 1.00 23.47 A
ATOM 2073 O GLY A 264 63.517 46.234 102.344 1.00 25.19 A
ATOM 2074 N VAL A 265 65.276 45.130 101.481 1.00 21.70 A
ATOM 2075 CA VAL A 265 66.151 46.296 101.570 1.00 20.78 A
ATOM 2076 CB VAL A 265 67.622 45.921 101.275 1.00 21.35 A
ATOM 2077 CG1 VAL A 265 68.017 44.695 102.095 1.00 24.04 A
ATOM 2078 CG2 VAL A 265 67.813 45.664 99.813 1.00 22.35 A
ATOM 2079 C VAL A 265 65.680 47.359100.580 1.00 18.55 A
ATOM 2080 O VAL A 265 65.093 47.036 99.549 1.00 17.60 A
ATOM 2081 N THR A 266 65.923 48.626 100.904 1.00 17.78 A
ATOM 2082 CA THR A 266 65.506 49.736 100.048 1.00 16.87 A
ATOM 2083 CB THRA266 64.862 50.865 100.877 1.00 16.48 A
ATOM 2084 OG1 THR A 266 65.849 51.453 101.736 1.00 16.65 A
ATOM 2085 CG2 THRA266 63.723 50.316 101.732 1.00 12.87 A
ATOM 2086 C THRA266 66.669 50.341 99.258 1.00 18.98 A
ATOM 2087 O THR A 266 66.486 50.820 98.132 1.00 18.37 A
ATOM 2088 N HIS A 267 67.859 50.320 99.855 1.00 17.65 A
ATOM 2089 CA HIS A 267 69.046 50.880 99.225 1.00 16.69 A
ATOM 2090 CB HIS A 267 69.520 52.138 99.964 1.00 17.57 A
ATOM 2091 CG HIS A 267 68.643 53.338 99.777 1.00 18.23 A
ATOM 2092 CD2 HIS A 267 68.878 54.526 99.167 1.00 16.69 A
ATOM 2093 ND1 HIS A 267 67.364 53.417 100.288 1.00 16.31 A
ATOM 2094 CE1 HIS A 267 66.852 54.602 100.003 1.00 16.71 A
ATOM 2095 NE2 HIS A 267 67.750 55.294 99.324 1.00 17.20 A
ATOM 2096 C HIS A 267 70.206 49.906 99.213 1.00 18.15 A
ATOM 2097 O HIS A 267 70.546 49.320 100.246 1.00 17.07 A
ATOM 2098 N VAL A 268 70.815 49.747 98.042 1.0018.17 A
ATOM 2099 CA VAL A 268 71.985 48.896 97.900 1.00 18.64 A
ATOM 2100 CB VAL A 268 71.967 48.088 96.591 1.00 19.08 A
ATOM 2101 CG1 VAL A 268 73.247 47.266 96.483 1.00 19.92 A
ATOM 2102 CG2 VALA 268 70.757 47.172 96.552 1.00 20.74 A
ATOM 2103 C VALA268 73.194 49.838 97.853 1.00 19.48 A
ATOM 2104 O VAL A 268 73.323 50.643 96.928 1.00 19.04 A
ATOM 2105 N GLU A 269 74.059 49.756 98.859 1.00 17.86 A
ATOM 2106 CA GLU A 269 75.252 50.593 98.896 1.00 16.31 A
ATOM 2107 CB GLU A 269 75.607 50.973 100.336 1.00 17.15 A
ATOM 2108 CG GLU A 269 76.874 51.811 100.473 1.00 16.77 A
ATOM 2109 CD GLU A 269 77.127 52.227 101.912 1.00 20.07 A
ATOM 2110 OE1 GLU A 269 76.184 52.758 102.539 1.00 17.96 A
ATOM 2111 OE2 GLU A 269 78.259 52.030 102.414 1.00 19.71 A
ATOM 2112 C GLU A 269 76.387 49.792 98.279 1.00 15.99 A
ATOM 2113 O GLU A 269 76.667 48.667 98.696 1.00 13.57 A
ATOM 2114 N LEU A 270 77.047 50.381 97.293 1.00 15.31 A
ATOM 2115 CA LEU A 270 78.126 49.696 96.599 1.00 16.82 A
ATOM 2116 CB LEU A 270 77.983 49.895 95.089 1.00 17.5 A
ATOM 2117 CG LEU A 270 76.658 49.588 94.394 1.0018.96 A
ATOM 2118 CD1 LEU A 270 76.752 50.039 92.936 1.00 18.54 A
ATOM 2119 CD2 LEU A 270 76.357 48.103 94.484 1.00 17.71 A
ATOM 2120 C LEU A 270 79.502 50.191 96.999 1.00 17.08 A
ATOM 2121 O LEUA270 79.722 51.397 97.140 1.00 17.94 A
ATOM 2122 N LEU A 271 80.435 49.269 97.187 1.00 15.02 A
ATOM 2123 CA LEU A 271 81.783 49.701 97.485 1.00 15.43 A
ATOM 2124 CB LEU A 271 82.670 48.500 97.842 1.00 14.08 A
ATOM 2125 CG LEU A 271 82.718 48.356 99.379 1.00 15.49 A
ATOM 2126 CD1 LEU A 271 81.389 47.824 99.897 1.00 14.26 A
ATOM 2127 CD2 LEU A 271 83.847 47.436 99.790 1.00 17.1 A
ATOM 2128 C LEU A 271 82.215 50.441 96.197 1.00 14.39 A
ATOM 2129 O LEU A 271 81.610 50.261 95.142 1.00 3.52 A
ATOM 2130 N PRO A 272 83.247 51.293 96.276 1.00 14.96 A
ATOM 2131 CD PRO A 272 84.194 51.313 97.408 1.00 12.46 A
ATOM 2132 CA PRO A 272 83.767 52.094 95.156 1.00 14.85 A
ATOM 2133 CB PROA272 85.202 52.379 95.583 1.00 16.45 A
ATOM 2134 CG PROA272 85.065 52.502 97.078 1.00 15.36 A
ATOM 2135 C PROA272 83.681 51.585 93_715 1.00 14.96 A
ATOM 2136 O PROA272 84.305 50.580 93.358 1.00 16.12 A
ATOM 2137 N VAL A 273 82.913 52.296 92.887 1.0015.03 A
ATOM 2138 CA VAL A 273 82.774 51.948 91.470 1.00 14.60 A
ATOM 2139 CB VALA 273 81.287 51.823 91.022 1.00 16.92 A
ATOM 2140 CG1 VAL A 273 80.649 50.613 91.692 1.00 16.10 A
ATOM 2141 CG2 VAL A 273 80.511 53.103 91.346 1.00 14.74 A
ATOM 2142 C VALA273 83.486 52.944 90.549 1.00 15.43 A
ATOM 2143 O VAL A 273 83.447 52.797 89.327 1.0015.51 A
ATOM 2144 N ASNA274 84.120 53.970 91.120 1.00 16.24 A
ATOM 2145 CA ASN A 274 84.880 54.909 90.291 1.00 15.78 A
ATOM 2146 CB ASN A 274 85.203 56.199 91.051 1.00 4.77 A
ATOM 2147 CG ASN A 274 86.163 55.976 92.204 1.00 14.94 A
ATOM 2148 OD1 ASNA274 87.383 56.057 92.042 1.0018.13 A
ATOM 2149 ND2 ASN A 274 85.618 55.681 93.371 1.00 9.38 A
ATOM 2150 C ASN A 274 86.166 54.131 89.997 1.00 16.63 A
ATOM 2151 O ASN A 274 86.472 53.161 90.698 1.00 15.03 A
ATOM 2152 N ASPA275 86.918 54.529 88.980 1.00 16.01 A
ATOM 2153 CA ASP A 275 88.124 53.782 88.658 1.00 16.95 A
ATOM 2154 CB ASP A 275 88.775 54.287 87.369 1.0015.24 A
ATOM 2155 CG ASP A 275 89.813 53.319 86.843 1.0015.44 A
ATOM 2156 OD1 ASP A 275 90.719 53.727 86.083 1.00 16.72 A
ATOM 2157 OD2 ASP A 275 89.711 52.129 87.194 1.00 14.78 A
ATOM 2158 C ASP A 275 89.144 53.839 89.780 1.0016.81 A
ATOM 2159 O ASPA275 89.475 54.918 90.270 1.00 8.03 A
ATOM 2160 N PHEA276 89.644 52.670 90.172 1.00 15.04 A
ATOM 2161 CA PHEA276 90.635 52.579 91.236 1.00 16.57 A
ATOM 2162 CB PHE A 276 89.983 52.090 92.536 1.00 17.08 A
ATOM 2163 CG PHE A 276 89.229 50.799 92.393 1.00 16.32 A
ATOM 2164 CD1 PHE A 276 87.838 50.798 92.319 1.00 17.14 A
ATOM 2165 CD2 PHE A 276 89.906 49.581 92.341 1.00 15.61 A
ATOM 2166 CE1 PHE A 276 87.127 49.602 92.199 1.00 17.46 A
ATOM 2167 CE2 PHE A 276 89.208 48.378 92.220 1.00 16.32 A
ATOM 2168 CZ PHEA276 87.815 48.387 92.150 1.00 16.92 A
ATOM 2169 C PHE A 276 91.781 51.641 90.870 1.00 16.56 A
ATOM 2170 O PHEA276 91.689 50.873 89.913 1.0015.47 A
ATOM 2171 N ALAA277 92.862 51.714 91.641 1.0017.20 A
ATOM 2172 CA ALA A 277 94.028 50.862 91.425 1.00 17.74 A
ATOM 2173 CB ALA A 277 95.311 51.639 91.712 1.0018.04 A
ATOM 2174 C ALAA 277 93.927 49.672 92.365 1.0018.25 A
ATOM 2175 O ALAA277 93.272 49.761 93.405 1.0019.69 A
ATOM 2176 N GLY A 278 94.569 48.562 92.006 1.00 17.95 A
ATOM 2177 CA GLY A 278 94.531 47.395 92.869 1.00 19.44 A
ATOM 2178 C GLY A 278 94.175 46.097 92.174 1.00 20.96 A
ATOM 2179 O GLY A 278 94.508 45.014 92.660 1.0022.61 A
ATOM 2180 N VAL A 279 93.488 46.198 91.043 1.00 19.57 A
ATOM 2181 CA VAL A 279 93.106 45.019 90.285 1.00 19.11 A
ATOM 2182 CB VAL A 279 91.589 44.758 90.389 1.00 17.32 A
ATOM 2183 CG1 VALA279 91.200 43.585 89.516 1.00 14.14 A
ATOM 2184 CG2 VAL A 279 91.212 44.491 91.836 1.00 16.27 A
ATOM 2185 C VALA279 93.479 45.242 88.826 1.00 21.60 A
ATOM 2186 O VAL A 279 93.042 46.217 88.211 1.00 20.77 A
ATOM 2187 N ASP A 280 94.310 44.357 88.283 1.00 21.93 A
ATOM 2188 CA ASP A 280 94.719 44.474 86.886 1.00 21.82 A
ATOM 2189 CB ASP A 280 95.855 43.505 86.568 1.00 22.69 A
ATOM 2190 CG ASP A 280 96.347 43.640 85.136 1.0024.51 A
ATOM 2191 OD1 ASP A 280 95.581 44.148 84.291 1.00 27.20 A
ATOM 2192 OD2 ASP A 280 97.490 43.227 84.850 1.00 26.29 A
ATOM 2193 C ASPA280 93.505 44.121 86.039 1.0022.73 A
ATOM 2194 O ASP A 280 93.092 42.960 85.985 1.00 22.04 A
ATOM 2195 N GLU A 281 92.934 45.120 85.376 1.00 21.46 A
ATOM 2196 CA GLU A 281 91.752 44.891 84.560 1.00 22.81 A
ATOM 2197 CB GLU A 281 91.21 46.237 84.056 1.00 21.43 A
ATOM 2198 CG GLU A 281 91.129 47.285 85.184 1.0020.01 A
ATOM 2199 CD GLU A 281 90.436 48.586 84.783 1.00 21.97 A
ATOM 2200 OE1 GLUA281 90.325 48.876 83.571 1.00 21.39 A
ATOM 2201 OE2 GLU A 281 90.011 49.331 85.690 1.00 19.87 A
ATOM 2202 C GLU A 281 92.028 43.917 83.411 1.0023.99 A
ATOM 2203 O GLU A 281 91.102 43.375 82.817 1.0025.09 A
ATOM 2204 N GLU A 282 93.302 43.683 83.111 1.00 25.46 A
ATOM 2205 CA GLU A 282 93.673 42.737 82.058 1.00 29.07 A
ATOM 2206 CB GLU A 282 95.097 43.011 81.556 1.00 29.96 A

ATOM 2207 CG GLU A 282 95.290 44.315 80.790 1.00 30.25 A
ATOM 2208 CD GLU A 282 94.700 44.277 79.393 1.00 30.58 A
ATOM 2209 OE1 GLU A 282 94.917 45.242 78.632 1.00 30.36 A
ATOM 2210 OE2 GLU A 282 94.021 43.286 79.052 1.00 33.43 A
ATOM 2211 C GLU A 282 93.604 41.304 82.606 1.00 30.32 A
ATOM 2212 O GLU A 282 93.294 40.368 81.872 1.0030.22 A
ATOM 2213 N LYS A 283 93.900 41.147 83.898 1.00 31.08 A
ATOM 2214 CA LYS A 283 93.877 39.843 84.576 1.0030.69 A
ATOM 2215 CB LYS A 283 95.307 39.338 84.810 1.00 33.15 A
ATOM 2216 CG LYS A 283 96.260 39.448 83.614 1.0038.05 A
ATOM 2217 CD LYS A 283 96.204 38.244 82.665 1.00 40.85 A
ATOM 2218 CE LYS A 283 94.935 38.201 81.829 1.0042.09 A
ATOM 2219 NZ LYS A 283 95.008 37.170 80.753 1.0042.96 A
ATOM 2220 C LYS A 283 93.193 40.039 85.935 1.00 29.68 A
ATOM 2221 O LYS A 283 93.833 39.927 86.981 1.00 28.23 A
ATOM 2222 N PRO A 284 91.880 40.322 85.930 1.00 28.59 A
ATOM 2223 CD PRO A 284 91.033 40.308 84.725 1.0028.17 A
ATOM 2224 CA PRO A 284 91.066 40.553 87.131 1.00 29.92 A
ATOM 2225 CB PRO A 284 89.670 40.806 86.556 1.00 29.01 A
ATOM 2226 CG PRO A 284 89.671 40.002 85.296 1.0028.54 A
ATOM 2227 C PROA284 91.065 39.489 88.231 1.00 30.08 A
ATOM 2228 O PROA284 91.007 39.827 89.410 1.0031.48 A
ATOM 2229 N LEUA285 91.132 38.215 87.858 1.0030.66 A
ATOM 2230 CA LEU A 285 91.127 37.132 88.846 1.00 30.29 A
ATOM 2231 CB LEU A 285 90.869 35.788 88.159 1.00 31.63 A
ATOM 2232 CG LEU A 285 89.540 35.599 87.424 1.0034.84 A
ATOM 2233 CD1 LEU A 285 89.647 34.406 86.485 1.0035.86 A
ATOM 2234 CD2 LEU A 285 88.408 35.413 88.425 1.00 34.75 A
ATOM 2235 C LEU A 285 92.430 37.027 89.638 1.00 29.58 A
ATOM 2236 O LEU A 285 92.476 36.360 90.673 1.00 29.21 A
ATOM 2237 N ASP A 286 93.481 37.688 89.158 1.0028.04 A
ATOM 2238 CA ASP A 286 94.787 37.625 89.811 1.00 27.89 A
ATOM 2239 CB ASP A 286 95.880 38.048 88.829 1.0028.32 A
ATOM 2240 CG ASP A 286 96.138 37.004 87.756 1.0027.72 A
ATOM 2241 OD1 ASP A 286 95.284 36.114 87.569 1.0027.30 A
ATOM 2242 OD2 ASP A 286 97.193 37.083 87.088 1.00 29.86 A
ATOM 2243 C ASP A 286 94.929 38.429 91.095 1.00 28.20 A
ATOM 2244 O ASP A 286 95.883 38.232 91.850 1.00 29.10 A
ATOM 2245 N ALAA287 93.998 39.340 91.347 1.0025.87 A
ATOM 2246 CA ALA A 287 94.073 40.141 92.558 1.0025.44 A
ATOM 2247 CB ALAA287 95.024 41.315 92.355 1.0022.49 A
ATOM 2248 C ALAA287 92.696 40.642 92.949 1.0026.42 A
ATOM 2249 O ALAA287 91.765 40.619 92.142 1.0026.72 A
ATOM 2250 N TYR A 288 92.578 41.092 94.194 1.0024.21 A
ATOM 2251 CA TYRA 288 91.318 41.606 94.707 1.0023.68 A
ATOM 2252 CB TYRA 288 90.644 40.561 95.617 1.0022.09 A
ATOM 2253 CG TYRA 288 89.425 41.080 96.365 1.0020.84 A
ATOM 2254 CD TYR A 288 89.559 41.975 97.429 1.00 18.76 A
ATOM 2255 CE1 TYR A 288 88.446 42.480 98.094 1.00 21.45 A
ATOM 2256 CD2 TYR A 288 88.137 40.700 95.988 1.00 20.65 A
ATOM 2257 CE2 TYR A 288 87.013 41.201 96.648 1.00 20.50 A
ATOM 2258 CZ TYR A 288 87.176 42.091 97.700 1.00 19.95 A
ATOM 2259 OH TYR A 288 86.075 42.593 98.357 1.00 16.48 A
ATOM 2260 C TYR A 288 91.543 42.899 95.482 1.00 23.04 A
ATOM 2261 O TYRA288 92.606 43.114 96.065 1.00 22.48 A
ATOM 2262 N ASN A 289 90.532 43.761 95.488 1.0022.85 A
ATOM 2263 CA ASN A 289 90.636 45.009 96.219 1.00 22.52 A
ATOM 2264 CB ASN A 289 91.576 45.974 95.491 1.00 22.22 A
ATOM 2265 CG ASN A 289 92.036 47.111 96.381 1.00 23.93 A
ATOM 2266 OD1 ASN A 289 91.264 48.014 96.708 1.00 24.00 A
ATOM 2267 ND2 ASN A 289 93.298 47.063 96.794 1.00 24.25 A
ATOM 2268 C ASN A 289 89.278 45.659 96.410 1.00 20.87 A
ATOM 2269 O ASNA289 88.444 45.653 95.501 1.00 20.27 A
ATOM 2270 N TRPA290 89.055 46.211 97.601 1.00 18.77 A
ATOM 2271 CA TRP A 290 87.799 46.890 97.898 1.00 18.09 A
ATOM 2272 CB TRP A 290 87.785 47.414 99.334 1.00 16.67 A
ATOM 2273 CG TRP A 290 87.505 46.367 100.372 1.00 18.16 A
ATOM 2274 CD2 TRP A 290 87.814 46.445 101.772 1.00 16.83 A
ATOM 2275 CE2 TRP A 290 87.307 45.273 102.379 1.00 16.36 A
ATOM 2276 CE3 TRP A 290 88.469 47.392 102.572 1.00 16.97 A
ATOM 2277 CD1 TRP A 290 86.841 45.181 100.192 1.00 18.04 A
ATOM 2278 NE 1 TRP A 290 86.719 44.521 101.395 1.00 17.81 A
ATOM 2279 CZ2 TRP A 290 87.435 45.022 103.751 1.00 16.71 A
ATOM 2280 CZ3 TRP A 290 88.597 47.141 103.942 1.00 16.18 A
ATOM 2281 CH2 TRP A 290 88.082 45.965 104.513 1.00 15.42 A
ATOM 2282 C TRP A 290 87.590 48.063 96.944 1.00 16.52 A
ATOM 2283 O TRPA290 86.459 48.374 96.583 1.00 19.11 A
ATOM 2284 N GLY A 291 88.684 48.711 96.550 1.00 14.44 A
ATOM 2285 CA GLY A 291 88.596 49.836 95.633 1.00 15.05 A
ATOM 2286 C GLY A 291 88.882 51.215 96.216 1.0015.21 A
ATOM 2287 O GLY A 291 88.659 52.226 95.552 1.00 15.72 A
ATOM 2288 N TYR A 292 89.391 51.268 97.443 1.00 15.10 A
ATOM 2289 CA TYRA292 89.683 52.548 98.087 1.00 16.58 A
ATOM 2290 CB TYRA292 89.617 52.382 99.608 1.00 16.83 A
ATOM 2291 CG TYRA292 88.225 52.055 100.102 1.00 19.17 A
ATOM 2292 CD1 TYR A 292 87.218 53.026 100.110 1.00 18.14 A
ATOM 2293 CE1 THR A 292 85.924 52.716 100.523 1.00 18.71 A
ATOM 2294 CD2 TYR A 292 87.901 50.766 100.520 1.0017.58 A
ATOM 2295 CE2 TYR A 292 86.613 50.445 100.929 1.00 18.47 A
ATOM 2296 CZ TYR A 292 85.629 51.423 100.929 1.00 19.09 A
ATOM 2297 OH TYR A 292 84.349 51.096 101.318 1.00 18.49 A
ATOM 2298 C TYRA292 91.022 53.161 97.679 1.00 16.62 A
ATOM 2299 O TYRA292 91.739 53.718 98.515 1.00 16.73 A
ATOM 2300 N ASN A 293 91.344 53.057 96.391 1.00 16.61 A
ATOM 2301 CA ASN A 293 92.589 53.595 95.825 1.00 17.78 A
ATOM 2302 CB ASN A 293 93.533 52.454 95.414 1.00 18.30 A
ATOM 2303 CG ASN A 293 93.952 51.582 96.591 1.0019.90 A
ATOM 2304 OD1 ASN A 293 94.493 52.073 97.577 1.00 19.37 A
ATOM 2305 ND2 ASN A 293 93.703 50.279 96.485 1.00 20.37 A
ATOM 2306 C ASNA293 92.161 54.375 94.585 1.00 18.75 A
ATOM 2307 O ASN A 293 92.373 53.929 93.455 1.00 18.72 A
ATOM 2308 N PRO A 294 91.561 55.560 94.786 1.0017.15 A
ATOM 2309 CD PRO A 294 91.425 56.232 96.088 1.00 18.21 A
ATOM 2310 CA PRO A 294 91.070 56.427 93.717 1.00 16.86 A
ATOM 2311 CB PRO A 294 90.522 57.639 94.473 1.00 16.64 A
ATOM 2312 CG PROA294 90.238 57.111 95.850 1.0017.91 A
ATOM 2313 C PROA294 92.076 56.840 92.651 1.00 17.63 A
ATOM 2314 O PRO A 294 93.146 57.371 92.964 1.00 16.29 A
ATOM 2315 N LEU A 295 91.715 56.591 91.393 1.00 16.07 A
ATOM 2316 CA LEU A 295 92.539 56.984 90.252 1.00 17.83 A
ATOM 2317 CB LEU A 295 92.835 55.793 89.332 1.00 17.67 A
ATOM 2318 CG LEU A 295 93.878 54.797 89.837 1.0019.46 A
ATOM 2319 CD1 LEU A 295 94.168 53.763 88.753 1.00 18.19 A
ATOM 2320 CD2 LEU A 295 95.148 55.551 90.221 1.00 16.60 A
ATOM 2321 C LEU A 295 91.795 58.045 89.453 1.00 16.67 A
ATOM 2322 O LEUA295 92.317 59.128 89.201 1.00 17.72 A
ATOM 2323 N HIS A 296 90.565 57.724 89.065 1.00 17.67 A
ATOM 2324 CA HIS A 296 89.739 58.631 88.276 1.0017.75 A
ATOM 2325 CB HIS A 296 89.665 58.109 86.850 1.00 16.53 A
ATOM 2326 CG HIS A 296 91.009 57.851 86.249 1.00 18.50 A
ATOM 2327 CD2 HIS A 296 91.579 56.711 85.791 1.0018.73 A
ATOM 2328 ND1 HISA296 91.955 58.843 86.093 1.00 18.08 A
ATOM 2329 CE1 HIS A 296 93.049 58.324 85.565 1.00 19.16 A
ATOM 2330 NE2 HIS A 296 92.848 57.032 85.373 1.0019.41 A
ATOM 2331 C HISA296 88.350 58.740 88.885 1.00 17.90 A
ATOM 2332 O HISA296 87.554 57.802 88.822 1.00 15.86 A
ATOM 2333 N PHE A 297 88.058 59.903 89.455 1.0018.21 A
ATOM 2334 CA PHEA297 86.783 60.119 90.129 1.00 17.58 A
ATOM 2335 CB PHE A 297 86.876 61.355 91.031 1.00 16.60 A
ATOM 2336 CG PHE A 297 87.778 61.168 92.225 1.0016.29 A
ATOM 2337 CD1 PHEA297 89.090 61.635 92.208 1.00 15.77 A
ATOM 2338 CD2 PHEA297 87.315 60.518 93.366 1.00 16.50 A
ATOM 2339 CE1PHEA297 89.923 61.458 93.317 1.0014.93 A
ATOM 2340 CE2 PHE A 297 88.142 60.333 94.480 1.00 14.91 A
ATOM 2341 CZ PHE A 297 89.444 60.805 94.454 1.0013.72 A
ATOM 2342 C PHEA297 85.538 60.223 89.263 1.00 17.17 A
ATOM 2343 O PHEA297 84.435 60.010 89.758 1.0017.00 A
ATOM 2344 N PHEA298 85.702 60.547 87.984 1.0016.70 A
ATOM 2345 CA PHE A 298 84.548 60.684 87.099 1.0016.21 A
ATOM 2346 CB PHE A 298 84.658 61.960 86.253 1.0014.34 A
ATOM 2347 CG PHE A 298 84.370 63.227 87.017 1.00 14.51 A
ATOM 2348 CD1 PHE A 298 85.296 63.741 87.915 1.0012.47 A
ATOM 2349 CD2 PHE A 298 83.157 63.901 86.844 1.0013.58 A
ATOM 2350 CE1 PHEA298 85.023 64.913 88.636 1.0015.67 A
ATOM 2351 CE2 PHEA298 82.873 65.073 87.560 1.0014.64 A
ATOM 2352 CZ PHE A 298 83.809 65.577 88.457 1.0014.18 A
ATOM 2353 C PHEA298 84.314 59.503 86.173 1.0016.19 A
ATOM 2354 O PHEA298 83.405 59.544 85.343 1.0017.07 A
ATOM 2355 N ALAA299 85.118 58.454 86.297 1.0015.17 A
ATOM 2356 CA ALAA299 84.937 57.293 85.428 1.0015.44 A
ATOM 2357 CB ALA A 299 86.126 57.143 84.487 1.0012.16 A
ATOM 2358 C ALAA299 84.751 56.025 86.244 1.0015.99 A
ATOM 2359 O ALAA299 85.190 55.951 87.388 1.0013.75 A
ATOM 2360 N PRO A 300 84.089 55.009 85.659 1.00 17.56 A
ATOM 2361 CD PRO A 300 83.307 55.125 84.416 1.00 17.61 A
ATOM 2362 CA PRO A 300 83.827 53.719 86.312 1.00 16.52 A
ATOM 2363 CB PRO A 300 82.709 53.111 85.461 1.0016.65 A
ATOM 2364 CG PRO A 300 82.088 54.309 84.751 1.00 17.78 A
ATOM 2365 C PRO A 300 85.044 52.793 86.355 1.00 17.68 A
ATOM 2366 O PRO A 300 85.925 52.870 85.499 1.00 16.38 A
ATOM 2367 N GLUA301 85.078 51.923 87.362 1.00 17.44 A
ATOM 2368 CA GLUA301 86.151 50.945 87.513 1.0018.81 A
ATOM 2369 CB GLUA301 85.997 50.198 88.845 1.0019.47 A
ATOM 2370 CG GLUA301 86.628 48.814 88.867 1.0018.46 A
ATOM 2371 CD GLUA301 88.142 48.837 88.733 1.0019.07 A
ATOM 2372 OE1 GLUA301 88.724 47.759 88.496 1.0017.38 A
ATOM 2373 OE2GLUA301 88.748 49.919 88.871 1.0016.16 A
ATOM 2374 C GLUA301 86.007 49.966 86.357 1.00 18.50 A
ATOM 2375 O GLUA301 84.88949.601 85.995 1.00 18.19 A
ATOM 2376 N GLYA302 87.129 49.523 85.795 1.0019.27 A
ATOM 2377 CA GLY A 302 87.073 48.604 84.666 1.00 19.53 A
ATOM 2378 C GLY A 302 86.987 47.102 84.921 1.0020.49 A
ATOM 2379 O GLY A 302 86.523 46.361 84.049 1.0020.33 A
ATOM 2380 N SERA 303 87.417 46.630 86.088 1.0018.74 A
ATOM 2381 CA SERA 303 87.370 45.193 86.358 1.0019.24 A
ATOM 2382 CB SERA303 88.186 44.845 87.611 1.0018.05 A
ATOM 2383 OG SERA303 87.576 45.357 88.782 1.00 20.17 A
ATOM 2384 C SERA303 85.951 44.635 86.505 1.0019.81 A
ATOM 2385 O SERA 303 85.751 43.421 86.428 1.00 18.42 A
ATOM 2386 N TYRA304 84.96845.511 86.712 1.00 19.84 A
ATOM 2387 CA TYR A 304 83.586 45.064 86.856 1.0018.50 A
ATOM 2388 CB TYRA 304 82.816 45.983 87.815 1.0018.03 A
ATOM 2389 CG TYRA 304 83.414 46.077 89.207 1.0016.57 A
ATOM 2390 CD1TYRA304 83.947 44.954 89.835 1.00 17.57 A
ATOM 2391 CE1 TYRA304 84.501 45.031 91.118 1.0017.09 A
ATOM 2392 CD2 TYR A 304 83.445 47.290 89.895 1.0015.94 A
ATOM 2393 CE2 TYR A 304 83.99647.381 91.180 1.0016.27 A
ATOM 2394 CZ TYRA304 84.522 46.247 91.781 1.0017.38 A
ATOM 2395 OH TYR A 304 85.078 46.322 93.031 1.0015.52 A
ATOM 2396 C TYR A 304 82.852 44.999 85.516 1.00 18.48 A
ATOM 2397 O TYRA304 81.691 44.584 85.456 1.00 19.37 A
ATOM 2398 N ALAA305 83.520 45.412 84.444 1.0018.93 A
ATOM 2399 CA ALA A 305 82.903 45.393 83.114 1.00 17.86 A
ATOM 2400 CB ALAA 305 83.359 46.605 82.309 1.0016.15 A
ATOM 2401 C ALA A 305 83.224 44.118 82.339 1.0018.27 A
ATOM 2402 O ALAA305 84.251 43.477 82.562 1.0017.14 A
ATOM 2403 N SERA 306 82.333 43.749 81.426 1.00 20.86 A
ATOM 2404 CA SERA 306 82.549 42.569 80.601 1.00 21.06 A
ATOM 2405 CB SERA 306 81.343 42.339 79.691 1.0020.64 A
ATOM 2406 OG SER A 306 81.162 43.429 78.803 1.0019.48 A
ATOM 2407 C SERA 306 83.800 42.805 79.748 1.0022.56 A
ATOM 2408 O SERA 306 84.586 41.887 79.501 1.0023.18 A
ATOM 2409 N ASN A 307 83.975 44.045 79.302 1.0020.98 A
ATOM 2410 CA ASN A 307 85.126 44.412 78.482 1.00 20.50 A
ATOM 2411 CB ASN A 307 84.708 44.523 77.014 1.0019.73 A
ATOM 2412 CG ASN A 307 85.858 44.930 76.095 1.0020.71 A
ATOM 2413 OD1 ASN307 85.670 45.075 74.887 1.00 23.96 A
ATOM 2414 ND2 ASN A 307 87.044 45.116 76.659 1.00 17.56 A
ATOM 2415 C ASN A 307 85.69945.745 78.953 1.00 18.98 A
ATOM 2416 O ASN A 307 85.123 46.799 78.691 1.0017.13 A
ATOM 2417 N PRO A 308 86.848 45.710 79.650 1.0019.45 A
ATOM 2418 CD PRO A 308 87.586 44.499 80.059 1.00 19.99 A
ATOM 2419 CA PRO A 308 87.506 46.918 80.163 1.00 19.91 A
ATOM 2420 CB PRO A 308 88.443 46.366 81.234 1.00 18.81 A
ATOM 2421 CG PRO A 308 88.882 45.070 80.638 1.0019.60 A
ATOM 2422 C PRO A 308 88.262 47.696 79.090 1.00 20.59 A
ATOM 2423 O PRO A 308 88.709 48.820 79.328 1.00 21.11 A
ATOM 2424 N HIS A 309 88.398 47.089 77.914 1.0021.20 A
ATOM 2425 CA HIS A 309 89.111 47.699 76.794 1.00 21.68 A
ATOM 2426 CB HIS A 309 89.553 46.618 75.805 1.0020.60 A
ATOM 2427 CG HIS A 309 90.410 45.563 76.420 1.0021.89 A
ATOM 2428 CD2 HIS A 309 90.129 44.292 76.793 1.00 22.89 A
ATOM 2429 ND1 HIS A 309 91.723 45.786 76.774 1.00 22.78 A
ATOM 2430 CE1 HISA309 92.213 44.698 77.341 1.0023.32 A
ATOM 2431 NE2 HISA309 91.266 43.777 77.366 1.0023.49 A
ATOM 2432 C HIS A 309 88.285 48.736 76.050 1.00 21.63 A
ATOM 2433 O HIS A 309 88.828 49.729 75.562 1.00 22.96 A
ATOM 2434 N ASPA310 86.982 48.489 75.939 1.0021.27 A
ATOM 2435 CAASPA310 86.091 49.414 75.245 1.0020.98 A
ATOM 2436 CBASPA310 84.896 48.670 74.647 1.0019.96 A
ATOM 2437CGASPA310 83.921 49.604 73.958 1.0021.71 A
ATOM 2438 OD1ASPA310 82.760 49.701 74.409 1.00 23.51 A
ATOM 2439OD2ASPA310 84.316 50.252 72.971 1.0020.19 A
ATOM 2440 C ASP310 85.615 50.460 76.244 1.00 19.94 A
ATOM 2441 O ASPA310 84.806 50.170 77.129 1.0021.63 A
ATOM 2442 N PROA311 86.119 1.696 76.112 1.00 18.10 A
ATOM 2443 CD PROA311 87.029 52.140 75.041 1.0016.95 A
ATOM 2444CAPROA311 85.778 52.817 76.991 1.0017.95 A
ATOM 2445 CB PROA311 86.291 54.023 76.210 1.0018.55 A
ATOM 2446 CG PROA311 87.519 53.479 75.564 1.00 17.22 A
ATOM 2447 C PROA311 84.306 52.947 77.371 1.00 17.66 A
ATOM 2448 O PROA311 83.978 53.133 78.542 1.0016.87 A
ATOM 2449 N GLNA312 83.420 52.836 76.389 1.0017.54 A
ATOM 2450 CA GLNA312 81.991 52.982 76.653 1.0018.20 A
ATOM 2451 CB GLN A 312 81.240 53.218 75.340 1.0016.59 A
ATOM 2452 CG GLN A 312 79.725 53.168 75.478 1.0017.14 A
ATOM 2453 CD GLN A 312 79.172 54.290 76.338 1.0020.68 A
ATOM 2454 OE1 GLNA312 78.253 54.078 77.129 1.00 22.39 A
ATOM 2455 NE2 GLNA312 79.720 55.495 76.181 1.00 15.85 A
ATOM 2456 C GLN A 312 81.321 51.829 77.405 1.00 17.68 A
ATOM 2457 O GLNA312 80.401 52.060 78.189 1.00 16.90 A
ATOM 2458 N THRA313 81.780 50.602 77.170 1.00 17.22 A
ATOM 2459 CA THRA313 81.179 49.422 77.792 1.00 18.53 A
ATOM 2460 CB THR A 313 81.989 48.141 77.448 1.00 18.73 A
ATOM 2461 OG1 THRA313 81.919 47.908 76.036 1.00 20.11 A
ATOM 2462 CG2 THR A 313 81.424 46.917 78.172 1.00 17.03 A
ATOM 2463 C THRA313 80.940 49.487 79.305 1.00 19.08 A
ATOM 2464 O THRA313 79.830 49.207 79.762 1.0020.64 A
ATOM 2465 N ARG A 314 81.953 49.854 80.087 1.00 18.30 A
ATOM 2466 CA ARG A 314 81.763 49.918 81.533 1.00 18.60 A
ATOM 2467 CB ARG A 314 83.085 50.243 82.244 1.0018.73 A
ATOM 2468 CG ARG A 314 83.668 51.602 81.935 1.0019.17 A
ATOM 2469 CD ARG A 314 85.024 51.783 82.616 1.0019.96 A
ATOM 2470 NE ARG A 314 86.104 51.024 81.984 1.0018.29 A
ATOM 2471 CZ ARGA314 87.368 51.047 82.404 1.0020.58 A
ATOM 2472 NH1 ARGA314 87.700 51.786 83.451 1.00 18.30 A
ATOM 2473 NH2 ARG A 314 88.305 50.342 81.778 1.0019.13 A
ATOM 2474 C ARGA314 80.679 50.924 81.929 1.00 18.37 A
ATOM 2475 O ARG A 314 79.955 50.716 82.909 1.0017.30 A
ATOM 2476 N LYSA315 80.561 52.003 81.162 1.0017.51 A
ATOM 2477 CA LYSA315 79.562 53.033 81.440 1.00 18.00 A
ATOM 2478 CB LYSA315 79.794 54.256 80.548 1.0016.82 A
ATOM 2479 CG LYSA315 81.140 54.936 80.771 1.0017.96 A
ATOM 2480 CD LYSA315 81.339 56.126 79.839 1.00 20.11 A
ATOM 2481 CE LYSA315 80.235 57.167 80.008 1.0023.38 A
ATOM 2482 NZ LYSA315 80.492 58.370 79.167 1.0028.14 A
ATOM 2483 C LYSA315 78.165 52.483 81.188 1.0018.93 A
ATOM 2484 O LYSA315 77.250 52.664 81.997 1.0019.25 A
ATOM 2485 N THR A 316 78.018 51.805 80.056 1.0019.00 A
ATOM 2486 CA THRA316 76.751 51.21479.663 1.0017.52 A
ATOM 2487 CB THRA316 76.886 50.486 78.302 1.00 17.40 A
ATOM 2488 OG1 THRA316 77.231 51.433 77.284 1.00 16.31 A
ATOM 2489CG2THRA316 75.582 49.804 77.921 1.00 17.10 A
ATOM 2490 C THRA316 76.260 50.222 80.711 1.00 16.96 A
ATOM 2491 O THR A 316 75.115 50.289 81.145 1.00 18.75 A
ATOM 2492 N GLUA317 77.139 49.324 81.135 1.00 16.42 A
ATOM 2493 CA GLUA317 76.773 48.292 82.098 1.00 19.37 A
ATOM 2494 CB GLU A 317 77.861 47.214 82.125 1.00 19.14 A
ATOM 2495 CG GLUA317 78.190 46.700 80.724 1.0020.50 A
ATOM 2496 CD GLU A 317 79.067 45.462 80.723 1.0021.16 A
ATOM 2497 OE1 GLUA317 79.907 45.310 81.631 1.00 21.91 A
ATOM 2498 OE2 GLUA317 78.927 44.645 79.796 1.0024.35 A
ATOM 2499 C GLUA317 76.458 48.786 83.512 1.00 19.03 A
ATOM 2500 O GLUA317 75.525 48.290 84.150 1.0019.32 A
ATOM 2501 N LEUA318 77.218 49.755 84.007 1.0017.68 A
ATOM 2502 CA LEU A 318 76.944 50.284 85.336 1.00 18.49 A
ATOM 2503 CB LEU A 318 78.029 51.277 85.759 1.00 17.16 A
ATOM 2504 CG LEU A 318 77.847 52.007 87.100 1.0017.30 A
ATOM 2505 CD1 LEUA318 77.589 51.014 88.240 1.00 13.50 A
ATOM 2506 CD2 LEUA318 79.099 52.833 87.378 1.0013.10 A
ATOM 2507 C LEU A 318 75.579 50.979 85.305 1.00 20.12 A
ATOM 2508 O LEU A 318 74.782 50.845 86.240 1.0019.50 A
ATOM 2509 N LYSA319 75.307 51.702 84.219 1.0020.10 A
ATOM 2510 CA LYSA319 74.036 52.411 84.071 1.0022.12 A
ATOM 2511 CB LYSA319 74.069 53.319 82.842 1.00 21.69 A
ATOM 2512 CG LYS A 319 75.055 54.476 82.973 1.00 22.14 A
ATOM 2513 CD LYSA319 74.983 55.421 81.779 1.0023.75 A
ATOM 2514 CE LYSA319 75.989 56.566 81.915 1.0024.20 A
ATOM 2515 NZ LYS A 319 75.879 57.537 80.790 1.00 22.14 A
ATOM 2516 C LYSA319 72.870 51.436 83.973 1.00 22.51 A
ATOM 2517 O LYSA319 71.786 51.696 84.501 1.0020.35 A
ATOM 2518 N GLNA320 73.097 50.313 83.300 1.0023.62 A
ATOM 2519 CA GLN A 320 72.062 49.297 83.161 1.00 24.43 A
ATOM 2520 CB GLNA320 72.498 48.216 82.177 1.0027.53 A
ATOM 2521 CG GLN A 320 72.154 48.534 80.742 1.0035.93 A
ATOM 2522 CD GLNA320 72.412 47.361 79.826 1.0040.22 A
ATOM 2523 OE1 GLNA320 72.065 46.220 80.150 1.0043.23 A
ATOM 2524 NE2 GLN A 320 73.010 47.630 78.670 1.0041.49 A
ATOM 2525 C GLNA320 71.773 48.668 84.514 1.00 22.47 A
ATOM 2526 O GLN A 320 70.616 48.482 84.889 1.00 22.79 A
ATOM 2527 N META321 72.835 48.341 85.243 1.0019.30 A
ATOM 2528 CA META321 72.685 47.743 86.559 1.0018.82 A
ATOM 2529 CB META321 74.060 47.552 87.205 1.0017.16 A
ATOM 2530 CG META321 74.043 46.762 88.494 1.0018.64 A
ATOM 2531 SD META321 75.680 46.673 89.262 1.0019.02 A
ATOM 2532 CE META321 75.627 48.126 90.297 1.0017.60 A
ATOM 2533 C META321 71.820 48.664 87.421 1.0017.97 A
ATOM 2534 O META321 70.858 48.227 88.046 1.00 18.39 A
ATOM 2535 N ILEA322 72.164 49.945 87.431 1.00 17.43 A
ATOM 2536 CA ILEA322 71.432 50.931 88.213 1.0017.48 A
ATOM 2537 CB ILE A 322 72.126 52.300 88.139 1.00 18.76 A
ATOM 2538 CG2 ILEA322 71.243 53.368 88.763 1.0017.87 A
ATOM 2539 CG1 ILEA322 73.480 52.223 88.850 1.0017.93 A
ATOM 2540 CD1 ILE A 322 74.333 53.457 88.673 1.00 17.10 A
ATOM 2541 C ILEA322 69.978 51.085 87.768 1.00 17.56 A
ATOM 2542 O ILE A 322 69.074 51.161 88.606 1.00 14.93 A
ATOM 2543 N ASN A 323 69.753 51.128 86.455 1.00 16.97 A
ATOM 2544 CA ASN A 323 68.402 51.275 85.931 1.00 17.70 A
ATOM 2545 CB ASN A 323 68.431 51.494 84.411 1.0017.91 A
ATOM 2546 CG ASNA323 67.063 51.885 83.848 1.0020.31 A
ATOM 2547 OD1 ASNA323 66.478 52.908 84.236 1.00 19.93 A
ATOM 2548 ND2 ASN A 323 66.547 51.071 82.931 1.0021.81 A
ATOM 2549 C ASN A 323 67.553 50.046 86.266 1.00 18.15 A
ATOM 2550 O ASNA323 66.380 50.174 86.614 1.0018.53 A
ATOM 2551 N THRA324 68.14848.861 86.167 1.00 17.27 A
ATOM 2552 CA THR A 324 67.432 47.624 86.463 1.00 20.35 A
ATOM 2553 CB THR A 324 68.297 46.396 86.142 1.00 20.62 A
ATOM 2554 OG1 THRA 324 68.730 46.461 84.780 1.00 22.20 A
ATOM 2555 CG2 THRA324 67.502 45.123 86.354 1.00 21.69 A
ATOM 2556 C THRA324 67.014 47.568 87.940 1.0021.79 A
ATOM 2557 O THRA 324 65.873 47.217 88.262 1.0020.91 A
ATOM 2558 N LEU A 325 67.946 47.903 88.831 1.0020.53 A
ATOM 2559 CA LEU A 325 67.657 47.920 90.261 1.00 21.51 A
ATOM 2560 CB LEU A 325 68.883 48.407 91.049 1.00 18.68 A
ATOM 2561 CG LEUA325 70.042 47.404 91.152 1.0018.00 A
ATOM 2562 CD1 LEUA325 71.302 48.082 91.637 1.0015.55 A
ATOM 2563 CD2 LEU A 325 69.647 46.283 92.099 1.00 20.36 A
ATOM 2564 C LEU A 325 66.477 48.856 90.488 1.0022.82 A
ATOM 2565 O LEU A 325 65.580 48.558 91.274 1.0023.67 A
ATOM 2566 N HIS A 326 66.484 49.985 89.780 1.0023.78 A
ATOM 2567 CA HIS A 326 65.415 50.974 89.882 1.00 24.13 A
ATOM 2568 CB HIS A 326 65.771 52.226 89.071 1.0022.40 A
ATOM 2569 CG HIS A 326 66.746 53.139 89.752 1.00 24.50 A
ATOM 2570 CD2HISA326 67.259 53.122 91.007 1.00 23.27 A
ATOM 2571 ND1HISA326 67.272 54.256 89.139 1.00 22.08 A
ATOM 2572 CE1HISA326 68.063 54.890 89.986 1.0023.04 A
ATOM 2573 NE2 HIS A 326 68.073 54.224 91.127 1.0023.03 A
ATOM 2574 C HIS A 326 64.087 50.397 89.386 1.0024.10 A
ATOM 2575 O HIS A 326 63.030 50.688 89.940 1.00 22.94 A
ATOM 2576 N GLN A 327 64.146 49.585 88.334 1.0024.92 A
ATOM 2577 CA GLN A 327 62.942 48.966 87.787 1.00 25.14 A
ATOM 2578 CB GLNA327 63.277 48.129 86.551 1.0026.44 A
ATOM 2579 CG GLN A 327 63.611 48.943 85.317 1.0031.54 A
ATOM 2580 CD GLN A 327 63.986 48.076 84.123 1.00 34.31 A
ATOM 2581 OE1 GLNA327 63.880 48.508 82.969 1.0032.87 A
ATOM 2582 NE2 GLN A 327 64.436 46.852 84.393 1.0034.73 A
ATOM 2583 C GLN A 327 62.318 48.069 88.848 1.00 23.95 A
ATOM 2584 O GLN A 327 61.099 47.983 88.959 1.00 23.57 A
ATOM 2585 N HIS A 328 63.168 47.415 89.632 1.00 21.71 A
ATOM 2586 CA HISA328 62.705 46.521 90.679 1.00 22.02 A
ATOM 2587 CB HIS A 328 63.675 45.346 90.799 1.0022.64 A
ATOM 2588 CG HIS A 328 63.687 44.465 89.588 1.0026.31 A
ATOM 2589 CD2HISA328 64.212 44.655 88.354 1.00 25.96 A
ATOM 2590 ND1 HIS A 328 63.051 43.242 89.549 1.00 26.03 A
ATOM 2591 CE1HISA328 63.184 42.717 88.344 1.00 26.12 A
ATOM 2592 NE2 HIS A 328 63.884 43.554 87.600 1.00 28.11 A
ATOM 2593 C HIS A 328 62.491 47.207 92.032 1.0020.63 A
ATOM 2594 O HIS A 328 62.419 46.551 93.073 1.00 21.92 A
ATOM 2595 N GLYA329 62.379 48.531 92.001 1.00 19.83 A
ATOM 2596 CA GLY A 329 62.136 49.302 93.209 1.00 17.04 A
ATOM 2597 C GLYA329 63.298 49.451 94.170 1.00 18.60 A
ATOM 2598 O GLY A 329 63.098 49.811 95.334 1.00 20.63 A
ATOM 2599 N LEU A 330 64.512 49.188 93.705 1.00 17.57 A
ATOM 2600 CA LEU A 330 65.681 49.303 94.576 1.00 18.82 A
ATOM 2601 CB LEU A 330 66.558 48.055 94.436 1.00 18.54 A
ATOM 2602 CG LEU A 330 65.946 46.738 94.930 1.0022.17 A
ATOM 2603 CD1 LEU A 330 66.826 45.577 94.505 1.0023.19 A
ATOM 2604 CD2 LEU A 330 65.795 46.774 96.446 1.00 21.55 A
ATOM 2605 C LEU A 330 66.508 50.551 94.266 1.00 18.39 A
ATOM 2606 O LEU A 330 66.720 50.895 93.099 1.00 14.16 A
ATOM 2607 N ARGA331 66.971 51.233 95.310 1.0016.21 A
ATOM 2608 CA ARGA331 67.788 52.422 95.102 1.0017.35 A
ATOM 2609 CB ARGA331 67.323 53.550 96.024 1.0019.63 A
ATOM 2610 CG ARGA331 66.289 54.458 95.361 1.0020.96 A
ATOM 2611 CD ARGA331 65.634 55.394 96.351 1.0026.24 A
ATOM 2612 NE ARGA331 64.335 54.880 96.766 1.00 31.48 A
ATOM 2613 CZ ARG A 331 63.176 55.313 96.289 1.0030.23 A
ATOM 2614 NH1 ARGA331 63.149 56.278 95.383 1.00 34.83 A
ATOM 2615 NH2 ARG A 331 62.045 54.772 96.710 1.00 32.92 A
ATOM 2616 C ARG A 331 69.277 52.121 95.282 1.0017.32 A
ATOM 2617 O ARGA331 69.650 51.084 95.849 1.0015.65 A
ATOM 2618 N VALA332 70.123 53.022 94.788 1.0016.06 A
ATOM 2619 CA VAL A 332 71.574 52.832 94.841 1.0015.20 A
ATOM 2620 CB VAL A 332 72.145 52.742 93.405 1.0016.09 A
ATOM 2621 CG1 VALA 332 73.638 52.432 93.439 1.0016.40 A
ATOM 2622 CG2 VAL A 332 71.383 51.683 92.616 1.0016.89 A
ATOM 2623 C VALA332 72.360 53.914 95.580 1.0015.88 A
ATOM 2624 O VALA332 72.122 55.110 95.401 1.0017.01 A
ATOM 2625 N ILE A 333 73.306 53.486 96.410 1.00 15.81 A
ATOM 2626 CA ILEA333 74.158 54.416 97.142 1.00 15.34 A
ATOM 2627 CB ILEA333 74.047 54.229 98.665 1.00 17.38 A
ATOM 2628 CG2 ILE A 333 75.115 55.080 99.380 1.0013.43 A
ATOM 2629 CG1 ILEA333 72.648 54.632 99.135 1.00 17.43 A
ATOM 2630 CD1 ILEA333 72.381 54.285 100.582 1.00 19.91 A
ATOM 2631 C ILEA333 75.601 54.160 96.719 1.0015.81 A
ATOM 2632 O ILEA333 76.054 53.018 96.685 1.00 15.12 A
ATOM 2633 N LEU A 334 76.319 55.224 96.389 1.00 14.97 A
ATOM 2634 CA LEU A 334 77.702 55.089 95.964 1.00 15.29 A
ATOM 2635 CB LEU A 334 77.955 55.954 94.722 1.0016.03 A
ATOM 2636 CG LEU A 334 77.966 55.298 93.333 1.0019.05 A
ATOM 2637 CD1 LEUA334 76.820 54.316 93.163 1.00 15.56 A
ATOM 2638 CD2 LEU A 334 77.898 56.393 92.283 1.00 19.00 A
ATOM 2639 C LEU A 334 78.693 55.470 97.055 1.00 15.66 A
ATOM 2640 O LEU A 334 78.622 56.561 97.624 1.00 16.54 A
ATOM 2641 N ASP A 335 79.609 54.554 97.346 1.00 14.02 A
ATOM 2642 CA ASP A 335 80.663 54.783 98.332 1.0014.82 A
ATOM 2643 CB ASP A 335 81.316 53.440 98.706 1.0015.50 A
ATOM 2644 CG ASP A 335 82.248 53.523 99.917 1.0017.09 A
ATOM 2645 OD1ASPA335 82.950 54.548 100.117 1.0018.12 A
ATOM 2646 OD2ASPA335 82.291 52.522100.666 1.00 14.63 A
ATOM 2647 C ASPA335 81.659 55.637 97.541 1.0015.88 A
ATOM 2648 O ASP A 335 82.105 55.228 96.466 1.00 14.22 A
ATOM 2649 N VAL A 336 81.986 56.823 98.043 1.00 16.96 A
ATOM 2650 CA VAL A 336 82.932 57.697 97.347 1.00 17.09 A
ATOM 2651 CB VALA 336 82.240 58.977 96.825 1.00 16.78 A
ATOM 2652 CG1 VALA 336 81.338 58.623 95.649 1.00 13.38 A
ATOM 2653 CG2 VALA 336 81.436 59.634 97.943 1.00 15.51 A
ATOM 2654 C VALA336 84.087 58.053 98.271 1.0018.09 A
ATOM 2655 O VAL A 336 83.902 58.208 99.478 1.0019.20 A
ATOM 2656 N VALA337 85.270 58.198 97.684 1.0017.57 A
ATOM 2657 CA VALA337 86.502 58.447 98.422 1.00 16.70 A
ATOM 2658 CB VALA 337 87.528 57.328 98.063 1.00 18.74 A
ATOM 2659 CG1 VALA337 88.653 57.271 99.075 1.0016.55 A
ATOM 2660 CG2 VALA 337 86.810 55.983 97.975 1.00 17.87 A
ATOM 2661 C VALA337 87.131 59.810 98.123 1.0017.32 A
ATOM 2662 O VALA337 88.151 59.883 97.446 1.0017.38 A
ATOM 2663 N PHEA338 86.543 60.884 98.639 1.0016.24 A
ATOM 2664 CA PHE A 338 87.078 62.217 98.383 1.00 16.67 A
ATOM 2665 CB PHE A 338 85.938 63.232 98.260 1.0016.07 A
ATOM 2666 CG PHEA338 84.970 62.931 97.147 1.00 15.20 A
ATOM 2667 CD1 PHEA338 85.412 62.804 95.830 1.0016.63 A
ATOM 2668 CD2 PHEA338 83.609 62.802 97.410 1.00 15.84 A
ATOM 2669 CE1 PHE A 338 84.507 62.553 94.784 1.0015.73 A
ATOM 2670 CE2 PHE A 338 82.695 62.553 96.377 1.0016.37 A
ATOM 2671 CZ PHE A 338 83.146 62.428 95.060 1.0014.73 A
ATOM 2672 C PHEA338 88.070 62.688 99.446 1.00 17.79 A
ATOM 2673 O PHEA338 88.393 63.875 99.517 1.0018.06 A
ATOM 2674 N ASN A 339 88.564 61.755 100.257 1.00 16.31 A
ATOM 2675 CA ASN A 339 89.522 62.088 101.311 1.00 17.83 A
ATOM 2676 CB ASN A 339 89.239 61.267 102.575 1.0016.97 A
ATOM 2677 CG ASN A 339 89.297 59.774 102.318 1.0018.78 A
ATOM 2678 OD1 ASNA339 88.273 59.123 102.075 1.00 20.28 A
ATOM 2679 ND2 ASN A 339 90.497 59.226 102.350 1.00 12.63 A
ATOM 2680 C ASN A 339 90.978 61.852 100.897 1.00 18.39 A
ATOM 2681 O ASN A 339 91.894 62.327101.569 1.00 18.05 A
ATOM 2682 N HIS A 340 91.193 61.128 99.800 1.0016.26 A
ATOM 2683 CA HIS A 340 92.551 60.845 99.353 1.00 17.25 A
ATOM 2684 CB HISA 340 93.224 59.886 100.342 1.00 18.05 A
ATOM 2685 CG HISA340 92.864 58.446 100.130 1.00 19.10 A
ATOM 2686 CD2 HIS A 340 91.860 57.692 100.637 1.00 18.56 A
ATOM 2687 ND1HISA340 93.579 57.615 99.292 1.0020.39 A
ATOM 2688 CE1 HIS A 340 93.030 56.412 99.295 1.0019.02 A
ATOM 2689 NE2 HIS A 340 91.986 56.433 100.103 1.0019.79 A
ATOM 2690 C HIS A 340 92.588 60.228 97.955 1.00 17.42 A
ATOM 2691 O HIS A 340 91.562 59.802 97.423 1.00 17.34 A
ATOM 2692 N VALA341 93.776 60.194 97.365 1.0016.45 A
ATOM 2693 CA VALA341 93.963 59.599 96.048 1.0018.11 A
ATOM 2694 CB VALA341 94.405 60.644 94.984 1.00 17.47 A
ATOM 2695 CG1VALA341 - 93.455 61.829 94.988 1.00 19.34 A
ATOM 2696 CG2VALA341 95.837 61.094 95.249 1.0018.71 A
ATOM 2697 C VALA341 95.063 58.555 96.165 1.00 18.88 A
ATOM 2698 O VAL A 341 95.829 58.550 97.128 1.00 18.05 A
ATOM 2699 N TYR A 342 95.140 57.668 95.186 1.0021.11 A
ATOM 2700 CA TYR A 342 96.173 56.649 95.186 1.0022.76 A
ATOM 2701 CB TYR A 342 95.745 55.488 94.289 1.0024.57 A
ATOM 2702 CG TYRA 342 96.752 54.362 94.199 1.0026.43 A
ATOM 2703 CD1 TYRA342 97.520 54.180 93.053 1.0027.70 A
ATOM 2704 CE1 TYRA342 98.43553.131 92.953 1.0028.90 A
ATOM 2705 CD2TYRA342 96.924 53.470 95.254 1.0026.53 A
ATOM 2706 CE2 TYR A 342 97.835 52.421 95.169 1.0029.72 A
ATOM 2707 CZ TYR A 342 98.585 52.256 94.014 1.0031.51 A
ATOM 2708 OH TYRA 342 99.476 51.211 93.917 1.0032.85 A
ATOM 2709 C TYRA342 97.459 57.296 94.656 1.00 24.39 A
ATOM 2710 O TYRA342 97.447 57.961 93.618 1.0025.16 A
ATOM 2711 N LYSA343 98.558 57.112 95.380 1.0025.35 A
ATOM 2712 CA LYSA343 99.848 57.672 94.984 1.00 25.64 A
ATOM 2713 CB LYS A 343 100.423 56.887 93.805 1.00 27.54 A
ATOM 2714 CG LYSA343 100.978 55.520 94.182 1.00 34.26 A
ATOM 2715 CD LYSA343 101.628 54.838 92.987 1.0037.73 A
ATOM 2716 CE LYS A 343 102.389 53.591 93.406 1.0040.41 A
ATOM 2717 NZ LYS A 343 103.555 53.911 94.286 1.00 42.60 A
ATOM 2718 C LYS A 343 99.763 59.152 94.626 1.00 24.56 A
ATOM 2719 O LYS A 343 99.742 59.522 93.454 1.00 24.04 A
ATOM 2720 N ARG A 344 99.722 59.989 95.652 1.0023.08 A
ATOM 2721 CA ARG A 344 99.634 61.438 95.497 1.00 24.18 A
ATOM 2722 CB ARG A 344 99.904 62.112 96.852 1.0022.29 A
ATOM 2723 CG ARG A 344 99.920 63.634 96.835 1.0023.73 A
ATOM 2724 CD ARG A 344 100.432 64.182 98.171 1.0026.63 A
ATOM 2725 NE ARG A 344 101.815 63.784 98.415 1.0026.45 A
ATOM 2726 CZ ARG A 344 102.851 64.239 97.71 1.00 28.11 A
ATOM 2727 NH1ARGA344 102.662 65.116 96.741 1.00 28.15 A
ATOM 2728 NH2 ARG A 344 104.073 63.799 97.978 1.0029.38 A
ATOM 2729 C ARGA344 100.589 62.009 94.453 1.00 24.52 A
ATOM 2730 O ARG A 344 100.190 62.803 93.599 1.0022.88 A
ATOM 2731 N GLU A 345 101.850 61.593 94.540 1.0027.29 A
ATOM 2732 CA GLU A 345 102.920 62.054 93.658 1.0029.52 A
ATOM 2733 CB GLU A 345 104.217 61.323 94.030 1.00 34.13 A
ATOM 2734 CG GLU A 345 104.576 61.489 95.515 1.0044.66 A
ATOM 2735 CD GLU A 345 105.780 60.657 95.959 1.0049.89 A
ATOM 2736 OE1 GLU A 345 106.144 60.732 97.156 1.0052.06 A
ATOM 2737 OE2 GLU A 345 106.361 59.930 95.122 1.0054.05 A
ATOM 2738 C GLU A 345 102.658 61.935 92.156 1.0027.40 A
ATOM 2739 O GLUA345 103.104 62.781 91.377 1.0026.86 A
ATOM 2740 N ASN A 346 101.932 60.900 91.748 1.00 25.36 A
ATOM 2741 CA ASN A 346 101.639 60.705 90.332 1.00 24.64 A
ATOM 2742 CB ASN A 346 102.026 59.285 89.907 1.00 28.49 A
ATOM 2743 CG ASN A 346 103.453 58.920 90.300 1.00 33.34 A
ATOM 2744 OD1 ASNA346 104.385 59.721 90.158 1.0034.18 A
ATOM 2745 ND2 ASNA346 103.631 57.696 90.785 1.00 36.56 A
ATOM 2746 C ASN A 346 100.166 60.944 90.002 1.0023.09 A
ATOM 2747 O ASN A 346 99.689 60.559 88.939 1.0022.41 A
ATOM 2748 N SERA 347 99.445 61.591 90.908 1.0020.76 A
ATOM 2749 CA SERA 347 98.028 61.840 90.689 1.00 19.59 A
ATOM 2750 CB SERA 347 97.330 62.105 92.031 1.0018.48 A
ATOM 2751 OG SER A 347 97.727 63.359 92.573 1.00 18.97 A
ATOM 2752 C SER A 347 97.749 63.007 89.743 1.00 17.46 A
ATOM 2753 O SERA347 98.552 63.930 89.616 1.0016.76 A
ATOM 2754 N PRO A 348 96.601 62.965 89.047 1.00 17.69 A
ATOM 2755 CD PRO A 348 95.698 61.808 88.890 1.0015.80 A
ATOM 2756 CA PRO A 348 96.234 64.044 88.125 1.00 16.65 A
ATOM 2757 CB PRO A 348 94.833 63.644 87.688 1.00 16.99 A
ATOM 2758 CG PRO A 348 94.953 62.143 87.615 1.00 16.28 A
ATOM 2759 C PRO A 348 96.265 65.394 88.854 1.00 17.50 A
ATOM 2760 O PRO A 348 96.698 66.403 88.292 1.00 16.05 A
ATOM 2761 N PHE A 349 95.813 65.409 90.110 1.00 16.25 A
ATOM 2762 CA PHE A 349 95.820 66.643 90.897 1.00 15.78 A
ATOM 2763 CB PHE A 349 95.282 66.410 92.312 1.00 13.66 A
ATOM 2764 CG PHE A 349 93.780 66.300 92.398 1.00 15.12 A
ATOM 2765 CD1 PHEA349 92.963 67.024 91.543 1.00 11.51 A
ATOM 2766 CD2 PHE A 349 93.187 65.513 93.383 1.00 16.41 A
ATOM 2767 CE1 PHE A 349 91.585 66.974 91.662 1.0013.63 A
ATOM 2768 CE2 PHE A 349 91.803 65.457 93.514 1.0018.01 A
ATOM 2769 CZ PHE A 349 90.999 66.191 92.649 1.0017.26 A
ATOM 2770 C PHE A 349 97.230 67.212 91.026 1.00 17.00 A
ATOM 2771 O PHEA349 97.495 68.350 90.640 1.0017.06 A
ATOM 2772 N GLU A 350 98.130 66.406 91.582 1.0018.34 A
ATOM 2773 CA GLU A 350 99.510 66.818 91.803 1.00 18.04 A
ATOM 2774 CB GLU A 350 100.253 65.715 92.560 1.00 19.57 A
ATOM 2775 CG GLU A 350 101.654 66.088 93.017 1.00 20.56 A
ATOM 2776 CD GLU A 350 101.678 67.311 93.909 1.00 21.48 A
ATOM 2777 OE1 GLU A 350 100.639 67.637 94.522 1.00 22.37 A
ATOM 2778 OE2 GLU A 350 102.748 67.942 94.005 1.00 25.61 A
ATOM 2779 C GLU A 350 100.268 67.187 90.526 1.00 17.47 A
ATOM 2780 O GLU A 350 101.060 68.131 90.525 1.00 16.94 A
ATOM 2781 N LYS A 351 100.030 66.452 89.444 1.00 16.67 A
ATOM 2782 CA LYS A 351 100.702 66.742 88.176 1.0018.53 A
ATOM 2783 CB LYSA351 100.598 65.540 87.234 1.00 19.18 A
ATOM 2784 CG LYSA351 101.557 64.404 87.554 1.0022.25 A
ATOM 2785 CD LYSA351 101.300 63.224 86.632 1.0027.50 A
ATOM 2786 CE LYSA351 102.336 62.139 86.809 1.0030.98 A
ATOM 2787 NZ LYS A 351 103.691 62.61086.401 1.0035.77 A
ATOM 2788 C LYSA351 100.160 67.986 87.463 1.0017.44 A
ATOM 2789 O LYSA351 100.827 68.547 86.593 1.0016.74 A
ATOM 2790 N THR A 352 98.956 68.419 87.829 1.0017.10 A
ATOM 2791 CA THR A 352 98.351 69.588 87.190 1.0016.87 A
ATOM 2792 CB THRA352 96.818 69.398 87.037 1.00 15.75 A
ATOM 2793 OG1 THRA352 96.558 68.153 86.364 1.00 16.16 A
ATOM 2794 CG2 THR A 352 96.214 70.530 86.217 1.0010.81 A
ATOM 2795 C THRA352 98.650 70.873 87.973 1.0016.91 A
ATOM 2796 O THR A 352 99.033 71.884 87.389 1.0016.09 A
ATOM 2797 N VALA353 98.465 70.824 89.290 1.00 15.55 A
ATOM 2798 CA VALA 353 98.740 71.963 90.162 1.0016.21 A
ATOM 2799 CB VAL A 353 97.447 72.709 90.567 1.00 15.99 A
ATOM 2800 CG1 VALA 353 97.816 74.026 91.256 1.00 15.35 A
ATOM 2801 CG2 VALA353 96.578 72.978 89.328 1.00 14.00 A
ATOM 2802 C VALA353 99.436 71.430 91.420 1.0017.02 A
ATOM 2803 O VALA353 98.795 71.133 92.435 1.0015.37 A
ATOM 2804 N PRO A 354 100.770 71.293 91.356 1.00 17.31 A
ATOM 2805 CD PRO A 354 101.602 71.724 90.218 1.0016.93 A
ATOM 2806 CA PRO A 354 101.606 70.792 92.452 1.00 18.10 A
ATOM 2807 CB PRO A 354 103.027 71.084 91.969 1.00 16.29 A
ATOM 2808 CG PRO A 354 102.905 71.008 90.484 1.0016.76 A
ATOM 2809 C PRO A 354 101.327 71.449 93.794 1.0018.81 A
ATOM 2810 O PRO A 354 101.309 72.673 93.894 1.00 19.00 A
ATOM 2811 N GLY A 355 101.099 70.620 94.811 1.0019.31 A
ATOM 2812 CA GLY A 355 100.859 71.108 96.158 1.00 18.32 A
ATOM 2813 C GLYA355 99.552 71.822 96.470 1.00 18.70 A
ATOM 2814 O GLY A 355 99.363 72.290 97.594 1.00 19.33 A
ATOM 2815 N TYRA356 98.642 71.898 95.509 1.00 14.83 A
ATOM 2816 CA TYRA 356 97.379 72.588 95.738 1.0014.91 A
ATOM 2817 CB TYRA356 96.850 73.160 94.420 1.00 13.98 A
ATOM 2818 CG TYR A 356 95.499 73.838 94.549 1.0016.87 A
ATOM 2819 CD1 TYRA356 95.404 75.197 94.852 1.0015.24 A
ATOM 2820 CE1TYRA356 94.165 75.823 94.974 1.0017.08 A
ATOM 2821 CD2TYRA356 94.315 73.118 94.375 1.0014.72 A
ATOM 2822 CE2 TYRA 356 93.07473.731 94.499 1.00 15.08 A
ATOM 2823 CZ TYRA356 93.003 75.085 94.798 1.00 17.79 A
ATOM 2824 OH TYRA356 91.774 75.703 94.934 1.00 17.83 A
ATOM 2825 C TYRA356 96.274 71.733 96.363 1.00 15.22 A
ATOM 2826 O TYRA 356 95.618 72.148 97.321 1.0015.61 A
ATOM 2827 N PHE A 357 96.078 70.544 95.808 1.00 13.91 A
ATOM 2828 CA PHE A 357 95.018 69.644 96.229 1.00 14.77 A
ATOM 2829 CB PHE A 357 94.664 68.738 95.054 1.00 13.46 A
ATOM 2830 CG PHEA357 93.997 69.462 93.917 1.00 13.26 A
ATOM 2831 CD1 PHEA357 92.653 69.798 93.986 1.00 13.73 A
ATOM 2832 CD2 PHE A 357 94.714 69.806 92.774 1.0014.21 A
ATOM 2833 CE1 PHEA357 92.027 70.467 92.924 1.0015.34 A
ATOM 2834 CE2PHEA357 94.099 70.473 91.712 1.0014.24 A
ATOM 2835 CZ PHE A 357 92.756 70.802 91.786 1.0014.89 A
ATOM 2836 C PHEA357 95.219 68.794 97.478 1.00 15.45 A
ATOM 2837 O PHEA357 94.337 68.021 97.839 1.0014.98 A
ATOM 2838 N PHE A 358 96.354 68.938 98.150 1.0016.07 A
ATOM 2839 CA PHE A 358 96.610 68.133 99.341 1.00 17.64 A
ATOM 2840 CB PHEA358 97.679 67.087 99.029 1.0015.99 A
ATOM 2841 CG PHE A 358 97.319 66.208 97.875 1.0015.38 A
ATOM 2842 CD1 PHEA358 96.473 65.114 98.055 1.0015.47 A
ATOM 2843 CD2 PHE A 358 97.751 66.522 96.593 1.00 13.23 A
ATOM 2844 CE1 PHEA358 96.058 64.349 96.972 1.0014.43 A
ATOM 2845 CE2 PHE A 358 97.343 65.763 95.500 1.0016.20 A
ATOM 2846 CZ PHE A 358 96.491 64.673 95.690 1.0015.40 A
ATOM 2847 C PHEA358 97.030 68.970100.528 1.0018.06 A
ATOM 2848 O PHEA358 97.800 69.916 100.389 1.0019.66 A
ATOM 2849 N ARGA359 96.521 68.626 101.702 1.0018.57 A
ATOM 2850 CA ARG A 359 96.875 69.383 102.890 1.00 19.37 A
ATOM 2851 CB ARG A 359 95.908 69.072 104.033 1.0019.03 A
ATOM 2852 CG ARG A 359 94.487 69.521 103.767 1.0016.47 A
ATOM 2853 CD ARG A 359 93.605 69.349 104.993 1.0016.07 A
ATOM 2854 NE ARG A 359 92.205 69.570 104.642 1.0016.54 A
ATOM 2855 CZ ARG A 359 91.231 68.695 104.865 1.00 13.44 A
ATOM 2856 NH1 ARG A 359 91.496 67.533 105.450 1.00 13.61 A
ATOM 2857 NH2ARGA359 89.997 68.973 104.476 1.00 14.73 A
ATOM 2858 C ARG A 359 98.291 69.065 103.323 1.00 21.04 A
ATOM 2859 O ARGA359 98.774 67.951 103.136 1.0020.06 A
ATOM 2860 N HIS A 360 98.951 70.059 103.904 1.0024.89 A
ATOM 2861 CA HIS A 360 100.312 69.897 104.392 1.0027.34 A
ATOM 2862 CB HIS A 360 101.270 70.792 103.603 1.00 27.91 A
ATOM 2863 CG HIS A 360 101.389 70.430 102.157 1.00 28.67 A
ATOM 2864 CD2HISA360 102.423 69.915 101.451 1.00 28.60 A
ATOM 2865 ND1HISA360 100.353 70.591 101.259 1.0027.75 A
ATOM 2866 CEIHISA 360 100.746 70.190 100.063 1.0027.44 A
ATOM 2867 NE2H1SA360 101.997 69.776 100.152 1.0028.70 A
ATOM 2868 C HIS A 360 100.381 70.271 105.871 1.0029.78 A
ATOM 2869 O HIS A 360 99.572 71.063 106.354 1.0028.20 A
ATOM 2870 N ASPA361 101.347 69.692 106.582 1.00 32.89 A
ATOM 2871 CA ASPA361 101.545 69.982108.000 1.005.71 A
ATOM 2872 CB ASPA361 102.285 68.823 108.690 1.00 35.17 A
ATOM 2873 CG ASPA361 103.683 68.578 108.124 1.00 37.20 A
ATOM 2874 OD1 ASPA361 104.267 67.518 108.441 1.0037.65 A
ATOM 2875 OD2ASPA361 104.210 69.433 107.379 1.00 36.31 A
ATOM 2876 C ASPA361 102.334 71.288 108.128 1.00 37.74 A
ATOM 2877 O ASPA361 102.765 71.857 107.123 1.0037.33 A
ATOM 2878 N GLU A 362 102.524 71.767 109.352 1.00 40.75 A
ATOM 2879 CA GLU A 362 103.244 73.020 109.565 1.00 42.77 A
ATOM 2880 CB GLU A 362 103.198 73.407 111.046 1.0044.00 A
ATOM 2881 CG GLU A 362 102.495 72.388 111.934 1.0045.73 A
ATOM 2882 CD GLU A 362 103.447 71.353 112.499 1.0046.57 A
ATOM 2883 OE1 GLU A 362 104.224 70.772 111.715 1.0048.85 A
ATOM 2884 OE2 GLU A 362 103.416 71.118 113.729 1.0046.13 A
ATOM 2885 C GLU A 362 104.690 73.010 109.065 1.00 43.65 A
ATOM 2886 O GLU A 362 105.323 74.061 108.972 1.0045.43 A
ATOM 2887 N CYS A 363 105.208 71.830 108.734 1.0043.94 A
ATOM 2888 CA CYS A 363 106.575 71.718 108.230 1.0043.66 A
ATOM 2889 CB CYS A 363 107.248 70.448 108.772 1.0043.71 A
ATOM 2890 SG CYS A 363 107.788 70.558 110.506 1.0045.42 A
ATOM 2891 C CYSA363 106.627 71.722 106.701 1.00 42.98 A
ATOM 2892 O CYSA363 107.704 71.698 106.107 1.0043.02 A
ATOM 2893 N GLYA364 105.459 71.750 106.067 1.0042.40 A
ATOM 2894 CA GLY A 364 105.405 71.763 104.615 1.0041.64 A
ATOM 2895 C GLYA364 105.271 70.397 103.961 1.0041.02 A
ATOM 2896 O GLYA364 105.281 70.292 102.735 1.0041.55 A
ATOM 2897 N LYSA365 105.154 69.347 104.767 1.0039.79 A
ATOM 2898 CA LYS A 365 105.013 67.997 104.230 1.00 38.53 A
ATOM 2899 CB LYS A 365 105.813 67.003 105.081 1.0039.08 A
ATOM 2900 CG LYS A 365 107.317 67.226 105.014 1.004.1.16 A
ATOM 2901 CD LYS A 365 107.800 67.158103.574 1.0041.72 A
ATOM 2902 CE LYS A 365 109.105 67.906 103.383 1.0043.64 A
ATOM 2903 NZ LYS A 365 109.485 67.978 101.946 1.0043.14 A
ATOM 2904 C LYSA365 103.546 67.570 104.158 1.00 37.43 A
ATOM 2905 O LYSA365 102.728 67.975 104.988 1.0034.88 A
ATOM 2906 N PROA366 103.193 66.756 103.147 1.0036.43 A
ATOM 2907 CD PRO A 366 104.063 66.269 102.060 1.00 36.92 A
ATOM 2908 CA PRO A 366 101.817 66.277 102.971 1.0035.66 A
ATOM 2909 CB PRO A 366 101.935 65.305 101.796 1.0036.08 A
ATOM 2910 CG PRO A 366 103.060 65.894 100.987 1.0036.93 A
ATOM 2911 C PROA366 101.321 65.588104.235 1.0034.36 A
ATOM 2912 O PRO A 366 101.998 64.708 104.768 1.0034.55 A
ATOM 2913 N SERA367 100.144 65.994 104.710 1.0033.32 A
ATOM 2914 CA SER A 367 99.556 65.417 105.917 1.0031.36 A
ATOM 2915 CB SERA 367 98.376 66.268 106.406 1.0033.26 A
ATOM 2916 OG SER A 367 97.259 66.171 105.534 1.00 32.67 A
ATOM 2917 C SERA367 99.082 63.997 105.643 1.00 30.41 A
ATOM 2918 O SERA367 98.949 63.582 104.485 1.00 28.36 A
ATOM 2919 N ASN A 368 98.812 63.258 106.712 1.0028.66 A
ATOM 2920 CA ASN A 368 98.380 61.882 106.569 1.0028.52 A
ATOM 2921 CB ASN A 368 99.604 60.966 106.645 1.0029.61 A
ATOM 2922 CG ASN A 368 99.268 59.516 106.377 1.0031.60 A
ATOM 2923 OD1 ASNA368 98.430 59.204 105.531 1.0032.12 A
ATOM 2924 ND2 ASN A 368 99.936 58.616 107.089 1.00 31.99 A
ATOM 2925 C ASNA368 97.336 61.483 107.607 1.0027.83 A
ATOM 2926 O ASN A 368 97.472 60.468 108.287 1.0028.06 A
ATOM 2927 N GLYA369 96.287 62.291 107.715 1.00 26.82 A
ATOM 2928 CA GLY A 369 95.218 61.999 108.650 1.0025.76 A
ATOM 2929 C GLY A 369 94.401 60.790 108.227 1.00 24.85 A
ATOM 2930 O GLYA369 93.840 60.089 109.071 1.0024.52 A
ATOM 2931 N THRA370 94.326 60.546 106.921 1.0022.91 A
ATOM 2932 CA THR A 370 93.576 59.409 106.393 1.00 22.92 A
ATOM 2933 CB THR A 370 93.441 59.475 104.853 1.0021.86 A
ATOM 2934 OG1 THRA 370 94.748 59.504 104.264 1.0019.65 A
ATOM 2935 CG2 THR A 370 92.655 60.712 104.429 1.0018.17 A
ATOM 2936 C THRA370 94.310 58.119 106.739 1.0025.04 A
ATOM 2937 O THR A 370 93.705 57.051 106.826 1.0024.67 A
ATOM 2938 N GLYA371 95.623 58.228 106.925 1.00 25.53 A
ATOM 2939 CAGLYA371 96.420 57.062 107.261 1.0024.95 A
ATOM 2940 C GLYA371 96.894 56.282106.048 1.0025.27 A
ATOM 2941 O GLYA371 97.539 55.242 106.191 1.0023.79 A
ATOM 2942 N VALA372 96.583 56.775 104.852 1.0024.95 A
ATOM 2943 CAVALA 372 96.998 56.083 103.637 1.00 24.43 A
ATOM 2944 CB VALA372 95.768 55.599 102.811 1.00 25.41 A
ATOM 2945 CG1 VALA372 95.042 54.493 103.566 1.00 19.42 A
ATOM 2946 CG2 VALA372 94.818 56.766 102.533 1.0026.21 A
ATOM 2947 C VALA372 97.938 56.885102.733 1.0024.55 A
ATOM 2948 O VALA372 98.122 56.544 101.562 1.0024.61 A
ATOM 2949 N GLYA373 98.529 57.951 103.267 1.00 23.85 A
ATOM 2950 CA GLY A 373 99.476 58.721 102.475 1.00 24.46 A
ATOM 2951 C GLY A 373 99.211 60.187 102.199 1.0023.66 A
ATOM 2952 O GLY A 373 100.157 60.968 102.096 1.00 23.93 A
ATOM 2953 N ASNA374 97.945 60.567 102.055 1.0022.58 A
ATOM 2954 CA ASN A 374 97.602 61.955 101.778 1.00 21.50 A
ATOM 2955 CB ASN A 374 97.807 62.282 100.293 1.00 20.90 A
ATOM 2956 CG ASN A 374 96.878 61.489 99.387 1.0023.44 A
ATOM 2957 OD1 ASNA374 97.155 60.338 99.044 1.00 25.20 A
ATOM 2958 ND2 ASN A 374 95.761 62.095 99.010 1.00 22.28 A
ATOM 2959 C ASN A 374 96.164 62.279 102.147 1.00 20.89 A
ATOM 2960 O ASNA374 95.324 61.389 102.292 1.0020.25 A
ATOM 2961 N ASPA375 95.899 63.573 102.292 1.00 19.35 A
ATOM 2962 CA ASP A 375 94.578 64.071 102.623 1.00 18.39 A
ATOM 2963 CB ASP A 375 94.581 64.743 103.998 1.0018.28 A
ATOM 2964 CG ASP A 375 94.932 63.790 105.117 1.00 20.82 A
ATOM 2965 OD1 ASPA375 95.231 64.278 106.232 1.0020.48 A
ATOM 2966 OD2 ASP A 375 94.903 62.563 104.886 1.00 22.71 A

ATOM 2967 C ASP A 375 94.220 65.114 101.579 1.00 16.95 A
ATOM 2968 O ASPA375 94.947 66.090101.412 1.0016.32 A
ATOM 2969 N ILEA376 93.114 64.912 100.872 1.00 15.92 A
ATOM 2970 CA ILEA376 92.688 65.890 99.881 1.00 16.61 A
ATOM 2971 CB ILE A 376 91.497 65.379 99.048 1.00 16.27 A
ATOM 2972 CG2 ILE A 376 90.879 66.532 98.250 1.00 15.25 A
ATOM 2973 CG1 ILE A 376 91.964 64.255 98.124 1.00 15.06 A
ATOM 2974 CD1 ILE A 376 90.877 63.688 97.247 1.00 16.36 A
ATOM 2975 C ILE A 376 92.257 67.139 100.633 1.00 17.26 A
ATOM 2976 O ILE A 376 91.492 67.054 101.594 1.00 16.86 A
ATOM 2977 N ALA A 377 92.761 68.291 100.202 1.00 16.16 A
ATOM 2978 CA ALA A 377 92.427 69.562 100.835 1.00 17.32 A
ATOM 2979 CB ALAA377 93.556 70.581 100.593 1.0017.97 A
ATOM 2980 C ALAA377 91.102 70.095 100.287 1.00 17.32 A
ATOM 2981 O ALAA 377 91.075 71.037 99.487 1.00 16.39 A
ATOM 2982 N SERA 378 90.007 69.488 100.732 1.00 16.63 A
ATOM 2983 CA SERA 378 88.666 69.869 100.291 1.00 16.62 A
ATOM 2984 CB SERA 378 87.621 69.029 101.037 1.00 14.29 A
ATOM 2985 OG SER A 378 87.728 69.223 102.435 1.00 15.07 A
ATOM 2986 C SERA378 88.329 71.358 100.454 1.00 16.12 A
ATOM 2987 O SERA 378 87.490 71.897 99.722 1.00 13.22 A
ATOM 2988 N GLU A 379 88.971 72.018 101.413 1.0015.36 A
ATOM 2989 CA GLU A 379 88.699 73.426 101.654 1.00 18.12 A
ATOM 2990 CB GLU A 379 89.221 73.854 103.037 1.00 18.65 A
ATOM 2991 CG GLU A 379 90.732 74.019 103.153 1.00 20.73 A
ATOM 2992 CD GLU A 379 91.480 72.712 103.391 1.00 24.35 A
ATOM 2993 OE1 GLU A 379 92.714 72.780 103.586 1.0024.02 A
ATOM 2994 OE2 GLU A 379 90.850 71.626 103.385 1.00 23.71 A
ATOM 2995 C GLU A 379 89.260 74.352 100.569 1.00 18.29 A
ATOM 2996 O GLU A 379 88.861 75.510 100.474 1.00 17.87 A
ATOM 2997 N ARG A 380 90.194 73.862 99.761 1.00 18.17 A
ATOM 2998 CA ARG A 380 90.725 74.696 98.688 1.00 18.99 A
ATOM 2999 CB ARG A 380 91.910 73.997 98.019 1.0019.72 A
ATOM 3000 CG ARG A 380 93.092 73.779 98.981 1.0023.03 A
ATOM 3001 CD ARG A 380 94.173 74.837 98.821 1.00 25.24 A
ATOM 3002 NE ARG A 380 93.654 76.198 98.937 1.0027.69 A
ATOM 3003 CZ ARG A 380 93.417 76.817 100.090 1.0029.56 A
ATOM 3004 NH1 ARG A 380 92.941 78.057 100.092 1.00 28.66 A
ATOM 3005 NH2 ARG A 380 93.665 76.202 101.242 1.00 28.22 A
ATOM 3006 C ARG A 380 89.524 74.834 97.751 1.00 18.14 A
ATOM 3007 O ARG A 380 88.841 73.849 97.478 1.00 18.08 A
ATOM 3008 N ARG A 381 89.255 76.046 97.270 1.00 16.47 A
ATOM 3009 CA ARG A 381 88.074 76.273 96.438 1.00 16.45 A
ATOM 3010 CB ARG A 381 87.951 77.761 96.103 1.00 15.49 A
ATOM 3011 CG ARG A 381 87.713 78.628 97.337 1.0017.80 A
ATOM 3012 CD ARG A 381 87.578 80.103 96.982 1.0022.01 A
ATOM 3013 NE ARG A 381 86.413 80.364 96.136 1.00 23.32 A
ATOM 3014 CZ ARG A 381 86.114 81.554 95.624 1.00 23.62 A
ATOM 3015 NH1 ARG A 381 86.896 82.599 95.870 1.00 24.70 A
ATOM 3016 NH2 ARG A 381 85.034 81.700 94.866 1.00 24.56 A
ATOM 3017 C ARG A 381 87.887 75.422 95.178 1.00 16.42 A
ATOM 3018 O ARG A 381 ' 86.755 75.076 94.839 1.00 15.20 A
ATOM 3019 N META 382 88.969 75.085 94.482 1.00 15.06 A
ATOM 3020 CA MET A 382 88.830 74.255 93.292 1.00 16.61 A
ATOM 3021 CB META382 90.022 74.447 92.358 1.00 16.57 A
ATOM 3022 CG META382 89.999 75.781 91.632 1.0015.50 A
ATOM 3023 SD META 382 88.429 76.073 90.766 1.0020.88 A
ATOM 3024 CE META 382 88.683 75.119 89.249 1.0013.19 A
ATOM 3025 C META 382 88.663 72.779 93.662 1.00 16.99 A
ATOM 3026 O META 382 88.216 71.977 92.838 1.00 18.63 A
ATOM 3027 N ALA A 383 89.016 72.430 94.900 1.00 15.41 A
ATOM 3028 CA ALAA383 88.860 71.058 95.395 1.00 16.53 A
ATOM 3029 CB ALAA383 89.732 70.826 96.618 1.00 15.13 A
ATOM 3030 C ALAA383 87.389 70.886 95.765 1.0014.55 A
ATOM 3031 O ALAA383 86.761 69.883 95.432 1.0013.88 A
ATOM 3032 N ARGA384 86.856 71.881 96.465 1.0014.35 A
ATOM 3033 CA ARG A 384 85.452 71.898 96.860 1.00 15.45 A
ATOM 3034 CB ARG A 384 85.154 73.182 97.633 1.00 13.28 A
ATOM 3035 CG ARG A 384 83.681 73.447 97.923 1.0014.78 A
ATOM 3036 CD ARGA384 83.538 74.718 98.755 1.0013.48 A
ATOM 3037 NE ARG A 384 84.193 74.575 100.054 1.00 16.63 A
ATOM 3038 CZ ARG A 384 84.628 75.592 100.797 1.00 9.03 A
ATOM 3039 NH1 ARG A 384 84.482 76.838 100.367 1.00 19.19 A
ATOM 3040 NH2 ARG A 384 85.207 75.366 101.975 1.00 16.43 A
ATOM 3041 C ARG A 384 84.610 71.847 95.582 1.00 16.83 A
ATOM 3042 O ARG A 384 83.604 71.140 95.515 1.00 18.06 A
ATOM 3043 N LYSA385 85.029 72.603 94.569 1.00 16.15 A
ATOM 3044 CA LYSA385 84.325 72.620 93.291 1.00 15.41 A
ATOM 3045 CB LYS A 385 85.014 73.576 92.314 1.0015.10 A
ATOM 3046 CG LYS A 385 84.470 73.532 90.884 1.00 16.31 A
ATOM 3047 CD LYSA385 85.443 74.197 89.918 1.00 16.48 A
ATOM 3048 CE LYS A 385 84.975 74.111 88.472 1.0017.95 A
ATOM 3049 NZ LYS A 385 83.862 75.060 88.191 1.00 19.52 A
ATOM 3050 C LYS A 385 84.344 71.214 92.701 1.00 15.91 A
ATOM 3051 O LYS A 385 83.327 70.721 92.203 1.00 15.62 A
ATOM 3052 N PHEA386 85.508 70.574 92.765 1.00 14.90 A
ATOM 3053 CA PHEA 386 85.678 69.229 92.217 1.00 15.24 A
ATOM 3054 CB PHE A 386 87.143 68.791 92.350 1.00 15.22 A
ATOM 3055 CG PHE A 386 87.421 67.408 91.821 1.00 15.85 A
ATOM 3056 CD1 PHE A 386 87.256 66.287 92.633 1.00 15.83 A
ATOM 3057 CD2 PHE A 386 87.860 67.228 90.513 1.00 14.56 A
ATOM 3058 CE1 PHE A 386 87.530 65.002 92.149 1.00 15.35 A
ATOM 3059 CE2 PHE A 386 88.134 65.949 90.019 1.00 16.26 A
ATOM 3060 CZ PHE A 386 87.969 64.834 90.843 1.00 16.27 A
ATOM 3061 C PHEA386 84.761 68.199 92.867 1.00 15.42 A
ATOM 3062 O PHEA386 84.053 67.465 92.177 1.00 15.09 A
ATOM 3063 N ILEA387 84.771 68.152 94.192 1.0014.85 A
ATOM 3064 CA ILEA387 83.943 67.205 94.925 1.0015.84 A
ATOM 3065 CB ILE A 387 84.200 67.321 96.449 1.00 15.76 A
ATOM 3066 CG2 ILE A 387 83.184 66.473 97.233 1.00 12.37 A
ATOM 3067 CG1 ILEA 387 85.641 66.890 96.748 1.00 13.46 A
ATOM 3068 CD1 ILE A 387 86.047 67.047 98.199 1.00 13.54 A
ATOM 3069 C ILE A 387 82.467 67.440 94.628 1.0015.74 A
ATOM 3070 O ILE A 387 81.712 66.493 94.409 1.00 16.91 A
ATOM 3071 N ALAA388 82.060 68.704 94.611 1.00 15.02 A
ATOM 3072 CA ALA A 388 80.674 69.041 94.328 1.0014.07 A
ATOM 3073 CB ALAA388 80.437 70.533 94.528 1.0011.74 A
ATOM 3074 C ALA A 388 80.375 68.645 92.888 1.0015.25 A
ATOM 3075 O ALAA388 79.345 68.020 92.607 1.00 15.05 A
ATOM 3076 N ASPA389 81.283 69.010 91.983 1.00 15.31 A
ATOM 3077 CA ASP A 389 81.138 68.684 90.567 1.00 16.86 A
ATOM 3078 CB ASP A 389 82.393 69.077 89.767 1.00 16.92 A
ATOM 3079 CG ASP A 389 82.419 70.547 89.361 1.00 19.91 A
ATOM 3080 OD1 ASP A 389 83.409 70.947 88.696 1.00 20.63 A
ATOM 3081 OD2 ASP A 389 81.472 71.298 89.689 1.00 16.06 A
ATOM 3082 C ASP A 389 80.940 67.182 90.401 1.00 16.66 A
ATOM 3083 O ASPA389 80.061 66.734 89.668 1.00 19.42 A
ATOM 3084 N CYS A 390 81.777 66.410 91.082 1.00 16.56 A
ATOM 3085 CA CYS A 390 81.729 64.963 90.975 1.00 15.81 A
ATOM 3086 CB CYS A 390 82.914 64.352 91.716 1.00 15.87 A
ATOM 3087 SG CYS A 390 83.220 62.633 91.284 1.00 16.80 A
ATOM 3088 C CYS A 390 80.428 64.360 91.482 1.0015.96 A
ATOM 3089 O CYSA390 79.843 63.501 90.830 1.00 14.57 A
ATOM 3090 N VAL A 391 79.980 64.811 92.648 1.00 15.97 A
ATOM 3091 CA VAL A 391 78.741 64.312 93.231 1.00 16.06 A
ATOM 3092 CB VAL A 391 78.481 64.957 94.614 1.00 15.77 A
ATOM 3093 CG1 VAL A 391 77.067 64.664 95.079 1.0014.13 A
ATOM 3094 CG2 VAL A 391 79.485 64.420 95.628 1.00 13.08 A
ATOM 3095 C VAL A 391 77.543 64.574 92.317 1.00 16.40 A
ATOM 3096 O VAL A 391 76.730 63.683 92.082 1.00 16.93 A
ATOM 3097 N VAL A 392 77.437 65.795 91.807 1.00 15.99 A
ATOM 3098 CA VAL A 392 76.333 66.159 90.927 1.00 17.45 A
ATOM 3099 CB VAL A 392 76.352 67.677 90.613 1.00 18.53 A
ATOM 3100 CG1 VALA392 75.402 67.985 89.469 1.00 18.25 A
ATOM 3101 CG2 VAL A 392 75.952 68.469 91.852 1.00 16.48 A
ATOM 3102 C VALA392 76.392 65.362 89.621 1.00 18.80 A
ATOM 3103 O VALA 392 75.357 64.979 89.069 1.00 19.27 A
ATOM 3104 N TYRA393 77.605 65.108 89.136 1.00 17.50 A
ATOM 3105 CA TYR A 393 77.788 64.338 87.907 1.00 17.57 A
ATOM 3106 CB TYRA 393 79.261 64.338 87.495 1.0014.01 A
ATOM 3107 CG TYR A 393 79.565 63.385 86.364 1.00 16.26 A
ATOM 3108 CD1 TYR A 393 79.200 63.685 85.047 1.00 16.03 A
ATOM 3109 CE1 TYRA 393 79.444 62.781 84.005 1.00 16.25 A
ATOM 3110 CD2 TYR A 393 80.183 62.159 86.614 1.0015.57 A
ATOM 3111 CE2 TYR A 393 80.429 61.249 85.585 1.00 16.63 A
ATOM 3112 CZ TYR A 393 80.057 61.565 84.285 1.00 17.63 A
ATOM 3113 OH TYRA393 80.303 60.659 83.276 1.0017.42 A
ATOM 3114 C TYRA393 77.302 62.884 88.043 1.00 17.50 A
ATOM 3115 O TYRA 393 76.662 62.353 87.136 1.00 15.98 A
ATOM 3116 N TRP A 394 77.616 62.228 89.156 1.0015.77 A
ATOM 3117 CA TRP A 394 77.176 60.848 89.316 1.00 16.52 A
ATOM 3118 CB TRP A 394 77.883 60.186 90.497 1.00 14.42 A
ATOM 3119 CG TRPA394 79.283 59.781 90.160 1.00 14.97 A
ATOM 3120 CD2 TRP A 394 79.691 58.557 89.527 1.00 14.89 A
ATOM 3121 CE2 TRP A 394 81.092 58.618 89.365 1.00 14.24 A
ATOM 3122 CE3 TRP A 394 79.005 57.417 89.082 1.00 13.81 A
ATOM 3123 CD1 TRP A 394 80.420 60.511 90.347 1.00 13.99 A
ATOM 3124 NE1 TRP A 394 81.510 59.821 89.872 1.0014.04 A
ATOM 3125 CZ2 TRP A 394 81.827 57.578 88.774 1.00 16.80 A
ATOM 3126 CZ3 TRP A 394 79.737 56.379 88.491 1.00 16.26 A
ATOM 3127 CH2 TRP A 394 81.134 56.471 88.344 1.00 16.79 A
ATOM 3128 C TRP A 394 75.660 60.774 89.475 1.00 17.35 A
ATOM 3129 O TRP A 394 75.025 59.795 89.069 1.00 17.58 A
ATOM 3130 N LEU A 395 75.082 61.818 90.056 1.00 16.41 A
ATOM 3131 CA LEU A 395 73.638 61.879 90.226 1.00 16.64 A
ATOM 3132 CB LEU A 395 73.254 63.067 91.116 1.00 14.73 A
ATOM 3133 CG LEU A 395 73.531 62.902 92.609 1.0014.08 A
ATOM 3134 CD1 LEU A 395 73.205 64.198 93.337 1.00 14.44 A
ATOM 3135 CD2 LEU A 395 72.698 61.745 93.155 1.0013.09 A
ATOM 3136 C LEU A 395 72.973 62.034 88.863 1.00 17.29 A
ATOM 3137 O LEU A 395 72.043 61.303 88.530 1.00 17.30 A
ATOM 3138 N GLU A 396 73.468 62.984 88.077 1.00 16.72 A
ATOM 3139 CA GLU A 396 72.920 63.269 86.757 1.00 18.39 A
ATOM 3140 CB GLU A 396 73.394 64.645 86.285 1.0019.72 A
ATOM 3141 CG GLU A 396 72.809 65.790 87.084 1.0023.71 A
ATOM 3142 CD GLU A 396 73.354 67.138 86.663 1.00 25.21 A
ATOM 3143 OE1 GLU A 396 72.865 68.161 87.182 1.0027.02 A
ATOM 3144 OE2 GLU A 396 74.271 67.179 85.820 1.00 28.37 A
ATOM 3145 C GLU A 396 73.232 62.245 85.680 1.00 18.33 A
ATOM 3146 O GLU A 396 72.340 61.824 84.951 1.00 20.55 A
ATOM 3147 N GLU A 397 74.497 61.852 85.580 1.00 19.11 A
ATOM 3148 CA GLU A 397 74.933 60.888 84.573 1.00 19.47 A
ATOM 3149 CB GLU A 397 76.443 61.028 84.343 1.0019.95 A
ATOM 3150 CG GLU A 397 77.016 60.161 83.222 1.0019.13 A
ATOM 3151 CD GLU A 397 76.635 60.651 81.829 1.0022.59 A
ATOM 3152 OE1 GLU A 397 76.116 61.784 81.707 1.00 21.20 A
ATOM 3153 OE2 GLU A 397 76.872 59.906 80.853 1.00 19.06 A
ATOM 3154 C GLU A 397 74.607 59.432 84.917 1.00 20.93 A
ATOM 3155 O GLU A 397 74.305 58.633 84.017 1.0020.02 A
ATOM 3156 N TYRA398 74.668 59.075 86.202 1.00 17.90 A
ATOM 3157 CA TYR A 398 74.382 57.694 86.589 1.00 18.03 A
ATOM 3158 CB TYRA398 75.587 57.107 87.326 1.0017.72 A
ATOM 3159 CG TYRA 398 76.758 56.913 86.396 1.00 18.31 A
ATOM 3160 CD1 TYR A 398 76.900 55.737 85.661 1.00 18.54 A
ATOM 3161 CE1 TYRA 398 77.941 55.579 84.744 1.0018.51 A
ATOM 3162 CD2 TYR A 398 77.690 57.932 86.194 1.00 18.37 A
ATOM 3163 CE2 TYR A 398 78.734 57.787 85.283 1.0017.54 A
ATOM 3164 CZ TYR A 398 78.853 56.607 84.559 1.00 18.91 A
ATOM 3165 OH TYR A 398 79.875 56.459 83.650 1.00 15.76 A
ATOM 3166 C TYRA398 73.088 57.488 87.373 1.0017.07 A
ATOM 3167 O TYR A 398 72.721 56.362 87.699 1.00 16.65 A
ATOM 3168 N ASN A 399 72.402 58.586 87.668 1.00 17.45 A
ATOM 3169 CA ASN A 399 71.112 58.542 88.344 1.00 17.28 A
ATOM 3170 CB ASN A 399 70.085 57.948 87.369 1.00 17.37 A
ATOM 3171 CG ASN A 399 68.651 58.210 87.783 1.00 18.08 A
ATOM 3172 OD1 ASN A 399 68.343 59.246 88.369 1.00 19.42 A
ATOM 3173 ND2 ASN A 399 67.756 57.281 87.449 1.00 16.87 A
ATOM 3174 C ASNA399 71.080 57.782 89.668 1.00 18.19 A
ATOM 3175 O ASN A 399 70.101 57.090 89.954 1.00 18.22 A
ATOM 3176 N VALA 400 72.134 57.908 90.478 1.00 16.98 A
ATOM 3177 CA VAL A 400 72.161 57.206 91.758 1.00 16.38 A
ATOM 3178 CB VAL A 400 73.611 56.997 92.290 1.00 15.42 A
ATOM 3179 CG1 VAL A 400 74.352 56.055 91.352 1.00 13.5 A
ATOM 3180 CG2 VAL A 400 74.345 58.328 92.427 1.00 13.66 A
ATOM 3181 C VALA400 71.293 57.903 92.799 1.00 15.96 A
ATOM 3182 O VALA400 70.772 58.992 92.558 1.00 13.33 A
ATOM 3183 N ASP A 401 71.150 57.277 93.963 1.00 16.87 A
ATOM 3184 CA ASP A 401 70.268 57.807 94.995 1.00 16.31 A
ATOM 3185 CB ASP A 401 69.187 56.766 95.261 1.00 16.67 A
ATOM 3186 CG ASP A 401 68.484 56.341 93.991 1.00 17.90 A
ATOM 3187 OD1 ASPA401 67.534 57.037 93.579 1.00 21.27 A
ATOM 3188 OD2 ASP A 401 68.897 55.327 93.394 1.00 16.79 A
ATOM 3189 C ASP A 401 70.890 58.241 96.306 1.00 16.10 A
ATOM 3190 O ASP A 401 70.176 58.474 97.281 1.00 16.88 A
ATOM 3191 N GLY A 402 72.211 58.353 96.341 1.00 16.15 A
ATOM 3192 CA GLY A 402 72.858 58.771 97.569 1.00 16.82 A
ATOM 3193 C GLY A 402 74.327 58.421 97.582 1.00 15.69 A
ATOM 3194 O GLY A 402 74.814 57.725 96.689 1.00 14.58 A
ATOM 3195 N PHE A 403 75.030 58.893 98.605 1.00 14.61 A
ATOM 3196 CA PHE A 403 76.458 58.647 98.720 1.00 14.63 A
ATOM 3197 CB PHEA403 77.260 59.858 98.221 1.0014.03 A
ATOM 3198 CG PHE A 403 76.958 60.243 96.806 1.0014.79 A
ATOM 3199 CD1 PHE A 403 75.894 61.095 96.513 1.00 15.70 A
ATOM 3200 CD2 PHE A 403 77.722 59.744 95.764 1.00 15.48 A
ATOM 3201 CE1 PHEA403 75.598 61.445 95.199 1.00 15.45 A
ATOM 3202 CE2 PHE A 403 77.435 60.087 94.436 1.00 16.67 A
ATOM 3203 CZ PHEA403 76.371 60.940 94.156 1.00 16.09 A
ATOM 3204 C PHE A 403 76.903 58.347 100.135 1.00 14.12 A
ATOM 3205 O PHEA403 76.397 58.919 101.096 1.00 14.31 A
ATOM 3206 N ARG A 404 77.865 57.443 100.243 1.00 13.41 A
ATOM 3207 CA ARG A 404 78.439 57.073 101.519 1.00 14.00 A
ATOM 3208 CB ARG A 404 78.475 55.548 101.658 1.00 14.23 A
ATOM 3209 CG ARG A 404 79.144 55.027 202.931 1.00 16.47 A
ATOM 3210 CD ARG A 404 80.606 54.662 102.693 1.00 17.37 A
ATOM 3211 NE ARG A 404 81.158 53.908 103.819 1.00 19.85 A
ATOM 3212 CZ ARG A 404 82.458 53.761 104.063 1.00 21.09 A
ATOM 3213 NH1 ARG A 404 83.359 54.317 103.260 1.00 19.37 A
ATOM 3214 NH2 ARG A 404 82.860 53.066 105.123 1.00 20.82 A
ATOM 3215 C ARG A 404 79.848 57.659 101.444 1.00 15.11 A
ATOM 3216 O ARGA404 80.683 57.208 100.653 1.00 13.52 A
ATOM 3217 N PHE A 405 80.103 58.683 102.249 1.00 14.14 A
ATOM 3218 CA PHE A 405 81.403 59.337 102.230 1.00 15.09 A
ATOM 3219 CB PHEA405 81.227 60.810 102.601 1.00 15.47 A
ATOM 3220 CG PHE A 405 80.420 61.586 101.591 1.00 16.90 A
ATOM 3221 CD1 PHE A 405 81.034 62.149 100.474 1.00 16.76 A
ATOM 3222 CD2 PHE A 405 79.041 61.713 101.731 1.00 17.52 A
ATOM 3223 CE1 PHE A 405 80.292 62.827 99.509 1.00 15.99 A
ATOM 3224 CE2 PHE A 405 78.284 62.388 100.774 1.00 18.46 A
ATOM 3225 CZ PHE A 405 78.914 62.948 99.656 1.00 17.61 A
ATOM 3226 C PHE A 405 82.461 58.677 103.103 1.00 17.10 A
ATOM 3227 O PHEA405 82.413 58.750 104.339 1.0017.10 A
ATOM 3228 N ASP A 406 83.406 58.011 102.441 1.00 16.24 A
ATOM 3229 CA ASP A 406 84.512 57.340 103.111 1.00 16.09 A
ATOM 3230 CB ASP A 406 85.398 56.639 102.070 1.00 16.76 A
ATOM 3231 CG ASP A 406 86.531 55.839 102.696 1.00 19.91 A
ATOM 3232 OD1 ASP A 406 86.251 54.911 103.492 1.00 19.47 A
ATOM 3233 OD2 ASP A 406 87.705 56.139 102.382 1.00 19.50 A
ATOM 3234 C ASP A 406 85.293 58.443 103.829 1.00 17.24 A
ATOM 3235 O ASPA406 85.599 59.471 103.230 1.00 17.71 A
ATOM 3236 N LEU A 407 85.593 58.234 105.108 1.00 16.73 A
ATOM 3237 CA LEU A 407 86.318 59.210 105.923 1.00 15.90 A
ATOM 3238 CB LEU A 407 87.821 59.179 105.610 1.00 15.09 A
ATOM 3239 CG LEU A 407 88.507 57.820 105.815 1.0016.80 A
ATOM 3240 CD1 LEU A 407 90.009 57.962 105.618 1.00 17.44 A
ATOM 3241 CD2 LEU A 407 88.213 57.286 107.215 1.00 16.62 A
ATOM 3242 C LEU A 407 85.769 60.624 105.749 1.00 15.67 A
ATOM 3243 O LEU A 407 86.498 61.555 105.407 1.00 13.69 A
ATOM 3244 N LEU A 408 84.470 60.763 105.999 1.00 16.34 A
ATOM 3245 CA LEU A 408 83.766 62.036 105.909 1.00 17.93 A
ATOM 3246 CB LEU A 408 82.339 61.867 106.440 1.00 19.60 A
ATOM 324? CG LEU A 408 81.168 62.625 105.805 1.0023.34 A
ATOM 3248 CD1 LEU A 408 79.988 62.581 106.767 1.00 21.22 A
ATOM 3249 CD2 LEU A 408 81.548 64.060 105.483 1.00 20.77 A
ATOM 3250 C LEU A 408 84.485 63.090 106.757 1.00 19.21 A
ATOM 3251 O LEU A 408 84.569 64.264 106.380 1.00 18.67 A
ATOM 3252 N GLY A 409 84.994 62.650 107.908 1.00 18.39 A
ATOM 3253 CA GLY A 409 85.690 63.533 108.827 1.00 18.22 A
ATOM 3254 C GLY A 409 86.896 64.266 108.269 1.00 19.56 A
ATOM 3255 O GLY A 409 87.362 65.240 108.868 1.00 18.19 A
ATOM 3256 N ILE A 410 87.413 63.803 107.134 1.00 20.85 A
ATOM 3257 CA ILE A 410 88.560 64.450 106.507 1.00 19.32 A
ATOM 3258 CB ILE A 410 89.297 63.485 105.543 1.00 20.71 A
ATOM 3259 CG2 ILE A 410 90.311 64.257 104.671 1.0021.46 A
ATOM 3260 CG1 ILE A 410 90.004 62.392 106.352 1.00 20.49 A
ATOM 3261 CD1 ILE A 410 91.078 62.910 107.314 1.00 18.71 A
ATOM 3262 C ILE A 410 88.085 65.682 105.740 1.00 19.97 A
ATOM 3263 O ILE A 410 88.835 66.647105.584 1.0021.44 A
ATOM 3264 N LEU A 411 86.838 65.654 105.275 1.00 16.46 A
ATOM 3265 CA LEU A 411 86.283 66.784 104.537 1.00 17.26 A
ATOM 3266 CB LEU A 411 85.068 66.343 103.711 1.00 17.87 A
ATOM 3267 CG LEU A 411 85.329 65.582 102.406 1.00 19.56 A
ATOM 3268 CD1 LEU A 411 86.023 64.259 102.693 1.00 18.58 A
ATOM 3269 CD2 LEU A 411 84.003 65.338 101.698 1.00 18.61 A
ATOM 3270 C LEU A 411 85.873 67.917 105.477 1.00 16.62 A
ATOM 3271 O LEU A 411 85.561 67.681 106.649 1.00 16.32 A
ATOM 3272 N ASP A 412 85.877 69.145 104.962 1.00 15.18 A
ATOM 3273 CA ASP A 412 85.494 70.301 105.761 1.00 15.60 A
ATOM 3274 CB ASP A 412 86.241 71.562 105.295 1.00 16.14 A
ATOM 3275 CG ASP A 412 85.793 72.037 103.925 1.00 18.12 A
ATOM 3276 OD1 ASP A 412 86.118 71.362 102.924 1.00 16.76 A
ATOM 3277 OD2 ASP A 412 85.106 73.082 103.855 1.00 18.35 A
ATOM 3278 C ASP A 412 83.981 70.515 105.660 1.00 17.53 A
ATOM 3279 O ASP A 412 83.365 70.227 104.629 1.00 15.73 A
ATOM 3280 N ILE A 413 83.392 71.028 106.736 1.00 18.07 A
ATOM 3281 CA ILE A 413 8 1.954 71.257 106.798 1.00 19.14 A
ATOM 3282 CB ILE A 413 81.556 71.860 108.173 1.00 18.60 A
ATOM 3283 CG2 ILE A 413 80.107 72.315 108.159 1.00 16.88 A
ATOM 3284 CG1 ILE A 413 81.752 70.805 109.274 1.00 20.50 A
ATOM 3285 CD1 ILE A 413 81.435 71.297 110.692 1.00 19.24 A
ATOM 3286 C ILEA413 81.389 72.124 105.672 1.00 20.58 A
ATOM 3287 O ILE A 413 80.342 71.799 105.104 1.00 21.05 A
ATOM 3288 N ASP A 414 82.067 73.217 105.335 1.00 20.55 A
ATOM 3289 CA ASP A 414 81.561 74.083 104.276 1.00 19.78 A
ATOM 3290 CB ASP A 414 82.440 75.323 104.124 1.00 20.57 A
ATOM 3291 CG ASP A 414 82.231 76.316 105.252 1.0024.99 A
ATOM 3292 OD1 ASP A 414 82.999 77.295 105.341 1.00 25.66 A
ATOM 3293 OD2 ASP A 414 81.290 76.114 106.053 1.00 26.79 A
ATOM 3294 C ASP A 414 81.447 73.345 102.955 1.00 19.23 A
ATOM 3295 O ASP A 414 80.464 73.512 102.236 1.00 20.48 A
ATOM 3296 N THR A 415 82.440 72.520 102.638 1.00 17.96 A
ATOM 3297 CA THR A 415 82.398 71.758 101.399 1.00 16.84 A
ATOM 3298 CB THR A 415 83.694 70.955 101.190 1.0016.99 A
ATOM 3299 OG1 THRA415 84.773 71.861 100.925 1.00 15.15 A
ATOM 3300 CG2 THR A 415 83.540 69.980 100.011 1.00 15.78 A
ATOM 3301 C THR A 415 81.209 70.798 101.422 1.00 16.78 A
ATOM 3302 O THR A 415 80.470 70.681 100.444 1.00 17.54 A
ATOM 3303 N VAL A 416 81.026 70.124 102.550 1.00 15.65 A
ATOM 3304 CA VAL A 416 79.931 69.179 102.710 1.00 16.38 A
ATOM 3305 CB VAL A 416 79.989 68.520 104.104 1.00 15.16 A
ATOM 3306 CG1 VAL A 416 78.754 67.687 104.342 1.00 15.90 A
ATOM 3307 CG2 VAL A 416 81.234 67.647 104.203 1.00 14.37 A
ATOM 3308 C VAL A 416 78.574 69.860 102.516 1.00 17.63 A
ATOM 3309 O VAL A 416 77.715 69.354 101.792 1.00 15.82 A
ATOM 3310 N LEU A 417 78.390 71.012 103.157 1.00 18.88 A
ATOM 3311 CA LEU A 417 77.138 71.758 103.051 1.00 19.28 A
ATOM 3312 CB LEU A 417 77.108 72.878 104.093 1.00 18.45 A
ATOM 3313 CG LEU A 417 77.084 72.411 105.548 1.00 17.35 A
ATOM 3314 CD1 LEU A 417 77.168 73.612 106.480 1.00 15.78 A
ATOM 3315 CD2 LEU A 417 75.807 71.628 105.801 1.00 18.65 A
ATOM 3316 C LEU A 417 76.963 72.342 101.650 1.00 19.25 A
ATOM 3317 O LEU A 417 75.847 72.532 101.169 1.00 19.28 A
ATOM 3318 N TYR A 418 78.078 72.622 100.992 1.00 20.11 A
ATOM 3319 CA TYR A 418 78.031 73.166 99.651 1.00 19.92 A
ATOM 3320 CB TYR A 418 79.411 73.698 99.266 1.00 21.11 A
ATOM 3321 CG TYRA418 79.478 74.282 97.881 1.0021.94 A
ATOM 3322 CD1 TYR A 418 80.136 73.611 96.860 1.00 20.04 A
ATOM 3323 CE1 TYR A 418 80.207 74.139 95.583 1.00 22.46 A
ATOM 3324 CD2 TYR A 418 78.881 75.512 97.590 1.0023.96 A
ATOM 3325 CE2 TYR A 418 78.944 76.052 96.308 1.0023.71 A
ATOM 3326 CZ TYRA418 79.612 75.355 95.311 1.00 23.94 A
ATOM 3327 OH TYR A 418 79.681 75.863 94.037 1.00 26.12 A
ATOM 3328 C TYR A 418 77.571 72.088 98.668 1.00 20.02 A
ATOM 3329 O TYR A 418 76.691 72.332 97.841 1.00 18.87 A
ATOM 3330 N MET A 419 78.140 70.888 98.769 1.00 19.88 A
ATOM 3331 CA MET A 419 77.746 69.821 97.859 1.00 20.23 A
ATOM 3332 CB MET A 419 78.760 68.663 97.875 1.00 19.40 A
ATOM 3333 CG MET A 419 78.568 67.598 98.938 1.00 22.37 A
ATOM 3334 SD MET A 419 77.277 66.340 98.663 1.0018.68 A
ATOM 3335 CE MET A 419 77.245 65.639 100.319 1.00 21.87 A
ATOM 3336 C MET A 419 76.354 69.311 98.194 1.00 20.51 A
ATOM 3337 O MET A 419 75.606 68.931 97.299 1.00 20.94 A
ATOM 3338 N LYSA420 76.003 69.311 99.477 1.0020.80 A
ATOM 3339 CA LYS A 420 74.685 68.841 99.890 1.00 21.77 A
ATOM 3340 CB LYS A 420 74.492 68.991 101.399 1.0020.34 A
ATOM 3341 CG LYS A 420 73.159 68.429 101.878 1.00 24.62 A
ATOM 3342 CD LYS A 420 72.882 68.748 103.333 1.00 26.78 A
ATOM 3343 CE LYS A 420 72.597 70.225 103.524 1.0027.27 A
ATOM 3344 NZ LYS A 420 72.115 70.490 104.903 1.00 28.76 A
ATOM 3345 C LYS A 420 73.620 69.654 99.172 1.00 22.63 A
ATOM 3346 O LYSA420 72.663 69.104 98.630 1.00 24.08 A
ATOM 3347 N GLU A 421 73.801 70.970 99.172 1.00 23.87 A
ATOM 3348 CA GLU A 421 72.869 71.879 98.521 1.00 25.99 A
ATOM 3349 CB GLU A 421 73.373 73.316 98.646 1.00 30.40 A
ATOM 3350 CG GLU A 421 72.433 74.370 98.086 1.0040.13 A
ATOM 3351 CD GLU A 421 72.968 75.781 98.286 1.0047.33 A
ATOM 3352 OE1 GLU A 421 73.985 76.138 97.644 1.00 51.03 A
ATOM 3353 OE2 GLU A 421 72.379 76.533 99.096 1.0050.22 A
ATOM 3354 C GLU A 421 72.696 71.515 97.052 1.00 24.96 A
ATOM 3355 O GLU A 421 71.576 71.323 96.578 1.0025.69 A
ATOM 3356 N LYS A 422 73.806 71.402 96.333 1.0024.61 A
ATOM 3357 CA LYS A 422 73.744 71.067 94.915 1.00 25.81 A
ATOM 3358 CB LYS A 422 75.119 71.233 94.270 1.0027.30 A
ATOM 3359 CG LYS A 422 75.617 72.667 94.232 1.00 31.88 A
ATOM 3360 CD LYS A 422 76.911 72.764 93.444 1.0035.98 A
ATOM 3361 CE LYS A 422 77.305 74.212 93.203 1.0038.95 A
ATOM 3362 NZ LYS A 422 78.555 74.304 92.382 1.0041.96 A
ATOM 3363 C LYS A 422 73.234 69.652 94.664 1.00 25.24 A
ATOM 3364 O LYS A 422 72.524 69.406 93.690 1.0024.18 A
ATOM 3365 N ALA A 423 73.599 68.726 95.543 1.00 24.46 A
ATOM 3366 CA ALAA423 73.185 67.335 95.396 1.00 24.59 A
ATOM 3367 CB ALA A 423 73.991 66.444 96.342 1.00 22.74 A
ATOM 3368 C ALAA423 71.687 67.138 95.634 1.0024.96 A
ATOM 3369 O ALA A 423 71.024 66.448 94.857 1.00 25.33 A
ATOM 3370 N THRA424 71.152 67.730 96.702 1.00 23.63 A
ATOM 3371 CA THRA424 69.726 67.585 96.989 1.00 24.64 A
ATOM 3372 CB THR A 424 69.364 68.081 98.407 1.00 23.46 A
ATOM 3373 OG1 THR A 424 69.746 69.454 98.546 1.00 22.46 A
ATOM 3374 CG2 THR A 424 70.071 67.236 99.464 1.00 22.28 A
ATOM 3375 C THRA424 68.869 68.340 95.975 1.00 24.96 A
ATOM 3376 O THRA424 67.726 67.966 95.718 1.00 24.96 A
ATOM 3377 N LYS A 425 69.413 69.403 95.395 1.0026.64 A
ATOM 3378 CA LYS A 425 68.654 70.153 94.402 1.00 27.10 A
ATOM 3379 CB LYS A 425 69.314 71.498 94.100 1.0031.06 A
ATOM 3380 CG LYS A 425 68.519 72.332 93.102 1.0036.42 A
ATOM 3381 CD LYS A 425 69.322 73.507 92.581 1.00 40.53 A
ATOM 3382 CE LYS A 425 68.581 74.218 91.457 1.0042.23 A
ATOM 3383 NZ LYS A 425 69.433 75.256 90.798 1.0044.83 A
ATOM 3384 C LYSA425 68.575 69.334 93.121 1.0025.58 A
ATOM 3385 O LYSA425 67.573 69.365 92.415 1.00 26.07 A
ATOM 3386 N ALA A 426 69.638 68.597 92.823 1.0023.84 A
ATOM 3387 CA ALAA426 69.660 67.775 91.620 1.00 24.17 A
ATOM 3388 CB ALAA426 71.099 67.457 91.224 1.00 18.85 A
ATOM 3389 C ALAA426 68.879 66.479 91.817 1.00 23.90 A
ATOM 3390 O ALA A 426 68.396 65.893 90.857 1.00 25.17 A
ATOM 3391 N LYS A 427 68.736 66.044 93.064 1.0023.91 A
ATOM 3392 CA LYS A 427 68.042 64.793 93.351 1.00 24.77 A
ATOM 3393 CB LYS A 427 69.030 63.626 93.237 1.0028.09 A
ATOM 3394 CG LYS A 427 68.676 62.577 92.201 1.00 31.37 A
ATOM 3395 CD LYS A 427 67.559 61.674 92.673 1.0030.44 A
ATOM 3396 CE LYS A 427 67.163 60.716 91.567 1.00 35.04 A
ATOM 3397 NZ LYS A 427 68.324 59.922 91.079 1.0036.91 A
ATOM 3398 C LYS A 427 67.454 64.812 94.753 1.0024.07 A
ATOM 3399 O LYS A 427 68.095 64.374 95.708 1.00 24.11 A
ATOM 3400 N PRO A 428 66.220 65.311 94.896 1.00 23.50 A
ATOM 3401 CD PRO A 428 65.371 65.960 93.88 1.00 23.01 A
ATOM 3402 CA PRO A 428 65.592 65.362 96.221 1.00 22.98 A
ATOM 3403 CB PRO A 428 64.245 66.031 95.938 1.00 22.93 A
ATOM 3404 CG PRO A428 64.534 66.895 94.728 1.0022.90 A
ATOM 3405 C PRO A 428 65.429 63.980 96.859 1.0022.38 A
ATOM 3406 O PRO A 428 65.126 63.003 96.175 1.00 21.92 A
ATOM 3407 N GLY A 429 65.651 63.906 98.168 1.0021.86 A
ATOM 3408 CA GLYA429 65.493 62.650 98.882 1.0022.08 A
ATOM 3409 C GLY A 429 66.705 61.743 98.997 1.00 21.49 A
ATOM 3410 O GLY A 429 66.655 60.744 99.712 1.00 22.95 A
ATOM 3411 N ILEA430 67.793 62.073 98.309 1.0020.43 A
ATOM 3412 CA ILEA430 68.992 61.242 98.358 1.0019.61 A
ATOM 3413 CB ILEA430 70.075 61.758 97.379 1.00 21.63 A
ATOM 3414 CG2 ILEA430 69.523 61.778 95.961 1.00 20.32 A
ATOM 3415 CG1 ILEA430 70.530 63.160 97.786 1.00 22.67 A
ATOM 3416 CD1 ILE A 430 71.617 63.728 96.882 1.00 24.11 A
ATOM 3417 C ILE A 430 69.605 61.142 99.757 1.00 18.32 A
ATOM 3418 O ILE A 430 69.478 62.049 100.577 1.00 17.69 A
ATOM 3419 N LEU A 431 70.276 60.027 100.016 1.00 17.39 A
ATOM 3420 CA LEU A 431 70.919 59.797 101.303 1.00 17.89 A
ATOM 3421 CB LEU A 431 70.851 58.309 101.667 1.00 18.22 A
ATOM 3422 CG LEU A 431 69.442 57.711 101.766 1.00 17.37 A
ATOM 3423 CD1 LEU A 431 69.519 56.244 102.212 1.00 17.54 A
ATOM 3424 CD2 LEU A 431 68.630 58.530 102.758 1.00 17.19 A
ATOM 3425 C LEU A 431 72.373 60.267 101.270 1.00 17.03 A
ATOM 3426 O LEU A 431 73.116 59.979 100.332 1.00 17.65 A
ATOM 3427 N LEU A 432 72.762 61.011 102.296 1.00 16.75 A
ATOM 3428 CA LEU A 432 74.116 61.534 102.399 1.00 15.70 A
ATOM 3429 CB LEU A 432 74.130 63.046 102.126 1.00 13.67 A
ATOM 3430 CG LEU A 432 73.618 63.518 100.755 1.00 15.80 A
ATOM 3431 CD1 LEU A 432 73.627 65.044 100.690 1.00 11.57 A
ATOM 3432 CD2 LEU A 432 74.497 62.937 99.643 1.00 12.70 A
ATOM 3433 C LEU A 432 74.570 61.254 103.819 1.00 16.54 A
ATOM 3434 O LEU A 432 74.010 61.795 104.781 1.00 15.98 A
ATOM 3435 N PHE A 433 75.580 60.401 103.948 1.00 14.76 A
ATOM 3436 CA PHE A 433 76.098 60.036 105.257 1.00 15.24 A
ATOM 3437 CB PHEA433 75.211 58.955 105.890 1.00 14.14 A
ATOM 3438 CG PHEA433 75.057 57.723 105.039 1.00 13.06 A
ATOM 3439 CD1 PHE A 433 74.104 57.674 104.023 1.00 13.21 A
ATOM 3440 CD2 PHE A 433 75.867 56.613 105.249 1.00 11.20 A
ATOM 3441 CE1 PHE A 433 73.955 56.538 103.229 1.00 12.57 A
ATOM 3442 CE2 PHE A 433 75.729 55.467 104.459 1.00 15.46 A
ATOM 3443 CZ PHE A 433 74.768 55.433 103.446 1.0015.00 A
ATOM 3444 C PHE A 433 77.18 59.508 105.099 1.00 15.32 A
ATOM 3445 O PHEA433 77.998 59.324 103.979 1.00 15.70 A
ATOM 3446 N GLY A 434 78.185 59.269 106.221 1.00 13.88 A
ATOM 3447 CA GLY A 434 79.543 58.764 106.165 1.00 15.62 A
ATOM 3448 C GLYA434 80.156 58.540 107.532 1.00 15.85 A
ATOM 3449 O GLY A 434 79.496 58.675 108.564 1.00 15.54 A
ATOM 3450 N GLU A 435 81.432 58.189 107.540 1.00 17.62 A
ATOM 3451 CA GLU A 435 82.133 57.954 108.789 1.00 22.16 A
ATOM 3452 CB GLU A 435 83.154 56.824 108.606 1.00 23.18 A
ATOM 3453 CG GLU A 435 83.907 56.872 107.284 1.00 29.20 A
ATOM 3454 CD GLU A 435 84.688 55.596 106.986 1.0029.58 A
ATOM 3455 OE1 GLU A 435 85.422 55.585 105.976 1.00 24.97 A
ATOM 3456 OE2 GLU A435 84.566 54.609 107.752 1.00 33.01 A
ATOM 3457 C GLU A 435 82.808 59.247 109.241 1.00 23.01 A
ATOM 3458 O GLU A 435 83.876 59.613 108.748 1.00 23.51 A
ATOM 3459 N GLY A 436 82.157 59.951 110.164 1.00 21.99 A
ATOM 3460 CA GLY A 436 82.709 61.194 110.667 1.00 22.80 A
ATOM 3461 C GLY A 436 83.658 60.970 111.828 1.00 25.38 A
ATOM 3462 O GLY A 436 83.493 61.560 112.900 1.0025.12 A
ATOM 3463 N TRP A 437 84.648 60.105 111.627 1.0026.62 A
ATOM 3464 CA TRP A 437 85.627 59.830 112.671 1.0030.84 A
ATOM 3465 CB TRP A 437 86.557 58.664 112.286 1.00 33.31 A
ATOM 3466 CG TRP A 437 85.876 57.396 111.822 1.00 38.17 A
ATOM 3467 CD2 TRP A 437 84.646 56.834 112.310 1.00 39.08 A
ATOM 3468 CE2 TRP A 437 84.396 55.661 111.559 1.00 40.71 A
ATOM 3469 CE3 TRP A 437 83.733 57.205 113.306 1.00 40.77 A
ATOM 3470 CD1 TRP A 437 86.308 56.561 110.832 1.00 38.77 A
ATOM 3471 NE1 TRP A 437 85.425 55.520 110.665 1.0040.64 A
ATOM 3472 CZ2 TRP A 437 83.268 54.858 111.770 1.00 40.61 A
ATOM 3473 CZ3 TRP A 437 82.610 56.404 113.517 1.00 42.57 A
ATOM 3474 CH2 TRP A 437 82.390 55.244 112.750 1.00 41.91 A
ATOM 3475 C TRPA437 86.476 61.084 112.817 1.00 30.62 A
ATOM 3476 O TRP A 437 86.6.96 61.808 111.843 1.00 28.36 A
ATOM 3477 N ASPA438 86.941 61.351 114.032 1.00 32.21 A
ATOM 3478 CA ASP A 438 87.803 62.502 114.254 1.00 33.83 A
ATOM 3479 CB ASP A 438 87.793 62.924 115.724 1.00 38.39 A
ATOM 3480 CG ASP A 438 88.550 64.221 115.958 1.0042.61 A
ATOM 3481 OD1 ASP A 438 88.036 65.292 115.561 1.00 42.92 A
ATOM 3482 OD2 ASP A 438 89.665 64.166 116.522 1.00 45.18 A
ATOM 3483 C ASPA438 89.194 62.008 113.864 1.00 32.35 A
ATOM 3484 O ASP A 438 89.716 61.073 114.470 1.00 31.60 A
ATOM 3485 N LEU A 439 89.780 62.616 112.838 1.00 30.53 A
ATOM 3486 CA LEU A 439 91.094 62.202 112.371 1.00 29.09 A
ATOM 3487 CB LEU A 439 90.989 61.680 110.938 1.00 31.14 A
ATOM 3488 CG LEU A 439 90.092 60.453 110.711 1.00 33.92 A
ATOM 3489 CD1 LEU A 439 89.798 60.303 109.223 1.00 34.17 A
ATOM 3490 CD2 LEU A 439 90.773 59.196 111.255 1.00 31.36 A
ATOM 3491 C LEU A 439 92.085 63.350 112.440 1.00 28.26 A
ATOM 3492 O LEU A 439 91.707 64.521 112.359 1.0028.68 A
ATOM 3493 N ALAA440 93.360 63.01 112.581 1.0026.37 A
ATOM 3494 CA ALA A 440 94.405 64.021 112.678 1.00 26.65 A
ATOM 3495 CB ALA A 440 95.659 63.419 113.323 1.00 26.13 A
ATOM 3496 C ALAA440 94.767 64.657 111.340 1.0025.71 A
ATOM 3497 O ALA A 440 95.870 64.449 110.828 1.0026.38 A
ATOM 3498 N THR A 441 93.839 65.419 110.769 1.00 22.80 A
ATOM 3499 CA THR A 441 94.113 66.103 109.513 1.00 22.63 A
ATOM 3500 CB THR A 441 93.006 65.843 108.457 1.00 22.56 A
ATOM 3501 OG1 THR A 441 93.416 66.383 107.196 1.00 20.67 A
ATOM 3502 CG2 THR A 441 91.691 66.470 108.88 1.00 22.07 A
ATOM 3503 C THRA441 94.198 67.59 109.868 1.0022.39 A
ATOM 3504 O THR A 441 93.508 68.047 110.772 1.00 24.07 A
ATOM 3505 N PROA442 95.058 68.335 109.177 1.00 23.92 A
ATOM 3506 CD PRO A 442 95.997 67.923 108.120 1.00 23.67 A
ATOM 3507 CA PRO A 442 95.197 69.766 109.479 1.00 24.46 A
ATOM 3508 CB PRO A 442 96.433 70.168 108.674 1.00 22.79 A
ATOM 3509 CG PRO A 442 96.390 69.244 107.506 1.00 25.25 A
ATOM 3510 C PRO A 442 93.995 70.673 109.211 1.00 24.94 A
ATOM 3511 O PROA442 93.954 71.401 108.221 1.00 27.69 A
ATOM 3512 N LEU A 443 93.019 70.629 110.110 1.00 24.91 A
ATOM 3513 CA LEU A 443 91.824 71.463 110.009 1.00 23.07 A
ATOM 3514 CB LEU A 443 90.686 70.722 109.310 1.00 23.08 A
ATOM 3515 CG LEU A 443 90.632 70.539 107.792 1.00 25.50 A
ATOM 3516 CD1 LEU A 443 89.380 69.721 107.456 1.00 22.58 A
ATOM 3517 CD2 LEU A 443 90.586 71.892 107.088 1.00 20.72 A
ATOM 3518 C LEU A 443 91.363 71.796 111.418 1.00 22.12 A
ATOM 3519 O LEU A 443 91.413 70.946 112.294 1.00 20.97 A
ATOM 3520 N PRO A 444 90.928 73.043 111.660 1.00 22.27 A
ATOM 3521 CD PRO A 444 90.958 74.234 110.789 1.00 20.74 A
ATOM 3522 CA PRO A 444 90.464 73.380 113.013 1.00 21.74 A
ATOM 3523 CB PRO A 444 89.947 74.803 112.853 1.00 20.34 A
ATOM 3524 CG PRO A 444 90.867 75.367 111.789 1.00 21.71 A
ATOM 3525 C PRO A 444 89.348 72.385 113.337 1.00 22.55 A
ATOM 3526 O PRO A 444 88.541 72.060 112.467 1.00 20.30 A
ATOM 3527 N HIS A 445 89.301 71.900 114.572 1.0023.30 A
ATOM 3528 CA HIS A 445 88.296 70.913 114.961 1.00 24.42 A
ATOM 3529 CB HIS A 445 88.407 70.621 116.463 1.00 24.47 A
ATOM 3530 CG HIS A 445 87.697 71.612 117.327 1.00 29.47 A
ATOM 3531 CD2 HIS A 445 88.101 72.798 117.843 1.00 30.25 A
ATOM 3532 ND1 HIS A 445 86.389 71.444 117.730 1.00 30.17 A
ATOM 3533 CE1 HIS A 445 86.018 72.484 118.457 1.00 32.22 A
ATOM 3534 NE2 HIS A 445 87.038 73.320 118.540 1.00 33.16 A
ATOM 3535 C HIS A 445 86.834 71.237 114.591 1.00 24.61 A
ATOM 3536 O HIS A 445 86.082 70.325 114.248 1.00 23.80 A
ATOM 3537 N GLU A 446 86.430 72.509 114.633 1.00 23.75 A
ATOM 3538 CA GLU A 446 85.040 72.861 114.307 1.00 24.96 A
ATOM 3539 CB GLU A 446 84.654 74.252 114.838 1.00 26.12 A
ATOM 3540 CG GLU A 446 85.530 74.795 115.930 1.00 30.15 A
ATOM 3541 CD GLUA446 86.658 75.629 115.384 1.0028.36 A
ATOM 3542 OE1 GLU A 446 86.538 76.873 115.402 1.00 28.79 A
ATOM 3543 OE2 GLU A 446 87.657 75.039 114.930 1.0027.58 A
ATOM 3544 C GLU A 446 84.734 72.846 112.819 1.00 23.77 A
ATOM 3545 O GLU A 446 83.583 73.007 112.430 1.00 22.94 A
ATOM 3546 N GLN A 447 85.761 72.665 111.993 1.0023.78 A
ATOM 3547 CA GLN A 447 85.589 72.652 110.542 1.00 23.71 A
ATOM 3548 CB GLN A 447 86.709 73.464 109.876 1.0024.90 A
ATOM 3549 CG GLN A 447 86.530 74.971 109.990 1.0026.29 A
ATOM 3550 CD GLN A 447 87.707 75.749 109.424 1.00 30.00 A
ATOM 3551 OE1 GLN A 447 88.282 75.379 108.398 1.0030.97 A
ATOM 3552 NE2 GLN A 447 88.060 76.846 110.085 1.00 31.61 A
ATOM 3553 C GLN A 447 85.538 71.251 109.938 1.0023.09 A
ATOM 3554 O GLN A 447 85.218 71.088 108.762 1.00 23.43 A
ATOM 3555 N LYS A 448 85.860 70.244 110.740 1.00 21.89 A
ATOM 3556 CA LYS A 448 85.835 68.864 110.273 1.00 21.10 A
ATOM 3557 CB LYS A 448 86.631 67.968 111.232 1.0021.40 A
ATOM 3558 CG LYS A 448 88.111 68.303 111.314 1.0023.02 A
ATOM 3559 CD LYS A 448 88.849 67.419 112.321 1.00 24.65 A
ATOM 3560 CE LYS A 448 90.344 67.728 112.318 1.00 26.23 A
ATOM 3561 NZ LYS A 448 91.116 66.930 113.316 1.0028.05 A
ATOM 3562 C LYS A 448 84.392 68.359 110.167 1.00 20.42 A
ATOM 3563 O LYS A 448 83.565 68.620 111.040 1.0019.05 A
ATOM 3564 N ALAA449 84.083 67.654 109.085 1.00 20.59 A
ATOM 3565 CA ALA A 449 82.741 67.117 108.91 1.00 20.03 A
ATOM 3566 CB ALA A 449 82.477 66.823 107.440 1.00 21.12 A
ATOM 3567 C ALAA449 82.654 65.842 109.736 1.00 20.82 A
ATOM 3568 0 ALAA449 82.285 64.789 109.232 1.0020.40 A
ATOM 3569 N ALAA450 83.006 65.951 111.016 1.00 22.73 A
ATOM 3570 CA ALA A 450 82.992 64.814 111.929 1.0022.48 A
ATOM 3571 CB ALA A 450 84.218 64.860 112.835 1.00 20.62 A
ATOM 3572 C ALAA450 81.726 64.741 112.779 1.0023.60 A
ATOM 3573 O ALAA450 80.994 65.725 112.928 1.0023.26 A
ATOM 3574 N LEU A 451 81.503 63.562 113.350 1.00 24.16 A
ATOM 3575 CA LEU A 451 80.349 63.281 114.190 1.00 24.54 A
ATOM 3576 CB LEU A 451 80.543 61.928 114.867 1.00 26.54 A
ATOM 3577 CG LEU A 451 79.372 60.945 114.890 1.00 29.54 A
ATOM 3578 CD1 LEU A 451 79.777 59.726115.710 1.0029.58 A
ATOM 3579 CD2 LEU A 451 78.132 61.607 115.480 1.00 29.33 A
ATOM 3580 C LEU A 451 80.123 64.355 115.252 1.00 24.51 A
ATOM 3581 O LEU A 451 78.994 64.768 115.503 1.00 24.49 A
ATOM 3582 N ALA A 452 81.203 64.806 115.874 1.0024.59 A
ATOM 3583 CA ALA A 452 81.107 65.823 116.910 1.00 23.54 A
ATOM 3584 CB ALAA452 82.491 66.119 117.480 1.00 24.75 A
ATOM 3585 C ALA A 452 80.458 67.110 116.416 1.00 23.49 A
ATOM 3586 O ALAA452 79.969 67.898 117.216 1.00 24.21 A
ATOM 3587 N ASN A 453 80.447 67.331 115.105 1.00 22.76 A
ATOM 3588 CA ASN A 453 79.840 68.546 114.573 1.00 20.96 A
ATOM 3589 CB ASN A 453 80.817 69.264 113.629 1.00 20.77 A
ATOM 3590 CG ASN A 453 82.100 69.695 114.331 1.00 23.41 A
ATOM 3591 OD1 ASN A 453 82.057 70.270 115.417 1.00 22.82 A
ATOM 3592 ND2 ASN A 453 83.244 69.421 113.711 1.00 19.55 A
ATOM 3593 C ASN A 453 78.510 68.284 113.857 1.00 19.88 A
ATOM 3594 O ASNA453 77.977 69.161 113.179 1.0017.74 A
ATOM 3595 N ALAA454 77.966 67.082 114.029 1.00 19.68 A
ATOM 3596 CA ALA A 454 76.698 66.722 113.396 1.00 18.96 A
ATOM 3597 CB ALA A 454 76.164 65.443 114.009 1.00 18.15 A
ATOM 3598 C ALA A 454 75.632 67.825 113.475 1.00 20.44 A
ATOM 3599 O ALAA454 74.864 68.012 112.529 1.0020.94 A
ATOM 3600 N PRO A 455 75.565 68.567 114.604 1.00 21.54 A
ATOM 3601 CD PRO A 455 76.258 68.346 115.890 1.00 21.08 A
ATOM 3602 CA PRO A 455 74.563 69.635 114.731 1.0022.22 A
ATOM 3603 CB PRO A 455 74.869 70.240 116.099 1.00 22.83 A
ATOM 3604 CG PRO A 455 75.339 69.045 116.886 1.00 22.39 A
ATOM 3605 C PROA455 74.619 70.673 113.616 1.00 23.81 A
ATOM 3606 O PRO A 455 73.595 71.244 113.244 1.00 24.72 A
ATOM 3607 N ARG A 456 75.812 70.915 113.081 1.00 24.90 A
ATOM 3608 CA ARG A 456 75.970 71.886 112.005 1.00 24.81 A
ATOM 3609 CB ARG A 456 77.348 72.545 112.069 1.00 26.77 A
ATOM 3610 CG ARG A 456 77.503 73.536 113.217 1.00 31.61 A
ATOM 3611 CD ARG A 456 78.639 74.497 112.936 1.00 34.64 A
ATOM 3612 NE ARG A 456 78.403 75.246 111.702 1.00 37.22 A
ATOM 3613 CZ ARG A 456 79.282 75.358 110.709 1.00 37.62 A
ATOM 3614 NH1 ARG A 456 78.969 76.063 109.628 1.00 35.58 A
ATOM 3615 NH2 ARG A 456 80.470 74.765 110.794 1.00 36.01 A
ATOM 3616 C ARG A 456 75.768 71.296 110.620 1.00 24.53 A
ATOM 3617 O ARGA456 75.823 72.019 109.628 1.0023.79 A
ATOM 3618 N META457 75.535 69.987 110.544 1.00 23.40 A
ATOM 3619 CA MET A 457 75.335 69.338 109.253 1.00 22.21 A
ATOM 3620 CB MET A 457 76.479 68.363 108.965 1.00 21.94 A
ATOM 3621 CG MET A 457 77.811 69.035 108.675 1.0022.17 A
ATOM 3622 SD MET A 457 79.151 67.839 108.521 1.0022.57 A
ATOM 3623 CE MET A 457 79.471 67.492 110.262 1.0020.64 A
ATOM 3624 C MET A 457 74.001 68.610 109.127 1.0022.60 A
ATOM 3625 O MET A 457 73.958 67.400 108.887 1.0022.20 A
ATOM 3626 N PRO A 458 72.887 69.340 109.292 1.0022.29 A
ATOM 3627 CD PRO A 458 72.721 70.798 109.448 1.00 22.58 A
ATOM 3628 CA PRO A 458 71.590 68.670 109.170 1.0022.05 A
ATOM 3629 CB PRO A 458 70.594 69.797 109.449 1.0021.79 A
ATOM 3630 CG PRO A 458 71.315 71.018 108.932 1.00 21.57 A
ATOM 3631 C PROA458 71.469 68.105 107.757 1.0020.93 A
ATOM 3632 O PROA458 71.913 68.731 106.801 1.0022.79 A
ATOM 3633 N GLY A 459 70.891 66.917 107.629 1.0020.34 A
ATOM 3634 CA GLY A 459 70.747 66.315 106.318 1.00 17.44 A
ATOM 3635 C GLY A 459 71.886 65.356 106.014 1.00 17.83 A
ATOM 3636 O GLY A 459 71.892 64.691 104.975 1.00 19.42 A
ATOM 3637 N ILE A 460 72.861 65.294 106.914 1.0014.97 A
ATOM 3638 CA ILE A 460 74.001 64.400 106.755 1.00 13.54 A
ATOM 3639 CB ILE A 460 75.357 65.164 106.827 1.00 13.07 A
ATOM 3640 CG2 ILE A 460 76.499 64.211 106.491 1.00 11.30 A
ATOM 3641 CG1 ILE A 460 75.373 66.352 105.856 1.00 12.01 A
ATOM 3642 CD1 ILE A 460 75.364 65.970 104.377 1.00 9.34 A
ATOM 3643 C ILE A 460 73.987 63.376 107.891 1.00 13.56 A
ATOM 3644 O ILE A 460 73.896 63.747 109.064 1.00 12.55 A
ATOM 3645 N GLY A 461 74.083 62.095 107.542 1.00 13.86 A
ATOM 3646 CA GLY A 461 74.092 61.046 108.550 1.00 14.06 A
ATOM 3647 C GLY A 461 75.496 60.557 108.895 1.00 16.95 A
ATOM 3648 O GLY A 461 76.462 60.820 108.162 1.00 16.89 A
ATOM 3649 N PHEA462 75.618 59.841 110.011 1.00 15.00 A
ATOM 3650 CA PHE A 462 76.912 59.321 110.442 1.00 15.91 A
ATOM 3651 CB PHE A 462 77.507 60.211 111.547 1.00 16.30 A
ATOM 3652 CG PHE A 462 77.651 61.650 111.155 1.00 16.69 A
ATOM 3653 CD1 PHE A 462 76.554 62.509 111.191 1.00 16.81 A
ATOM 3654 CD2 PHE A 462 78.872 62.139 110.701 1.00 15.23 A
ATOM 3655 CE1 PHE A 462 76.671 63.837 110.775 1.0017.78 A
ATOM 3656 CE2 PHE A 462 79.003 63.464 110.282 1.00 17.14 A
ATOM 3657 CZ PHE A 462 77.898 64.315 110.318 1.00 17.75 A
ATOM 3658 C PHE A 462 76.806 57.894 110.972 1.00 15.65 A
ATOM 3659 O PHE A 462 75.836 57.542 111.638 1.00 15.28 A
ATOM 3660 N PHEA463 77.802 57.069 110.673 1.00 16.02 A
ATOM 3661 CA PHE A 463 77.799 55.704 111.177 1.00 17.70 A
ATOM 3662 CB PHE A 463 79.001 54.938 110.636 1.00 16.10 A
ATOM 3663 CG PHE A 463 78.796 54.416 109.245 1.00 17.34 A
ATOM 3664 CD1 PHE A 463 77.986 53.307 109.020 1.00 16.15 A
ATOM 3665 CD2 PHE A 463 79.378 55.053 108.157 1.00 14.81 A
ATOM 3666 CE1 PHE A 463 77.756 52.840 107.725 1.00 16.04 A
ATOM 3667 CE2 PHE A 463 79.154 54.596 106.862 1.00 15.85 A
ATOM 3668 CZ PHE A 463 78.339 53.486 106.647 1.00 14.32 A
ATOM 3669 C PHE A 463 77.865 55.796 112.699 1.00 18.30 A
ATOM 3670 O PHE A 463 78.770 56.425 113.250 1.00 17.49 A
ATOM 3671 N ASN A 464 76.898 55.180 113.372 1.00 18.49 A
ATOM 3672 CA ASN A 464 76.850 55.220 114.829 1.00 19.64 A
ATOM 3673 CB ASN A 464 75.423 54.971 115.307 1.00 19.64 A
ATOM 3674 CG ASN A 464 75.197 55.450 116.729 1.00 21.27 A
ATOM 3675 OD1 ASN A 464 76.097 55.381 117.572 1.00 22.57 A
ATOM 3676 ND2 ASN A 464 73.992 55.934 117.004 1.00 17.01 A
ATOM 3677 C ASN A 464 77.785 54.188 115.465 1.00 21.31 A
ATOM 3678 O ASN A 464 77.365 53.072 115.775 1.00 21.91 A
ATOM 3679 N ASP A 465 79.045 54.566 115.667 1.0021.05 A
ATOM 3680 CA ASP A 465 80.021 53.660 116.265 1.00 24.38 A
ATOM 3681 CB ASP A 465 81.435 54.252 116.171 1.00 27.12 A
ATOM 3682 CG ASP A 465 81.518 55.667 116.723 1.00 32.89 A
ATOM 3683 OD1 ASP A 465 82.652 56.151 116.939 1.00 35.65 A
ATOM 3684 OD2 ASP A 465 80.456 56.300 116.932 1.00 35.38 A
ATOM 3685 C ASPA465 79.684 53.330 117.722 1.00 24.14 A
ATOM 3686 O ASP A 465 80.016 52.246 118.207 1.00 24.28 A
ATOM 3687 N META466 79.028 54.261 118.414 1.00 23.27 A
ATOM 3688 CA MET A 466 78.627 54.049 119.807 1.00 23.00 A
ATOM 3689 CB MET A 466 77.971 55.318 120.373 1.00 22.56 A
ATOM 3690 CG MET A 466 77.462 55.217 121.821 1.00 25.63 A
ATOM 3691 SD MET A 466 75.971 54.203 122.059 1.00 25.66 A
ATOM 3692 CE MET A 466 74.689 55.343 121.568 1.00 25.95 A
ATOM 3693 C META466 77.637 52.887 119.864 1.0022.80 A
ATOM 3694 O MET A 466 77.868 51.896 120.553 1.0023.68 A
ATOM 3695 N PHEA467 76.541 53.009 119.121 1.0021.61 A
ATOM 3696 CA PHEA467 75.511 51.973 119.101 1.0022.16 A
ATOM 3697 CB PHE A 467 74.370 52.386 118.156 1.00 19.95 A
ATOM 3698 CG PHE A 467 73.097 51.592 118.331 1.00 18.22 A
ATOM 3699 CD1 PHE A 467 72.043 51.754 117.435 1.00 18.43 A
ATOM 3700 CD2 PHE A 467 72.941 50.695 119.385 1.00 18.79 A
ATOM 3701 CE1 PHE A 467 70.852 51.038 117.583 1.00 18.95 A
ATOM 3702 CE2 PHE A 467 71.754 49.970 119.545 1.00 17.07 A
ATOM 3703 CZ PHEA467 70.708 50.142 118.643 1.0018.42 A
ATOM 3704 C PHEA467 76.121 50.647 118.651 1.0023.24 A
ATOM 3705 O PHEA467 75.809 49.586 119.199 1.00 22.00 A
ATOM 3706 N ARG A 468 77.004 50.720 117.660 1.0024.59 A
ATOM 3707 CA ARG A 468 77.665 49.529 117.128 1.0025.38 A
ATOM 3708 CB ARG A 468 78.659 49.924 116.030 1.00 24.86 A
ATOM 3709 CG ARG A 468 79.438 48.754 115.432 1.00 26.04 A
ATOM 3710 CD ARG A 468 80.723 49.249 114.781 1.00 26.14 A
ATOM 3711 NE ARG A 468 81.638 49.821 115.769 1.00 26.53 A
ATOM 3712 CZ ARG A 468 82.669 50.606 115.469 1.0028.64 A
ATOM 3713 NH1 ARG A 468 82.918 50.920 114.203 1.00 29.08 A
ATOM 3714 NH2 ARG A 468 83.456 51.076 116.431 1.00 24.28 A
ATOM 3715 C ARG A 468 78.402 48.744 118.215 1.00 25.90 A
ATOM 3716 O ARG A 468 78.129 47.558 118.433 1.00 25.20 A
ATOM 3717 N ASPA469 79.336 49.408 118.893 1.00 24.54 A
ATOM 3718 CA ASP A 469 80.119 48.764 119.940 1.00 25.61 A
ATOM 3719 CB ASP A 469 81.316 49.646 120.316 1.00 27.88 A
ATOM 3720 CG ASP A 469 82.410 49.623 119.255 1.00 29.91 A
ATOM 3721 OD1 ASP A 469 83.454 50.289 119.439 1.00 31.98 A
ATOM 3722 OD2 ASP A 469 82.223 48.927 118.234 1.00 29.75 A
ATOM 3723 C ASP A 469 79.302 48.406 121.182 1.00 25.36 A
ATOM 3724 O ASP A 469 79.641 47.475 121.910 1.00 24.70 A
ATOM 3725 N ALAA 470 78.221 49.140 121.417 1.00 24.79 A
ATOM 3726 CA ALA A 470 77.357 48.877 122.558 1.00 23.29 A

ATOM 3727 CB ALAA470 76.281 49.959 122.656 1.00 21.32 A
ATOM 3728 C ALAA470 76.707 47.505 122.382 1.00 23.68 A
ATOM 3729 O ALAA 470 76.584 46.733 123.331 1.00 23.57 A
ATOM 3730 N VAL A 471 76.303 47.210 121.152 1.00 23.55 A
ATOM 3731 CA VAL A 471 75.648 45.949 120.821 1.00 24.04 A
ATOM 3732 CB VAL A 471 74.818 46.090 119.513 1.00 23.61 A
ATOM 3733 CG1 VAL A 471 74.248 44.742 119.099 1.00 22.61 A
ATOM 3734 CG2 VAL A 471 73.699 47.098 119.715 1.00 23.45 A
ATOM 3735 C VAL A 471 76.611 44.773 120.648 1.0025.26 A
ATOM 3736 O VAL A 471 76.443 43.723 121.279 1.0024.65 A
ATOM 3737 N LYS A 472 77.609 44.957 119.787 1.0024.81 A
ATOM 3738 CA LYS A 472 78.590 43.921 119.478 1.0024.49 A
ATOM 3739 CB LYS A 472 79.157 44.161 118.077 1.0024.88 A
ATOM 3740 CG LYS A 472 80.330 43.255 117.708 1.00 23.59 A
ATOM 3741 CD LYS A 472 80.903 43.618 116.341 1.00 24.59 A
ATOM 3742 CE LYSA472 81.522 45.011 116.360 1.00 24.08 A
ATOM 3743 NZ LYS A 472 82.206 45.328 115.083 1.00 24.91 A
ATOM 3744 C LYS A 472 79.751 43.802 120.456 1.00 25.82 A
ATOM 3745 O LYS A 472 80.192 42.697 120.782 1.00 24.90 A
ATOM 3746 N GLY A 473 80.243 44.947 120.912 1.00 27.47 A
ATOM 3747 CA GLY A 473 81.375 44.975 121.817 1.00 28.63 A
ATOM 3748 C GLYA473 82.449 45.760 121.096 1.00 30.20 A
ATOM 3749 O GLY A 473 82.460 45.779 119.866 1.00 30.40 A
ATOM 3750 N ASN A 474 83.335 46.420 121.838 1.00 31.72 A
ATOM 3751 CA ASN A 474 84.408 47.207 121.227 1.00 33.39 A
ATOM 3752 CB ASN A 474 85.465 47.536 122.285 1.00 33.23 A
ATOM 3753 CG ASN A 474 86.474 48.550 121.801 1.00 34.10 A
ATOM 3754 OD1 ASN A 474 87.353 48.239 120.995 1.00 32.81 A
ATOM 3755 ND2 ASN A 474 86.344 49.782 122.280 1.00 33.61 A
ATOM 3756 C ASN A 474 85.035 46.422 120.065 1.00 33.88 A
ATOM 3757 O ASN A 474 85.396 45.256 120.226 1.00 32.99 A
ATOM 3758 N THRA475 85.162 47.054 118.899 1.00 35.59 A
ATOM 3759 CA THR A 475 85.717 46.366 117.731 1.00 38.34 A
ATOM 3760 CB THR A 475 85.216 46.980 116.393 1.00 37.82 A
ATOM 3761 OG1 THR A 475 86.040 48.092 116.032 1.00 39.30 A
ATOM 3762 CG2 THR A 475 83.785 47.448 116.523 1.00 36.18 A
ATOM 3763 C THR A 475 87.239 46.334117.677 1.0039.52 A
ATOM 3764 O THRA475 87.818 45.721 116.780 1.0041.19 A
ATOM 3765 N PHEA476 87.890 46.983 118.634 1.00 41.64 A
ATOM 3766 CA PHE A 476 89.347 47.009 118.654 1.0043.87 A
ATOM 3767 CB PHE A 476 89.806 48.462 118.694 1.00 45.66 A
ATOM 3768 CG PHE A 476 89.219 49.286 117.587 1.00 47.66 A
ATOM 3769 CD1 PHE A 476 89.577 49.041 116.261 1.00 47.51 A
ATOM 3770 CD2 PHE A 476 88.249 50.248 117.855 1.00 48.23 A
ATOM 3771 CE1 PHEA476 88.976 49.737 115.216 1.00 48.69 A
ATOM 3772 CE2 PHE A 476 87.638 50.953116.816 1.00 49.46 A
ATOM 3773 CZ PHE A 476 88.003 50.695 115.492 1.0049.29 A
ATOM 3774 C PHEA476 89.938 46.188 119.798 1.00 43.72 A
ATOM 3775 O PHEA476 91.079 46.392 120.212 1.00 45.02 A
ATOM 3776 N HIS A 477 89.134 45.252 120.290 1.00 43.20 A
ATOM 3777 CA HIS A 477 89.516 44.334 121.357 1.00 41.99 A
ATOM 3778 CB HIS A 477 89.114 44.879 122.731 1.00 43.16 A
ATOM 3779 CG HIS A 477 89.846 46.122 123.129 1.00 45.66 A
ATOM 3780 CD2 HIS A 477 89.394 47.351 123.477 1.00 46.84 A
ATOM 3781 ND1 HIS A 477 91.220 46.182 123.213 1.00 47.87 A
ATOM 3782 CE1 HIS A 477 91.583 47.394 123.595 1.00 47.48 A
ATOM 3783 NE2 HIS A 477 90.494 48.122 123.762 1.00 46.61 A
ATOM 3784 C HIS A 477 88.745 43.056 121.064 1.00 40.85 A
ATOM 3785 O HIS A 477 87.554 42.959 121.360 1.00 39.36 A
ATOM 3786 N LEU A 478 89.429 42.087 120.467 1.00 41.59 A
ATOM 3787 CA LEU A 478 88.819 40.811 120.106 1.00 42.42 A
ATOM 3788 CB LEU A 478 89.900 39.776 119.781 1.00 43.87 A
ATOM 3789 CG LEU A 478 90.639 39.947 118.453 1.00 45.99 A
ATOM 3790 CD1 LEU A 478 91.473 41.213 118.502 1.00 47.67 A
ATOM 3791 CD2 LEU A 478 91.522 38.734 118.188 1.00 46.84 A
ATOM 3792 C LEU A 478 87.883 40.233 121.156 1.00 41.76 A
ATOM 3793 O LEU A 478 86.775 39.801 120.836 1.0042.62 A
ATOM 3794 N LYS A 479 88.328 40.224 122.407 1.00 40.83 A
ATOM 3795 CA LYS A 479 87.526 39.674 123.491 1.00 39.97 A
ATOM 3796 CB LYS A 479 88.434 39.242 124.648 1.0041.87 A
ATOM 3797 CG LYS A 479 88.640 37.737 124.734 1.00 45.29 A
ATOM 3798 CD LYS A 479 89.416 37.193 123.543 1.00 47.67 A
ATOM 3799 CE LYS A 479 89.180 35.696 123.374 1.0049.25 A
ATOM 3800 NZ LYS A 479 89.247 34.955 124.667 1.00 50.73 A
ATOM 3801 C LYS A 479 86.419 40.579 124.027 1.00 38.70 A
ATOM 3802 O LYS A 479 85.474 40.088 124.645 1.00 39.52 A
ATOM 3803 N ALAA480 86.521 41.887 123.803 1.00 5.20 A
ATOM 3804 CA ALA A 480 85.496 42.800 124.304 1.00 34.13 A
ATOM 3805 CB ALAA480 85.746 44.210 123.791 1.00 34.76 A
ATOM 3806 C ALAA480 84.112 42.321 123.880 1.00 33.73 A
ATOM 3807 O ALAA480 83.918 41.877 122.750 1.00 34.11 A
ATOM 3808 N THR A 481 83.149 42.408 124.790 1.00 33.26 A
ATOM 3809 CA THR A 481 81.792 41.963 124.500 1.00 31.77 A
ATOM 3810 CB THR A 481 81.379 40.802 125.426 1.00 32.44 A
ATOM 3811 OG1 THR A 481 81.493 41.221 126.791 1.04 31.26 A
ATOM 3812 CG2 THR A 481 82.258 39.584 125.187 1.00 32.10 A
ATOM 3813 C THR A 481 80.759 43.066 124.662 1.00 30.44 A
ATOM 3814 O THR A 481 80.937 43.987 125.461 1.00 28.80 A
ATOM 3815 N GLY A 482 79.675 42.951 123.897 1.00 29.21 A
ATOM 3816 CA GLY A 482 78.593 43.916 123.959 1.00 27.90 A
ATOM 3817 C GLYA482 77.343 43.273 124.546 1.0027.35 A
ATOM 3818 O GLYA482 77.350 42.092 124.897 1.00 26.14 A
ATOM 3819 N PHEA483 76.265 44.041 124.647 1.0025.66 A
ATOM 3820 CA PHE A 483 75.017 43.531 125.202 1.00 24.97 A
ATOM 3821 CB PHE A 483 73.906 44.567 125.034 1.0023.80 A
ATOM 3822 CG PHE A 483 72.579 44.127 125.583 1.00 23.02 A
ATOM 3823 CD1 PHE A 483 72.355 44.094 126.955 1.00 21.70 A
ATOM 3824 CD2 PHE A 483 71.558 43.729 124.725 1.0023.42 A
ATOM 3825 CE1 PHE A 483 71.135 43.672 127.468 1.00 21.13 A
ATOM 3826 CE2 PHE A 483 70.329 43.302 125.226 1.0024.00 A
ATOM 3827 CZ PHE A 483 70.117 43.274 126.603 1.00 24.20 A
ATOM 3828 C PHEA483 74.588 42.218 124.549 1.00 25.14 A
ATOM 3829 O PHE A 483 74.248 41.257 125.236 1.00 26.61 A
ATOM 3830 N ALAA484 74.603 42.180123.220 1.0024.35 A
ATOM 3831 CA ALA A 484 74.201 40.985 122.486 1.00 24.13 A
ATOM 3832 CB ALA A 484 74.195 41.266 120.988 1.00 20.95 A
ATOM 3833 C ALAA484 75.106 39.798 122.792 1.00 23.73 A
ATOM 3834 O ALAA484 74.702 38.648 122.625 1.00 22.09 A
ATOM 3835 N LEU A 485 76.331 40.075 123.229 1.00 25.16 A
ATOM 3836 CA LEU A 485 77.265 39.005 123.564 1.00 28.77 A
ATOM 3837 CB LEU A 485 78.689 39.346 123.101 1.00 26.73 A
ATOM 3838 CG LEU A 485 78.914 39.510 121.593 1.00 29.21 A
ATOM 3839 CD1 LEU A485 80.409 39.473 121.297 1.00 27.79 A
ATOM 3840 CD2 LEU A 485 78.207 38.403 120.826 1.00 28.77 A
ATOM 3841 C LEU A 485 77.258 38.718 125.068 1.00 30.92 A
ATOM 3842 O LEU A 485 78.099 37.970 125.567 1.00 32.85 A
ATOM 3843 N GLY A 486 76.315 39.327 125.786 1.0030.61 A
ATOM 3844 CA GLY A 486 76.207 39.083 127.212 1.00 32.46 A
ATOM 3845 C GLY A 486 76.754 40.106 128.191 1.00 33.37 A
ATOM 3846 O GLY A 486 76.697 39.882 129.400 1.00 33.00 A
ATOM 3847 N ASN A 487 77.286 41.221 127.707 1.00 33.57 A
ATOM 3848 CA ASN A 487 77.813 42.220 128.630 1.00 33.65 A
ATOM 3849 CB ASN A 487 78.968 42.993 127.997 1.00 34.58 A
ATOM 3850 CG ASN A 487 79.801 43.727 129.029 1.00 35.75 A
ATOM 3851 OD1 ASN A 487 79.265 44.353 129.944 1.00 36.11 A
ATOM 3852 ND2 ASN A 487 81.117 43.656 128.888 1.00 36.99 A
ATOM 3853 C ASN A 487 76.721 43.200 129.061 1.00 34.19 A
ATOM 38540 ASN A 487 76.192 43.960 128.243 1.00 34.06 A
ATOM 3855 N GLY A 488 76.394 43.183 130.350 1.00 33.15 A
ATOM 3856 CA GLY A 488 75.366 44.068 130.866 1.00 34.03 A
ATOM 3857 C GLY A 488 75.792 45.521 130.999 1.00 33.99 A
ATOM 3858 O GLYA488 74.950 46.407 131.130 1.00 32.77 A
ATOM 3859 N GLU A 489 77.098 45.761 130.963 1.00 35.20 A
ATOM 3860 CA GLU A 489 77.655 47.107 131.090 1.00 35.66 A
ATOM 3861 CB GLUA489 79.185 47.044 131.096 1.0038.02 A
ATOM 3862 CG GLU A 489 79.797 46.419 132.338 1.00 44.75 A
ATOM 3863 CD GLU A 489 79.539 47.237 133.595 1.00 49.58 A
ATOM 3864 OE1 GLU A 489 78.370 47.306 134.042 1.00 50.68 A
ATOM 3865 OE2 GLU A 489 80.510 47.816 134.133 1.00 53.01 A
ATOM 3866 C GLU A 489 77.213 48.061 129.984 1.00 34.88 A
ATOM 3867 O GLU A 489 77.206 49.276 130.170 1.00 34.34 A
ATOM 3868 N SERA490 76.842 47.518 128.832 1.00 32.45 A
ATOM 3869 CA SERA 490 76.436 48.370 127.730 1.00 30.88 A
ATOM 3870 CB SERA490 77.170 47.939126.460 1.0030.81 A
ATOM 3871 OG SERA490 77.214 46.532 126.353 1.00 29.96 A
ATOM 3872 C SERA490 74.934 48.421 127.478 1.00 29.69 A
ATOM 3873 O SERA490 74.491 49.001 126.487 1.00 29.28 A
ATOM 3874 N ALA A 491 74.154 47.841 128.383 1.00 27.03 A
ATOM 3875 CA ALA A 491 72.699 47.821 128.239 1.0026.22 A
ATOM 3876 CB ALA A 491 72.065 47.152 129.455 1.00 24.83 A
ATOM 3877 C ALAA491 72.092 49.210 128.036 1.00 25.11 A
ATOM 3878 O ALA A 491 71.240 49.402 127.162 1.0023.62 A
ATOM 3879 N GLN A492 72.535 50.174 128.837 1.00 24.68 A
ATOM 3880 CA GLN A 492 72.018 51.537 128.754 1.00 25.01 A
ATOM 3881 CB GLN A 492 72.551 52.383 129.911 1.00 27.48 A
ATOM 3882 CG GLN A 492 72.087 53.828 129.858 1.00 31.81 A
ATOM 3883 CD GLN A 492 72.626 54.661 131.002 1.00 34.57 A
ATOM 3884 OE1 GLN A 492 73.838 54.729 131.223 1.00 37.64 A
ATOM 3885 NE2 GLN A 492 71.728 55.309 131.731 1.00 36.83 A
ATOM 3886 C GLN A 492 72.362 52.212 127.434 1.00 24.04 A
ATOM 3887 O GLN A 492 71.517 52.870 126.831 1.0022.74 A
ATOM 3888 N ALAA493 73.608 52.061 126.996 1.00 23.03 A
ATOM 3889 CA ALA A 493 74.037 52.645 125.729 1.00 23.17 A
ATOM 3890 CB ALAA493 75.512 52.341 125.484 1.00 20.69 A
ATOM 3891 C ALAA493 73.173 52.052 124.607 1.0021.86 A
ATOM 3892 O ALAA493 72.792 52.749 123.668 1.00 20.97 A
ATOM 3893 N VALA494 72.864 50.762 124.720 1.00 20.14 A
ATOM 3894 CA VALA494 72.034 50.084 123.731 1.0019.34 A
ATOM 3895 CB VAL A 494 71.934 48.571 124.028 1.0018.56 A
ATOM 3896 CG1 VAL A 494 70.795 47.955 123.230 1.00 14.74 A
ATOM 3897 CG2 VALA494 73.249 47.890 123.682 1.00 16.26 A
ATOM 3898 C VALA494 70.629 50.687 123.712 1.00 19.82 A
ATOM 3899 O VALA494 70.080 50.959 122.645 1.00 18.90 A
ATOM 3900 N META 495 70.054 50.897 124.894 1.00 20.79 A
ATOM 3901 CA META495 68.718 51.479 125.004 1.00 20.50 A
ATOM 3902 CB MET A 495 68.330 51.646 126.471 1.00 20.73 A
ATOM 3903 CG MET A 495 66.936 52.199 126.674 1.0020.60 A
ATOM 3904 SD META 495 65.702 51.083 126.009 1.00 23.31 A
ATOM 3905 CE META 495 64.970 52.091 124.724 1.00 20.97 A
ATOM 3906 C META495 68.716 52.844 124.325 1.0020.55 A
ATOM 3907 O META 495 67.757 53.217 123.644 1.0019.89 A
ATOM 3908 N HIS A 496 69.806 53.580 124.524 1.00 19.68 A
ATOM 3909 CA HIS A 496 69.987 54.902 123.936 1.00 19.32 A
ATOM 3910 CB HIS A 496 71.330 55.490 124.414 1.00 20.92 A
ATOM 3911 CG HIS A 496 71.597 56.888 123.941 1.00 22.19 A
ATOM 3912 CD2 HIS A 496 72.751 57.589 123.846 1.00 21.20 A
ATOM 3913 ND1 HIS A 496 70.598 57.741 123.518 1.00 22.46 A
ATOM 3914 CE1 HIS A 496 71.126 58.904 123.181 1.00 20.08 A
ATOM 3915 NE2 HIS A 496 72.431 58.839 123.371 1.00 22.38 A
ATOM 3916 C HIS A 496 69.944 54.760 122.409 1.00 18.68 A
ATOM 3917 O HIS A 496 69.242 55.504 121.729 1.00 18.35 A
ATOM 3918 N GLYA497 70.679 53.790121.877 1.00 18.94 A
ATOM 3919 CA GLY A 497 70.677 53.573 120.440 1.00 19.97 A
ATOM 3920 C GLY A 497 69.284 53.194 119.956 1.00 22.74 A
ATOM 3921 O GLY A 497 68.838 53.626 118.888 1.00 22.62 A
ATOM 3922 N ILE A 498 68.588 52.380 120.744 1.00 22.05 A
ATOM 3923 CA ILEA498 67.241 51.962 120.388 1.00 20.72 A
ATOM 3924 CB ILEA498 66.688 50.965 121.438 1.00 21.21 A
ATOM 3925 CG2 ILEA498 65.184 50.802 121.284 1.00 21.18 A
ATOM 3926 CG1 ILE A 498 67.408 49.621 121.291 1.00 19.96 A
ATOM 3927 CD1 ILE A 498 67.085 48.627 122.377 1.00 20.34 A
ATOM 3928 C ILE A 498 66.347 53.197 120.300 1.0020.57 A
ATOM 3929 O ILE A 498 65.392 53.235 119.517 1.00 19.92 A
ATOM 3930 N ALAA499 66.685 54.217 121.085 1.00 19.58 A
ATOM 3931 CA ALA A 499 65.917 55.457 121.110 1.00 18.90 A
ATOM 3932 CB ALA A 499 65.993 56.097 122.505 1.00 17.97 A
ATOM 3933 C ALAA499 66.369 56.458 120.052 1.00 19.83 A
ATOM 3934 O ALAA499 65.922 57.601 120.054 1.0022.19 A
ATOM 3935 N GLY A 500 67.264 56.041 119.161 1.00 20.00 A
ATOM 3936 CA GLY A 500 67.711 56.941 118.110 1.00 20.92 A
ATOM 3937 C GLY A 500 69.005 57.699 118.353 1.00 22.74 A
ATOM 3938 O GLY A 500 69.417 58.494 117.511 1.00 20.53 A
ATOM 3939 N SER A 501 69.639 57.479 119.503 1.00 22.60 A
ATOM 3940 CA SER A 501 70.905 58.142 119.815 1.00 22.56 A
ATOM 3941 CB SER A 501 71.976 57.677 118.829 1.00 20.91 A
ATOM 3942 OG SERA 501 72.079 56.270 118.837 1.00 18.73 A
ATOM 3943 C SER A 501 70.824 59.671 119.787 1.00 23.99 A
ATOM 3944 O SER A 501 71.817 60.349 119.508 1.00 21.28 A
ATOM 3945 N SERA 502 69.643 60.206 120.078 1.0024.88 A
ATOM 3946 CA SERA 502 69.443 61.645 120.072 1.0027.46 A
ATOM 3947 CB SERA 502 68.225 61.994 119.205 1.00 28.36 A
ATOM 3948 OG SERA 502 68.417 61.565 117.858 1.00 27.13 A
ATOM 3949 C SERA502 69.255 62.167 121.491 1.00 29.43 A
ATOM 3950 O SERA502 68.532 63.135 121.718 1.00 32.06 A
ATOM 3951 N GLY A 503 69.917 61.519 122.443 1.00 29.64 A
ATOM 3952 CA GLY A 503 69.813 61.926 123.831 1.00 32.25 A
ATOM 3953 C GLY A 503 68.985 60.975 124.682 1.00 33.57 A
ATOM 3954 O GLY A 503 67.844 60.648 124.336 1.00 34.23 A
ATOM 3955 N TRP A 504 69.564 60.528 125.794 1.00 34.30 A
ATOM 3956 CA TRP A 504 68.890 59.618 126.718 1.00 35.88 A
ATOM 3957 CB TRP A 504 69.194 58.161 126.363 1.00 35.71 A
ATOM 3958 CG TRP A 504 68.412 57.174 127.185 1.00 35.16 A
ATOM 3959 CD2 TRP A 504 67.072 56.734 126.941 1.00 33.65 A
ATOM 3960 CE2 TRP A 504 66.728 55.842 127.981 1.00 33.67 A
ATOM 3961 CE3 TRP A 504 66.127 57.009 125.945 1.00 33.12 A
ATOM 3962 CD1 TRPA 504 68.819 56.547 128.333 1.00 36.01 A
ATOM 3963 NE1 TRP A 504 67.811 55.743 128.816 1.00 35.33 A
ATOM 3964 CZ2 TRP A 504 65.481 55.225 128.053 1.00 31.49 A
ATOM 3965 CZ3 TRP A 504 64.886 56.393 126.017 1.00 32.76 A
ATOM 3966 CH2 TRP A 504 64.576 55.511 127.065 1.00 32.14 A
ATOM 3967 C TRP A 504 69.311 59.884 128.159 1.0036.88 A
ATOM 3968 O TRP A 504 70.414 59.528 128.573 1.00 36.49 A
ATOM 3969 N LYS A 505 68.419 60.517 128.914 1.00 40.46 A
ATOM 3970 CA LYS A 505 68.675 60.842 130.311 1.00 42.18 A
ATOM 3971 CB LYS A 505 68.556 59.577 131.172 1.00 43.78 A
ATOM 3972 CG LYS A 505 67.210 58.872 131.031 1.00 45.63 A
ATOM 3973 CD LYS A 505 67.016 57.781 132.077 1.00 46.63 A
ATOM 3974 CE LYS A 505 65.703 57.031 131.85 1.00 45.99 A
ATOM 3975 NZ LYS A 505 64.519 57.936 131.869 1.00 46.29 A
ATOM 3976 C LYS A 505 70.044 61.493 130.510 1.00 43.03 A
ATOM 3977 O LYS A 505 70.306 62.580 129.991 1.00 43.87 A
ATOM 3978 N ALA A 506 70.914 60.822 131.258 1.00 43.56 A
ATOM 3979 CA ALA A 506 72.248 61.340 131.540 1.00 43.69 A
ATOM 3980 CB ALA A 506 72.822 60.643 132.768 1.0044.84 A
ATOM 3981 C ALA A 506 73.217 61.199 130.371 1.00 44.02 A
ATOM 3982 O ALAA506 74.331 61.730 130.416 1.0044.54 A
ATOM 3983 N LEU A 507 72.804 60.482 129.330 1.00 41.99 A
ATOM 3984 CA LEU A 507 73.665 60.289 128.170 1.00 39.36 A
ATOM 3985 CB LEU A 507 73.354 58.951 127.492 1.00 40.92 A
ATOM 3986 CG LEU A 507 73.593 57.677 128.307 1.0041.00 A
ATOM 3987 CD1 LEU A 507 73.076 56.465 127.543 1.00 40.48 A
ATOM 3988 CD2 LEU A 507 75.080 57.532 128.598 1.00 42.37 A
ATOM 3989 C LEU A 507 73.484 61.426 127.175 1.00 37.26 A
ATOM 3990 O LEU A 507 72.359 61.754 126.786 1.00 36.26 A
ATOM 3991 N ALAA508 74.598 62.034126.777 1.0033.76 A
ATOM 3992 CA ALA A 508 74.560 63.131 125.815 1.00 31.42 A
ATOM 3993 CB ALA A 508 75.901 63.846 125.785 1.0030.14 A
ATOM 3994 C ALAA508 74.230 62.577 124.427 1.0028.54 A
ATOM 3995 O ALA A 508 74.628 61.464 124.084 1.0027.57 A
ATOM 3996 N PRO A 509 73.496 63.352 123.616 1.0026.96 A
ATOM 3997 CD PRO A 509 72.934 64.670 123.964 1.00 27.17 A
ATOM 3998 CA PRO A 509 73.101 62.959 122.259 1.00 26.92 A
ATOM 3999 CB PRO A 509 72.363 64.194 121.745 1.00 25.98 A
ATOM 4000 CG PRO A 509 71.787 64.790 122.999 1.00 26.92 A
ATOM 4001 C PRO A 509 74.286 62.599 121.364 1.00 26.40 A
ATOM 4002 O PRO A 509 75.354 63.210 121.458 1.00 26.89 A
ATOM 4003 N ILE A 510 74.106 61.596 120.511 1.00 25.31 A
ATOM 4004 CA ILE A 510 75.169 61.213 119.590 1.00 25.11 A
ATOM 4005 CB ILE A 510 75.092 59.726 119.185 1.0025.59 A
ATOM 4006 CG2 ILE A 510 76.221 59.403 118.209 1.0023.39 A
ATOM 4007 CG1 ILE A 510 75.177 58.834 120.430 1.00 25.50 A
ATOM 4008 CD1 ILE A 510 76.433 59.046 121.271 1.00 24.63 A
ATOM 4009 C ILE A 510 74.968 62.072 118.351 1.00 25.14 A
ATOM 4010 O ILE A 510 75.929 62.548 117.746 1.00 26.25 A
ATOM 4011 N VAL A 511 73.701 62.252 117.981 1.00 23.82 A
ATOM 4012 CA VAL A 511 73.312 63.080 116.837 1.00 22.94 A
ATOM 4013 CB VAL A 511 73.087 62.245 115.554 1.00 20.54 A
ATOM 4014 CG1 VAL A 511 74.413 61.755 115.014 1.0018.37 A
ATOM 4015 CG2 VAL A 511 72.166 61.078 115.848 1.00 18.73 A
ATOM 4016 C VAL A 511 72.01 63.805 117.195 1.00 23.01 A
ATOM 4017 O VAL A 511 71.233 63.327 118.014 1.0022.84 A
ATOM 4018 N PRO A 512 71.771 64.971 116.588 1.0023.94 A
ATOM 4019 CD PRO A 512 72.677 65.777 115.753 1.0023.93 A
ATOM 4020 CA PRO A 512 70.546 65.715 116.897 1.00 25.15 A
ATOM 4021 CB PRO A 512 70.706 66.994 116.076 1.00 24.45 A
ATOM 4022 CG PRO A 512 72.203 67.181 116.046 1.00 24.29 A
ATOM 4023 C PRO A 512 69.245 64.986 116.575 1.00 26.10 A
ATOM 4024 O PRO A 512 68.314 64.983 117.382 1.00 26.95 A
ATOM 4025 N GLU A 513 69.182 64.370 115.400 1.0024.95 A
ATOM 4026 CA GLU A 513 67.976 63.672 114.974 1.00 24.62 A
ATOM 4027 CB GLU A 513 67.309 64.454 113.839 1.00 26.65 A
ATOM 4028 CG GLU A 513 66.896 65.867 114.231 1.00 29.13 A
ATOM 4029 CD GLU A 513 65.775 65.886 115.260 1.00 32.48 A
ATOM 4030 OE1 GLU A 513 65.502 66.966 115.825 1.00 32.11 A
ATOM 4031 OE2 GLU A 513 65.161 64.821 115.496 1.00 32.46 A
ATOM 4032 C GLU A 513 68.279 62.249 114.525 1.00 24.48 A
ATOM 4033 O GLU A 513 69.383 61.957 114.065 1.00 25.03 A
ATOM 4034 N PRO A 514 67.293 61.346 114.634 1.00 22.55 A
ATOM 4035 CD PRO A 514 65.900 61.584 115.051 1.00 22.52 A
ATOM 4036 CA PRO A 514 67.491 59.948 114.236 1.00 22.00 A
ATOM 4037 CB PRO A 514 66.170 59.283 114.641 1.00 21.42 A
ATOM 4038 CG PRO A 514 65.172 60.394 114.453 1.00 23.44 A
ATOM 4039 C PRO A 514 67.839 59.735 112.764 1.00 21.23 A
ATOM 4040 O PRO A 514 68.445 58.723 112.413 1.00 21.12 A
ATOM 4041 N SER A 515 67.466 60.678 111.903 1.0020.25 A
ATOM 4042 CA SER A 515 67.774 60.541 110.482 1.0019.61 A
ATOM 4043 CB SER A 515 67.087 61.644 109.667 1.00 20.22 A
ATOM 4044 OG SER A 515 67.639 62.916 109.971 1.00 21.31 A
ATOM 4045 C SER A 515 69.288 60.606 110.247 1.00 19.43 A
ATOM 4046 O SER A 515 69.778 60.227 109.181 1.00 18.05 A
ATOM 4047 N GLN A 516 70.028 61.083 111.243 1.00 18.02 A
ATOM 4048 CA GLN A 516 71.474 61.191 111.109 1.00 18.60 A
ATOM 4049 CB GLN A 516 71.971 62.498 111.725 1.00 17.49 A
ATOM 4050 CG GLN A 516 71.256 63.729111.214 1.00 19.93 A
ATOM 4051 CD GLN A 516 71.867 65.000 111.762 1.00 23.39 A
ATOM 4052 OE 1 GLN A 516 72.867 65.506 111.236 1.00 21.39 A
ATOM 4053 NE2 GLN A 516 71.281 65.517 112.841 1.00 24.83 A
ATOM 4054 C GLN A 516 72.224 60.020 111.734 1.00 18.80 A
ATOM 4055 O GLN A 516 73.455 59.960 111.666 1.00 17.92 A
ATOM 4056 N SER A 517 71.488 59.088 112.333 1.00 18.67 A
ATOM 4057 CA SERA517 72.117 57.927 112.953 1.00 18.18 A
ATOM 4058 CB SER A 517 71.493 57.617 114.314 1.00 16.17 A
ATOM 4059 OG SER A 517 72.112 56.464 114.870 1.0014.27 A
ATOM 4060 C SER A 517 72.047 56.671 112.108 1.00 17.22 A
ATOM 4061 O SER A 517 70.999 56.043 112.004 1.00 19.65 A
ATOM 4062 N ILE A 518 73.173 56.306 111.509 1.00 17.78 A
ATOM 4063 CA ILE A 518 73.253 55.103 110.693 1.00 16.03 A
ATOM 4064 CB ILE A 518 74.393 55.218 109.660 1.00 15.57 A
ATOM 4065 CG2 ILE A 518 74.425 53.984 108.763 1.00 14.15 A
ATOM 4066 CG1 ILE A 518 74.188 56.474 108.809 1.00 14.69 A
ATOM 4067 CD 1 ILE A 518 72.912 56.462 107.981 1.00 11.69 A
ATOM 4068 C ILE A 518 73.545 53.969 111.672 1.00 17.18 A
ATOM 4069 O ILE A 518 74.669 53.848 112.176 1.00 17.15 A
ATOM 4070 N ASN A 519 72.527 53.160 111.956 1.00 16.80 A
ATOM 4071 CA ASN A 519 72.654 52.052 112.903 1.00 18.25 A
ATOM 4072 CB ASN A 519 71.311 51.808 113.608 1.00 15.43 A
ATOM 4073 CG ASN A 519 70.787 53.047 114.314 1.0015.29 A
ATOM 4074 OD1 ASN A 519 71.553 53.822 114.886 1.00 14.12 A
ATOM 4075 ND2 ASN A 519 69.473 53.231 114.291 1.00 14.02 A
ATOM 4076 C ASN A 519 73.127 50.758 112.242 1.00 18.71 A
ATOM 4077 O ASN A 519 72.526 50.294 111.271 1.00 20.16 A
ATOM 4078 N TYR A 520 74.193 50.170 112.785 1.00 19.72 A
ATOM 4079 CA TYR A 520 74.756 48.931 112.238 1.00 19.09 A
ATOM 4080 CB TYR A 520 75.583 49.251 110.987 1.00 19.87 A
ATOM 4081 CG TYRA520 76.898 49.947 111.300 1.00 19.50 A
ATOM 4082 CD1 TYR A 520 76.930 51.114 112.066 1.0020.06 A
ATOM 4083 CE1 THR A 520 78.136 51.730 112.395 1.00 19.47 A
ATOM 4084 CD2 TYR A 520 78.112 49.416 110.864 1.00 21.90 A
ATOM 4085 CE2 TYR A 520 79.329 50.027 111.186 1.00 20.40 A
ATOM 4086 CZ TYR A 520 79.330 51.182 111.951 1.00 21.80 A
ATOM 4087 OH TYR A 520 80.522 51.791 112.266 1.00 21.55 A
ATOM 4088 C TYR A 520 75.660 48.218 113.252 1.00 19.86 A
ATOM 4089 O TYR A 520 76.078 48.813 114.250 1.00 18.33 A
ATOM 4090 N VAL A 521 75.955 46.944 112.989 1.00 18.97 A
ATOM 4091 CA VAL A 521 76.845 46.160 113.846 1.00 19.95 A
ATOM 4092 CB VAL A 521 76.087 45.095 114.674 1.00 19.34 A
ATOM 4093 CG1 VAL A 521 75.106 45.777 115.624 1.00 20.43 A
ATOM 4094 CG2 VAL A 521 75.371 44.121 113.746 1.00 19.50 A
ATOM 4095 C VAL A 521 77.900 45.450 113.000 1.00 19.70 A
ATOM 4096 O VAL A 521 78.723 44.702 113.525 1.00 18.81 A
ATOM 4097 N GLU A 522 77.867 45.690 111.689 1.00 18.62 A
ATOM 4098 CA GLU A 522 78.822 45.085 110.764 1.00 18.19 A
ATOM 4099 CB GLU A 522 78.395 43.651 110.412 1.00 18.30 A
ATOM 4100 CG GLU A 522 79.426 42.866 109.588 1.00 18.72 A
ATOM 4101 CD GLU A 522 78.989 41.429 109.296 1.0022.26 A
ATOM 4102 OE1 GLU A 522 78.755 40.664 110.258 1.00 22.45 A
ATOM 4103 OE2 GLU A 522 78.880 41.058 108.105 1.00 19.37 A
ATOM 4104 C GLU A 522 78.945 45.897 109.477 1.00 18.84 A
ATOM 4105 O GLU A 522 77.992 46.540 109.039 1.00 18.20 A
ATOM 4106 N SERA 523 80.132 45.872 108.884 1.00 19.76 A
ATOM 4107 CA SER A 523 80.389 46.568 107.623 1.00 19.86 A
ATOM 4108 CB SERA523 80.783 48.026 107.860 1.00 19.65 A
ATOM 4109 OG SER A 523 82.098 48.112 108.372 1.00 21.70 A
ATOM 4110 C SERA523 81.545 45.830 106.973 1.00 20.71 A
ATOM 4111 O SER A 523 81.988 44.806 107.497 1.0020.76 A
ATOM 4112 N HIS A 524 82.041 46.338 105.846 1.00 20.36 A
ATOM 4113 CA HIS A 524 83.148 45.672 105.174 1.00 21.42 A
ATOM 4114 CB HIS A 524 83.408 46.278 103.776 1.0021.19 A
ATOM 4115 CG HIS A 524 83.941 47.680 103.798 1.0020.53 A
ATOM 4116 CD2 HIS A 524 85.208 48.152 103.707 1.00 19.61 A
ATOM 4117 ND1 HIS A 524 83.131 48.787 103.934 1.00 20.64 A
ATOM 4118 CE1 HIS A 524 83.874 49.880 103.924 1.00 21.26 A
ATOM 4119 NE2 HIS A 524 85.138 49.522 103.788 1.00 20.78 A
ATOM 4120 C HIS A 524 84.411 45.746 106.030 1.00 20.97 A
ATOM 4121 O HIS A 524 85.271 44.874 105.937 1.00 23.31 A
ATOM 4122 N ASP A 525 84.511 46.779 106.864 1.00 21.13 A
ATOM 4123 CA ASP A 525 85.663 46.964 107.752 1.00 22.88 A
ATOM 4124 CB ASP A 525 85.767 48.425 108.209 1.00 24.26 A
ATOM 4125 CG ASP A 525 86.230 49.352 107.109 1.00 25.15 A
ATOM 4126 OD1 ASP A 525 85.457 50.259 106.737 1.00 25.40 A
ATOM 4127 OD2 ASP A 525 87.368 49.175 106.621 1.00 25.12 A
ATOM 4128 C ASP A 525 85.603 46.089 109.001 1.00 22.61 A
ATOM 4129 O ASP A 525 84.520 45.756 109.483 1.00 23.43 A
ATOM 4130 N ASN A 526 86.776 45.736 109.522 1.00 22.08 A
ATOM 4131 CA ASN A 526 86.892 44.919 110.729 1.00 22.59 A
ATOM 4132 CB ASN A 526 86.194 45.625 111.898 1.00 23.45 A
ATOM 4133 CG ASN A 526 86.569 47.092 111.994 1.00 25.24 A
ATOM 4134 OD 1 ASN A 526 85.748 47.976 111.745 1.00 23.35 A
ATOM 4135 ND2 ASN A 526 87.825 47.357 112.345 1.00 26.82 A
ATOM 4136 C ASN A 526 86.315 43.512 110.569 1.00 23.34 A
ATOM 4137 O ASN A 526 85.812 43.158 109.504 1.00 22.41 A
ATOM 4138 N HIS A 527 86.401 42.711 111.631 1.0021.71 A
ATOM 4139 CA HIS A 527 85.868 41.352 111.609 1.00 21.39 A
ATOM 4140 CB HIS A 527 86.090 40.641 112.962 1.00 21.85 A
ATOM 4141 CG HIS A 527 87.528 40.439 113.335 1.00 21.78 A
ATOM 4142 CD2 HIS A 527 88.266 40.959 114.343 1.00 21.10 A
ATOM 4143 ND1 HIS A 527 88.368 39.590 112.645 1.0023.48 A
ATOM 4144 CE1 HIS A 527 89.561 39.597 113.212 1.00 21.18 A
ATOM 4145 NE2 HIS A 527 89.525 40.420 114.245 1.00 23.16 A
ATOM 4146 C HIS A 527 84.361 41.425 111.383 1.00 21.58 A
ATOM 4147 O HIS A 527 83.714 42.392 111.793 1.00 22.73 A
ATOM 4148 N THR A 528 83.799 40.406 110.740 1.00 20.96 A
ATOM 4149 CA THR A 528 82.356 40.357 110.553 1.00 20.13 A
ATOM 4150 CB THR A 528 81.928 39.163 109.666 1.00 22.27 A
ATOM 4151 OG1 THR A 528 82.318 37.936 110.300 1.00 20.32 A
ATOM 4152 CG2 THR A 528 82.578 39.254 108.281 1.00 20.75 A
ATOM 4153 C THR A 528 81.820 40.117 111.967 1.00 21.15 A
ATOM 4154 O THR A 528 82.579 39.732 112.856 1.0020.41 A
ATOM 4155 N PHE A 529 80.529 40.342 112.183 1.00 21.66 A
ATOM 4156 CA PHE A 529 79.949 40.125 113.502 1.00 21.87 A
ATOM 4157 CB PHE A 529 78.443 40.427 113.486 1.0020.79 A
ATOM 4158 CG PHE A 529 77.810 40.456 114.857 1.00 21.40 A
ATOM 4159 CD1 PHEA529 77.573 39.274 115.562 1.00 19.48 A
ATOM 4160 CD2 PHE A 529 77.470 41.672 115.453 1.00 19.37 A
ATOM 4161 CE1 PHE A 529 77.007 39.301 116.841 1.0020.15 A
ATOM 4162 CE2 PHE A 529 76.903 41.713 116.730 1.00 21.73 A
ATOM 4163 CZ PHE A 529 76.671 40.523 117.427 1.0021.47 A
ATOM 4164 C PHE A 529 80.193 38.680 113.939 1.00 23.50 A
ATOM 4165 O PHE A 529 80.532 38.421 115.099 1.00 22.81 A
ATOM 4166 N TRP A 530 80.032 37.743 113.007 1.0024.63 A
ATOM 4167 CA TRP A 530 80.239 36.328 113.312 1.00 26.04 A
ATOM 4168 CB TRP A 530 79.878 35.453 112.106 1.0025.59 A
ATOM 4169 CG TRP A 530 80.083 33.989 112.377 1.0026.34 A
ATOM 4170 CD2 TRP A 530 79.187 33.113 113.077 1.00 26.29 A
ATOM 4171 CE2 TRP A 530 79.826 31.858 113.180 1.0025.76 A
ATOM 4172 CE3 TRP A 530 77.908 33.271 113.634 1.0026.39 A
ATOM 4173 CD1 TRP A 530 81.192 33.246 112.088 1.00 23.75 A
ATOM 4174 NE1 TRP A 530 81.046 31.967 112.565 1.00 22.20 A
ATOM 4175 CZ2 TRP A 530 79.231 30.765 113.820 1.00 24.68 A
ATOM 4176 CZ3 TRP A 530 77.317 32.184 114.270 1.00 24.34 A
ATOM 4177 CH2 TRP A 530 77.980 30.949 114.358 1.00 24.68 A
ATOM 4178 C TRP A 530 81.666 35.996113.766 1.0026.92 A
ATOM 4179 O TRP A 530 81.854 35.294 114.760 1.00 25.97 A
ATOM 4180 N ASP A 531 82.665 36.486 113.036 1.00 26.53 A
ATOM 4181 CA ASP A 531 84.059 36.223 113.391 1.00 27.87 A
ATOM 4182 CB ASP A 531 84.999 36.644 112.253 1.00 27.59 A
ATOM 4183 CG ASP A 531 84.891 35.737 111.035 1.00 28.22 A
ATOM 4184 OD 1 ASP A 531 84.095 34.777 111.067 1.00 26.63 A
ATOM 4185 OD2 ASP A 531 85.605 35.984 110.040 1.00 30.33 A
ATOM 4186 C ASP A 531 84.441 36.959 114.672 1.0028.34 A
ATOM 4187 O ASP A 531 85.224 36.457 115.476 1.0031.17 A
ATOM 4188 N LYS A 532 83.891 38.153 114.857 1.0028.08 A
ATOM 4189 CA LYS A 532 84.166 38.943 116.051 1.00 28.43 A
ATOM 4190 CB LYS A 532 83.447 40.290 115.952 1.00 28.77 A
ATOM 4191 CG LYS A 532 83.139 40.961 117.285 1.00 29.07 A
ATOM 4192 CD LYS A 532 84.386 41.382 118.018 1.00 30.24 A
ATOM 4193 CE LYS A 532 84.017 42.085 119.316 1.0031.68 A
ATOM 4194 NZ LYS A 532 85.223 42.559 120.041 1.00 30.62 A
ATOM 4195 C LYS A 532 83.668 38.172 117.272 1.0029.62 A
ATOM 4196 O LYS A 532 84.419 37.905 118.214 1.00 27.69 A
ATOM 4197 N MET A 533 82.387 37.824 117.234 1.00 29.44 A
ATOM 4198 CA MET A 533 81.745 37.078 118.302 1.00 29.13 A
ATOM 4199 CB MET A 533 80.282 36.826 117.931 1.00 27.91 A
ATOM 4200 CG MET A 533 79.575 35.781 118.756 1.00 25.84 A
ATOM 4201 SD MET A 533 77.817 35.776 118.370 1.00 25.42 A
ATOM 4202 CE MET A 533 77.858 35.312 116.601 1.00 22.79 A
ATOM 4203 C META 533 82.477 35.762 118.554 1.00 29.82 A
ATOM 4204 O MET A 533 82.676 35.365 119.701 1.00 28.37 A
ATOM 4205 N SER A 534 82.894 35.096 117.481 1.00 30.01 A
ATOM 4206 CA SERA 534 83.607 33.833 117.611 1.00 30.32 A
ATOM 4207 CB SERA 534 83.923 33.258 116.226 1.00 30.55 A
ATOM 4208 OG SER A 534 82.731 32.851 115.559 1.0026.45 A
ATOM 4209 C SER A 534 84.884 33.975 118.438 1.00 31.84 A
ATOM 4210 O SER A 534 85.247 33.065 119.182 1.00 33.87 A
ATOM 4211 N PHE A 535 85.561 35.114 118.322 1.00 33.46 A
ATOM 4212 CA PHE A 535 86.780 35.355 119.094 1.00 33.19 A
ATOM 4213 CB PHE A 535 87.594 36.510 118.493 1.00 35.14 A
ATOM 4214 CG PHE A 535 88.350 36.138 117.246 1.00 37.64 A
ATOM 4215 CD1 PHE A 535 89.302 35.118 117.271 1.00 37.24 A
ATOM 4216 CD2 PHE A 535 88.109 36.802 116.045 1.00 35.56 A
ATOM 4217 CE 1 PHE A 535 89.998 34.763 116.117 1.00 36.98 A
ATOM 4218 CE2 PHE A 535 88.800 36.455 114.887 1.00 36.05 A
ATOM 4219 CZ PHE A 535 89.746 35.434 114.923 1.00 36.65 A
ATOM 4220 C PHE A 535 86.445 35.695 120.542 1.00 32.37 A
ATOM 4221 O PHEA535 87.113 35.240 121.465 1.0033.53 A
ATOM 4222 N ALAA536 85.405 36.497 120.736 1.0031.84 A
ATOM 4223 CA ALAA 536 85.002 36.908 122.075 1.00 32.11 A
ATOM 4224 CB ALAA536 84.132 38.157 121.992 1.0031.82 A
ATOM 4225 C ALAA 536 84.274 35.815 122.861 1.00 31.78 A
ATOM 4226 O ALAA536 84.269 35.835 124.089 1.0032.29 A
ATOM 4227 N LEU A 537 83.656 34.875 122.152 1.00 30.36 A
ATOM 4228 CA LEU A 537 82.915 33.771 122.775 1.00 31.32 A
ATOM 4229 CB LEU A 537 81.426 33.894 122.439 1.00 30.69 A
ATOM 4230 CG LEU A 537 80.533 34.732 123.353 1.00 31.94 A
ATOM 4231 CD1 LEU A 537 81.276 35.944 123.870 1.00 32.38 A
ATOM 4232 CD2 LEU A 537 79.283 35.127 122.588 1.00 30.63 A
ATOM 4233 C LEU A 537 83.437 32.420 122.283 1.0029.98 A
ATOM 4234 O LEU A 537 82.708 31.657 121.649 1.00 29.18 A
ATOM 4235 N PRO A 538 84.701 32.099 122.595 1.0030.57 A
ATOM 4236 CD PRO A 538 85.580 32.844 123.516 1.00 30.31 A
ATOM 4237 CA PRO A 538 85.335 30.845 122.178 1.00 31.52 A
ATOM 4238 CB PRO A 538 86.765 31.006 122.685 1.0031.61 A
ATOM 4239 CG PRO A 538 86.570 31.790 123.945 1.0030.29 A
ATOM 4240 C PRO A 538 84.693 29.548 122.661 1.00 33.07 A
ATOM 4241 O PRO A 538 84.902 28.499 122.054 1.00 33.11 A
ATOM 4242 N GLN A 539 83.914 29.611 123.739 1.0035.16 A
ATOM 4243 CA GLN A 539 83.279 28.406 124.274 1.00 36.46 A
ATOM 4244 CB GLN A 539 83.475 28.325 125.789 1.0038.05 A
ATOM 4245 CG GLN A 539 84.496 27.289 126.212 1.00 41.79 A
ATOM 4246 CD GLN A 539 85.850 27.528 125.587 1.00 42.76 A
ATOM 4247 OE1 GLN A 539 86.494 28.544 125.849 1.00 45.78 A
ATOM 4248 NE2 GLN A 539 86.291 26.595 124.749 1.00 42.84 A
ATOM 4249 C GLN A 539 81.802 28.263 123.965 1.00 36.40 A
ATOM 4250 O GLN A 539 81.148 27.351 124.467 1.00 36.66 A
ATOM 4251 N GLU A 540 81.264 29.157 123.148 1.00 36.40 A
ATOM 4252 CA GLU A 540 79.854 29.066 122.812 1.00 37.01 A
ATOM 4253 CB GLU A 540 79.243 30.456 122.685 1.00 38.13 A
ATOM 4254 CG GLU A 540 78.975 31.089 124.028 1.00 44.67 A
ATOM 4255 CD GLU A 540 77.735 31.954 124.018 1.00 46.80 A
ATOM 4256 OE1 GLU A 540 76.643 31.429 123.708 1.0048.49 A
ATOM 4257 OE2 GLU A 540 77.853 33.157 124.324 1.0049.54 A
ATOM 4258 C GLU A 540 79.628 28.280 121.537 1.00 35.88 A
ATOM 4259 O GLU A 540 80.384 28.417 120.576 1.00 36.10 A
ATOM 4260 N ASN A 541 78.589 27.451 121.537 1.00 34.44 A
ATOM 4261 CA ASN A 541 78.273 26.647 120.364 1.00 34.93 A
ATOM 4262 CB ASN A 541 77.287 25.518 120.719 1.00 34.88 A
ATOM 4263 CG ASN A 541 75.953 26.037 121.220 1.00 38.27 A
ATOM 4264 OD1 ASN A 541 75.315 26.866 120.573 1.00 41.29 A
ATOM 4265 ND2 ASN A 541 75.516 25.540 122.375 1.00 38.87 A
ATOM 4266 C ASN A 541 77.696 27.520 119.249 1.00 32.77 A
ATOM 4267 O ASN A 541 77.225 28.633 119.491 1.00 31.26 A
ATOM 4268 N ASP A 542 77.749 27.008 118.026 1.00 31.01 A
ATOM 4269 CA ASP A 542 77.238 27.725 116.868 1.00 30.53 A
ATOM 4270 CB ASP A 542 77.393 26.862 115.620 1.00 28.24 A
ATOM 4271 CG ASP A 542 78.825 26.774 115.155 1.00 27.58 A
ATOM 4272 OD1 ASP A 542 79.101 25.967 114.244 1.0026.06 A
ATOM 4273 OD2 ASP A 542 79.669 27.520 115.697 1.00 23.83 A
ATOM 4274 C ASP A 542 75.783 28.145 117.025 1.00 30.39 A
ATOM 4275 O ASPA542 75.406 29.248 116.624 1.00 30.61 A
ATOM 4276 N SERA543 74.970 27.267 117.609 1.00 29.15 A
ATOM 4277 CA SERA 543 73.553 27.557 117.808 1.00 29.50 A
ATOM 4278 CB SER A 543 72.858 26.371 118.482 1.00 30.12 A
ATOM 4279 OG SER A 543 71.465 26.607 118.594 1.0030.10 A
ATOM 4280 C SERA543 73.341 28.822 118.642 1.00 28.77 A
ATOM 4281 O SER A 543 72.491 29.651 118.315 1.0028.98 A
ATOM 4282 N ARG A 544 74.111 28.965 119.716 1.0028.57 A
ATOM 4283 CA ARG A 544 74.005 30.140 120.575 1.0029.34 A
ATOM 4284 CB ARG A 544 74.714 29.899 121.902 1.00 30.61 A
ATOM 4285 CG ARG A 544 73.895 29.058 122.849 1.00 35.27 A
ATOM 4286 CD ARG A 544 74.552 28.980 124.203 1.00 34.89 A
ATOM 4287 NE ARG A 544 73.739 28.208 125.129 1.0034.59 A
ATOM 4288 CZ ARG A 544 74.080 27.975 126.388 1.00 32.06 A
ATOM 4289 NH1 ARG A 544 75.222 28.459 126.859 1.00 31.21 A
ATOM 4290 NH2 ARG A 544 73.280 27.265 127.169 1.00 32.78 A
ATOM 4291 C ARG A 544 74.573 31.385 119.912 1.00 27.36 A
ATOM 4292 O ARG A 544 74.022 32.476 120.069 1.00 25.31 A
ATOM 4293 N LYS A 545 75.680 31.221 119.190 1.0027.73 A
ATOM 4294 CA LYS A 545 76.302 32.334 118.474 1.00 28.48 A
ATOM 4295 CB LYS A 545 77.538 31.861 117.690 1.00 30.79 A
ATOM 4296 CG LYS A 545 78.822 31.590 118.497 1.00 31.39 A
ATOM 4297 CD LYS A 545 79.804 30.770 117.627 1.00 34.03 A
ATOM 4298 CE LYS A 545 81.285 31.097 117.853 1.00 34.65 A
ATOM 4299 NZ LYS A 545 81.870 30.588 119.134 1.00 38.35 A
ATOM 4300 C LYS A 545 75.261 32.894 117.494 1.00 26.12 A
ATOM 4301 O LYS A 545 75.072 34.104 117.397 1.00 27.43 A
ATOM 4302 N ARG A 546 74.575 32.005 116.781 1.00 25.44 A
ATOM 4303 CA ARG A 546 73.560 32.432 115.819 1.00 24.41 A
ATOM 4304 CB ARG A 546 72.977 31.235 115.059 1.00 25.15 A
ATOM 4305 CG ARG A 546 73.941 30.549 114.102 1.0025.85 A
ATOM 4306 CD ARG A 546 73.177 29.703 113.084 1.0029.20 A
ATOM 4307 NE ARG A 546 72.350 28.653 113.688 1.00 31.00 A
ATOM 4308 CZ ARG A 546 72.814 27.500 114.168 1.00 32.00 A
ATOM 4309 NH1 ARG A 546 74.110 27.225 114.126 1.00 29.13 A
ATOM 4310 NH2 ARG A 546 71.974 26.607 114.681 1.00 31.91 A
ATOM 4311 C ARG A 546 72.414 33.203 116.463 1.00 24.60 A
ATOM 4312 O ARG A 546 71.910 34.166 115.885 1.00 23.12 A
ATOM 4313 N SER A 547 71.990 32.781 117.652 1.00 24.13 A
ATOM 4314 CA SERA 547 70.895 33.467 118.323 1.00 25.27 A
ATOM 4315 CB SERA 547 70.431 32.681 119.561 1.00 24.42 A
ATOM 4316 OG SER A 547 71.430 32.659 120.563 1.00 30.36 A
ATOM 4317 C SER A 547 71.354 34.870 118.718 1.00 24.28 A
ATOM 4318 O SERA547 70.580 35.828 118.664 1.00 23.91 A
ATOM 4319 N ARG A 548 72.623 34.988 119.097 1.00 24.66 A
ATOM 4320 CA ARG A 548 73.180 36.276 119.483 1.00 24.19 A
ATOM 4321 CB ARG A 548 74.549 36.087 120.131 1.00 26.04 A
ATOM 4322 CG ARG A 548 74.478 35.607 121.569 1.0029.26 A
ATOM 4323 CD ARG A 548 75.802 35.826 122.270 1.00 35.13 A
ATOM 4324 NE ARG A 548 75.641 35.874 123.721 1.0042.02 A
ATOM 4325 CZ ARG A 548 75.334 34.823 124.468 1.00 42.71 A
ATOM 4326 NH1 ARG A 548 75.159 33.639 123.898 1.00 46.47 A
ATOM 4327 NH2 ARG A 548 75.199 34.954 125.779 1.00 43.76 A
ATOM 4328 C ARG A 548 73.296 37.234 118.297 1.00 24.37 A
ATOM 4329 O ARG A 548 73.019 38.431 118.429 1.0022.91 A
ATOM 4330 N GLN A 549 73.702 36.713 117.142 1.00 23.60 A
ATOM 4331 CA GLN A 549 73.827 37.554 115.957 1.00 23.35 A
ATOM 4332 CB GLN A 549 74.610 36.835 114.854 1.00 22.34 A
ATOM 4333 CG GLN A 549 74.672 37.626 113.555 1.00 22.96 A
ATOM 4334 CD GLN A 549 75.950 37.391 112.785 1.0022.24 A
ATOM 4335 OE1 GLN A 549 76.091 37.844 111.649 1.00 19.82 A
ATOM 4336 NE2 GLN A 549 76.897 36.693 113.402 1.00 18.74 A
ATOM 4337 C GLN A 549 72.441 37.958 115.463 1.00 23.45 A
ATOM 4338 O GLN A 549 72.236 39.098 115.047 1.0025.03 A
ATOM 4339 N ARG A 550 71.483 37.035 115.510 1.00 21.97 A
ATOM 4340 CA ARG A 550 70.127 37.381 115.103 1.00 23.03 A
ATOM 4341 CB ARG A 550 69.204 36.156 115.141 1.00 23.41 A
ATOM 4342 CG ARG A 550 69.488 35.081 114.088 1.0026.67 A
ATOM 4343 CD ARG A 550 68.308 34.106 113.987 1.00 26.87 A
ATOM 4344 NE ARG A 550 67.959 33.523 115.284 1.0027.43 A
ATOM 4345 CZ ARG A 550 68.460 32.386 115.758 1.0027.12 A
ATOM 4346 NH1 ARG A 550 68.085 31.945 116.951 1.00 25.56 A
ATOM 4347 NH2 ARG A 550 69.316 31.674 115.032 1.00 24.90 A
ATOM 4348 C ARG A 550 69.599 38.447 116.082 1.00 23.31 A
ATOM 4349 O ARG A 550 68.848 39.346 115.698 1.0021.78 A
ATOM 4350 N LEU A 551 70.001 38.334 117.348 1.0022.05 A
ATOM 4351 CA LEU A 551 69.584 39.281 118.382 1.0021.16 A
ATOM 4352 CB LEU A 551 70.103 38.835 119.755 1.00 19.62 A
ATOM 4353 CG LEU A 551 69.982 39.825 120.918 1.00 20.89 A
ATOM 4354 CD1 LEU A 551 68.530 40.253 121.085 1.0020.46 A
ATOM 4355 CD2 LEU A 551 70.511 39.180 122.200 1.00 20.40 A
ATOM 4356 C LEU A 551 70.094 40.689 118.080 1.00 20.47 A
ATOM 4357 O LEU A 551 69.368 41.667 118.253 1.00 19.63 A
ATOM 4358 N ALA A 552 71.346 40.784 117.636 1.00 19.79 A
ATOM 4359 CA ALA A 552 71.942 42.074 117.309 1.00 19.63 A
ATOM 4360 CB ALA A 552 73.418 41.900 116.932 1.00 19.93 A
ATOM 4361 C ALA A 552 71.169 42.701 116.151 1.00 20.04 A
ATOM 4362 O ALAA552 70.995 43.917 116.092 1.00 19.40 A
ATOM 4363 N ALA A 553 70.691 41.859 115.240 1.00 19.35 A
ATOM 4364 CA ALAA 553 69.934 42.335 114.092 1.00 18.62 A
ATOM 4365 CB ALA A 553 69.738 41.199 113.090 1.00 20.16 A
ATOM 4366 C ALAA 553 68.582 42.906 114.516 1.00 19.00 A
ATOM 4367 O ALAA553 68.143 43.945 114.005 1.00 18.51 A
ATOM 4368 N ALA A 554 67.918 42.226 115.445 1.00 18.64 A
ATOM 4369 CA ALA A 554 66.619 42.683 115.930 1.00 18.84 A
ATOM 4370 CB ALA A 554 66.028 41.658 116.909 1.00 17.39 A
ATOM 4371 C ALAA554 66.785 44.040116.615 1.00 17.53 A
ATOM 4372 O ALA A 554 65.981 44.954 116.424 1.00 18.81 A
ATOM 4373 N ILE A 555 67.841 44.167 117.408 1.00 17.56 A
ATOM 4374 CA ILE A 555 68.108 45.414 118.108 1.00 17.87 A
ATOM 4375 CB ILE A 555 69.390 45.296 118.974 1.00 17.46 A
ATOM 4376 CG2 ILEA555 69.857 46.663 119.426 1.00 14.65 A
ATOM 4377 CG1 ILE A 555 69.105 44.401 120.191 1.00 16.23 A
ATOM 4378 CD1 ILE A 555 70.342 44.044 120.997 1.00 16.39 A
ATOM 4379 C ILE A 555 68.245 46.561 117.111 1.00 18.92 A
ATOM 4380 O ILE A 555 67.572 47.586 117.236 1.00 18.64 A
ATOM 4381 N ILEA556 69.103 46.374 116.113 1.00 19.41 A
ATOM 4382 CA ILE A 556 69.334 47.396 115.094 1.00 19.69 A
ATOM 4383 CB ILE A 556 70.470 46.984 114.139 1.00 20.43 A
ATOM 4384 CG2 ILE A 556 70.563 47.967 112.974 1.00 23.05 A
ATOM 4385 CG1 ILE A 556 71.796 46.926 114.901 1.00 21.99 A
ATOM 4386 CD1 ILE A 556 72.310 48.274 115.361 1.00 21.62 A
ATOM 4387 C ILE A 556 68.093 47.674 114.258 1.00 20.30 A
ATOM 4388 O ILE A 556 67.699 48.834 114.099 1.00 21.07 A
ATOM 4389 N LEU A 557 67.473 46.616 113.736 1.00 19.33 A
ATOM 4390 CA LEU A 557 66.291 46.766 112.890 1.00 19.45 A
ATOM 4391 CB LEU A 557 65.964 45.443 112.182 1.00 17.26 A
ATOM 4392 CG LEU A 557 66.906 45.055 111.032 1.00 18.31 A
ATOM 4393 CD1 LEU A 557 66.629 43.624 110.581 1.00 13.78 A
ATOM 4394 CD2 LEU A 557 66.735 46.029 109.868 1.00 16.51 A
ATOM 4395 C LEU A 557 65.040 47.302 113.577 1.00 18.21 A
ATOM 4396 O LEU A 557 64.152 47.828 112.913 1.0021.14 A
ATOM 4397 N LEU A 558 64.958 47.179 114.895 1.00 18.54 A
ATOM 4398 CA LEU A 558 63.792 47.684 115.613 1.00 17.17 A
ATOM 4399 CB LEU A 558 63.226 46.604 116.549 1.00 15.13 A
ATOM 4400 CG LEU A 558 62.761 45.321 115.843 1.0013.08 A
ATOM 4401 CD1 LEU A 558 61.985 44.452 116.812 1.00 13.87 A
ATOM 4402 CD2 LEU A 558 61.890 45.666 114.643 1.00 11.57 A
ATOM 4403 C LEU A 558 64.142 48.949 116.394 1.00 18.52 A
ATOM 4404 O LEU A 558 63.407 49.368 117.299 1.00 19.76 A
ATOM 4405 N ALA A 559 65.270 49.554 116.028 1.00 16.48 A
ATOM 4406 CA ALA A 559 65.745 50.788 116.658 1.00 16.28 A
ATOM 4407 CB ALA A 559 67.262 50.734 116.840 1.00 11.94 A
ATOM 4408 C ALAA559 65.386 52.025 115.835 1.0015.93 A
ATOM 4409 O ALA A 559 65.338 51.981 114.601 1.00 16.68 A
ATOM 4410 N GLN A 560 65.125 53.132 116.516 1.00 16.07 A
ATOM 4411 CA GLN A 560 64.839 54.364 115.802 1.00 15.96 A
ATOM 4412 CB GLN A 560 64.476 55.492 116.776 1.00 15.36 A
ATOM 4413 CG GLN A 560 63.229 55.230 117.633 1.00 14.64 A
ATOM 4414 CD GLN A 560 61.999 54.919 116.797 1.00 16.26 A
ATOM 4415 OE1 GLN A 560 61.674 55.647 115.857 1.00 15.78 A
ATOM 4416 NE2 GLN A 560 61.303 53.836 117.140 1.00 15.54 A
ATOM 4417 C GLN A 560 66.159 54.677 115.094 1.00 17.16 A
ATOM 4418 O GLN A 560 67.242 54.379 115.620 1.00 14.67 A
ATOM 4419 N GLY A 561 66.076 55.259 113.904 1.00 17.08 A
ATOM 4420 CA GLY A 561 67.284 55.569 113.169 1.00 18.12 A
ATOM 4421 C GLY A 561 67.258 54.992 111.767 1.00 18.27 A
ATOM 4422 O GLY A 561 66.191 54.681 111.236 1.00 18.20 A
ATOM 4423 N VALA562 68.433 54.849 111.164 1.00 17.59 A
ATOM 4424 CA VAL A 562 68.537 54.322 109.807 1.00 17.72 A
ATOM 4425 CB VAL A 562 69.255 55.344 108.892 1.00 18.23 A
ATOM 4426 CG1 VALA 562 69.214 54.884 107.442 1.00 17.00 A
ATOM 4427 CG2 VAL A 562 68.597 56.717 109.034 1.00 15.87 A
ATOM 4428 C VAL A 562 69.308 52.996 109.815 1.00 18.06 A
ATOM 4429 O VALA562 70.531 52.986 109.906 1.00 19.33 A
ATOM 4430 N PRO A 563 68.595 51.860 109.720 1.00 17.50 A
ATOM 4431 CD PRO A 563 67.133 51.732 109.581 1.00 16.10 A
ATOM 4432 CA PRO A 563 69.238 50.540 109.722 1.00 17.10 A
ATOM 4433 CB PRO A 563 68.052 49.575 109.756 1.00 17.94 A
ATOM 4434 CG PRO A 563 66.983 50.324 109.024 1.00 17.74 A
ATOM 4435 C PROA563 70.166 50.289 108.539 1.00 17.46 A
ATOM 4436 O PROA563 69.846 50.612 107.395 1.00 17.57 A
ATOM 4437 N PHE A 564 71.315 49.695 108.839 1.00 16.19 A
ATOM 4438 CA PHE A 564 72.339 49.400 107.852 1.00 15.31 A
ATOM 4439 CB PHE A 564 73.506 50.378 108.058 1.0015.38 A
ATOM 4440 CG PHE A 564 74.695 50.127 107.180 1.0015.88 A
ATOM 4441 CD1 PHE A 564 75.626 49.149 107.508 1.00 16.97 A
ATOM 4442 CD2 PHE A 564 74.911 50.904 106.046 1.0018.50 A
ATOM 4443 CE1 PHE A 564 76.760 48.952 106.723 1.00 17.35 A
ATOM 4444 CE2 PHE A 564 76.046 50.715 105.249 1.00 15.95 A
ATOM 4445 CZ PHE A 564 76.969 49.740 105.589 1.00 16.57 A
ATOM 4446 C PHE A 564 72.768 47.951 108.058 1.00 16.90 A
ATOM 4447 O PHE A 564 73.078 47.532 109.181 1.00 17.31 A
ATOM 4448 N ILE A 565 72.772 47.190 106.970 1.00 14.63 A
ATOM 4449 CA ILE A 565 73.124 45.780 107.016 1.00 14.87 A
ATOM 4450 CB ILE A 565 71.891 44.913 106.651 1.00 16.81 A
ATOM 4451 CG2 ILE A 565 72.271 43.440 106.608 1.00 14.27 A
ATOM 4452 CG ILE A 565 70.768 45.160 107.664 1.00 16.47 A
ATOM 4453 CD1 ILE A 565 69.402 44.757 107.156 1.00 23.31 A
ATOM 4454 C ILE A 565 74.262 45.420 106.073 1.00 16.19 A
ATOM 4455 O ILE A 565 74.219 45.746 104.883 1.00 16.28 A
ATOM 4456 N HIS A 566 75.281 44.746 106.601 1.00 16.30 A
ATOM 4457 CA HIS A 566 76.400 44.330 105.770 1.00 17.55 A
ATOM 4458 CB HIS A 566 77.623 43.999 106.624 1.00 17.18 A
ATOM 4459 CG HIS A 566 78.828 43.620 105.818 1.00 18.95 A
ATOM 4460 CD2 HIS A 566 79.339 44.144 104.677 1.00 20.11 A
ATOM 4461 ND1 HIS A 566 79.654 42.572 106.160 1.00 18.87 A
ATOM 4462 CE1 HIS A 566 80.620 42.464 105.264 1.0020.64 A
ATOM 4463 NE2 HIS A 566 80.451 43.406 104.353 1.00 20.50 A
ATOM 4464 C HIS A 566 75.974 43.082 104.998 1.00 18.07 A
ATOM 4465 O HIS A 566 75.425 42.149 105.580 1.00 19.00 A
ATOM 4466 N SERA567 76.225 43.064 103.694 1.00 17.39 A
ATOM 4467 CA SER A 567 75.855 41.920 102.863 1.00 16.64 A
ATOM 4468 CB SER A 567 76.396 42.103 101.442 1.00 18.16 A
ATOM 4469 OG SER A 567 75.908 41.085 100.582 1.00 21.04 A
ATOM 4470 C SER A 567 76.393 40.610 103.449 1.00 15.63 A
ATOM 4471 O SER A 567 77.599 40.471 103.674 1.00 12.94 A
ATOM 4472 N GLY A 568 75.492 39.659 103.694 1.00 16.22 A
ATOM 4473 CA GLY A 568 75.891 38.374 104.249 1.00 17.15 A
ATOM 4474 C GLY A 568 75.736 38.297 105.758 1.00 19.61 A
ATOM 4475 O GLY A 568 75.801 37.216 106.350 1.00 20.15 A
ATOM 4476 N GLN A 569 75.538 39.449 106.389 1.0019.58 A
ATOM 4477 CA GLN A 569 75.363 39.499 107.833 1.00 21.38 A
ATOM 4478 CB GLN A 569 75.130 40.945 108.290 1.0020.22 A
ATOM 4479 CG GLN A 569 75.145 41.159 109.799 1.0021.53 A
ATOM 4480 CD GLN A 569 74.720 42.571 110.188 1.00 22.59 A
ATOM 4481 OE1 GLN A 569 75.104 43.552 109.540 1.0021.95 A
ATOM 4482 NE2 GLN A 569 73.934 42.682 111.254 1.0021.93 A
ATOM 4483 C GLN A 569 74.146 38.644 108.177 1.00 20.54 A
ATOM 4484 O GLN A 569 74.141 37.932 109.182 1.00 21.45 A
ATOM 4485 N GLU A 570 73.129 38.713 107.321 1.00 19.16 A
ATOM 4486 CA GLU A 570 71.891 37.966 107.524 1.00 20.65 A

ATOM 4487 CB GLU A 570 70.900 38.224 106.380 1.00 19.76 A
ATOM 4488 CG GLU A 570 71.370 37.726 105.030 1.00 21.24 A
ATOM 4489 CD GLU A 570 72.138 38.772 104.246 1.00 23.09 A
ATOM 4490 OE1 GLU A 570 72.817 39.611 104.880 1.00 24.31 A
ATOM 4491 OE2 GLU A 570 72.071 38.742 102.994 1.00 18.72 A
ATOM 4492 C GLU A 570 72.140 36.470 107.650 1.00 21.16 A
ATOM 4493 O GLU A 570 71.346 35.759 108.270 1.00 22.21 A
ATOM 4494 N PHE A 571 73.218 35.981 107.041 1.00 21.82 A
ATOM 4495 CA PHE A 571 73.538 34.567 107.160 1.0021.07 A
ATOM 4496 CB PHE A 571 73.332 33.795 105.831 1.00 21.21 A
ATOM 4497 CG PHE A 571 74.072 34.348 104.642 1.0020.94 A
ATOM 4498 CD1 PHE A 571 75.457 34.243 104.541 1.00 20.56 A
ATOM 4499 CD2 PHE A 571 73.366 34.908 103.579 1.0021.74 A
ATOM 4500 CE1 PHE A 571 76.132 34.682 103.393 1.0020.13 A
ATOM 4501 CE2 PHE A 571 74.033 35.352 102.422 1.00 21.54 A
ATOM 4502 CZ PHE A 571 75.419 35.235 102.332 1.00 17.69 A
ATOM 4503 C PHE A 571 74.918 34.320 107.758 1.00 22.05 A
ATOM 4504 O PHE A 571 75.693 33.493 107.286 1.0021.23 A
ATOM 4505 N PHE A 572 75.204 35.071 108.818 1.00 22.09 A
ATOM 4506 CA PHE A 572 76.438 34.930 109.574 1.00 22.31 A
ATOM 4507 CB PHE A 572 76.320 33.664 110.425 1.00 21.69 A
ATOM 4508 CG PHE A 572 74.962 33.495 111.038 1.0020.46 A
ATOM 4509 CD1 PHE A 572 74.559 34.298 112.104 1.00 21.57 A
ATOM 4510 CD2 PHE A 572 74.055 32.584 110.505 1.00 20.24 A
ATOM 4511 CE1 PHE A 572 73.272 34.200 112.627 1.00 19.82 A
ATOM 4512 CE2 PHE A 572 72.763 32.479 111.019 1.00 18.96 A
ATOM 4513 CZ PHE A 572 72.370 33.287 112.080 1.00 20.35 A
ATOM 4514 C PHEA572 77.706 34.891 108.736 1.00 22.01 A
ATOM 4515 O PHE A 572 78.595 34.070 108.967 1.0021.06 A
ATOM 4516 N ARG A 573 77.797 35.802 107.777 1.00 22.31 A
ATOM 4517 CA ARG A 573 78.960 35.868 106.909 1.00 21.85 A
ATOM 4518 CB ARGA573 78.938 37.174 106.118 1.00 22.67 A
ATOM 4519 CG ARG A 573 80.145 37.371 105.225 1.0022.10 A
ATOM 4520 CD ARG A 573 80.021 38.651 104.418 1.0021.70 A
ATOM 4521 NE ARG A 573 81.082 38.762 103.421 1.00 21.13 A
ATOM 4522 CZ ARG A 573 81.167 39.740 102.528 1.00 19.59 A
ATOM 4523 NH1 ARG A 573 80.253 40.698 102.506 1.00 19.45 A
ATOM 4524 NH2 ARG A 73 82.163 39.756 101.654 1.00 20.03 A
ATOM 4525 C ARG A 573 80.266 35.769 107.701 1.00 22.67 A
ATOM 4526 O ARG A 573 80.453 36.447 108.714 1.00 19.72 A
ATOM 4527 N THR A 574 81.164 34.911 107.235 1.00 21.10 A
ATOM 4528 CA THR A 574 82.454 34.743 107.881 1.00 21.09 A
ATOM 4529 CB THRA 574 82.643 33.297 108.434 1.00 21.30 A
ATOM 4530 OG1 THR A 574 83.998 33.124 108.869 1.00 22.12 A
ATOM 4531 CG2 THR A 574 82.330 32.252 107.364 1.00 18.94 A
ATOM 4532 C THR A 574 83.553 35.024 106.868 1.0020.67 A
ATOM 4533 O THRA 574 83.377 34.777 105.677 1.0020.78 A
ATOM 4534 N LYS A 575 84.674 35.555 107.346 1.00 21.49 A
ATOM 4535 CA LYS A 575 85.829 35.853 106.501 1.00 22.38 A
ATOM 4536 CB LYS A 575 86.158 37.353 106.540 1.00 21.81 A
ATOM 4537 CG LYS A 575 85.210 38.229 105.738 1.0020.72 A
ATOM 4538 CD LYS A 575 85.545 39.691 105.930 1.0019.00 A
ATOM 4539 CE LYS A 575 84.610 40.592 105.152 1.00 17.56 A
ATOM 4540 NZ LYS A 575 84.944 42.021 105.418 1.00 17.89 A
ATOM 4541 C LYS A 575 87.044 35.054 106.984 1.0023.23 A
ATOM 4542 O LYS A 575 88.186 35.486 106.822 1.00 20.74 A
ATOM 4543 N GLN A 576 86.788 33.889 107.575 1.0025.88 A
ATOM 4544 CA GLN A 576 87.859 33.033 108.084 1.00 29.34 A
ATOM 4545 CB GLN A 576 88.761 32.566 106.939 1.0031.52 A
ATOM 4546 CG GLN A 576 88.401 31.206 106.384 1.00 37.49 A
ATOM 4547 CD GLN A 576 86.918 31.052 106.138 1.00 40.05 A
ATOM 4548 OE1 GLN A 576 86.334 31.777 105.333 1.00 44.45 A
ATOM 4549 NE2 GLN A 576 86.296 30.105 106.834 1.00 39.61 A
ATOM 4550 C GLN A 576 88.707 33.730 109.140 1.00 29.25 A
ATOM 4551 O GLN A 576 89.894 33.432 109.288 1.00 30.44 A
ATOM 4552 N GLY A 577 88.100 34.666 109.864 1.00 28.90 A
ATOM 4553 CA GLY A 577 88.819 35.373 110.909 1.00 26.94 A
ATOM 4554 C GLY A 577 89.625 36.572 110.453 1.00 26.81 A
ATOM 4555 O GLY A 577 90.247 37.240 111.279 1.00 27.03 A
ATOM 4556 N VALA 578 89.627 36.844 109.149 1.0025.37 A
ATOM 4557 CA VAL A 578 90.362 37.987 108.602 1.00 24.94 A
ATOM 4558 CB VAL A 578 90.355 37.963 107.051 1.0025.10 A
ATOM 4559 CG1 VAL A 578 91.066 39.193 106.497 1.00 23.25 A
ATOM 4560 CG2 VAL A 578 91.034 36.692 106.552 1.00 24.63 A
ATOM 4561 C VAL A 578 89.733 39.296 109.084 1.0025.55 A
ATOM 4562 O VAL A 578 88.536 39.521 108.906 1.0024.67 A
ATOM 4563 N GLU A 579 90.543 40.158 109.691 1.00 25.86 A
ATOM 4564 CA GLU A 579 90.061 41.438 110.203 1.00 27.39 A
ATOM 4565 CB GLU A 579 90.982 41.924 111.327 1.00 30.18 A
ATOM 4566 CG GLU A 579 90.626 43.301 111.869 1.00 37.43 A
ATOM 4567 CD GLU A 579 91.436 43.672 113.092 1.00 42.42 A
ATOM 4568 OE1 GLU A 579 91.274 43.011 114.138 1.00 44.01 A
ATOM 4569 OE2 GLU A 579 92.241 44.623 113.010 1.00 47.55 A
ATOM 4570 C GLU A 579 89.924 42.550 109.152 1.00 26.56 A
ATOM 4571 O GLUA579 88.951 43.311 109.164 1.00 25.69 A
ATOM 4572 N ASN A 580 90.900 42.639 108.255 1.00 25.71 A
ATOM 4573 CA ASN A 580 90.919 43.674 107.222 1.00 24.68 A
ATOM 4574 CB ASN A 580 92.071 44.639 107.513 1.0025.44 A
ATOM 4575 CG ASN A 580 92.039 45.874 106.643 1.00 25.77 A
ATOM 4576 OD1 ASN A 580 93.019 46.609 06.566 1.00 27.61 A
ATOM 4577 ND2 ASN A 580 90.911 46.116 105.995 1.00 26.88 A
ATOM 4578 C ASN A 580 91.122 43.003 105.867 1.00 23.20 A
ATOM 4579 O ASN A 580 92.253 42.850 105.402 1.00 20.89 A
ATOM 4580 N SER A 581 90.021 42.619 105.228 1.0022.28 A
ATOM 4581 CA SER A 581 90.091 41.909 103.955 1.0022.64 A
ATOM 4582 CB SER A 581 88.889 40.965 103.834 1.00 22.06 A
ATOM 4583 OG SER A 581 87.669 41.689 103.757 1.00 20.28 A
ATOM 4584 C SER A 581 90.174 42.760 102.690 1.00 23.39 A
ATOM 4585 O SER A 581 89.769 42.296 101.626 1.00 23.68 A
ATOM 4586 N TYRA582 90.719 43.972 102.785 1.0022.49 A
ATOM 4587 CA TYR A 582 90.795 44.855 101.620 1.00 24.07 A
ATOM 4588 CB TYR A 582 91.470 46.188 101.982 1.00 24.12 A
ATOM 4589 CG TYRA582 92.971 46.124 102.164 1.0024.26 A
ATOM 4590 CD1 TYR A 582 93.534 45.619 103.336 1.00 23.18 A
ATOM 4591 CE1 TYR A 582 94.916 45.573 103.508 1.0023.26 A
ATOM 4592 CD2 TYRA582 93.829 46.581 101.163 1.00 25.89 A
ATOM 4593 CE2 TYR A 582 95.217 46.539 101.323 1.0024.52 A
ATOM 4594 CZ TYR A 582 95.752 46.037 102.497 1.00 24.58 A
ATOM 4595 OH TYR A 582 97.117 46.013 102.665 1.00 24.12 A
ATOM 4596 C TYR A 582 91.458 44.279 100.366 1.00 23.73 A
ATOM 4597 O TYR A 582 91.103 44.669 99.257 1.00 25.39 A
ATOM 4598 N GLN A 583 92.411 43.364 100.525 1.00 24.19 A
ATOM 4599 CA GLN A 583 93.084 42.768 99.366 1.00 24.81 A
ATOM 4600 CB GLN A 583 94.522 43.277 99.263 1.00 25.46 A
ATOM 4601 CG GLN A 583 95.375 42.875 100.459 1.00 29.26 A
ATOM 4602 CD GLN A 583 96.828 43.299 100.349 1.00 30.97 A
ATOM 4603 OE1 GLN A 583 97.607 43.106 101.283 1.00 33.46 A
ATOM 4604 NE2 GLN A 583 97.204 43.877 99.210 1.00 30.60 A
ATOM 4605 C GLN A 583 93.111 41.243 99.443 1.00 24.65 A
ATOM 4606 O GLN A 583 93.878 40.592 98.734 1.00 25.36 A
ATOM 4607 N SER A 584 92.278 40.670 100.302 1.00 24.91 A
ATOM 4608 CA SERA 584 92.239 39.218 100.455 1.00 24.12 A
ATOM 4609 CB SER A 584 91.515 38.845 101.747 1.00 23.22 A
ATOM 4610 OG SER A 584 92.223 39.348 102.864 1.00 23.35 A
ATOM 4611 C SERA584 91.563 38.559 99.268 1.0024.28 A
ATOM 4612 O SER A 584 90.778 39.188 98.561 1.0024.31 A
ATOM 4613 N SER A 585 91.864 37.284 99.058 1.00 23.66 A
ATOM 4614 CA SER A 585 91.308 36.539 97.936 1.00 23.79 A
ATOM 4615 CB SERA 585 91.948 35.155 97.862 1.0022.00 A
ATOM 4616 OG SERA585 91.510 34.363 98.946 1.0023.19 A
ATOM 4617 C SER A 585 89.794 36.387 98.014 1.00 23.09 A
ATOM 4618 O SERA585 89.177 36.672 99.041 1.00 23.19 A
ATOM 4619 N ASP A 586 89.211 35.927 96.912 1.0021.96 A
ATOM 4620 CA ASP A 586 87.775 35.715 96.814 1.00 23.51 A
ATOM 4621 CB ASP A 586 87.407 35.297 95.389 1.00 23.32 A
ATOM 4622 CG ASP A 586 87.611 36.417 94.384 1.00 24.02 A
ATOM 4623 OD1 ASP A 586 86.996 37.487 94.563 1.00 23.29 A
ATOM 4624 OD2 ASP A 586 88.380 36.229 93.418 1.00 24.67 A
ATOM 4625 C ASP A 586 87.252 34.668 97.794 1.00 25.16 A
ATOM 4626 O ASP A 586 86.111 34.757 98.250 1.0026.24 A
ATOM 4627 N SER A 587 88.069 33.666 98.109 1.0025.37 A
ATOM 4628 CA SERA 587 87.635 32.633 99.043 1.0027.46 A
ATOM 4629 CB SERA587 88.710 31.554 99.202 1.0026.56 A
ATOM 4630 OG SER A 587 89.927 32.113 99.660 1.00 34.00 A
ATOM 4631 C SER A 587 87.361 33.303 100.381 1.0026.96 A
ATOM 4632 O SER A 587 86.518 32.855 101.155 1.0029.73 A
ATOM 4633 N ILEA 588 88.078 34.388 100.640 1.0025.22 A
ATOM 4634 CA ILE A 588 87.903 35.143 101.869 1.00 22.61 A
ATOM 4635 CB ILE A 588 89.167 35.968 102.202 1.00 21.92 A
ATOM 4636 CG2 ILE A 588 88.902 36.877 103.401 1.00 23.21 A
ATOM 4637 CG1 ILE A 588 90.342 35.033 102.492 1.00 21.81 A
ATOM 4638 CD1 ILE A 588 90.072 34.028 103.611 1.00 19.11 A
ATOM 4639 C ILEA588 86.726 36.114 101.751 1.00 23.79 A
ATOM 4640 O ILE A 588 85.862 36.165 102.627 1.00 23.73 A
ATOM 4641 N ASNA589 86.682 36.859 100.649 1.00 22.41 A
ATOM 4642 CA ASN A 589 85.656 37.878 100.449 1.0022.59 A
ATOM 4643 CB ASN A 589 86.280 39.081 99.748 1.0022.05 A
ATOM 4644 CG ASN A 589 87.224 39.838 100.638 1.00 22.52 A
ATOM 4645 OD1 ASN A 589 86.831 40.319 101.700 1.00 23.98 A
ATOM 4646 ND2 ASN A 589 88.479 39.954 100.214 1.00 20.70 A
ATOM 4647 C ASN A 589 84.347 37.561 99.738 1.0022.67 A
ATOM 4648 O ASN A 589 83.393 38.327 99.847 1.0022.58 A
ATOM 4649 N GLN A 590 84.281 36.456 99.013 1.00 22.78 A
ATOM 4650 CA GLN A 590 83.060 36.155 98.283 1.00 23.40 A
ATOM 4651 CB GLN A 590 83.240 34.873 97.464 1.0025.57 A
ATOM 4652 CG GLN A 590 83.144 33.586 98.262 1.0029.86 A
ATOM 4653 CD GLN A 590 83.592 32.381 97.461 1.0031.72 A
ATOM 4654 OE1 GLN A 590 83.349 32.293 96.257 1.00 35.73 A
ATOM 4655 NE2 GLN A 590 84.242 31.440 98.129 1.00 34.43 A
ATOM 4656 C GLN A 590 81.835 36.036 99.179 1.0020.80 A
ATOM 4657 O GLN A 590 81.940 35.693 100.356 1.00 20.40 A
ATOM 4658 N LEU A 591 80.677 36.363 98.621 1.00 19.60 A
ATOM 4659 CA LEU A 591 79.431 36.241 99.354 1.00 20.39 A
ATOM 4660 CB LEU A 591 78.321 37.070 98.695 1.00 21.05 A
ATOM 4661 CG LEU A 591 77.425 37.954 99.577 1.00 21.15 A
ATOM 4662 CD1 LEU A 591 76.004 37.856 99.065 1.00 18.59 A
ATOM 4663 CD2 LEU A 591 77.484 37.532 101.038 1.00 19.40 A
ATOM 4664 C LEU A 591 79.138 34.754 99.197 1.00 20.56 A
ATOM 4665 O LEU A 591 78.941 34.273 98.082 1.0020.76 A
ATOM 4666 N ASP A 592 79.136 34.026 100.306 1.00 20.98 A
ATOM 4667 CA ASP A 592 78.916 32.588 100.274 1.00 20.21 A
ATOM 4668 CB ASP A 592 79.439 31.957 101.560 1.0020.14 A
ATOM 4669 CG ASP A 592 79.545 30.451 101.461 1.00 21.92 A
ATOM 4670 OD1 ASP A 592 78.954 29.872 100.521 1.00 17.40 A
ATOM 4671 OD2 ASP A 592 80.215 29.851 102.326 1.00 22.89 A
ATOM 4672 C ASP A 592 77.455 32.206 100.096 1.0021.42 A
ATOM 4673 O ASP A 592 76.725 32.106 101.077 1.00 20.28 A
ATOM 4674 N TRP A 593 77.031 31.973 98.854 1.00 22.29 A
ATOM 4675 CA TRP A 593 75.640 31.604 98.610 1.0022.74 A
ATOM 4676 CB TRP A 593 75.293 31.752 97.125 1.0020.56 A
ATOM 4677 CG TRP A 593 75.278 33.191 96.687 1.00 19.84 A
ATOM 4678 CD2 TRP A 593 74.223 34.141 96.899 1.00 19.21 A
ATOM 4679 CE2 TRP A 593 74.668 35.383 96.385 1.00 18.27 A
ATOM 4680 CE3 TRP A 593 72.945 34.064 97.478 1.00 17.99 A
ATOM 4681 CD1 TRP A 593 76.290 33.877 96.070 1.00 19.48 A
ATOM 4682 NE1 TRP A 593 75.931 35.193 95.887 1.00 19.69 A
ATOM 4683 CZ2 TRP A 593 73.880 36.541 96.429 1.00 16.91 A
ATOM 4684 CZ3 TRP A 593 72.159 35.217 97.524 1.00 17.95 A
ATOM 4685 CH2 TRP A 593 72.634 36.441 96.999 1.00 17.26 A
ATOM 4686 C TRP A 593 75.295 30.202 99.118 1.00 25.22 A
ATOM 4687 O TRP A 593 74.118 29.847 99.213 1.00 25.81 A
ATOM 4688 N ASP A 594 76.316 29.407 99.441 1.0026.12 A
ATOM 4689 CA ASP A 594 76.097 28.068 99.997 1.00 26.69 A
ATOM 4690 CB ASP A 594 77.403 27.257 100.046 1.00 25.13 A
ATOM 4691 CG ASP A 594 77.763 26.623 98.709 1.00 27.15 A
ATOM 4692 OD1 ASP A 594 78.862 26.032 98.618 1.00 27.43 A
ATOM 4693 OD2 ASP A 594 76.957 26.708 97.755 1.00 25.34 A
ATOM 4694 C ASP A 594 75.601 28.300 101.429 1.0026.57 A
ATOM 4695 O ASP A 594 74.657 27.654 101.891 1.00 27.19 A
ATOM 4696 N ARG A 595 76.252 29.233 102.120 1.00 25.45 A
ATOM 4697 CA ARG A 595 75.882 29.571 103.487 1.00 26.15 A
ATOM 4698 CB ARG A 595 76.915 30.537 104.095 1.0025.76 A
ATOM 4699 CG ARG A 595 76.598 30.966 105.520 1.00 26.44 A
ATOM 4700 CD ARG A 595 77.758 31.685 106.200 1.0027.74 A
ATOM 4701 NE ARG A 595 78.805 30.754 106.597 1.00 32.41 A
ATOM 4702 CZ ARG A 595 79.351 30.699 107.810 1.0029.54 A
ATOM 4703 NH1 ARG A 595 78.958 31.527 108.768 1.00 29.65 A
ATOM 4704 NH2 ARG A 595 80.289 29.803 108.067 1.00 29.55 A
ATOM 4705 C ARG A 595 74.474 30.182 103.531 1.00 24.64 A
ATOM 4706 O ARG A 595 73.737 29.970 104.490 1.0025.00 A
ATOM 4707 N ARG A 596 74.096 30.927 102.493 1.0023.80 A
ATOM 4708 CA ARG A 596 72.767 31.531 102.449 1.00 24.94 A
ATOM 4709 CB ARG A 596 72.627 32.477 101.238 1.00 25.18 A
ATOM 4710 CG ARG A 596 72.009 31.864 99.984 1.0030.47 A
ATOM 4711 CD ARG A 596 70.600 32.397 99.707 1.0030.56 A
ATOM 4712 NE ARG A 596 69.724 32.195 100.853 1.00 34.27 A
ATOM 4713 CZ ARG A 596 68.417 32.442 100.871 1.00 32.36 A
ATOM 4714 NH1 ARG A 596 67.790 32.906 99.798 1.00 28.43 A
ATOM 4715 NH2 ARG A 596 67.736 32.223 101.984 1.00 30.29 A
ATOM 4716 C ARG A 596 71.701 30.428 102.396 1.00 25.67 A
ATOM 4717 O ARG A 596 70.589 30.610 102.898 1.0021.60 A
ATOM 4718 N GLU A 597 72.048 29.290 101.785 1.0025.95 A
ATOM 4719 CA GLU A 597 71.128 28.151 101.691 1.00 27.98 A
ATOM 4720 CB GLU A 597 71.636 27.071 100.726 1.00 29.44 A
ATOM 4721 CG GLU A 597 71.894 27.471 99.301 1.0034.45 A
ATOM 4722 CD GLU A 597 72.339 26.276 98.461 1.0036.45 A
ATOM 4723 OE1 GLU A 597 73.156 25.470 98.959 1.0036.79 A
ATOM 4724 OE2 GLU A 597 71.882 26.146 97.305 1.00 37.44 A
ATOM 4725 C GLU A 597 71.031 27.490 103.061 1.0025.91 A
ATOM 4726 O GLU A 597 69.947 27.252 103.581 1.0025.89 A
ATOM 4727 N THR A 598 72.189 27.178 103.625 1.00 25.17 A
ATOM 4728 CA THR A 598 72.247 26.524 104.915 1.00 26.57 A
ATOM 4729 CB THR A 598 73.699 26.391 105.392 1.00 25.13 A
ATOM 4730 OG1 THR A 598 74.438 25.625 104.434 1.00 24.89 A
ATOM 4731 CG2 THR A 598 73.757 25.692 106.750 1.00 24.70 A
ATOM 4732 C THRA598 71.433 27.248 105.975 1.0027.39 A
ATOM 4733 O THR A 598 70.704 26.619 106.740 1.0028.12 A
ATOM 4734 N PHE A 599 71.540 28.572 106.009 1.00 28.31 A
ATOM 4735 CA PHE A 599 70.819 29.358 107.003 1.00 27.83 A
ATOM 4736 CB PHE A 599 71.812 30.250 107.740 1.00 26.90 A
ATOM 4737 CG PHE A 599 72.939 29.483 108.364 1.0028.10 A
ATOM 4738 CD1 PHE A 599 72.702 28.636 109.444 1.00 28.48 A
ATOM 4739 CD2 PHE A 599 74.218 29.537 107.825 1.00 28.08 A
ATOM 4740 CE1 PHE A 599 73.721 27.851 109.971 1.00 27.52 A
ATOM 4741 CE2 PHE A 599 75.244 28.755 108.346 1.00 28.08 A
ATOM 4742 CZ PHE A 599 74.993 27.910 109.419 1.00 28.13 A
ATOM 4743 C PHE A 599 69.677 30.179 106.428 1.0027.77 A
ATOM 4744 O PHE A 599 69.423 31.298 106.864 1.0029.62 A
ATOM 4745 N LYS A 600 68.975 29.608 105.457 1.00 28.27 A
ATOM 4746 CA LYS A 600 67.859 30.293 104.828 1.00 30.12 A
ATOM 4747 CB LYS A 600 67.217 29.391 103.772 1.0032.46 A
ATOM 4748 CG LYS A 600 66.537 28.158 104.340 1.00 37.11 A
ATOM 4749 CD LYS A 600 65.983 27.258 103.234 1.0041.17 A
ATOM 4750 CE LYS A 600 67.097 26.577 102.430 1.0043.90 A
ATOM 4751 NZ LYS A 600 67.886 25.604 103.250 1.0044.32 A
ATOM 4752 C LYSA600 66.813 30.700 105.862 1.0030.32 A
ATOM 4753 O LYSA600 66.121 31.705 105.693 1.00 29.30 A
ATOM 4754 N GLU A 601 66.698 29.921 106.936 1.00 30.36 A
ATOM 4755 CA GLU A 601 65.720 30.221 107.978 1.00 30.55 A
ATOM 4756 CB GLU A 601 65.558 29.020 108.916 1.00 33.16 A
ATOM 4757 CG GLU A 601 65.013 27.789 108.194 1.00 37.64 A
ATOM 4758 CD GLU A 601 63.695 28.065 107.465 1.00 40.27 A
ATOM 4759 OE1 GLU A 601 63.403 27.350 106.479 1.00 40.65 A
ATOM 4760 OE2 GLU A 601 62.951 28.986 107.881 1.00 40.48 A
ATOM 4761 C GLU A 601 66.106 31.470 108.761 1.00 28.72 A
ATOM 4762 O GLU A 601 65.260 32.318 109.044 1.00 27.10 A
ATOM 4763 N ASP A 602 67.385 31.580 109.106 1.0026.76 A
ATOM 4764 CA ASP A 602 67.882 32.742 109.828 1.00 25.21 A
ATOM 4765 CB ASP A 602 69.356 32.541 110.170 1.0026.12 A
ATOM 4766 CG ASP A 602 69.583 31.316 111.032 1.0029.88 A
ATOM 4767 OD1 ASP A 602 69.149 31.326 112.203 1.00 30.35 A
ATOM 4768 OD2 ASP A 602 70.184 30.337 110.538 1.00 33.41 A
ATOM 4769 C ASP A 602 67.693 33.957 108.917 1.00 23.72 A
ATOM 4770 O ASP A 602 67.208 35.009 109.346 1.00 21.17 A
ATOM 4771 N VAL A 603 68.060 33.799 107.648 1.00 20.44 A
ATOM 4772 CA VAL A 603 67.896 34.880 106.685 1.00 20.45 A
ATOM 4773 CB VAL A 603 68.239 34.419 105.258 1.00 17.58 A
ATOM 4774 CG1 VAL A 603 67.807 35.472 104.247 1.00 15.72 A
ATOM 4775 CG2 VAL A 603 69.729 34.158 105.152 1.00 18.05 A
ATOM 4776 C VALA603 66.441 35.324 106.718 1.00 22.56 A
ATOM 4777 O VAL A 603 66.138 36.520 106.695 1.00 20.54 A
ATOM 4778 N HIS A 604 65.543 34.345 106.794 1.00 24.91 A
ATOM 4779 CA HIS A 604 64.112 34.621 106.823 1.00 27.25 A
ATOM 4780 CB HIS A 604 63.327 33.310 106.773 1.00 30.65 A
ATOM 4781 CG HIS A 604 61.850 33.498 106.641 1.00 35.12 A
ATOM 4782 CD2 HIS A 604 61.085 33.811 105.568 1.00 37.32 A
ATOM 4783 ND1 HIS A 604 60.985 33.385 107.709 1.00 37.79 A
ATOM 4784 CE1 HIS A 604 59.750 33.620 107.300 1.00 39.46 A
ATOM 4785 NE2 HIS A 604 59.784 33.881 106.005 1.00 40.52 A
ATOM 4786 C HIS A 604 63.702 35.434 108.050 1.00 26.56 A
ATOM 4787 O HIS A 604 62.780 36.247 107.977 1.0025.88 A
ATOM 4788 N TYR A 605 64.384 35.216 109.173 1.00 25.83 A
ATOM 4789 CA TYR A 605 64.079 35.956 110.396 1.00 26.81 A
ATOM 4790 CB TYRA605 64.949 35.451 111.554 1.00 26.55 A
ATOM 4791 CG TYR A 605 64.724 36.161 112.879 1.00 27.04 A
ATOM 4792 CD1 TYR A 605 65.701 37.000 113.419 1.00 26.19 A
ATOM 4793 CE1 TYR A 605 65.516 37.630 114.648 1.0025.84 A
ATOM 4794 CD2 TYR A 605 63.546 35.973 113.604 1.0026.74 A
ATOM 4795 CE2 TYR A 605 63.348 36.600 114.838 1.0027.93 A
ATOM 4796 CZ TYR A 605 64.337 37.425 115.353 1.0027.87 A
ATOM 4797 OH TYR A 605 64.148 38.038 116.573 1.00 25.19 A
ATOM 4798 C TYR A 605 64.348 37.439 110.140 1.00 27.26 A
ATOM 4799 O TYR A 605 63.519 38.303 110.453 1.0026.00 A
ATOM 4800 N ILE A 606 65.511 37.724 109.560 1.0027.07 A
ATOM 4801 CA ILEA606 65.891 39.094 109.240 1.00 27.15 A
ATOM 4802 CB ILEA606 67.315 39.146 108.622 1.00 28.45 A
ATOM 4803 CG2 ILE A 606 67.513 40.435 107.845 1.00 28.85 A
ATOM 4804 CG1 ILE A 606 68.367 39.065 109.732 1.00 29.62 A
ATOM 4805 CD1 ILE A 606 68.326 37.805 110.533 1.00 33.42 A
ATOM 4806 C ILE A 606 64.876 39.684 108.265 1.00 26.03 A
ATOM 4807 O ILE A 606 64.509 40.855 108.364 1.00 25.36 A
ATOM 4808 N ARG A 607 64.411 38.861 107.331 1.00 25.53 A
ATOM 4809 CA ARG A 607 63.430 39.309 106.352 1.00 25.85 A
ATOM 4810 CB ARG A 607 63.169 38.204 105.327 1.00 27.07 A
ATOM 4811 CG ARG A 607 62.129 38.552 104.286 1.0029.78 A
ATOM 4812 CD ARG A 607 61.905 37.380 103.348 1.00 33.48 A
ATOM 4813 NE ARG A 607 60.812 37.621 102.407 1.0037.07 A
ATOM 4814 CZ ARG A 607 60.386 36.730 101.514 1.00 39.04 A
ATOM 4815 NH1 ARG A 607 60.963 35.535 101.439 1.00 40.00 A
ATOM 4816 NH2 ARG A 607 59.385 37.028 100.695 1.00 38.65 A
ATOM 4817 C ARG A 607 62.125 39.703 107.044 1.00 26.15 A
ATOM 4818 O ARGA607 61.485 40.684 106.661 1.0025.55 A
ATOM 4819 N ARGA608 61.731 38.943 108.065 1.0026.14 A
ATOM 4820 CA ARG A 608 60.501 39.251 108.795 1.00 27.19 A
ATOM 4821 CB ARG A 608 60.058 38.057 109.648 1.0031.61 A
ATOM 4822 CG ARG A 608 59.557 36.881 108.807 1.00 38.05 A
ATOM 4823 CD ARG A 608 58.332 36.222 109.424 1.0044.00 A
ATOM 4824 NE ARG A 608 57.260 37.187 109.678 1.00 48.55 A
ATOM 4825 CZ ARG A 608 56.037 36.859 110.090 1.00 50.83 A
ATOM 4826 NH1 ARG A 608 55.717 35.585 110.293 1.00 51.00 A
ATOM 4827 NH2 ARG A 608 55.135 37.808 110.310 1.00 50.72 A
ATOM 4828 C ARG A 608 60.678 40.492 109.663 1.00 24.27 A
ATOM 4829 O ARG A 608 59.743 41.272 109.839 1.00 24.31 A
ATOM 4830 N LEU A 609 61.879 40.676 110.203 1.00 21.17 A
ATOM 4831 CA LEU A 609 62.163 41.858 111.011 1.00 19.74 A
ATOM 4832 CB LEU A 609 63.567 41.771 111.617 1.00 18.29 A
ATOM 4833 CG LEU A 609 63.757 40.804 112.792 1.00 17.24 A
ATOM 4834 CD1 LEU A 609 65.238 40.672 113.101 1.00 14.96 A
ATOM 4835 CD2 LEU A 609 63.000 41.317 114.013 1.00 17.24 A
ATOM 4836 C LEU A 609 62.061 43.094 110.109 1.00 19.46 A
ATOM 4837 O LEU A 609 61.547 44.132 110.516 1.00 16.83 A
ATOM 4838 N ILE A 610 62.542 42.976 108.874 1.00 19.02 A
ATOM 4839 CA ILE A 610 62.485 44.099 107.943 1.00 19.00 A
ATOM 4840 CB ILE A 610 63.385 43.837 106.695 1.00 17.59 A
ATOM 4841 CG2 ILE A 610 63.162 44.921 105.631 1.00 16.97 A
ATOM 4842 CG1 ILE A 610 64.858 43.816 107.136 1.00 15.56 A
ATOM 4843 CD1 ILE A 610 65.844 43.334 106.079 1.00 15.37 A
ATOM 4844 C ILE A 610 61.036 44.373 107.541 1.00 19.05 A
ATOM 4845 O ILE A 610 60.613 45.527 107.481 1.00 19.78 A
ATOM 4846 N SER A 611 60.270 43.318 107.279 1.00 19.47 A
ATOM 4847 CA SER A 611 58.865 43.489 106.927 1.00 20.31 A
ATOM 4848 CB SER A 611 58.227 42.142 106.574 1.00 20.33 A
ATOM 4849 OG SER A 611 58.700 41.678 105.318 1.00 25.55 A
ATOM 4850 C SER A 611 58.128 44.116 108.108 1.00 18.94 A
ATOM 4851 O SER A 611 57.243 44.956 107.931 1.00 20.11 A
ATOM 4852 N LEU A 612 58.500 43.713 109.316 1.00 18.58 A
ATOM 4853 CA LEU A 612 57.870 44.260 110.516 1.00 20.09 A
ATOM 4854 CB LEU A 612 58.455 43.592 111.764 1.00 20.66 A
ATOM 4855 CG LEU A 612 57.873 43.993 113.118 1.00 23.25 A
ATOM 4856 CD1 LEUA612 56.391 43.661 113.153 1.00 25.55 A
ATOM 4857 CD2 LEU A 612 58.610 43.266 114.232 1.00 22.71 A
ATOM 4858 C LEU A 612 58.097 45.776 110.570 1.00 20.22 A
ATOM 4859 O LEU A 612 57.156 46.556 110.749 1.00 19.24 A
ATOM 4860 N ARG A 613 59.349 46.186 110.390 1.00 19.65 A
ATOM 4861 CA ARG A 613 59.704 47.602 110.416 1.00 19.77 A
ATOM 4862 CB ARG A 613 61.208 47.785 110.178 1.00 18.13 A
ATOM 4863 CG ARG A 613 61.687 49.220 110.353 1.00 15.98 A
ATOM 4864 CD ARG A 613 63.181 49.362 110.101 1.00 16.44 A
ATOM 4865 NE ARG A 613 63.628 50.749 110.240 1.00 19.44 A
ATOM 4866 CZ ARG A 613 63.832 51.361 111.403 1.00 18.97 A
ATOM 4867 NH1 ARG A 613 63.637 50.715 112.546 1.00 19.26 A
ATOM 4868 NH2 ARG A 613 64.224 52.627 111.424 1.00 20.19 A
ATOM 4869 C ARG A 613 58.930 48.386 109.365 1.00 20.78 A
ATOM 4870 O ARG A 613 58.441 49.487 109.631 1.00 21.25 A
ATOM 4871 N LYS A 614 58.829 47.815 108.169 1.00 21.28 A
ATOM 4872 CA LYS A 614 58.118 48.457 107.071 1.00 22.58 A
ATOM 4873 CB LYS A 614 58.300 47.653 105.778 1.00 24.01 A
ATOM 4874 CG LYS A 614 57.633 48.314 104.579 1.00 24.46 A
ATOM 4875 CD LYS A 614 57.761 47.505 103.303 1.00 24.81 A
ATOM 4876 CE LYS A 614 57.160 48.288 102.133 1.0026.95 A
ATOM 4877 NZ LYS A 614 57.187 47.532 100.851 1.00 29.60 A
ATOM 4878 C LYS A 614 56.623 48.619 107.337 1.00 22.40 A
ATOM 4879 O LYS A 614 56.031 49.639 106.995 1.00 21.76 A
ATOM 4880 N ALA A 615 56.023 47.608 107.955 1.00 22.97 A
ATOM 4881 CA ALA A 615 54.594 47.617 108.236 1.00 23.65 A
ATOM 4882 CB ALA A 615 54.092 46.180 108.380 1.0021.24 A
ATOM 4883 C ALA A 615 54.180 48.428 109.459 1.00 25.23 A
ATOM 4884 O ALA A 615 52.987 48.626 109.691 1.00 26.82 A
ATOM 4885 N HIS A 616 55.146 48.903 110.238 1.0025.58 A
ATOM 4886 CA HIS A 616 54.812 49.662 111.437 1.00 24.84 A
ATOM 4887 CB HIS A 616 54.915 48.746 112.659 1.00 25.07 A
ATOM 4888 CG HIS A 616 54.007 47.558 112.591 1.00 28.13 A
ATOM 4889 CD2 HIS A 616 54.272 46.245 112.395 1.00 29.30 A
ATOM 4890 ND1 HIS A 616 52.636 47.664 112.676 1.00 27.52 A
ATOM 4891 CE1 HIS A 616 52.094 46.468 112.534 1.00 28.91 A
ATOM 4892 NE2 HIS A 616 53.065 45.589 112.361 1.00 30.59 A
ATOM 4893 C HIS A 616 55.656 50.912 111.653 1.00 24.03 A
ATOM 4894 O HIS A 616 56.787 50.831 112.129 1.00 23.79 A
ATOM 4895 N PRO A 617 55.101 52.092 111.314 1.00 23.35 A
ATOM 4896 CD PRO A 617 53.806 52.251 110.631 1.00 22.58 A
ATOM 4897 CA PRO A 617 55.769 53.392 111.456 1.00 23.24 A
ATOM 4898 CB PRO A 617 54.700 54.378 110.989 1.00 21.96 A
ATOM 4899 CG PRO A 617 53.967 53.594 109.955 1.00 22.54 A
ATOM 4900 C PRO A 617 56.241 53.685 112.883 1.00 22.88 A
ATOM 4901 O PRO A 617 57.050 54.588 113.105 1.00 21.38 A
ATOM 4902 N ALA A 618 55.736 52.925 113.846 1.0021.37 A
ATOM 4903 CA ALA A 618 56.132 53.124 115.236 1.00 23.04 A
ATOM 4904 CB ALA A 618 55.386 52.138 116.147 1.00 21.36 A
ATOM 4905 C ALA A 618 57.644 52.947 115.393 1.00 22:52 A
ATOM 4906 O ALAA618 58.254 53.517 116.299 1.0023.52 A
ATOM 4907 N PHE A 619 58.244 52.151 114.513 1.00 22.03 A
ATOM 4908 CA PHE A 619 59.685 51.901 114.555 1.00 22.00 A
ATOM 4909 CB PHE A 619 60.005 50.516 113.994 1.0020.67 A
ATOM 4910 CG PHE A 619 59.495 49.388 114.834 1.00 22.53 A
ATOM 4911 CD1 PHE A 619 60.052 49.128 116.083 1.00 21.39 A
ATOM 4912 CD2 PHE A 619 58.453 48.584 114.383 1.00 20.30 A
ATOM 4913 CE1 PHE A 619 59.580 48.084 116.869 1.0021.99 A
ATOM 4914 CE2 PHE A 619 57.975 47.537 115.162 1.0020.60 A
ATOM 4915 CZ PHE A 619 58.536 47.286 116.404 1.0021.19 A
ATOM 4916 C PHE A 619 60.435 52.934 113.732 1.00 22.31 A
ATOM 4917 O PHE A 619 61.665 52.918 113.674 1.00 22.32 A
ATOM 4918 N ARG A 620 59.689 53.834 113.100 1.00 21.45 A
ATOM 4919 CA ARG A 620 60.288 54.841 112.239 1.00 21.41 A
ATOM 4920 CB ARG A 620 59.999 54.481 110.777 1.00 19.97 A
ATOM 4921 CG ARG A 620 60.568 53.129 110.375 1.00 20.58 A
ATOM 4922 CD ARG A 620 60.245 52.756 108.937 1.00 20.40 A
ATOM 4923 NE ARG A 620 58.904 52.200 108.777 1.0021.32 A
ATOM 4924 CZ ARG A 620 57.837 52.887 108.378 1.00 21.63 A
ATOM 4925 NH1 ARG A 620 57.938 54.177 108.092 1.00 18.60 A
ATOM 4926 NH2 ARG A 620 56.667 52.275 108.254 1.00 21.40 A
ATOM 4927 C ARG A 620 59.844 56.273 112.523 1.00 20.59 A
ATOM 4928 O ARG A 620 59.448 57.002 111.613 1.00 20.40 A
ATOM 4929 N LEU A 621 59.906 56.668 113.787 1.0021.14 A
ATOM 4930 CA LEU A 621 59.536 58.025 114.172 1.00 22.61 A
ATOM 4931 CB LEU A 621 59.558 58.142 115.698 1.0021.63 A
ATOM 4932 CG LEU A 621 58.613 57.133 116.367 1.00 22.20 A
ATOM 4933 CD1 LEU A 621 58.748 57.195 117.893 1.00 22.56 A
ATOM 4934 CD2 LEU A 621 57.175 57.437 115.939 1.0020.34 A
ATOM 4935 C LEU A 621 60.566 58.947 113.51 1.00 24.17 A
ATOM 4936 O LEU A 621 61.761 58.640 113.483 1.00 25.23 A
ATOM 4937 N ARG A 622 60.104 60.074 112.982 1.00 25.07 A
ATOM 4938 CA ARG A 622 60.979 60.994 112.261 1.00 26.07 A
ATOM 4939 CB ARG A 622 60.174 61.665 111.144 1.00 27.02 A
ATOM 4940 CG ARG A 622 59.063 60.773 110.607 1.0028.85 A
ATOM 4941 CD ARG A 622 59.272 60.328 109.169 1.00 29.74 A
ATOM 4942 NE ARG A 622 58.594 61.212 108.232 1.00 31.32 A
ATOM 4943 CZ ARG A 622 58.264 60.888 106.984 1.00 30.69 A
ATOM 4944 NH1 ARGA622 58.541 59.686 106.494 1.00 28.79 A
ATOM 4945 NH2 ARG A 622 57.653 61.781 106.221 1.00 31.39 A
ATOM 4946 C ARG A 622 61.726 62.058 113.062 1.00 26.22 A
ATOM 4947 O ARG A 622 62.532 62.795 112.491 1.00 27.45 A
ATOM 4948 N SERA623 61.483 62.150 114.367 1.0024.42 A
ATOM 4949 CA SER A 623 62.179 63.160 115.163 1.00 24.24 A
ATOM 4950 CB SERA 623 61.410 64.491 115.137 1.00 24.59 A
ATOM 4951 OG SERA623 60.240 64.428 115.934 1.0027.18 A
ATOM 4952 C SERA623 62.412 62.755 116.609 1.00 23.04 A
ATOM 4953 O SERA623 61.690 61.923 117.159 1.00 23.64 A
ATOM 4954 N ALA A 624 63.423 63.360 117.225 1.00 21.57 A
ATOM 4955 CA ALA A 624 63.750 63.077 118.615 1.00 22.56 A
ATOM 4956 CB ALAA 624 65.002 63.851 119.024 1.0020.99 A
ATOM 4957 C ALA A 624 62.568 63.457 119.512 1.00 22.63 A
ATOM 4958 O ALA A 624 62.370 62.868 120.577 1.00 23.29 A
ATOM 4959 N ALA A 625 61.782 64.434 119.070 1.00 22.97 A
ATOM 4960 CA ALA A 625 60.617 64.887 119.823 1.00 24.06 A
ATOM 4961 CB ALA A 625 60.116 66.222 119.272 1.00 22.48 A
ATOM 4962 C ALA A 625 59.505 63.843 119.767 1.00 24.99 A
ATOM 4963 O ALAA625 58.870 63.555 120.778 1.00 25.65 A
ATOM 4964 N ASP A 626 59.262 63.278 118.589 1.0025.82 A
ATOM 4965 CA ASP A 626 58.229 62.255 118.468 1.00 27.80 A
ATOM 4966 CB ASP A 626 58.026 61.840 117.010 1.00 30.58 A
ATOM 4967 CG ASP A 626 57.386 62.925 116.174 1.0035.31 A
ATOM 4968 OD 1 ASP A 626 56.497 63.630 116.695 1.00 38.85 A
ATOM 4969 OD2 ASP A 626 57.759 63.058 114.988 1.00 37.79 A
ATOM 4970 C ASP A 626 58.633 61.027 119.281 1.00 27.81 A
ATOM 4971 O ASPA626 57.798 60.405 119.942 1.00 27.98 A
ATOM 4972 N ILE A 627 59.916 60.678 119.224 1.00 26.70 A
ATOM 4973 CA ILE A 627 60.428 59.522 119.959 1.00 27.83 A
ATOM 4974 CB ILE A 627 61.944 59.312 119.698 1.00 24.77 A
ATOM 4975 CG2 ILE A 627 62.503 58.293 120.666 1.00 22.44 A
ATOM 4976 CG1 ILE A 627 62.169 58.863 118.250 1.00 24.59 A
ATOM 4977 CD1 ILE A 627 63.633 58.687 117.872 1.00 24.44 A
ATOM 4978 C ILE A 627 60.197 59.739 121.450 1.00 28.97 A
ATOM 4979 O ILE A 627 59.764 58.836 122.165 1.00 29.58 A
ATOM 4980 N GLN A 628 60.484 60.954 121.899 1.00 30.07 A
ATOM 4981 CA GLN A 628 60.315 61.351 123.293 1.00 31.55 A
ATOM 4982 CB GLN A 628 60.738 62.815 123.450 1.00 33.84 A
ATOM 4983 CG GLN A 628 60.782 63.327 124.873 1.00 41.49 A
ATOM 4984 CD GLN A 628 62.127 63.092 125.529 1.0045.93 A
ATOM 4985 OE1 GLN A 628 62.324 63.406 126.703 1.0049.82 A
ATOM 4986 NE2 GLN A 628 63.068 62.542 124.768 1.00 49.25 A
ATOM 4987 C GLN A 628 58.845 61.215 123.681 1.00 29.80 A
ATOM 4988 O GLNA628 58.503 61.050 124.853 1.0026.82 A
ATOM 4989 N ARG A 629 57.988 61.267 122.670 1.00 29.31 A
ATOM 4990 CA ARG A 629 56.548 61.220 122.853 1.00 30.90 A
ATOM 4991 CB ARG A 629 55.927 62.268 121.930 1.0033.56 A
ATOM 4992 CG ARG A 629 54.593 62.831 122.361 1.00 39.60 A
ATOM 4993 CD ARG A 629 54.197 63.945 121.400 1.00 44.21 A
ATOM 4994 NE ARG A 629 55.235 64.973 121.326 1.00 48.24 A
ATOM 4995 CZ ARG A 629 55.712 65.489 120.194 1.00 50.13 A
ATOM 4996 NH1 ARG A 629 55.250 65.076 119.020 1.00 49.72 A
ATOM 4997 NH2 ARG A 629 56.657 66.421 120.237 1.00 50.07 A
ATOM 4998 C ARGA629 55.874 59.865 122.618 1.00 31.00 A
ATOM 4999 O ARG A 629 54.683 59.718 122.898 1.00 30.75 A
ATOM 5000 N HIS A 630 56.611 58.876 122.111 1.0029.57 A
ATOM 5001 CA HIS A 630 56.006 57.573 121.850 1.00 27.64 A
ATOM 5002 CB HIS A 630 55.618 57.465 120.373 1.0027.34 A
ATOM 5003 CG HIS A 630 54.561 58.438 119.958 1.0029.13 A
ATOM 5004 CD2 HIS A 630 53.214 58.317 119.901 1.00 30.01 A
ATOM 5005 ND1 HIS A 630 54.844 59.733 119.582 1.00 32.26 A
ATOM 5006 CE1 HIS A 630 53.717 60.368 119.310 1.00 32.47 A
ATOM 5007 NE2 HIS A 630 52.712 59.530 119.496 1.00 30.83 A
ATOM 5008 C HIS A 630 56.835 56.354 122.233 1.00 27.03 A
ATOM 5009 O HIS A 630 56.369 55.218 122.114 1.00 25.69 A
ATOM 5010 N LEU A 631 58.055 56.582 122.696 1.00 24.52 A
ATOM 5011 CA LEU A 631 58.923 55.478 123.074 1.00 23.54 A
ATOM 5012 CB LEU A 631 60.159 55.464 122.171 1.00 22.85 A
ATOM 5013 CG LEU A 631 61.234 54.389 122.360 1.00 22.89 A
ATOM 5014 CD1 LEU A 631 62.121 54.333 121.118 1.00 21.07 A
ATOM 5015 CD2 LEU A 631 62.057 54.697 123.585 1.00 23.60 A
ATOM 5016 C LEU A 631 59.344 55.603 124.526 1.00 21.99 A
ATOM 5017 O LEU A 631 59.742 56.675 124.966 1.00 23.04 A
ATOM 5018 N GLU A 632 59.252 54.509 125.272 1.00 21.84 A
ATOM 5019 CA GLU A 632 59.653 54.530 126.671 1.00 22.39 A
ATOM 5020 CB GLU A 632 58.460 54.877 127.575 1.0021.13 A
ATOM 5021 CG GLU A 632 57.693 53.692 128.122 1.0020.61 A
ATOM 5022 CD GLU A 632 56.485 54.117 128.933 1.0022.46 A
ATOM 5023 OE1 GLU A 632 56.514 55.229 129.496 1.00 21.89 A
ATOM 5024 OE2 GLU A 632 55.511 53.338 129.020 1.00 23.29 A
ATOM 5025 C GLU A 632 60.255 53.195 127.079 1.00 21.95 A
ATOM 5026 O GLU A 632 59.844 52.142 126.597 1.0022.45 A
ATOM 5027 N CYS A 633 61.244 53.259 127.959 1.00 23.04 A
ATOM 5028 CA CYS A 633 61.932 52.075 128.456 1.00 26.71 A
ATOM 5029 CB CYS A 633 63.363 52.439 128.875 1.00 23.59 A
ATOM 5030 SG CYS A 633 64.319 51.089 129.607 1.00 29.51 A
ATOM 5031 C CYSA633 61.160 51.528 129.650 1.00 27.72 A
ATOM 5032 O CYS A 633 60.801 52.279130.554 1.0028.89 A
ATOM 5033 N LEU A 634 60.908 50.221 129.644 1.0028.69 A
ATOM 5034 CA LEU A 634 60.168 49.563 130.722 1.00 29.95 A
ATOM 5035 CB LEU A 634 59.065 48.682 130.130 1.00 29.63 A
ATOM 5036 CG LEU A 634 57.628 49.213 130.131 1.00 30.99 A
ATOM 5037 CD1 LEU A 634 57.587 50.680 129.754 1.00 32.27 A
ATOM 5038 CD2 LEU A 634 56.803 48.380 129.168 1.00 30.02 A
ATOM 5039 C LEU A 634 61.057 48.720 131.631 1.00 30.63 A
ATOM 5040 O LEU A 634 60.716 48.486 132.789 1.00 31.40 A
ATOM 5041 N THR A 635 62.193 48.267 131.108 1.00 30.33 A
ATOM 5042 CA THR A 635 63.119 47.444 131.878 1.00 30.30 A
ATOM 5043 CB THR A 635 62.698 45.964 131.862 1.00 31.77 A
ATOM 5044 OG1 THR A 635 61.379 45.830 132.404 1.00 32.01 A
ATOM 5045 CG2 THR A 635 63.674 45.129 132.680 1.00 31.17 A
ATOM 5046 C THR A 635 64.525 47.526 131.308 1.00 31.40 A
ATOM 5047 O THRA635 64.714 47.467 130.091 1.00 31.86 A
ATOM 5048 N LEU A 636 65.515 47.647 132.185 1.00 31.39 A
ATOM 5049 CA LEU A 636 66.896 47.728 131.740 1.00 33.88 A
ATOM 5050 CB LEU A 636 67.307 49.197 131.614 1.00 34.70 A
ATOM 5051 CG LEU A 636 68.458 49.526 130.662 1.00 36.15 A
ATOM 5052 CD1 LEU A 636 68.508 51.021 130.422 1.00 36.69 A
ATOM 5053 CD2 LEU A 636 69.766 49.027 131.241 1.00 37.72 A
ATOM 5054 C LEU A 636 67.825 46.979 132.700 1.00 35.08 A
ATOM 5055 O LEU A 636 68.367 47.559 133.638 1.00 36.95 A
ATOM 5056 N LYS A 637 67.990 45.681 132.453 1.00 35.66 A
ATOM 5057 CA LYS A 637 68.841 44.816 133.265 1.00 35.50 A
ATOM 5058 CB LYS A 637 68.064 43.571 133.701 1.00 36.00 A
ATOM 5059 CG LYS A 637 66.803 43.868 134.500 1.00 38.28 A
ATOM 5060 CD LYS A 637 66.087 42.582 134.894 1.00 40.72 A
ATOM 5061 CE LYS A 637 64.860 42.861 135.749 1.0043.26 A
ATOM 5062 NZ LYS A 637 65.206 43.597 137.002 1.00 43.30 A
ATOM 5063 C LYS A 637 70.049 44.403 132.431 1.00 35.43 A
ATOM 5064 O LYS A 637 70.114 44.716 131.242 1.00 34.50 A
ATOM 5065 N GLU A 638 70.985 43.678 133.042 1.00 34.92 A
ATOM 5066 CA GLU A 638 72.204 43.254 132.347 1.00 36.21 A
ATOM 5067 CB GLU A 638 73.136 42.487 133.296 1.0037.46 A
ATOM 5068 CG GLU A 638 72.573 41.173 133.794 1.00 42.30 A
ATOM 5069 CD GLU A 638 73.634 40.267 134.409 1.0046.05 A
ATOM 5070 OE1 GLU A 638 73.254 39.266 135.057 1.00 47.36 A
ATOM 5071 OE2 GLU A 638 74.842 40.549 134.237 1.00 47.18 A
ATOM 5072 C GLU A 638 72.004 42.421 131.085 1.00 34.80 A
ATOM 5073 O GLU A 638 72.789 42.533 130.145 1.00 34.31 A
ATOM 5074 N HIS A 639 70.975 41.578 131.063 1.00 34.66 A
ATOM 5075 CA HIS A 639 70.708 40.739 129.892 1.00 34.61 A
ATOM 5076 CB HIS A 639 70.944 39.261 130.211 1.00 37.78 A
ATOM 5077 CG HIS A 639 72.276 38.975 130.824 1.00 42.31 A
ATOM 5078 CD2 HIS A 639 72.615 38.262 131.925 1.00 43.38 A
ATOM 5079 ND1 HIS A 639 73.459 39.428 130.281 1.00 43.89 A
ATOM 5080 CE1 HIS A 639 74.469 39.006 131.021 1.00 45.38 A
ATOM 5081 NE2 HIS A 639 73.984 38.297 132.024 1.0045.53 A
ATOM 5082 C HIS A 639 69.277 40.890 129.405 1.00 32.78 A
ATOM 5083 O HIS A 639 68.815 40.115 128.563 1.00 32.27 A
ATOM 5084 N LEU A 640 68.569 41.881 129.929 1.00 31.20 A
ATOM 5085 CA LEU A 640 67.186 42.078 129.535 1.00 30.24 A
ATOM 5086 CB LEU A 640 66.265 41.433 130.580 1.00 32.42 A
ATOM 5087 CG LEU A 640 64.759 41.259 130.331 1.00 33.50 A
ATOM 5088 CD1 LEU A 640 64.067 42.603 130.157 1.00 32.82 A
ATOM 5089 CD2 LEU A 640 64.562 40.395 129.110 1.00 35.76 A
ATOM 5090 C LEU A 640 66.834 43.549 129.368 1.00 29.30 A
ATOM 5091 O LEU A 640 66.987 44.343 130.293 1.00 29.17 A
ATOM 5092 N ILE A 641 66.372 43.903 128.173 1.00 28.10 A
ATOM 5093 CA ILE A 641 65.946 45.263 127.878 1.00 24.59 A
ATOM 5094 CB ILEA641 66.838 45.959 126.825 1.00 24.83 A
ATOM 5095 CG2 ILE A 641 66.229 47.311 126.440 1.00 22.45 A
ATOM 5096 CG1 ILE A 641 68.248 46.169 127.376 1.00 22.97 A
ATOM 5097 CD 1 ILE A 641 69.198 46.793 126.366 1.00 23.64 A
ATOM 5098 C ILE A 641 64.548 45.170 127.306 1.00 23.93 A
ATOM 5099 O ILE A 641 64.291 44.387 126.392 1.00 26.30 A
ATOM 5100 N ALA A 642 63.639 45.962 127.854 1.00 23.17 A
ATOM 5101 CA ALA A 642 62.270 45.976 127.376 1.00 21.08 A
ATOM 5102 CB ALAA 642 61.351 45.288 128.369 1.00 22.07 A
ATOM 5103 C ALA A 642 61.849 47.418 127.203 1.00 21.70 A
ATOM 5104 O ALAA 642 62.005 48.230 128.110 1.00 21.15 A
ATOM 5105 N TYR A 643 61.340 47.742 126.022 1.00 21.09 A
ATOM 5106 CA TYR A 643 60.872 49.087 125.761 1.00 20.94 A
ATOM 5107 CB TYR A 643 61.874 49.878 124.910 1.0020.19 A
ATOM 5108 CG TYRA 643 62.119 49.345 123.513 1.00 17.21 A
ATOM 5109 CD1 TYR A 643 62.941 48.239 123.299 1.00 19.11 A
ATOM 5110 CE1 TYR A 643 63.195 47.759 122.007 1.00 16.72 A
ATOM 5111 CD2 TYR A 643 61.549 49.963 122.402 1.00 18.17 A
ATOM 5112 CE2 TYR A 643 61.794 49.492 121.104 1.00 17.92 A
ATOM 5113 CZ TYR A 643 62.620 48.389 120.922 1.00 17.71 A
ATOM 5114 OH TYRA643 62.866 47.916 119.659 1.00 19.89 A
ATOM 5115 C TYRA643 59.549 48.943 125.036 1.00 22.38 A
ATOM 5116 O TYR A 643 59.211 47.854124.560 1.0022.68 A
ATOM 5117 N ARGA644 58.797 50.032 124.959 1.00 21.70 A
ATOM 5118 CA ARG A 644 57.508 49.989 124.301 1.00 22.01 A
ATOM 5119 CB ARG A 644 56.405 49.715 125.333 1.00 22.08 A
ATOM 5120 CG ARG A 644 56.288 50.783 126.420 1.0024.59 A
ATOM 5121 CD ARG A 644 55.147 51.746 126.133 1.0026.30 A
ATOM 5122 NE ARG A 644 53.850 51.097 126.314 1.00 29.44 A
ATOM 5123 CZ ARG A 644 53.307 50.815 127.497 1.0029.33 A
ATOM 5124 NH1 ARG A 644 53.942 51.127 128.619 1.00 28.71 A
ATOM 5125 NH2 ARG A 644 52.128 50.209 127.560 1.00 28.96 A
ATOM 5126 C ARGA644 57.215 51.273 123.551 1.00 22.27 A
ATOM 5127 O ARG A 644 57.700 52.353 123.905 1.00 22.98 A
ATOM 5128 N LEU A 645 56.417 51.134 122.502 1.0022.23 A
ATOM 5129 CA LEU A 645 56.005 52.253 121.674 1.00 23.91 A
ATOM 5130 CB LEU A 645 56.279 51.928 120.196 1.00 23.38 A
ATOM 5131 CG LEU A 645 57.745 51.934 119.713 1.00 25.27 A
ATOM 5132 CD1 LEU A 645 58.661 51.285 120.720 1.00 28.98 A
ATOM 5133 CD2 LEU A 645 57.851 51.192 118.389 1.00 27.95 A
ATOM 5134 C LEU A 645 54.509 52.415 121.959 1.00 25.01 A
ATOM 5135 O LEU A 645 53.734 51.471 121.793 1.00 23.65 A
ATOM 5136 N TYR A 646 54.114 53.606 122.404 1.00 26.28 A
ATOM 5137 CA TYRA646 52.725 53.873 122.761 1.00 28.46 A
ATOM 5138 CB TYR A 646 52.625 54.031 124.277 1.00 30.28 A
ATOM 5139 CG TYRA 646 53.376 55.238 124.792 1.00 31.49 A
ATOM 5140 CD1 TYR A 646 52.749 56.478 124.896 1.00 31.17 A
ATOM 5141 CE1 TYR A 646 53.452 57.610 125.323 1.00 33.44 A
ATOM 5142 CD2 TYR A 646 54.728 55.150 125.128 1.00 30.96 A
ATOM 5143 CE2 TYR A 646 55.441 56.272 125.552 1.00 32.50 A
ATOM 5144 CZ TYR A 646 54.796 57.499 125.645 1.00 32.93 A
ATOM 5145 OH TYR A 646 55.496 58.616 126.036 1.00 34.11 A
ATOM 5146 C TYRA 646 52.133 55.107 122.084 1.00 29.94 A
ATOM 5147 O TYRA 646 52.818 55.801 121.335 1.00 29.90 A
ATOM 5148 N ASP A 647 50.862 55.379 122.385 1.00 31.56 A
ATOM 5149 CA ASP A 647 50.126 56.511 121.820 1.00 33.76 A
ATOM 5150 CB ASP A 647 50.607 57.841 122.416 1.00 37.77 A
ATOM 5151 CG ASP A 647 49.984 58.134 123.769 1.00 42.69 A
ATOM 5152 OD1 ASP A 647 50.299 59.190 124.360 1.00 46.23 A
ATOM 5153 OD2 ASP A 647 49.173 57.310 124.244 1.00 46.17 A
ATOM 5154 C ASP A 647 50.286 56.535 120.315 1.00 33.98 A
ATOM 5155 O ASPA647 50.601 57.570 119.725 1.0030.60 A
ATOM 5156 N LEU A 648 50.050 55.378 119.704 1.00 35.88 A
ATOM 5157 CA LEU A 648 50.178 55.215 118.265 1.00 39.05 A
ATOM 5158 CB LEU A 648 50.732 53.820 117.968 1.00 35.67 A
ATOM 5159 CG LEU A 648 51.960 53.422 118.796 1.00 34.88 A
ATOM 5160 CD1 LEU A 648 52.357 51.993 118.474 1.00 33.70 A
ATOM 5161 CD2 LEU A 648 53.108 54.376 118.512 1.00 33.96 A
ATOM 5162 C LEU A 648 48.856 55.417 117.526 1.00 43.12 A
ATOM 5163 O LEU A 648 48.593 54.739 116.530 1.00 44.32 A
ATOM 5164 N ASP A 649 48.035 56.347 118.011 1.00 47.09 A
ATOM 5165 CA ASP A 649 46.738 56.639 117.398 1.00 51.83 A
ATOM 5166 CB ASP A 649 46.249 58.026 117.818 1.00 55.00 A
ATOM 5167 CG ASP A 649 46.017 58.134 119.316 1.00 58.24 A
ATOM 5168 OD1 ASP A 649 46.967 57.867 120.090 1.00 58.13 A
ATOM 5169 OD2 ASP A 649 44.885 58.487 119.715 1.00 59.32 A
ATOM 5170 C ASP A 649 46.850 56.567 115.884 1.00 53.01 A
ATOM 5171 O ASPA649 46.623 55.512 115.289 1.00 54.48 A
ATOM 5172 N GLU A 650 47.185 57.680 115.246 1.00 53.68 A
ATOM 5173 CA GLU A 650 47.335 57.620 113.806 1.00 55.19 A
ATOM 5174 CB GLU A 650 46.613 58.764 113.098 1.00 59.36 A
ATOM 5175 CG GLU A 650 46.098 58.335 111.719 1.00 63.32 A
ATOM 5176 CD GLU A 650 47.075 57.408 110.993 1.0065.78 A
ATOM 5177 OE1 GLU A 650 48.139 57.889 110.547 1.00 67.95 A
ATOM 5178 OE2 GLU A 650 46.784 56.194 110.881 1.00 66.18 A
ATOM 5179 C GLU A 650 48.817 57.653 113.478 1.00 53.8 A
ATOM 5180 O GLU A 650 49.317 58.568 112.820 1.00 54.19 A
ATOM 5181 N VALA 651 49.512 56.640 113.976 1.00 50.50 A
ATOM 5182 CA VAL A 651 50.935 56.479 113.752 1.00 46.85 A
ATOM 5183 CB VAL A 651 51.738 56.646 115.064 1.00 47.23 A
ATOM 5184 CG1 VAL A 651 53.189 56.254 114.841 1.00 46.11 A
ATOM 5185 CG2 VAL A 651 51.648 58.083 115.555 1.00 48.24 A
ATOM 5186 C VALA 651 51.107 55.054 113.262 1.00 44.58 A
ATOM 5187 O VAL A 651 51.628 54.808 112.174 1.00 43.18 A
ATOM 5188 N ASP A 652 50.627 54.121 114.075 1.00 41.86 A
ATOM 5189 CA ASP A 652 50.742 52.709 113.774 1.00 39.56 A
ATOM 5190 CB ASP A 652 51.639 52.056 114.825 1.00 37.02 A
ATOM 5191 CG ASP A 652 52.363 50.845 114.299 1.00 35.17 A
ATOM 5192 OD I ASP A 652 51.727 49.784 114.136 1.00 34.65 A
ATOM 5193 OD2 ASP A 652 53.575 50.963 114.040 1.00 34.01 A
ATOM 5194 C ASP A 652 49.390 52.005 113.728 1.00 39.38 A
ATOM 5195 O ASPA652 48.397 52.496 114.265 1.00 37.82 A
ATOM 5196 N GLUA653 49.368 50.850 113.071 1.00 39.70 A
ATOM 5197 CA GLU A 653 48.159 50.046 112.949 1.00 40.11 A
ATOM 5198 CB GLU A 653 48.378 48.917 111.944 1.00 42.51 A
ATOM 5199 CG GLU A 653 47.165 48.038 111.731 1.00 48.38 A
ATOM 5200 CD GLU A 653 47.493 46.756 110.985 1.00 51.17 A
ATOM 5201 OE1 GLU A 653 46.548 46.005 110.658 1.00 52.32 A
ATOM 5202 OE2 GLU A 653 48.692 46.499 110.732 1.00 52.47 A
ATOM 5203 C GLU A 653 47.857 49.449 114.315 1.00 38.91 A
ATOM 5204 O GLU A653 46.709 49.161 114.645 1.00 38.63 A
ATOM 5205 N TRP A 654 48.907 49.257 115.103 1.00 37.04 A
ATOM 5206 CA TRP A 654 48.764 48.702 116.432 1.00 36.24 A
ATOM 5207 CB TRP A 654 49.905 47.727 116.715 1.00 35.72 A
ATOM 5208 CG TRP A 654 49.856 46.510 115.836 1.00 33.97 A
ATOM 5209 CD2 TRP A 654 50.937 45.623 115.531 1.00 32.33 A
ATOM 5210 CE2 TRP A 654 50.414 44.593 114.721 1.00 32.80 A
ATOM 5211 CE3 TRP A 654 52.296 45.597 115.865 1.00 32.12 A
ATOM 5212 CD1 TRP A 654 48.753 45.996 115.213 1.00 33.10 A
ATOM 5213 NE1 TRP A 654 49.080 44.846 114.543 1.00 32.67 A
ATOM 5214 CZ2 TRP A 654 51.206 43.544 114.237 1.0032.30 A
ATOM 5215 CZ3 TRP A 654 53.083 44.554 115.385 1.00 31.33 A
ATOM 5216 CH2 TRP A 654 52.534 43.544 1 4.579 1.00 32.42 A
ATOM 5217 C TRP A 654 48.727 49.807 117.480 1.00 36.94 A
ATOM 5218 O TRP A 654 49.394 50.829 117.345 1.00 37.75 A
ATOM 5219 N LYS A 655 47.929 49.591 118.518 1.00 36.83 A
ATOM 5220 CA LYS A 655 47.763 50.553 119.601 1.00 37.05 A
ATOM 5221 CB LYS A 655 46.530 50.160 120.427 1.00 39.95 A
ATOM 5222 CG LYS A 655 46.255 51.011 121.663 1.0044.02 A
ATOM 5223 CD LYS A 655 44.920 50.608 122.304 1.00 46.89 A
ATOM 5224 CE LYS A 655 44.733 51.227 123.688 1.0048.37 A
ATOM 5225 NZ LYS A 655 44.779 52.719 123.674 1.00 48.75 A
ATOM 5226 C LYS A 655 49.001 50.654 120.496 1.00 34.30 A
ATOM 5227 O LYS A 655 49.448 51.752 120.833 1.00 34.40 A
ATOM 5228 N ASP A 656 49.557 49.506 120.867 1.00 31.32 A
ATOM 5229 CA ASP A 656 50.732 49.463 121.730 1.0027.29 A
ATOM 5230 CB ASP A 656 50.303 49.201 123.179 1.00 28.75 A
ATOM 5231 CG ASP A 656 51.413 49.470 124.181 1.0029.61 A
ATOM 5232 OD1 ASP A 656 51.575 48.670 125.127 1.00 29.29 A
ATOM 5233 OD2 ASP A 656 52.113 50.492 124.036 1.0032.51 A
ATOM 5234 C ASP A 656 51.653 48.341 121.266 1.0025.30 A
ATOM 5235 O ASP A 656 51.186 47.273 120.861 1.00 24.00 A
ATOM 5236 N ILE A 657 52.959 48.585 121.324 1.00 23.14 A
ATOM 5237 CA ILE A 657 53.944 47.587 120.916 1.00 20.94 A
ATOM 5238 CB ILE A 657 54.604 47.959 119.562 1.00 20.71 A
ATOM 5239 CG2 ILE A 657 55.637 46.909 119.185 1.00 20.39 A
ATOM 5240 CG1 ILE A 657 53.543 48.070 118.466 1.00 20.05 A
ATOM 5241 CD1 ILE A 657 54.101 48.451117.103 1.0021.10 A
ATOM 5242 C ILEA657 55.047 47.441 121.967 1.00 21.18 A
ATOM 5243 O ILEA657 55.691 48.419 122.350 1.00 20.43 A
ATOM 5244 N ILEA658 55.264 46.214 122.423 1.00 20.71 A
ATOM 5245 CA ILEA658 56.286 45.943 123.423 1.0020.72 A
ATOM 5246 CB ILE A 658 55.674 45.275 124.683 1.00 20.99 A

ATOM 5247 CG2 ILE A 658 56.767 44.987 125.701 1.00 16.73 A
ATOM 5248 CG1 ILE A 658 54.603 46.191 125.294 1.00 22.73 A
ATOM 5249 CD1 ILE A 658 53.944 45.626 126.565 1.00 21.84 A
ATOM 5250 C ILE A 658 57.363 45.029 122.853 1.00 19.94 A
ATOM 5251 O ILE A 658 57.060 43.983 122.280 1.00 22.66 A
ATOM 5252 N VALA 659 58.619 45.432 123.012 1.00 19.14 A
ATOM 5253 CA VAL A 659 59.755 44.656 122.521 1.00 19.55 A
ATOM 5254 CB VAL A 659 60.549 45.445 121.453 1.00 20.52 A
ATOM 5255 CG1 VAL A 659 61.718 44.613 120.958 1.00 19.57 A
ATOM 5256 CG2 VAL A 659 59.628 45.835 120.295 1.00 19.46 A
ATOM 5257 C VAL A 659 60.709 44.320 123.666 1.00 21.50 A
ATOM 5258 O VALA 659 61.142 45.209 124.402 1.00 19.38 A
ATOM 5259 N ILE A 660 61.046 43.040 123.798 1.00 22.50 A
ATOM 5260 CA ILE A 660 61.951 42.589 124.852 1.00 25.06 A
ATOM 5261 CB ILE A 660 61.230 41.642 125.838 1.00 25.76 A
ATOM 5262 CG2 ILE A 660 62.139 41.320 127.013 1.00 24.99 A
ATOM 5263 CG1 ILE A 660 59.921 42.279 126.315 1.00 27.13 A
ATOM 5264 CD1 ILE A 660 58.986 41.311 127.018 1.00 27.13 A
ATOM 5265 C ILEA660 63.143 41.833 124.265 1.00 25.75 A
ATOM 5266 O ILE A 660 62.973 40.830 123.571 1.00 26.20 A
ATOM 5267 N HIS A 661 64.345 42.321 124.551 1.0025.73 A
ATOM 5268 CA HIS A 661 65.571 41.695 124.069 1.00 25.78 A
ATOM 5269 CB HIS A 661 66.514 42.757 123.481 1.00 24.84 A
ATOM 5270 CG HIS A 661 66.014 43.374 122.208 1.0023.27 A
ATOM 5271 CD2 HIS A 661 65.508 42.812 121.086 1.00 21.57 A
ATOM 5272 ND1 HIS A 661 66.006 44.735 121.990 1.00 23.11 A
ATOM 5273 CE1 HIS A 661 65.516 44.985 120.789 1.00 21.92 A
ATOM 5274 NE2 HIS A 661 65.207 43.835 120.219 1.0023.99 A
ATOM 5275 C HIS A 661 66.253 40.967 125.225 1.00 26.70 A
ATOM 5276 O HIS A 661 66.569 41.572 126.247 1.0027.19 A
ATOM 5277 N HIS A 662 66.469 39.666 125.055 1.0028.88 A
ATOM 5278 CA HIS A 662 67.102 38.836 126.078 1.00 29.28 A
ATOM 5279 CB HIS A 662 66.200 37.642 126.406 1.00 30.38 A
ATOM 5280 CG HIS A 662 66.600 36.899 127.644 1.00 33.24 A
ATOM 5281 CD2 HIS A 662 67.371 37.265 128.696 1.00 34.03 A
ATOM 5282 ND1 HIS A 662 66.165 35.620 127.916 1.00 32.26 A
ATOM 5283 CE1 HIS A 662 66.652 35.230 129.081 1.00 33.38 A
ATOM 5284 NE2 HIS A 662 67.386 36.210 129.575 1.00 33.71 A
ATOM 5285 C HIS A 662 68.446 38.339 125.555 1.00 28.00 A
ATOM 5286 O HIS A 662 68.514 37.733 124.490 1.00 29.75 A
ATOM 5287 N ALAA 663 69.512 38.586 126.309 1.00 27.64 A
ATOM 5288 CA ALA A 663 70.847 38.176 125.888 1.00 26.72 A
ATOM 5289 CB ALAA 663 71.774 39.393 125.868 1.00 24.47 A
ATOM 5290 C ALA A 663 71.471 37.056 126.725 1.0026.78 A
ATOM 5291 O ALA A 663 72.694 36.945 126.803 1.00 25.94 A
ATOM 5292 N SERA 664 70.638 36.232 127.350 1.00 27.82 A
ATOM 5293 CA SERA 664 71.134 35.117 128.153 1.00 29.00 A
ATOM 5294 CB SERA 664 71.083 35.452 129.649 1.00 30.26 A
ATOM 5295 OG SERA 664 69.770 35.781 130.074 1.00 34.25 A
ATOM 5296 C SERA664 70.303 33.876 127.848 1.00 29.88 A
ATOM 5297 O SERA664 69.109 33.972 127.554 1.00 29.09 A
ATOM 5298 N PRO A 665 70.931 32.689127.903 1.0031.23 A
ATOM 5299 CD PRO A 665 72.359 32.472 128.199 1.00 31.99 A
ATOM 5300 CA PRO A 665 70.257 31.415 127.624 1.00 33.80 A
ATOM 5301 CB PRO A 665 71.424 30.440 127.470 1.0031.57 A
ATOM 5302 CG PRO A 665 72.421 30.974 128.438 1.00 32.90 A
ATOM 5303 C PRO A 665 69.232 30.952 128.665 1.00 36.04 A
ATOM 5304 O PRO A 665 68.395 30.090 128.380 1.00 36.95 A
ATOM 5305 N ASP A 666 69.289 31.520 129.865 1.00 37.29 A
ATOM 5306 CA ASP A 666 68.345 31.134 130.904 1.00 38.66 A
ATOM 5307 CB ASP A 666 68.773 31.709 132.259 1.00 40.57 A
ATOM 5308 CG ASP A 666 68.830 33.222 132.265 1.00 43.61 A
ATOM 5309 OD1 ASP A 666 67.760 33.863 132.198 1.00 43.96 A
ATOM 5310 OD2 ASP A 666 69.952 33.769 132.338 1.00 45.91 A
ATOM 5311 C ASP A 666 66.933 31.594 130.544 1.00 39.04 A
ATOM 5312 0 ASPA 666 66.729 32.308 129.560 1.00 38.42 A
ATOM 5313 N SER A 667 65.960 31.167 131.340 1.00 38.67 A
ATOM 5314 CA SERA 667 64.567 31.519 131.107 1.00 38.85 A
ATOM 5315 CB SERA 667 63.727 30.244 131.014 1.00 40.11 A
ATOM 5316 OG SERA667 62.355 30.547 130.854 1.0043.62 A
ATOM 5317 C SERA667 64.036 32.414 132.227 1.00 38.48 A
ATOM 5318 O SERA 667 64.370 32.222 133.397 1.00 37.73 A
ATOM 5319 N VALA668 63.218 33.398 131.868 1.00 37.37 A
ATOM 5320 CA VAL A 668 62.656 34.305 132.860 1.00 36.75 A
ATOM 5321 CB VAL A 668 63.539 35.560 133.055 1.00 37.07 A
ATOM 5322 CG1 VAL A 668 64.926 35.158 133.520 1.00 35.83 A
ATOM 5323 CG2 VAL A 668 63.613 36.363 131.755 1.00 37.13 A
ATOM 5324 C VALA 668 61.260 34.772 132.479 1.00 36.96 A
ATOM 5325 O VAL A 668 60.874 34.724 131.312 1.00 36.19 A
ATOM 5326 N GLU A 669 60.509 35.214 133.483 1.00 37.11 A
ATOM 5327 CA GLU A 669 59.160 35.728 133.286 1.00 37.60 A
ATOM 5328 CB GLU A 669 58.228 35.264 134.414 1.0041.63 A
ATOM 5329 CG GLU A 669 57.909 33.778 134.418 1.0047.14 A
ATOM 5330 CD GLU A 669 56.996 33.360 133.274 1.00 51.48 A
ATOM 5331 OE1 GLU A 669 56.668 32.155133.186 1.00 53.81 A
ATOM 5332 OE2 GLU A 669 56.603 34.229 132.465 1.00 54.18 A
ATOM 5333 C GLU A 669 59.258 37.249 133.307 1.00 35.47 A
ATOM 5334 O GLU A 669 60.085 37.814 134.026 1.00 35.19 A
ATOM 5335 N TRPA670 58.420 37.909 132.518 1.00 33.33 A
ATOM 5336 CA TRP A 670 58.423 39.364 132.458 1.00 31.85 A
ATOM 5337 CB TRP A 670 59.002 39.836 131.118 1.00 31.07 A
ATOM 5338 CG TRP A 670 59.195 41.318 131.048 1.00 28.57 A
ATOM 5339 CD2 TRP A 670 58.330 42.264 130.410 1.00 27.50 A
ATOM 5340 CE2 TRP A 670 58.873 43.548 130.639 1.00 26.41 A
ATOM 5341 CE3TRPA670 57.146 42.150 129.668 1.00 27.54 A
ATOM 5342 CD1 TRP A 670 60.201 42.043 131.621 1.0027.62 A
ATOM 5343 NE1 TRP A 670 60.014 43.385 131.381 1.00 26.49 A
ATOM 5344 CZ2 TRP A 670 58.274 44.714 130.153 1.00 26.14 A
ATOM 5345 CZ3 TRP A 670 56.548 43.313 129.182 1.0029.40 A
ATOM 5346 CH2 TRP A 670 57.116 44.578 129.429 1.00 27.09 A
ATOM 5347 C TRPA670 57.000 39.886 132.614 1.00 31.62 A
ATOM 5348 O TRP A 670 56.100 39.478 131.876 1.00 31.17 A
ATOM 5349 N ARG A 671 56.800 40.784 133.576 1.00 30.80 A
ATOM 5350 CA ARG A 671 55.481 41.356 133.818 1.00 31.11 A
ATOM 5351 CB ARG A 671 55.355 41.836 135.271 1.00 32.27 A
ATOM 5352 CG ARG A 671 54.566 40.896 136.176 1.00 37.17 A
ATOM 5353 CD ARG A 671 53.224 41.489 136.635 1.00 38.03 A
ATOM 5354 NE ARG A 671 52.355 41.872 135.527 1.00 39.05 A
ATOM 5355 CZ ARG A 671 51.086 42.254 135.660 1.00 39.32 A
ATOM 5356 NH1 ARG A 671 50.522 42.303 136.858 1.00 38.17 A
ATOM 5357 NH2 ARG A 671 50.381 42.601 134.592 1.00 38.91 A
ATOM 5358 C ARG A 671 55.186 42.520 132.887 1.00 30.94 A
ATOM 5359 O ARG A 671 55.980 43.454 132.772 1.00 30.52 A
ATOM 5360 N LEU A 672 54.044 42.453 132.214 1.00 31.50 A
ATOM 5361 CA LEU A 672 53.636 43.523 131.319 1.00 32.83 A
ATOM 5362 CB LEU A 672 52.491 43.062 130.415 1.00 31.49 A
ATOM 5363 CG LEU A 672 52.847 42.176 129.215 1.00 32.26 A
ATOM 5364 CD1 LEU A 672 53.425 40.841 129.680 1.00 31.74 A
ATOM 5365 CD2 LEU A 672 51.600 41.963 128.376 1.00 30.06 A
ATOM 5366 C LEU A 672 53.180 44.694 132.190 1.00 35.03 A
ATOM 5367 O LEU A 672 52.784 44.500 133.342 1.00 34.09 A
ATOM 5368 N PRO A 673 53.241 45.925 131.654 1.00 36.28 A
ATOM 5369 CD PRO A 673 53.743 46.272 130.311 1.00 35.89 A
ATOM 5370 CA PRO A 673 52.835 47.130 132.383 1.00 36.77 A
ATOM 5371 CB PROA673 53.325 48.259 131.475 1.0037.01 A
ATOM 5372 CG PRO A 673 53.181 47.664 130.109 1.00 37.12 A
ATOM 5373 C PRO A 673 51.332 47.207 132.660 1.00 37.04 A
ATOM 5374 O PRO A 673 50.850 48.179 133.248 1.00 36.97 A
ATOM 5375 N ASN A 674 50.594 46.190 132.229 1.00 37.26 A
ATOM 5376 CA ASN A 674 49.155 46.152 132.457 1.00 37.43 A
ATOM 5377 CB ASN A 674 48.420 47.058 131.454 1.00 37.02 A
ATOM 5378 CG ASN A 674 48.564 46.596 130.008 1.00 38.09 A
ATOM 5379 OD1 ASN A 674 47.920 45.635 129.580 1.00 36.46 A
ATOM 5380 ND2 ASN A 674 49.408 47.289 129.247 1.00 37.05 A
ATOM 5381 C ASN A 674 48.598 44.729 132.418 1.00 38.80 A
ATOM 5382 O ASN A 674 49.356 43.755 132.405 1.00 37.64 A
ATOM 5383 N ASP A 675 47.273 44.615 132.411 1.00 40.23 A
ATOM 5384 CA ASP A 675 46.611 43.315 132.401 1.00 41.27 A
ATOM 5385 CB ASP A 675 45.678 43.212 133.607 1.00 42.85 A
ATOM 5386 CG ASP A 675 46.433 43.104 134.909 1.00 44.88 A
ATOM 5387 OD1 ASP A 675 47.508 43.727 135.023 1.00 47.31 A
ATOM 5388 OD2 ASP A 675 45.951 42.405 135.824 1.00 47.63 A
ATOM 5389 C ASP A 675 45.827 43.029 131.129 1.00 40.89 A
ATOM 5390 O ASP A 675 45.146 42.009 131.034 1.00 42.50 A
ATOM 5391 N ILEA676 45.918 43.924 130.153 1.00 39.93 A
ATOM 5392 CA ILE A 676 45.214 43.733 128.891 1.00 39.32 A
ATOM 5393 CB ILE A 676 45.266 45.016 128.036 1.00 38.92 A
ATOM 5394 CG2 ILE A 676 44.499 44.814 126.734 1.00 37.50 A
ATOM 5395 CG1 ILE A 676 44.677 46.186 128.830 1.00 38.79 A
ATOM 5396 CD1 ILE A 676 44.778 47.532 128.126 1.00 37.75 A
ATOM 5397 C ILEA676 45.861 42.583 128.117 1.0038.92 A
ATOM 5398 O ILEA676 47.074 42.399 128.168 1.00 38.99 A
ATOM 5399 N PROA677 45.053 41.772 127.414 1.00 38.57 A
ATOM 5400 CD PRO A 677 43.584 41.695 127.448 1.00 38.01 A
ATOM 5401 CA PRO A 677 45.610 40.653 126.647 1.00 38.04 A
ATOM 5402 CB PRO A 677 44.367 39.870 126.216 1.00 37.30 A
ATOM 5403 CG PRO A 677 43.350 40.226 127.249 1.00 37.53 A
ATOM 5404 C PROA677 46.387 41.173 125.439 1.00 37.66 A
ATOM 5405 O PRO A 677 45.910 42.052 124.719 1.00 37.38 A
ATOM 5406 N TYRA 678 47.579 40.630 125.221 1.00 36.88 A
ATOM 5407 CA TYR A 678 48.407 41.032 124.087 1.00 37.34 A
ATOM 5408 CB TYRA 678 49.777 41.526 124.560 1.00 37.42 A
ATOM 5409 CG TYRA 678 49.822 42.980 124.978 1.00 39.15 A
ATOM 5410 CD1 TYRA678 49.087 43.438 126.071 1.0039.45 A
ATOM 5411 CE1 TYR A 678 49.136 44.776 126.461 1.00 38.90 A
ATOM 5412 CD2 TYRA678 50.611 43.899 124.280 1.00 38.07 A
ATOM 5413 CE2 TYR A 678 50.666 45.236 124.661 1.00 38.27 A
ATOM 5414 CZ TYR A 678 49.928 45.669 125.752 1.00 38.31 A
ATOM 5415 OH TYRA678 49.982 46.987 126.137 1.00 34.85 A
ATOM 5416 C TYRA678 48.612 39.856 123.147 1.00 36.80 A
ATOM 5417 O TYRA678 48.333 38.711 123.499 1.00 37.59 A
ATOM 5418 N ARG A 679 49.100 40.145 121.946 1.00 36.25 A
ATOM 5419 CA ARGA679 49.375 39.106 120.961 1.00 34.19 A
ATOM 5420 CB ARG A 679 48.964 39.573 119.562 1.00 34.69 A
ATOM 5421 CG ARG A 679 47.471 39.664 119.344 1.00 37.11 A
ATOM 5422 CD ARG A 679 46.833 38.280 119.390 1.00 39.39 A
ATOM 5423 NE ARG A 679 45.409 38.319 119.074 1.00 40.60 A
ATOM 5424 CZ ARG A 679 44.607 37.257 119.091 1.00 42.99 A
ATOM 5425 NH1 ARG A 679 43.322 37.393 118.787 1.00 42.34 A
ATOM 5426 NH2 ARG A 679 45.086 36.060 119.412 1.00 43.33 A
ATOM 5427 C ARG A 679 50.873 38.812 120.980 1.0032.70 A
ATOM 5428 O ARG A 679 51.687 39.736 120.945 1.0032.80 A⁻
ATOM 5429 N LEU A 680 51.240 37.536 121.057 1.00 30.30 A
ATOM 5430 CA LEU A 680 52.649 37.167 121.054 1.00 29.85 A
ATOM 5431 CB LEU A 680 52.856 35.830 121.768 1.00 30.50 A
ATOM 5432 CG LEU A 680 54.288 35.366 122.069 1.00 32.52 A
ATOM 5433 CD1 LEUA680 55.083 35.215 120.780 1.00 36.09 A
ATOM 5434 CD2 LEU A 680 54.970 36.359 122.978 1.00 34.60 A
ATOM 5435 C LEU A 680 53.062 37.059 119.588 1.00 30.78 A
ATOM 5436 O LEUA680 52.813 36.048 118.933 1.00 31.81 A
ATOM 5437 N LEU A 681 53.685 38.111 119.069 1.00 29.84 A
ATOM 5438 CA LEU A 681 54.100 38.117 117.674 1.00 29.97 A
ATOM 5439 CB LEU A 681 54.249 39.557 117.174 1.00 29.36 A
ATOM 5440 CG LEU A 681 54.827 39.697 115.761 1.00 29.50 A
ATOM 5441 CD1 LEU A 681 53.889 39.050 114.755 1.00 28.83 A
ATOM 5442 CD2 LEU A 681 55.033 41.164 115.433 1.00 30.52 A
ATOM 5443 C LEU A 681 55.401 37.369 117.414 1.00 29.29 A
ATOM 5444 O LEU A 681 55.57136.755116.3591.0029.28 A
ATOM 5445 N CYS A 682 56.312 37.409 118.379 1.00 28.81 A
ATOM 5446 CA CYS A 682 57.610 36.776 118.209 1.00 28.68 A
ATOM 5447 CB CYS A 682 58.502 37.712 117.384 1.00 28.25 A
ATOM 5448 SG CYS A 682 60.216 37.205 117.184 1.00 28.85 A
ATOM 5449 C CYS A 682 58.281 36.458 119.541 1.00 29.22 A
ATOM 5450 O CYS A 682 57.998 37.092 120.559 1.00 30.70 A
ATOM 5451 N ASP A 683 59.168 35.469 119.526 1.0028.42 A
ATOM 5452 CA ASP A 683 59.901 35.068 120.722 1.0029.63 A
ATOM 5453 CB ASP A 683 59.098 34.029 121.522 1.00 30.50 A
ATOM 5454 CG ASP A 683 58.907 32.716 120.772 1.00 32.85 A
ATOM 5455 OD Asp683 58.100 31.888 121.243 1.00 32.01 A
ATOM 5456 OD2 ASP A 683 59.561 32.504 119.724 1.00 32.54 A
ATOM 5457 C ASP A 683 61.250 34.503 120.274 1.00 28.13 A
ATOM 5458 O ASP A 683 61.547 34.487 119.083 1.0029.03 A
ATOM 5459 N PRO A 684 62.088 34.044 121.215 1.00 27.72 A
ATOM 5460 CD PRO A 684 61.977 34.121 122.684 1.0027.48 A
ATOM 5461 CA PRO A 684 63.390 33.499 120.828 1.00 27.83 A
ATOM 5462 CB PRO A 684 63.911 32.921 122.137 1.00 28.72 A
ATOM 5463 CG PRO A 684 63.408 33.906 123.133 1.00 26.19 A
ATOM 5464 C PRO A 684 63.368 32.471 119.697 1.00 29.02 A
ATOM 5465 O PRO A 684 64.388 32.229 119.059 1.00 29.31 A
ATOM 5466 N SERA685 62.208 31.871 119.450 1.00 30.78 A
ATOM 5467 CA SERA 685 62.071 30.872 118.388 1.00 31.18 A
ATOM 5468 CB SER A 685 60.886 29.949 118.674 1.00 30.51 A
ATOM 5469 OG SERA 685 61.025 29.316 119.929 1.00 35.53 A
ATOM 5470 C SERA685 61.847 31.547 117.042 1.0031.98 A
ATOM 5471 O SERA685 62.103 30.966 115.990 1.0032.48 A
ATOM 5472 N GLY A 686 61.353 32.779 117.085 1.0031.50 A
ATOM 5473 CA GLY A 686 61.087 33.504 115.862 1.00 31.77 A
ATOM 5474 C GLY A 686 59.642 33.952 115.823 1.00 32.02 A
ATOM 5475 O GLYA686 58.902 33.771 116.790 1.0032.43 A
ATOM 5476 N PHEA687 59.234 34.539 114.705 1.00 32.06 A
ATOM 5477 CA PHE A 687 57.869 35.009 114.563 1.00 34.05 A
ATOM 5478 CB PHE A 687 57.718 35.820 113.277 1.0031.98 A
ATOM 5479 CG PHE A 687 58.425 37.139 113.317 1.00 31.24 A
ATOM 5480 CD1 PHE A 687 59.811 37.205 113.220 1.0030.90 A
ATOM 5481 CD2 PHE A 687 57.708 38.319 113.478 1.00 30.25 A
ATOM 5482 CE1 PHE A 687 60.474 38.433 113.284 1.00 29.92 A
ATOM 5483 CE2 PHE A 687 58.360 39.549 113.543 1.00 30.92 A
ATOM 5484 CZ PHE A 687 59.746 39.605 113.447 1.0029.08 A
ATOM 5485 C PHEA687 56.869 33.863 114.581 1.00 36.21 A
ATOM 5486 O PHE A 687 57.118 32.793 114.023 1.00 36.33 A
ATOM 5487 N GLN A 688 55.737 34.107 115.233 1.00 38.05 A
ATOM 5488 CA GLN A 688 54.668 33.126 115.360 1.00 40.88 A
ATOM 5489 CB GLN A 688 54.010 33.276 116.733 1.00 39.40 A
ATOM 5490 CG GLN A 688 55.007 33.279 117.883 1.00 39.50 A
ATOM 5491 CD GLNA688 55.697 31.937 118.067 1.00 41.62 A
ATOM 5492 OE1 GLN A 688 55.090 30.972 118.537 1.00 42.28 A
ATOM 5493 NE2 GLN A 688 56.970 31.868 117.690 1.00 41.76 A
ATOM 5494 C GLN A 688 53.633 33.336 114.251 1.00 43.55 A
ATOM 5495 O GLN A 688 53.163 34.457 114.031 1.00 43.08 A
ATOM 5496 N GLU A 689 53.278 32.256 113.560 1.00 46.65 A
ATOM 5497 CA GLU A 689 52.312 32.332 112.468 1.00 50.83 A
ATOM 5498 CB GLU A 689 52.147 30.961 111.811 1.00 53.49 A
ATOM 5499 CG GLU A 689 53.379 30.529 111.039 1.00 57.92 A
ATOM 5500 CD GLU A 689 53.964 31.669 110.219 1.00 61.45 A
ATOM 5501 OE1 GLU A 689 53.213 32.275 109.419 1.00 63.30 A
ATOM 5502 OE2 GLU A 689 55.171 31.962 110.378 1.00 62.70 A
ATOM 5503 C GLU A 689 50.956 32.876 112.892 1.00 51.54 A
ATOM 5504 O GLU A 689 50.448 33.825 112.292 1.00 52.34 A
ATOM 5505 N ASP A 690 50.362 32.269 113.912 1.00 52.28 A
ATOM 5506 CA ASP A 690 49.076 32.739 114.407 1.00 53.63 A
ATOM 5507 CB ASP A 690 48.038 31.622 114.377 1.00 54.40 A
ATOM 5508 CG ASP A 690 47.944 30.966 113.025 1.00 55.90 A
ATOM 5509 OD1 ASP A 690 48.787 30.091 112.736 1.00 57.05 A
ATOM 5510 OD2 ASP A 690 47.042 31.338 112.246 1.00 57.54 A
ATOM 5511 C ASP A 690 49.288 33.214 115.828 1.00 53.04 A
ATOM 5512 O ASP A 690 49.163 32.440116.776 1.00 54.88 A
ATOM 5513 N PRO A 691 49.630 34.500 115.991 1.00 51.77 A
ATOM 5514 CD PRO A 691 49.754 35.516 114.930 1.00 51.25 A
ATOM 5515 CA PRO A 691 49.870 35.098 117.303 1.00 50.83 A
ATOM 5516 CB PRO A 691 49.809 36.591 117.010 1.00 50.97 A
ATOM 5517 CG PRO A 691 50.422 36.666 115.655 1.00 1 1.46 A
ATOM 5518 C PRO A 691 48.864 34.670 118.362 1.00 49.01 A
ATOM 5519 O PRO A 691 47.667 34.935 118.250 1.00 47.71 A
ATOM 5520 N THR A 692 49.364 33.982 119.379 1.00 48.47 A
ATOM 5521 CA THR A 692 48.530 33.537 120.480 1.00 48.33 A
ATOM 5522 CB THR A 692 49.222 32.431 121.293 1.00 48.26 A
ATOM 5523 OG1 THR A 692 48.413 32.096 122.425 1.00 52.42 A
ATOM 5524 CG2 THR A 692 50.575 32.902 121.789 1.00 47.91 A
ATOM 5525 C THR A 692 48.363 34.758 121.371 1.00 47.84 A
ATOM 5526 O THR A 692 49.040 35.768 121.170 1.00 48.23 A
ATOM 5527 N GLU A 693 47.465 34.680 122.345 1.00 46.06 A
ATOM 5528 CA GLU A 693 47.272 35.799 123.252 1.00 45.40 A
ATOM 5529 CB GLU A 693 45.801 36.218 123.299 1.00 46.80 A
ATOM 5530 CG GLU A 693 45.404 37.194 122.204 1.00 48.86 A
ATOM 5531 CD GLU A 693 44.126 37.955 122.527 1.0050.68 A
ATOM 5532 OE1 GLU A 693 43.803 38.923 121.802 1.00 51.42 A
ATOM 5533 OE2 GLU A 693 43.441 37.588 123.504 1.00 52.10 A
ATOM 5534 C GLU A 693 47.754 35.464 124.653 1.00 44.53 A
ATOM 5535 O GLUA693 47.412 34.417 125.201 1.00 45.71 A
ATOM 5536 N ILEA694 48.566 36.349 125.224 1.00 42.89 A
ATOM 5537 CA ILE A 694 49.075 36.154 126.576 1.00 42.24 A
ATOM 5538 CB ILE A 694 50.621 36.077 126.583 1.00 40.96 A
ATOM 5539 CG2 ILE A 694 51.077 34.882 125.764 1.00 41.39 A
ATOM 5540 CG1 ILE A 694 51.229 37.359 126.012 1.00 40.65 A
ATOM 5541 CD1 ILE A 694 51.333 38.489 127.005 1.00 39.38 A
ATOM 5542 C ILE A 694 48.586 37.305 127.461 1.00 42.45 A
ATOM 5543 O ILE A 694 48.125 38.330 126.949 1.00 41.11 A
ATOM 5544 N LYS A 695 48.672 37.136 128.779 1.00 42.05 A
ATOM 5545 CA LYS A 695 48.214 38.170 129.707 1.00 42.33 A
ATOM 5546 CB LYS A 695 46.789 37.855 130.178 1.00 44.90 A
ATOM 5547 CG LYS A 695 45.797 37.613 129.054 1.00 47.60 A
ATOM 5548 CD LYS A 695 44.441 37.157 129.583 1.0050.28 A
ATOM 5549 CE LYS A 695 43.525 36.725 128.438 1.00 1.32 A
ATOM 5550 NZ LYS A 695 42.182 36.289 128.911 1.00 50.9I A
ATOM 5551 C LYS A 695 49.112 38.342 130.933 1.00 41.37 A
ATOM 5552 O LYSA695 49.724 37.385 131.409 1.00 40.68 A
ATOM 5553 N LYS A 696 49.165 39.576 131.432 1.00 40.20 A
ATOM 5554 CA LYS A 696 49.942 39.954 132.611 1.00 39.37 A
ATOM 5555 CB LYS A 696 49.313 39.352 133.876 1.00 39.60 A
ATOM 5556 CG LYS A 696 47.867 39.792 134.109 1.00 41.21 A
ATOM 5557 CD LYS A 696 47.437 39.696 135.578 1.00 42.30 A
ATOM 5558 CE LYSA696 47.431 38.269 136.098 1.0043.25 A
ATOM 5559 NZ LYS A 696 46.924 38.194 137.502 1.00 45.00 A
ATOM 5560 C LYSA696 51.433 39.628 132.572 1.00 38.91 A
ATOM 5561 O LYSA696 52.265 40.481 132.886 1.00 37.50 A
ATOM 5562 N THR A 697 51.777 38.399 132.204 1.00 38.70 A
ATOM 5563 CA THR A 697 53.179 38.001 132.I34 1.00 38.61 A
ATOM 5564 CB THR A 697 53.585 37.108 133.328 1.00 39.37 A
ATOM 5565 OG1 THR A 697 52.766 35.932 133.346 1.00 42.30 A
ATOM 5566 CG2 THR A 697 53.421 37.856 134.638 1.00 39.98 A
ATOM 5567 C THRA697 53.466 37.235 130.853 1.00 37.13 A
ATOM 5568 O THR A 697 52.554 36.830 130.136 1.00 38.04 A
ATOM 5569 N VAL A 698 54.747 37.045 130.569 1.00 35.32 A
ATOM 5570 CA VAL A 698 55.162 36.315 129.386 1.00 34.33 A
ATOM 5571 CB VAL A 698 55.268 37.246 128.151 1.00 35.55 A
ATOM 5572 CG1 VAL A 698 56.445 38.208 128.306 1.0033.52 A
ATOM 5573 CG2 VAL A 698 55.408 36.411 126.890 1.00 34.78 A
ATOM 5574 C VALA698 56.517 35.686 129.671 1.00 33.13 A
ATOM 5575 O VALA698 57.325 36.241 130.420 1.00 31.13 A
ATOM 5576 N ALA A 699 56.757 34.517 129.090 1.00 31.60 A
ATOM 5577 CA ALA A 699 58.021 33.836 129.299 1.00 32.22 A
ATOM 5578 CB ALAA 699 57.830 32.325 129.209 1.00 31.42 A
ATOM 5579 C ALAA699 59.040 34.294 128.269 1.0032.42 A
ATOM 5580 O ALA A 699 58.718 34.453 127.093 1.00 34.41 A
ATOM 5581 N VALA700 60.265 34.516 128.731 1.00 31.73 A
ATOM 5582 CA VAL A 700 61.365 34.932 127.875 1.00 31.38 A
ATOM 5583 CB VAL A 700 62.001 36.246 128.382 1.00 3.39 A
ATOM 5584 CG1 VALA 700 63.062 36.717 127.406 1.00 31.14 A
ATOM 5585 CG2 VAL A 700 60.929 37.311 128.551 1.00 30.26 A
ATOM 5586 C VAL A 700 62.383 33.801 127.967 1.0031.44 A
ATOM 5587 O VAL A 700 63.264 33.814 128.820 1.00 30.28 A
ATOM 5588 N ASN A 701 62.253 32.825 127.077 1.00 33.32 A
ATOM 5589 CA ASN A 701 63.128. 31.660 127.088 1.00 35.24 A
ATOM 5590 CB ASN A 701 62.328 30.423 126.692 1.00 37.83 A
ATOM 5591 CG ASN A 701 61.079 30.257 127.526 1.0041.20 A
ATOM 5592 OD1 ASN A 701 61.148 30.159 128.750 1.00 41.38 A
ATOM 5593 ND2 ASN A 701 59.924 30.230 126.866 1.00 45.20 A
ATOM 5594 C ASN A 701 64.367 31.744 126.215 1.00 33.90 A
ATOM 5595 O ASN A 701 64.283 31.657 124.996 1.00 33.30 A
ATOM 5596 N GLY A 702 65.518 31.894 126.857 1.00 33.08 A
ATOM 5597 CA GLY A 702 66.771 31.961 126.131 1.00 32.55 A
ATOM 5598 C GLYA702 67.026 33.224125.331 1.0032.24 A
ATOM 5599 O GLYA702 66.235 34.172 125.342 1.00 32.41 A
ATOM 5600 N ILE A 703 68.152 33.218 124.628 1.00 30.75 A
ATOM 5601 CA ILE A 703 68.578 34.337 123.801 1.00 29.32 A
ATOM 5602 CB ILE A 703 69.984 34.085 123.253 1.00 27.83 A
ATOM 5603 CG2 ILE A 703 70.428 35.253 122.386 1.00 27.56 A
ATOM 5604 CG1 ILE A 703 70.947 33.878 124.420 1.00 27.33 A
ATOM 5605 CD1 ILE A 703 72.325 33.459 123.994 1.00 26.70 A
ATOM 5606 C ILE A 703 67.631 34.590 122.634 1.00 28.96 A
ATOM 5607 O ILE A 703 67.320 33.688 121.857 1.00 29.69 A
ATOM 5608 N GLY A 704 67.171 35.829 122.519 1.00 27.28 A
ATOM 5609 CA GLY A 704 66.270 36.166 121.440 1.00 26.82 A
ATOM 5610 C GLY A 704 65.461 37.399 121.758 1.00 26.55 A
ATOM 5611 O GLY A 704 65.751 38.122 122.713 1.00 26.65 A
ATOM 5612 N THR A 705 64.430 37.628 120.957 1.00 24.56 A
ATOM 5613 CA THR A 705 63.573 38.785 121.126 1.00 24.35 A
ATOM 5614 CB THR A 705 63.729 39.779 119.939 1.00 22.58 A
ATOM 5615 OG1 THR A 705 65.101 40.165 119.813 1.00 19.50 A
ATOM 5616 CG2 THR A 705 62.869 41.016 120.153 1.00 20.96 A
ATOM 5617 C THR A 705 62.117 38.361 121.180 1.00 25.10 A
ATOM 5618 O THR A 705 61.686 37.482 120.430 1.00 23.63 A
ATOM 5619 N VAL A 706 61.365 38.982 122.080 1.00 25.16 A
ATOM 5620 CA VAL A 706 59.946 38.702 122.184 1.00 26.76 A
ATOM 5621 CB VAL A 706 59.559 38.196 123.593 1.00 26.40 A
ATOM 5622 CG1 VAL A 706 60.087 39.123 124.639 1.00 29.98 A
ATOM 5623 CG2 VAL A 706 58.055 38.079 123.702 1.00 28.12 A
ATOM 5624 C VALA 706 59.209 39.997 121.855 1.00 26.04 A
ATOM 5625 O VAL A 706 59.524 41.062 122.390 1.00 27.51 A
ATOM 5626 N ILE A 707 58.246 39.906 120.949 1.00 25.07 A
ATOM 5627 CA ILE A 707 57.485 41.074 120.547 1.00 24.27 A
ATOM 5628 CB ILE A 707 57.674 41.358 119.045 1.00 23.26 A
ATOM 5629 CG2 ILE A 707 56.933 42.631 118.654 1.00 22.04 A
ATOM 5630 CG1 ILE A 707 59.167 41.490 118.736 1.00 21.31 A
ATOM 5631 CD1 ILE A 707 59.481 41.629 117.268 1.00 21.58 A
ATOM 5632 C ILE A 707 56.010 40.858 120.846 1.00 24.92 A
ATOM 5633 O ILE A 707 55.440 39.823 120.510 1.00 25.06 A
ATOM 5634 N LEU A 708 55.406 41.847 121.492 1.00 25.90 A
ATOM 5635 CA LEU A 708 53.998 41.801 121.860 1.00 25.95 A
ATOM 5636 CB LEU A 708 53.852 41.818 123.386 1.00 25.03 A
ATOM 5637 CG LEU A 708 54.515 40.665 124.138 1.00 25.58 A
ATOM 5638 CD1 LEU A 708 54.522 40.945 125.635 1.00 24.52 A
ATOM 5639 CD2 LEU A 708 53.765 39.377 123.828 1.00 25.47 A
ATOM 5640 C LEU A 708 53.326 43.032 121.283 1.00 25.77 A
ATOM 5641 O LEU A 708 53.919 44.105 121.252 1.00 25.52 A
ATOM 5642 N TYRA 709 52.090 42.888 120.826 1.00 26.87 A
ATOM 5643 CA TYRA 709 51.394 44.032 120.273 1.00 29.03 A
ATOM 5644 CB TYRA 709 51.585 44.091 118.751 1.0028.04 A
ATOM 5645 CG TYR A 709 50.867 43.008 117.980 1.00 28.04 A
ATOM 5646 CD1 TYR A 709 49.528 43.151 117.625 1.00 28.84 A
ATOM 5647 CE1 TYR A 709 48.864 42.156 116.913 1.00 29.42 A
ATOM 5648 CD2 TYR A 709 51.528 41.838 117.604 1.00 27.00 A
ATOM 5649 CE2 TYR A 709 50.876 40.840 116.896 1.00 28.36 A
ATOM 5650 CZ TYR A 709 49.545 41.006 116.554 1.00 30.53 A
ATOM 5651 OH TYRA 709 48.889 40.017 115.860 1.00 33.19 A
ATOM 5652 C TYRA 709 49.920 44.003 120.619 1.00 30.62 A
ATOM 5653 O TYRA709 49.345 42.945 120.878 1.00 30.64 A
ATOM 5654 N LEU A 710 49.320 45.185 120.619 1.00 32.33 A
ATOM 5655 CA LEU A 710 47.915 45.340 120.929 1.00 34.98 A
ATOM 5656 CB LEU A 710 47.766 46.203 122.182 1.00 34.98 A
ATOM 5657 CG LEU A 710 46.520 46.081 123.060 1.00 35.54 A
ATOM 5658 CD1 LEU A 710 46.559 47.193 124.099 1.00 35.33 A
ATOM 5659 CD2 LEU A 710 45.256 46.186 122.232 1.00 36.47 A
ATOM 5660 C LEU A 710 47.255 46.033 119.739 1.00 37.94 A
ATOM 5661 O LEU A 710 47.578 47.177 119.428 1.00 38.85 A
ATOM 5662 N ALA A 711 46.349 45.339 119.062 1.00 40.68 A
ATOM 5663 CA ALA A 711 45.656 45.933 117.929 1.00 45.03 A
ATOM 5664 CB ALA A 711 44.916 44.862 117.147 1.00 44.38 A
ATOM 5665 C ALA A 711 44.675 46.963 118.482 1.00 48.06 A
ATOM 5666 O ALA A 711 44.369 46.955 119.675 1.00 48.76 A
ATOM 5667 N SER A 712 44.186 47.855 117.630 1.00 51.73 A
ATOM 5668 CA SERA 712 43.246 48.876 118.086 1.00 55.35 A
ATOM 5669 CB SERA 712 43.746 50.270 117.685 1.00 55.94 A
ATOM 5670 OG SERA 712 43.953 50.366116.286 1.00 56.18 A
ATOM 5671 C SER A 712 41.833 48.652 117.548 1.00 56.68 A
ATOM 5672 O SER A 712 41.664 47.761 116.685 1.00 57.76 A
ATOM 5673 OXT SER A 712 40.913 49.373 117.998 1.00 57.74 A
ATOM 5674 CB MET B 1 157.213 63.344 155.329 1.00 56.23 B
ATOM 5675 CG MET B 1 158.062 62.872 156.508 1.00 58.96 B
ATOM 5676 SD MET B 1 159.285 64.099 157.059 1.00 64.32 B
ATOM 5677 CE MET B 1 160.825 63.401 156.383 1.00 60.94 B
ATOM 5678 C MET B 1 155.165 62.075 156.024 1.0050.57 B
ATOM 5679 O MET B 1 153.994 62.461 155.971 1.0050.75 B
ATOM 5680 N MET B 1 155.410 62.887 153.686 1.00 55.13 B
ATOM 5681 CA METB 1 156.136 62.347 154.875 1.00 53.68 B
ATOM 5682 N VAL B 2 155.665 61.400 157.055 1.00 45.98 B
ATOM 5683 CA VALB 2 154.881 61.066158.238 1.0039.99 B
ATOM 5684 CB VALB 2 154.219 62.313 158.842 1.00 39.73 B
ATOM 5685 CG1 VAL B 2 153.370 61.921 160.041 1.00 38.80 B
ATOM 5686 CG2 VAL B 2 155.286 63.304 159.262 1.00 39.32 B
ATOM 5687 C VALB 2 153.804 60.018 158.005 1.00 36.54 B
ATOM 5688 O VALB 2 152.785 60.278 157.367 1.0034.42 B
ATOM 5689 N SER B 3 154.047 58.828 158.540 1.00 32.37 B
ATOM 5690 CA SER B 3 153.106 57.722 158.430 1.00 29.68 B
ATOM 5691 CB SER B 3 153.105 57.163 157.009 1.0029.45 B
ATOM 5692 OG SER B 3 154.396 56.712 156.651 1.0028.52 B
ATOM 5693 C SER B 3 153.526 56.632 159.405 1.0027.18 B
ATOM 5694 O SER B 3 154.626 56.673 159.958 1.00 26.72 B
ATOM 5695 N ILE B 4 152.646 55.665 159.629 1.00 25.37 B
ATOM 5696 CA ILE B 4 152.973 54.569 160.521 1.00 25.66 B
ATOM 5697 CB ILE B 4 151.731 53.709 160.843 1.00 24.76 B
ATOM 5698 CG2 ILE B 4 152.125 52.542 161.728 1.00 26.38 B
ATOM 5699 CG1 ILE B 4 150.675 54.562 161.547 1.00 25.10 B
ATOM 5700 CD1 ILE B 4 149.420 53.794 161.939 1.00 25.05 B
ATOM 5701 C ILE B 4 154.007 53.718 159.790 1.00 26.07 B
ATOM 5702 O ILE B 4 153.845 53.415 158.608 1.00 25.23 B
ATOM 5703 N ARG B 5 155.081 53.353 160.482 1.0026.08 B
ATOM 5704 CA ARG B 5 156.116 52.537 159.867 1.00 26.93 B
ATOM 5705 CB ARG B 5 157.381 52.538 160.722 1.0027.69 B
ATOM 5706 CG ARG B 5 158.585 51.964 160.007 1.00 30.88 B
ATOM 5707 CD ARG B 5 159.780 51.926 160.925 1.00 36.83 B
ATOM 5708 NE ARG B 5 161.021 51.725 160.187 1.0041.50 B
ATOM 5709 CZ ARG B 5 162.228 51.763 160.740 1.00 43.69 B
ATOM 5710 NH1 ARG B 5 162.351 51.993 162.042 1.0043.27 B
ATOM 5711 NH2 ARG B 5 163.311 51.582 159.992 1.00 44.61 B
ATOM 5712 C ARG B 5 155.611 51.107 159.706 1.0025.96 B
ATOM 5713 O ARG B 5 155.094 50.519 160.655 1.00 25.21 B
ATOM 5714 N ARG B 6 155.771 50.554 158.507 1.00 24.32 B
ATOM 5715 CA ARG B 6 155.313 49.199 158.229 1.00 24.40 B
ATOM 5716 CB ARG B 6 153.943 49.249 157.532 1.00 23.67 B
ATOM 5717 CG ARG B 6 152.786 49.428 158.512 1.00 27.30 B
ATOM 5718 CD ARG B 6 151.400 49.414 157.856 1.00 27.20 B
ATOM 5719 NE ARG B 6 151.030 50.728 157.351 1.00 30.01 B
ATOM 5720 CZ ARG B 6 149.915 51.380 157.673 1.00 27.01 B
ATOM 5721 NH1 ARG B 6 149.030 50.851 158.507 1.00 26.39 B
ATOM 5722 NH2 ARG B 6 149.698 52.581 157.166 1.00 25.64 B
ATOM 5723 C ARG B 6 156.298 48.372 157.405 1.00 24.16 B
ATOM 5724 O ARG B 6 156.951 48.887 156.496 1.0022.38 B
ATOM 5725 N SER B 7 156.402 47.087 157.745 1.00 23.21 B
ATOM 5726 CA SER B 7 157.289 46.152 157.048 1.00 23.31 B
ATOM 5727 CB SER B 7 157.313 44.793 157.759 1.00 21.49 B
ATOM 5728 OG SER B 7 157.640 44.921 159.130 1.0025.22 B
ATOM 5729 C SER B 7 156.779 45.951 155.622 1.00 22.30 B
ATOM 5730 O SER B 7 157.552 45.975 154.668 1.00 21.99 B
ATOM 5731 N PHE B 8 155.472 45.726 155.504 1.0022.38 B
ATOM 5732 CA PHE B 8 154.802 45.534 154.218 1.00 21.66 B
ATOM 5733 CB PHE B 8 154.878 44.066 153.760 1.00 19.76 B
ATOM 5734 CG PHE B 8 154.261 43.081 154.713 1.00 21.64 B
ATOM 5735 CD1 PHE B 8 152.997 42.553 154.467 1.00 19.56 B
ATOM 5736 CD2 PHE B 8 154.960 42.650 155.841 1.00 21.25 B
ATOM 5737 CE1 PHE B 8 152.438 41.607 155.324 1.00 19.10 B
ATOM 5738 CE2 PHE B 8 154.409 41.705 156.707 1.00 19.43 B
ATOM 5739 CZ PHE B 8 153.145 41.182 156.446 1.00 21.14 B
ATOM 5740 C PHE B 8 153.354 45.999 154.353 1.00 21.71 B
ATOM 5741 O PHE B 8 152.967 46.516 155.405 1.00 20.62 B
ATOM 5742 N GLU B 9 152.560 45.831 153.301 1.0020.74 B
ATOM 5743 CA GLU B 9 151.171 46.277 153.336 1.0021.82 B
ATOM 5744 CB GLU B 9 150.984 47.442 152.363 1.00 23.37 B
ATOM 5745 CG GLU B 9 151.931 48.602 152.591 1.00 25.06 B
ATOM 5746 CD GLU B 9 151.867 49.627 151.477 1.00 25.76 B
ATOM 5747 OE1 GLUB 9 152.099 49.257 150.311 1.0024.44 B
ATOM 5748 OE2 GLU B 9 151.585 50.806 151.767 1.00 32.12 B
ATOM 5749 C GLU B 9 150.170 45.180 152.993 1.00 21.96 B
ATOM 5750 O GLU B 9 150.454 44.301 152.180 1.00 22.46 B
ATOM 5751 N ALAB 10 148.996 45.246 153.613 1.00 19.71 B
ATOM 5752 CA ALA B 10 147.937 44.276 153.373 1.00 21.14 B
ATOM 5753 CB ALA B 10 147.989 43.169 154.420 1.00 19.71 B
ATOM 5754 C ALA B 10 146.574 44.967 153.400 1.00 21.86 B
ATOM 5755 O ALA B 10 146.269 45.721 154.324 1.0020.64 B
ATOM 5756 N TYRB 11 145.767 44.714 152.374 1.00 23.25 B
ATOM 5757 CA TYR B 11 144.437 45.300 152.271 1.00 23.63 B
ATOM 5758 CB TYR B 11 144.369 46.292 151.110 1.0023.62 B
ATOM 5759 CG TYR B 11 145.444 47.344 151.112 1.00 25.25 B
ATOM 5760 CD1 TYR B 11 146.726 47.056 150.651 1.00 25.30 B
ATOM 5761 CE1 TYR B 11 147.726 48.029 150.647 1.00 27.39 B
ATOM 5762 CD2 TYR B 11 145.181 48.637 151.574 1.0025.70 B
ATOM 5763 CE2 TYR B 11 146.175 49.621 151.575 1.00 26.13 B
ATOM 5764 CZ TYR B 11 147.444 49.310 151.111 1.00 25.88 B
ATOM 5765 OH TYR B 11 148.435 50.268 151.114 1.00 26.11 B
ATOM 5766 C TYRB 11 143.374 44.235 152.035 1.00 24.48 B
ATOM 5767 O TYRB 11 143.625 43.221 151.387 1.0025.76 B
ATOM 5768 N VAL B 12 142.183 44.472 152.568 1.00 24.72 B
ATOM 5769 CA VAL B 12 141.066 43.564 152.378 1.00 24.28 B
ATOM 5770 CB VAL B 12 140.204 43.488 153.660 1.00 24.03 B
ATOM 5771 CG1 VAL B 12 139.970 44.887 154.216 1.00 24.68 B
ATOM 5772 CG2 VAL B 12 138.892 42.791 153.370 1.00 22.19 B
ATOM 5773 C VAL B 12 140.262 44.129 151.198 1.00 25.12 B
ATOM 5774 O VALB 12 139.549 45.119 151.337 1.00 25.19 B
ATOM 5775 N ASP B 13 140.403 43.512 150.026 1.00 25.71 B
ATOM 5776 CA ASP B 13 139.703 43.986 148.839 1.00 26.05 B
ATOM 5777 CB ASP B 13 140.608 43.849 147.616 1.00 25.32 B
ATOM 5778 CG ASP B 13 141.777 44.823 147.659 1.00 28.06 B
ATOM 5779 OD1 ASP B 13 141.555 45.984 148.067 1.00 28.17 B
ATOM 5780 OD2 ASP B 13 142.907 44.443 147.282 1.00 26.63 B
ATOM 5781 C ASP B 13 138.334 43.364 148.574 1.00 25.67 B
ATOM 5782 O ASPB 13 137.613 43.810 147.683 1.00 27.69 B
ATOM 5783 N ASP B 14 137.982 42.337 149.338 1.00 24.88 B
ATOM 5784 CA ASP B 14 136.674 41.697 149.223 1.00 25.22 B
ATOM 5785 CB ASP B 14 136.613 40.741 148.025 1.00 25.25 B
ATOM 5786 CG ASP B 14 135.183 40.496 147.547 1.00 24.13 B
ATOM 5787 OD1 ASP B 14 134.982 40.308 146.328 1.00 25.54 B
ATOM 5788 OD2 ASP B 14 134.257 40.484 148.385 1.00 23.89 B
ATOM 5789 C ASP B 14 136.435 40.964 150.536 1.00 24.59 B
ATOM 5790 O ASP B 14 137.342 40.869 151.358 1.00 24.69 B
ATOM 5791 N MET B 15 135.223 40.465 150.748 1.0025.21 B
ATOM 5792 CA MET B 15 134.891 39.796 152.002 1.00 26.98 B
ATOM 5793 CB MET B 15 133.480 39.207 151.929 1.00 26.74 B
ATOM 5794 CG MET B 15 132.396 40.250 151.718 1.00 24.90 B
ATOM 5795 SD MET B 15 132.561 41.610 152.868 1.00 28.88 B
ATOM 5796 CE MET B 15 131.722 40.948 154.321 1.00 27.85 B
ATOM 5797 C MET B 15 135.869 38.720 152.466 1.00 29.32 B
ATOM 5798 O METB 15 136.051 38.521 153.670 1.0031.17 B
ATOM 5799 N ASN B 16 136.502 38.026 151.529 1.00 29.78 B
ATOM 5800 CA ASN B 16 137.439 36.979 151.907 1.00 31.48 B
ATOM 5801 CB ASN B 16 136.767 35.612 151.760 1.0034.45 B
ATOM 5802 CG ASN B 16 136.431 35.292 150.330 1.0037.79 B
ATOM 5803 OD1 ASN B 16 136.004 36.168 149.571 1.00 41.38 B
ATOM 5804 ND2 ASN B 16 136.611 34.036 149.947 1.00 41.39 B
ATOM 5805 C ASN B 16 138.706 37.039 151.062 1.00 30.27 B
ATOM 5806 O ASN B 16 139.345 36.020 150.804 1.00 32.13 B
ATOM 5807 N ILE B 17 139.070 38.241 150.639 1.00 27.77 B
ATOM 5808 CA ILE B 17 140.264 38.423 149.830 1.00 27.57 B
ATOM 5809 CB ILE B 17 139.889 38.816 148.393 1.00 27.14 B
ATOM 5810 CG2 ILE B 17 141.130 39.226 147.619 1.00 24.20 B
ATOM 5811 CG1 ILE B 17 139.179 37.638 147.721 1.00 27.36 B
ATOM 5812 CD1 ILE B 17 138.573 37.970 146.385 1.00 31.52 B
ATOM 5813 C ILE B 17 141.204 39.473 150.416 1.00 28.36 B
ATOM 5814 O ILE B 17 140.821 40.624 150.633 1.00 27.90 B
ATOM 5815 N ILE B 18 142.440 39.059 150.674 1.00 27.87 B
ATOM 5816 CA ILE B 18 143.445 39.947 151.228 1.0026.10 B
ATOM 5817 CB ILE B 18 143.955 39.419 152.586 1.00 26.90 B
ATOM 5818 CG2 ILE B 18 8 145.205 40.182 153.015 1.00 23.00 B
ATOM 5819 CE1 ILE B 18 142.837 39.547 153.627 1.00 27.68 B
ATOM 5820 CD1 ILE B 18 143.195 39.018 154.988 1.00 3025 B
ATOM 5821 C ILE B 18 144.616 40.096 150.270 1.00 26.42 B
ATOM 5822 O ILE B 18 145.237 39.111 149.868 1.00 26.54 B
ATOM 5823 N THR B 19 144.905 41.340 149.904 1.00 25.39 B
ATOM 5824 CA THR B 19 146.003 41.645 149.000 1.00 26.05 B
ATOM 5825 CB THR B 19 145.621 42.797 148.037 1.00 25.86 B
ATOM 5826 OG1 THR B 19 144.507 42.395 147.230 1.00 26.83 B
ATOM 5827 CG2 THR B 19 146.782 43.151 147.136 1.00 26.59 B
ATOM 5828 C THR B 19 147.221 42.054 149.829 1.00 27.40 B
ATOM 5829 O THR B 19 147.142 42.967 150.662 1.00 26.46 B
ATOM 5830 N VAL B 20 148.339 41.365 149.610 1.00 25.87 B
ATOM 5831 CA VAL B 20 149.572 41.656 150.332 1.00 24.45 B
ATOM 5832 CB VAL B 20 150.164 40.376 150.965 1.00 23.43 B
ATOM 5833 CG1 VALB 20 151.504 40.685 151.606 1.0021.71 B
ATOM 5834 CG2 VAL B 20 149.203 39.813 152.002 1.00 20.70 B
ATOM 5835 C VALB 20 150.599 42.263 149.385 1.00 25.44 B
ATOM 5836 O VAL B 20 150.924 41.669 148.358 1.00 27.00 B
ATOM 5837 N LEU B 21 151.098 43.450 149.727 1.00 23.86 B
ATOM 5838 CA LEU B 21 152.092 44.131 148.904 1.00 24.11 B
ATOM 5839 CB LEU B 21 151.653 45.566 148.602 1.00 23.15 B
ATOM 5840 CG LEU B 21 150.271 45.747 147.965 1.0023.90 B
ATOM 5841 CD1 LEU B 21 150.050 47.227 147.687 1.00 23.52 B
ATOM 5842 CD2 LEU B 21 150.162 44.929 146.675 1.00 22.89 B
ATOM 5843 C LEU B 21 153.434 44.149 149.619 1.00 24.81 B
ATOM 5844 O LEU B 21 153.565 44.669 150.726 1.00 24.69 B
ATOM 5845 N ILE B 22 154.436 43.574 148.974 1.00 25.33 B
ATOM 5846 CA ILE B 22 155.758 43.510 149.558 1.00 24.49 B
ATOM 5847 CB ILE B 22 156.263 42.058 149.565 1.00 25.96 B
ATOM 5848 CG2 ILE B 22 157.705 41.993 150.066 1.00 25.30 B
ATOM 5849 CG1 ILE B 22 155.347 41.220 150.452 1.00 25.97 B
ATOM 5850 CD1 ILE B 22 155.498 39.764 150.241 1.00 31.16 B
ATOM 5851 C ILE B 22 156.740. 44.371 148.795 1.00 24.02 B
ATOM 5852 O ILE B 22 156.841 44.277 147.579 1.00 22.57 B
ATOM 5853 N PRO B 23 157.465 45.245 149.501 1.00 24.42 B
ATOM 5854 CD PRO B 23 157.432 45.579 150.931 1.00 23.45 B
ATOM 5855 CA PRO B 23 158.427 46.080 148.780 1.00 25.93 B
ATOM 5856 CB PRO B 23 159.075 46.909 149.894 1.00 26.26 B
ATOM 5857 CG PRO B 23 158.807 46.119 151.148 1.00 26.53 B
ATOM 5858 C PRO B 23 159.417 45.180 148.029 1.00 26.82 B
ATOM 5859 O PRO B 23 159.886 44.175 148.565 1.00 24.81 B
ATOM 5860 N ALA B 24 159.710 45.542 146.784 1.00 26.47 B
ATOM 5861 CA ALA B 24 160.605 44.766 145.936 1.00 29.34 B
ATOM 5862 CB ALA B 24 160.990 45.579 144.699 1.00 27.55 B
ATOM 5863 C ALAB 24 161.858 44.288 146.657 1.0030.60 B
ATOM 5864 O ALA B 24 162.217 43.112 146.589 1.00 31.07 B
ATOM 5865 N GLU B 25 162.518 45.208 147.347 1.00 32.33 B
ATOM 5866 CA GLU B 25 163.740 44.902 148.076 1.00 33.04 B
ATOM 5867 CB GLU B 25 164.258 46.169 148.760 1.00 35.85 B
ATOM 5868 CG GLU B 25 163.144 47.059 149.310 1.00 43.41 B
ATOM 5869 CD GLU B 25 163.614 47.965 150.432 1.0046.82 B
ATOM 5870 OE1 GLU B 25 164.676 48.607 150.272 1.00 50.48 B
ATOM 5871 OE2 GLU B 25 162.917 48.039 151.471 1.00 47.34 B
ATOM 5872 C GLU B 25 163.581 43.794 149.116 1.00 30.62 B
ATOM 5873 O GLU B 25 164.572 43.257 149.602 1.0031.51 B
ATOM 5874 N GLN B 26 162.344 43.445 149.449 1.0028.03 B
ATOM 5875 CA GLN B 26 162.098 42.417 150.458 1.00 25.60 B
ATOM 5876 CB GLN B 26 161.332 43.032 151.629 1.00 27.17 B
ATOM 5877 CG GLN B 26 161.975 44.286 152.193 1.00 27.08 B
ATOM 5878 CD GLN B 26 163.167 43.988 153.071 1.0027.52 B
ATOM 5879 OE1 GLN B 26 163.972 44.869 153.354 1.00 30.82 B
ATOM 5880 NE2 GLN B 26 163.279 42.748 153.522 1.0027.99 B
ATOM 5881 C GLN B 26 161.320 41.212 149.931 1.00 24.28 B
ATOM 5882 O GLN B 26 160.902 40.348 150.701 1.00 22.17 B
ATOM 5883 N LYS B 27 161.130 41.161 148.619 1.00 22.40 B
ATOM 5884 CA LYS B 27 160.391 40.074 147.998 1.00 23.80 B
ATOM 5885 CB LYS B 27 160.458 40.207 146.474 1.0023.24 B
ATOM 5886 CG LYS B 27 159.814 39.056 145.709 1.00 25.66 B
ATOM 5887 CD LYS B 27 159.973 39.252 144.206 1.0029.59 B
ATOM 5888 CE LYS B 27 159.385 38.086 143.412 1.0033.80 B
ATOM 5889 NZ LYS B 27 159.551 38.274 141.929 1.00 35.39 B
ATOM 5890 C LYS B 27 160.915 38.701 148.412 1.0024.30 B
ATOM 5891 O LYS B 27 160.144 37.791 148.716 1.00 23.40 B
ATOM 5892 N GLU B 28 162.233 38.567 148.432 1.00 24.73 B
ATOM 5893 CA GLU B 28 162.879 37.309 148.767 1.0026.59 B
ATOM 5894 CB GLU B 28 164.267 37.293 148.121 1.0027.53 B
ATOM 5895 CG GLU B 28 164.674 35.978 147.481 1.00 31.91 B
ATOM 5896 CD GLU B 28 163.567 35.346 146.668 1.0031.58 B
ATOM 5897 OE1 GLU B 28 162.731 34.639 147.264 1.00 36.75 B
ATOM 5898 OE2 GLU B 28 163.521 35.561 145.439 1.0031.20 B
ATOM 5899 C GLU B 28 162.975 37.072 150.279 1.00 27.99 B
ATOM 5900 O GLU B 28 163.421 36.008 150.717 1.00 27.26 B
ATOM 5901 N ILE B 29 162.545 38.057 151.072 1.00 27.75 B
ATOM 5902 CA ILE B 29 162.589 37.950 152.532 1.00 27.78 B
ATOM 5903 CB ILE B 29 163.264 39.195 153.149 1.0029.29 B
ATOM 5904 CG2 ILE B 29 163.215 39.127 154.674 1.0026.47 B
ATOM 5905 CG1 ILE B 29 164.712 39.279 152.667 1.00 28.91 B
ATOM 5906 CD1 ILE B 29 165.397 40.579 153.015 1.00 32.65 B
ATOM 5907 C ILE B 29 161.216 37.759 153.185 1.00 28.43 B
ATOM 5908 O ILE B 29 161.018 36.820 53.958 1.00 29.58 B
ATOM 5909 N MET B 30 160.276 38.649 152.878 1.00 27.81 B
ATOM 5910 CA MET B 30 158.926 38.579 153.438 1.00 26.88 B
ATOM 5911 CB MET B 30 158.367 39.991 153.616 1.00 28.20 B
ATOM 5912 CG MET B 30 159.060 40.796 154.716 1.00 29.60 B
ATOM 5913 SD MET B 30 158.682 42.570 154.641 1.00 29.35 B
ATOM 5914 CE MET B 30 160.297 43.283 155.013 1.00 29.28 B
ATOM 5915 C MET B 30 158.036 37.770 152.506 1.0027.44 B
ATOM 5916 O MET B 30 157.141 38.301 151.854 1.0028.02 B
ATOM 5917 N THRB 31 158.284 36.467 152.472 1.0027.30 B
ATOM 5918 CA THR B 31 157.570 35.543 151.601 1.00 25.85 B
ATOM 5919 CB THR B 31 158.549 34.514 151.041 1.00 27.33 B
ATOM 5920 OG1 THR B 31 159.060 33.729 152.127 1.00 28.82 B
ATOM 5921 CG2 THR B 31 159.719 35.203 150.348 1.00 28.02 B
ATOM 5922 C THR B 31 156.452 34.757 152.274 1.00 25.60 B
ATOM 5923 O THR B 31 156.383 34.673 153.499 1.0027.04 B
ATOM 5924 N PRO B 32 155.550 34.174 151.472 1.00 24.39 B
ATOM 5925 CD PRO B 32 155.369 34.328 150.018 1.00 23.98 B
ATOM 5926 CA PRO B 32 154.461 33.388 152.062 1.00 25.62 B
ATOM 5927 CB PRO B 32 153.533 33.119 150.872 1.0024.39 B
ATOM 5928 CG PRO B 32 154.456 33.172 149.686 1.00 25.11 B
ATOM 5929 C PRO B 32 155.099 32.118 152.631 1.00 25.30 B
ATOM 5930 O PRO B 32 156.260 31.836 152.353 1.0027.02 B
ATOM 5931 N PRO B 33 154.351 31.320 153.403 1.00 25.75 B
ATOM 5932 CD PRO B 33 154.995 30.265 154.202 1.00 24.17 B
ATOM 5933 CA PRO B 33 152.949 31.471 153.802 1.00 25.41 B
ATOM 5934 CB PRO B 33 152.687 30.231 154.671 1.00 25.04 B
ATOM 5935 CG PRO B 33 153.862 29.316 154.410 1.00 25.22 B
ATOM 5936 C PRO B 33 152.637 32.740 154.576 1.00 25.54 B
ATOM 5937 O PRO B 33 153.430 33.194 155.399 1.0025.85 B
ATOM 5938 N PHE B 34 151.470 33.305 154.292 1.00 26.31 B
ATOM 5939 CA PHE B 34 150.988 34.479 154.998 1.00 25.56 B
ATOM 5940 CB PHE B 34 150.456 35.526 154.024 1.0023.79 B
ATOM 5941 CG PHE B 34 151.522 36.210 153.236 1.0024.17 B
ATOM 5942 CD1 PHE B 34 151.587 36.062 151.858 1.00 23.27 B
ATOM 5943 CD2 PHE B 34 152.471 37.002 153.872 1.0023.90 B
ATOM 5944 CE1 PHE B 34 152.579 36.690 151.122 1.0024.07 B
ATOM 5945 CE2 PHE B 34 153.467 37.635 153.145 1.00 24.80 B
ATOM 5946 CZ PHE B 34 153.520 37.478 151.763 1.00 24.85 B
ATOM 5947 C PHE B 34 149.849 33.978 155.876 1.00 26.75 B
ATOM 5948 O PHE B 34 149.102 33.075 155.480 1.0026.89 B
ATOM 5949 N ARG B 35 149.726 34.537 157.072 1.00 27.62 B
ATOM 5950 CA ARG B 35 148.651 34.134 157.971 1.00 28.64 B
ATOM 5951 CB ARG B 35 149.208 33.380 159.182 1.0029.03 B
ATOM 5952 CG ARG B 35 149.868 32.047 158.835 1.0029.20 B
ATOM 5953 CD ARG B 35 150.232 31.265 160.090 1.0029.43 B
ATOM 5954 NE ARG B 35 149.052 30.772 160.801 1.00 29.56 B
ATOM 5955 CZ ARG B 35 148.285 29.769 160.383 1.00 30.19 B
ATOM 5956 NH1 ARG B 35 148.568 29.136 159.251 1.00 29.63 B
ATOM 5957 NH2 ARG B 35 147.229 29.399 161.097 1.00 29.79 B
ATOM 5958 C ARG B 35 147.872 35.357 158.428 1.00 29.28 B
ATOM 5959 O ARG B 35 148.424 36.452 158.518 1.0029.25 B
ATOM 5960 N LEU B 36 146.583 35.168 158.693 1.00 30.48 B
ATOM 5961 CA LEU B 36 145.719 36.252 159.148 1.00 29.55 B
ATOM 5962 CB LEU B 36 144.416 36.259 158.347 1.00 28.88 B
ATOM 5963 CG LEU B 36 143.453 37.425 158.598 1.0028.22 B
ATOM 5964 CD1 LEU B 36 144.144 38.741 158.276 1.00 26.87 B
ATOM 5965 CD2 LEU B 36 142.204 37.250 157.738 1.00 26.87 B
ATOM 5966 C LEU B 36 145.426 36.054 160.633 1.00 29.83 B
ATOM 5967 O LEU B 36 144.933 35.000 161.046 1.00 29.35 B
ATOM 5968 N GLU B 37 145.732 37.073 161.430 1.00 29.24 B
ATOM 5969 CA GLU B 37 145.534 37.004 162.874 1.00 30.97 B
ATOM 5970 CB GLU B 37 146.883 37.107 163.593 1.0030.30 B
ATOM 5971 CG GLU B 37 147.951 36.132 163.131 1.0031.20 B
ATOM 5972 CD GLU B 37 149.330 36.493 163.665 1.0032.84 B
ATOM 5973 OE1 GLU B 37 150.289 35.739 163.405 1.00 35.31 B
ATOM 5974 OE2 GLU B 37 149.459 37.537 164.341 1.00 34.28 B
ATOM 5975 C GLU B 37 144.627 38.087 163.453 1.00 31.89 B
ATOM 5976 O GLU B 37 144.421 39.147 162.858 1.00 30.90 B
ATOM 5977 N THRB 38 144.093 37.783 164.632 1.00 33.62 B
ATOM 5978 CA THR B 38 143.258 38.687 165.414 1.00 35.50 B
ATOM 5979 CB THRB 38 141.750 38.502 165.164 1.00 35.39 B
ATOM 5980 OG1 THRB 38 141.342 37.208 165.620 1.00 37.62 B
ATOM 5981 CG2 THR B 38 141.431 38.652 163.691 1.00 36.68 B
ATOM 5982 C THR B 38 143.568 38.246 166.837 1.00 36.62 B
ATOM 5983 O THR B 38 144.309 37.282 167.031 1.00 35.55 B
ATOM 5984 N GLU B 39 143.017 38.937 167.825 1.0038.05 B
ATOM 5985 CA GLU B 39 143.259 38.579 169.220 1.00 40.18 B
ATOM 5986 CB GLU B 39 142.635 39.625 170.144 1.0039.80 B
ATOM 5987 CG GLU B 39 141.140 39.814 169.925 1.00 41.01 B
ATOM 5988 CD GLU B 39 140.504 40.740 170.944 1.0040.15 B
ATOM 5989 OE1 GLU B 39 139.294 41.009 170.816 1.00 40.58 B
ATOM 5990 OE2 GLU B 39 141.210 41.193 171.869 1.00 40.07 B
ATOM 5991 C GLU B 39 142.694 37.197 169.569 1.00 41.98 B
ATOM 5992 O GLU B 39 142.969 36.659 170.643 1.00 42.47 B
ATOM 5993 N ILE B 40 141.909 36.624 168.663 1.00 42.96 B
ATOM 5994 CA ILE B 40 141.306 35.320 168.906 1.00 45.22 B
ATOM 5995 CB ILE B 40 139.776 35.457 169.113 1.00 47.55 B
ATOM 5996 CG2 ILE B 40 139.090 34.101 168.973 1.00 49.61 B
ATOM 5997 CG1 ILE B 40 139.494 36.065 170.488 1.00 49.85 B
ATOM 5998 CD1 ILE B 40 138.014 36.200 170.799 1.00 52.44 B
ATOM 5999 C ILE B 40 141.558 34.296 167.801 1.00 45.19 B
ATOM 6000 O ILE B 40 141.520 33.091 168.051 1.00 45.56 B
ATOM 6001 N THR B 41 141.821 34.765 166.586 1.00 43.88 B
ATOM 6002 CA THR B 41 142.044 33.845 165.479 1.00 44.01 B
ATOM 6003 CB THR B 41 140.950 34.005 164.403 1.00 43.83 B
ATOM 6004 OG1 THR B 41 140.857 35.382 164.016 1.00 44.76 B
ATOM 6005 CG2 THR B 41 139.605 33.530 164.936 1.00 42.56 B
ATOM 6006 C THR B 41 143.406 33.922 164.793 1.00 43.21 B

ATOM 6007 O THR B 41 144.079 34.955 164.803 1.00 42.60 B
ATOM 6008 N ASP B 42 143.788 32.798 164.192 1.00 42.23 B
ATOM 6009 CA ASP B 42 145.049 32.653 163.474 1.00 40.95 B
ATOM 6010 CB ASP B 42 146.130 32.120 164.418 1.00 41.05 B
ATOM 6011 CG ASP B 42 147.467 31.926 163.730 1.00 40.62 B
ATOM 6012 OD1 ASP B 42 148.387 31.378 164.368 1.00 41.45 B
ATOM 6013 OD2 ASP B 42 147.603 32.323 162.558 1.0040.71 B
ATOM 6014 C ASP B 42 144.807 31.650 162.349 1.00 39.96 B
ATOM 6015 O ASPB 42 144.894 30.444 162.562 1.00 39.27 B
ATOM 6016 N PHE B 43 144.499 32.152 161.157 1.0038.52 B
ATOM 6017 CA PHE B 43 144.229 31.293 160.012 1.00 37.51 B
ATOM 6018 CB PHE B 43 142.828 31.571 159.452 1.0039.01 B
ATOM 6019 CG PHE B 43 141.711 31.179 160.376 1.0042.42 B
ATOM 6020 CD1 PHE B 43 141.357 31.988 161.448 1.00 42.99 B
ATOM 6021 CD2 PHE B 43 141.013 29.990 160.176 1.00 43.85 B
ATOM 6022 CE1 PHE B 43 140.321 31.616 162.309 1.00 43.37 B
ATOM 6023 CE2 PHE B 43 139.980 29.612 161.031 1.00 43.32 B
ATOM 6024 CZ PHE B 43 139.635 30.429 162.100 1.0042.74 B
ATOM 6025 C PHE B 43 145.242 31.455 158.883 1.00 36.10 B
ATOM 6026 O PHE B 43 145.893 32.493 158.758 1.00 36.34 B
ATOM 6027 N PRO B 44 145.388 30.420 158.044 1.00 33.49 B
ATOM 6028 CD PRO B 44 144.833 29.059 158.165 1.00 33.39 B
ATOM 6029 CA PRO B 44 146.331 30.493 156.931 1.0032.99 B
ATOM 6030 CB PRO B 44 146.608 29.028 156.629 1.0033.40 B
ATOM 6031 CG PRO B 44 145.265 28.412 156.863 1.00 32.98 B
ATOM 6032 C PRO B 44 145.664 31.184 155.759 1.00 31.80 B
ATOM 6033 O PRO B 44 144.439 31.179 155.645 1.00 32.96 B
ATOM 6034 N LEU B 45 146.467 31.791 154.898 1.00 29.65 B
ATOM 6035 CA LEU B 45 145.938 32.443 153.716 1.00 27.78 B
ATOM 6036 CB LEU B 45 146.402 33.899 153.636 1.00 27.82 B
ATOM 6037 CG LEU B 45 145.790 34.863 154.657 1.00 29.57 B
ATOM 6038 CD1 LEU B 45 146.567 36.169 154.663 1.00 25.91 B
ATOM 6039 CD2 LEU B 45 144.320 35.097 154.319 1.0026.82 B
ATOM 6040 C LEU B 45 146.470 31.668 152.525 1.00 28.65 B
ATOM 6041 O LEU B 45 147.612 31.206 152.532 1.00 27.90 B
ATOM 6042 N ALA B 46 145.632 31.509 151.509 1.00 27.96 B
ATOM 6043 CA ALA B 46 146.036 30.800 150.309 1.00 29.22 B
ATOM 6044 CB ALAB 46 144.925 29.845 149.870 1.00 28.18 B
ATOM 6045 C ALA B 46 146.325 31.829 149.216 1.00 28.38 B
ATOM 6046 O ALA B 46 145.548 32.761 149.018 1.00 27.76 B
ATOM 6047 N VAL B 47 147.453 31.673 148.528 1.00 29.86 B
ATOM 6048 CA VAL B 47 147.817 32.592 147.452 1.00 31.07 B
ATOM 6049 CB VAL B 47 149.343 32.621 147.214 1.0029.69 B
ATOM 6050 CG1 VALB 47 149.668 33.514 146.017 1.0030.14 B
ATOM 6051 CG2 VAL B 47 150.052 33.131 148.453 1.00 30.40 B
ATOM 6052 C VAL B 47 147.129 32.143 146.166 1.00 33.15 B
ATOM 6053 O VAL B 47 147.437 31.077 145.628 1.00 34.40 B
ATOM 6054 N ARG B 48 146.199 32.957 145.678 1.00 34.08 B
ATOM 6055 CA ARG B 48 145.463 32.631 144.465 1.00 34.87 B
ATOM 6056 CB ARG B 48 144.052 33.222 144.537 1.0036.51 B
ATOM 6057 CG ARG B 48 143.070 32.611 143.547 1.0039.03 B
ATOM 6058 CD ARG B 48 141.663 33.140143.765 1.00 40.73 B
ATOM 6059 NE ARG B 48 141.587 34.577 143.523 1.00 41.77 B
ATOM 6060 CZ ARG B 48 140.486 35.304 143.681 1.00 42.69 B
ATOM 6061 NH1 ARG B 48 140.512 36.608 143.437 1.0041.90 B
ATOM 6062 NH2 ARG B 48 139.360 34.726 144.087 1.00 42.47 B
ATOM 6063 C ARG B 48 146.176 33.132 143.210 1.00 34.91 B
ATOM 6064 O ARG B 48 145.970 32.603 142.119 1.00 35.01 B
ATOM 6065 N GLU B 49 147.018 34.147 143.369 1.00 34.37 B
ATOM 6066 CA GLU B 49 147.760 34.713 142.249 1.0035.58 B
ATOM 6067 CB GLU B 49 146.808 35.461 141.307 1.00 38.88 B
ATOM 6068 CG GLU B 49 145.993 36.519 142.019 1.0044.17 B
ATOM 6069 CD GLU B 49 144.762 36.967 141.252 1.0047.25 B
ATOM 6070 CE1 GLU B 49 144.916 37.655 140.220 1.00 50.21 B
ATOM 6071 OE2 GLU B 49 143.638 36.630 141.690 1.0047.96 B
ATOM 6072 C GLU B 49 148.804 35.671 142.792 1.0035.11 B
ATOM 6073 O GLU B 49 148.650 36.199 143.893 1.0036.08 B
ATOM 6074 N GLU B 50 149.859 35.900 142.018 1.00 33.89 B
ATOM 6075 CA GLU B 50 150.928 36.793 142.436 1.00 33.96 B
ATOM 6076 CB GLU B 50 151.877 36.056 143.388 1.0032.26 B
ATOM 6077 CG GLU B 50 152.546 34.829 142.771 1.00 32.34 B
ATOM 6078 CD GLU B 50 153.418 34.057 143.753 1.00 29.54 B
ATOM 6079 OE1 GLU B 50 152.881 33.302 144.592 1.0028.87 B
ATOM 6080 OE2 GLU B 50 154.650 34.213 143.683 1.00 30.21 B
ATOM 6081 C GLU B 50 151.709 37.320 141.236 1.0035.57 B
ATOM 6082 O GLU B 50 151.993 36.580 140.295 1.00 37.86 B
ATOM 6083 N TYR B 51 152.047 38.604 141.270 1.00 35.41 B
ATOM 6084 CA TYR B 51 152.812 39.225 140.194 1.00 35.18 B
ATOM 6085 CB TYR B 51 151.908 39.558 138.999 1.0034.44 B
ATOM 6086 CG TYR B 51 150.619 40.254 139.356 1.00 36.05 B
ATOM 6087 CD1 TYR B 51 150.580 41.630 139.577 1.00 35.12 B
ATOM 6088 CE1 TYR B 51 149.387 42.269 139.915 1.00 35.76 B
ATOM 6089 CD2 TYR B 51 149.432 39.531 139.484 1.0037.62 B
ATOM 6090 CE2 TYR B 51 148.237 40.159 139.824 1.00 37.34 B
ATOM 6091 CZ TYR B 51 148.221 41.527 140.037 1.0036.83 B
ATOM 6092 OH TYR B 51 147.038 42.146 140.374 1.0038.21 B
ATOM 6093 C TYR B 51 153.496 40.471 140.717 1.00 35.48 B
ATOM 6094 O TYRB 51 153.246 40.894 141.844 1.00 34.34 B
ATOM 6095 N SER B 52 154.358 41.068 139.906 1.00 37.18 B
ATOM 6096 CA SER B 52 155.082 42.244 140.358 1.00 40.01 B
ATOM 6097 CB SER B 52 156.565 42.108 139.999 1.00 40.58 B
ATOM 6098 OG SER B 52 156.746 42.013 138.602 1.00 41.31 B
ATOM 6099 C SER B 52 154.571 43.587 139.864 1.00 41.12 B
ATOM 6100 O SER B 52 154.157 43.731 138.716 1.00 41.99 B
ATOM 6101 N LEU B 53 154.601 44.568 140.759 1.0042.10 B
ATOM 6102 CA LEU B 53 154.196 45.928 140.443 1.00 43.83 B
ATOM 6103 CB LEU B 53 153.400 46.538 141.597 1.00 44.04 B
ATOM 6104 CG LEU B 53 151.887 46.297 141.575 1.00 45.23 B
ATOM 6105 CD1 LEUB 53 151.582 44.855 141.226 1.0045.62 B
ATOM 6106 CD2 LEU B 53 151.301 46.660 142.929 1.00 46.02 B
ATOM 6107 C LEU B 53 155.482 46.710 140.213 1.00 45.98 B
ATOM 6108 O LEU B 53 156.573 46.135 140.232. 1.00 46.25 B
ATOM 6109 N GLU B 54 155.365 48.016 140.005 1.00 46.95 B
ATOM 6110 CA GLU B 54 156.541 48.838 139.747 1.00 48.38 B
ATOM 6111 CB GLU B 54 156.147 50.317 139.723 1.00 52.05 B
ATOM 6112 CG GLU B 54 157.265 51.242 139.268 1.0056.68 B
ATOM 6113 CD GLU B 54 156.810 52.684 139.150 1.0059.28 B
ATOM 6114 OE1 GLU B 54 155.921 52.960 138.312 1.00 59.74 B
ATOM 6115 OE2 GLU B 54 157.339 53.538 139.896 1.0061.23 B
ATOM 6116 C GLU B 54 157.689 48.619 140.739 1.00 46.18 B
ATOM 6117 O GLU B 54 158.831 48.403 140.330 1.00 46.72 B
ATOM 6118 N ALA B 55 157.389 48.670 142.033 1.00 42.01 B
ATOM 6119 CA ALA B 55 158.415 48.483 143.053 1.00 38.23 B
ATOM 6120 CB ALA B 55 158.919 49.841 143.537 1.00 37.53 B
ATOM 6121 C ALA B 55 157.884 47.671 144.231 1.00 36.17 B
ATOM 6122 O ALA B 55 158.399 47.755 145.344 1.00 35.00 B
ATOM 6123 N LYS B 56 156.847 46.885 143.974 1.0033.42 B
ATOM 6124 CA LYS B 56 156.243 46.054 145.002 1.00 30.34 B
ATOM 6125 CB LYS B 56 154.988 46.726 145.563 1.0030.61 B
ATOM 6126 CG LYS B 56 155.232 48.052 146.258 1.00 32.67 B
ATOM 6127 CD LYS B 56 153.927 48.643 146.767 1.0032.47 B
ATOM 6128 CE LYS B 56 154.173 49.908 147.569 1.00 33.37 B
ATOM 6129 NZ LYS B 56 152.903 50.460 148.115 1.0034.89 B
ATOM 6130 C LYS B 56 155.846 44.717 144.404 1.00 29.11 B
ATOM 6131 O LYS B 56 155.518 44.632 143.221 1.00 27.49 B
ATOM 6132 N TYR B 57 155.888 43.668 145.214 1.0026.49 B
ATOM 6133 CA TYR B 57 155.472 42.364 144.739 1.00 25.24 B
ATOM 6134 CB TYR B 57 156.473 41.275 145.117 1.0026.34 B
ATOM 6135 CG TYR B 57 156.256 40.022 144.311 1 1.00 26.18 B
ATOM 6136 CD1 TYR B 57 156.481 40.012 142.933 1.00 27.08 B
ATOM 6137 CE1 TYR B 57 156.229 38.875 142.170 1.0027.37 B
ATOM 6138 CD2 TYR B 57 155.778 38.859 144.909 1.0028.48 B
ATOM 6139 CE2 TYR B 57 155.524 37.714 144.157 1.00 28.92 B
ATOM 6140 CZ TYR B 57 155.751 37.733 142.789 1.0028.49 B
ATOM 6141 OH TYR B 57 155.487 36.611 142.045 1.0031.70 B
ATOM 6142 C TYR B 57 154.130 42.109 145.405 1.00 25.98 B
ATOM 6143 O TYR B 57 153.991 42.222 146.632 1.0024.86 B
ATOM 6144 N LYS B 58 153.147 41.763 144.584 1.00 23.38 B
ATOM 6145 CA LYS B 58 151.788 41.548 145.045 1.00 22.69 B
ATOM 6146 CB LYS B 58 150.853 42.378 144.163 1.0024.19 B
ATOM 6147 CG LYS B 58 149.375 42.104 144.347 1.00 25.81 B
ATOM 6148 CD LYS B 58 148.565 43.022 143.454 1.0027.19 B
ATOM 6149 CE LYS B 58 147.077 42.806 143.640 1.0030.77 B
ATOM 6150 NZ LYS B 58 146.300 43.759 142.797 1.00 34.53 B
ATOM 6151 C LYS B 58 151.291 40.112 145.085 1.0023.23 B
ATOM 6152 O LYS B 58 151.525 39.332 144.159 1.00 24.14 B
ATOM 6153 N TYR B 59 150.593 39.776 146.167 1.00 23.00 B
ATOM 6154 CA TYR B 59 149.990 38.459 146.335 1.00 24.46 B
ATOM 6155 CB TYRB 59 150.584 37.685 147.517 1.00 23.30 B
ATOM 6156 CG TYR B 59 152.057 37.398 147.454 1.00 23.43 B
ATOM 6157 CDI TYR B 59 152.987 38.353 147.855 1.00 23.37 B
ATOM 6158 CE1 TYR B 59 154.351 38.082 147.835 1.00 24.55 B
ATOM 6159 CD2 TYR B 59 152.525 36.154 147.022 1.00 23.74 B
ATOM 6160 CE2 TYR B 59 153.893 35.868 146.995 1.00 23.99 B
ATOM 6161 CZ TYR B 59 154.799 36.838 147.407 1.00 25.04 B
ATOM 6162 OH TYR B 59 156.148 36.573 147.412 1.00 24.22 B
ATOM 6163 C TYR B 59 148.524 38.691 146.669 1.00 25.89 B
ATOM 6164 O TYR B 59 148.228 39.401 147.630 1.00 28.20 B
ATOM 6165 N VAL B 60 147.600 38.128 145.896 1.00 25.35 B
ATOM 6166 CA VAL B 60 146.197 38.294 146.254 1.00 24.92 B
ATOM 6167 CB VAL B 60 145.287 38.571 145.016 1.0025.24 B
ATOM 6168 CG1 VAL B 60 146.126 39.111 143.875 1.00 23.89 B
ATOM 6169 CG2 VAL B 60 144.495 37.343 144.629 1.00 24.86 B
ATOM 6170 C VAL B 60 145.855 36.979 146.942 1.00 24.47 B
ATOM 6171 O VALB 60 145.970 35.910 146.351 1.00 25.14 B
ATOM 6172 N CYS B 61 145.474 37.063 148.211 1.00 24.68 B
ATOM 6173 CA CYS B 61 145.181 35.870 148.990 1.00 26.35 B
ATOM 6174 CB CYS B 61 145.991 35.893 150.280 1.00 24.96 B
ATOM 6175 SG CYS B 61 147.724 36.278 150.041 1.00 26.79 B
ATOM 6176 C CYS B 61 143.718 35.668 149.335 1.00 27.68 B
ATOM 6177 O CYS B 61 142.945 36.621 149.407 1.0026.99 B
ATOM 6178 N VALB 62 143.361 34.407 149.560 1.0028.71 B
ATOM 6179 CA VAL B 62 142.004 34.021 149.907 1.00 28.92 B
ATOM 6180 CB VAL B 62 141.477 32.920 148.970 1.00 28.38 B
ATOM 6181 CG1 VAL B 62 140.159 32.384 149.492 1.00 26.93 B
ATOM 6182 CG2 VAL B 62 141.312 33.466 147.565 1.00 26.12 B
ATOM 6183 C VAL B 62 141.980 33.479 151.323 1.00 31.13 B
ATOM 6184 O VALB 62 142.859 32.713 151.714 1.00 31.24 B
ATOM 6185 N SER B 63 140.972 33.885 152.087 1.00 32.98 B
ATOM 6186 CA SER B 63 140.817 33.427 153.461 1.00 36.00 B
ATOM 6187 CB SER B 63 140.585 34.615 154.398 1.0036.93 B
ATOM 6188 OG SER B 63 139.379 35.293 154.076 1.00 39.45 B
ATOM 6189 C SER B 63 139.620 32.486 153.526 1.0037.06 B
ATOM 6190 O SERB 63 138.647 32.661 152.794 1.0036.27 B
ATOM 6191 N ASPB 64 139.692 31.489 154.399 1.0039.55 B
ATOM 6192 CA ASP B 64 138.600 30.534 154.540 1.00 42.70 B
ATOM 6193 CB ASP B 64 139.040 29.342 155.397 1.00 45.20 B
ATOM 6194 CG ASP B 64 139.993 28.408 154.659 1.0047.56 B
ATOM 6195 ODI ASP B 64 140.648 27.573 155.325 1.0049.11 B
ATOM 6196 OD2 ASP B 64 140.079 28.501 153.413 1.00 47.87 B
ATOM 6197 C ASP B 64 137.414 31.227 155.185 1.00 43.34 B
ATOM 6198 O ASP B 64 136.297 31.174 154.675 1.00 43.59 B
ATOM 6199 N ALAB 65 137.671 31.886 156.308 1.00 43.74 B
ATOM 6200 CA ALA B 65 136.629 32.597 157.028 1.00 43.04 B
ATOM 6201 CB ALA B 65 136.852 32.461 158.531 1.00 42.92 B
ATOM 6202 C ALA B 65 136.634 34.067 156.627 1.00 42.62 B
ATOM 6203 O ALA B 65 137.694 34.670 156.461 1.00 43.46 B
ATOM 6204 N PRO B 66 135.446 34.658 156.441 1.00 41.57 B
ATOM 6205 CD PRO B 66 134.125 34.013 156.344 1.0042.52 B
ATOM 6206 CA PRO B 66 135.364 36.071 156.060 1.00 40.11 B
ATOM 6207 CB PRO B 66 133.862 36.300 155.922 1.0041.24 B
ATOM 6208 CG PRO B 66 133.372 34.967 155.440 1.0042.49 B
ATOM 6209 C PRO B 66 135.984 36.946 157.145 1.00 37.78 B
ATOM 6210 O PRO B 66 135.964 36.593 158.322 1.0038.59 B
ATOM 6211 N VAL B 67 136.537 38.085156.750 1.00 35.74 B
ATOM 6212 CA VAL B 67 137.157 38.987 157.711 1.00 33.80 B
ATOM 6213 CB VALB 67 137.854 40.160 156.992 1.0034.70 B
ATOM 6214 CG1 VAL B 67 138.434 41.129 158.014 1.00 34.12 B
ATOM 6215 CG2 VAL B 67 138.948 39.628 156.079 1.00 32.85 B
ATOM 6216 C VALB 67 136.124 39.549 158.685 1.00 32.89 B
ATOM 6217 O VAL B 67 135.006 39.877 158.297 1.00 32.24 B
ATOM 6218 N THR B 68 136.498 39.652 159.954 1.00 31.69 B
ATOM 6219 CA THR B 68 135.588 40.192 160.956 1.00 31.68 B
ATOM 6220 CB THR B 68 135.691 39.426 162.286 1.00 31.34 B
ATOM 6221 OG 1 THR B 68 135.535 38.024 162.044 1.00 32.63 B
ATOM 6222 CG2 THR B 68 134.595 39.879 163.239 1.00 32.68 B
ATOM 6223 C THR B 68 135.917 41.662 161.209 1.00 30.22 B
ATOM 6224 O THR B 68 136.811 41.986 161.991 1.00 31.10 B
ATOM 6225 N PHE B 69 135.189 42.548 160.543 1.0027.63 B
ATOM 6226 CA PHE B 69 135.418 43.975 160.693 1.00 24.87 B
ATOM 6227 CB PHE B 69 134.587 44.748 159.670 1.0023.57 B
ATOM 6228 CG PHE B 69 134.960 44.451 158.249 1.0022.90 B
ATOM 6229 CD1 PHE B 69 134.402 43.365 157.579 1.00 24.00 B
ATOM 6230 CD2 PHE B 69 135.894 45.238 157.586 1.00 23.94 B
ATOM 6231 CE1 PHE B 69 134.772 43.067 156.264 1.0022.85 B
ATOM 6232 CE2 PHE B 69 136.274 44.948 156.268 1.00 24.77 B
ATOM 6233 CZ PHE B 69 135.710 43.860 155.609 1.00 22.51 B
ATOM 6234 C PHE B 69 135.115 44.459 162.099 1.00 24.48 B
ATOM 6235 O PHE B 69 134.115 44.067 162.700 1.00 24.15 B
ATOM 6236 N GLY B 70 135.9.91 45.316 162.617 1.00 24.19 B
ATOM 6237 CA GLY B 70 135.825 45.838 163.959 1.00 23.12 B
ATOM 6238 C GLY B 70 137.005 45.442 164.826 1.0024.25 B
ATOM 6239 0 GLY B 70 137.331 46.110 165.812 1.0023.68 B
ATOM 6240 N LYS B 71 137.651 44.343 164.459 1.0023.83 B
ATOM 6241 CA LYS B 71 138.804 43.860 165.202 1.00 25.68 B
ATOM 6242 CB LYS B 71 138.671 42.356 165.471 1.00 28.69 B
ATOM 6243 CG LYS B 71 137.515 41.986 166.398 1.00 33.36 B
ATOM 6244 CD LYS B 71 137.743 42.539 167.799 1.0036.97 B
ATOM 6245 CE LYS B 71 136.505 42.397 168.682 1.0039.77 B
ATOM 6246 NZ LYS B 71 136.076 40.979 168.835 1.00 42.21 B
ATOM 6247 C LYS B 71 140.056 44.133 164.392 1.00 25.22 B
ATOM 6248 O LYS B 71 1 140.039 44.059 163.167 1.00 24.54 B
ATOM 6249 N ILE B 72 141.143 44.470 165.073 1.00 25.56 B
ATOM 6250 CA ILE B 72 142.393 44.729 164.382 1.00 25.86 B
ATOM 6251 CB ILE B 72 143.433 45.354 165.334 1.00 27.03 B
ATOM 6252 CG2 ILE B 72 144.793 45.434 164.649 1.00 25.87 B
ATOM 6253 CG1 ILE B 72 142.962 46.749 165.761 1.00 27.27 B
ATOM 6254 CD1 ILE B 72 143.769 47.352 166.895 1.00 29.50 B
ATOM 6255 C ILE B 72 142.911 43.398 163.848 1.00 27.06 B
ATOM 6256 O ILE B 72 142.938 42.396 164.566 1.00 25.67 B
ATOM 6257 N HIS B 73 143.300 43.385 162.580 1.00 26.56 B
ATOM 6258 CA HIS B 73 143.816 42.175 161.959 1.00 27.81 B
ATOM 6259 CB HIS B 73 143.027 41.841 160.687 1.0029.69 B
ATOM 6260 CG HIS B 73 141.605 41.437 160.935 1.00 32.13 B
ATOM 6261 CD2 HIS B 73 140.480 42.178 161.077 1.00 31.69 B
ATOM 6262 NDI HIS B 73 141.213 40.120 161.055 1.00 32.87 B
ATOM 6263 CE1 HIS B 73 139.908 40.068 161.256 1.0033.04 B
ATOM 6264 NE2 HIS B 73 139.440 41.304 161.275 1.00 33.79 B
ATOM 6265 C HIS B 73 145.279 42.370 161.588 1.00 29.45 B
ATOM 6266 0 HIS B 73 145.685 43.443 161.123 1.00 27.93 B
ATOM 6267 N CYS B 74 146.071 41.32.7 161.798 1.00 29.05 B
ATOM 6268 CA CYS B 74 147.479 41.381 161.453 1.00 29.37 B
ATOM 6269 CB CYS B 74 148.352 41.230 162.707 1.00 29.30 B
ATOM 6270 SG CYS B 74 148.535 42.761 163.683 1.00 36.10 B
ATOM 6271 C CYS B 74 147.795 40.282 160.449 1.00 28.24 B
ATOM 6272 0 CYS B 74 147.313 39.156 160.569 1.00 25.92 B
ATOM 6273 N VAL B 75 148.574 40.628 159.433 1.00 26.69 B
ATOM 6274 CA VAL B 75 148.975 39.653 158.433 1.00 28.13 B
ATOM 6275 CB VAL B 75 148.815 40.201 157.001 1.00 27.69 B
ATOM 6276 CG1 VAL B 75 149.382 39.205 155.995 1.00 26.75 B
ATOM 6277 CG2 VAL B 75 147.342 40.456 156.713 1.00 26.82 B
ATOM 6278 C VALB 75 150.437 39.329 158.708 1.00 27.98 B
ATOM 6279 O VAL B 75 151.286 40.218 158.757 1.00 28.33 B
ATOM 6280 N ARG B 76 150.719 38.048 158.907 1.0028.49 B
ATOM 6281 CA ARG B 76 152.070 37.600 159.210 1.00 28.52 B
ATOM 6282 CB ARG B 76 152.035 36.661 160.420 I .00 28.51 B
ATOM 6283 CG ARG B 76 153.383 36.064 160.818 1.0028.95 B
ATOM 6284 CD ARG B 76 153.197 35.013 161.902 1.0029.51 B
ATOM 6285 NE ARG B 76 154.463 34.528 162.452 1.0031.72 B
ATOM 6286 CZ ARG B 76 155.335 33.772 161.794 1.0031.22 B
ATOM 6287 NH1 ARG B 76 155.085 33.401 160.548 1.00 30.67 B
ATOM 6288 NH2 ARG B 76 156.464 33.391 162.383 1.00 29.83 B
ATOM 6289 C ARG B 76 152.719 36.884 158.035 1.00 27.29 B
ATOM 6290 O ARG B 76 152.126 35.981 157.445 1.0028.55 B
ATOM 6291 N ALA B 77 153.932 37.302 157.689 1.00 26.18 B
ATOM 6292 CA ALA B 77 154.683 36.658 156.614 1.00 25.88 B
ATOM 6293 CB ALA B 77 155.620 37.658 155.951 1.00 23.94 B
ATOM 6294 C ALA B 77 155.481 35.538 157.293 1.0025.07 B
ATOM 6295 O ALA B 77 155.645 35.554 158.514 1.0023.27 B
ATOM 6296 N SER B 78 155.972 34.573 156.520 1.00 25.41 B
ATOM 6297 CA SER B 78 156.725 33.459 157.102 1.00 24.51 B
ATOM 6298 CB SER B 78 157.011 32.387 156.035 1.00 25.98 B
ATOM 6299 OG SER B 78 157.874 32.862 155.012 1.0027.40 B
ATOM 6300 C SER B 78 158.035 33.899 157.758 1.00 23.53 B
ATOM 6301 O SERB 78 158.607 33.172 158.568 1.00 22.18 B
ATOM 6302 N SER B 79 158.504 35.094157.410 1.00 23.31 B
ATOM 6303 CA SER B 79 159.748 35.627 157.960 1.00 21.64 B
ATOM 6304 CB SER B 79 160.266 36.752 157.075 1.0021.45 B
ATOM 6305 OG SER B 79 159.300 37.787 156.996 1.00 23.31 B
ATOM 6306 C SER B 79 159.547 36.170 159.363 1.00 21.22 B
ATOM 6307 O SER B 79 160.510 36.528 160.037 1.00 22.62 B
ATOM 6308 N GLY B 80 158.293 36.250 159.792 1.0020.78 B
ATOM 6309 CA GLY B 80 158.001 36.772 161.115 1.00 20.59 B
ATOM 6310 C GLYB 80 157.473 38.199 161.094 1.0021.90 B
ATOM 6311 O GLYB 80 156.840 38.635 162.055 1.00 21.37 B
ATOM 6312 N HIS B 81 157.724 38.937 160.014 1.00 21.40 B
ATOM 6313 CA HIS B 81 157.242 40.315 159.929 1.00 22.76 B
ATOM 6314 CB HIS B 81 157.781 41.019 158.682 1.0022.99 B
ATOM 6315 CG HIS B 81 159.273 41.082158.616 1.0023.95 B
ATOM 6316 CD2 HIS B 81 160.145 42.020 159.055 1.00 24.35 B
ATOM 6317 NDI HIS B 81 160.036 40.087 158.043 1.00 23.10 B
ATOM 6318 CE1 HIS B 81 161.314 40.411 158.129 1.0023.90 B
ATOM 6319 NE2 HIS B 81 161.407 41.579 158.739 1.00 23.91 B
ATOM 6320 C HIS B 81 155.722 40.348 159.889 1.00 23.90 B
ATOM 6321 O HIS B 81 155.083 39.400 159.423 1.00 25.88 B
ATOM 6322 N LYS B 82 155.145 41.444 160.373 1.00 24.04 B
ATOM 6323 CA LYS B 82 153.697 41.594 160.391 1.00 23.63 B
ATOM 6324 CB LYS B 82 153.139 41.265 161.778 1.0023.78 B
ATOM 6325 CG LYS B 82 153.433 39.860 162.259 1.0024.64 B
ATOM 6326 CD LYS B 82 152.772 39.609 163.598 1.00 26.15 B
ATOM 6327 CE LYS B 82 153.015 38.190 164.072 1.00 25.55 B
ATOM 6328 NZ LYS B 82 152.200 37.900 165.275 1.00 24.98 B
ATOM 6329 C LYS B 82 153.261 43.000 160.018 1.0024.20 B
ATOM 6330 0 LYS B 82 154.052 43.942 160.049 1.0022.79 B
ATOM 6331 N THRB 83 151.989 43.131 159.662 1.00 23.28 B
ATOM 6332 CA THR B 83 151.438 44.425 159.316 1.00 24.86 B
ATOM 6333 CB THR B 83 151.606 44.741 157.814 1.00 25.76 B
ATOM 6334 OG1 THR B 83 151.303 46.121 157.587 1.00 24.86 B
ATOM 6335 CG2 THR B 83 150.671 43.886 156.971 1.00 26.72 B
ATOM 6336 C THR B 83 149.961 44.438 159.666 1.0024.39 B
ATOM 6337 O THR B 83 149.335 43.385 159.806 1.00 24.99 B
ATOM 6338 N ASP B 84 149.413 45.634 159.828 1.00 23.11 B
ATOM 6339 CA ASP B 84 148.006 45.774 160.154 1.00 21.96 B
ATOM 6340 CB ASP B 84 147.768 47.077 160.928 1.00 20.98 B
ATOM 6341 CG ASP B 84 148.231 48.304 160.167 1.0021.81 B
ATOM 6342 OD1 ASP B 84 147.371 49.118 159.765 1.00 25.04 B
ATOM 6343 OD2 ASP B 84 149.453 48.460 159.967 1.00 22.14 B
ATOM 6344 C ASP B 84 147.191 45.760 158.864 1.00 20.79 B
ATOM 6345 O ASPB 84 147.570 46.387 157.870 1.00 20.29 B
ATOM 6346 N LEU B 85 146.082 45.027 158.892 1.00 21.51 B
ATOM 6347 CA LEU B 85 145.188 44.900 157.746 1.00 20.82 B
ATOM 6348 CB LEU B 85 144.131 43.832 158.023 1.0019.58 B
ATOM 6349 CG LEU B 85 143.623 42.929 156.894 1.0023.09 B
ATOM 6350 CD1 LEU B 85 142.180 42.552 157.209 1.00 19.71 B
ATOM 6351 CD2 LEU B 85 143.704 43.614 155.539 1.00 22.19 B
ATOM 6352 C LEU B 85 144.483 46.226 157.480 1.00 21.68 B
ATOM 6353 O LEU B 85 143.656 46.674 158.281 1.00 22.07 B
ATOM 6354 N GLN B 86 144.816 46.849 156.358 1.00 19.87 B
ATOM 6355 CA GLN B 86 144.206 48.111 155.975 1.00 20.75 B
ATOM 6356 CB GLN B 86 145.219 48.973 155.216 1.0018.15 B
ATOM 6357 CG GLN B 86 146.257 49.611 156.118 1.00 16.98 B
ATOM 6358 CD GLN B 86 147.305 50.396 55.357 1.0020.92 B
ATOM 6359 OE1 GLN B 86 148.451 49.956 155.220 1.00 21.72 B
ATOM 6360 NE2 GLN B 86 146.920 51.564154.849 1.00 21.58 B
ATOM 6361 C GLN B 86 142.957 47.877 155.122 1.00 21.82 B
ATOM 6362 O GLN B 86 142.710 46.767 154.660 1.00 23.26 B
ATOM 6363 N ILE B 87 142.177 48.933 154.916 1.00 22.52 B
ATOM 6364 CA ILE B 87 140.947 48.854 154.143 1.00 22.23 B
ATOM 6365 CB ILE B 87 140.005 50.009 154.512 1.00 23.34 B
ATOM 6366 CG2 ILE B 87 138.689 49.864 153.755 1.00 23.04 B
ATOM 6367 CG1 ILE B 87 139.765 50.011 156.028 1.00 23.45 B
ATOM 6368 CD 1 ILE B 87 138.974 51.204 156.541 1.00 23.53 B
ATOM 6369 C ILE B 87 141.192 48.890 152.639 1.00 23.38 B
ATOM 6370 O ILE B 87 141.860 49.788 152.133 1.00 23.37 B
ATOM 6371 N GLY B 88 140.643 47.910 151.929 1.00 22.74 B
ATOM 6372 CA GLY B 88 140.810 47.865 150.489 1.00 22.15 B
ATOM 6373 C GLY B 88 139.522 48.213 149.770 1.00 22.94 B
ATOM 6374 O GLYB 88 138.726 49.014 150.266 1.00 23.06 B
ATOM 6375 N ALA B 89 139.307 47.598 148.608 1.0021.07 B
ATOM 6376 CA ALA B 89 138.116 47.849 147.803 1.00 21.23 B
ATOM 6377 CB ALA B 89 138.376 47.432 146.362 1.00 19.33 B
ATOM 6378 C ALAB 89 136.861 47.150 148.319 1.0022.02 B
ATOM 6379 0 ALA B 89 135.773 47.350 47.782 1.0023.27 B
ATOM 6380 N VAL B 90 137.011 46.345 149.366 1.0022.02 B
ATOM 6381 CA VAL B 90 135.891 45.601 149.933 1.00 20.61 B
ATOM 6382 CB VAL B 90 136.325 44.824 151.200 1.00 19.64 B
ATOM 6383 CG1 VAL B 90 136.606 45.801 152.337 1.00 18.34 B
ATOM 6384 CG2 VAL B 90 135.240 43.821 151.602 1.00 14.43 B
ATOM 6385 C VAL B 90 134.665 46.441 150.297 1.00 21.83 B
ATOM 6386 O VAL B 90 133.529 45.959 150.202 1.00 20.46 B
ATOM 6387 N ILE B 91 134.883 47.686 150.714 I.00 21.26 B
ATOM 6388 CA ILE B 91 133.764 48.538 151.113 1.00 23.49 B
ATOM 6389 CB ILE B 91 134.258 49.846 151.824 1.00 24.15 B
ATOM 6390 CG2 ILE B 91 134.683 49.528 153.242 1.00 20.46 B
ATOM 6391 CGI ILE B 91 135.438 50.471 151.075 1.00 24.75 B
ATOM 6392 CD 1 ILE B 91 135.063 1.238 149.860 1.00 28.22 B
ATOM 6393 C ILE B 91 132.803 48.896 149.983 1.00 24.59 B
ATOM 6394 O ILE B 91 131.691 49.356 150.236 1.00 25.65 B
ATOM 6395 N ARG B 92 133.217 48.665 148.742 1.00 23.83 B
ATOM 6396 CA ARG B 92 132.370 48.976 147.599 1.00 24.99 B
ATOM 6397 CB ARG B 92 133.206 49.632 146.497 1.0024.50 B
ATOM 6398 CG ARG B 92 133.676 51.038 146.857 z B
ATOM 6399 CD ARG B 92 134.286 51.761 145.659 1.00 26.62 B
ATOM 6400 NE ARG B 92 135.635 51.295 145.364 1.0026.60 B
ATOM 6401 CZ ARG B 92 136.701 51.569 146.112 1.00 28.10 B
ATOM 6402 NH1 ARG B 92 137.893 51.093 145.766 1.00 2b.68 B
ATOM 6403 NH2 ARG B 92 136.578 52.329 147.198 1.00 26.40 B
ATOM 6404 C ARG B 92 131.618 47.770 147.031 1.00 25.03 B
ATOM 6405 O ARG B 92 130.836 47.915 146.088 1.00 23.39 B
ATOM 6406 N THR B 93 131.841 46.588 147.607 1.00 24.22 B
ATOM 6407 CA THR B 93 131.175 45.377 147.127 1.00 22.78 B
ATOM 6408 CB THR B 93 131.926 44.084 147.538 1.00 22.80 B
ATOM 6409 OG1 THR B 93 131.813 43.887 148.954 1.0021.45 B
ATOM 6410 CG2 THR B 93 133.383 44.167 147.146 1.00 21.62 B
ATOM 6411 C THR B 93 129.750 45.252 147.647 1.00 22.24 B
ATOM 6412 O THR B 93 129.412 45.763 148.714 1.0021.42 B
ATOM 6413 N ALAB 94 128.921 44.557 146.877 1.0021.95 B
ATOM 6414 CA ALA B 94 127.532 44.336 147.245 1.00 21.52 B
ATOM 6415 CB ALA B 94 126.806 43.598 146.118 1.00 21.15 B
ATOM 6416 C ALA B 94 127.460 43.533 148.536 1.0020.02 B
ATOM 6417 O ALAB 94 126.614 43.794 149.390 1.0022.90 B
ATOM 6418 N ALAB 95 128.357 42.563 148.683 1.00 I 9.10 B
ATOM 6419 CA ALA B 95 128.388 41.719 149.881 1.00 18.90 B
ATOM 6420 CB ALA B 95 129.451 40.633 149.727 1.00 16.32 B
ATOM 6421 C ALA B 95 128.647 42.528 151.154 1.00 19.57 B
ATOM 6422 O ALA B 95 128.004 42.315 152.182 1.00 19.36 B
ATOM 6423 N PHE B 96 129.608 43.443 151.089 1.0020.61 B
ATOM 6424 CA PHE B 96 129.923 44.288 152.235 1.00 21.08 B
ATOM 6425 CB PHE B 96 131.101 45.204 151.895 1.00 22.89 B
ATOM 6426 CG PHE B 96 131.528 46.094 153.026 1.0025.73 B
ATOM 6427 CD1 PHE B 96 132.418 45.636 153.994 1.00 25.14 B
ATOM 6428 CD2 PHE B 96 131.041 47.397 153.124 1.0024.09 B
ATOM 6429 CE1 PHE B 96 132.819 46.462 155.043 1.00 25. S 3 B
ATOM 6430 CE2 PHE B 96 131.435 48.231 154.170 1.00 24.15 B
ATOM 6431 CZ PHE B 96 132.326 47.764 155.131 1.00 24.79 B
ATOM 6432 C PHE B 96 128.682 45.133 152.548 1.0021.49 B
ATOM 6433 O PHE B 96 128.265 45.247 153.703 1.00 19.90 B
ATOM 6434 N ASP B 97 128.096 45.713 151.502 1.00 20.09 B
ATOM 6435 CA ASP B 97 126.905 46.553 151.627 1.00 21.06 B
ATOM 6436 CB ASP B 97 126.507 47.078 150.239 1.00 20.18 B
ATOM 6437 CG ASP B 97 125.968 48.507 150.273 1.0019.02 B
ATOM 6438 OD 1 ASP B 97 126.158 49.209 151.286 1.00 19.13 B
ATOM 6439 OD2 ASP B 97 125.363 48.937 149.270 1.00 20.12 B
ATOM 6440 C ASP B 97 125.731 45.791 152.264 1.00 22.02 B
ATOM 6441 O ASP B 97 125.064 46.300 153.168 1.00 21.77 B
ATOM 6442 N ASP B 98 125.485 44.568 151.801 1.00 23.94 B
ATOM 6443 CA ASP B 98 124.388 43.764 152.337 1.00 25.72 B
ATOM 6444 CB ASP B 98 124.145 42.543 151.448 1.00 28.94 B
ATOM 6445 CG ASP B 98 123.600 42.922 150.077 1.0034.20 B
ATOM 6446 OD1 ASP B 98 123.411 42.014 149.239 1.00 36.79 B
ATOM 6447 OD2 ASP B 98 123.359 44.129 149.841 1.00 34.86 B
ATOM 6448 C ASP B 98 124.652 43.319 153.770 1.00 25.12 B
ATOM 6449 O ASP B 98 123.734 43.219 154.584 1.00 23.36 B
ATOM 6450 N GLU B 99 125.918 43.065 154.069 1.00 25.04 B
ATOM 6451 CA GLU B 99 126.329 42.638 155.397 1.00 26.74 B
ATOM 6452 CB GLU B 99 127.791 42.174 155.352 1.0030.72 B
ATOM 6453 CG GLU B 99 128.506 42.140 156.701 1.0036.83 B
ATOM 6454 CD GLU B 99 128.062 40.995 157.590 1.0040.49 B
ATOM 6455 OEI GLU B 99 128.467 40.982 158.775 1.00 44.51 B
ATOM 6456 OE2 GLU B 99 127.319 40.108 157.111 1.00 41.39 B
ATOM 6457 C GLU B 99 126.176 43.747 156.441 1.00 25.55 B
ATOM 6458 0 GLU B 99 125.747 43.492 157.568 1.00 24.91 B
ATOM 6459 N PHE B 100 126.503 44.978 156.065 1.00 22.43 B
ATOM 6460 CA PHE B 100 126.441 46.067 157.024 1.00 22.70 B
ATOM 6461 CB PHE B 100 127.787 46.797 157.055 1.00 22.43 B
ATOM 6462 CG PHE B 100 128.933 45.920 157.466 1.00 21.36 B
ATOM 6463 CD1 PHE B 100 129.823 45.425 156.520 1.0021.10 B
ATOM 6464 CD2 PHE B 100 129.093 45.546 158.796 1.00 20.20 B
ATOM 6465 CE1 PHE B 100 130.857 44.565 156.892 1.00 20.79 B
ATOM 6466 CE2 PHE B 100 130.123 44.686 159.176 1.00 20.83 B
ATOM 6467 CZ PHE B 100 131.006 44.195 158.217 1.00 17.82 B
ATOM 6468 C PHE B 100 125.322 47.094 156.924 1.00 23.01 B
ATOM 6469 O PHE B 100 125.298 48.041 157.709 1.00 21.82 B
ATOM 6470 N TYR B 101 124.391 46.928 155.991 1.0022.93 B
ATOM 6471 CA TYR B 101 1 123.317 47.908 155.896 1.00 22.20 B
ATOM 6472 CB TYR B 101 122.291 47.529 154.827 1.00 21.86 B
ATOM 6473 CG TYR B 101 121.097 48.457 154.861 1.00 21.36 B
ATOM 6474 CD1 TYR B 101 121.192 49.762 154.377 1.00 20.98 B
ATOM 6475 CE1 TYR B 101 120.131 50.663 154.513 1.04 20.82 B
ATOM 6476 CD2 TYR B 101 119.904 48.069 155.479 1.00 21.03 B
ATOM 6477 CE2 TYR B 101 118.839 48.963 155.622 1.00 19.96 B
ATOM 6478 CZ TYR B 101 118.964 50.257 155.138 1.00 20.77 B
ATOM 6479 OH TYR B 101 117.934 51.153 155.298 1.00 20.61 B
ATOM 6480 C TYR B 101 122.590 48.065 157.230 1.00 21.89 B
ATOM 6481 O TYR B 101 122.106 47.088 157.806 1.00 22.63 B
ATOM 6482 N TYR B 102 122.506 49.301 157.710 1.00 20.23 B
ATOM 6483 CA TYR B 102 121.832 49.591 158.970 1.00 21.41 B
ATOM 6484 CB TYR B 102 122.764 50.367 159.900 1.00 18.92 B
ATOM 6485 CG TYR B 102 122.109 50.804 161.189 1.0019.37 B
ATOM 6486 CD1 TYR B 102 121.692 49.872 162.142 1.00 17.79 B
ATOM 6487 CEI TYR B 102 121.088 50.283 163.333 1.00 16.36 B
ATOM 6488 CD2 TYR B 102 121.905 52.157 161.457 1.0018.95 B
ATOM 6489 CE2 TYR B 102 121.307 52.575 162.637 1.00 19.03 B
ATOM 6490 CZ TYR B 102 120.900 51.637 163.571 1.00 18.12 B
ATOM 6491 OH TYR B 102 120.305 52.077 164.733 1.00 18.64 B
ATOM 6492 C TYR B 102 120.569 50.402 158.699 1.00 22.66 B
ATOM 6493 0 TYR B 102 120.631 51.502 158.149 1.00 23.38 B
ATOM 6494 N ASP B 103 119.426 49.860 159.101 1.00 23.98 B
ATOM 6495 CA ASP B 103 118.143 50.514 158.866 1.00 25.98 B
ATOM 6496 CB ASP B 103 117.072 49.445 158.619 1.00 28.79 B
ATOM 6497 CG ASP B 103 115.857 49.991 157.889 1.00 32.69 B
ATOM 6498 OD1 ASP B 103 116.002 50.449 156.733 1.00 33.80 B
ATOM 6499 OD2 ASP B 103 114.753 49.961 158.469 1.00 37.06 B
ATOM 6500 C ASP B 103 117.678 51.463 159.974 1.00 25.35 B
ATOM 6501 O ASP B 103 116.668 52.149 159.812 1.00 26.47 B
ATOM 6502 N GLY B 104 118.414 51.514 161.083 1.00 22.89 B
ATOM 6503 CA GLY B 104 118.035 52.381 162.194 1.00 23.39 B
ATOM 6504 C GLY B 104 118.424 53.850 162.075 1.00 23.70 B
ATOM 6505 0 GLY B 104 118.978 54.282 161.059 1.00 23.46 B
ATOM 6506 N GLU B 105 118.131 54.633 163.110 1.00 22.54 B
ATOM 6507 CA GLU B 105 118.463 56.051 163.069 1.00 24.01. B
ATOM 6508 CB GLU B 105 117.496 56.870 163.928 1.00 25.19 B
ATOM 6509 CG GLU B 105 117.465 56.483 165.382 1.00 31.77 B
ATOM 6510 CD GLU B 105 116.729 57.505 166.229 1.00 35.58 B
ATOM 6511 OE1 GLU B 105 116.525 57.237 167.433 1.00 40.04 B
ATOM 6512 OE2 GLU B 105 116.363 58.579 165.696 1.00 35.94 B
ATOM 6513 C GLU B 105 119.895 56.318 163.508 1.00 22.71 B
ATOM 6514 O GLU B 105 120.398 55.711 164.455 1.00 22.27 B
ATOM 6515 N LEU B 106 120.543 57.240 162.806 1.00 22.20 B
ATOM 6516 CA LEU B 106 121.926 57.602 163.079 1.00 20.88 B
ATOM 6517 CB LEU B 106 122.756 57.423 161.812 1.00 21.94 B
ATOM 6518 CG LEU B 106 122.800 56.001 161.244 1.00 22.13 B
ATOM 6519 CD1 LEU B 106 123.369 56.012 159.832 1.00 20.10 B
ATOM 6520 CD2 LEU B 106 123.637 55.134 162.157 1.00 21.11 B
ATOM 6521 C LEU B 106 122.043 59.039 163.562 1.00 20.47 B
ATOM 6522 O LEU B 106 121.111 59.836 163.410 1.00 19.79 B
ATOM 6523 N GLY B 107 123.195 59.364 164.139 1.00 19.03 B
ATOM 6524 CA GLY B 107 123.427 60.708 164.630 1.00 18.39 B
ATOM 6525 C GLY B 107 123.283 60.797 166.135 1.00 20.03 B
ATOM 6526 O GLY B 107 123.486 59.815 166.850 1.00 20.32 B
ATOM 6527 N ALA B 108 122.935 61.982 166.619 1.00 19.18 B
ATOM 6528 CA ALA B 108 122.768 62.201 168.045 1.00 20.81 B
ATOM 6529 CB ALA B 108 123.446 63.506 168.452 1.00 17.52 B
ATOM 6530 C ALA B 108 121.287 62.246 168.388 1.00 20.84 B
ATOM 6531 O ALA B 108 120.576 63.160 167.983 1.00 22.95 B
ATOM 6532 N VAL B 109 120.823 61.245 169.125 1.00 21.86 B
ATOM 6533 CA VAL B 109 119.426 61.183 169.529 1.00 24.58 B
ATOM 6534 CB VAL B 109 118.925 59.735 169.533 1.0026.77 B
ATOM 6535 CG VAL B 109 117.413 59.714 169.702 1.00 27.65 B
ATOM 6536 CG2 VAL B 109 119.339 59.042 168.234 1.00 24.94 B
ATOM 6537 C VAL B 109 119.309 61.774 170.932 1.00 25.69 B
ATOM 6538 0 VAL B 109 119.778 61.185 171.902 1.00 25.78 B
ATOM 6539 N TYR B 110 118.673 62.938 171.027 1.00 26.55 B
ATOM 6540 CA TYR B 110 118.533 63.646 172.292 1.00 27.75 B
ATOM 6541 CB TYR B 110 118.590 65.149 172.037 1.0028.52 B
ATOM 6542 CG TYR B 110 1-18.442 65.996 173.282 1.00 27.56 B
ATOM 6543 CD1 TYR B 110 119.546 66.300 174.078 1.00 26.00 B
ATOM 6544 CE1 TYR B 110 119.414 67.090 175.217 1.0027.82 B
ATOM 6545 CD2 TYR B 110 117.195 66.498 173.660 1.0028.44 B
ATOM 6546 CE2 TYR B 110 117.051 67.283 174.802 1.00 25.73 B
ATOM 6547 CZ TYR B 110 118.162 67.579 175.573 1.00 27.50 B
ATOM 6548 OH TYR B 110 118.028 68.374 176.687 1.00 29.53 B
ATOM 6549 C TYR B 110 117.315 63.365 173.166 1.00 29.21 B
ATOM 6550 O TYR B 110 116.206 63.150 172.686 1.00 29.12 B
ATOM 6551 N THR B 111 117.569 63.408 174.469 1.00 30.87 B
ATOM 6552 CA THR B 111 116.584 63.226 175.533 1.0031.75 B
ATOM 6553 CB THR B 111 116.549 61.767 176.051 1.00 33.04 B
ATOM 6554 OG1 THR B 111 115.626 60.998 175.271 1.00 31.10 B
ATOM 6555 CG2 THR B 111 I 116.132 61.724 177.506 1.00 34.63 B
ATOM 6556 C THR B 111 117.093 64.137 176.650 1.00 32.05 B
ATOM 6557 O THR B 111 118.302 64.321 I 76.804 1.00 30.31 B
ATOM 6558 N ALA B 112 116.184 64.723 177.418 1.00 33.66 B
ATOM 6559 CA ALA B 112 116.593 65.609 .178.500 1.00 34.23 B
ATOM 6560 CB ALA B 112 115.368 66.142 179.222 1.00 35.79 B
ATOM 6561 C ALA B 112 117.499 64.865 179.479 1.0034.14 B
ATOM 6562 O ALA B 112 118.406 65.449 180.067 1.00 33.40 B
ATOM 6563 N ASP B 113 117.248 63.568 179.624 1.00 34.75 B
ATOM 6564 CA ASP B 113 117.998 62.702 180.532 1.00 35.75 B
ATOM 6565 CB ASP B 113 117.156 61.457 180.849 1.0040.34 B
ATOM 6566 CG ASP B 113 117.852 60.496 181.801 1.00 44.25 B
ATOM 6567 OD1 ASP B 113 117.475 59.304 181.821 1.00 48.45 B
ATOM 6568 OD2 ASP B 113 118.766 60.928 182.535 1.00 47.09 B
ATOM 6569 C ASP B 113 119.352 62.255 179.979 1.00 34.67 B
ATOM 6570 O ASP B 113 120.354 62.215 180.700 1.0032.99 B
ATOM 6571 N HIS B 114 119.374 61.912 178.697 1.00 32.63 B
ATOM 6572 CA HIS B 114 120.596 61.434 178.065 1.00 31.09 B
ATOM 6573 CB HIS B 114 120.780 59.946 178.366 1.00 28.99 B
ATOM 6574 CG HIS B 114 119.645 59.101 177.880 1.0029.48 B
ATOM 6575 CD2 HIS B 114 I 19.451 58.463 176.701 1.00 29.71 B
ATOM 6576 ND1 HIS B 114 118.496 58.904 178.615 1.00 31.77 B
ATOM 6577 CE1 HIS B 114 117.642 58.183 177.908 1.00 31.27 B
ATOM 6578 NE2 HIS B 114 118.197 57.904 176.743 1.00 30.24 B
ATOM 6579 C HIS B 114 120.550 61.610 176.551 1.00 29.09 B
ATOM 6580 O HIS B 114 119.512 61.950 175.981 1.00 28.02 B
ATOM 6581 N THR B 11 121.691 61.374 175.909 1.00 26.40 B
ATOM 6582 CA THR B 115 121.790 61.460 174.460 1.00 23.98 B
ATOM 6583 CB THR B 115 122.604 62.688 173.992 1.00 21.90 B
ATOM 6584 OG 1 THR B 115 121.910 63.892 174.334 1.00 22.07 B
ATOM 6585 CG2 THR B 115 122.798 62.653 172.481 1.00 22.57 B
ATOM 6586 C THR B 115 122.505 60.206 173.996 1.00 23.13 B
ATOM 6587 O THR B1 15 123.497 59.793 174.596 1.00 22.51 B
ATOM 6588 N VAL B 116 121.990 59.596 172.938 1.00 22.10 B
ATOM 6589 CA VAL B 116 122.588 58.396 172.372 1.00 20.72 B
ATOM 6590 CB VALB 116 121.510 57.339 172.036 1.00 20.41 B
ATOM 6591 CG1 VAL B 116 122.157 56.087 171.477 1.00 17.26 B
ATOM 6592 CG2 VAL B 116 120.700 57.013 173.281 1.00 20.01 B
ATOM 6593 C VAL B 116 123.290 58.803 171.082 1.00 21.73 B
ATOM 6594 O VAL B 116 122.693 59.465 170.231 1.00 20.95 B
ATOM 6595 N PHE B 117 124.558 58.429 170.941 1.00 20.30 B
ATOM 6596 CA PHEB 117 125.296 58.760 169.730 1.00 19.04 B
ATOM 6597 CB PHE B 117 126.646 59.406 170.055 1.00 18.01 B
ATOM 6598 CG PHEB 117 126.540 60.721 170.776 1.00 17.76 B
ATOM 6599 CD 1 PHE B 117 126.499 60.767 172.168 1.00 17.93 B
ATOM 6600 CD2 PHE B 117 126.497 61.915 170.065 1.00 16.80 B
ATOM 6601 CE1 PHE B 117 126.423 61.985 172.840 1.00 18.33 B
ATOM 6602 CE2 PHE B 117 126.419 63.144 170.726 1.00 16.31 B
ATOM 6603 CZ PHE B 117 126.383 63.181 172.114 1.0017.82 B
ATOM 6604 C PHE B 117 125.531 57.499 168.922 1.00 19.06 B
ATOM 6605 O PHE B 117 125.960 56.477 169.458 1.00 19.52 B
ATOM 6606 N LYS B 118 125.237 57.573 167.629 1.00 19.12 B
ATOM 6607 CA LYS B 118 125.424 56.436 166.741 1.00 18.53 B
ATOM 6608 CB LYS B 118 124.108 55.686 166.551 1.00 20.16 B
ATOM 6609 CG LYS B 118 123.600 55.019 167.821 1.00 22.62 B
ATOM 6610 CD LYS B 118 122.326 54.217 167.sus7 1.00 23.97 B
ATOM 6611 CE LYS B 118 121.141 55.128 167.293 1.0024.11 B
ATOM 6612 NZ LYS B 118 8 119.909 54.342 166.997 1.00 27.18 B
ATOM 6613 C LYS B 118 125.971 56.876 165.392 1.00 18.88 B
ATOM 6614 O LYS B 118 125.536 57.879 164.815 1.00 16.70 B
ATOM 6615 N VAL B 119 126.942 56.120 164.898 1.00 18.12 B
ATOM 6616 CA VAL B 119 127.564 56.418 163.624 1.00 17.94 B
ATOM 6617 CB VAL B 119 128.847 57.281 163.827 1.00 19.84 B
ATOM 6618 CG1 VAL B 119 129.784 56.599 164.814 1.00 19.23 B
ATOM 6619 CG2 VAL B 119 129.557 57.511 162.490 1.00 20.76 B
ATOM 6620 C VAL B 119 127.906 55.107 162.938 1.00 16.87 B
ATOM 6621 O VAL B 119 128.308 54.140 163.588 1.00 18.83 B
ATOM 6622 N TRP B 120 127.737 55.080 161.623 1.00 16.76 B
ATOM 6623 CA TRP B 120 128.012 53.896 160.818 1.00 16.33 B
ATOM 6624 CB TRP B 120 127.067 53.874 159.605 1.0015.72 B
ATOM 6625 CG TRP B 120 127.046 52.59 158.830 1.0015.52 B
ATOM 6626 CD2 TRP B 120 127.521 52.365 157.492 1.00 14.59 B
ATOM 6627 CE2 TRP B 120 127.253 51.017 157.164 1.00 14.23 B
ATOM 6628 CE3 TRP B 120 128.144 53.182 156.537 1.00 14.80 B
ATOM 6629 CD1 TRP B 120 126.529 1.382 159.246 1.00 14.13 B
ATOM 6630 NE1 TRP B 120 126.649 50.444 158.251 1.00 13.87 B
ATOM 6631 CZ2 TRP B 120 127.585 50.466 155.920 1.0015.66 B
ATOM 6632 CZ3 TRP B 120 128.475 52.633 155.295 1.00 14.63 B
ATOM 6633 CH2 TRP B 120 128.193 51.288 155.002 1.00 5.24 B
ATOM 6634 C TRP B 120 129.463 53.927 160.340 1.00 15.50 B
ATOM 6635 O TRP B 120 129.807 54.717 159.460 1.00 16.05 B
ATOM 6636 N ALA B 121 130.304 53.072 160.921 1.00 14.72 B
ATOM 6637 CA ALA B 121 131.719 52.985 160.553 1.00 14.81 B
ATOM 6638 CB ALA B 121 132.569 53.780 161.531 1.00 12.36 B
ATOM 6639 C ALA B 121 132.140 51.513 160.555 1.00 17.11 B
ATOM 6640 O ALA B 121 132.978 51.086 161.353 1.00 17.57 B
ATOM 6641 N PRO B 122 131.564 50.723 159.639 1.00 17.30 B
ATOM 6642 CD PRO B 122 130.691 51.214 158.557 1.00 16.96 B
ATOM 6643 CA PRO B 122 131.823 49.288 159.488 1.00 17.14 B
ATOM 6644 CB PRO B 122 130.896 48.890 158.332 1.00 14.89 B
ATOM 6645 CG PRO B 122 130.844 50.131 157.504 1.00 16.74 B
ATOM 6646 C PRO B 122 133.271 48.864 159.250 1.00 18.37 B
ATOM 6647 O PRO B 122 133.676 47.784 159.673 1.00 19.53 B
ATOM 6648 N ALA B 123 134.055 49.698 158.580 1.00 16.84 B
ATOM 6649 CA ALA B 123 135.440 49.329 158.311 1.00 18.61 B
ATOM 6650 CB ALA B 123 135.898 49.938 156.984 1.00 16.81 B
ATOM 6651 C ALA B 123 136.393 49.744 159.427 1.0017.14 B
ATOM 6652 O ALA B 123 137.572 49.396 159.401 1.00 18.80 B
ATOM 6653 N ALA B 124 135.884 50.483 160.402 1.00 16.03 B
ATOM 6654 CA ALA B 124 136.715 50.952 161.498 1.00 17.51 B
ATOM 6655 CB ALA B 124 136.088 52.190 162.134 1.00 17.13 B
ATOM 6656 C ALA B 124 136.946 49.883 162.553 1.00 17.62 B
ATOM 6657 O ALA B 124 136.260 48.865 162.579 1.00 18.98 B
ATOM 6658 N THR B 125 137.923 50.127 163.422 1.00 17.88 B
ATOM 6659 CA THR B 125 138.260 49.200 164.497 1.00 17.70 B
ATOM 6660 CB THR B 125 139.754 48.821 164.460 1.00 19.62 B
ATOM 6661 OG1 THR B 125 140.548 50.015 164.419 1.00 22.03 B
ATOM 6662 CG2 THR B 125 140.061 47.968 163.234 1.00 18.95 B
ATOM 6663 C THR B 125 137.963 49.845 165.845 1.00 17.81 B
ATOM 6664 O THR B 125 138.094 49.211 166.890 1.00 16.55 B
ATOM 6665 N SER B 126 137.565 51.113 165.817 1.00 16.99 B
ATOM 6666 CA SER B 126 137.256 51.825 167.045 1.00 18.52 B
ATOM 6667 CB SER B 126 138.546 52.042 167.845 1.00 20.72 B
ATOM 6668 OG SER B 126 138.270 52.269 169.208 1.00 26.43 B
ATOM 6669 C SER B 126 136.601 53.165 166.734 1.00 17.57 B
ATOM 6670 O SER B 126 136.736 53.685 165.628 1.00 17.12 B
ATOM 6671 N ALA B 127 135.895 53.720 167.716 1.00 17.71 B
ATOM 6672 CA ALA B 127 135.218 55.005 167.556 1.00 17.99 B
ATOM 6673 CB ALA B 127 133.908 54.818 166.789 1.00 7.12 B
ATOM 6674 C ALA B 127 134.936 55.624 168.922 1.00 17.32 B
ATOM 6675 O ALA B 127 134.850 54.924 169.927 1.00 18.73 B
ATOM 6676 N ALA B 128 134.788 56.940 168.949 1.00 15.72 B
ATOM 6677 CA ALA B 128 134.512 57.648 170.190 1.00 15.75 B
ATOM 6678 CB ALA B 128 135.810 57.904 170.948 1.00 15.51 B
ATOM 6679 C ALA B 128 133.828 58.970 169.881 1.00 15.60 B
ATOM 6680 O ALA B 128 133.842 59.436 168.742 1.00 15.16 B
ATOM 6681 N VAL B 129 133.223 59.567 170.898 1.00 15.83 B
ATOM 6682 CA VAL B 129 132.559 60.837 170.722 1.00 17.37 B
ATOM 6683 CB VAL B 129 131.050 60.762 171.102 1.00 16.76 B
ATOM 6684 CG VALA 129 130.872 60.419 172.574 1.00 18.73 B
ATOM 6685 CG2 VAL B 129 130.380 62.084 170.778 1.00 19.77 B
ATOM 6686 C VAL B 129 133.287 61.864 171.581 1.00 18.02 B
ATOM 6687 O VAL B 129 133.499 61.660 172.776 1.00 18.84 B
ATOM 6688 N LYS B 130 133.687 62.956 170.943 1.00 19.22 B
ATOM 6689 CA LYS B 130 134.411 64.032 171.600 1.00 20.46 B
ATOM 6690 CB LYS B 130 135.583 64.479 170.714 1.00 22.37 B
ATOM 6691 CG LYS B 130 136.499 65.518 171.339 1.00 29.73 B
ATOM 6692 CD LYS B 130 137.720 65.792 170.457 1.00 34.78 B
ATOM 6693 CE LYS B 130 138.609 64.553 170.335 1.00 39.28 B
ATOM 6694 NZ LYS B 130 139.832 64.765 169.489 1.00 43.02 B
ATOM 6695 C LYS B 130 133.457 65.191 171.863 1.00 20.24 B
ATOM 6696 O LYS B 130 132.983 65.863 170.933 1.00 16.94 B
ATOM 6697 N LEU B 131 133.184 65.416 173.143 1.00 19.62 B
ATOM 6698 CA LEU B 131 132.275 66.468 173.573 1.00 19.68 B
ATOM 6699 CB LEU B 131 131.342 65.927 174.656 1.00 18.82 B
ATOM 6700 CG LEU B 131 130.542 64.681 174.280 1.00 19.24 B
ATOM 6701 CD1 LEU B 131 129.883 64.100 175.520 1.00 18.09 B
ATOM 6702 CD2 LEU B 131 129.506 65.036 173.222 1.00 16.95 B
ATOM 6703 C LEU B 131 133.009 67.680 174.119 1.00 21.26 B
ATOM 6704 O LEU B 131 133.913 67.544 174.942 1.00 20.94 B
ATOM 6705 N SER B 132 132.622 68.864 173.654 1.00 22.62 B
ATOM 6706 CA SER B 132 133.225 70.098 174.135 1.00 25.83 B
ATOM 6707 CB SERB 132 134.318 70.595 173.173 1.00 23.94 B
ATOM 6708 OG SER B 132 133.831 70.834 171.866 1.00 25.21 B
ATOM 6709 C SER B 132 132.133 71.150 174.327 1.00 28.62 B
ATOM 6710 O SER B 132 131.078 71.091 173.696 1.00 28.49 B
ATOM 6711 N HIS B 133 132.386 72.103 175.215 1.00 32.84 B
ATOM 6712 CA HIS B 133 131.418 73.150 175.519 1.00 36.34 B
ATOM 6713 CB HIS B 133 130.551 72.685 176.693 1.0037.48 B
ATOM 6714 CG HIS B 133 129.404 73.590 177.007 1.00 39.28 B
ATOM 6715 CD2 HIS B 133 129.368 74.879 177.420 1.00 40.61 B
ATOM 6716 ND1 HIS B 133 128.092 73.171 176.944 1.00 40.84 B
ATOM 6717 CE1 HIS B 133 127.298 74.163 177.304 1.00 40.92 B
ATOM 6718 NE2 HIS B 133 128.047 75.211 177.598 1.0040.94 B
ATOM 6719 C HIS B 133 132.180 74.429 175.879 1.00 39.24 B
ATOM 6720 O HIS B 133 133.175 74.380 176.605 1.00 38.89 B
ATOM 6721 N PRO B 134 131.722 75.589 175.373 1.00 41.85 B
ATOM 6722 CD PRO B 134 130.504 75.735 174.553 1.00 43.26 B
ATOM 6723 CA PRO B 134 132.335 76.902 175.615 1.00 43.88 B
ATOM 6724 CB PRO B 134 131.243 77.869 175.175 1.00 43.99 B
ATOM 6725 CG PRO B 134 130.637 77.150 174.024 1.00 43.48 B
ATOM 6726 C PRO B 134 132.803 77.165 177.051 1.00 45.59 B
ATOM 6727 O PRO B 134 133.904 77.680 177.266 1.00 46.68 B
ATOM 6728 N ASN B 135 131.973 76.820 178.031 1.00 46.17 B
ATOM 6729 CA ASN B 135 132.336 77.026 179.429 1.00 48.43 B
ATOM 6730 CB ASN B 135 131.590 78.242 180.008 1.00 49.32 B
ATOM 6731 CG ASN B 135 130.229 78.473 179.355 1.00 50.95 B
ATOM 6732 OD1 ASN B 135 130.070 79.381 178.530 1.00 49.31 B
ATOM 6733 ND2 ASN B 135 129.245 77.652 179.719 1.00 48.79 B
ATOM 6734 C ASN B 135 132.101 75.801 180.315 1.0049.13 B
ATOM 6735 O ASN B 135 131.576 75.918 181.427 1.0050.20 B
ATOM 6736 N LYS B 136 132.497 74.629 179.822 1.00 48.20 B
ATOM 6737 CA LYS B 136 132.346 73.382 180.571 1.00 46.73 B
ATOM 6738 CB LYS B 136 131.036 72.688 180.198 1.00 46.69 B
ATOM 6739 CG LYS B 136 129.788 73.439 180.635 1.0049.05 B
ATOM 6740 CD LYS B 136 128.526 72.646 180.316 1.00 50.97 B
ATOM 6741 CE LYS B 136 127.272 73.382 180.765 1.00 52.64 B
ATOM 6742 NZ LYS B 136 126.026 72.647 180.389 1.00 52.73 B
ATOM 6743 C LYS B 136 133.520 72.435 180.326 1.00 45.99 B
ATOM 6744 O LYS B 136 134.350 72.667 179.442 1.00 45.67 B
ATOM 6745 N SER B 137 133.590 71.367 181.114 1.00 44.31 B
ATOM 6746 CA SER B 137 134.667 70.397 180.977 1.0042.20 B
ATOM 6747 CB SER B 137 134.755 69.515 182.224 1.00 43.74 B
ATOM 6748 OG SER B 137 135.108 70.275 183.366 1.00 47.04 B
ATOM 6749 C SERB 137 134.467 69.512 179.757 1.00 40.05 B
ATOM 6750 O SER B 137 133.365 69.022 179.512 1.0040.02 B
ATOM 6751 N GLY B 138 135.540 69.318 178.995 1.00 37.03 B
ATOM 6752 CA GLY B 138 135.474 68.470 177.820 1.00 33.00 B
ATOM 6753 C GLY B 138 135.561 67.012 178.232 1.00 30.67 B
ATOM 6754 O GLY B 138 136.147 66.683 179.264 1.00 31.94 B
ATOM 6755 N ARG B 139 134.974 66.133 177.430 1.00 27.12 B
ATOM 6756 CA ARG B 139 134.983 64.706 177.721 1.00 24.17 B
ATOM 6757 CB ARG B 139 133.769 64.326 178.582 1.00 23.27 B
ATOM 6758 CG ARG B 139 133.593 65.135 179.866 1.0027.78 B
ATOM 6759 CD ARG B 139 134.629 64.764 180.924 1.00 25.55 B
ATOM 6760 NE ARG B 139 134.448 63.406 181.434 1.00 24.99 B
ATOM 6761 CZ ARG B 139 133.422 63.011 182.183 1.00 25.40 B
ATOM 6762 NH1 ARG B 139 132.466 63.868 182.521 1.00 25.99 B
ATOM 6763 NH2 ARG B 139 133.358 61.758 182.606 1.00 23.29 B
ATOM 6764 C ARG B 139 134.910 63.925 176.412 1.00 22.78 B
ATOM 6765 O ARG B 139 134.154 64.282 175.506 1.00 21.54 B
ATOM 6766 N THR B 140 135.696 62.861 176.316 1.00 20.09 B

ATOM 6767 CA THR B 140 135.679 62.012 175.133 1.00 19.52 B
ATOM 6768 CB THR B 140 137.006 62.091 174.363 1.00 17.88 B
ATOM 6769 OG1 THR B 140 137.199 63.428 173.888 1.00 16.78 B
ATOM 6770 CG2 THR B 140 136.989 61.131 173.179 1.00 17.98 B
ATOM 6771 C THR B 140 135.426 60.570 175.584 1.00 19.50 B
ATOM 6772 O THR B 140 136.218 59.988 176.334 1.0018.40 B
ATOM 6773 N PHE B 141 134.318 60.003 175.127 1.00 19.15 B
ATOM 6774 CA PHE B 141 133.947 58.646 175.505 1.00 19.81 B
ATOM 6775 CB PHE B 141 132.521 58.627 176.066 1.0019.03 B
ATOM 6776 CG PHE B 141 132.339 59.476 177.289 1.00 18.66 B
ATOM 6777 CD1 PHE B 141 131.351 60.457 177.329 1.00 19.73 B
ATOM 6778 CD2 PHE B 141 133.147 59.295 178.405 1.00 18.27 B
ATOM 6779 CE1 PHE B 141 131.170 61.247 178.466 1.00 19.14 B
ATOM 6780 CE2 PHE B 141 132.976 60.080 179.550 1.00 18.32 B
ATOM 6781 CZ PHE B 141 131.985 61.057 179.578 1.00 18.81 B
ATOM 6782 C PHE B 141 134.041 57.666 174.352 1.00 20.20 B
ATOM 6783 O PHE B 141 133.670 57.975 173.219 1.00 19.67 B
ATOM 6784 N GLN B 142 134.537 56.474 174.655 1.00 21.44 B
ATOM 6785 CA GLN B 142 134.670 55.435 173.649 1.00 23.73 B
ATOM 6786 CB GLN B 142 135.536 54.285 174.168 1.00 25.28 B
ATOM 6787 CG GLN B 142 136.070 53.409 173.054 1.00 33.58 B
ATOM 6788 CD GLN B 142 136.711 52.137 173.557 1.0036.09 B
ATOM 6789 OE1 GLN B 142 137.492 51.509 172.845 1.00 39.48 B
ATOM 6790 NE2 GLN B 142 136.374 51.740 174.781 1.00 37.45 B
ATOM 6791 C GLN B 142 133.286 54.901 173.314 1.00 21.94 B
ATOM 6792 O GLN B 142 132.407 54.859 174.172 1.00 22.06 B
ATOM 6793 N MET B 143 133.096 54.499 172.065 1.00 20.31 B
ATOM 6794 CA MET B 143 131.818 53.960 171.635 1.00 20.50 B
ATOM 6795 CB MET B 143 131.379 54.630 70.330 1.00 20.65 B
ATOM 6796 CG MET B 143 131.084 56.118 170.503 1.00 20.99 B
ATOM 6797 SD MET B 143 130.890 57.026 168.962 1.0022.87 B
ATOM 6798 CE MET B 143 129.163 56.760 168.626 1.00 21.23 B
ATOM 6799 C MET B 143 131.935 52.454 171.455 1.00 20.16 B
ATOM 6800 O MET B 143 133.010 51.935 171.157 1.00 19.72 B
ATOM 6801 N THR B 144 130.822 51.756 171.645 1.0019.66 B
ATOM 6802 CA THR B 144 130.796 50.306 171.517 1.00 19.97 B
ATOM 6803 CB THR B 144 129.898 49.679 172.605 1.00 20.91 B
ATOM 6804 OG1 THR B 144 130.380 50.073 173.896 1.00 21.73 B
ATOM 6805 CG2 THR B 144 129.914 48.161 172.511 11.00 18.93 B
ATOM 6806 C THR B 144 130.271 49.906 170.153 1.00 19.33 B
ATOM 6807 O THR B 144 129.326 50.508 169.648 1.00 18.71 B
ATOM 6808 N ARG B 145 130.884 48.895 169.547 1.00 19.56 B
ATOM 6809 CA ARG B 145 130.426 48.451 168.241 1.00 19.55 B
ATOM 6810 CB ARG B 145 131.534 47.724 167.474 1.00 19.33 B
ATOM 6811 CG ARG B 145 131.135 47.395 166.033 1.00 19.30 B
ATOM 6812 CD ARG B 145 132.244 46.687 165.270 1.00 19.41 B
ATOM 6813 NE ARG B 145 131.828 46.266 163.932 1.00 16.91 B
ATOM 6814 CZ ARG B 145 132.338 46.750 162.802 1.00 17.63 B
ATOM 6815 NH1 ARG B 145 133.285 47.681 162.839 1.00 15.50 B
ATOM 6816 NH2 ARG B 145 131.917 46.290 161.630 1.00 16.16 B
ATOM 6817 C ARG B 145 129.243 47.517 168.406 1.00 21.25 B
ATOM 6818 O ARG B 145 129.315 46.544 169.151 1.00 21.59 B
ATOM 6819 N LEU B 146 128.148 47.826 167.720 1.00 21.29 B
ATOM 6820 CA LEU B 146 126.950 46.997 167.767 1.00 21.25 B
ATOM 6821 CB LEU B 146 125.702 47.871 167.911 1.00 19.45 B
ATOM 6822 CG LEU B 146 125.702 48.860 169.081 1.00 21.23 B
ATOM 6823 CD1 LEU B 146 124.423 49.683 169.058 1.00 19.44 B
ATOM 6824 CD2 LEU B 146 125.834 48.113 170.396 1.00 18.99 B
ATOM 6825 C LEU B 146 126.894 46.232 166.447 1.00 22.24 B
ATOM 6826 O LEU B 146 127.899 46.123 165.745 1.00 22.10 B
ATOM 6827 N GLU B 147 125.725 45.702 166.109 1.00 23.23 B
ATOM 6828 CA GLU B 147 125.566 44.972 164.859 1.00 23.92 B
ATOM 6829 CB GLU B 147 124.277 44.146 164.876 1.00 26.70 B
ATOM 6830 CG GLU B 147 124.123 43.182 166.054 1.0034.85 B
ATOM 6831 CD GLU B 147 125.224 42.133 166.135 1.00 38.16 B
ATOM 6832 OE1 GLU B 147 126.288 42.414 166.735 1.00 40.48 B
ATOM 6833 OE2 GLU B 147 125.026 41.025 165.593 1.00 42.38 B
ATOM 6834 C GLU B 147 125.486 45.992 163.723 1.00 23.19 B
ATOM 6835 O GLU B 147 125.241 47.176 163.959 1.00 20.38 B
ATOM 6836 N LYS B 148 125.701 45.527 162.496 1.0022.07 B
ATOM 6837 CA LYS B 148 125.623 46.379 161.314 1.00 22.62 B
ATOM 6838 CB LYS B 148 124.216 46.982 161.211 1.00 21.58 B
ATOM 6839 CG LYS B 148 123.101 45.941 161.190 1.00 21.28 B
ATOM 6840 CD LYS B 148 123.316 44.909 160.074 1.00 22.82 B
ATOM 6841 CE LYS B 148 122.188 43.875 160.045 1.00 24.91 B
ATOM 6842 NZ LYS B 148 122.396 42.837 158.998 1.0025.96 B
ATOM 6843 C LYS B 148 126.660 47.496 161.238 1.00 23.27 B
ATOM 6844 O LYS B 148 126.398 48.540 160.642 1.00 22.71 B
ATOM 6845 N GLY B 149 127.826 47.281 161.846 1.0023.16 B
ATOM 6846 CA GLY B 149 128.884 48.282 161.808 1.00 21.23 B
ATOM 6847 C GLY B 149 128.574 49.623 162.457 1.00 20.38 B
ATOM 6848 O GLY B 149 129.221 50.629 162.156 1.0019.48 B
ATOM 6849 N VAL B 150 127.587 49.644 163.349 1.00 19.25 B
ATOM 6850 CA VAL B 150 127.208 50.870 164.040 1.00 17.85 B
ATOM 6851 CB VAL B 150 125.680 50.929 164.287 1.00 17.70 B
ATOM 6852 CG1 VAL B 150 125.347 52.071 165.239 1.00 12.74 B
ATOM 6853 CG2 VAL B 150 124.938 51.114 162.961 1.00 17.17 B
ATOM 6854 C VAL B 150 127.906 50.981 165.391 1.00 18.53 B
ATOM 6855 O VAL B 150 127.917 50.032 166.170 1.00 20.48 B
ATOM 6856 N TYR B 151 128.502 52.137 165.659 1.00 18.48 B
ATOM 6857 CA TYR B 151 129.160 52.378 166.939 1.00 18.00 B
ATOM 6858 CB TYR B 151 130.518 53.054 166.736 1.00 17.13 B
ATOM 6859 CG TYR B 151 131.595 52.090 166.312 1.00 17.82 B
ATOM 6860 CD1 TYR B 151 132.425 51.483 167.259 1.00 19.37 B
ATOM 6861 CE1 TYR B 151 133.382 50.542 166.884 1.00 17.56 B
ATOM 6862 CD2 TYR B 151 131.750 51.734 164.975 1.00 17.11 B
ATOM 6863 CE2 TYR B 151 132.700 50.794 164.587 1.00 18.51 B
ATOM 6864 CZ TYR B 151 133.512 50.201 165.548 1.00 18.53 B
ATOM 6865 OH TYR B 151 134.442 49.261 165.173 1.00 18.33 B
ATOM 6866 C TYR B 151 128.228 53.293 167.714 1.00 18.38 B
ATOM 6867 O TYR B 151 127.688 54.254 167.158 1.00 18.90 B
ATOM 6868 N ALA B 152 128.031 52.994 168.992 1.0017.53 B
ATOM 6869 CA ALA B 152 127.136 53.798 169.810 1.00 16.85 B
ATOM 6870 CB ALA B 152 125.742 53.168 169.827 1.00 14.33 B
ATOM 6871 C ALA B 152 127.616 53.983 171.233 1.00 16.35 B
ATOM 6872 O ALA B 152 128.450 53.234 171.729 1.00 16.90 B
ATOM 6873 N VAL B 153 127.068 55.000 171.881 1.0017.47 B
ATOM 6874 CA VAL B 153 127.376 55.296 173.263 1.00 18.32 B
ATOM 6875 CB VAL B 153 128.742 56.020 173.431 1.00 19.57 B
ATOM 6876 CG1 VALB 153 128.636 57.485 173.015 1.00 17.84 B
ATOM 6877 CG2 VAL B 153 129.209 55.900 174.881 1.00 21.60 B
ATOM 6878 C VAL B 153 126.264 56.183 173.797 1.00 19.05 B
ATOM 6879 O VAL B 153 125.831 57.132 173.138 1.00 18.07 B
ATOM 6880 N THR B 154 125.783 55.847 174.985 1.00 18.95 B
ATOM 6881 CA THR B 154 124.739 56.620 175.622 1.00 19.68 B
ATOM 6882 CB THR B 154 123.681 55.700 176.240 1.00 20.04 B
ATOM 6883 OG1 THR B 154 123.181 54.811 175.231 1.00 19.13 B
ATOM 6884 CG2 THR B 154 122.534 56.517 176.810 1.00 19.63 B
ATOM 6885 C THR B 154 125.416 57.441 176.711 1.00 21.06 B
ATOM 6886 O THR B 154 126.108 56.896 177.568 1.00 22.74 B
ATOM 6887 N VALB 155 125.228 58.755 176.662 1.00 21.86 B
ATOM 6888 CA VAL B 155 125.834 59.655 177.630 1.00 21.14 B
ATOM 6889 CB VAL B 155 126.618 60.781 176.911 1.00 20.82 B
ATOM 6890 CG1 VALB 155 127.286 61.703 177.925 1.00 19.66 B
ATOM 6891 CG2 VAL B 155 127.654 60.170 175.987 1.00 19.14 B
ATOM 6892 C VAL B 155 124.762 60.272 178.515 1.00 23.20 B
ATOM 6893 O VAL B 155 123.883 60.996 178.034 1.00 21.33 B
ATOM 6894 N THR B 156 124.843 59.970 179.811 1.00 23.14 B
ATOM 6895 CA THR B 156 123.892 60.474 180.794 1.00 24.20 B
ATOM 6896 CB THR B 156 124.049 59.739 182.144 1.00 26.18 B
ATOM 6897 OG1 THR B 156 123.646 58.370 181.990 1.00 28.33 B
ATOM 6898 CG2 THR B 156 123.197 60.401 183.221 1.00 25.91 B
ATOM 6899 C THR B 156 124.088 61.960 181.020 1.00 23.96 B
ATOM 6900 O THR B 156 125.209 62.462 180.969 1.00 25.22 B
ATOM 6901 N GLY B 157 122.990 62.659 181.278 1.00 24.84 B
ATOM 6902 CA GLY B 157 123.061 64.090 181.505 1.00 25.28 B
ATOM 6903 C GLY B 157 122.388 64.830 180.366 1.0026.64 B
ATOM 6904 O GLY B 157 122.058 64.220 179.348 1.00 26.37 B
ATOM 6905 N ASP B 158 122.169 66.131 180.539 1.00 27.06 B
ATOM 6906 CA ASP B 158 121.539 66.952 179.507 1.00 28.83 B
ATOM 6907 CB ASP B 158 120.712 68.074 180.149 1.00 30.11 B
ATOM 6908 CG ASP B 158 120.043 68.975 179.122 1.00 33.57 B
ATOM 6909 OD1 ASP B 158 120.175 68.710 177.906 1.00 35.30 B
ATOM 6910 OD2 ASP B 158 119.382 69.954 179.529 1.00 36.10 B
ATOM 6911 C ASP B 158 122.648 67.547 178.647 1.00 27.45 B
ATOM 6912 O ASP B 158 123.329 68.482 179.062 1.00 27.31 B
ATOM 6913 N LEU B 159 122.825 67.007 177.447 1.00 27.46 B
ATOM 6914 CA LEU B 159 123.878 67.484 176.555 1.00 26.43 B
ATOM 6915 CB LEU B 159 124.383 66.336 175.673 1.00 25.52 B
ATOM 6916 CG LEU B 159 125.031 65.153 176.395 1.00 25.38 B
ATOM 6917 CD1 LEU B 159 125.853 64.359 175.395 1.00 24.23 B
ATOM 6918 CD2 LEU B 159 125.932 65.647 177.514 1.00 24.50 B
ATOM 6919 C LEU B 159 123.515 5 68.667 175.668 1.00 25.71 B
ATOM 6920 O LEU B 159 124.263 68.994 174.743 1.00 26.86 B
ATOM 6921 N HIS B 160 122.386 69.316 175.939 1.00 23.58 B
ATOM 6922 CA HIS B 160 121.988 70.460 175.123 1.00 21.33 B
ATOM 6923 CB HIS B 160 120.668 71.057 175.615 1.0020.95 B
ATOM 6924 CG HIS B 160 120.329 72.355 174.959 1.00 17.83 B
ATOM 6925 CD2 HIS B 160 119.725 72.624 173.778 1.00 16.22 B
ATOM 6926 ND1 HIS B 160 120.715 73.572 175.477 1.00 18.89 B
ATOM 6927 CE1 HIS B 160 120.367 74.535 174.642 1.00 17.70 B
ATOM 6928 NE2 HIS B 160 119.765 73.986 173.603 1.00 20.18 B
ATOM 6929 C HIS B 160 123.061 71.543 175.125 1.00 21.71 B
ATOM 6930 O HIS B 160 123.554 71.935 176.180 1.00 23.63 B
ATOM 6931 N GLY B 161 123.425 72.027 173.942 1.00 20.84 B
ATOM 6932 CA GLY B 161 124.447 73.055 173.863 1.00 19.45 B
ATOM 6933 C GLY B 161 125.857 72.507 173.710 1.0019.94 B
ATOM 6934 O GLY B 161 126.779 73.247 173.360 1.00 19.59 B
ATOM 6935 N TYR B 162 126.042 71.216 173.970 1.00 18.97 B
ATOM 6936 CA TYR B 162 127.368 70.618 173.831 1.00 20.53 B
ATOM 6937 CB TYR B 162 127.437 69.247 174.509 1.0020.37 B
ATOM 6938 CG TYR B 162 127.766 69.296 175.984 1.0025.36 B
ATOM 6939 CE1 TYR B 162 126.815 69.703 176.924 1.00 25.73 B
ATOM 6940 CE1 TYR B 162 127.121 69.751 178.285 1.0027.91 B
ATOM 6941 CD2 TYR B 162 129.035 68.940 176.441 1.00 25.77 B
ATOM 6942 CE2 TYR B 162 129.353 68.985 177.796 1.00 28.24 B
ATOM 6943 CZ TYR B 162 128.393 69.390 178.711 1.00 29.07 B
ATOM 6944 OH TYR B 162 128.706 69.431 180.050 1.0033.30 B
ATOM 6945 C TYR B 162 127.766 70.442 172.372 1.00 19.93 B
ATOM 6946 O TYR B 162 126.951 70.048 171.539 1.00 18.63 B
ATOM 6947 N GLU B 163 129.022 70.742 172.070 1.00 18.45 B
ATOM 6948 CA GLU B 163 129.535 70.572 170.722 1.00 19.25 B
ATOM 6949 CB GLU B 163 130.675 71.550 170.459 1.0020.32 B
ATOM 6950 CG GLU B 163 130.361 72.983 170.836 1.0021.64 B
ATOM 6951 CD GLU B 163 131.607 73.838 170.893 1.0022.28 B
ATOM 6952 OE1 GLU B 163 132.687 73.286 171.190 1.00 23.76 B
ATOM 6953 OE2 GLU B 163 131.508 75.058 170.659 1.00 24.12 B
ATOM 6954 C GLU B 163 130.080 69.152 170.677 1.00 18.91 B
ATOM 6955 O GLU B 163 130.626 68.666 171.669 1.00 19.38 B
ATOM 6956 N TYR B 164 129.928 68.472 169.546 1.00 17.93 B
ATOM 6957 CA TYR B 164 130.449 67.122 169.451 1.00 16.73 B
ATOM 6958 CB TYR B 164 129.361 66.084 169.754 1.00 17.13 B
ATOM 6959 CG TYR B 164 128.308 65.904 168.677 1.00 17.52 B
ATOM 6960 CD1 TYR B 164 128.090 64.655 168.105 1.00 17.38 B
ATOM 6961 CE1 TYR B 164 127.078 64.456 167.180 1.00 18.18 B
ATOM 6962 CD2 TYR B 164 127.482 66.962 168.282 1.00 18.79 B
ATOM 6963 CE2 TYR B 164 126.461 66.771 167.347 1.00 16.08 B
ATOM 6964 CZ TYR B 164 126.267 65.514 166.806 1.00 17.32 B
ATOM 6965 OH TYR B 164 125.262 65.293 163.895 1.00 19.09 B
ATOM 6966 C TYR B 164 131.068 66.813 168.107 1.00 19.33 B
ATOM 6967 O TYR B 164 130.753 67.438 167.087 1.00 18.24 B
ATOM 6968 N LEU B 165 131.964 65.833 168.136 1.00 17.85 B
ATOM 6969 CA LEU B 165 132.662 65.356 166.963 1.00 15.45 B
ATOM 6970 CB LEU B 165 134.039 66.023 166.851 1.00 14.70 B
ATOM 6971 CG LEU B 165 134.102 67.538 166.618 1.00 14.92 B
ATOM 6972 CD1 LEU B 165 135.535 68.031 166.865 1.00 11.45 B
ATOM 6973 CD2 LEU B 165 133.625 67.877 165.186 1.00 10.35 B
ATOM 6974 C LEU B 165 132.833 63.856 167.182 1.00 15.97 B
ATOM 6975 O LEU B 165 132.769 63.373 168.316 1.00 15.10 B
ATOM 6976 N PHE B 166 133.019 63.119 166.098 1.00 14.88 B
ATOM 6977 CA PHE B 166 133.238 61.692 166.197 1.00 16.82 B
ATOM 6978 CB PHE B 166 132.347 60.926 165.209 1.00 16.61 B
ATOM 6979 CG PHE B 166 130.905 60.833 165.624 1.00 16.79 B
ATOM 6980 CD1 PHE B 166 129.897 60.782 164.662 1.00 17.94 B
ATOM 6981 CD2 PHE B 166 130.548 60.781 166.968 1.00 18.57 B
ATOM 6982 CE1 PHE B 166 128.553 60.680 165.031 1.00 17.13 B
ATOM 6983 CE2 PHE B 166 129.206 60.679 167.349 1.00 18.78 B
ATOM 6984 CZ PHE B 166 128.207 60.630 166.376 1.00 18.39 B
ATOM 6985 C PHE B 166 134.698 61.451 165.853 1.00 18.52 B
ATOM 6986 O PHE B 166 135.273 62.134 165.004 1.00 19.02 B
ATOM 6987 N CYS B 167 135.303 60.499 166.543 1.00 19.97 B
ATOM 6988 CA CYS B 167 136.681 60.130 166.280 1.00 21.21 B
ATOM 6989 CB CYS B 167 137.494 60.115 167.581 1.00 23.03 B
ATOM 6990 SG CYS B 167 137.699 61.746 168.369 1.00 31.71 B
ATOM 6991 C CYS B 167 136.546 58.725 165.704 1.00 19.34 B
ATOM 6992 O CYS B 167 135.997 57.844 166.353 1.00 20.22 B
ATOM 6993 N ILE B 168 137.015 58.522 164.478 1.00 17.70 B
ATOM 6994 CA ILE B 168 136.908 57.219 163.841 1.00 15.91 B
ATOM 6995 CB ILE B 168 136.070 57.310 162.539 1.00 16.75 B
ATOM 6996 CG2 ILE B 168 135.782 55.918 162.009 1.00 12.92 B
ATOM 6997 CG1 ILE B 168 134.764 58.081 162.795 1.00 13.49 B
ATOM 6998 CD1 ILE B 168 133.898 57.505 163.887 1.00 14.36 B
ATOM 6999 C ILE B 168 138.298 56.673 163.510 1.00 17.15 B
ATOM 7000 O ILE B 168 139.134 57.373 162.937 1.00 15.90 B
ATOM 7001 N CYS B 169 138.541 55.422 163.883 1.00 16.56 B
ATOM 7002 CA CYS B 169 139.827 54.798 163.633 1.00 17.20 B
ATOM 7003 CB CYS B 169 140.302 54.028 164.874 1.00 19.66 B
ATOM 7004 SG CYS B 169 142.015 53.383 164.767 1.00 20.20 B
ATOM 7005 C CYS B 169 139.767 53.849 162.445 1.00 17.80 B
ATOM 7006 O CYS B 169 139.274 52.725 162.560 1.00 14.89 B
ATOM 7007 N ASN B 170 140.251 54.323 161.300 1.00 16.54 B
ATOM 7008 CA ASN B 170 140.303 53.512 160.091 1.00 17.34 B
ATOM 7009 CB ASN B 170 139.591 54.210 158.921 1.00 15.06 B
ATOM 7010 CG ASN B 170 138.073 54.201 159.060 1.00 15.07 B
ATOM 7011 OD1 ASN B 170 137.447 53.140 159.138 1.00 5.10 B
ATOM 7012 ND2 ASN B 170 137.473 55.384 159.082 1.00 15.58 B
ATOM 7013 C ASN B 170 141.787 53.350 159.767 1.00 18.93 B
ATOM 7014 O ASN B 170 142.550 54.313 159.872 1.00 20.70 B
ATOM 7015 N ASN B 171 142.201 52.135 159.408 1.00 19.40 B
ATOM 7016 CA ASN B 171 143.598 51.869 159.052 1.00 17.98 B
ATOM 7017 CB ASN B 171 143.954 52.623 157.767 1.00 17.45 B
ATOM 7018 CG ASN B 171 142.979 52.339 156.634 1.00 21.07 B
ATOM 7019 OD1 ASN B 171 142.471 53.262 155.992 1.00 24.21 B
ATOM 7020 ND2 ASN B 171 142.719 51.063 156.381 1.00 16.34 B
ATOM 7021 C ASN B 171 144.574 52.278 160.159 1.00 16.61 B
ATOM 7022 O ASN B 171 145.684 52.727 159.881 1.00 16.79 B
ATOM 7023 N SER B 172 144.162 52.115 161.409 1.00 15.21 B
ATOM 7024 CA SER B 172 144.999 52.482 162.549 1.00 18.45 B
ATOM 7025 CB SER B 172 146.350 51.759 162.506 1.00 19.62 B
ATOM 7026 OG SER B 172 146.217 50.416 162.925 1.00 25.50 B
ATOM 7027 C SER B 172 145.236 53.986 162.658 1.00 17.70 B
ATOM 7028 O SER B 172 146.071 54.433 163.452 1.00 18.02 B
ATOM 7029 N GLU B 173 144.513 54.762 161.857 1.00 17.38 B
ATOM 7030 CA GLU B 173 144.632 56.210 161.920 1.00 17.24 B
ATOM 7031 CB GLU B 173 144.967 56.822 160.554 1.00 17.16 B
ATOM 7032 CG GLU B 173 145.181 58.335 160.658 1.00 19.91 B
ATOM 7033 CD GLU B 173 145.704 58.986 159.385 1.00 22.71 B
ATOM 7034 OE1 GLU B 173 146.228 58.273 158.499 1.00 24.08 B
ATOM 7035 OE2 GLU B 173 145.605 60.228 159.285 1.00 20.69 B
ATOM 7036 C GLU B 173 143.316 56.786 162.414 1.00 16.39 B
ATOM 7037 O GLU B 173 142.248 56.471 161.879 1.00 15.20 B
ATOM 7038 N TRP B 174 143.397 57.624 163.440 1.00 15.50 B
ATOM 7039 CA TRP B 174 142.211 58.261 164.003 1.00 15.44 B
ATOM 7040 CB TRP B 174 142.422 58.605 165.483 1.00 13.35 B
ATOM 7041 CG TRP B 174 142.361 57.431 166.406 1.00 14.23 B
ATOM 7042 CD2 TRP B 174 141.194 56.919 167.052 1.00 12.18 B
ATOM 7043 CE2 TRP B 174 141.594 55.800 167.812 1.00 13.07 B
ATOM 7044 CE3 TRP B 174 139.844 57.297 167.059 1.00 13.58 B
ATOM 7045 CD1 TRP B 174 143.395 56.624 166.787 1.00 15.35 B
ATOM 7046 NE1 TRPB 174 142.943 55.643 167.634 1.00 14.61 B
ATOM 7047 CZ2 TRP B 174 140.695 55.054 168.576 1.00 13.93 B
ATOM 7048 CZ3 TRP B 174 138.946 56.552 167.820 1.00 13.41 B
ATOM 7049 CH2 TRP B 174 139.379 55.444 168.568 1.00 13.60 B
ATOM 7050 C TRP B 174 141.891 59.545 163.262 1.00 15.81 B
ATOM 7051 O TRP B 174 142.779 60.354 162.999 1.00 16.35 B
ATOM 7052 N MET B 175 140.625 59.732 162.916 1.00 17.76 B
ATOM 7053 CA MET B I75 140.218 60.957 162.248 1.00 19.60 B
ATOM 7054 CB MET B 175 139.892 60.700 160.777 1.00 21.43 B
ATOM 7055 CG MET B 175 141.134 60.772 159.900 1.00 23.04 B
ATOM 7056 SD MET B 175 140.785 60.652 158.151 1.00 31.07 B
ATOM 7057 CE MET B 175 142.411 60.164 157.515 1.00 25.54 B
ATOM 7058 C MET B 175 139.048 61.623 162.952 1.0020.26 B
ATOM 7059 O MET B 175 138.081 60.975 163.353 1.00 22.41 B
ATOM 7060 N GLU B 176 139.168 62.930 163.115 1.00 20.61 B
ATOM 7061 CA GLU B 176 138.157 63.740 163.775 1.00 22.13 B
ATOM 7062 CB GLU B 176 138.835 64.931 164.439 1.00 21.53 B
ATOM 7063 CG GLU B 176 138.068 65.555 165.560 1.0027.46 B
ATOM 7064 CD GLU B 176 138.903 66.593 166.289 1.0030.95 B
ATOM 7065 OE1 GLU B 176 139.105 67.695 165.721 1.00 31.89 B
ATOM 7066 OE2 GLU B 176 139.367 66.293 167.413 1.00 27.50 B
ATOM 7067 C GLU B 176 137.179 64.228 162.717 1.00 22.08 B
ATOM 7068 O GLU B 176 137.586 64.722 161.664 1.00 22.42 B
ATOM 7069 N THR B 177 135.887 64.102 162.989 1.00 21.11 B
ATOM 7070 CA THR B 177 134.915 64.534 162.006 1.00 18.65 B
ATOM 7071 CB THR B 177 134.644 63.418 160.978 1.00 19.69 B
ATOM 7072 OG1 THR B 177 133.721 63.889 159.987 1.00 21.63 B
ATOM 7073 CG2 THR B 177 134.041 62.207 161.667 1.00 19.70 B
ATOM 7074 C THR B 177 133.583 64.933 162.603 1.00 16.55 B
ATOM 7075 O THR B 177 133.211 64.491 163.686 1.00 16.54 B
ATOM 7076 N VAL B 178 132.869 65.787 161.885 1.00 14.18 B
ATOM 7077 CA VAL B 178 131.551 66.183 162.313 1.00 14.01 B
ATOM 7078 CB VAL B 178 131.068 67.451 161.566 1.00 13.99 B
ATOM 7079 CG1 VAL B 178 129.567 67.657 161.792 1.00 11.53 B
ATOM 7080 CG2 VAL B 178 131.847 68.671 162.058 1.00 13.14 B
ATOM 7081 C VAL B 178 130.642 65.004 161.948 1.00 14.71 B
ATOM 7082 O VAL B 178 130.957 64.202 161.062 1.00 16.91 B
ATOM 7083 N ASP B 179 129.525 64.906 162.647 1.00 14.84 B
ATOM 7084 CA ASP B 179 128.526 63.871 162.427 1.00 14.92 B
ATOM 7085 CB ASP B 179 127.516 63.958 163.580 1.00 17.03 B
ATOM 7086 CG ASP B 179 126.362 62.996 163.446 1.00 17.71 B
ATOM 7087 OD1 ASP B 179 126.384 62.141 162.540 1.00 14.83 B
ATOM 7088 OD2 ASP B 179 125.427 63.105 164.273 1.00 18.59 B
ATOM 7089 C ASP B 179 127.857 64.155 161.073 1.00 15.58 B
ATOM 7090 O ASP B 179 127.445 65.288 160.813 1.00 13.70 B
ATOM 7091 N GLN B 180 127.766 63.145 160.205 1.00 14.69 B
ATOM 7092 CA GLN B 180 127.129 63.344 158.897 1.00 17.02 B
ATOM 7093 CB GLN B 180 127.161 62.061 158.059 1.0017.64 B
ATOM 7094 CG GLN B 180 128.542 61.470 157.840 1.00 17.25 B
ATOM 7095 CD GLN B 180 128.484 60.013 157.412 1.0018.06 B
ATOM 7096 OE1 GLN B 180 129.481 59.299 157.480 1.00 19.10 B
ATOM 7097 NE2 GLN B 180 127.312 59.566 156.969 1.00 14.88 B
ATOM 7098 C GLN B 180 125.674 63.735 159.116 1.00 17.31 B
ATOM 7099 O GLN B 180 125.054 64.380 158.263 1.00 16.79 B
ATOM 7100 N TYR B 181 125.138 63.339 160.268 1.00 16.21 B
ATOM 7101 CA TYR B 181 123.755 63.625 160.615 1.00 15.87 B
ATOM 7102 CB TYR B 181 123.090 62.374 161.212 1.0015.03 B
ATOM 7103 CG TYR B 181 122.732 61.307 160.196 1.00 14.42 B
ATOM 7104 CD1 TYR B 181 121.426 61.173 159.728 1.00 13.42 B
ATOM 7105 CE1 TYR B 181 121.086 60.175 158.806 1.00 14.77 B
ATOM 7106 CD2 TYR B 181 123.700 60.418 159.714 1.00 13.01 B
ATOM 7107 CE2 TYR B 181 123.374 59.420 158.796 1.00 13.91 B
ATOM 7108 CZ TYR B 181 122.065 59.302 158.349 1.00 15.72 B
ATOM 7109 OH TYR B 181 121.734 58.299 157.469 1.00 14.86 B
ATOM 7110 C TYR B 181 123.607 64.790 161.588 1.00 16.80 B
ATOM 7111 O TYR B 181 122.556 64.943 162.206 1.00 15.66 B
ATOM 7112 N ALA B 182 124.650 65.608 161.724 1.00 18.19 B
ATOM 7113 CA ALA B 182 124.598 66.764 162.626 1.00 19.16 B
ATOM 7114 CB ALA B 182 125.916 67.534 162.582 1.00 18.76 B
ATOM 7115 C ALA B 182 123.451 67.690 162.228 1.00 19.14 B
ATOM 7116 O ALA B 182 123.415 68.200 161.106 1.0019.30 B
ATOM 7117 N LYS B 183 122.521 67.911 163.149 1.00 17.81 B
ATOM 7118 CA LYS B 183 121.376 68.765 162.870 1.00 19.40 B
ATOM 7119 CB LYS B 183 120.115 68.151 163.489 1.0019.24 B
ATOM 7120 CG LYS B 183 119.757 66.812 162.840 1.0020.60 B
ATOM 7121 CD LYS B 183 118.594 66.117 163.529 1.0023.44 B
ATOM 7122 CE LYS B 183 118.257 64.796 162.839 1.00 25.63 B
ATOM 7123 NZ LYS B 183 117.143 64.087 163.545 1.00 30.59 B
ATOM 7124 C LYS B 183 121.588 70.203 163.334 1.00 18.78 B
ATOM 7125 O LYS B 183 120.715 71.057 163.174 1.00 17.85 B
ATOM 7126 N ALA B 184 122.760 70.457 163.908 1.00 17.71 B
ATOM 7127 CA ALA B 184 123.143 71.791 164.354 1.00 17.22 B
ATOM 7128 CB ALA B 184 122.600 72.071 165.753 1.00 16.87 B
ATOM 7129 C ALA B 184 124.668 71.823 164.351 1.00 17.17 B
ATOM 7130 O ALA B 184 125.305 70.798 164.579 1.0017.31 B
ATOM 7131 N VAL B 185 125.246 72.986 164.064 1.0017.09 B
ATOM 7132 CA VAL B 185 126.701 73.140 164.034 1.00 17.99 B
ATOM 7133 CB VAL B 185 127.281 72.975 162.592 1.00 18.14 B
ATOM 7134 CG1 VAL B 185 127.061 71.565 162.085 1.00 17.26 B
ATOM 7135 CG2 VAL B 185 126.633 73.986 161.654 1.00 18.71 B
ATOM 7136 C VAL B 185 127.120 74.523 164.529 1.00 17.75 B
ATOM 7137 O VAL B 185 126.303 75.436 164.604 1.00 19.10 B
ATOM 7138 N THR B 186 128.400 74.662 164.858 1.00 17.64 B
ATOM 7139 CA THRB 186 128.965 75.929 165.311 1.00 16.39 B
ATOM 7140 CB THR B 186 130.270 75.709 166.081 1.00 17.35 B
ATOM 7141 OG1 THR B 186 131.217 75.044 165.231 1.00 17.81 B
ATOM 7142 CG2 THR B 186 130.022 74.869 167.323 1.00 16.19 B
ATOM 7143 C THR B 186 129.286 76.779 164.079 1.00 17.93 B
ATOM 7144 O THR B 186 129.147 76.313 162.937 1.00 16.27 B
ATOM 7145 N VAL B 187 129.736 78.013 164.306 1.00 16.38 B
ATOM 7146 CA VAL B 187 130.053 78.911 163.201 1.00 15.40 B
ATOM 7147 CB VAL B 187 130.651 80.254 163.722 1.00 14.42 B
ATOM 7148 CG1 VAL B 187 132.011 80.026 164.370 1.00 11.02 B
ATOM 7149 CG2 VAL B 187 130.739 81.253 162.582 1.00 9.66 B
ATOM 7150 C VAL B 187 131.000 78.258 162.183 1.00 16.18 B
ATOM 7151 O VAL B 187 131.994 77.635 162.550 1.00 15.48 B
ATOM 7152 N ASN B 188 130.669 78.403 160.900 1.00 16.26 B
ATOM 7153 CA ASN B 188 131.451 77.823 159.809 1.00 16.51 B
ATOM 7154 CB ASN B 188 132.875 78.405 159.762 1.00 14.77 B
ATOM 7155 CG ASN B 188 132.920 79.792 159.141 1.00 15.98 B
ATOM 7156 OD1 ASN B 188 132.170 80.090 158.213 1.00 17.36 B
ATOM 7157 ND2 ASN B 188 133.813 80.638 159.635 1.00 15.34 B
ATOM 7158 C ASN B 188 131.526 76.294 159.860 1.00 17.06 B
ATOM 7159 O ASN B 188 132.489 75.700 159.378 1.00 17.20 B
ATOM 7160 N GLY B 189 130.506 75.673 160.449 1.00 18.04 B
ATOM 7161 CA GLY B 189 130.427 74.219 160.537 1.00 17.83 B
ATOM 7162 C GLY B 189 131.621 73.423 161.049 1.00 19.35 B
ATOM 7163 O GLY B 189 131.844 72.291 160.615 1.00 18.38 B
ATOM 7164 N GLU B 190 132.374 73.989 161.984 1.00 19.14 B
ATOM 7165 CA GLU B 190 133.543 73.310 162.527 1.00 20.54 B
ATOM 7166 CB GLU B 190 134.422 74.322 163.270 1.00 23.36 B
ATOM 7167 CG GLU B 190 134.669 75.582 162.440 1.00 30.35 B
ATOM 7168 CD GLU B 190 135.686 76.531 163.053 1.00 34.40 B
ATOM 7169 OE1 GLU B 190 135.575 76.845 164.259 1.00 36.95 B
ATOM 7170 OE2 GLU B 190 136.594 76.975 162.318 1.00 36.90 B
ATOM 7171 C GLU B 190 133.170 72.141 163.439 1.00 18.70 B
ATOM 7172 O0 GLU B 190 133.844 71.112 163.447 1.00 18.65 B
ATOM 7173 N LYS B 191 132.096 72.287 164.200 1.00 15.38 B
ATOM 7174 CA LYS B 191 131.676 71.211 165.085 1.00 16.52 B
ATOM 7175 CB LYS B 191 132.073 71.506 166.536 1.00 17.82 B
ATOM 7176 CG LYS B 191 133.564 71.541 166.843 1.00 18.61 B
ATOM 7177 CD LYS B 191 133.757 71.885 168.323 1.00 21.19 B
ATOM 7178 CE LYS B 191 135.206 71.784 168.767 1.00 23.51 B
ATOM 7179 NZ LYS B 191 136.087 72.657 167.956 1.00 25.80 B
ATOM 7180 C LYS B 191 130.175 71.021 165.048 1.00 15.35 B
ATOM 7181 O LYS B 191 129.432 71.951 164.748 1.00 18.65 B
ATOM 7182 N GLY B 192 129.732 69.811 165.360 1.0014.39 B
ATOM 7183 CA GLY B 192 128.310 69.548 165.414 1.00 14.07 B
ATOM 7184 C GLY B 192 127.861 70.023 166.786 1.00 16.93 B
ATOM 7185 O GLY B 192 128.694 70.229 167.676 1.00 16.68 B
ATOM 7186 N VALB 193 126.560 70.212 166.971 1.00 17.43 B
ATOM 7187 CA VAL B 193 126.048 70.665 168.257 1.00 17.77 B
ATOM 7188 CB VAL B 193 125.768 72.179 168.251 1.00 17.46 B
ATOM 7189 CG1 VAL B 193 125.254 72.612 169.614 1.00 16.09 B
ATOM 7190 CG2 VAL B 193 127.038 72.949 167.881 1.00 18.13 B
ATOM 7191 C VAL B 193 124.756 69.953 168.614 1.00 18.73 B
ATOM 7192 O VALB 193 123.858 69.836 167.785 1.00 19.61 B
ATOM 7193 N VAL B 194 124.675 69.475 169.850 1.00 19.43 B
ATOM 7194 CA VALB 194 123.484 68.794 170.337 1.00 19.78 B
ATOM 7195 CB VALB 194 123.829 67.824 171.494 1.00 19.88 B
ATOM 7196 CG1 VAL B 194 122.572 67.106 171.967 1.00 17.52 B
ATOM 7197 CG2 VAL B 194 124.884 66.812 171.032 1.00 19.86 B
ATOM 7198 C VAL B 194 122.530' 69.870 170.849 1.00 21.39 B
ATOM 7199 O VALB 194 122.923 70.736 171.641 1.00 19.96 B
ATOM 7200 N LEU B 195 121.288 69.834 170.381 1.00 21.79 B
ATOM 7201 CA LEU B 195 120.292 70.815 170.807 1.00 23.65 B
ATOM 7202 CB LEU B 195 120.060 71.874 169.716 1.00 21.40 B
ATOM 7203 CG LEU B 195 121.194 72.835 169.322 1.00 22.37 B
ATOM 7204 CD1 LEU B 195 120.683 73.813 168.267 1.00 19.33 B
ATOM 7205 CD2 LEU B 195 121.683 73.603 170.540 1.00 19.26 B
ATOM 7206 C LEU B 195 118.970 70.135 171.122 1.00 24.60 B
ATOM 7207 O LEU B 195 118.531 69.253 170.392 1.00 24.84 B
ATOM 7208 N ARG B 196 118.342 70.531 172.223 1.0026.56 B
ATOM 7209 CA ARG B 196 117.053 69.958 172.577 1.00 26.86 B
ATOM 7210 CB ARG B 196 116.630 70.393 173.981 1.00 26.58 B
ATOM 7211 CG ARG B 196 116.295 71.876 174.106 1.00 29.78 B
ATOM 7212 CD ARG B 196 115.542 72.130 175.405 1.00 31.39 B
ATOM 7213 NE ARG B 196 116.341 71.776 176.576 1.00 32.41 B
ATOM 7214 CZ ARG B 196 117.322 72.532 177.062 1.00 33.15 B
ATOM 7215 NH1 ARG B 196 117.618 73.683 176.475 1.00 32.39 B
ATOM 7216 NH2 ARG B 196 118.004 72.139 178.131 1.00 32.42 B
ATOM 7217 C ARG B 196 116.060 70.510 171.561 1.00 25.83 B
ATOM 7218 O ARG B 196 116.298 71.563 170.967 1.0025.88 B
ATOM 7219 N PRO B 197 114.948 69.800 171.326 1.00 25.94 B
ATOM 7220 CD PRO B 197 114.571 68.452 171.794 1.00 27.19 B
ATOM 7221 CA PRO B 197 113.974 70.314 170.359 1.00 27.19 B
ATOM 7222 CB PRO B 197 112.813 69.333 170.496 1.00 26.20 B
ATOM 7223 CG PRO B 197 113.531 68.031 170.770 1.00 25.50 B
ATOM 7224 C PRO B 197 113.590 71.742 170.741 1.00 27.42 B
ATOM 7225 O PRO B 197 113.366 72.026 171.918 1.00 26.65 B
ATOM 7226 N ASP B 198 113.533 72.636 169.758 1.00 29.77 B
ATOM 7227 CA ASP B 198 113.190 74.030 170.025 1.00 32.40 B
ATOM 7228 CB ASP B 198 113.764 74.949 168.930 1.00 34.44 B
ATOM 7229 CG ASP B 198 113.253 74.611 167.533 1.00 37.95 B
ATOM 7230 OD1 ASP B 198 112.024 74.675 167.309 1.00 39.17 B
ATOM 7231 OD2 ASP B 198 114.086 74.292 166.653 1.00 37.92 B
ATOM 7232 C ASP B 198 111.691 74.280 170.207 1.00 32.91 B
ATOM 7233 O ASP B 198 110.864 73.395 169.993 1.00 32.65 B
ATOM 7234 N GLN B 199 111.366 75.506 170.604 1.00 35.86 B
ATOM 7235 CA GLN B 199 109.999 75.943 170.881 1.00 37.84 B
ATOM 7236 CB GLN B 199 110.042 76.960 172.031 1.00 39.47 B
ATOM 7237 CG GLN B 199 108.693 77.430 172.558 1.00 44.37 B
ATOM 7238 CD GLN B 199 108.200 76.612 173.734 1.00 46.54 B
ATOM 7239 OE1 GLN B 199 108.029 75.396 173.633 1.00 45.78 B
ATOM 7240 NE2 GLN B 199 107.964 77.281 174.863 1.00 47.31 B
ATOM 7241 C GLN B 199 109.272 76.559 169.682 1.00 38.00 B
ATOM 7242 O GLN B 199 108.592 77.573 169.824 1.00 40.11 B
ATOM 7243 N MET B 200 109.393 75.952 168.508 1.00 37.68 B
ATOM 7244 CA MET B 200 108.733 76.490 167.317 1.00 36.20 B
ATOM 7245 CB MET B 200 109.419 75.971 166.053 1.00 35.53 B
ATOM 7246 CG MET B 200 108.779 76.454 164.756 1.00 34.40 B
ATOM 7247 SD MET B 200 108.884 78.246 164.526 1.00 32.78 B
ATOM 7248 CE MET B 200 107.507 78.519 163.422 1.00 33.77 B
ATOM 7249 C MET B 200 107.237 76.186 167.226 1.00 35.66 B
ATOM 7250 O MET B 200 106.815 75.037 167.349 1.00 34.90 B
ATOM 7251 N LYS B 201 106.442 77.226 166.998 1.00 35.12 B
ATOM 7252 CA LYS B 201 104.998 77.067 166.863 1.00 35.81 B
ATOM 7253 CB LYS B 201 104.260 78.087 167.741 1.00 38.52 B
ATOM 7254 CG LYS B 201 102.743 78.059 167.565 1.00 42.89 B
ATOM 7255 CD LYS B 201 102.002 78.776 168.697 1.00 47.09 B
ATOM 7256 CE LYS B 201 102.261 80.282 168.714 1.00 48.03 B
ATOM 7257 NZ LYS B 201 101.551 80.947 169.849 1.0046.03 B
ATOM 7258 C LYS B 201 104.585 77.252 165.402 1.00 34.16 B
ATOM 7259 O LYS B 201 104.478 78.381 164.924 1.00 32.64 B
ATOM 7260 N TRP B 202 104.360 76.143 164.697 1.00 33.01 B
ATOM 7261 CA TRP B 202 103.958 76.205 163.292 1.00 32.30 B
ATOM 7262 CB TRP B 202 104.188 74.864 162.584 1.0032.19 B
ATOM 7263 CG TRP B 202 105.606 74.395 162.570 1.0031.76 B
ATOM 7264 CD2 TRP B 202 106.607 74.721 161.597 1.00 30.41 B
ATOM 7265 CE2 TRP B 202 107.799 74.073 161.991 1.00 30.81 B
ATOM 7266 CE3 TRP B 202 106.614 75.501 160.432 1.00 30.64 B
ATOM 7267 CD1 TRP B 202 106.212 73.586 163.489 1.00 30.57 B
ATOM 7268 NE1 TRP B 202 107.531 73.389 163.148 1.0031.26 B
ATOM 7269 CZ2 TRP B 202 108.989 74.181 161.260 1.00 29.11 B
ATOM 7270 CZ3 TRP B 202 107.799 75.611 159.705 1.0029.06 B
ATOM 7271 CH2 TRP B 202 108.969 74.952 160.125 1.00 28.09 B
ATOM 7272 C TRP B 202 102.483 76.554 163.169 1.00 32.07 B
ATOM 7273 O TRP B 202 101.692 76.225 164.049 1.00 33.51 B
ATOM 7274 N THR B 203 102.117 77.219 162.077 1.00 31.57 B
ATOM 7275 CA THR B 203 100.726 77.579 161.840 1.00 29.59 B
ATOM 7276 CB THR B 203 100.600 78.692 160.773 1.00 29.98 B
ATOM 7277 OG1 THR B 203 99.228 79.087 160.661 1.00 30.90 B
ATOM 7278 CG2 THR B 203 101.098 78.206 159.413 1.00 29.59 B
ATOM 7279 C THR B 203 99.998 76.320 161.361 1.00 29.67 B
ATOM 7280 O THR B 203 100.617 75.269 161.200 1.00 28.02 B
ATOM 7281 N ALA B 204 98.693 76.421 161.137 1.00 29.26 B
ATOM 7282 CA ALA B 204 97.913 75.271 160.698 1.00 29.74 B
ATOM 7283 CB ALA B 204 96.455 75.677 160.484 1.00 28.83 B
ATOM 7284 C ALA B 204 98.485 74.655 159.419 1.0030.47 B
ATOM 7285 O ALA B 204 98.828 75.362 158.472 1.00 31.26 B
ATOM 7286 N PRO B 205 98.593 73.320 159.384 1.0029.63 B
ATOM 7287 CD PRO B 205 98.249 72.399 160.482 1.00 31.10 B
ATOM 7288 CA PRO B 205 99.121 72.582 158.235 1.00 29.29 B
ATOM 7289 CB PRO B 205 98.949 71.122 158.652 1.00 29.83 B
ATOM 7290 CG PRO B 205 99.073 71.176 160.139 1.00 31.08 B
ATOM 7291 C PRO B 205 98.368 72.887 156.950 1.00 29.51 B
ATOM 7292 O PRO B 205 97.136 72.947 156.945 1.00 28.19 B
ATOM 7293 N LEU B 206 99.113 73.085 155.866 1.00 28.18 B
ATOM 7294 CA LEU B 206 98.513 73.355 154.569 1.00 26.09 B
ATOM 7295 CB LEU B 206 99.538 73.960 153.613 1.00 24.86 B
ATOM 7296 CG LEU B 206 100.200 75.281 153.989 1.00 26.52 B
ATOM 7297 CD1 LEU B 206 101.124 75.716 152.861 1.00 26.17 B
ATOM 7298 CD2 LEU B 206 99.138 76.336 154.239 1.00 27.12 B
ATOM 7299 C LEU B 206 98.054 72.021 154.008 1.00 26.12 B
ATOM 7300 O LEU B 206 98.708 71.003 154.220 1.00 25.07 B
ATOM 7301 N LYS B 207 96.925 72.015153.307 1.0026.80 B
ATOM 7302 CA LYS B 207 96.436 70.784 152.713 1.00 28.34 B
ATOM 7303 CB LYS B 207 95.012 70.965 152.171 1.00 32.02 B
ATOM 7304 CG LYS B 207 94.830 72.057 151.116 1.00 36.03 B
ATOM 7305 CD LYS B 207 94.594 73.440 151.737 1.0040.52 B
ATOM 7306 CE LYS B 207 95.893 74.156 152.085 1.00 38.59 B
ATOM 7307 NZ LYS B 207 95.641 75.448 152.758 1.0040.13 B
ATOM 7308 C LYS B 207 97.406 70.437 151.587 1.00 28.08 B
ATOM 7309 O LYS B 207 98.108 71.311 151.079 1.00 27.60 B
ATOM 7310 N PRO B 208 97.472 69.156 151.190 1.00 27.39 B
ATOM 7311 CD PRO B 208 96.585 68.038 151.549 1.00 27.08 B
ATOM 7312 CA PRO B 208 98.390 68.768 150.115 1.00 26.21 B
ATOM 7313 CB PRO B 208 98.079 67.285 149.910 1.00 26.82 B
ATOM 7314 CG PRO B 208 96.636 67.184 150.304 1.00 26.92 B
ATOM 7315 C PRO B 208 98.163 69.591 148.858 1.00 25.34 B
ATOM 7316 O PRO B 208 97.023 69.856 148.479 1.00 24.75 B
ATOM 7317 N PHE B 209 99.250 70.007 148.220 1.00 23.12 B
ATOM 7318 CA PHE B 209 99.124 70.795 147.014 1.00 20.66 B
ATOM 7319 CB PHE B 209 100.496 71.239 146.514 1.00 19.03 B
ATOM 7320 CG PHE B 209 100.427 72.307 145.468 1.00 18.10 B
ATOM 7321 CD1 PHE B 209 100.740 72.028 144.145 1.00 16.83 B
ATOM 7322 CD2 PHE B 209 99.988 73.588 145.801 1.00 16.63 B
ATOM 7323 CE1 PHE B 209 100.616 73.013 143.165 1.00 16.52 B
ATOM 7324 CE2 PHE B 209 99.860 74.573 144.835 1.00 14.47 B
ATOM 7325 CZ PHE B 209 100.175 74.285 143.511 1.00 15.30 B
ATOM 7326 C PHE B 209 98.410 69.943 145.971 1.00 20.12 B
ATOM 7327 O PHE B 209 98.757 68.787 145.752 1.00 19.74 B
ATOM 7328 N SER B 210 97.397 70.533 145.352 1.00 21.22 B
ATOM 7329 CA SER B 210 96.561 69.882 144.347 1.0021.56 B
ATOM 7330 CB SER B 210 95.676 70.929 143.676 1.00 21.15 B
ATOM 7331 OG SER B 210 96.468 71.887 142.988 1.00 21.28 B
ATOM 7332 C SER B 210 97.245 69.068 143.251 1.00 21.69 B
ATOM 7333 O SER B 210 97.013 67.865 143.131 1.00 22.73 B
ATOM 7334 N HIS B 211 98.075 69.725 142.447 1.00 21.01 B
ATOM 7335 CA HIS B 211 98.738 69.056 141.328 1.00 19.13 B
ATOM 7336 CB HIS B 211 97.717 68.874 140.197 1.00 21.08 B
ATOM 7337 CG HIS B 211 98.196 68.018 139.066 1.00 21.49 B
ATOM 7338 CD2 HIS B 211 98.964 68.310 137.990 1.00 20.95 B
ATOM 7339 ND1 HIS B 211 97.865 66.684 138.950 1.00 21.02 B
ATOM 7340 CE1 HIS B 211 98.408 66.193 137.849 1.00 21.48 B
ATOM 7341 NE2 HIS B 211 99.079 67.159 137.248 1.00 22.65 B
ATOM 7342 C HIS B 211 99.905 69.910 140.827 1.00 18.75 B
ATOM 7343 O HIS B 211 99.820 71.142 140.816 1.00 19.33 B
ATOM 7344 N PRO B 212 101.001 69.269 140.381 1.00 17.55 B
ATOM 7345 CD PRO B 212 101.276 67.818 140.357 1.00 17.16 B
ATOM 7346 CA PRO B 212 102.156 70.025 139.886 1.00 16.83 B
ATOM 7347 CB PRO B 212 103.069 68.932 139.334 1.00 17.14 B
ATOM 7348 CG PRO B 212 102.782 67.772 140.268 1.00 15.78 B
ATOM 7349 C PRO B 212 101.839 71.113 138.854 1.00 16.08 B
ATOM 7350 O PRO B 212 102.360 72.222 138.960 1.00 16.72 B
ATOM 7351 N VALB 213 100.990 70.820 137.869 1.00 16.13 B
ATOM 7352 CA VAL B 213 100.670 71.828 136.852 1.00 17.77 B
ATOM 7353 CB VAL B 213 99.857 71.239 135.667 1.00 17.65 B
ATOM 7354 CG1 VAL B 213 100.598 70.045 135.072 1.00 19.14 B
ATOM 7355 CG2 VAL B 213 98.458 70.861 136.119 1.00 14.99 B
ATOM 7356 C VAL B 213 99.906 73.033 137.407 1.00 17.62 B
ATOM 7357 O VAL B 213 99.655 74.000 136.685 1.0017.52 B
ATOM 7358 N ASP B 214 99.536 72.967 138.683 1.00 16.79 B
ATOM 7359 CA ASP B 214 98.827 74.066 139.334 1.00 17.44 B
ATOM 7360 CB ASP B 214 97.812 73.525 140.352 1.00 18.47 B
ATOM 7361 CG ASP B 214 96.607 72.866 139.691 1.00 19.37 B
ATOM 7362 OD1 ASP B 214 95.885 72.121 140.385 1.00 21.93 B
ATOM 7363 OD2 ASP B 214 96.373 73.099 138.487 1.00 17.14 B
ATOM 7364 C ASP B 214 99.825 74.969 140.057 1.00 17.60 B
ATOM 7365 O ASP B 214 99.442 75.975 140.661 1.00 17.20 B
ATOM 7366 N ALA B 215 101.105 74.607 139.993 1.0016.30 B
ATOM 7367 CA ALA B 215 102.150 75.381 140.651 1.00 16.57 B
ATOM 7368 CB ALA B 215 103.215 74.438 141.217 1.00 17.52 B
ATOM 7369 C ALA B 215 102.821 76.414 139.754 1.00 16.74 B
ATOM 7370 O ALA B 215 102.847 76.282 138.531 1.00 16.41 B
ATOM 7371 N VAL B 216 103.349 77.453 140.391 1.00 17.22 B
ATOM 7372 CA VAL B 216 104.086 78.520 139.715 1.00 16.64 B
ATOM 7373 CB VAL B 216 103.277 79.830 139.623 1.00 16.51 B
ATOM 7374 CG1 VAL B 216 104.079 80.883 138.856 1.00 15.02 B
ATOM 7375 CG2 VAL B 216 101.953 79.575 138.921 1.00 16.79 B
ATOM 7376 C VAL B 216 105.280 78.716 140.641 1.00 16.17 B
ATOM 7377 O VAL B 216 105.141 79.246 141.738 1.00 14.85 B
ATOM 7378 N ILE B 217 106.443 78.254 140.198 1.00 15.86 B
ATOM 7379 CA ILE B 217 107.665 78.316 140.990 1.00 15.41 B
ATOM 7380 CB ILE B 217 108.533 77.068 140.676 1.00 15.41 B
ATOM 7381 CG2 ILE B 217 109.749 77.021 141.580 1.00 12.66 B
ATOM 7382 CG1 ILE B 217 107.684 75.802 140.870 1.00 14.08 B
ATOM 7383 CD1 ILE B 217 108.290 74.526 140.291 1.00 13.41 B
ATOM 7384 C ILE B 217 108.469 79.607 140.785 1.00 16.60 B
ATOM 7385 O ILE B 217 108.600 80.112 139.667 1.00 16.57 B
ATOM 7386 N TYR B 218 109.000 80.134 141.882 1.00 15.66 B
ATOM 7387 CA TYR B 218 109.772 81.371 141.864 1.00 15.30 B
ATOM 7388 CB TYR B 218 109.009 82.455 142.650 1.00 13.44 B
ATOM 7389 CG TYR B 218 109.527 83.871 142.478 1.00 12.59 B
ATOM 7390 CD1 TYR B 218 110.111 84.548 143.541 1.00 13.81 B
ATOM 7391 CE1 TYR B 218 110.563 85.861 143.407 1.0013.79 B
ATOM 7392 CD2 TYR B 218 109.408 84.542 141.259 1.00 13.83 B
ATOM 7393 CE2 TYR B 218 109.859 85.862 141.115 1.00 15.36 B
ATOM 7394 CZ TYR B 218 110.434 86.509 142.201 1.00 13.44 B
ATOM 7395 OH TYR B 218 110.873 87.805 142.095 1.0015.59 B
ATOM 7396 C TYR B 218 111-115 81.065 142.521 1.00 15.82 B
ATOM 7397 O TYR B 218 111.177 80.860 143.734 1.00 16.66 B
ATOM 7398 N GLU B 219 112.179 81.024 141.719 1.00 15.76 B
ATOM 7399 CA GLU B 219 113.514 80.696 142.223 1.00 17.16 B
ATOM 7400 CB GLU B 219 114.357 80.024 141.133 1.00 16.29 B
ATOM 7401 CG GLU B 219 115.625 79.350 141.675 1.0017.50 B
ATOM 7402 CD GLU B 219 116.677 79.079 140.601 1.00 17.76 B
ATOM 7403 OE1 GLU B 219 117.547 78.212 140.832 1.00 16.51 B
ATOM 7404 OE2 GLU B 219 116.646 79.735 139.538 1.00 13.80 B
ATOM 7405 C GLU B 219 114.290 81.892 142.754 1.00 16.73 B
ATOM 7406 O GLU B 219 114.576 82.832 142.014 1.00 17.92 B
ATOM 7407 N THR B 220 114.658 81.846 144.030 1.00 16.50 B
ATOM 7408 CA THR B 220 115.403 82.952 144.613 1.00 17.08 B
ATOM 7409 CB THR B 220 114.451 83.918 145.365 1.00 17.31 B
ATOM 7410 OG1 THR B 220 115.175 85.081 145.776 1.00 20.93 B
ATOM 7411 CG2 THR B 220 113.865 83.249 146.591 1.00 18.22 B
ATOM 7412 C THR B 220 116.518 82.509 145.563 1.00 14.44 B
ATOM 7413 O THR B 220 116.476 81.422 146.139 1.00 14.93 B
ATOM 7414 N HIS B 221 117.523 83.366 145.693 1.00 13.26 B
ATOM 7415 CA HIS B 221 118.661 83.144 146.583 1.00 13.93 B
ATOM 7416 CB HIS B 221 119.957 83.565 145.884 1.00 15.25 B
ATOM 7417 CG HIS B 221 121.187 83.371 146.710 1.00 16.15 B
ATOM 7418 CD2 HIS B 221 121.742 84.141 147.674 1.00 14.72 B
ATOM 7419 ND1 HIS B 221 122.003 82.266 146.586 1.00 19.03 B
ATOM 7420 CE1 HIS B 221 123.007 82.365 147.439 1.00 15.06 B
ATOM 7421 NE2 HIS B 221 122.871 83.494 148.111 1.00 14.76 B
ATOM 7422 C HIS B 221 118.370 84.072 147.760 1.00 14.08 B
ATOM 7423 O HIS B 221 118.092 85.255 147.561 1.00 13.48 B
ATOM 7424 N LEU B 222 118.421 83.543 148.976 1.00 15.15 B
ATOM 7425 CA LEU B 222 118.129 84.338 150.166 1.00 14.89 B
ATOM 7426 CB LEU B 222 118.289 83.475 151.419 1.00 14.90 B
ATOM 7427 CG LEU B 222 117.166 82.455 151.632 1.00 15.09 B
ATOM 7428 CD1 LEU B 222 117.352 81.756 152.978 1.00 14.59 B
ATOM 7429 CD2 LEU B 222 115.816 83.171 151.609 1.00 16.33 B
ATOM 7430 C LEU B 222 118.922 85.639 150.321 1.00 14.98 B
ATOM 7431 O LEU B 222 118.410 86.613 150.870 1.00 16.00 B
ATOM 7432 N ARG B 223 120.161 85.667 149.843 1.00 14.91 B
ATOM 7433 CA ARG B 223 120.964 86.882 149.946 1.00 16.55 B
ATOM 7434 CB ARG B 223 122.447 86.553 149.781 1.00 16.62 B
ATOM 7435 CG ARG B 223 123.403 87.733 149.902 1.00 18.17 B
ATOM 7436 CD ARG B 223 124.789 87.215 150.256 1.00 20.54 B
ATOM 7437 NE ARG B 223 125.854 88.193 150.070 1.00 19.45 B
ATOM 7438 CZ ARG B 223 127.121 87.971 150.411 1.00 21.06 B
ATOM 7439 NH1 ARG B 223 128.045 88.902 150.208 1.00 23.21 B
ATOM 7440 NH2 ARG B 223 127.460 86.820 150.971 1.00 19.02 B
ATOM 7441 C ARG B 223 120.514 87.879 148.879 1.00 17.48 B
ATOM 7442 O ARG B 223 120.216 89.036 149.186 1.00 17.00 B
ATOM 7443 N ASP B 224 120.448 87.418 147.632 1.00 16.22 B
ATOM 7444 CA ASP B 224 120.020 88.265 146.521 1.00 15.14 B
ATOM 7445 CB ASP B 224 119.785 87.437 145.252 1.00 15.12 B
ATOM 7446 CG ASP B 224 121.001 86.670 144.797 1.00 13.74 B
ATOM 7447 OD1 ASP B 224 120.867 85.927 143.803 1.00 15.49 B
ATOM 7448 OD2 ASP B 224 122.077 86.800 145.407 1.00 16.07 B
ATOM 7449 C ASP B 224 118.695 88.951 146.821 1.00 15.90 B
ATOM 7450 O ASP B 224 118.552 90.161 146.684 1.00 17.09 B
ATOM 7451 N PHE B 225 117.720 88.138 147.208 1.00 16.44 B
ATOM 7452 CA PHE B 225 116.366 88.591 147.459 1.00 17.10 B
ATOM 7453 CB PHE B 225 115.547 87.477 148.121 1.00 15.04 B
ATOM 7454 CG PHE B 225 114.090 87.801 148.218 1.00 14.53 B
ATOM 7455 CD1 PHE B 225 113.286 87.768 147.083 1.00 13.75 B
ATOM 7456 CD2 PHE B 225 113.539 88.232 149.420 1.00 16.15 B
ATOM 7457 CE1 PHEB225 111.949 88.167 147.138 1.00 16.62 B
ATOM 7458 CE2 PHE B 225 112.203 88.634 149.489 1.00 16.18 B
ATOM 7459 CZ PHE B 225 111.405 88.602 148.344 1.00 14.70 B
ATOM 7460 C PHE B 225 116.157 89.875 148.246 1.00 18.40 B
ATOM 7461 O PHE B 225 115.240 90.633 147.942 1.00 20.28 B
ATOM 7462 N SER B 226 117.002 90.141 149.235 1.00 19.67 B
ATOM 7463 CA SER B 226 116.807 91.326 150.065 1.00 19.15 B
ATOM 7464 CB SER B 226 116.174 90.894 151.387 1.00 17.70 B
ATOM 7465 OG SER B 226 117.001 89.936 152.034 1.00 17.91 B
ATOM 7466 C SER B 226 118.038 92.183 150.366 1.00 20.01 B
ATOM 7467 O SER B 226 117.92593.211151.033 1.00 20.39 B
ATOM 7468 N ILE B 227 119.206 91.784 149.881 1.00 21.21 B
ATOM 7469 CA ILE B 227 120.415 92.549 150.165 1.0021.85 B
ATOM 7470 CB ILE B 227 121.669 91.807 149.649 1.00 20.50 B
ATOM 7471 CG2 ILE B 227 121.698 91.801 148.140 1.00 16.14 B
ATOM 7472 CG1 ILE B 227 122.928 92.469 150.212 1.00 21.58 B
ATOM 7473 CD1 ILE B 227 123.068 92.311 151.698 1.00 19.65 B
ATOM 7474 C ILE B 227 120.423 93.994 149.628 1.00 23.50 B
ATOM 7475 O ILE B 227 121.022 94.879 150.238 1.00 24.57 B
ATOM 7476 N HIS B 228 119.756 94.230 148.501 1.00 24.56 B
ATOM 7477 CA HIS B 228 119.701 95.565 147.892 1.00 25.15 B
ATOM 7478 CB HIS B 228 118.799 95.527 146.654 1.00 24.48 B
ATOM 7479 CG HIS B 228 118.805 96.792 145.854 1.00 26.43 B
ATOM 7480 CD2 HIS B 228 119.376 97.089 144.663 1.00 24.61 B
ATOM 7481 ND1 HIS B 228 118.157 97.938 146.264 1.00 27.34 B
ATOM 7482 CE1 HIS B 228 118.326 98.884 145.358 1.00 27.71 B
ATOM 7483 NE2 HIS B 228 119.062 98.395 144.377 1.00 27.22 B
ATOM 7484 C HIS B 228 119.204 96.618 148.887 1.00 25.43 B
ATOM 7485 O HIS B 228 118.203 96.419 149.573 1.00 25.00 B
ATOM 7486 N GLU B 229 119.909 97.745 148.954 1.00 27.13 B
ATOM 7487 CA GLU B 229 119.569 98.816 149.891 1.00 28.63 B
ATOM 7488 CB GLU B 229 120.547 99.983 149.729 1.00 31.35 B
ATOM 7489 CG GLU B 229 120.711 100.462 148.303 1.00 39.31 B
ATOM 7490 CD GLU B 229 121.455 101.785 148.216 1.00 44.91 B
ATOM 7491 OE1 GLU B 229 122.578 101.879 148.767 1.00 45.17 B
ATOM 7492 OE2 GLU B 229 120.915 102.729 147.593 1.00 47.07 B
ATOM 7493 C GLU B 229 118.138 99.353 149.850 1.00 27.84 B
ATOM 7494 O GLU B 229 117.657 99.889 150.847 1.00 27.87 B
ATOM 7495 N ASN B 230 117.458 99.211 148.715 1.00 27.01 B
ATOM 7496 CA ASN B 230 116.083 99.704 148.579 1.00 26.43 B
ATOM 7497 CB ASN B 230 115.925 100.424 147.230 1.00 27.61 B
ATOM 7498 CG ASN B 230 114.583 101.131 147.083 1.00 29.89 B
ATOM 7499 OD1 ASN B 230 114.160 101.456 145.971 1.00 33.05 B
ATOM 7500 ND2 ASN B 230 113.914 101.382 148.201 1.00 31.24 B
ATOM 7501 C ASN B 230 115.071 98.559 148.672 1.00 26.35 B
ATOM 7502 O ASN B 230 113.906 98.718 148.306 1.00 26.33 B
ATOM 7503 N SER B 231 115.511 97.409 149.172 1.00 25.33 B
ATOM 7504 CA SER B 231 114.637 96.245 149.273 1.00 24.62 B
ATOM 7505 CB SER B 231 115.420 95.028 149.770 1.00 23.62 B
ATOM 7506 OG SER B 231 115.728 95.153 151.145 1.00 26.74 B
ATOM 7507 C SER B 231 113.455 96.484 150.193 1.00 23.94 B
ATOM 7508 O SER B 231 112.423 95.825 150.070 1.0022.74 B
ATOM 7509 N GLY B 232 113.607 97.421 151.123 1.00 23.76 B
ATOM 7510 CA GLY B 232 112.525 97.714 152.045 1.00 23.11 B
ATOM 7511 C GLY B 232 112.412 96.707 153.177 1.00 23.69 B
ATOM 7512 O GLY B 232 111.459 96.745 153.956 1.00 22.90 B
ATOM 7513 N MET B 233 113.376 95.795 153.268 1.0024.23 B
ATOM 7514 CA MET B 233 113.372 94.798 154.330 1.00 23.35 B
ATOM 7515 CB MET B 233 113.535 93.391 153.744 1.00 22.44 B
ATOM 7516 CG MET B 233 112.290 92.895 153.025 1.00 20.77 B
ATOM 7517 SD MET B 233 112.573 91.435 152.001 1.00 18.76 B
ATOM 7518 CE MET B 233 113.261 92.206 150.518 1.00 9.17 B
ATOM 7519 C MET B 233 114.495 95.110 155.303 1.00 23.64 B
ATOM 7520 O MET B 233 115.573 95.533 154.900 1.00 24.13 B
ATOM 7521 N ILE B 234 114.226 94.912 156.589 1.00 24.74 B
ATOM 7522 CA ILE B 234 115.198 95.184 157.645 1.00 25.13 B
ATOM 7523 CB ILE B 234 114.487 95.257 159.023 1.00 25.24 B
ATOM 7524 CG2 ILE B 234 115.506 95.396 160.149 1.00 24.08 B
ATOM 7525 CG1 ILE B 234 113.518 96.444 159.040 1.00 26.01 B
ATOM 7526 CD1 ILE B 234 112.671 96.524 160.301 1.00 26.10 B

ATOM 7527 C ILE B 234 116.321 94.145 157.717 1.00 26.17 B
ATOM 7528 O ILE B 234 117.505 94.497 157.749 1.00 26.18 B
ATOM 7529 N ASN B 235 115.936 92.872 157.745 1.00 25.59 B
ATOM 7530 CA ASN B 235 116.874 91.754 157.834 1.00 24.63 B
ATOM 7531 CB ASN B 235 116.141 90.547 158.419 1.00 23.92 B
ATOM 7532 CG ASN B 235 115.593 90.823 159.809 1.0024.39 B
ATOM 7533 OD1 ASN B 235 116.348 90.908 160.776 1.00 23.49 B
ATOM 7534 ND2 ASN B 235 114.276 90.978 159.911 1.00 20.24 B
ATOM 7535 C ASN B 235 117.471 91.400 156.473 1.0024.30 B
ATOM 7536 O ASN B 235 117.335 90.275 155.994 1.00 25.75 B
ATOM 7537 N LYS B 236 118.150 92.365 155.868 1.00 24.04 B
ATOM 7538 CA LYS B 236 118.738 92.194 154.545 1.00 23.23 B
ATOM 7539 CB LYS B 236 119.427 93.490 154.118 1.00 22.64 B
ATOM 7540 CG LYS B 236 118.459 94.640 153.890 1.00 23.78 B
ATOM 7541 CD LYS B 236 119.163 95.836 153.258 1.00 26.67 B
ATOM 7542 CE LYS B 236 118.160 96.820 152.681 1.00 23.94 B
ATOM 7543 NZ LYS B 236 117.212 97.283 153.711 1.00 24.5 B
ATOM 7544 C LYS B 236 119.701 91.034 154.377 1.00 22.93 B
ATOM 7545 O LYS B 236 120.709 90.943 155.079 1.00 23.31 B
ATOM 7546 N GLY B 237 119.377 90.157 153.427 1.00 21.91 B
ATOM 7547 CA GLY B 237 120.209 89.001 153.134 1.00 19.80 B
ATOM 7548 C GLY B 237 120.089 87.867 154.133 1.00 19.08 B
ATOM 7549 O GLY B 237 120.906 86.940 154.130 1.00 18.59 B
ATOM 7550 N LYS B 238 119.061 87.928 154.974 1.00 17.96 B
ATOM 7551 CA LYS B 238 118.857 86.921 156.008 1.00 18.12 B
ATOM 7552 CB LYS B 238 118.954 87.581 157.384 1.0019.45 B
ATOM 7553 CG LYS B 238 120.206 88.418 157.592 1.0020.97 B
ATOM 7554 CD LYS B 238 121.480 87.581 157.632 1.0020.28 B
ATOM 7555 CE LYS B 238 122.691 88.477 157.880 1.00 24.14 B
ATOM 7556 NZ LYS B 238 123.985 87.739 157.991 1.00 26.38 B
ATOM 7557 C LYS B 238 117.527 86.174 155.911 1.00 18.20 B
ATOM 7558 O LYS B 238 116.617 86.585 155.186 1.00 15.87 B
ATOM 7559 N TYR B 239 117.438 85.077 156.663 1.00 17.07 B
ATOM 7560 CA TYR B 239 116.252 84.230 156.714 1.00 16.83 B
ATOM 7561 CB TYR B 239 116.404 83.150 157.799 1.00 15.96 B
ATOM 7562 CG TYR B 239 117.408 82.054 157.505 1.00 16.43 B
ATOM 7563 CD1 TYR B 239 118.467 81.802 158.374 1.00 15.56 B
ATOM 7564 CE1 TYR B 239 119.386 80.790 158.113 1.00 18.79 B
ATOM 7565 CD2 TYR B 239 117.292 81.263 156.365 1.00 13.82 B
ATOM 7566 CE2 TYR B 239 118.201 80.252 156.092 1.00 14.46 B
ATOM 7567 CZ TYR B 239 119.248 80.017 156.965 1.00 18.48 B
ATOM 7568 OH TYR B 239 120.166 79.019 156.685 1.00 16.31 B
ATOM 7569 C TYRB239 115.014 85.051 157.048 1.00 19.18 B
ATOM 7570 O TYR B 239 113.964 84.897 156.421 1.00 19.34 B
ATOM 7571 N LEU B 240 115.148 85.922 158.044 1.00 18.12 B
ATOM 7572 CA LEU B 240 114.031 86.738 158.506 1.00 18.44 B
ATOM 7573 CB LEU B 240 114.409 87.443 159.813 1.00 17.20 B
ATOM 7574 CG LEU B 240 114.556 86.503 161.015 1.00 18.52 B
ATOM 7575 CD1 LEU B 240 114.982 87.287 162.266 1.00 20.39 B
ATOM 7576 CD2 LEU B 240 113.232 85.802 161.258 1.00 3.90 B
ATOM 7577 C LEU B 240 113.464 87.755 157.528 1.00 17.88 B
ATOM 7578 O LEU B 240 112.331 88.192 157.696 1.00 19.63 B
ATOM 7579 N ALA B 241 114.226 88.123 156.503 1.00 17.13 B
ATOM 7580 CA ALA B 241 113.741 89.110 155.545 1.00 17.18 B
ATOM 7581 CB ALA B 241 114.732 89.263 154.401 1.00 16.55 B
ATOM 7582 C ALA B 241 112.364 88.737 155.002 1.0018.96 B
ATOM 7583 O ALA B 241 111.493 89.600 154.830 1.00 18.60 B
ATOM 7584 N LEU B 242 112.166 87.445 154.756 1.00 18.09 B
ATOM 7585 CA LEU B 242 110.909 86.946 154.215 1.00 18.63 B
ATOM 7586 CB LEU B 242 111.148 85.599 153.523 1.00 20.34 B
ATOM 7587 CG LEU B 242 110.799 85.451 152.036 1.00 21.61 B
ATOM 7588 CD1 LEU B 242 110.366 86.783 151.426 1.00 19.85 B
ATOM 7589 CD2 LEU B 242 112.006 84.880 151.305 1.00 20.22 B
ATOM 7590 C LEU B 242 109.757 86.823 155.221 1.00 19.82 B
ATOM 7591 O LEU B 242 108.676 86.363 154.863 1.00 17.37 B
ATOM 7592 N THR B 243 109.985 87.221 156.473 1.00 20.45 B
ATOM 7593 CA THR B 243 108.924 87.192 157.482 1.00 21.01 B
ATOM 7594 CB THR B 243 109.449 86.828 158.897 1.00 21.07 B
ATOM 7595 OG1 THR B 243 110.420 87.796 159.312 1.00 20.06 B
ATOM 7596 CG2 THR B 243 110.087 85.444 158.907 1.00 23.25 B
ATOM 7597 C THR B 243 108.316 88.597 157.563 1.00 22.45 B
ATOM 7598 O THR B 243 107.248 88.790 158.141 1.00 24.00 B
ATOM 7599 N GLU B 244 109.001 89.572 156.972 1.00 21.40 B
ATOM 7600 CA GLU B 244 108.550 90.958 156.995 1.00 22.07 B
ATOM 7601 CB GLU B 244 109.725 91.884 156.660 1.00 19.84 B
ATOM 7602 CG GLU B 244 110.895 91.714 157.625 1.00 19.83 B
ATOM 7603 CD GLU B 244 112.063 92.635 157.324 1.00 20.65 B
ATOM 7604 OE1 GLU B 244 111.830 93.848 157.142 1.00 22.78 B
ATOM 7605 OE2 GLU B 244 113.217 92.150 157.285 1.00 18.08 B
ATOM 7606 C GLU B 244 107.379 91.218 156.052 1.00 23.27 B
ATOM 7607 O GLU B 244 107.513 91.132 154.827 1.00 22.49 B
ATOM 7608 N THR B 245 106.231 91.549 156.639 1.00 23.75 B
ATOM 7609 CA THR B 245 105.013 91.811 155.876 1.00 26.55 B
ATOM 7610 CB THR B 245 103.763 91.481 156.707 1.00 28.21 B
ATOM 7611 OG1 THR B 245 103.752 92.292 157.892 1.00 26.67 B
ATOM 7612 CG2 THR B 245 103.749 90.003 157.095 1.00 28.52 B
ATOM 7613 C THR B 245 104.859 93.249 155.378 1.00 27.52 B
ATOM 7614 O THR B 245 105.409 94.190 155.955 1.00 26.46 B
ATOM 7615 N ASP B 246 104.097 93.395 154.297 1.00 28.33 B
ATOM 7616 CA ASP B 246 103.804 94.691 153.695 1.00 29.60 B
ATOM 7617 CB ASP B 246 102.746 95.401 154.530 1.00 28.41 B
ATOM 7618 CG ASP B 246 101.457 94.627 154.581 1.00 29.42 B
ATOM 7619 OD1 ASP 246 100.996 94.316 155.695 1.00 32.60 B
ATOM 7620 OD2 ASP B 246 100.912 94.321 153.500 1.00 29.24 B
ATOM 7621 C ASP B 246 105.002 95.597 153.501 1.00 30.39 B
ATOM 7622 O ASP B 246 104.943 96.803 153.754 1.00 31.78 B
ATOM 7623 N THR B 247 106.088 95.007 153.034 1.00 30.02 B
ATOM 7624 CA THR B 247 107.308 95.746 152.788 1.00 30.34 B
ATOM 7625 CB THR B 247 108.511 94.788 152.811 1.00 29.65 B
ATOM 7626 OG1 THR B 247 108.819 94.459 154.168 1.00 31.11 B
ATOM 7627 CG2 THR B 247 109.71595.416152.1681.0032.66 B
ATOM 7628 C THR B 247 107.217 96.440 151.435 1.00 30.39 B
ATOM 7629 O THR B 247 106.487 95.994 150.549 1.00 29.22 B
ATOM 7630 N GLN B 248 107.950 97.534 151.275 1.00 30.69 B
ATOM 7631 CA GLN B 248 107.933 98.240 150.007 1.00 33.83 B
ATOM 7632 CB GLN B 248 106.685 99.124 149.911 1.00 35.56 B
ATOM 7633 CG GLN B 248 106.604 100.240 150.928 1.00 38.38 B
ATOM 7634 CD GLN B 248 105.289 100.997 150.834 1.00 41.98 B
ATOM 7635 OE1 GLN B 248 105.131 102.069 151.421 1.00 44.54 B
ATOM 7636 NE2 GLN B 248 104.333 100.436 150.097 1.00 42.18 B
ATOM 7637 C GLN B 248 109.188 99.067 149.769 1.00 33.53 B
ATOM 7638 O GLN B 248 109.909 99.412 150.707 1.00 33.37 B
ATOM 7639 N THR B 249 109.452 99.363 148.499 1.00 33.44 B
ATOM 7640 CA THR B 249 110.615 100.155 148.117 1.00 33.74 B
ATOM 7641 CB THR B 249 110.820 100.134 146.591 1.00 34.51 B
ATOM 7642 OG1 THR B 249 109.694 100.744 145.945 1.00 34.20 B
ATOM 7643 CG2 THR B 249 110.967 98.706 146.100 1.00 34.34 B
ATOM 7644 C THR B 249 110.443 101.607 148.564 1.00 34.30 B
ATOM 7645 O THR B 249 109.382 101.996 149.056 1.00 32.88 B
ATOM 7646 N ALA B 250 111.491 102.403 148.385 1.00 35.26 B
ATOM 7647 CA ALA B 250 111.461 103.808 148.774 1.00 36.99 B
ATOM 7648 CB ALA B 250 112.747 104.502 148.324 1.00 36.17 B
ATOM 7649 C ALA B 250 110.250 104.531 148.193 1.00 37.93 B
ATOM 7650 O ALA B 250 109.591 105.309 148.890 1.00 37.85 B
ATOM 7651 N ASN B 251 109.949 104.260 146.925 1.00 37.11 B
ATOM 7652 CA ASN B 25.1 108.828 104.915 146.264 1.00 37.66 B
ATOM 7653 CB ASN B 251 109.155 105.131 144.789 1.00 39.67 B
ATOM 7654 CG ASN B 251 110.491 105.818 144.594 1.0044.03 B
ATOM 7655 OD1 ASN B 251 110.756 106.863 145.190 1.00 44.91 B
ATOM 7656 ND2 ASN B 251 111.343 105.232 143.759 1.00 46.21 B
ATOM 7657 C ASN B 251 107.474 104.226 146.397 1.00 35.81 B
ATOM 7658 O ASN B 251 106.539 104.539 145.658 1.00 37.10 B
ATOM 7659 N GLY B 252 107.364 103.291 147.331 1.00 33.56 B
ATOM 7660 CA GLY B 252 106.089 102.630 147.544 1.00 32.27 B
ATOM 7661 C GLY B 252 105.763 101.361 146.780 1.00 32.10 B
ATOM 7662 O GLY B 252 104.698 100.786 147.000 1.00 31.99 B
ATOM 7663 N SER B 253 106.635 100.914 145.881 1.00 31.12 B
ATOM 7664 CA SER B 253 106.341 99.679 145.155 1.00 30.51 B
ATOM 7665 CB SER B 253 107.266 99.513 143.947 1.00 30.21 B
ATOM 7666 OG SER B 253 106.964 100.464 142.944 1.00 32.88 B
ATOM 7667 C SER B 253 106.518 98.501 146.106 1.00 29.49 B
ATOM 7668 O SER B 253 107.453 98.476 146.907 1.00 29.63 B
ATOM 7669 N SER B 254 105.610 97.533 146.021 1.00 27.23 B
ATOM 7670 CA SER B 254 105.668 96.355 146.876 1.00 26.08 B
ATOM 7671 CB SER B 254 104.516 95.401 146.541 1.00 25.35 B
ATOM 7672 OG SER B 254 104.682 94.822 145.256 1.00 26.1 B
ATOM 7673 C SER B 254 106.997 95.617 146.726 1.00 24.37 B
ATOM 7674 O SER B 254 107.629 95.653 145.669 1.00 24.22 B
ATOM 7675 N SER B 255 107.413 94.950 147.793 1.00 23.38 B
ATOM 7676 CA SER B 255 108.656 94.186 147.791 1.00 22.19 B
ATOM 7677 CB SER B 255 109.841 95.090 148.146 1.00 23.24 B
ATOM 7678 OG SER B 255 109.782 95.508 149.501 1.00 20.93 B
ATOM 7679 C SER B 255 108.544 93.052 148.810 1.00 19.92 B
ATOM 7680 O SER B 255 107.517 92.910 149.472 1.00 19.70 B
ATOM 7681 N GLY B 256 109.597 92.245 148.919 1.00 19.69 B
ATOM 7682 CA GLY B 256 109.606 91.142 149.865 1.00 16.61 B
ATOM 7683 C GLY B 256 108.441 90.174 149.771 1.00 18.23 B
ATOM 7684 O GLY B 256 107.950 89.868 148.683 1.00 18.01 B
ATOM 7685 N LEU B 257 108.006 89.689 150.932 1.00 18.60 B
ATOM 7686 CA LEU B 257 106.904 88.740 151.042 1.00 17.97 B
ATOM 7687 CB LEU B 257 106.560 88.527 152.518 1.00 17.73 B
ATOM 7688 CG LEU B 257 105.876 87.240 152.989 1.00 21.47 B
ATOM 7689 CD1 LEU B 257 105.219 87.526 154.334 1.00 23.03 B
ATOM 7690 CD2 LEU B 257 104.834 86.758 152.005 1.00 23.55 B
ATOM 7691 C LEU B 257 105.657 89.234 150.304 1.00 18.78 B
ATOM 7692 O LEU B 257 105.037 88.487 149.538 1.00 17.93 B
ATOM 7693 N ALA B 258 105.293 90.489 150.555 1.00 18.64 B
ATOM 7694 CA ALA B 258 104.121 91.101 149.937 1.00 19.72 B
ATOM 7695 CB ALA B 258 103.976 92.555 150.416 1.00 18.34 B
ATOM 7696 C ALA B 258 104.208 91.051 148.412 1.00 19.57 B
ATOM 7697 O ALA B 258 103.224 90.742 147.745 1.00 19.94 B
ATOM 7698 N TYR B 259 105.390 91.352 147.872 1.00 19.33 B
ATOM 7699 CA TYR B 259 105.614 91.332 146.426 1.00 18.41 B
ATOM 7700 CB TYR B 259 107.020 91.848 146.104 1.00 16.85 B
ATOM 7701 CG TYR B 259 107.421 91.742 144.638 1.00 17.99 B
ATOM 7702 CD1 TYR B 259 106.919 92.632 143.681 1.00 18.27 B
ATOM 7703 CE1 TYR B 259 107.288 92.531 142.337 1.0017.95 B
ATOM 7704 CD2 TYR B 259 108.304 90.746 144.211 1.00 17.04 B
ATOM 7705 CE2 TYR B 259 108.679 90.632 142.874 1.00 16.16 B
ATOM 7706 CZ TYR B 259 108.172 91.526 141.943 1.0019.65 B
ATOM 7707 OH TYR B 259 108.560 91.414 140.628 1.00 19.39 B
ATOM 7708 C TYR B 259 105.444 89.924 145.846 1.00 18.06 B
ATOM 7709 O TYR B 259 104.738 89.731 144.856 1.00 18.07 B
ATOM 7710 N VAL B 260 106.087 88.940 146.462 1.00 17.97 B
ATOM 7711 CA VAL B 260 105.987 87.572 145.969 1.00 17.63 B
ATOM 7712 CB VAL B 260 106.893 86.619 146.772 1.00 17.89 B
ATOM 7713 CG1 VAL B 260 106.637 85.178 146.357 1.00 17.79 B
ATOM 7714 CG2 VAL B 260 108.351 86.974 146.525 1.00 16.87 B
ATOM 7715 C VAL B 260 104.542 87.085 146.022 1.00 18.82 B
ATOM 7716 O VAL B 260 104.053 86.474 145.072 1.00 17.94 B
ATOM 7717 N LYS B 261 103.861 87.359 147.130 1.00 19.17 B
ATOM 7718 CA LYS B 261 102.464 86.965 147.277 1.00 22.16 B
ATOM 7719 CB LYS B 261 101.935 87.422 148.636 1.00 23.84 B
ATOM 7720 CG LYS B 261 100.451 87.194 148.844 1.00 23.56 B
ATOM 7721 CD LYS B 261 100.102 85.717 148.869 1.00 24.09 B
ATOM 7722 CE LYS B 261 98.632 85.530 149.212 1.00 23.40 B
ATOM 7723 NZ LYS B 261 98.262 84.106 149.385 1.00 24.42 B
ATOM 7724 C LYS B 261 101.639 87.614 146.163 1.00 22.84 B
ATOM 7725 O LYS B 261 100.843 86.961 145.491 1.00 22.83 B
ATOM 7726 N GLU B 262 101.847 88.911 145.974 1.00 23.05 B
ATOM 7727 CA GLU B 262 101.139 89.666 144.950 1.00 24.30 B
ATOM 7728 CB GLU B 262 101.556 91.131 145.051 1.00 25.41 B
ATOM 7729 CG GLU B 262 100.965 92.056 144.010 1.00 28.22 B
ATOM 7730 CD GLU B 262 101.406 93.485 144.234 1.00 29.17 B
ATOM 7731 OE1 GLU B 262 100.941 94.099 145.212 1.00 33.32 B
ATOM 7732 OE2 GLU B 262 102.232 93.990 143.449 1.00 32.84 B
ATOM 7733 C GLU B 262 101.378 89.123 143.528 1.00 24.14 B
ATOM 7734 O GLU B 262 100.464 89.121 142.698 1.00 22.64 B
ATOM 7735 N LEU B 263 102.598 88.661 143.253 1.00 23.65 B
ATOM 7736 CA LEU B 263 102.931 88.114 141.937 1.00 23.30 B
ATOM 7737 CB LEU B 263 104.366 87.587 141.907 1.00 24.61 B
ATOM 7738 CG LEU B 263 105.460 88.567 141.490 1.00 26.47 B
ATOM 7739 CD1 LEU B 263 106.816 87.863 141.509 1.00 25.59 B
ATOM 7740 CD2 LEU B 263 105.145 89.101 140.099 1.00 24.73 B
ATOM 7741 C LEU B 263 101.999 86.982 141.549 1.00 22.72 B'
ATOM 7742 O LEU B 263 101.669 86.824 140.374 1.00 22.67 B
ATOM 7743 N GLY B 264 101.590 86.194 142.543 1.00 21.42 B
ATOM 7744 CA GLY B 264 100.703 85.071 142.299 1.00 19.95 B
ATOM 7745 C GLY B 264 101.430 83.732 142.309 1.00 20.79 B
ATOM 7746 O GLY B 264 100.837 82.689 142.022 1.00 9.20 B
ATOM 7747 N VAL B 265 102.717 83.745 142.640 1.00 19.34 B
ATOM 7748 CA VAL B 265 103.477 82.502 142.667 1.00 20.35 B
ATOM 7749 CB VAL B 265 104.988 82.776 142.856 1.00 21.98 B
ATOM 7750 CG1 VAL B 265 105.509 83.652 141.723 1.00 20.02 B
ATOM 7751 CG2 VAL B 265 105.228 83.434 144.179 1.00 21.55 B
ATOM 7752 C VAL B 265 102.979 81.582 143.787 1.00 19.17 B
ATOM 7753 O VAL B 265 102.432 82.046 144.791 1.00 18.67 B
ATOM 7754 N THR B 266 103.161 80.278 143.617 1.00 17.04 B
ATOM 7755 CA THR B 266 102.712 79.333 144.632 1.00 16.88 B
ATOM 7756 CB THR B 266 101.948 78.145 144.004 1.00 16.46 B
ATOM 7757 OG1 THR B 266 102.829 77.411 143.143 1.00 14.43 B
ATOM 7758 CG2 THR B 266 100.741 78.650 143.200 1.00 13.08 B
ATOM 7759 C THR B 266 103.850 78.768 145.467 1.00 17.09 B
ATOM 7760 O THR B 266 103.699 78.560 146.671 1.00 17.20 B
ATOM 7761 N HIS B 267 104.987 78.519 144.827 1.00 16.85 B
ATOM 7762 CA HIS B 267 106.143 77.950 145.518 1.00 15.85 B
ATOM 7763 CB HIS B 267 106.451 76.535 145.006 1.00 15.18 B
ATOM 7764 CG HIS B 267 105.399 75.514 145.316 1.00 14.97 B
ATOM 7765 CD2 HIS B 267 105.457 74.366 146.033 1.00 16.01 B
ATOM 7766 ND1 HIS B 267 104.121 75.578 144.803 1.0017.50 B
ATOM 7767 CE1 HIS B 267 103.440 74.511 145.186 1.00 17.49 B
ATOM 7768 NE2 HIS B 267 104.228 73.759 145.933 1.0017.85 B
ATOM 7769 C HIS B 267 107.404 78.782 145.348 1.00 16.06 B
ATOM 7770 O HIS B 267 107.800 79.127 144.224 1.00 15.74 B
ATOM 7771 N VAL B 268 108.040 79.099 146.467 1.00 15.72 B
ATOM 7772 CA VAL B 268 109.281 79.847 146.428 1.00 15.90 B
ATOM 7773 CB VAL B 268 109.393 80.818 147.629 1.00 17.92 B
ATOM 7774 CG1 VAL B 268 110.688 81.606 147.542 1.00 17.42 B
ATOM 7775 CG2 VAL B 268 108.202 81.766 147.651 1.00 16.89 B
ATOM 7776 C VAL B 268 110.399 78.804 146.516 1.00 15.20 B
ATOM 7777 O VAL B 268 110.483 78.065 147.493 1.00 13.42 B
ATOM 7778 N GLU B 269 111.231 78.716 145.483 1.00 14.30 B
ATOM 7779 CA GLU B 269 112.341 77.766 145.503 1.00 13.57 B
ATOM 7780 CB GLU B 269 112.598 77.173 144.114 1.00 14.65 B
ATOM 7781 CG GLU B 269 113.800 76.225 144.054 1.00 15.39 B
ATOM 7782 CD GLU B 269 113.964 75.568 142.689 1.00 18.03 B
ATOM 7783 OE1 GLU B 269 112.962 75.041 142.165 1.00 20.08 B
ATOM 7784 OE2 GLU B 269 115.088 75.569 142.143 1.00 16.83 B
ATOM 7785 C GLU B 269 113.578 78.511 145.968 1.00 14.20 B
ATOM 7786 O GLU B 269 113.961 79.523 145.373 1.00 13.98 B
ATOM 7787 N LEU B 270 114.200 78.001 147.028 1.00 14.18 B
ATOM 7788 CA LEU B 270 115.383 78.618 147.608 1.00 14.54 B
ATOM 7789 CB LEU B 270 115.281 78.595 149.134 1.00 13.40 B
ATOM 7790 CG LEU B 270 114.021 79.150 149.804 1.00 16.51 B
ATOM 7791 CD1 LEU B 270 114.072 78.860 151.305 1.00 13.64 B
ATOM 7792 CD2 LEU B 270 113.906 80.646 149.535 1.00 17.51 B
ATOM 7793 C LEU B 270 116.671 77.915 147.212 1.00 16.45 B
ATOM 794 O LEU B 270 116.739 76.685 147.227 1.00 18.27 B
ATOM 7795 N LEU B 271 117.693 78.687 146.854 1.00 15.29 B
ATOM 7796 CA LEU B 271 118.975 78.082 146.532 1.00 15.15 B
ATOM 7797 CB LEU B 271 119.952 79.142 146.002 1.00 14.45 B
ATOM 7798 CG LEU B 271 120.010 79.178 144.462 1.00 14.51 B
ATOM 7799 CD LEU271 118.715 79.766 143.896 1.00 11.13 B
ATOM 7800 CD2 LEU B 271 121.203 79.998 143.998 1.00 11.93 B
ATOM 7801 C LEU B 271 119.461 77.425 147.845 1.00 14.69 B
ATOM 7802 O LEU B 271 118.974 77.766 148.924 1.00 14.69 B
ATOM 7803 N PRO B 272 120.414 76.480 147.769 1.00 15.03 B
ATOM 7804 CD PRO B 272 121.264 76.228 146.588 1.00 16.40 B
ATOM 7805 CA PRO B 272 120.946 75.765 148.938 1.00 14.80 B
ATOM 7806 CB PRO B 272 122.373 75.446 148.523 1.00 15.12 B
ATOM 7807 CG PRO B 272 122.185 75.096 147.075 1.0015.48 B
ATOM 7808 C PRO B 272 120.846 76.477 150.284 1.00 16.11 B
ATOM 7809 O PRO B 272 121.384 77.566 150.487 1.00 16.23 B
ATOM 7810 N VAL B 273 120.170 75.816 151.213 1.00 15.69 B
ATOM 7811 CA VAL B 273 119.932 76.370 152.530 1.00 17.22 B
ATOM 7812 CB VAL B 273 118.388 76.532 152.689 1.00 17.89 B
ATOM 7813 CG1 VAL B 273 117.847 75.653 153.781 1.00 15.54 B
ATOM 7814 CG2 VAL B 273 118.045 77.986 152.855 1.00 15.04 B
ATOM 7815 C VAL B 273 120.586 75.537 153.651 1.00 18.18 B
ATOM 7816 O VAL B 273 120.533 75.894 154.834 1.00 17.76 B
ATOM 7817 N ASN B 274 121.216 74.431 153.266 1.00 16.91 B
ATOM 7818 CA ASN B 274 121.923 73.573 154.216 1.00 17.32 B
ATOM 7819 CB ASN B 274 122.027 72.148 153.663 1.00 15.58 B
ATOM 7820 CG ASN B 274 122.933 72.064 152.448 1.00 17.33 B
ATOM 7821 OD1 ASN B 274 124.136 71.819 152.570 1.00 18.86 B
ATOM 7822 ND2 ASN B 274 122.365 72.294 151.271 1.00 14.09 B
ATOM 7823 C ASN B 274 123.317 74.209 154.319 1.00 17.23 B
ATOM 7824 O ASN B 274 123.695 74.991 153.448 1.0017.14 B
ATOM 7825 N ASP B 275 124.085 73.877 155.354 1.00 16.34 B
ATOM 7826 CA ASP B 275 125.405 74.491 155.516 1.00 16.80 B
ATOM 7827 CB ASP B 275 126.033 74.122 156.859 1.00 16.07 B
ATOM 7828 CG ASP B 275 127.186 75.048 157.223 1.00 16.59 B
ATOM 7829 OD1 ASP B 275 128.036 74.672 158.057 1.00 16.42 B
ATOM 7830 OD2 ASP B 275 127.228 76.168 156.672 1.00 14.17 B
ATOM 7831 C ASP B 275 126.407 74.172 154.414 1.00 16.10 B
ATOM 7832 O ASP B 275 126.698 73.010 154.127 1.00 16.95 B
ATOM 7833 N PHE B 276 126.960 75.227 153.831 1.00 14.38 B
ATOM 7834 CA PHE B 276 127.920 75.103 152.750 1.00 13.69 B
ATOM 7835 CB PHE B 276 127.253 75.508 151.429 1.0013.21 B
ATOM 7836 CG PHE B 276 126.714 76.920 151.424 1.00 13.12 B
ATOM 7837 CD1 PHE B 276 125.344 77.159 151.527 1.00 14.29 B
ATOM 7838 CD2 PHE B 276 127.576 78.009 151.306 1.00 13.15 B
ATOM 7839 CE1 PHE B 276 124.839 78.462 151.506 1.00 12.98 B
ATOM 7840 CE2 PHE B 276 127.089 79.318 151.283 1.00 12.01 B
ATOM 7841 CZ PHE B 276 125.713 79.547 151.382 1.00 14.75 B
ATOM 7842 C PHE B 276 129. 147 75.988 152.981 1.00 13.76 B
ATOM 7843 O PHE B 276 129.164 76.822 153.885 1.00 12.03 B
ATOM 7844 N ALA B 277 130.165 75.796 152.146 1.00 13.63 B
ATOM 7845 CA ALA B 277 131.396 76.580 152.209 1.00 12.68 B
ATOM 7846 CB ALA B 277 132.617 75.676 151.975 1.00 10.26 B
ATOM 7847 C ALA B 277 13I.311 77.632 151.105 1.0013.68 B
ATOM 7848 O ALA B 277 130.528 77.474 150.164 1.00 13.27 B
ATOM 7849 N GLY B 278 132.112 78.691 151.213 1.00 12.82 B
ATOM 7850 CA GLY B 278 132.097 79.731 150.198 1.00 16.05 B
ATOM 7851 C GLY B 278 131.886 81.139 150.738 1.00 17.21 B
ATOM 7852 O GLY B 278 132.145 82.135 150.051 1.00 17.39 B
ATOM 7853 N VAL B 279 131.382 81.221 151.960 1.0015.44 B
ATOM 7854 CA VAL B 279 131.162 82.497 152.609 1.00 16.82 B
ATOM 7855 CB VAL B 279 129.657 82.914 152.577 1.00 17.10 B
ATOM 7856 CG1 VAL B 279 129.459 84.222 153.344 1.00 16.38 B
ATOM 7857 CG2 VAL B 279 129.187 83.086 151.132 1.00 15.53 B
ATOM 7858 C VAL B 279 131.610 82.304 154.051 1.00 16.03 B
ATOM 7859 O VAL B 279 131.161 81.379 154.717 1.00 15.36 B
ATOM 7860 N ASP B 280 132.530 83.149 154.509 1.00 17.41 B
ATOM 7861 CA ASP B 280 133.024 83.083 155.884 1.00 17.36 B
ATOM 7862 CB ASP B 280 134.293 83.926 156.045 1.00 19.09 B
ATOM 7863 CG ASP B 280 134.968 83.730 157.400 1.00 18.44 B
ATOM 7864 OD1 ASP B 280 134.277 83.435 158.405 1.00 16.89 B
ATOM 7865 OD2 ASP B 280 136.203 83.889 157.456 1.00 22.31 B
ATOM 7866 C ASP B 280 131.924 83.692 156.733 1.00 18.15 B
ATOM 7867 O ASP B 280 131.588 84.869 156.567 1.00 16.96 B
ATOM 7868 N GLU B 281 131.364 82.899 157.638 1.00 18.04 B
ATOM 7869 CA GLU B 281 130.287 83.380 158.495 1.00 20.09 B
ATOM 7870 CB GLU B 281 129.625 82.187 159.202 1.00 19.56 B
ATOM 7871 CG GLU B 281 128.843 81.291 158.235 1.00 18.17 B
ATOM 7872 CD GLU B 281 128.470 79.932 158.816 1.00 18.97 B
ATOM 7873 OE1 GLU B 281 128.583 79.749 160.047 1.00 16.31 B
ATOM 7874 OE2 GLU B 281 128.055 79.049 158.032 1.00 14.54 B
ATOM 7875 C GLU B 281 130.754 84.445 159.496 1.00 21.75 B
ATOM 7876 O GLU B 281 129.936 85.113 160.129 1.00 22.67 B
ATOM 7877 N GLU B 282 132.068 84.616 159.622 1.00 21.46 B
ATOM 7878 CA GLU B 282 132.615 85.624 160.530 1.00 22.42 B
ATOM 7879 CB GLU B 282 134.011 85.215 161.021 1.00 19.69 B
ATOM 7880 CG GLU B 282 134.037 83.985 161.927 1.00 21.07 B
ATOM 7881 CD GLU B 282 133.462 84.244 163.318 1.00 25.08. B
ATOM 7882 OE1 GLU B 282 133.555 83.337 164.173 1.00 26.04 B
ATOM 7883 OE2 GLU B 282 132.917 85.343 163.566 1.00 25.39 B
ATOM 7884 C GLU B 282 132.689 86.965 159.798 1.00 23.36 B
ATOM 7885 O GLU B 282 132.808 88.020 160.421 1.00 24.30 B
ATOM 7886 N LYS B 283 132.639 86.910 158.471 1.00 22.29 B
ATOM 7887 CA LYS B 283 132.662 88.106 157.634 1.00 24.13 B
ATOM 7888 CB LYS B 283 134.098 88.539 157.312 1.00 26.44 B
ATOM 7889 CG LYS B 283 135.184 87.566 157.725 1.00 31.14 B
ATOM 7890 CD LYS B 283 136.524 88.282 157.790 1.00 33.34 B
ATOM 7891 CE LYS B 283 137.663 87.338158.134 1.0037.31 B
ATOM 7892 NZ LYS B 283 137.922 86.380 157.023 1.00 40.07 B
ATOM 7893 C LYS B 283 131.900 87.802 156.349 1.00 22.59 B
ATOM 7894 O LYS B 283 132.475 87.758 155.261 1.00 22.90 B
ATOM 7895 N PRO B 284 130.577 87.609 156.469 1.00 20.94 B
ATOM 7896 CD PRO B 284 129.836 87.811 157.729 1.00 20.49 B
ATOM 7897 CA PRO B 284 129.663 87.295 155.369 1.00 21.53 B
ATOM 7898 CB PRO B 284 128.299 87.225 156.062 1.00 21.41 B
ATOM 7899 CG PRO B 284 128.458 88.141 157.244 1.0021.67 B
ATOM 7900 C PRO B 284 129.665 88.216 154.154 1.00 21.87 B
ATOM 7901 O PRO B 284 129.339 87.782 153.052 1.00 22.86 B
ATOM 7902 N LEU B 285 130.041 89.476 154.338 1.0023.46 B
ATOM 7903 CA LEU B 285 130.060 90.413 153.223 1.00 24.31 B
ATOM 7904 CB LEU B 285 129.832 91.842 153.729 1.00 26.47 B
ATOM 7905 CG LEU B 285 128.431 92. 151 154.262 1.00 28.18 B
ATOM 7906 CD1 LEU B 285 128.410 93.518 154.917 1.00 29.56 B
ATOM 7907 CD2 LEU B 285 127.431 92.085 153.128 1.00 29.42 B
ATOM 7908 C LEU B 285 131.341 90.365 152.404 1.00 24.22 B
ATOM 7909 O LEU B 285 131.391 90.926 151.317 1.00 26.80 B
ATOM 7910 N ASP B 286 132.372 89.697152.918 1.00 25.25 B
ATOM 7911 CA ASP B 286 133.657 89.605 152.218 1.00 24.27 B
ATOM 7912 CB ASP B 286 134.752 89.110 153.171 1.00 25.44 B
ATOM 7913 CG ASP B 286 135.261 90.210 154.093 1.0028.78 B
ATOM 7914 OD1 ASP B 286 134.594 91.260 154.183 1.00 31.06 B
ATOM 7915 OD2 ASP B 286 136.319 90.029 154.731 1.00 27.30 B
ATOM 7916 C ASP B 286 133.619 88.720 150.983 1.00 23.33 B
ATOM 7917 O ASP B 286 134.489 88.816 150.118 1.00 23.46 B
ATOM 7918 N ALA B 287 132.615 87.856 150.900 1.0022.69 B
ATOM 7919 CA ALA B 287 132.485 86.965 149.754 1.00 21.20 B
ATOM 7920 CB ALA B 287 133.323 85.713 149.968 1.00 19.86 B
ATOM 7921 C ALA B 287 131.029 86.591 149.530 1.0020.12 B
ATOM 7922 O ALA B 287 130.169 86.858 150.373 1.00 20.67 B
ATOM 7923 N TYR B 288 130.761 85.975 148.384 1.0019.88 B
ATOM 7924 CA TYR B 288 129.415 85.545 148.018 1.00 17.64 B
ATOM 7925 CB TYR B 288 128.821 86.503 146.976 1.0018.28 B
ATOM 7926 CG TYR B 288 127.493 86.051 146.391 1.00 17.87 B
ATOM 7927 CD1 TYR B 288 127.432 85.051 145.418 1.00 15.45 B
ATOM 7928 CE1 TYR B 288 126.203 84.615 144.907 1.00 18.14 B
ATOM 7929 CD2 TYR B 288 126.294 86.607 146.838 1.00 16.94 B
ATOM 7930 CE2 TYR B 288 125.070 86.182 146.340 1.00 18.01 B
ATOM 7931 CZ TYR B 288 125.026 85.187 145.377 1.0019.22 B
ATOM 7932 OH TYR B 288 123.800 84.775 144.899 1.0020.13 B
ATOM 7933 C TYR B 288 129.474 84.143 147.430 1.00 17.09 B
ATOM 7934 O TYR B 288 130.485 83.758 146.850 1.00 16.72 B
ATOM 7935 N ASN B 289 128.395 83.382 147.585 1.00 16.30 B
ATOM 7936 CA ASN B 289 128.335 82.048 147.010 1.00 16.46 B
ATOM 7937 CB ASN B 289 129.204 81.076 147.799 1.00 16.37 B
ATOM 7938 CG ASN B 289 129.620 79.880 146.969 1.00 18.52 B
ATOM 7939 OD1 ASN B 289 128.831 78.962 146.730 1.00 19.43 B
ATOM 7940 ND2 ASN B 289 130.862 79.898 146.498 1.00 16.61 B
ATOM 7941 C ASN B 289 126.914 81.508 146.925 1.00 16.44 B
ATOM 7942 O ASN B 289 126.124 81.664 147.856 1.00 14.40 B
ATOM 7943 N TRP B 290 126.590 80.880 145.796 1.00 15.66 B
ATOM 7944 CA TRP B 290 125.263 80.305 145.609 1.00 14.67 B
ATOM 7945 CB TRP B 290 125.130 79.655 144.233 1.00 12.90 B
ATOM 7946 CG TRP B 290 124.831 80.614 143.139 1.00 13.66 B
ATOM 7947 CD2 TRP B 290 124.989 80.381 141.735 1.00 13.75 B
ATOM 7948 CE2 TRP B 290 124.53981.538141.0691.0015.15 B
ATOM 7949 CE3 TRP B 290 125.467 79.305 140.977 1.00 14.94 B
ATOM 7950 CD1 TRP B 290 124.305 81.868 143.266 1.00 12.31 B
ATOM 7951 NE1 TRP B 290 124.127 82.429 142.027 1.00 16.53 B
ATOM 7952 CZ2 TRP B 290 124.555 81.654 139.672 1.00 16.71 B
ATOM 7953 CZ3 TRP B 290 125.483 79.418 139.585 1.00 16.48 B
ATOM 7954 CH2 TRP B 290 125.029 80.587 138.950 1.00 16.62 B
ATOM 7955 C TRP B 290 124.948 79.262 146.665 1.00 13.88 B
ATOM 7956 O TRP B 290 123.800 79.127 147.077 1.00 14.86 B
ATOM 7957 N GLY B 291 125.962 78.510 147.082 1.00 13.56 B
ATOM 7958 CA GLY B 291 125.749 77.493 148.096 1.00 13.85 B
ATOM 7959 C GLY B 291 125.905 76.051 147.637 1.00 15.24 B
ATOM 7960 O GLY B 291 125.535 75.130 148.366 1.00 14.03 B
ATOM 7961 N TYR B 292 126.463 75.835 146.448 1.00 15.25 B
ATOM 7962 CA TYR B 292 126.637 74.470 145.954 1.00 15.27 B
ATOM 7963 CB TYR B 292 126.537 74.453 144.421 1.00 14.39 B
ATOM 7964 CG TYR B 292 125.160 74.870 143.936 1.00 14.78 B
ATOM 7965 CD1 TYR B 292 124.062 74.018 144.068 1.00 12.29 B
ATOM 7966 CE1 TYRB 292 122.775 74.439 143.719 1.00 13.59 B
ATOM 7967 CD2 TYR B 292 124.936 76.154 143.432 1.00 14.12 B
ATOM 7968 CE2 TYR B 292 123.656 76.582 143.081 1.00 12.42 B
ATOM 7969 CZ TYR B 292 122.582 75.725 143.228 1.0013.54 B
ATOM 7970 OH TYR B 292 121.320 76.163 142.897 1.00 12.07 B
ATOM 7971 C TYR B 292 127.932 73.802 146.437 1.00 16.31 B
ATOM 7972 O TYR B 292 128.565 73.054 145.706 1.00 16.05 B
ATOM 7973 N ASN B 293 128.305 74.069 147.687 1.00 17.06 B
ATOM 7974 CA ASN B 293 129.505 73.479 148.283 1.00 17.98 B
ATOM 7975 CB ASN B 293 130.591 74.542 148.482 1.00 17.67 B
ATOM 7976 CG ASN B 293 131.026 75.178 147.176 1.00 19.55 B
ATOM 7977 OD1 ASN B 293 131.499 74.496 146.270 1.00 20.08 B
ATOM 7978 ND2 ASN B 293 130.863 76.493 147.073 1.00 19.07 B
ATOM 7979 C ASN B 293 129.098 72.884 149.630 1.00 16.57 B
ATOM 7980 O ASN B 293 129.441 73.413 150.682 1.00 18.31 B
ATOM 7981 N PRO B 294 128.360 71.763 149.599 1.00 16.95 B
ATOM 7982 CD PRO B 294 128.036 71.058 148.343 1.00 18.10 B
ATOM 7983 CA PRO B 294 127.840 71.018 150.752 1.00 14.80 B
ATOM 7984 CB PRO B 294 127.187 69.790 150.115 1.00 14.75 B
ATOM 7985 CG PRO B 294 126.827 70.250 148.735 1.00 16.32 B
ATOM 7986 C PRO B 294 128.848 70.604 151.809 1.00 15.24 B
ATOM 7987 O PRO B 294 129.823 69.916 151.508 1.00 12.62 B
ATOM 7988 N LEU B 295 128.595 71.025 153.047 1.00 14.05 B
ATOM 7989 CA LEU B 295 129.430 70.657 154.185 1.00 14.65 B
ATOM 7990 CB LEU B 295 129.885 71.894 154.973 1.0015.95 B
ATOM 7991 CG LEU B 295 131.016 72.742 154.388 1.00 19.28 B
ATOM 7992 CD1 LEU B 295 131.230 73.975 155.257 1.00 18.71 B
ATOM 7993 CD2 LEU B 295 132.293 71.907 154.301 1.00 16.13 B
ATOM 7994 C LEU B 295 128.578 69.762 155.093 1.00 13.87 B
ATOM 7995 O LEU B 295 128.948 68.632 155.381 1.00 14.51 B
ATOM 7996 N HIS B 296 127.428 70.274 155.524 1.00 13.39 B
ATOM 7997 CA HIS B 296 126.527 69.528 156.400 1.00 13.37 B
ATOM 7998 CB HIS B 296 126.565 70.152 157.800 1.00 11.76 B
ATOM 7999 CG HIS B 296 127.957 70.355 158.318 1.00 10.73 B
ATOM 8000 CD2 HIS B 296 128.673 71.486 158.534 1.00 11.49 B
ATOM 8001 ND1 HIS B 296 128.812 69.307 158.587 1.00 10.34 B
ATOM 8002 CE1 HIS B 296 129.993 69.781 158.940 1.00 8.88 B
ATOM 8003 NE2 HIS B 296 129.936 71.100 158.916 1.00 9.76 B
ATOM 8004 C HIS B 296 125.117 69.563 155.799 1.00 14.01 B
ATOM 8005 O HIS B 296 124.535 70.633 155.621 1.00 15.77 B
ATOM 8006 N PHE B 297 124.582 68.384 155.482 1.00 13.91 B
ATOM 8007 CA PHE B 297 123.268 68.260 154.855 1.00 13.98 B
ATOM 8008 CB PHE B 297 123.175 66.938 154.076 1.0013.61 B
ATOM 8009 CG PHE B 297 124.129 66.838 152.907 1.00 14.95 B
ATOM 8010 CD1 PHE B 297 125.419 66.336 153.077 1.00 14.54 B
ATOM 8011 CD2 PHE B 297 123.734 67.237 151.630 1.00 16.76 B
ATOM 8012 CE1 PHE B 297 126.301 66.229 151.992 1.00 12.36 B
ATOM 8013 CE2 PHE B 297 124.613 67.135 150.535 1.00 5.44 B
ATOM 8014 CZ PHE B 297 125.897 66.628 150.722 1.00 12.00 B
ATOM 8015 C PHE B 297 122.054 68.365 155.778 1.0014.04 B
ATOM 8016 O PHE B 297 120.947 68.620 155.310 1.00 13.56 B
ATOM 8017 N PHE B 298 122.254 68.167 157.079 1.00 14.06 B
ATOM 8018 CA PHE B 298 121.147 68.219 158.023 1.00 13.17 B
ATOM 8019 CB PHE B 298 121.197 67.014 158.974 1.00 13.37 B
ATOM 8020 CG PHE B 298 120.838 65.707 158.322 1.00 12.21 B
ATOM 8021 CD1 PHE B 298 121.717 65.089 157.432 1.00 10.78 B
ATOM 8022 CD2 PHE B 298 119.612 65.100 158.587 1.00 10.68 B
ATOM 8023 CE1 PHE B 298 121.377 63.877 156.810 1.00 11.54 B
ATOM 8024 CE2 PHE B 298 119.262 63.890 157.972 1.00 13.07 B
ATOM 8025 CZ PHE B 298 120.151 63.278 157.078 1.00 9.82 B
ATOM 8026 C PHE B 298 121.092 69.498 158.839 1.00 14.90 B
ATOM 8027 O PHE B 298 120.207 69.662 159.684 1.00 15.97 B
ATOM 8028 N ALA B 299 122.039 70.399 158.601 1.00 15.02 B
ATOM 8029 CA ALA B 299 122.071 71.664 159.325 1.00 14.83 B
ATOM 8030 CB ALA B 299 123.344 71.760 160.175 1.00 13.41 B
ATOM 8031 C ALA B 299 122.002 72.831 158.351 1.00 14.73 B
ATOM 8032 O ALA B 299 122.462 72.734 157.216 1.00 16.83 B
ATOM 8033 N PRO B 300 121.422 73.957 158.787 1.00 14.84 B
ATOM 8034 CD PRO B 300 120.638 74.102 160.020 1.00 13.08 B
ATOM 8035 CA PRO B 300 121.287 75.154 157.951 1.00 14.56 B
ATOM 8036 CB PRO B 300 120.225 75.973 158.680 1.00 12.12 B
ATOM 8037 CG PRO B 300 119.519 74.975 159.547 1.00 13.44 B
ATOM 8038 C PRO B 300 122.568 75.962 157.776 1.00 14.78 B
ATOM 8039 O PRO B 300 123.499 75.857 158.577 1.00 14.57 B
ATOM 8040 N GLU B 301 122.589 76.771 156.717 1.00 15.55 B
ATOM 8041 CA GLU B 301 123.704 77.654 156.412 1.00 14.06 B
ATOM 8042 CB GLU B 301 123.508 78.282 155.028 1.00 14.24 B
ATOM 8043 CG GLU B 301 124.281 79.568 154.806 1.00 15.96 B
ATOM 8044 CD GLU B 301 125.787 79.386 154.866 1.00 14.20 B
ATOM 8045 OE1 GLU B 301 126.500 80.411 154.860 1.00 14.82 B
ATOM 8046 OE2 GLU B 301 126.261 78.231 154.908 1.0011.83 B
ATOM 8047 C GLU B 301 123.714 78.736 157.488 1.00 15.66 B
ATOM 8048 O GLU B 301 122.658 79.182 157.942 1.00 13.34 B
ATOM 8049 N GLY B 302 124.905 79.163 157.890 1.00 16.40 B
ATOM 8050 CA GLY B 302 125.001 80.167 158.930 1.00 16.75 B
ATOM 8051 C GLY B 302 125.006 81.629 158.522 1.00 18.31 B
ATOM 8052 O GLY B 302 124.603 82.478 159.318 1.00 18.29 B
ATOM 8053 N SER B 303 125.442 81.941 157.302 1.00 18.51 B
ATOM 8054 CA SERB 303 125.502 83.342 156.881 1.00 17.68 B
ATOM 8055 CB SER B 303 126.368 83.504 155.619 1.00 17.26 B
ATOM 8056 OG SER B 303 125.727 83.002 154.460 1.00 19.05 B
ATOM 8057 C SER B 303 124.141 83.993 156.670 1.00 17.82 B
ATOM 8058 O SERB 303 124.068 85.209 .156.525 1.00 16.93 B
ATOM 8059 N TYR B 304 123.069 83.198 156.654 1.00 17.60 B
ATOM 8060 CA TYR B 304 121.725 83.754 156.490 1.00 16.57 B
ATOM 8061 CB TYR B 304 120.821 82.815 155.675 1.00 18.16 B
ATOM 8062 CG TYR B 304 121.323 82.512 154.277 1.00 17.86 B
ATOM 8063 CD1 TYR B 304 121.833 83.521 153.464 1.00 17.16 B
ATOM 8064 CE1 TYR B 304 122.297 83.251 152.180 1.0018.24 B
ATOM 8065 CD2 TYR B 304 121.284 81.212 153.770 1.00 19.36 B
ATOM 8066 CE2 TYR B 304 121.747 80.927 152.483 1.00 19.93 B
ATOM 8067 CZ TYR B 304 122.252 81.953 151.697 1.00 19.18 B
ATOM 8068 OH TYR B 304 122.715 81.680 150.435 1.00 17.99 B
ATOM 8069 C TYR B 304 121.073 84.019 157.849 1.00 16.67 B
ATOM 8070 O TYR B 304 119.963 84.562 157.930 1.0014.63 B
ATOM 8071 N ALA B 305 121.761 83.631 158.916 1.00 16.41 B
ATOM 8072 CA ALA B 305 121.242 83.827 160.262 1.00 16.94 B
ATOM 8073 CB ALA B 305 121.717 82.709 161.172 1.00 18.29 B
ATOM 8074 C ALA B 305 121.676 85.173 160.827 1.00 18.59 B
ATOM 8075 O ALA B 305 122.711 85.705 160.444 1.0019.57 B
ATOM 8076 N SER B 306 120.872 85.731 161.727 1.00 19.80 B
ATOM 8077 CA SER B 306 121.219 87.004 162.344 1.00 21.90 B
ATOM 8078 CB SER B 306 120.114 87.454 163.304 1.00 19.91 B
ATOM 8079 OG SER B 306 119.857 86.455 164.277 1.00 20.43 B
ATOM 8080 C SER B 306 122.520 86.800 163.117 1.00 22.46 B
ATOM 8081 O SER B 306 123.405 87.655 163.118 1.00 24.28 B
ATOM 8082 N ASN B 307 122.633 85.650 163.765 1.00 21.55 B
ATOM 8083 CA ASN B 307 123.819 85.327 164.548 1.00 22.43 B
ATOM 8084 CB ASN B 307 123.494 85.484 166.036 1.00 21.34 B
ATOM 8085 CG ASN B 307 124.649 85.103 166.939 1.00 23.13 B
ATOM 8086 OD1 ASN B 307 124.480 84.993 168.156 1.00 22.89 B
ATOM 8087 ND2 ASN B 307 125.825 84.907 166.358 1.00 21.40 B
ATOM 8088 C ASN B 307 124.238 83.891 164.234 1.00 20.60 B
ATOM 8089 O ASN B 307 123.559 82.939 164.612 1.00 20.21 B
ATOM 8090 N PRO B 308 125.360 83.722 163.521 1.00 20.30 B
ATOM 8091 CD PRO B 308 126.108 84.760 162.789 1.00 20.37 B
ATOM 8092 CA PRO B 308 125.842 82.385 163.166 1.00 21.30 B
ATOM 8093 CB PRO B 308 126.754 82.661 161.972 1.00 21.66 B
ATOM 8094 CG PRO B 308 127.319 84.006 162.294 1.00 19.67 B ATOM 8095 C PRO B 308 126.558 81.650 164.291 1.00 22.33 B
ATOM 8096 O PRO B 308 126.799 80.449 164.196 1.00 22.37 B
ATOM 8097 N HIS B 309 126.882 82.373 165.359 1.00 22.85 B
ATOM 8098 CA HIS B 309 127.590 81.800 166.505 1.00 23.31 B
ATOM 8099 CB HIS B 309 128.262 82.922 167.302 1.00 20.31 B
ATOM 8100 CG HIS B 309 129.075 83.851 166.452 1.0020.94 B
ATOM 8101 CD2 HIS B 309 128.765 85.041 165.886 1.00 19.50 B
ATOM 8102 ND1 HIS B 309 130.361 83.560 166.047 1.00 20.56 B
ATOM 8103 CE1 HIS B 309 130.807 84.530 165.270 1.00 19.27 B
ATOM 8104 NE2 HIS B 309 129.858 85.441 165.157 1.00 20.03 B
ATOM 8105 C HIS B 309 126.678 80.976 167.416 1.00 23.36 B
ATOM 8106 O HIS B 309 127.137 80.049 168.077 1.00 26.73 B
ATOM 8107 N ASP B 310 125.393 81.312 167.459 1.00 23.16 B
ATOM 8108 CA ASP B 310 124.444 80.564 168.284 1.00 22.54 B
ATOM 8109 CB ASP B 310 123.346 81.485 168.830 1.00 23.01 B
ATOM 8110 CG ASP B 310 122.328 80.739 169.686 1.00 25.26 B
ATOM 8111 OD1 ASP B 310 121.113 80.877 169.435 1.00 29.68 B
ATOM 8112 OD2 ASP B 310 122.736 80.016 170.617 1.00 25.38 B
ATOM 8113 C ASP B 310 123.814 79.474 167.413 1.00 21.22 B
ATOM 8114 O ASP B 310 123.018 79.764 166.521 1.00 19.50 B
ATOM 8115 N PRO B 311 124.166 78.204 167.669 1.00 21.41 B
ATOM 8116 CD PRO B 311 124.997 77.769 168.809 1.00 21.29 B
ATOM 8117 CA PRO B 311 123.660 77.046 166.924 1.00 21.39 B
ATOM 8118 CB PRO B 311 124.065 75.866 167.806 1.00 20.99 B
ATOM 8119 CG PRO B 311 125.334 76.342 168.437 1.00 21.98 B
ATOM 8120 C PRO B 311 122.156 77.053 166.637 1.00 21.33 B
ATOM 8121 O PRO B 311 121.726 76.740 165.524 1.0020.86 B
ATOM 8122 N GLN B 312 121.362 77.419 167.636 1.00 19.84 B
ATOM 8123 CA GLN B 312 119.909 77.424 167.495 1.00 18.87 B
ATOM 8124 CB GLN B 312 119.255 77.446 168.883 1.00 17.77 B
ATOM 8125 CG GLN B 312 117.733 77.559 168.864 1.0018.15 B
ATOM 8126 CD GLN B 312 117.053 76.342 168.251 1.00 19.24 B
ATOM 8127 OE1 GLN B 312 116.181 76.469 167.387 1.0017.63 B
ATOM 8128 NE2 GLN B 312 117.445 75.157 168.702 1.00 19.05 B
ATOM 8129 C GLN B 312 119.315 78.547 166.650 1.00 19.33 B
ATOM 8130 O GLN B 312 118.274 78.357 166.015 1.00 21.15 B
ATOM 8131 N THR B 313 119.962 79.708 166.644 1.00 17.30 B
ATOM 8132 CA THR B 313 119.458 80.858 165.908 1.00 18.86 B
ATOM 8133 CB THR B 313 120.417 82.060 166.047 1.00 19.25 B
ATOM 8134 OG1 THR B 313 120.519 82.421 167.429 1.00 21.10 B
ATOM 8135 CG2 THR B 313 119.900 83.264 165.265 1.00 18.86 B
ATOM 8136 C THR B 313 119.151 80.624 164.424 1.00 20.27 B
ATOM 8137 O THR B 313 118.068 80.986 163.956 1.00 21.01 B
ATOM 8138 N ARG B 314 120.085 80.029 163.685 1.00 9.05 B
ATOM 8139 CA ARG B 314 119.854 79.784 162.262 1.00 18.84 B
ATOM 8140 CB ARG B 314 121.098 79.169 161.607 1.00 17.43 B
ATOM 8141 CG ARG B 314 121.488 77.802 162.132 1.00 16.87 B
ATOM 8142 CD ARG B 314 122.791 77.325 161.510 1.00 16.33 B
ATOM 8143 NE ARG B 314 123.944 78.101 161.957 1.00 13.68 B
ATOM 8144 CZ ARG B 314 125.164 78.013 161.427 1.00 16.29 B
ATOM 8145 NH1 ARG B 314 125.410 77.180 160.417 1.00 12.39 B
ATOM 8146 NH2 ARG B 314 126.144 78.765 161.901 1.00 13.13 B
ATOM 8147 C ARG B 314 118.646 78.875 162.045 1.00 18.50 B
ATOM 8148 O ARG B 314 117.863 79.084 161.120 1.00 19.54 B
ATOM 8149 N LYS B 315 118.488 77.878 162.907 1.00 17.94 B
ATOM 8150 CA LYS B 315 117.367 76.947 162.802 1.00 19.58 B
ATOM 8151 CB LYS B 315 117.538 75.807 163.813 1.0020.26 B
ATOM 8152 CG LYS B 315 118.766 74.922 163.575 1.00 23.30 B
ATOM 8153 CD LYS B 315 118.838 73.795 164.597 1.0023.82 B
ATOM 8154 CE LYS B 315 117.560 72.965 164.580 1.00 26.99 B
ATOM 8155 NZ LYS B 315 117.471 72.044 165.744 1.00 30.35 B
ATOM 8156 C LYS B 315 116.025 77.648 163.048 1.00 20.01 B
ATOM 8157 O LYS B 315 115.067 77.482 162.292 1.00 19.79 B
ATOM 8158 N THR B 316 115.968 78.428 164.120 1.00 19.69 B
ATOM 8159 CA THR B 316 114.760 79.147 164.477 1.00 19.68 B
ATOM 8160 CB THR B 316 114.942 79.876 165.832 1.00 19.97 B
ATOM 8161 OG1 THR B 316 114.822 78.926 166.898 1.00 23.58 B
ATOM 8162 CG2 THR B 316 113.907 80.970 166.012 1.00 22.24 B
ATOM 8163 C THR B 316 114.356 80.152 163.402 1.00 18.66 B
ATOM 8164 O THR B 316 113.184 80.241 163.048 1.00 20.31 B
ATOM 8165 N GLU B 317 115.316 80.894 162.866 1.00 16.66 B
ATOM 8166 CA GLU B 317 114.981 81.888 161.857 1.00 17.67 B
ATOM 8167 CB GLU B 317 116.135 82.882 161.707 1.00 17.88 B
ATOM 8168 CG GLU B 317 116.553 83.475 163.054 1.00 15.51 B
ATOM 8169 CD GLU B 317 117.576 84.581 162.941 1.00 16.47 B
ATOM 8170 OE1 GLU B 317 118.413 84.550 162.009 1.00 18.48 B
ATOM 8171 OE2 GLU B 317 117.555 85.482 163.805 1.00 19.53 B
ATOM 8172 C GLU B 317 114.578 81.289 160.505 1.00 18.55 B
ATOM 8173 O GLU B 317 113.785 81.886 159.775 1.00 20.08 B
ATOM 8174 N LEU B 318 115.104 80.112 160.170 1.00 18.26 B
ATOM 8175 CA LEU B 318 114.737 79.466 158.907 1.00 16.46 B
ATOM 8176 CB LEU B 318 115.700 78.312 158.580 1.00 15.81 B
ATOM 8177 CG LEU B 318 115.385 77.411 157.364 1.00 12.87 B
ATOM 8178 CD1 LEU B 318 115.142 78.255 156.112 1.00 12.44 B
ATOM 8179 CD2 LEU B 318 116.552 76.475 157.139 1.00 14.26 B
ATOM 8180 C LEU B 318 113.304 78.940 159.029 1.00 15.99 B
ATOM 8181 O LEU B 318 112.512 79.037 158.088 1.0014.77 B
ATOM 8182 N LYS B 319 112.977 78.395 160.198 1.00 17.04 B
ATOM 8183 CA LYS B 319 111.634 77.870 160.456 1.00 17.32 B
ATOM 8184 CB LYS B 319 111.592 77.141 161.802 1.0013.83 B
ATOM 8185 CG LYS B 319 112.257 75.767 161.805 1.00 17.68 B
ATOM 8186 CD LYS B 319 112.225 75.150 163.207 1.00 18.62 B
ATOM 8187 CE LYS B 319 112.616 73.682 163.191 1.00 20.67 B
ATOM 8188 NZ LYS B 319 112.478 73.058 164.532 1.00 22.19 B
ATOM 8189 C LYS B 319 110.587 78.986 160.449 1.00 18.29 B
ATOM 8190 O LYS B 319 109.487 78.812 159.927 1.00 19.51 B
ATOM 8191 N GLN B 320 110.921 80.130 161.030 1.00 17.67 B
ATOM 8192 CA GLN B 320 109.981 81.241 161.063 1.00 20.82 B
ATOM 8193 CB GLN B 320 110.510 82.344 161.986 1.00 20.56 B
ATOM 8194 CG GLN B 320 110.733 81.838 163.400 1.0027.93 B
ATOM 8195 CD GLN B 320 111.229 82.903 164.363 1.00 31.44 B
ATOM 8196 OE1 GLN B 320 112.198 83.610 164.085 1.00 34.87 B
ATOM 8197 NE2 GLN B 320 110.574 83.005 165.516 1.00 32.09 B
ATOM 8198 C GLN B 320 109.744 81.776 159.649 1.00 20.69 B
ATOM 8199 O GLN B 320 108.633 82.189 159.305 1.00 21.70 B
ATOM 8200 N MET B 321 110.791 81.760 158.831 1.0020.18 B
ATOM 8201 CA MET B 321 110.689 82.228 157.454 1.0020.53 B
ATOM 8202 CB MET B 321 112.058 82.182 156.773 1.00 19.63 B
ATOM 8203 CG MET B 321 112.056 82.761 155.381 1.0022.71 B
ATOM 8204 SD MET B 321 113.605 82.489 154.503 1.00 23.96 B
ATOM 8205 CE MET B 321 13.197 80.980 153.636 1.0020.86 B
ATOM 8206 C MET B 321 109.720 81.313 156.716 1.00 18.88 B
ATOM 8207 O MET B 321 108.810 81.773 156.036 1.00 21.09 B
ATOM 8208 N ILE B 322 109.918 80.009 156.870 1.00 18.36 B
ATOM 8209 CA ILE B 322 109.067 79.022 156.228 1.00 18.57 B
ATOM 8210 CB ILE B 322 109.631 77.603 156.423 1.00 17.24 B
ATOM 8211 CG2 ILE B 322 108.641 76.572 155.900 1.00 14.41 B
ATOM 8212 CG1 ILE B 322 110.977 77.483 155.696 1.00 16.23 B
ATOM 8213 CD1 ILE B 322 111.801 76.278 156.113 1.00 13.22 B
ATOM 8214 C ILE B 322 107.638 79.078 156.766 1.00 19.98 B
ATOM 8215 O ILE B 322 106.678 78.979 155.996 1.00 19.35 B
ATOM 8216 N ASN B 323 107.489 79.247 158.078 1.00 19.81 B
ATOM 8217 CA ASN B 323 106.153 79.311 158.661 1.00 20.68 B
ATOM 8218 CB ASN B 323 106.205 79.285 160.193 1.00 20.49 B
ATOM 8219 CG ASN B 323 104.819 79.120 160.817 1.00 22.22 B
ATOM 8220 OD1 ASN B 323 104.116 78.148 160.541 1.00 22.52 B
ATOM 8221 ND2 ASN B 323 104.423 80.073 161.656 1.00 21.36 B
ATOM 8222 C ASN B 323 105.418 80.565 158.202 1.00 20.57 B
ATOM 8223 O ASN B 323 104.215 80.520 157.945 1.00 22.16 B
ATOM 8224 N THR B 324 106.131 81.681 158.096 1.00 18.78 B
ATOM 8225 CA THR B 324 105.499 82.915 157.650 1.00 18.42 B
ATOM 8226 CB THR B 324 106.464 84.114 157.766 1.00 19.16 B
ATOM 8227 OG1 THR B 324 106.911 84.228 159.121 1.00 21.91 B
ATOM 8228 CG2 THR B 324 105.761 85.409 157.395 1.00 19.26 B
ATOM 8229 C THR B 324 105.004 82.774 156.205 1.00 18.69 B
ATOM 8230 O THR B 324 103.925 83.252 155.867 1.00 17.22 B
ATOM 8231 N LEU B 325 105.790 82.117 155.354 1.00 19.12 B
ATOM 8232 CA LEU B 325 105.381 81.900 153.966 1.00 18.59 B
ATOM 8233 CB LEU B 325 106.477 81.161 153.192 1.00 20.09 B
ATOM 8234 CG LEU B 325 107.726 82.001 152.893 1.00 21.81 B
ATOM 8235 CD1 LEU B 325 108.839 81.121 152.368 1.00 24.37 B
ATOM 8236 CD2 LEU B 325 107.378 83.074 151.880 1.00 23.39 B
ATOM 8237 C LEU B 325 104.091 81.078 153.961 1.00 18.32 B
ATOM 8238 O LEU B 325 103.160 81.370 153.213 1.00 17.28 B
ATOM 8239 N HIS B 326 104.048 80.048 154.802 1.00 16.60 B
ATOM 8240 CA HIS B 326 102.867 79.204 154.913 1.00 17.84 B
ATOM 8241 CB HIS B 326 103.126 78.076 155.914 1.0017.26 B
ATOM 8242 CG HIS B 326 103.999 76.984 155.379 1.0017.55 B
ATOM 8243 CD2 HIS B 326 104.339 76.655 154.110 1.00 15.23 B
ATOM 8244 ND1 HIS B 326 104.588 76.039 156.191 1.00 14.91 B
ATOM 8245 CE1 HIS B 326 105.250 75.173 155.445 1.00 16.82 B
ATOM 8246 NE2 HIS B 326 105.113 75.524 154.179 1.00 17.74 B
ATOM 8247 C HIS B 326 101.656 80.029 155.362 1.00 19.11 B
ATOM 8248 O HIS B 326 100.561 79.886 154.820 1.00 17.65 B
ATOM 8249 N GLN B 327 101.863 80.887 156.358 1.00 18.95 B
ATOM 8250 CA GLN B 327 100.792 81.728 156.872 1.00 20.95 B
ATOM 8251 CB GLN B 327 101.338 82.678 157.946 1.0022.00 B
ATOM 8252 CG GLN B 327 101.643 82.005 159.272 1.00 24.56 B
ATOM 8253 CD GLN B 327 102.225 82.964 160.300 1.0028.49 B
ATOM 8254 OE1 GLN B 327 101.923 82.865 161.491 1.00 28.09 B
ATOM 8255 NE2 GLN B 327 103.074 83.886 159.847 1.00 26.82 B
ATOM 8256 C GLN B 327 100.144 82.538 155.754 1.00 22.18 B
ATOM 8257 O GLN B 327 98.944 82.81 155.784 1.00 24.14 B
ATOM 8258 N HIS B 328 100.944 82.914 154.765 1.00 21.76 B
ATOM 8259 CA HIS B 328 100.454 83.705 153.640 1.00 22.16 B
ATOM 8260 CB HIS B 328 101.487 84.777 153.291 1.0020.43 B
ATOM 8261 CG HIS B 328 101.689 85.782 154.381 1.00 22.73 B
ATOM 8262 CD2 HIS B 328 102.323 85.685 155.575 1.00 22.69 B
ATOM 8263 ND1 HIS B 328 101.133 87.042 154.343 1.00 22.70 B
ATOM 8264 CE1 HIS B 328 101.414 87.678 155.467 1.00 23.35 B
ATOM 8265 NE2 HIS B 328 102.134 86.877 156.232 1.00 23.07 B
ATOM 8266 C HIS B 328 100.116 82.870 152.408 1.00 21.88 B
ATOM 8267 O HIS B 328 100.103 83.382 151.292 1.00 23.37 B
ATOM 8268 N GLY B 329 99.841 81.585 152.622 1.00 21.32 B
ATOM 8269 CA GLY B 329 99.481 80.695 151.529 1.00 21.96 B
ATOM 8270 C GLY B 329 100.595 80.293 150.573 1.00 22.44 B
ATOM 8271 O GLY B 329 100.325 79.717 149.516 1.0023.10 B
ATOM 8272 N LEU B 330 101.842 80.578 150.928 1.00 20.10 B
ATOM 8273 CA LEU B 330 102.960 80.232 150.059 1.00 19.75 B
ATOM 8274 CB LEU B 330 103.937 81.410 149.969 1.00 18.80 B
ATOM 8275 CG LEU B 330 103.394 82.666 149.275 1.00 20.07 B
ATOM 8276 CD1 LEU B 330 104.383 83.816 149.419 1.00 17.34 B
ATOM 8277 CD2 LEU B 330 103.132 82.359 147.802 1.00 20.12 B
ATOM 8278 C LEU B 330 103.695 78.973 150.521 1.00 19.68 B
ATOM 8279 O LEU B 330 103.799 78.708 151.723 1.00 17.84 B
ATOM 8280 N ARG B 331 104.195 78.198 149.559 1.00 17.81 B
ATOM 8281 CA ARG B 331 104.923 76.970 149.863 1.00 17.71 B
ATOM 8282 CB ARG B 331 104.296 75.793 149.104 1.00 16.00 B
ATOM 8283 CG ARG B 331 102.907 75.427 149.632 1.00 16.90 B
ATOM 8284 CD ARG B 331 102.276 74.249 148.884 1.00 18.49 B
ATOM 8285 NE ARG B 331 101.037 73.779 149.515 1.00 17.27 B
ATOM 8286 CZ ARG B 331 99.868 74.412 149.448 1.00 17.28 B

ATOM 8287 NH1 ARG B 331 98.802 73.908 150.055 1.00 18.99 B
ATOM 8288 NH2 ARG B 331 99.757 75.540 148.765 1.00 15.22 B
ATOM 8289 C ARG B 331 106.423 77.104 149.557 1.0017.10 B
ATOM 8290 O ARG B 331 106.845 78.024 148.844 1.00 16.72 B
ATOM 8291 N VAL B 332 107.219 76.187 150.100 1.0014.02 B
ATOM 8292 CA VAL B 332 108.674 76.232 149.941 1.00 13.61 B
ATOM 8293 CB VAL B 332 109.361 76.482 151.318 1.00 13.35 B
ATOM 8294 CG1 VAL B 332 110.877 76.424 151.180 1.00 10.81 B
ATOM 8295 CG2 VAL B 332 108.933 77.829 151.878 1.00 14.06 B
ATOM 8296 C VAL B 332 109.315 74.987 149.327 1.00 14.31 1 B
ATOM 8297 O VAL B 332 109.044 73.852 149.739 1.00 14.64 B
ATOM 8298 N ILE B 333 110.180 75.21 148.347 1.00 13.36 B
ATOM 8299 CA ILE B 333 110.899 74.121 147.696 1.00 14.16 B
ATOM 8300 CB ILE B 333 110.726 74.150 146.167 1.00 14.57 B
ATOM 8301 CG2 ILE B 333 111.588 73.070 145.519 1.00 10.30 B
ATOM 8302 CG1 ILEB333 109.255 73.941 145.809 1.00 15.71 B
ATOM 8303 CD1 ILE B 333 108.942 74.285 144.368 1.00 18.08 B
ATOM 8304 C ILE B 333 112.366 74.335 148.025 1.00 14.10 B
ATOM 8305 O ILE B 333 112.873 75.450 147.919 1.00 16.35 B
ATOM 8306 N LEU B 334 113.045 73.271 148.428 1.00 13.32 B
ATOM 8307 CA LEU B 334 114.455 73.364 148.774 1.00 12.88 B
ATOM 8308 CB LEU B 334 114.719 72.623 150.088 1.00 12.18 B
ATOM 8309 CG LEU B 334 114.891 73.415 151.391 1.00 13.12 B
ATOM 8310 CD1 LEU B 334 113.992 74.631 151.437 1.00 10.61 B
ATOM 8311 CD2 LEU B 334 114.620 72.492 152.561 1.00 11.23 B
ATOM 8312 C LEU B 334 115.357 72.803 147.686 1.00 13.50 B
ATOM 8313 O LEU B 334 115.131 71.707 147.179 1.0014.75 B
ATOM 8314 N ASP B 335 116.370 73.581 147.322 1.00 15.34 B
ATOM 8315 CA ASP B 335 117.361 73.172 146.327 1.00 15.14 B
ATOM 8316 CB ASP B 335 118.118 74.400 145.805 1.00 14.22 B
ATOM 8317 CG ASP B 335 119.032 74.087 144.629 1.00 16.66 B
ATOM 8318 OD1 ASP B 335 119.627 72.985 144.583 1.00 16.63 B
ATOM 8319 OD2 ASP B 335 119.172 74.968 143.751 1.00 13.38 B
ATOM 8320 C ASP B 335 118.311 72.313 147.155 1.00 16.15 B
ATOM 8321 O ASP B 335 118.920 72.812 148.109 1.00 14.37 B
ATOM 8322 N VAL B 336 118.415 71.029 146.830 1.00 15.48 B
ATOM 8323 CA VAL B 336 119.308 70.147 147.576 1.00 15.33 B
ATOM 8324 CB VAL B 336 118.536 68.982 148.241 1.00 13.97 B
ATOM 8325 CG1 VAL B 336 117.717 69.522 149.409 1.00 15.74 B
ATOM 8326 CG2 VAL B 336 117.622 68.310 147.237 1.00 14.15 B
ATOM 8327 C VAL B 336 120.398 69.628 146.650 1.00 15.61 B
ATOM 8328 O VAL B 336 120.168 69.411 145.462 1.00 13.72 B
ATOM 8329 N VAL B 337 121.584 69.427 147.215 1.00 16.40 B
ATOM 8330 CA VAL B 337 122.748 69.014 146.450 1.00 17.33 B
ATOM 8331 CB VAL B 337 123.836 70.092 146.599 1.00 17.47 B
ATOM 8332 CG1 VAL B 337 124.926 69.914 145.552 1.00 17.07 B
ATOM 8333 CG2 VAL B 337 123.194 71.466 146.507 1.00 18.78 B
ATOM 8334 C VAL B 337 123.325 67.663 146.866 1.00 17.96 B
ATOM 8335 O VAL B 337 124.400 67.605 147.463 1.00 19.41 B
ATOM 8336 N PHE B 338 122.634 66.581 146.523 1.00 17.15 B
ATOM 8337 CA PHE B 338 123.094 65.244 146.885 1.00 17.51 B
ATOM 8338 CB PHE B 338 121.897 64.329 147.139 1.00 16.86 B
ATOM 8339 CG PHE B 338 121.029 64.780 148.265 1.00 18.43 B
ATOM 8340 CD1 PHE B 338 121.539 64.874 149.555 1.00 18.45 B
ATOM 8341 CD2 PHE B 338 119.698 65.122 148.041 1.00 19.21 B
ATOM 8342 CE1 PHE B 338 120.737 65.301 150.607 1.0018.84 B
ATOM 8343 CE2 PHE B 338 118.889 65.549 149.085 1.00 17.15 B
ATOM 8344 CZ PHE B 338 119.408 65.638 150.369 1.00 17.99 B
ATOM 8345 C PHE B 338 124.000 64.587 145.853 1.00 18.72 B
ATOM 8346 O PHE B 338 124.195 63.374 145.886 1.00 19.13 B
ATOM 8347 N ASN B 339 124.567 65.373 144.946 1.00 19.61 B
ATOM 8348 CA ASN B 339 125.431 64.803 143.916 1.00 20.33 B
ATOM 8349 CB ASN B 339 125.192 65.508 142.583 1.00 16.71 B
ATOM 8350 CG ASN B 339 125.416 67.000 142.677 1.00 18.30 B
ATOM 8351 OD1 ASN B 339 124.491 67.769 142.951 1.00 17.84 B
ATOM 8352 ND2 ASN B 339 126.655 67.418 142.468 1.00 15.95 B
ATOM 8353 C ASN B 339 126.918 64.870 144.257 1.00 21.32 B
ATOM 8354 O ASN B 339 127.738 64.208 143.609 1.00 20.58 B
ATOM 8355 N HIS B 340 127.268 65.662 145.267 1.00 19.56 B
ATOM 8356 CA HIS B 340 128.666 65.793 145.653 1.00 20.51 B
ATOM 8357 CB HIS B 340 129.429 66.548 144.564 1.00 19.58 B
ATOM 8358 CG HIS B 340 129.243 68.033 144.616 1.00 20.31 B
ATOM 8359 CD2 HIS B 340 128.341 68.842 144.013 1.00 19.39 B
ATOM 8360 ND 1 HIS B 340 130.034 68.854 145.392 1.00 22.73 B
ATOM 8361 CE1 HIS B 340 129.628 70.105 145.261 1.00 21.81 B
ATOM 8362 NE2 HIS B 340 128.602 70.125 144.431 1.00 19.89 B
ATOM 8363 C HIS B 340 128.803 66.555 146.965 1.00 21.47 B
ATOM 8364 O HIS B 340 127.820 67.059 147.503 1.00 20.96 B
ATOM 8365 N VAL B 341 130.031 66.631 147.471 1.00 20.40 B
ATOM 8366 CA VAL B 341 130.306 67.373 148.690 1.00 21.37 B
ATOM 8367 CB VAL B 341 130.672 66.454 149.891 1.00 20.58 B
ATOM 8368 CE1 VAL B 341 129.629 65.383 150.064 1.00 18.27 B
ATOM 8369 CG2 VAL B 341 132.061 65.860 149.696 1.00 22.21 B
ATOM 8370 C VAL B 341 131.488 68.301 148.436 1.00 22.65 B
ATOM 8371 O VAL B 341 132.173 68.193 147.417 1.00 22.38 B
ATOM 8372 N TYR B 342 131.711 69.218 149.368 1.0022.35 B
ATOM 8373 CA TYR B 342 132.825 70.141 149.270 1.00 23.99 B
ATOM 8374 CB TYR B 342 132.545 71.394 150.111 1.00 23.70 B
ATOM 8375 CG TYR B 342 133.608 72.458 149.993 1.0024.43 B
ATOM 8376 CD1 TYR B 342 134.411 72.800 151.084 1.00 26.86 B
ATOM 8377 CE1 TYR B 342 135.416 73.766 150.967 1.00 26.87 B
ATOM 8378 CD2 TYR B 342 133.833 73.108 148.779 1.0026.58 B
ATOM 8379 CE2 TYR B 342 134.834 74.069 148.649 1.00 27.72 B
ATOM 8380 CZ TYR B 342 135.621 74.392 149.746 1.0027.49 B
ATOM 8381 OH TYR B 342 136.619 75.326 149.602 1.00 29.95 B
ATOM 8382 C TYR B 342 134.049 69.397 149.810 1.00 24.44 B
ATOM 8383 O TYR B 342 134.013 68.847 150.917 1.00 24.87 B
ATOM 8384 N LYS B 343 135.119 69.364 149.022 1.00 25.17 B
ATOM 8385 CA LYS B 343 136.357 68.688 149.417 1.00 25.41 B
ATOM 8386 CB LYS B 343 137.078 69.488 150.503 1.00 25.59 B
ATOM 8387 CG LYS B 343 137.689 70.786 150.018 1.00 31.28 B
ATOM 8388 CD LYS B 343 138.545 71.416 151.110 1.00 36.07 B
ATOM 8389 CE LYS B 343 139.207 72.701 150.634 1.00 38.55 B
ATOM 8390 NZ LYS B 343 140.110 72.466 149.474 1.00 41.14 B
ATOM 8391 C LYS B 343 136.148 67.256 149.902 1.0024.83 B
ATOM 8392 O LYS B 343 136.131 66.990 151.106 1.0024.93 B
ATOM 8393 N ARG B 344 136.006 66.338 148.953 1.00 23.69 B
ATOM 8394 CA ARG B 344 135.801 64.927 149.251 1.0024.17 B
ATOM 8395 CB ARG B 344 136.005 64.091 147.980 1.00 24.96 B
ATOM 8396 CG ARG B 344 136.017 62.583 148.218 1.00 24.69 B
ATOM 8397 CD ARG B 344 136.347 61.825 146.937 1.00 27.99 B
ATOM 8398 NE ARG B 344 137.719 62.066 146.497 1.00 30.38 B
ATOM 8399 CZ ARG B 344 138.792 61.571 147.105 1.0031.75 B
ATOM 8400 NH1 ARG B 344 138.650 60.805 148.176 1.00 32.68 B
ATOM 8401 NH2 ARG B 344 140.009 61.854 146.658 1.00 32.97 B
ATOM 8402 C ARG B 344 136.701 64.368 150.349 1.00 25.54 B
ATOM 8403 O ARG B 344 136.221 63.739 151.294 1.0025.48 B
ATOM 8404 N GLU B 345 138.004 64.598 150.216 1.00 26.94 B
ATOM 8405 CA GLU B 345 138.990 64.075 11.160 1.00 29.10 B
ATOM 8406 CB GLU B 345 140.404 64.464 150.710 1.00 31.13 B
ATOM 8407 CG GLU B3 45 140.479 65.015 149.290 1.0040.14 B
ATOM 8408 CD GLU B 345 140.111 66.493 149.215 1.00 41.70 B
ATOM 8409 OE1 GLU B 345 139.677 66.946 148.135 1.00 40.37 B
ATOM 8410 OE2 GLU B 345 140.271 67.200 150.236 1.00 44.11 B
ATOM 8411 C GLU B 345 138.809 64.457 152.626 1.00 28.77 B
ATOM 8412 O GLU B 345 139.271 63.741 153.514 1.00 29.95 B
ATOM 8413 N ASN B 346 138.141 65.570 152.893 1.0026.55 B
ATOM 8414 CA ASN B 346 137.963 65.994 154.270 1.00 25.57 B
ATOM 8415 CB ASN B 346 138.505 67.407 154.451 1.00 28.11 B
ATOM 8416 CG ASN B 346 139.927 67.538 153.973 1.00 34.13 B
ATOM 8417 OD1 ASN B 346 140.793 66.724 154.320 1.00 34.78 B
ATOM 8418 ND2 ASN B 346 140.186 68.565 153.166 1.00 37.91 B
ATOM 8419 C ASN B 346 136.520 65.951 154.712 1.00 23.94 B
ATOM 8420 O ASN B 346 136.186 66.421 155.798 1.00 23.54 B
ATOM 8421 N SER B 347 135.664 65.384 153.872 1.00 20.84 B
ATOM 8422 CA SER B 347 134.249 65.300 154.197 1.00 18.79 B
ATOM 8423 CB SER B 347 133.433 64.948 152.946 1.00 17.65 B
ATOM 8424 OG SER B 347 133.753 63.645 152.477 1.00 17.34 B
ATOM 8425 C SER B 347 133.999 64.249 155.271 1.00 17.40 B
ATOM 8426 O SER B 347 134.801 63.332 155.464 1.00 16.23 B
ATOM 8427 N PRO B 348 132.893 64.395 156.011 1.00 17.01 B
ATOM 8428 CD PRO B 348 132.087 65.626 156.118 1.00 15.99 B
ATOM 8429 CA PRO B 348 132.532 63.446 157.065 1.00 16.33 B
ATOM 8430 CB PRO B 348 131.202 63.990 157.560 1.00 15.99 B
ATOM 8431 CG PRO B 348 131.424 65.467 157.470 1.00 16.89 B
ATOM 8432 C PRO B 348 132.411 62.036 156.484 1.00 16.27 B
ATOM 8433 O PRO B 348 132.765 61.053 157.130 1.00 18.62 B
ATOM 8434 N PHE B 349 131.902 61.952 155.260 1.00 15.21 B
ATOM 8435 CA PHE B 349 131.742 60.675 154.572 1.00 14.89 B
ATOM 8436 CB PHE B 349 131.206 60.890 153.148 1.00 13.81 B
ATOM 8437 CG PHE B 349 129.725 61.140 153.068 1.00 14.82 B
ATOM 8438 CD1 PHE B 349 128.840 60.503 153.932 1.00 15.57 B
ATOM 8439 CD2 PHE B 349 129.206 61.962 152.071 1.00 17.56 B
ATOM 8440 CE1 PHE B 349 127.460 60.676 153.804 1.00 15.82 B
ATOM 8441 CE2 PHE B 349 127.828 62.141 151.933 1.00 18.89 B
ATOM 8442 CZ PHE B 349 126.953 61.493 152.804 1.00 16.06 B
ATOM 8443 C PHE B 349 133.066 59.913 154.465 1.00 14.26 B
ATOM 8444 O PHE B 349 133.169 58.754 154.864 1.00 14.58 B
ATOM 8445 N GLU B 350 134.075 60.583 153.925 1.00 15.98 B
ATOM 8446 CA GLU B 350 135.388 59.985 153.708 1.00 18.24 B
ATOM 8447 CB GLU B 350 136.206 60.910 152.808 1.00 19.23 B
ATOM 8448 CG GLU B 350 137.503 60.332 152.287 1.00 23.32 B
ATOM 8449 CD GLU B 350 137.311 59.080 151.448 1.0023.95 B
ATOM 8450 OE 1 GLU B 350 136.219 58.883 150.880 1.00 23.62 B
ATOM 8451 OE2 GLU B 350 138.274 58.295 151.348 1.00 29.11 B
ATOM 8452 C GLU B 350 136.156 59.671 154.990 1.00 18.18 B
ATOM 8453 O GLU B 350 136.884 58.685 155.052 1.00 19.76 B
ATOM 8454 N LYS B 351 135.990 60.504 156.010 1.00 18.56 B
ATOM 8455 CA LYS B 351 136.673 60.293 157.280 1.00 20.51 B
ATOM 8456 CB LYS B 351 136.678 61.581 158.109 1.0021.59 B
ATOM 8457 CG LYS B 351 137.540 62.694 157.521 1.00 25.28 B
ATOM 8458 CD LYS B 351 137.731 63.824 158.523 1.0028.73 B
ATOM 8459 CE LYS B 351 138.718 64.856 158.005 1.00 32.60 B
ATOM 8460 NZ LYS B 351 140.033 64.225 157.662 1.00 37.26 B
ATOM 8461 C LYS B 351 136.021 59.178 158.088 1.00 20.05 B
ATOM 8462 O LYS B 35 136.654 58.583 158.962 1.00 20.62 B
ATOM 8463 N THR B 352 134.758 58.891 157.788 1.00 18.22 B
ATOM 8464 CA THR B 352 134.029 57.850 158.508 1.00 16.30 B
ATOM 8465 CB THR B 352 132.546 58.253 158.670 1.00 16.14 B
ATOM 8466 OG1 THR B 352 132.479 59.550 159.284 1.00 15.21 B
ATOM 8467 CG2 THR B 352 131.816 57.266 159.567 1.00 17.88 B
ATOM 8468 C THR B 352 134.161 56.478 157.838 1.00 14.94 B
ATOM 8469 O THR B 352 134.475 55.490 158.506 1.00 13.50 B
ATOM 8470 N VAL B 353 133.931 56.417 156.526 1.00 14.40 B
ATOM 8471 CA VAL B 353 134.056 55.169 155.770 1.00 14.48 B
ATOM 8472 CB VAL B 353 132.685 54.525 155.487 1.00 16.40 B
ATOM 8473 CG1 VAL B 353 132.881 53.099 154.989 1.00 17.19 B
ATOM 8474 CG2 VAL B 353 131.829 54.533 156.742 1.00 16.32 B
ATOM 8475 C VAL B 353 134.736 55.506 154.442 1.00 17.66 B
ATOM 8476 O VAL B 353 134.074 55.756 153.423 1.00 15.43 B
ATOM 8477 N PRO B 354 136.079 55.516 154.438 1.00 17.96 B
ATOM 8478 CD PRO B 354 136.914 55.073 155.566 1.00 18.37 B
ATOM 8479 CA PRO B 354 136.912 55.825 153.272 1.00 18.16 B
ATOM 8480 CB PRO B 354 138.336 55.573 153.783 1.00 19.01 B
ATOM 8481 CG PRO B 354 138.219 55.740 155.261 1.00 18.90 B
ATOM 8482 C PRO B 354 136.607 54.995 152.033 1.00 17.05 B
ATOM 8483 O PRO B 354 136.587 53.771 152.096 1.0018.75 B
ATOM 8484 N GLY B 355 136.375 55.674 150.913 1.00 18.65 B
ATOM 8485 CA GLY B 355 136.108 54.994 149.657 1.00 17.98 B
ATOM 8486 C GLY B 355 134.707 54.454 149.436 1.00 18.93 B
ATOM 8487 O GLY B 355 134.422 53.906 148.371 1.00 20.79 B
ATOM 8488 N TYR B 356 133.828 54.616 150.418 1.00 17.24 B
ATOM 8489 CA TYR B 356 132.462 54.113 150.305 1.00 17.83 B
ATOM 8490 CB TYR B 356 131.914 53.766 151.695 1.00 16.90 B
ATOM 8491 CG TYR B 356 130.484 53.261 151.677 1.00 18.57 B
ATOM 8492 CD1 TYRB356 130.201 51.918 151.425 1.0018.63 B
ATOM 8493 CE1 TYR B 356 128.882 51.451 151.391 1.00 19.51 B
ATOM 8494 CD2 TYR B 356 129.413 54.130 151.894 1.00 17.06 B
ATOM 8495 CE2 TYR B 356 128.093 53.677 151.857 1.00 18.03 B
ATOM 8496 CZ TYR B 356 127.833 52.337 151.605 1.00 21.46 B
ATOM 8497 OH TYR B 356 126.529 51.885 151.560 1.0021.49 B
ATOM 8498 C TYR B 356 131.452 55.043 149.624 1.00 18.20 B
ATOM 8499 O TYR B 356 130.710 54.615 148.744 1.00 16.76 B
ATOM 8500 N PHE B 357 131.425 56.307 150.040 1.00 17.11 B
ATOM 8501 CA PHE B 357 130.451 57.271 149.530 1.00 17.22 B
ATOM 8502 CB PHE B 357 130.216 58.341 150.595 1.00 14.59 B
ATOM 8503 CG PHE B 357 129.557 57.810 151.837 1.00 15.91 B
ATOM 8504 CD1 PHE B 357 128.192 57.537 151.851 1.00 14.84 B
ATOM 8505 CD2 PHE B 357 130.308 57.542 152.978 1.00 15.17 B
ATOM 8506 CE1 PHE B 357 127.576 57.003 152.982 1.00 14.92 B
ATOM 8507 CE2 PHE B 357 129.704 57.007 154.119 1.00 19.72 B
ATOM 8508 CZ PHE B 357 128.331 56.736 154.120 1.00 17.61 B
ATOM 8509 C PHE B 357 130.683 57.950 148.187 1.00 18.87 B
ATOM 8510 O PHE B 357 129.856 58.755 147.753 1.00 19.31 B
ATOM 8511 N PHE B 358 131.775 57.619 147.512 1.00 17.55 B
ATOM 8512 CA PHE B 358 132.059 58.259 146.243 1.00 20.32 B
ATOM 8513 CB PHE B 358 133.227 59.231 146.429 1.00 18.52 B
ATOM 8514 CG PHE B 358 132.961 60.292 147.465 1.00 18.17 B
ATOM 8515 CD1 PHE B 358 132.252 61.448 147.134 1.00 16.88 B
ATOM 8516 CD2 PHE B 358 133.363 60.111 148.781 1.00 14.18 B
ATOM 8517 CE1 PHE B 358 131.945 62.403 148.098 1.00 16.34 B
ATOM 8518 CE2 PHE B 358 133.059 61.065 149.758 1.00 17.70 B
ATOM 8519 CZ PHE B 358 132.347 62.212 149.413 1.00 16.26 B
ATOM 8520 C PHE B 358 132.349 57.248 145.142 1.00 22.06 B
ATOM 8521 O PHE B 358 132.858 56.160 145.406 1.0023.90 B
ATOM 8522 N ARG B 359 132.003 57.604 143.910 1.0022.96 B
ATOM 8523 CA ARG B 359 132.228 56.711 142.782 1.00 24.71 B
ATOM 8524 CB ARG B 359 131.255 57.021 141.639 1.00 23.45 B
ATOM 8525 CG ARG B 359 129.802 56.845 142.007 1.00 23.69 B
ATOM 8526 CD ARG B 359 128.911 56.742 140.782 1.0022.01 B
ATOM 8527 NE ARG B 359 127.507 56.700 141.178 1.00 21.53 B
ATOM 8528 CZ ARG B 359 126.637 57.679 140.956 1.00 20.40 B
ATOM 8529 NH1 ARG B 359 127.024 58.784 140.326 1.00 18.10 B
ATOM 8530 NH2 ARG B 359 125.386 57.561 141.386 1.00 18.55 B
ATOM 8531 C ARG B 359 133.655 56.825 142.270 1.00 26.86 B
ATOM 8532 O ARG B 359 134.332 57.830 142.488 1.00 26.85 B
ATOM 8533 N HIS B 360 134.104 5.780 141.588 1.00 31.55 B
ATOM 8534 CA HIS B 360 135.443 55.752 141.021 1.00 35.78 B
ATOM 8535 CB HIS B 360 136.335 54.803 141.834 1.0037.75 B
ATOM 8536 CG HIS B 360 136.478 55.196 143.275 1.00 39.76 B
ATOM 8537 CD2 HIS B 360 137.507 55.765 143.947 1.00 40.07 B
ATOM 8538 ND1 HIS B 360 135.458 55.049 144.192 1.00 41.16 B
ATOM 8539 CE1 HIS B 360 135.853 55.513 145.366 1.00 39.94 B
ATOM 8540 NE2 HIS B 360 137.092 55.953 145.243 1.00 39.88 B
ATOM 8541 C HIS B 360 135.362 55.296 139.565 1.00 37.37 B
ATOM 8542 O HIS B 360 134.524 54.463 139.219 1.00 36.51 B
ATOM 8543 N ASP B 361 136.223 55.853 138.715 1.00 40.12 B
ATOM 8544 CA ASP B 361 136.243 55.489 137.300 1.00 42.97 B
ATOM 8545 CB ASP B 361 137.030 56.535 136.491 1.00 43.74 B
ATOM 8546 CG ASP B 361 138.489 56.660 136.927 1.00 44.43 B
ATOM 8547 OD1 ASP B 361 139.159 57.603 136.451 1.00 43.36 B
ATOM 8548 OD2 ASP B 361 138.971 55.826 137.728 1.00 43.45 B
ATOM 8549 C ASP B 361 136.823 54.083 137.091 1.00 44.76 B
ATOM 8550 O ASP B 361 136.782 53.253 137.999 1.00 46.50 B
ATOM 8551 N GLU B 362 137.362 53.811 135.906 1.00 46.11 B
ATOM 8552 CA GLU B 362 137.908 52.487 135.623 1.00 48.73 B
ATOM 8553 CB GLU B 362 137.760 52.152 134.134 1.00 49.19 B
ATOM 8554 CG GLU B 362 138.644 52.978 133.212 1.00 51.86 B
ATOM 8555 CD GLU B 362 138.005 54.286 132.797 1.00 53.89 B
ATOM 8556 OE1 GLU B 362 137.564 55.045 133.685 1.00 54.84 B
ATOM 8557 OE2 GLU B 362 137.947 54.559 131.576 1.00 55.56 B
ATOM 8558 C GLU B 362 139.369 52.294 136.037 1.00 49.94 B
ATOM 8559 O GLU B 362 139.928 51.213 135.845 1.0050.37 B
ATOM 8560 N CYS B 363 139.989 53.329 136.598 1.00 50.61 B
ATOM 8561 CA CYS B 363 141.382 53.224 137.029 1.00 51.57 B
ATOM 8562 CB CYS B 363 142.240 54.265 136.306 1.00 51.88 B
ATOM 8563 SG CYS B 363 142.438 53.943 134.533 1.0055.60 B
ATOM 8564 C CYS B 363 141.543 53.368 138.545 1.0051.62 B
ATOM 8565 O CYS B 363 142.662 53.369 139.063 1.00 51.59 B
ATOM 8566 N GLY B 364 140.420 53.484 139.248 1.00 50.31 B
ATOM 8567 CA GLY B 364 140.458 53.614 140.692 1.00 49.43 B
ATOM 8568 C GLY B 364 140.410 55.039 141.212 1.0049.33 B
ATOM 8569 O GLY B 364 140.440 55.255 142.422 1.00 49.70 B
ATOM 8570 N LYS B 365 140.331 56.013 140.310 1.00 48.60 B
ATOM 8571 CA LYS B 365 140.286 57.418 140.709 1.00 47.07 B
ATOM 8572 CB LYS B 365 141.017 58.284 139.676 1.0048.54 B
ATOM 8573 CG LYS B 365 142.456 57.851 139.386 1.00 49.94 B
ATOM 8574 CD LYS B 365 143.278 57.681 140.663 1.00 49.96 B
ATOM 8575 CE LYS B 365 143.247 58.933 141.526 1.00 50.85 B
ATOM 8576 NZ LYS B 365 143.722 60.133 140.789 1.00 51.00 B
ATOM 8577 C LYS B 365 138.851 57.931 140.886 1.0045.82 B
ATOM 8578 O LYS B 365 137.928 57.479 140.202 1.0044.30 B
ATOM 8579 N PRO B 366 138.647 58.883 141.815 1.00 44.27 B
ATOM 8580 CD PRO B 366 139.631 59.434 142.764 1.00 44.48 B
ATOM 8581 CA PRO B 366 137.316 59.446 142.068 1.00 42.25 B
ATOM 8582 CB PRO B 366 137.591 60.517 143.120 1.00 42.42 B
ATOM 8583 CG PRO B 366 138.740 59.942 143.880 1.00 44.75 B
ATOM 8584 C PRO B 366 136.707 60.032 140.800 1.00 40.41 B
ATOM 8585 O PRO B 366 137.302 60.904 140.167 1.0039.92 B
ATOM 8586 N SER B 367 135.525 59.548 140.432 1.00 37.57 B
ATOM 8587 CA SER B 367 134.843 60.036 139.240 1.00 35.84 B
ATOM 8588 CB SER B 367 133.555 59.244 139.017 1.00 35.67 B
ATOM 8589 OG SER B 367 132.699 59.336 140.142 1.00 37.04 B
ATOM 8590 C SER B 367 134.532 61.528 139.371 1.00 34.77 B
ATOM 8591 O SER B 367 134.487 62.070 140.477 1.00 32.11 B
ATOM 8592 N ASN B 368 134.311 62.187 138.237 1.00 34.51 B
ATOM 8593 CA ASN B 368 134.035 63.618 138.232 1.00 33.90 B
ATOM 8594 CB ASN B 368 135.326 64.369 137.875 1.00 34.55 B
ATOM 8595 CG ASN B 368 135.204 65.869 138.058 1.00 34.80 B
ATOM 8596 OD1 ASN B 368 134.652 66.339 139.051 1.00 31.64 B
ATOM 8597 ND2 ASN B 368 135.734 66.628 137.104 1.00 34.80 B
ATOM 8598 C ASN B 368 132.902 64.002 137.271 1.00 33.68 B
ATOM 8599 O ASN B 368 133.031 64.938 136.477 1.00 32.15 B
ATOM 8600 N GLY B 369 131.790 63.275 137.355 1.00 33.66 B
ATOM 8601 CA GLY B 369 130.649 63.553 136.497 1.00 33.07 B
ATOM 8602 C GLY B 369 130.027 64.910 136.776 1.00 32.79 B
ATOM 8603 O GLY B 369 129.552 65.588 135.858 1.00 31.70 B
ATOM 8604 N THR B 370 130.025 65.307 138.045 1.00 31.38 B
ATOM 8605 CA THR B 370 129.465 66.595 138.434 1.00 30.97 B
ATOM 8606 CB THR B 370 129.348 66.736 139.973 1.00 30.13 B
ATOM 8607 OG1 THR B 370 130.634 66.538 140.575 1.00 29.22 B
ATOM 8608 CG2 THR B 370 128.357 65.718 140.533 1.00 31.15 B
ATOM 8609 C THR B 370 130.357 67.711 137.918 1.00 31.65 B
ATOM 8610 O THR B 370 129.889 68.814 137.634 1.00 32.05 B
ATOM 8611 N GLY B 371 131.649 67.418 137.805 1.00 31.91 B
ATOM 8612 CA GLY B 371 132.588 68.414 137.325 1.00 31.96 B
ATOM 8613 C GLY B 371 133.102 69.313 138.433 1.00 31.96 B
ATOM 8614 O GLY B 371 133.706 70.354 138.164 1.00 31.33 B
ATOM 8615 N VAL B 372 132.859 68.920 139.682 1.00 30.88 B
ATOM 8616 CA VAL B 372 133.316 69.701 140.825 1.00 30.63 B
ATOM 8617 CB VAL B 372 132.122 70.259 141.664 1.00 30.63 B
ATOM 8618 CG1 VAL B 372 131.455 71.404 140.919 1.00 27.49 B
ATOM 8619 CG2 VAL B 372 131.110 69.160 141.948 1.00 31.43 B
ATOM 8620 C VAL B 372 134.256 68.908 141.736 1.00 30.92 B
ATOM 8621 O VAL B 372 134.509 69.305 142.877 1.00 30.25 B
ATOM 8622 N GLY B 373 134.760 67.782 141.233 1.00 29.50 B
ATOM 8623 CA GLY B 373 135.700 66.985 142.003 1.00 28.94 B
ATOM 8624 C GLY B 373 135.286 65.624 142.534 1.00 28.60 B
ATOM 8625 O GLY B 373 136.150 64.815 142.876 1.00 28.93 B
ATOM 8626 N ASN B 374 133.987 65.360 142.628 1.00 26.70 B
ATOM 8627 CA ASN B 374 133.530 64.071 143.135 1.00 25.10 B
ATOM 8628 CB ASN B 374 133.776 63.967 144.645 1.00 25.57 B
ATOM 8629 CG ASN B 374 133.046 65.045 145.436 1.00 28.11 B
ATOM 8630 OD1 ASN B 374 133.545 66.162 145.597 1.00 30.80 B
ATOM 8631 ND2 ASN B 374 131.854 64.718 145.924 1.00 25.58 B
ATOM 8632 C ASN B 374 132.059 63.814 142.860 1.00 22.55 B
ATOM 8633 O ASN B 374 131.271 64.747 142.698 1.00 24.13 B
ATOM 8634 N ASP B 375 131.706 62.536 142.795 1.00 19.33 B
ATOM 8635 CA ASP B 375 130.330 62.113 142.566 1.00 18.19 B
ATOM 8636 CB ASP B 375 130.217 61.282 141.283 1.00 19.82 B
ATOM 8637 CG ASP B 375 130.616 62.053 140.038 1.00 20.66 B
ATOM 8.638 OD1 ASP B 375 130.822 61.401 138.991 1.00 21.69 B
ATOM 8639 OD2 ASP B 375 130.714 63.300 140.100 1.00 19.21 B
ATOM 8640 C ASP B 375 129.919 61.234 143.740 1.00 18.02 B
ATOM 8641 O ASP B 375 130.579 60.237 144.032 1.00 15.66 B
ATOM 8642 N ILE B 376 128.834 61.596 144.413 1.00 17.87 B
ATOM 8643 CA ILE B 376 128.358 60.794 145.529 1.00 18.53 B
ATOM 8644 CB ILE B 376 127.294 61.562 146.333 1.00 18.93 B
ATOM 8645 CG2 ILE B 376 126.527 60.612 147.244 1.00 17.07 B
ATOM 8646 CG1 ILE B 376 127.984 62.666 147.143 1.00 18.16 B
ATOM 8647 CD1 ILE B 376 127.053 63.526 147.955 1.00 19.30 B
ATOM 8648 C ILE B 376 127.782 59.483 145.001 1.00 19.06 B
ATOM 8649 O ILE B 376 126.947 59.483 144.099 1.00 20.64 B
ATOM 8650 N ALA B 377 128.240 58.369 145.560 1.00 18.12 B
ATOM 8651 CA ALA B 377 127.788 57.049 145.140 1.00 16.15 B
ATOM 8652 CB ALA B 377 128.781 55.996 145.618 1.00 17.55 B
ATOM 8653 C ALA B 377 126.399 56.758 145.695 1.00 16.37 B
ATOM 8654 O ALA B 377 126.222 55.884 146.551 1.00 13.19 B
ATOM 8655 N SER B 378 125.409 57.484 145.193 1.00 15.53 B
ATOM 8656 CA SER B 378 124.043 57.321 145.664 1.00 16.46 B
ATOM 8657 CB SER B 378 123.095 58.202 144.845 1.00 16.75 B
ATOM 8658 OG SER B 378 123.067 57.802 143.489 1.0019.58 B
ATOM 8659 C SER B 378 123.535 55.884 145.660 1.00 16.98 B
ATOM 8660 O SERB 378 122.649 55.549 146.444 1.00 15.59 B
ATOM 8661 N GLU B 379 124.101 55.037 144.800 1.00 16.95 B
ATOM 8662 CA GLU B 379 123.658 53.649 144.704 1.00 20.30 B
ATOM 8663 CB GLU B 379 124.137 52.998 143.397 1.00 21.26 B
ATOM 8664 CG GLU B 379 125.628 52.680 143.312 1.00 20.88 B
ATOM 8665 CD GLU B 379 126.461 53.866 142.862 1.0022.07 B
ATOM 8666 OE1 GLU B 379 127.612 53.649 142.429 1.00 24.98 B
ATOM 8667 OE2 GLU B 379 125.974 55.015 142.946 1.00 22.68 B
ATOM 8668 C GLU B 379 124.042 52.758 145.880 1.00 22.89 B
ATOM 8669 O GLU B 379 123.423 51.710 146.083 1.00 23.80 B
ATOM 8670 N ARG B 380 125.060 53.141 146.647 1.00 22.70 B
ATOM 8671 CA ARG B 380 125.423 52.335 147.807 1.00 23.51 B
ATOM 8672 CB ARG B 380 126.741 52.810 148.418 1.00 27.03 B
ATOM 8673 CG ARG B 380 127.928 52.026 147.886 1.00 31.12 B
ATOM 8674 CD ARG B 380 128.962 52.917 147.260 1.00 33.95 B
ATOM 8675 NE ARG B 380 129.644 52.237 146.163 1.00 40.05 B
ATOM 8676 CZ ARG B 380 130.740 52.694 145.565 1.00 41.72 B
ATOM 8677 NH1 ARG B 380 131.293 53.840 145.961 1.00 40.98 B
ATOM 8678 NH2 ARG B 380 131.279 52.009 144.564 1.00 40.77 B
ATOM 8679 C ARG B 380 124.272 52.485 148.790 1.00 23.55 B
ATOM 8680 O ARG B 380 123.755 53.588 148.987 1.00 23.25 B
ATOM 8681 N ARG B 381 123.869 51.379149.406 1.00 20.48 B
ATOM 8682 CA ARG B 381 122.732 51.401 150.309 1.00 20.20 B
ATOM 8683 CB ARG B 381 122.486 49.991 150.854 1.00 19.12 B
ATOM 8684 CG ARG B 381 122.075 49.028 149.744 1.00 21.35 B
ATOM 8685 CD ARG B 381 121.825 47.623 150.241 1.00 23.01 B
ATOM 8686 NE ARG B 381 120.639 47.530 151.083 1.00 25.25 B
ATOM 8687 CZ ARG B 381 120.292 46.436 151.753 1.00 26.59 B
ATOM 8688 NH1 ARG B 381 121.046 45.344 151.678 1.00 26.70 B
ATOM 8689 NH2 ARG B 381 119.195 46.431 152.498 1.00 26.87 B
ATOM 8690 C ARG B 381 122.723 52.439 151.426 1.00 19.85 B
ATOM 8691 O ARG B 381 121.683 53.050 151.682 1.00 19.89 B
ATOM 8692 N MET B 382 123.853 52.666 152.087 1.00 18.34 B
ATOM 8693 CA MET B 382 123.863 53.663 153.155 1.00 17.14 B
ATOM 8694 CB MET B 382 125.042 53.424 154.093 1.00 17.92 B
ATOM 8695 CG MET B 382 124.871 52.174 154.944 1.00 17.01 B
ATOM 8696 SD MET B 382 123.358 52.179 155.958 1.00 16.97 B
ATOM 8697 CE MET B 382 123.917 53.136 157.386 1.00 15.27 B
ATOM 8698 C MET B 382 123.860 55.102 152.629 1.00 17.61 B
ATOM 8699 O MET B 382 123.520 56.039 153.362 1.00 17.92 B
ATOM 8700 N ALA B 383 124.231 55.280 151.362 1.00 16.22 B
ATOM 8701 CA ALA B 383 124.217 56.606 150.741 1.00 15.35 B
ATOM 8702 CB ALA B 383 125.087 56.621 149.486 1.00 14.07 B
ATOM 8703 C ALA B 383 122.760 56.878 150.373 1.00 13.94 B
ATOM 8704 O ALA B 383 122.229 57.970 150.592 1.00 13.29 B
ATOM 8705 N ARG B 384 122.127 55.856 149.813 1.00 14.20 B
ATOM 8706 CA ARG B 384 120.728 55.914 149.424 1.00 15.12 B
ATOM 8707 CB ARG B 384 120.307 54.573 148.817 1.00 14.00 B
ATOM 8708 CG ARG B 384 118.802 54.382 148.684 1.00 17.82 B
ATOM 8709 CD ARG B 384 118.509 53.065 147.981 1.00 19.48 B
ATOM 8710 NE ARG B 384 119.063 53.066 146.630 1.00 20.81 B
ATOM 8711 CZ ARG B 384 119.382 51.970 145.952 1.0023.25 B
ATOM 8712 NH1 ARG B 384 119.205 50.778 146.502 1.00 24.08 B
ATOM 8713 NH2 ARG B 384 119.878 52.066 144.725 1.00 23.23 B
ATOM 8714 C ARG B 384 119.907 56.205 150.672 1.00 14.90 B
ATOM 8715 O ARG B 384 118.952 56.978 150.636 1.00 15.37 B
ATOM 8716 N LYS B 385 120.292 55.576 151.778 1.00 15.05 B
ATOM 8717 CA LYS B 385 119.619 55.772 153.057 1.00 13.41 B
ATOM 8718 CB LYS B 385 120.191 54.798 154.095 1.0014.85 B
ATOM 8719 CG LYS B 385 119.748 55.053 155.540 1.0014.39 B
ATOM 8720 CD LYS B 385 120.573 54.204 156.504 1.00 16.45 B
ATOM 8721 CE LYS B 385 120.322 54.572 157.956 1.00 15.01 B
ATOM 8722 NZ LYS B 385 118.948 54.228 158.382 1.00 16.27 B
ATOM 8723 C LYS B 385 119.815 57.213 153.535 1.00 14.06 B
ATOM 8724 O LYS B 385 118.875 57.864 154.000 1.0013.46 B
ATOM 8725 N PHE B 386 121.045 57.704 153.424 1.00 14.53 B
ATOM 8726 CA PHE B 386 121.368 59.067 153.842 1.00 15.82 B
ATOM 8727 CB PHE B 386 122.851 59.347 153.584 1.00 16.92 B
ATOM 8728 CG PHE B 386 123.275 60.754 153.897 1.0019.35 B
ATOM 8729 CD1 PHE B 386 123.182 61.755 152.937 1.00 20.02 B
ATOM 8730 CD2 PHE B 386 123.785 61.075 155.150 1.00 21.33 B
ATOM 8731 CE1 PHE B 386 123.597 63.055 153.222 1.00 22.21 B
ATOM 8732 CE2 PHE B 386 124.201 62.374 155.446 1.00 20.93 B
ATOM 8733 CZ PHE B 386 124.108 63.364 154.481 1.00 21.52 B
ATOM 8734 C PHE B 386 120.510 60.095 153.113 1.00 14.70 B
ATOM 8735 O PHE B 386 119.936 60.998 153.726 1.00 14.14 B
ATOM 8736 N ILE B 387 120.425 59.941 151.798 1.00 14.87 B
ATOM 8737 CA ILE B 387 119.656 60.846 150.960 1.00 13.54 B
ATOM 8738 CB ILE B 387 119.849 60.490 149.475 1.00 13.57 B
ATOM 8739 CG2 ILE B 387 118.849 61.249 148.609 1.00 13.64 B
ATOM 8740 CG1 ILE B 387 121.292 60.812 149.069 1.00 11.12 B
ATOM 8741 CD1 ILE B 387 121.677 60.293 147.716 1.00 9.86 B
ATOM 8742 C ILE B 387 118.180 60.811 151.315 1.00 15.17 B
ATOM 8743 O ILE B 387 117.532 61.860 151.412 1.00 16.64 B
ATOM 8744 N ALA B 388 117.647 59.610 151.519 1.00 14.46 B
ATOM 8745 CA ALA B 388 116.242 59.483 151.877 1.00 14.76 B
ATOM 8746 CB ALA B 388 115.821 58.019 151.860 1.00 16.28 B
ATOM 8747 C ALAB388 116.021 60.090 153.260 1.0014.91 B
ATOM 8748 O ALA B 388 115.060 60.828 153.463 1.00 14.36 B
ATOM 8749 N ASP B 389 116.913 59.781 154.205 1.00 14.64 B
ATOM 8750 CA ASP B 389 116.815 60.323 155.571 1.00 15.12 B
ATOM 8751 CB ASP B 389 118.008 59.890 156.435 1.00 12.66 B
ATOM 8752 CG ASP B 389 117.928 58.448 156.882 1.00 15.23 B
ATOM 8753 OD1 ASP B 389 118.918 57.973 157.485 1.00 17.06 B
ATOM 8754 OD2 ASP B 389 116.888 57.794 156.645 1.00 15.97 B
ATOM 8755 C ASP B 389 116.807 61.849 155.554 1.00 14.92 B
ATOM 8756 O ASP B 389 115.996 62.489 156.226 1.00 13.99 B
ATOM 8757 N CYS B 390 117.729 62.423 154.789 1.00 14.79 B
ATOM 8758 CA CYS B 390 117.854 63.870 154.707 1.00 14.85 B
ATOM 8759 CB CYS B 390 119.026 64.247 153.795 1.00 13.00 B
ATOM 8760 SG CYS B 390 119.568 65.947 154.007 1.00 14.55 B
ATOM 8761 C CYS B 390 116.575 64.520 154.203 1.0014.69 B
ATOM 8762 O CYS B 390 116.057 65.449 154.821 1.00 15.94 B
ATOM 8763 N VAL B 391 116.066 64.031 153.080 1.0015.63 B
ATOM 8764 CA VAL B 391 114.843 64.585 152.514 1.00 14.87 B
ATOM 8765 CB VAL B 391 114.432 63.855 151.219 1.00 14.52 B
ATOM 8766 CG1 VAL B 391 113.073 64.363 150.755 1.00 14.77 B
ATOM 8767 CG2 VAL B 391 115.479 64.099 150.131 1.00 12.75 B
ATOM 8768 C VAL B 391 113.697 64.493 153.514 1.00 15.04 B
ATOM 8769 O VAL B 391 112.940 65.450 153.693 1.00 15.21 B
ATOM 8770 N VAL B 392 113.566 63.345 154.167 1.00 14.71 B
ATOM 8771 CA VAL B 392 112.498 63.179 155.138 1.00 16.10 B
ATOM 8772 CB VAL B 392 112.410 61.720 155.630 1.00 17.08 B
ATOM 8773 CG1 VAL B 392 111.472 61.628 156.823 1.00 19.36 B
ATOM 8774 CG2 VAL B 392 111.903 60.831 154.506 1.00 16.86 B
ATOM 8775 C VAL B 392 112.694 64.119 156.326 1.00 16.03 B
ATOM 8776 O VAL B 392 111.730 64.682 156.844 1.00 17.09 B
ATOM 8777 N TYR B 393 113.937 64.299 156.758 1.00 15.35 B
ATOM 8778 CA TYR B 393 114.191 65.193 157.880 1.00 16.32 B
ATOM 8779 CB TYR B 393 115.661 65.171 158.276 1.00 16.14 B
ATOM 8780 CG TYR B 393 116.015 66.238 159.294 1.00 17.58 B
ATOM 8781 CD1 TYR B 393 115.659 66.104 160.637 1.00 16.62 B
ATOM 8782 CE1 TYR B 393 116.000 67.090 161.576 1.00 18.16 B
ATOM 8783 CD2 TYR B 393 116.714 67.383 158.910 1.00 17.94 B
ATOM 8784 CE2 TYR B 393 117.055 68.368 159.832 1.00 17.41 B
ATOM 8785 CZ TYR B 393 116.700 68.218 161.161 1.00 18.76 B
ATOM 8786 OH TYR B 393 117.067 69.195 162.064 1.00 17.39 B
ATOM 8787 C TYR B 393 113.777 66.636 157.573 1.00 16.93 B
ATOM 8788 O TYR B 393 113.105 67.274 158.384 1.00 17.66 B
ATOM 8789 N TRP B 394 114.166 67.160 156.412 1.00 15.07 B
ATOM 8790 CA TRP B 394 113.787 68.533 156.097 1.00 15.79 B
ATOM 8791 CB TRP B 394 114.472 69.030 154.815 1.00 11.79 B
ATOM 8792 CG TRP B 394 115.940 69.286 155.015 1.00 12.99 B
ATOM 8793 CD2 TRP B 394 116.543 70.489 155.514 1.00 12.70 B
ATOM 8794 CE2 TRP B 394 117.930 70.249 155.620 1.00 12.34 B
ATOM 8795 CE3 TRP B 394 116.045 71.746 155.885 1.00 13.72 B
ATOM 8796 CD1 TRP B 394 116.958 68.395 154.840 1.00 13.08 B
ATOM 8797 NE1 TRP B 394 118.158 68.966 155.200 1.00 12.84 B
ATOM 8798 CZ2 TRP B 394 118.828 71.220 156.082 1.00 13.66 B
ATOM 8799 CZ3 TRP B 394 116.939 72.713 156.344 1.00 14.94 B
ATOM 8800 CH2 TRP B 394 118.313 72.441 156.438 1.0014.21 B
ATOM 8801 C TRP B 394 112.274 68.680 155.999 1.00 15.50 B
ATOM 8802 O TRP B 394 111.728 69.738 156.315 1.00 16.72 B
ATOM 8803 N LEU B 395 111.595 67.616 155.585 1.00 15.75 B
ATOM 8804 CA LEU B 395 110.143 67.646 155.487 1.00 16.44 B
ATOM 8805 CB LEU B 395 109.629 66.411 154.741 1.00 17.62 B
ATOM 8806 CG LEU B 395 109.854 66.407 153.224 1.00 18.24 B
ATOM 8807 CD1 LEU B 395 109.503 65.046 152.642 1.00 16.14 B
ATOM 8808 CD2 LEU B 395 109.013 67.509 152.589 1.00 15.38 B
ATOM 8809 C LEU B 395 109.516 67.699 156.876 1.00 18.39 B
ATOM 8810 O LEU B 395 108.644 68.530 157.141 1.00 17.91 B
ATOM 8811 N GLU B 396 109.975 66.821 157.762 1.00 19.09 B
ATOM 8812 CA GLU B 396 109.452 66.745 159.122 1.00 22.22 B
ATOM 8813 CB GLU B 396 109.857 65.416 159.765 1.00 23.91 B
ATOM 8814 CG GLU B 396 109.318 64.193 159.050 1.00 28.46 B
ATOM 8815 CD GLU B 396 109.735 62.893 159.717 1.0031.62 B
ATOM 8816 OE1 GLU B 396 109.285 61.823 159.259 1.00 35.17 B
ATOM 8817 OE2 GLU B 396 110.513 62.936 160.697 1.00 31.64 B
ATOM 8818 C GLU B 396 109.891 67.889 160.033 1.00 22.79 B
ATOM 8819 O GLU B 396 109.065 68.513 160.701 1.00 23.45 B
ATOM 8820 N GLU B 397 111.188 68.164 160.060 1.00 21.74 B
ATOM 8821 CA GLU B 397 111.720 69.216 160.918 1.00 20.55 B
ATOM 8822 CB GLU B 397 113.224 68.997 161.118 1.00 19.12 B
ATOM 8823 CG GLU B 397 113.888 69.936 162.104 1.0019.00 B
ATOM 8824 CD GLU B 397 113.384 69.743 163.526 1.00 23.99 B
ATOM 8825 OE1 GLU B 397 112.784 68.680 163.812 1.00 22.99 B
ATOM 8826 OE2 GLU B 397 113.597 70.652 164.359 1.00 24.73 B
ATOM 8827 C GLU B 397 111.475 70.647 160.430 1.00 20.14 B
ATOM 8828 O GLU B 397 111.184 71.529 161.231 1.00 22.27 B
ATOM 8829 N TYR B 398 111.592 70.887 159.128 1.00 18.61 B
ATOM 8830 CA TYR B 398 111.408 72.240 158.608 1.00 17.35 B
ATOM 8831 CB TYR B 398 112.617 72.625 157.766 1.00 15.66 B
ATOM 8832 CG TYR B 398 113.847 72.799 158.618 1.00 16.42 B
ATOM 8833 CD1 TYR B 398 114.189 74.045 159.136 1.00 15.98 B
ATOM 8834 CE1 TYR B 398 115.292 74.196 159.982 1.00 18.15 B
ATOM 8835 CD2 TYR B 398 114.639 71.702 158.964 1.00 17.31 B
ATOM 8836 CE2 TYR B 398 115.741 71.845 159.806 1.00 17.88 B
ATOM 8837 CZ TYR B 398 116.060 73.093 160.309 1.00 16.79 B
ATOM 8838 OH TYR B 398 117.157 73.242 161.123 1.0018.76 B
ATOM 8839 C TYR B 398 110.114 72.495 157.847 1.00 17.70 B
ATOM 8840 O TYR B 398 109.876 73.610 157.369 1.00 15.95 B
ATOM 8841 N ASN B 399 109.292 71.454 157.737 1.00 15.91 B
ATOM 8842 CA ASN B 399 107.983 71.551 157.106 1.00 16.50 B
ATOM 8843 CB ASN B 399 107.077 72.385 158.024 1.00 15.57 B
ATOM 8844 CG ASN B 399 105.592 72.118 157.801 1.00 20.29 B
ATOM 8845 OD1 ASN B 399 105.194 71.029 157.380 1.00 21.50 B
ATOM 8846 ND2 ASN B 399 104.765 73.108 158.108 1.00 21.01 B
ATOM 8847 C ASN B 399 107.957 72.120 155.678 1.00 16.14 B
ATOM 8848 O ASN B 399 107.056 72.889 155.339 1.00 16.75 B
ATOM 8849 N VAL B 400 108.924 71.741 154.841 1.00 15.97 B
ATOM 8850 CA VAL B 400 108.956 72.239 153.471 1.00 13.15 B
ATOM 8851 CB VAL B 400 110.382 72.194 152.885 1.00 12.72 B
ATOM 8852 CG1 VALB400 111.277 73.125 153.699 1.0012.61 B
ATOM 8853 CG2 VAL B 400 110.926 70.775 152.882 1.00 12.69 B
ATOM 8854 C VAL B 400 107.968 71.515 152.557 1.00 14.78 B
ATOM 8855 O VAL B 400 107.310 70.559 152.974 1.00 14.41 B
ATOM 8856 N ASP B 401 107.880 71.959 151.307 1.00 14.26 B
ATOM 8857 CA ASP B 401 106.896 71.417 150.374 1.00 16.05 B
ATOM 8858 CB ASP B 401 105.898 72.533 150.050 1.00 16.71 B
ATOM 8859 CG ASP B 401 105.384 73.234 151.307 1.00 18.50 B
ATOM 8860 OD1 ASP B 401 104.461 72.698 151.957 1.00 17.70 B
ATOM 8861 OD2 ASP B 401 105.918 74.309 151.656 1.00 18.08 B
ATOM 8862 C ASP B 401 107.413 70.806 149.072 1.00 16.29 B
ATOM 8863 O ASP B 401 106.647 70.617 148.123 1.00 15.52 B
ATOM 8864 N GLY B 402 108.701 70.496 149.020 1.00 16.07 B
ATOM 8865 CA GLY B 402 109.247 69.916 147.811 1.00 17.35 B
ATOM 8866 C GLY B 402 110.729 70.175 147.696 1.00 16.57 B
ATOM 8867 O GLY B 402 111.294 70.943 148.478 1.00 17.81 B
ATOM 8868 N PHE B 403 111.360 69.536 146.719 1.00 15.00 B
ATOM 8869 CA PHE B 403 112.787 69.696 146.517 1.00 14.42 B
ATOM 8870 CB PHE B 403 113.539 68.512 147.124 1.00 12.46 B
ATOM 8871 CG PHE B 403 113.310 68.344 148.596 1.00 14.31 B
ATOM 8872 CD1 PHE B 403 112.157 67.723 149.068 1.00 13.71 B
ATOM 8873 CD2 PHE B 403 114.231 68.839 149.516 1.00 12.24 B
ATOM 8874 CE1 PHE B 403 111.921 67.595 150.437 1.00 12.65 B
ATOM 8875 CE2 PHE B 403 114.003 68.718 150.888 1.00 12.90 B
ATOM 8876 CZ PHE B 403 112.847 68.094 151.349 1.00 11.74 B
ATOM 8877 C PHE B 403 113.184 69.832 145.056 1.00 16.00 B
ATOM 8878 O PHE B 403 112.565 69.253 144.164 1.0015.56 B
ATOM 8879 N ARG B 404 114.225 70.619 144.827 1.00 15.00 B
ATOM 8880 CA ARG B 404 114.765 70.809 143.496 1.00 16.52 B
ATOM 8881 CB ARG B 404 114.895 72.301 143.181 1.00 16.43 B
ATOM 8882 CG ARG B 404 115.561 72.630 141.852 1.00 15.86 B
ATOM 8883 CD ARG B 404 117.072 72.784 142.009 1.00 17.82 B
ATOM 8884 NE ARG B 404 117.686 73.376 140.823 1.00 16.47 B
ATOM 8885 CZ ARG B 404 118.996 73.405 140.586 1.00 17.72 B
ATOM 8886 NH1 ARG B 404 119.845 72.874 141.454 1.00 15.28 B
ATOM 8887 NH2 ARG B 404 119.462 73.969 139.475 1.00 19.72 B
ATOM 8888 C ARG B 404 116.125 70.146 143.643 1.00 16.40 B
ATOM 8889 O ARG B 404 116.981 70.626 144.386 1.00 16.47 B
ATOM 8890 N PHE B 405 116.304 69.022 142.962 1.00 16.09 B
ATOM 8891 CA PHE B 405 117.547 68.266 143.052 1.00 15.78 B
ATOM 8892 CB PHE B 405 117.254 66.777 142.832 1.00 15.53 B
ATOM 8893 CG PHE B 405 116.428 66.159 143.921 1.00 16.80 B
ATOM 8894 CD1 PHE B 405 117.037 65.606 145.045 1.00 15.17 B
ATOM 8895 CD2 PHE B 405 115.039 66.144 143.835 1.00 14.95 B
ATOM 8896 CE1 PHE B 405 116.274 65.045 146.073 1.00 16.10 B
ATOM 8897 CE2 PHE B 405 114.268 65.587 144.855 1.00 15.68 B
ATOM 8898 CZ PHE B 405 114.888 65.036 145.979 1.00 13.89 B
ATOM 8899 C PHE B 405 118.642 68.721 142.101 1.00 15.50 B
ATOM 8900 O PHE B 405 118.544 68.546 140.888 1.00 14.00 B
ATOM 8901 N ASPB406 119.690 69.306 142.670 1.00 16.25 B
ATOM 8902 CA ASP B 406 120.829 69.763 141.886 1.00 16.43 B
ATOM 8903 CB ASP B 406 121.851 70.451 142.799 1.00 15.89 B
ATOM 8904 CG ASP B 406 123.018 71.046 142.026 1.00 18.89 B
ATOM 8905 OD1 ASP B 406 122.781 71.930 141.171 1.00 20.03 B
ATOM 8906 OD2 ASP B 406 124.171 70.627 142.272 1.00 16.96 B
ATOM 8907 C ASP B 406 121.450 68.529 141.220 1.00 15.68 B
ATOM 8908 O ASP B 406 121.640 67.501 141.867 1.00 15.11 B
ATOM 8909 N LEU B 407 121.745 68.638 139.927 1.00 15.54 B
ATOM 8910 CA LEU B 407 122.329 67.549 139.142 1.00 15.00 B
ATOM 8911 CB LEU B 407 123.843 67.466 139.381 1.00 13.76 B
ATOM 8912 CG LEU B 407 124.610 68.752 139.021 1.00 15.56 B
ATOM 8913 CD1 LEU B 407 126.101 68.539 139.255 1.00 15.48 B
ATOM 8914 CD2 LEU B 407 124.355 69.133 137.556 1.00 10.87 B
ATOM 8915 C LEU B 407 121.662 66.205 139.427 1.00 14.95 B
ATOM 8916 O LEU B 407 122.318 65.208 139.741 1.00 14.49 B
ATOM 8917 N LEU B 408 120.340 66.198 139.298 1.00 15.37 B
ATOM 8918 CA LEU B 408 119.532 65.010 139.522 1.00 16.10 B
ATOM 8919 CB LEU B 408 118.102 65.284 139.069 1.00 14.78 B
ATOM 8920 CG LEU B 408 116.905 64.592 139.730 1.00 16.57 B
ATOM 8921 CD1 LEU B 408 115.885 64.315 138.648 1.00 15.17 B
ATOM 8922 CD2 LEU B 408 117.293 63.314 140.446 1.00 12.60 B
ATOM 8923 C LEU B 408 120.098 63.838 138.720 1.00 16.77 B
ATOM 8924 O LEU B 408 120.035 62.688 139.154 1.00 8.75 B
ATOM 8925 N GLY B 409 120.653 64.151 137.550 1.00 16.57 B
ATOM 8926 CA GLY B 409 121.211 63.137 136.671 1.00 16.14 B
ATOM 8927 C GLY B 409 122.392 62.346 137.200 1.00 17.48 B
ATOM 8928 O GLY B 409 122.788 61.345 136.600 1.00 17.94 B
ATOM 8929 N ILE B 410 122.957 62.791 138.315 1.00 18.71 B
ATOM 8930 CA ILE B 410 124.099 62.120 138.935 1.00 18.98 B
ATOM 8931 CB ILE B 410 124.966 63.137 139.751 1.00 19.54 B
ATOM 8932 CG2 ILE B 410 126.047 62.409 140.555 1.00 19.95 B
ATOM 8933 CG1 ILE B 410 125.622 64.141 138.806 1.00 20.40 B
ATOM 8934 CD1 ILE B 410 126.638 63.526 137.861 1.00 18.52 B
ATOM 8935 C ILE B 410 123.599 61.017 139.870 1.00 18.81 B
ATOM 8936 O ILE B 410 124.334 60.083 140.191 1.00 20.09 B
ATOM 8937 N LEU B 411 122.348 61.131 140.308 1.00 18.11 B
ATOM 8938 CA LEU B 411 121.759 60.136 141.202 1.00 18.18 B
ATOM 8939 CB LEU B 411 120.601 60.748 142.000 1.00 17.35 B
ATOM 8940 CG LEU B 411 120.906 61.549 143.269 1.00 18.80 B
ATOM 8941 CD1 LEU B 411 121.800 62.738 142.959 1.00 17.89 B
ATOM 8942 CD2 LEU B 411 119.598 62.016 143.881 1.00 18.62 B
ATOM 8943 C LEU B 411 121.233 58.942 140.416 1.00 18.09 B
ATOM 8944 O LEU B 411 120.877 59.078 139.248 1.00 18.46 B
ATOM 8945 N ASP B 412 121.189 57.772 141.052 1.0018.54 B
ATOM 8946 CA ASP B 412 120.663 56.582 140.386 1.00 19.14 B
ATOM 8947 CB ASP B 412 121.290 55.292 140.942 1.00 19.58 B
ATOM 8948 CG ASP B 412 120.938 55.043 142.394 1.00 21.42 B
ATOM 8949 OD1 ASP B 412 121.397 55.822 143.259 1.00 19.85 B
ATOM 8950 OD2 ASP B 412 120.205 54.063 142.663 1.00 20.43 B
ATOM 8951 C ASP B 412 119.147 56.548 140.560 1.00 18.22 B
ATOM 8952 O ASP B 412 118.607 57.044 141.553 1.00 17.55 B
ATOM 8953 N ILE B 413 118.465 55.968 139.580 1.00 18.50 B
ATOM 8954 CA ILE B 413 117.013 55.891 139.591 1.00 18.10 B
ATOM 8955 CB ILE B 413 116.503 55.230 138.294 1.00 18.88 B
ATOM 8956 CG2 ILE B 413 114.999 54.998 138.378 1.00 17.83 B
ATOM 8957 CG1 ILE B 413 116.857 56.124 137.103 1.00 20.51 B
ATOM 8958 CD1 ILE B 413 116.514 55.535 135.747 1.00 23.77 B
ATOM 8959 C ILE B 413 116.394 55.190 140.796 1.00 18.42 B
ATOM 8960 O ILE B 413 115.412 55.675 141.353 1.00 19.79 B
ATOM 8961 N ASP B 414 116.950 54.055 141.206 1.00 17.84 B
ATOM 8962 CA ASP B 414 116.389 53.348 142.348 1.00 17.06 B
ATOM 8963 CB ASP B 414 117.159 52.053 142.607 1.00 19.38 B
ATOM 8964 CG ASP B 414 116.812 50.951 141.608 1.00 21.50 B
ATOM 8965 OD1 ASP B 414 117.392 49.854 141.718 1.00 24.24 B
ATOM 8966 OD2 ASP B 414 115.961 51.172 140.720 1.00 20.96 B
ATOM 8967 C ASP B 414 116.389 54.220 143.605 1.00 17.00 B
ATOM 8968 O ASP B 414 115.438 54.199 144.386 1.00 15.80 B
ATOM 8969 N THR B 415 117.455 54.988 143.799 1.00 16.33 B
ATOM 8970 CA THR B 415 117.549 55.863 144.964 1.00 16.98 B
ATOM 8971 CB THR B 415 118.938 56.540 145.041 1.00 17.21 B
ATOM 8972 OG1 THR B 415 119.923 55.555 145.370 1.00 16.66 B
ATOM 8973 CG2 THR B 415 118.950 57.654 146.087 1.00 16.15 B
ATOM 8974 C THR B 415 116.463 56.935 144.891 1.00 16.13 B
ATOM 8975 O THR B 415 115.764 57.188 145.870 1.00 16.15 B
ATOM 8976 N VAL B 416 116.316 57.550 143.722 1.00 14.45 B
ATOM 8977 CA VAL B 416 115.312 58.588 143.537 1.00 15.85 B
ATOM 8978 CB VAL B 416 115.397 59.187 142.115 1.00 16.94 B
ATOM 8979 CG1 VAL B 416 114.219 60.117 141.851 1.00 15.09 B
ATOM 8980 CG2 VAL B 416 116.710 59.958 141.975 1.00 16.47 B
ATOM 8981 C VAL B 416 113.910 58.052 143.798 1.00 17.66 B
ATOM 8982 O VAL B 416 113.111 58.703 144.469 1.00 18.47 B
ATOM 8983 N LEU B 417 113.615 56.861 143.280 1.00 18.64 B
ATOM 8984 CA LEU B 417 112.304 56.254 143.483 1.00 19.29 B
ATOM 8985 CB LEU B 417 112.142 55.014 142.596 1.00 19.49 B
ATOM 8986 CG LEU B 417 112.109 55.289 141.087 1.00 19.75 B
ATOM 8987 CD1 LEU B 417 111.861 54.000 140.320 1.00 17.70 B
ATOM 8988 CD2 LEU B 417 111.009 56.300 140.792 1.00 19.80 B
ATOM 8989 C LEU B 417 112.115 55.881 144.947 1.00 20.59 B
ATOM 8990 O LEU B 417 111.011 55.994 145.495 1.00 21.64 B
ATOM 8991 N TYR B 418 113.198 55.445 145.584 1.00 19.75 B
ATOM 8992 CA TYR B 418 113.143 55.074 146.993 1.00 18:49 B
ATOM 8993 CB TYR B 418 114.441 54.377 147.414 1.00 18.61 B
ATOM 8994 CG TYR B 418 114.513 54.026 148.886 1.00 20.28 B
ATOM 8995 CD1 TYR B 418 115.159 54.868 149.793 1.00 20.49 B
ATOM 8996 CE1 TYR B 418 115.234 54.550 151.149 1.00 20.46 B
ATOM 8997 CD2 TYR B 418 113.936 52.852 149.373 1.00 20.62 B
ATOM 8998 CE2 TYR B 418 114.002 52.525 150.728 1.00 22.15 B
ATOM 8999 CZ TYR B 418 114.654 53.378 151.608 1.00 22.19 B
ATOM 9000 OH TYR B 418 114.733 53.055 152.941 1.00 24.64 B
ATOM 9001 C TYR B 418 112.886 56.308 147.852 1.00 18.66 B
ATOM 9002 O TYR B 418 112.057 56.264 148.762 1.00 19.83 B
ATOM 9003 N MET B 419 113.572 57.417 147.574 1.00 18.62 B
ATOM 9004 CA MET B 419 113.320 58.610 148.370 1.00 17.65 B
ATOM 9005 CB MET B 419 114.417 59.665 148.177 1.00 18.36 B
ATOM 9006 CG MET B 419 114.251 60.591 147.008 1.00 21.51 B
ATOM 9007 SD MET B 419 113.109 61.982 147.195 1.00 18.81 B
ATOM 9008 CE MET B 419 113.031 62.448 145.498 1.00 17.28 B
ATOM 9009 C MET B 419 111.951 59.187 148.023 1.00 17.62 B
ATOM 9010 O MET B 419 111.304 59.783 148.875 1.00 17.03 B
ATOM 9011 N LYS B 420 111.499 58.997 146.784 1.00 17.82 B
ATOM 9012 CA LYS B 420 110.184 59.509 146.396 1.00 20.65 B
ATOM 9013 CB LYS B 420 109.859 59.195 144.930 1.00 19.60 B
ATOM 9014 CG LYS B 420 108.498 59.750 144.511 1.00 23.47 B
ATOM 9015 CD LYS B 420 108.108 59.369 143.078 1.0026.55 B
ATOM 9016 CE LYS B 420 107.726 57.905 142.984 1.00 26.71 B
ATOM 9017 NZ LYS B 420 107.257 57.536 141.625 1.00 29.62 B
ATOM 9018 C LYS B 420 109.087 58.903 147.268 1.00 20.16 B
ATOM 9019 O LYS B 420 108.263 59.623 147.832 1.00 17.90 B
ATOM 9020 N GLU B 421 109.071 57.576 147.362 1.00 21.92 B
ATOM 9021 CA GLU B 421 108.072 56.885 148.173 1.00 25.45 B
ATOM 9022 CB GLU B 421 108.339 55.373 148.180 1.00 29.81 B
ATOM 9023 CG GLU B 421 108.084 54.691 146.841 1.00 40.35 B
ATOM 9024 CD GLU B 421 108.563 53.240 146.803 1.0047.34 B
ATOM 9025 OE1 GLU B 421 108.109 52.432 147.648 1.00 49.96 B
ATOM 9026 OE2 GLU B 421 109.394 52.907 145.922 1.00 50.53 B
ATOM 9027 C GLU B 421 108.052 57.419 149.610 1.00 25.03 B
ATOM 9028 O GLU B 421 106.981 57.683 150.163 1.00 25.23 B
ATOM 9029 N LYS B 422 109.230 57.585 150.209 1.00 22.70 B
ATOM 9030 CA LYS B 422 109.312 58.090 151.576 1.00 25.00 B
ATOM 9031 CB LYS B 422 110.716 57.878 152.143 1.0026.58 B
ATOM 9032 CG LYS B 422 110.996 56.453 152.573 1.00 30.33 B
ATOM 9033 CD LYS B 422 112.332 56.360 153.280 1.0034.75 B
ATOM 9034 CE LYS B 422 112.558 54.973 153.861 1.00 38.20 B
ATOM 9035 NZ LYS B 422 113.767 54.936 154.745 1.00 40.11 B
ATOM 9036 C LYS B 422 108.939 59.565 151.692 1.00 24.83 B
ATOM 9037 O LYS B 422 108.285 59.974 152.652 1.00 23.36 B
ATOM 9038 N ALA B 423 109.351 60.363 150.713 1.00 24.08 B
ATOM 9039 CA ALA B 423 109.049 61.793 150.721 1.00 25.10 B
ATOM 9040 CB ALA B 423 109.824 62.504 149.601 1.00 22.91 B
ATOM 9041 C ALA B 423 107.548 62.061 150.576 1.0024.74 B
ATOM 9042 O ALA B 423 106.967 62.825 151.354 1.00 23.52 B
ATOM 9043 N THR B 424 106.925 61.441 149.578 1.00 23.25 B
ATOM 9044 CA THR B 424 105.494 61.629 149.356 1.00 24.84 B
ATOM 9045 CB THR B 424 105.035 60.977 148.029 1.00 22.52 B
ATOM 9046 OG1 THR B 424 105.297 59.574 148.066 1.00 23.21 1 B

ATOM 9047 CG2 THR B 424 105.775 61.591 146.850 1.00 21.90 B
ATOM 9048 C THR B 424 104.684 61.044 150.516 1.00 25.61 B
ATOM 9049 O THR B 424 103.617 61.550 150.860 1.0026.99 B
ATOM 9050 N LYS B 425 105.187 59.975 151.119 1.00 25.91 B
ATOM 9051 CA LYS B 425 104.488 59.380 152.245 1.00 27.38 B
ATOM 9052 CB LYS B 425 105.170 58.078 152.668 1.00 30.48 B
ATOM 9053 CG LYS B 425 104.437 57.316 153.759 1.0035.87 B
ATOM 9054 CD LYS B 425 105.213 56.068 154.178 1.00 39.99 B
ATOM 9055 CE LYS B 425 104.508 55.329 155.313 1.00 41.97 B
ATOM 9056 NZ LYS B 425 105.288 54.162 155.819 1.00 42.79 B
ATOM 9057 C LYS B 425 104.536 60.405 153.380 1.00 26.39 B
ATOM 9058 O LYS B 425 103.519 60.702 154.011 1.00 24.86 B
ATOM 9059 N ALA B 426 105.720 60.962 153.614 1.00 24.64 B
ATOM 9060 CA ALA B 426 105.899 61.959 154.662 1.00 23.52 B
ATOM 9061 CB ALA B 426 107.376 62.288 154.830 1.00 22.55 B
ATOM 9062 C ALA B 426 105.115 63.234 154.366 1.0024.07 B
ATOM 9063 O ALA B 426 104.719 63.949 155.282 1.00 24.41 B
ATOM 9064 N LYS B 427 104.888 63.530 153.092 1.0025.06 B
ATOM 9065 CA LYS B 427 104.146 64.738 152.754 1.00 25.84 B
ATOM 9066 CB LYS B 427 105.056 65.957 152.837 1.00 27.45 B
ATOM 9067 CG LYS B 427 104.290 67.257 153.022 1.00 29.18 B
ATOM 9068 CD LYS B 427 105.239 68.422 153.138 1.00 31.24 B
ATOM 9069 CE LYS B 427 104.649 69.518 153.996 1.00 32.74 B
ATOM 9070 NZ LYS B 427 104.431 69.059 155.394 1.00 34.24 B
ATOM 9071 C LYS B 427 103.492 64.698 151.382 1.00 25.32 B
ATOM 9072 O LYS B 427 104.112 65.042 150.376 1.00 26.58 B
ATOM 9073 N PRO B 428 102.219 64.288 151.328 1.00 24.13 B
ATOM 9074 CD PRO B 428 101.387 63.874 152.471 1.00 23.79 B
ATOM 9075 CA PRO B 428 101.465 64.201 150.074 1.00 23.21 B
ATOM 9076 CB PRO B 428 100.066 63.788 150.538 1.00 22.08 B
ATOM 9077 CG PRO B 428 100.332 63.026 151.805 1.00 23.71 B
ATOM 9078 C PRO B 428 101.432 65.526 149.309 1.0022.82 B
ATOM 9079 O PRO B 428 101.310 66.594 149.906 1.00 22.16 B
ATOM 9080 N GLY B 429 101.547 65.444 147.988 1.0021.65 B
ATOM 9081 CA GLY B 429 101.490 66.637 147.164 1.00 20.03 B
ATOM 9082 C GLY B 429 102.771 67.424 146.974 1.00 19.75 B
ATOM 9083 O GLY B 429 102.763 68.429 146.272 1.00 21.10 B
ATOM 9084 N ILE B 430 103.871 66.998 147.585 1.00 19.50 B
ATOM 9085 CA ILE B 430 105.119 67.735 147.419 1.00 20.51 B
ATOM 9086 CB ILE B 430 106.238 67.213 148.343 1.00 22.50 B
ATOM 9087 CG2 ILE B 430 105.836 67.384 149.795 1.00 25.30 B
ATOM 9088 CG1 ILE B 430 106.551 65.753 148.015 1.00 23.17 B
ATOM 9089 CD1 ILE B 430 107.753 65.229 148.785 1.00 27.49 B
ATOM 9090 C ILE B 430 105.638 67.649 145.991 1.00 18.16 B
ATOM 9091 O ILE B 430 105.378 66.679 145.282 1.00 16.99 B
ATOM 9092 N LEU B 431 106.374 68.672 145.576 1.00 16.23 B
ATOM 9093 CA LEU B 431 106.950 68.692 144.244 1.00 15.30 B
ATOM 9094 CB LEU B 431 107.008 70.119 143.706 1.00 15.37 B
ATOM 9095 CG LEU B 431 105.693 70.893 143.682 1.00 16.73 B
ATOM 9096 CD1 LEU B 431 105.922 72.225 142.982 1.00 15.79 B
ATOM 9097 CD2 LEU B 431 104.622 70.080 142.964 1.00 16.59 B
ATOM 9098 C LEU B 431 108.363 68.123 144.326 1.00 15.46 B
ATOM 9099 O LEU B 431 109.136 68.492 145.215 1.00 15.92 B
ATOM 9100 N LEU B 432 108.696 67.227 143.403 1.00 13.06 B
ATOM 9101 CA LEU B 432 110.019 66.619 143.372 1.00 12.83 B
ATOM 9102 CB LEU B 432 109.950 65.165 143.847 1.00 13.30 B
ATOM 9103 CG LEU B 432 109.479 64.886 145.278 1.00 16.27 B
ATOM 9104 CD1 LEU B 432 109.266 63.385 145.447 1.00 13.59 B
ATOM 9105 CD2 LEU B 432 110.508 65.405 146.283 1.00 14.88 B
ATOM 9106 C LEU B 432 110.519 66.655 141.941 1.00 13.63 B
ATOM 9107 O LEU B 432 110.013 65.927 141.084 1.00 15.10 B
ATOM 9108 N PHE B 433 111.505 67.506 141.682 1.00 13.26 B
ATOM 9109 CA PHE B 433 112.063 67.634 140.347 1.00 13.59 B
ATOM 9110 CB PHE B 433 111.272 68.674 139.547 1.00 12.52 B
ATOM 9111 CG PHE B 433 111.208 70.031 140.209 1.00 4.48 B
ATOM 9112 CD1 PHE B 433 110.298 70.280 141.232 1.00 13.08 B
ATOM 9113 CD2 PHE B 433 112.088 71.048 139.833 1.00 10.68 B
ATOM 9114 CE1 PHE B 433 110.265 71.525 141.876 1.00 13.53 B
ATOM 9115 CE2 PHE B 433 112.063 72.290 140.467 1.00 10.70 B
ATOM 9116 CZ PHE B 433 111.151 72.530 141.491 1.00 12.93 B
ATOM 9117 C PHE B 433 113.533 68.043 140.416 1.00 15.51 B
ATOM 9118 O PHE B 433 114.070 68.310 141.493 1.00 15.48 B
ATOM 9119 N GLY B 434 114.179 68.087 139.257 1.00 15.50 B
ATOM 9120 CA GLY B 434 115.573 68.474 139.213 1.00 17.00 B
ATOM 9121 C GLY B 434 116.101 68.540 137.799 1.00 17.93 B
ATOM 9122 O GLY B 434 115.368 68.317 136.835 1.00 20.95 B
ATOM 9123 N GLU B 435 117.379 68.857 137.666 1.00 19.69 B
ATOM 9124 CA GLU B 435 117.985 68.939 136.349 1.00 21.65 B
ATOM 9125 CB GLU B 435 119.058 70.030 136.340 1.0023.17 B
ATOM 9126 CG GLU B 435 120.006 69.977 137.515 1.00 26.84 B
ATOM 9127 CD GLU B 435 120.875 71.221 137.631 1.0030.02 B
ATOM 9128 OE1 GLU B 435 121.747 71.239 138.529 1.00 23.78 B
ATOM 9129 OE2 GLU B 435 120.684 72.176 136.831 1.00 32.08 B
ATOM 9130 C GLU B 435 118.570 67.579 135.984 1.00 22.37 B
ATOM 9131 O GLU B 435 119.631 67.187 136.475 1.00 22.52 B
ATOM 9132 N GLY B 436 117.855 66.857 135.130 1.00 22.44 B
ATOM 9133 CA GLY B 436 118.299 65.540 134.716 1.00 24.40 B
ATOM 9134 C GLY B 436 119.211 65.556 133.506 1.0025.67 B
ATOM 9135 O GLY B 436 118.938 64.884 132.510 1.0025.95 B
ATOM 9136 N TRP B 437 120.293 66.326 133.586 1.00 26.75 B
ATOM 9137 CA TRP B 437 121.254 66.408 132.490 1.00 28.20 B
ATOM 9138 CB TRP B 437 122.276 67.522 132.751 1.00 29.59 B
ATOM 9139 CG TRP B 437 121.693 68.874 133.101 1.00 32.87 B
ATOM 9140 CD2 TRP B 437 120.473 69.456 132.608 1.00 32.72 B
ATOM 9141 CE2 TRP B 437 120.350 70.734 133.205 1.00 32.38 B
ATOM 9142 CE3 TRP B 437 119.475 69.024 131.720 1.00 32.28 B
ATOM 9143 CD1 TRP B 437 122.239 69.800 133.947 1.00 32.35 B
ATOM 9144 NE1 TRP B 437 121.439 70.914 134.015 1.00 32.97 B
ATOM 9145 CZ2 TRP B 437 119.268 71.585 132.945 1.00 30.35 B
ATOM 9146 CZ3 TRP B 437 118.398 69.872 131.459 1.00 32.70 B
ATOM 9147 CH2 TRP B 437 118.306 71.141 132.073 1.00 32.53 B
ATOM 9148 C TRP B 437 121.994 65.072 132.401 1.00 28.88 B
ATOM 9149 O TRP B 437 122.199 64.402 133.416 1.00 27.56 B
ATOM 9150 N ASP B 438 122.385 64.674 131.194 1.0029.77 B
ATOM 9151 CA ASP B 438 123.125 63.431 131.046 1.00 31.44 B
ATOM 9152 CB ASP B 438 122.963 62.869 129.633 1.00 35.35 B
ATOM 9153 CG ASP B 438 123.701 61.555 129.445 1.00 40.95 B
ATOM 9154 OD1 ASP B 438 123.753 60.760 130.408 1.00 44.87 B
ATOM 9155 OD2 ASP B 438 124.219 61.311 128.335 1.00 45.51 B
ATOM 9156 C ASP B 438 124.589 63.754 131.339 1.00 30.37 B
ATOM 9157 O ASP B 438 125.246 64.447 130.563 1.00 30.70 B
ATOM 9158 N LEU B 439 125.094 63.265 132.467 1.00 28.65 B
ATOM 9159 CA LEU B 439 126.472 63.544 132.853 1.00 27.72 B
ATOM 9160 CB LEU B 439 126.492 64.241 134.214 1.00 30.42 B
ATOM 9161 CG LEU B 439 125.592 65.482 134.328 1.00 30.90 B
ATOM 9162 CD1 LEU B 439 125.582 65.967 135.767 1.00 31.96 B
ATOM 9163 CD2 LEU B 439 126.079 66.581 133.400 1.00 30.10 B
ATOM 9164 C LEU B 439 127.354 62.304 132.882 1.00 27.45 B
ATOM 9165 O LEU B 439 126.879 61.186 133.084 1.00 27.88 B
ATOM 9166 N ALA B 440 128.650 62.522 132.692 1.00 26.83 B
ATOM 9167 CA ALA B 440 129.633 61.447 132.650 1.00 27.40 B
ATOM 9168 CB ALA B 440 130.870 61.912 131.886 1.0024.95 B
ATOM 9169 C ALAB440 130.052 60.882 134.001 1.00 27.72 B
ATOM 9170 O ALA B 440 131.210 61.017 134.406 1.00 29.31 B
ATOM 9171 N THR B 441 129.119 60.243 134.696 1.00 26.30 B
ATOM 9172 CA THR B 441 129.434 59.636 135.979 1.00 23.80 B
ATOM 9173 CB THR B 441 128.417 60.048 137.070 1.00 21.64 B
ATOM 9174 OG1 THR B 441 128.810 59.478 138.324 1.00 21.71 B
ATOM 9175 CG2 THR B 441 127.017 59.580 136.709 1.00 20.89 B
ATOM 9176 C THR B 441 129.393 58.134 135.733 1.00 24.00 B
ATOM 9177 O THR B 441 128.646 57.663 134.883 1.00 24.18 B
ATOM 9178 N PRO B 442 130.201 57.359 136.467 1.0025.42 B
ATOM 9179 CD PRO B 442 131.196 57.775 137.471 1.00 25.48 B
ATOM 9180 CA PRO B 442 130.223 55.906 136.277 1.00 26.82 B
ATOM 9181 CB PRO B 442 131.447 55.475 137.091 1.00 26.89 B
ATOM 9182 CG PRO B 442 131.480 56.485 138.197 1.0027.16 B
ATOM 9183 C PRO B 442 128.960 55.120 136.639 1.00 26.31 B
ATOM 9184 O PRO B 442 128.927 54.392 137.627 1.0027.32 B
ATOM 9185 N LEU B 443 127.926 55.277 135.822 1.00 26.23 B
ATOM 9186 CA LEU B 443 126.657 54.567 135.989 1.0025.56 B
ATOM 9187 CB LEU B 443 125.604 55.431 136.688 1.0025.45 B
ATOM 9188 CG LEU B 443 125.492 55.455 138.212 1.00 25.43 B
ATOM 9189 CD1 LEU B 443 124.328 56.353 138.590 1.00 23.82 B
ATOM 9190 CD2 LEU B 443 125.270 54.054 138.758 1.00 23.65 B
ATOM 9191 C LEU B 443 126.176 54.286 134.579 1.00 24.51 B
ATOM 9192 O LEU B 443 126.319 55.128 133.701 1.00 25.28 B
ATOM 9193 N PRO B 444 125.620 53.095 134.334 1.00 25.06 B
ATOM 9194 CD PRO B 444 125.561 51.866 135.143 1.00 24.31 B
ATOM 9195 CA PRO B 444 125.158 52.846 132.968 1.00 24.20 B
ATOM 9196 CB PRO B 444 124.625 51.420 133.040 1.00 25.01 B
ATOM 9197 CG PRO B 444 125.516 50.791 134.077 1.00 25.27 B
ATOM 9198 C PRO B 444 124.080 53.859 132.610 1.00 22.97 B
ATOM 9199 O PRO B 444 123.263 54.227 133.448 1.00 22.57 B
ATOM 9200 N HIS B 445 124.099 54.306 131.362 1.00 23.40 B
ATOM 9201 CA HIS B 445 123.153 55.291 130.854 1.0024.08 B
ATOM 9202 CB HIS B 445 123.173 55.258 129.324 1.00 24.66 B
ATOM 9203 CG HIS B 445 122.303 56.294 128.687 1.00 28.16 B
ATOM 9204 CD2 HIS B 445 121.132 56.185 128.014 1.00 29.17 B
ATOM 9205 ND1 HIS B 445 122.597 57.641 128.728 1.00 30.20 B
ATOM 9206 CE1 HIS B 445 121.645 58.317 128.109 1.00 31.27 B
ATOM 9207 NE2 HIS B 445 120.744 57.457 127.667 1.00 30.74 B
ATOM 9208 C HIS B 445 121.703 55.161 131.350 1.00 24.43 B
ATOM 9209 O HIS B 445 121.108 56.150 131.780 1.00 24.46 B
ATOM 9210 N GLU B 446 121.138 53.957 131.297 1.00 22.85 B
ATOM 9211 CA GLU B 446 119.747 53.749 131.712 1.00 25.56 B
ATOM 9212 CB GLU B 446 119.189 52.429 131.157 1.00 25.16 B
ATOM 9213 CG GLU B 446 119.964 51.840 130.005 1.00 32.27 B
ATOM 9214 CD GLU B 446 121.132 51.003 130.466 1.00 29.53 B
ATOM 9215 OE1 GLU B 446 121.11 49.784 130.209 1.00 32.04 B
ATOM 9216 OE2 GLU B 446 122.062 51.561 131.083 1.00 28.66 B
ATOM 9217 C GLU B 446 119.502 53.748 133.214 1.00 24.46 B
ATOM 9218 O GLU B 446 118.357 53.637 133.649 1.00 23.39 B
ATOM 9219 N GLN B 447 120.559 53.861 134.006 1.00 23.95 B
ATOM 9220 CA GLN B 447 120.397 53.845 135.453 1.00 25.16 B
ATOM 9221 CB GLN B 447 121.442 52.915 136.072 1.0026.98 B
ATOM 9222 CG GLN B 447 121.148 51.443 135.837 1.00 28.62 B
ATOM 9223 CD GLN B 447 122.328 50.554 136.171 1.00 30.65 B
ATOM 9224 OE1 GLN B 447 123.012 50.761 137.174 1.00 29.23 B
ATOM 9225 NE2 GLN B 447 122.567 49.549 135.335 1.00 31.96 B
ATOM 9226 C GLN B 447 120.452 55.215 136.118 1.00 24.19 B
ATOM 9227 O GLN B 447 120.154 55.342 137.306 1.00 25.00 B
ATOM 9228 N LYS B 448 120.831 56.234 135.354 1.0024.97 B
ATOM 9229 CA LYS B 448 120.924 57.595 135.874 1.00 25.98 B
ATOM 9230 CB LYS B 448 121.794 58.465 134.965 1.00 27.69 B
ATOM 9231 CG LYS B 448 123.207 57.970 134.721 1.00 29.20 B
ATOM 9232 CD LYS B 448 123.836 58.808 133.619 1.00 32.86 B
ATOM 9233 CE LYS B 448 125.171 58.264 133.150 1.00 35.36 B
ATOM 9234 NZ LYS B 448 125.603 59.005 131.926 1.00 37.54 B
ATOM 9235 C LYS B 448 119.532 58.206 135.941 1.00 26.08 B
ATOM 9236 O LYS B 448 118.653 57.855 135.150 1.00 27.33 B
ATOM 9237 N ALA B 449 119.331 59.123 136.882 1.0024.31 B
ATOM 9238 CA ALA B 449 118.039 59.778 137.029 1.00 23.12 B
ATOM 9239 CB ALA B 449 117.829 60.206 138.488 1.00 20.47 B
ATOM 9240 C ALA B 449 118.006 60.987 136.094 1.00 22.29 B
ATOM 9241 O ALA B 449 117.744 62.116 136.515 1.00 22.20 B
ATOM 9242 N ALA B 450 118.276 60.733 134.816 1.0021.70 B
ATOM 9243 CA ALA B 450 118.300 61.786 133.808 1.00 21.13 B
ATOM 9244 CB ALA B 450 119.439 61.527 132.809 1.00 22.52 B
ATOM 9245 C ALA B 450 116.977 61.912 133.070 1.00 19.88 B
ATOM 9246 O ALA B 450 116.132 61.022 133.127 1.00 19.21 B
ATOM 9247 N LEU B 451 116.807 63.036 132.384 1.00 21.63 B
ATOM 9248 CA LEU B 451 115.598 63.310 131.610 1.00 23.16 B
ATOM 9249 CB LEU B 451 115.760 64.614 130.828 1.00 22.89 B
ATOM 9250 CG LEU B 451 114.613 65.623 130.756 1.0023.70 B
ATOM 9251 CD1 LEU B 451 114.954 66.632 129.670 1.00 21.60 B
ATOM 9252 CD2 LEU B 451 113.285 64.944 130.452 1.00 20.38 B
ATOM 9253 C LEU B 451 115.366 62.177 130.620 1.00 23.84 B
ATOM 9254 O LEU B 451 114.235 61.727 130.422 1.0023.98 B
ATOM 9255 N ALA B 452 116.453 61.721 130.004 1.0024.09 B
ATOM 9256 CA ALA B 452 116.389 60.646 129.018 1.00 26.03 B
ATOM 9257 CB ALA B 452 117.794 60.314 128.511 1.00 25.41 B
ATOM 9258 C ALA B 452 115.721 59.388 129.561 1.0026.20 B
ATOM 9259 O ALA B 452 115.344 58.509 128.797 1.0028.19 B
ATOM 9260 N ASN B 453 115.571 59.301 130.878 1.0026.86 B
ATOM 9261 CA ASN B 453 114.940 58.134 131.488 1.00 26.91 B
ATOM 9262 CB ASN B 453 115.934 57.427 132.421 1.00 27.53 B
ATOM 9263 CG ASN B 453 117.177 56.930 131.683 1.00 29.79 B
ATOM 9264 OD1 ASN B 453 117.072 56.292 130.634 1.00 29.54 B
ATOM 9265 ND2 ASN B 453 118.358 57.216 132.233 1.00 27.58 B
ATOM 9266 C ASN B 453 113.664 58.513 132.244 1.00 26.00 B
ATOM 9267 O ASN B 453 113.174 57.759 133.088 1.0024.98 B
ATOM 9268 N ALA B 454 113.119 59.682 131.913 1.00 25.29 B
ATOM 9269 CA ALA B 454 111.894 60.183 132.544 1.00 25.01 B
ATOM 9270 CB ALA B 454 111.360 61.393 131.763 1.00 24.41 B
ATOM 9271 C ALA B 454 110.782 59.146 132.695 1.0023.43 B
ATOM 9272 O ALA B 454 110.088 59.124 133.708 1.00 23.78 B
ATOM 9273 N PRO B 455 110.590 58.277 131.689 1.00 23.20 B
ATOM 9274 CD PRO B 455 111.198 58.230 130.347 1.00 22.33 B
ATOM 9275 CA PRO B 455 109.526 57.276 131.808 1.00 23.42 B
ATOM 9276 CB PRO B 455 109.635 56.496 130.498 1.00 23.66 B
ATOM 9277 CG PRO B 455 110.120 57.537 129.536 1.0022.93 B
ATOM 9278 C PRO B 455 109.631 56.374 133.031 1.00 22.98 B
ATOM 9279 O PRO B 455 108.617 55.899 133.539 1.00 20.64 B
ATOM 9280 N ARG B 456 110.850 56.137 133.507 1.00 24.88 B
ATOM 9281 CA ARG B 456 111.037 55.277 134.676 1.00 26.44 B
ATOM 9282 CB ARG B 456 112.412 54.601 134.639 1.00 30.07 B
ATOM 9283 CG ARG B 456 112.496 53.399 133.705 1.00 35.31 B
ATOM 9284 CD ARG B 456 113.836 52.698 133.849 1.00 38.77 B
ATOM 9285 NE ARG B 456 114.043 52.179 135.200 1.00 43.24 B
ATOM 9286 CZ ARG B 456 115.194 51.676 135.641 1.00 45.47 B
ATOM 9287 NH1 ARG B 456 115.294 51.225 136.886 1.00 47.31 B
ATOM 9288 NH2 ARG B 456 116.252 51.628 134.842 1.00 45.85 B
ATOM 9289 C ARG B 456 110.883 56.016 135.998 1.00 26.53 B
ATOM 9290 O ARG B 456 111.026 55.421 137.064 1.00 26.90 B
ATOM 9291 N MET B 457 110.583 57.310 135.935 1.0025.50 B
ATOM 9292 CA MET B 457 110.433 58.088 137.152 1.00 23.57 B
ATOM 9293 CB MET B 457 111.633 59.018 137.322 1.00 23.77 B
ATOM 9294 CG MET B 457 112.943 58.272 137.501 1.00 23.28 B
ATOM 9295 SD MET B 457 114.352 59.374 137.542 1.00 22.89 B
ATOM 9296 CE MET B 457 114.845 59.354 135.810 1.00 21.18 B
ATOM 9297 C MET B 457 109.149 58.891 137.239 1.00 23.32 B
ATOM 9298 O MET B 457 109.172 60.121 137.300 1.00 23.17 B
ATOM 9299 N PRO B 458 107.999 58.210 137.225 1.00 22.43 B
ATOM 9300 CD PRO B 458 107.702 56.769 137.215 1.00 24.47 B
ATOM 9301 CA PRO B 458 106.775 59.006 137.325 1.00 22.23 B
ATOM 9302 CB PRO B 458 105.669 57.953 137.243 1.0022.37 B
ATOM 9303 CG PRO B 458 106.320 56.730 137.842 1.00 23.56 B
ATOM 9304 C PRO B 458 106.831 59.711 138.678 1.00 21.72 B
ATOM 9305 O PRO B 458 107.455 59.203 139.611 1.00 22.14 B
ATOM 9306 N GLY B 459 106.210 60.881 138.778 1.00 20.57 B
ATOM 9307 CA GLY B 459 106.232 61.614 140.030 1.00 18.37 B
ATOM 9308 C GLY B 459 107.453 62.512 140.164 1.00 18.24 B
ATOM 9309 O GLY B 459 107.571 63.255 141.137 1.0019.64 B
ATOM 9310 N ILE B 460 108.360 62.448 139.191 1.00 16.76 B
ATOM 9311 CA ILE B 460 109.580 63.257 139.211 1.00 15.07 B
ATOM 9312 CB ILE B 460 110.854 62.378 139.127 1.00 16.09 B
ATOM 9313 CG2 ILE B 460 112.094 63.258 139.227 1.00 15.32 B
ATOM 9314 CG1 ILE B 460 110.853 61.316 140.238 1.00 15.14 B
ATOM 9315 CD1 ILE B 460 110.846 61.880 141.661 1.00 12.86 B
ATOM 9316 C ILE B 460 109.615 64.213 138.025 1.00 15.85 B
ATOM 9317 O ILE B 460 109.439 63.793 136.881 1.00 16.11 B
ATOM 9318 N GLY B 461 109.861 65.492 138.303 1.00 13.95 B
ATOM 9319 CA GLY B 461 109.918 66.491 137.250 1.00 13.69 B
ATOM 9320 C GLY B 461 111.328 66.796 136.767 1.00 14.19 B
ATOM 9321 O GLY B 461 112.316 66.483 137.435 1.00 13.99 B
ATOM 9322 N PHE B 462 111.424 67.412 135.595 1.00 14.57 B
ATOM 9323 CA PHE B 462 112.716 67.750 135.015 1.00 15.31 B
ATOM 9324 CB PHE B 462 113.126 66.707 133.965 1.00 13.44 B
ATOM 9325 CG PHE B 462 113.164 65.303 134.490 1.00 16.66 B
ATOM 9326 CD1 PHE B 462 111.993 64.561 134.614 1.00 16.50 B
ATOM 9327 CD2 PHE B 462 114.361 64.741 134.915 1.00 14.98 B
ATOM 9328 CE1 PHE B 462 112.013 63.281 135.159 1.00 17.02 B
ATOM 9329 CE2 PHE B 462 114.389 63.460 135.462 1.00 18.14 B
ATOM 9330 CZ PHE B 462 113.207 62.729 135.584 1.00 16.50 B
ATOM 9331 C PHE B 462 112.695 69.109 134.346 1.00 5.63 B
ATOM 9332 O PHE B 462 111.719 69.463 133.687 1.00 16.14 B
ATOM 9333 N PHE B 463 113.774 69.868 134.521 1.00 15.54 B
ATOM 9334 CA PHE B 463 113.886 71.163 133.869 1.00 15.89 B
ATOM 9335 CB PHE B 463 115.174 71.871 134.294 1.0013.38 B
ATOM 9336 CG PHE B 463 115.056 72.571 135.614 1.00 15.14 B
ATOM 9337 CD1 PHE B 463 114.358 73.775 135.712 1.00 14.51 B
ATOM 9338 CD2 PHE B 463 115.572 71.997 136.773 1.00 13.22 B
ATOM 9339 CE1 PHE B 463 114.168 74.394 136.953 1.00 15.58 B
ATOM 9340 CE2 PHE B 463 115.389 72.605 138.018 1.00 14.82 B
ATOM 9341 CZ PHE B 463 114.682 73.806 138.108 1.00 14.49 B
ATOM 9342 C PHEB463 113.903 70.851 132.377 1.00 17.75 B
ATOM 9343 O PHE B 463 114.716 70.050 131.900 1.00 16.73 B
ATOM 9344 N ASN B 464 112.990 71.477 131.646 1.00 18.51 B
ATOM 9345 CA ASN B 464 112.862 71.241 130.220 1.00 17.62 B
ATOM 9346 CB ASN B 464 111.418 71.515 129.808 1.00 17.29 B
ATOM 9347 CG ASN B 464 111.103 71.008 128.426 1.00 17.72 B
ATOM 9348 OD1 ASN B 464 111.943 71.072 127.531 1.00 16.06 B
ATOM 9349 ND2 ASN B 464 109.881 70.508 128.237 1.00 15.25 B
ATOM 9350 C ASN B 464 113.825 72.105 129.405 1.00 18.16 B
ATOM 9351 O ASN B 464 113.496 73.233 129.048 1.00 17.71 B
ATOM 9352 N ASP B 465 115.009 71.572 129.104 1.00 18.04 B
ATOM 9353 CA ASP B 465 116.000 72.326 128.336 1.00 20.59 B
ATOM 9354 CB ASP B 465 117.370 71.630 128.367 1.00 23.32 B
ATOM 9355 CG ASP B 465 117.323 70.214 127.809 1.00 29.51 B
ATOM 9356 OD1 ASP B 465 118.361 69.743 127.301 1.00 31.89 B
ATOM 9357 OD2 ASP B 465 116.256 69.567 127.886 1.00 33.47 B
ATOM 9358 C ASP B 465 115.568 72.540 126.889 1.00 19.87 B
ATOM 9359 O ASP B 465 115.883 73.573 126.288 1.0020.08 B
ATOM 9360 N MET B 466 114.858 71.565 126.329 1.00 18.69 B
ATOM 9361 CA MET B 466 114.368 71.671 124.954 1.0019.72 B
ATOM 9362 CB MET B 466 113.518 70.444 124.599 1.00 19.21 B
ATOM 9363 CG MET B 466 112.899 70.459 123.192 1.00 23.02 B
ATOM 9364 SD MET B 466 111.478 71.584 122.959 1.00 21.67 B
ATOM 9365 CE MET B 466 110.141 70.570 123.589 1.00 21.16 B
ATOM 9366 C MET B 466 113.521 72.936 124.820 1.00 19.15 B
ATOM 9367 O MET B 466 113.814 73.817 124.014 1.00 19.64 B
ATOM 9368 N PHE B 467 112.473 73.016 125.632 1.00 17.37 B
ATOM 9369 CA PHE B 467 111.562 74.152 125.612 1.00 16.42 B
ATOM 9370 CB PHE B 467 110.478 73.940 126.669 1.00 12.71 B
ATOM 9371 CG PHE B 467 109.247 74.772 126.468 1.00 12.29 B
ATOM 9372 CD1 PHE B 467 108.246 74.786 127.440 1.00 11.34 B
ATOM 9373 CD2 PHE B 467 109.071 75.532 125.310 1.00 11.42 B
ATOM 9374 CE1 PHE B 467 107.085 75.540 127.266 1.00 11.11 B
ATOM 9375 CE2 PHE B 467 107.912 76.291 125.123 1.00 10.13 B
ATOM 9376 CZ PHE B 467 106.916 76.295 126.103 1.00 10.98 B
ATOM 9377 C PHE B 467 112.326 75.452 125.870 1.00 17.42 B
ATOM 9378 O PHE B 467 112.124 76.448 125.177 1.00 18.37 B
ATOM 9379 N ARG B 468 113.204 75.428 126.870 1.00 17.36 B
ATOM 9380 CA ARG B 468 114.018 76.585 127.231 1.00 17.22 B
ATOM 9381 CB ARG B 468 115.063 76.165 128.276 1.0017.84 B
ATOM 9382 CG ARG B 468 116.025 77.267 128.746 1.00 19.77 B
ATOM 9383 CD ARG B 468 117.255 76.652 129.433 1.00 19.17 B
ATOM 9384 NE ARG B 468 118.129 75.977 128.470 1.00 21.03 B
ATOM 9385 CZ ARG B 468 119.045 75.061 128.783 1.00 21.97 B
ATOM 9386 NH1 ARG B 468 119.213 74.694 130.046 1.00 22.96 B
ATOM 9387 NH2 ARG B 468 119.800 74.514 127.833 1.00 15.88 B
ATOM 9388 C ARG B 468 114.730 77.175 126.004 1.00 19.09 B
ATOM 9389 O ARG B 468 114.522 78.338 125.644 1.00 18.24 B
ATOM 9390 N ASPB469 115.564 76.361 125.361 1.00 19.63 B
ATOM 9391 CA ASP B 469 116.324 76.802 124.196 1.00 21.85 B
ATOM 9392 CB ASP B 469 117.396 75.766 123.850 1.00 24.00 B
ATOM 9393 CG ASP B 469 118.535 75.748 124.863 1.00 27.25 B
ATOM 9394 OD1 ASP B 469 119.392 74.845 124.782 1.0033.14 B
ATOM 9395 OD2 ASP B 469 118.577 76.640 125.737 1.00 27.00 B
ATOM 9396 C ASP B 469 115.473 77.113 122.968 1.00 21.88 B
ATOM 9397 O ASP B 469 115.848 77.958 122.156 1.00 20.61 B
ATOM 9398 N ALA B 470 114.329 76.446 122.836 1.00 20.96 B
ATOM 9399 CA ALA B 470 113.441 76.691 121.706 1.00 20.93 B
ATOM 9400 CB ALA B 470 112.279 75.713 121.730 1.00 22.76 B
ATOM 9401 C ALAB470 112.914 78.124 121.758 1.0020.72 B
ATOM 9402 O ALA B 470 112.881 78.823 120.743 1.0018.47 B
ATOM 9403 N VAL B 471 112.508 78.549 122.952 1.00 18.58 B
ATOM 9404 CA VAL B 471 111.972 79.889 123.167 1.00 18.46 B
ATOM 9405 CB VAL B 471 111.202 79.964 124.512 1.00 18.15 B
ATOM 9406 CG1 VAL B 471 110.790 81.397 124.810 1.00 15.17 B
ATOM 9407 CG2 VAL B 471 109.984 79.064 124.465 1.00 16.80 B
ATOM 9408 C VAL B 471 113.051 80.977 123.180 1.00 20.61 B
ATOM 9409 O VAL B 471 112.941 81.983 122.477 1.00 21.06 B
ATOM 9410 N LYS B 472 114.094 80.763 123.975 1.00 21.10 B
ATOM 9411 CA LYS B 472 115.180 81.728 124.117 1.00 21.44 B
ATOM 9412 CB LYS B 472 115.778 81.612 125.520 1.00 21.26 B
ATOM 9413 CG LYS B 472 117.007 82.470 125.755 1.00 22.35 B
ATOM 9414 CD LYS B 472 117.588 82.218 127.136 1.0023.96 B
ATOM 9415 CE LYS B 472 118.027 80.771 127.274 1.0026.16 B
ATOM 9416 NZ LYS B 472 118.641 80.511 128.597 1.00 29.73 B
ATOM 9417 C LYS B 472 116.310 81.616 123.097 1.0021.67 B
ATOM 9418 O LYS B 472 116.806 82.625 122.603 1.00 22.15 B
ATOM 9419 N GLY B 473 116.722 80.390 122.804 1.00 21.78 B
ATOM 9420 CA GLY B 473 117.815 80.168 121.877 1.00 24.59 B
ATOM 9421 C GLY B 473 118.866 79.366 122.618 1.00 27.14 B
ATOM 9422 O GLY B 473 118.934 79.430 123.845 1.00 27.34 B
ATOM 9423 N ASN B 474 119.674 78.600 121.894 1.00 29.07 B
ATOM 9424 CA ASN B 474 120.714 77.787 122.519 1.00 32.17 B
ATOM 9425 CB ASN B 474 121.748 77.368 121.466 1.00 33.89 B
ATOM 9426 CG ASN B 474 122.753 76.367 121.999 1.00 34.40 B
ATOM 9427 OD1 ASN B 474 123.530 76.668 122.905 1.00 34.27 B
ATOM 9428 ND2 ASN B 474 122.738 75.162 121.441 1.00 34.79 B
ATOM 9429 C ASN B 474 121.385 78.599 123.626 1.00 33.77 B
ATOM 9430 O ASN B 474 121.813 79.726 123.394 1.00 33.11 B
ATOM 9431 N THR B 475 121.469 78.036 124.829 1.00 36.11 B
ATOM 9432 CA THR B 475 122.076 78.754 125.950 1.00 39.04 B
ATOM 9433 CB THR B 475 121.652 78.154 127.323 1.00 38.94 B
ATOM 9434 OG1 THR B 475 122.025 76.773 127.382 1.00 41.75 B
ATOM 9435 CG2 THR B 475 120.159 78.272 127.528 1.00 35.29 B
ATOM 9436 C THR B 475 123.603 78.805 125.909 1.00 41.13 B
ATOM 9437 O THR B 475 124.213 79.689 126.512 1.0042.59 B
ATOM 9438 N PHE B 476 124.225 77.874 125.193 1.00 43.50 B
ATOM 9439 CA PHE B 476 125.682 77.842 125.124 1.00 46.59 B
ATOM 9440 CB PHE B 476 126.159 76.390 125.210 1.0048.53 B
ATOM 9441 CG PHE B 476 125.647 75.673 126.429 1.0050.64 B
ATOM 9442 CD1 PHE B 476 124.525 74.854 126.354 1.00 51.57 B
ATOM 9443 CD2 PHE B 476 126.250 75.869 127.671 1.00 51.61 B
ATOM 9444 CE1 PHE B 476 124.008 74.243 127.499 1.00 52.72 B
ATOM 9445 CE2 PHE B 476 125.740 75.263 128.821 1.00 52.85 B
ATOM 9446 CZ PHE B 476 124.618 74.450 128.734 1.00 52.41 B
ATOM 9447 C PHE B 476 126.304 78.561 123.922 1.0046.52 B
ATOM 9448 O PHE B 476 127.517 78.520 123.722 1.00 47.52 B
ATOM 9449 N HIS B 477 125.465 79.221 123.133 1.00 46.19 B
ATOM 9450 CA HIS B 477 125.917 79.990 121.978 1.00 45.86 B
ATOM 9451 CB HIS B 477 125.454 79.346 120.674 1.0046.39 B
ATOM 9452 CG HIS B 477 126.248 78.141 120.284 1.00 48.21 B
ATOM 9453 CD2 HIS B 477 125.908 76.832 120.212 1.00 48.82 B
ATOM 9454 ND1 HIS B 477 127.573 78.214 119.909 1.00 48.84 B
ATOM 9455 CE1 HIS B 477 128.015 77.002 119.623 1.00 49.46 B
ATOM 9456 NE2 HIS B 477 127.024 76.145 119.799 1.00 49.94 B
ATOM 9457 C HIS B 477 125.303 81.370 122.135 1.00 45.71 B
ATOM 9458 O HIS B 477 124.173 81.616 121.717 1.00 45.86 B
ATOM 9459 N LEU B 478 126.066 82.255 122.761 1.00 45.64 B
ATOM 9460 CA LEU B 478 125.656 83.623 123.046 1.00 46.18 B
ATOM 9461 CB LEU B 478 126.903 84.485 123.257 1.00 48.30 B
ATOM 9462 CG LEU B 478 126.736 85.764 124.078 1.00 49.48 B
ATOM 9463 CD1 LEU B 478 126.293 85.400 125.489 1.00 50.64 B
ATOM 9464 CD2 LEU B 478 128.052 86.530 124.111 1.00 50.32 B
ATOM 9465 C LEU B 478 124.746 84.288 122.011 1.00 45.60 B
ATOM 9466 O LEU B 478 123.735 84.897 122.368 1.0045.44 B
ATOM 9467 N LYS B 479 125.106 84.173 120.735 1.00 44.35 B
ATOM 9468 CA LYS B 479 124.335 84.791 119.658 1.00 42.67 B
ATOM 9469 CB LYS B 479 125.272 85.194 118.519 1.0044.79 B
ATOM 9470 CG LYS B 479 126.227 86.322 118.863 1.0047.92 B
ATOM 9471 CD LYS B 479 127.268 86.501 117.768 1.0050.80 B
ATOM 9472 CE LYS B 479 128.128 87.719 118.026 1.00 52.22 B
ATOM 9473 NZ LYS B 479 127.280 88.945 118.067 1.0055.70 B
ATOM 9474 C LYS B 479 123.196 83.948 119.089 1.00 40.35 B
ATOM 9475 O LYS B 479 122.469 84.406 118.207 1.00 40.13 B
ATOM 9476 N ALA B 480 123.037 82.722 119.572 1.0036.87 B
ATOM 9477 CA ALA B 480 121.963 81.868 119.075 1.00 34.34 B
ATOM 9478 CB ALA B 480 122.143 80.444 119.580 1.00 33.34 B
ATOM 9479 C ALA B 480 120.628 82.431 119.546 1.00 31.59 B
ATOM 9480 O ALA B 480 120.471 82.769 120.715 1.00 33.30 B
ATOM 9481 N THR B 481 119.671 82.538 118.634 1.00 28.12 B
ATOM 9482 CA THR B 481 118.359 83.064 118.981 1.00 26.91 B
ATOM 9483 CB THR B 481 117.943 84.209 118.040 1.0027.57 B
ATOM 9484 OG1 THR B 481 117.891 83.719 116.695 1.00 30.21 B
ATOM 9485 CG2 THR B 481 118.930 85.359 118.123 1.00 25.76 B
ATOM 9486 C THR B 481 117.280 81.989 118.906 1.00 25.43 B
ATOM 9487 O THR B 481 117.406 81.010 118.170 1.00 23.31 B
ATOM 9488 N GLY B 482 116.217 82.183 119.675 1.00 23.13 B
ATOM 9489 CA GLY B 482 115.120 81.234 119.672 1.00 23.18 B
ATOM 9490 C GLY B 482 113.877 81.923 119.138 1.0021.52 B
ATOM 9491 O GLY B 482 113.938 83.087 118.743 1.00 20.31 B
ATOM 9492 N PHE B 483 112.750 81.219 119.138 1.00 19.63 B
ATOM 9493 CA PHE B 483 111.501 81.779 118.640 1.00 18.71 B
ATOM 9494 CB PHE B 483 110.324 80.886 119.023 1.00 17.69 B
ATOM 9495 CG PHE B 483 109.006 81.395 118.524 1.00 17.81 B
ATOM 9496 CD1 PHE B 483 108.659 81.264 117.179 1.00 17.68 B
ATOM 9497 CD2 PHE B 483 108.128 82.047 119.381 1.00 16.79 B
ATOM 9498 CE1 PHE B 483 107.452 81.780 116.697 1.00 18.07 B
ATOM 9499 CE2 PHE B 483 106.920 82.564 118.907 1.00 19.76 B
ATOM 9500 CZ PHE B 483 106.583 82.429 117.560 1.00 16.43 B
ATOM 9501 C PHE B 483 111.201 83.196 119.128 1.00 19.93 B
ATOM 9502 O PHE B 483 110.949 84.099 118.326 1.00 19.87 B
ATOM 9503 N ALA B 484 111.212 83.382 120.445 1.00 19.56 B
ATOM 9504 CA ALA B 484 110.916 84.680 121.038 1.00 20.21 B
ATOM 9505 CB ALA B 484 110.964 84.581 122.563 1.00 17.38 B
ATOM 9506 C ALA B 484 111.854 85.779 120.551 1.00 21.96 B
ATOM 9507 O ALA B 484 111.472 86.952 120.522 1.00 22.13 B
ATOM 9508 N LEU B 485 113.075 85.403 120.174 1.0021.83 B
ATOM 9509 CA LEU B 485 114.056 86.369 119.690 1.00 22.72 B
ATOM 9510 CB LEU B 485 115.470 86.018 120.190 1.00 20.56 B
ATOM 9511 CG LEU B 485 115.711 86.032 121.709 1.0021.61 B
ATOM 9512 CD1 LEU B 485 117.193 85.864 121.992 1.00 20.20 B
ATOM 9513 CD2 LEU B 485 115.215 87.333 122.317 1.00 19.66 B
ATOM 9514 C LEU B 485 114.055 86.477 118.162 1.00 24.25 B
ATOM 9515 O LEU B 485 114.949 87.094 117.583 1.0024.53 B
ATOM 9516 N GLY B 486 113.064 85.863 117.516 1.00 23.66 B
ATOM 9517 CA GLY B 486 112.959 85.954 116.069 1.00 25.22 B
ATOM 9518 C GLY B 486 113.418 84.797 115.202 1.00 26.28 B
ATOM 9519 O GLYB486 113.395 84.898 113.973 1.00 27.05 B
ATOM 9520 N ASN B 487 113.835 83.697 115.811 1.00 27.37 B
ATOM 9521 CA ASN B 487 114.286 82.551 115.033 1.00 29.89 B
ATOM 9522 CB ASN B 487 115.311 81.748 115.834 1.0028.90 B
ATOM 9523 CG ASN B 487 116.132 80.824 114.965 1.00 30.37 B
ATOM 9524 OD1 ASN B 487 115.611 80.178 114.054 1.00 31.84 B
ATOM 9525 ND2 ASN B 487 117.425 80.747 115.247 1.00 30.67 B
ATOM 9526 C ASN B 487 113.087 81.661 114.679 1.00 31.53 B
ATOM 9527 O ASN B 487 112.445 81.091 115.562 1.00 32.41 B
ATOM 9528 N GLY B 488 112.789 81.545 113.388 1.00 32.80 B
ATOM 9529 CA GLY B 488 111.663 80.731 112.958 1.00 33.32 B
ATOM 9530 C GLY B 488 111.942 79.240 112.972 1.00 34.03 B
ATOM 9531 O GLY B 488 111.026 78.419 112.974 1.00 34.95 B
ATOM 9532 N GLU B 489 113.217 78.884 112.994 1.00 33.86 B
ATOM 9533 CA GLU B 489 113.622 77.486 112.998 1.00 35.08 B
ATOM 9534 CB GLU B 489 115.143 77.395 112.953 1.00 38.50 B
ATOM 9535 CG GLU B 489 115.770 77.958 111.706 1.0044.33 B
ATOM 9536 CD GLU B 489 115.715 76.981 110.556 1.00 49.71 B
ATOM 9537 OE1 GLU B 489 114.620 76.800 109.976 1.00 51.35 B
ATOM 9538 OE2 GLU B 489 116.773 76.385 110.246 1.00 51.91 B
ATOM 9539 C GLU B 489 113.131 76.721 114.219 1.00 32.90 B
ATOM 9540 O GLU B 489 113.099 75.494 114.212 1.00 32.59 B
ATOM 9541 N SERB 490 112.753 77.437 115.268 1.00 30.94 B
ATOM 9542 CA SER B 490 112.315 76.775 116.489 1.0029.90 B
ATOM 9543 CB SER B 490 113.121 77.316 117.672 1.00 30.41 B
ATOM 9544 OG SER B 490 113.384 78.696 117.510 1.00 31.14 B
ATOM 9545 C SER B 490 110.829 76.850 116.796 1.00 28.58 B
ATOM 9546 O SER B 490 110.391 76.415 117.861 1.00 28.34 B
ATOM 9547 N ALA B 491 110.048 77.375 115.860 1.00 26.21 B
ATOM 9548 CA ALA B 491 108.611 77.497 116.067 1.00 25.07 B
ATOM 9549 CB ALA B 491 107.945 78.047 114.812 1.00 26.43 B
ATOM 9550 C ALA B 491 107.952 76.183 116.472 1.00 23.62 B
ATOM 9551 O ALA B 491 107.171 76.148 117.425 1.0022.57 B
ATOM 9552 N GLN B 492 108.262 75.101 115.762 1.00 21.13 B
ATOM 9553 CA GLN B 492 107.652 73.820 116.090 1.00 20.86 B
ATOM 9554 CB GLN B 492 108.025 72.745 115.066 1.00 22.93 B
ATOM 9555 CG GLN B 492 107.364 71.401 115.375 1.0027.58 B
ATOM 9556 CD GLN B 492 107.593 70.345 114.304 1.00 30.36 B
ATOM 9557 OE1 GLN B 492 108.732 70.038 113.941 1.00 31.02 B
ATOM 9558 NE2 GLN B 492 106.503 69.774 113.804 1.00 29.85 B
ATOM 9559 C GLN B 492 108.014 73.338 117.492 1.00 20.32 B
ATOM 9560 O GLN B 492 107.136 72.955 118.269 1.00 19.00 B
ATOM 9561 N ALA B 493 109.302 73.350 117.816 1.0019.39 B
ATOM 9562 CA ALA B 493 109.745 72.916 119.136 1.00 18.48 B
ATOM 9563 CB ALA B 493 111.251 73.154 119.294 1.00 15.62 B
ATOM 9564 C ALA B 493 108.970 73.682 120.211 1.00 18.54 B
ATOM 9565 O ALA B 493 108.587 73.119 121.233 1.0019.40 B
ATOM 9566 N VAL B 494 108.735 74.967 119.972 1.00 19.18 B
ATOM 9567 CA VAL B 494 108.000 75.789 120.925 1.00 18.01 B
ATOM 9568 CB VAL B 494 108.016 77.274 120.501 1.00 16.91 B
ATOM 9569 CG1 VALA 494 107.009 78.072 121.316 1.00 12.70 B
ATOM 9570 CG2 VAL B 494 109.421 77.843 120.691 1.00 15.26 B
ATOM 9571 C VAL B 494 106.557 75.302 121.068 1.00 20.47 B
ATOM 9572 O VAL B 494 106.035 75.204 122.189 1.00 19.52 B
ATOM 9573 N MET B 495 105.915 74.986 119.943 1.00 18.34 B
ATOM 9574 CA MET B 495 104.534 74.503 119.985 1.00 19.99 B
ATOM 9575 CB MET B 495 104.008 74.228 118.567 1.00 21.58 B
ATOM 9576 CG MET B 495 102.536 73.805 118.499 1.00 21.95 B
ATOM 9577 SD MET B 495 101.389 75.018 119.234 1.00 22.12 B
ATOM 9578 CE MET B 495 100.569 74.044 120.495 1.00 18.80 B
ATOM 9579 C MET B 495 104.466 73.230 120.822 1.00 18.84 B
ATOM 9580 O MET B 495 103.523 73.034 121.590 1.00 20.68 B
ATOM 9581 N HIS B 496 105.476 72.374 120.671 1.00 17.04 B
ATOM 9582 CA HIS B 496 105.562 71.117 121.414 1.00 17.74 B
ATOM 9583 CB HIS B 496 106.873 70.401 121.038 1.00 16.62 B
ATOM 9584 CG HIS B 496 107.013 69.015 121.597 1.00 18.01 B
ATOM 9585 CD2 HIS B 496 108.026 68.118 121.499 1.00 17.91 B
ATOM 9586 ND1 HIS B 496 106.038 68.407 122.359 1.00 20.01 B
ATOM 9587 CE1 HIS B 496 106.443 67.197 122.707 1.00 18.13 B
ATOM 9588 NE2 HIS B 496 107.647 66.998 122.198 1.00 18.80 B
ATOM 9589 C HIS B 496 105.523 71.453 122.919 1.00 18.25 B
ATOM 9590 O HIS B 496 104.707 70.906 123.677 1.00 14.46 B
ATOM 9591 N GLY B 497 106.397 72.372 123.330 1.00 17.67 B
ATOM 9592 CA GLY B 497 106.454 72.788 124.724 1.00 17.09 B
ATOM 9593 C GLY B 497 105.129 73.350 125.205 1.00 16.67 B
ATOM 9594 O GLY B 497 104.695 73.073 126.327 1.00 16.54 B
ATOM 9595 N ILE B 498 104.477 74.139 124.355 1.00 16.94 B
ATOM 9596 CA ILE B 498 103.186 74.723 124.707 1.00 16.98 B
ATOM 9597 CB ILE B 498 102.678 75.659 123.581 1.00 16.62 B
ATOM 9598 CG2 ILE B 498 101.213 76.034 123.818 1.00 14.36 B
ATOM 9599 CG1 ILE B 498 103.564 76.908 123.527 1.00 17.75 B
ATOM 9600 CD1 ILE B 498 103.236 77.863 122.391 1.00 19.91 B
ATOM 9601 C ILE B 498 102.183 73.594 124.940 1.00 16.83 B
ATOM 9602 O ILE B 498 101.243 73.729 125.719 1.00 16.77 B
ATOM 9603 N ALA B 499 102.408 72.469 124.273 1.00 17.86 B
ATOM 9604 CA ALA B 499 101.530 71.312 124.409 1.00 18.79 B
ATOM 9605 CB ALA B 499 101.539 70.490 123.113 1.00 16.78 B
ATOM 9606 C ALA B 499 101.906 70.426 125.595 1.00 17.68 B
ATOM 9607 O ALA B 499 101.290 69.385 125.813 1.00 18.63 B
ATOM 9608 N GLY B 500 102.915 70.827 126.361 1.00 17.57 B
ATOM 9609 CA GLY B 500 103.303 70.026 127.510 1.00 18.23 B
ATOM 9610 C GLY B 500 104.483 69.091 127.288 1.00 19.69 B
ATOM 9611 O GLY B 500 104.798 68.273 128.156 1.00 17.97 B
ATOM 9612 N SERB 501 105.130 69.199 126.129 1.00 18.75 B
ATOM 9613 CA SER B 501 106.296 68.373 125.812 1.00 20.07 B
ATOM 9614 CB SER B 501 107.487 68.820 126.663 1.00 18.23 B
ATOM 9615 OG SER B 501 107.712 70.209 126.523 1.00 19.88 B
ATOM 9616 C SER B 501 106.075 66.867 126.003 1.00 21.74 B
ATOM 9617 O SER B 501 107.030 66.110 126.204 1.00 17.81 B
ATOM 9618 N SER B 502 104.818 66.438 125.939 1.00 24.71 B
ATOM 9619 CA SER B 502 104.483 65.025 126.097 1.00 28.94 B
ATOM 9620 CB SER B 502 103.274 64.869 127.022 1.00 28.99 B
ATOM 9621 OG SER B 502 103.549 65.394 128.312 1.00 31.05 B
ATOM 9622 C SER B 502 104.172 64.414 124.735 1.00 31.95 B
ATOM 9623 O SER B 502 103.312 63.544 124.615 1.0033.23 B
ATOM 9624 N GLY B 503 104.884 64.879 123.713 1.00 32.99 B
ATOM 9625 CA GLY B 503 104.669 64.385 122.369 1.00 35.25 B
ATOM 9626 C GLY B 503 103.884 65.379 121.528 1.00 37.31 B
ATOM 9627 O GLY B 503 102.867 65.925 121.963 1.00 37.29 B
ATOM 9628 N TRP B 504 104.371 65.634 120.322 1.0038.34 B
ATOM 9629 CA TRP B 504 103.695 66.549 119.417 1.00 40.77 B
ATOM 9630 CB TRP B 504 104.362 67.928 119.413 1.00 39.15 B
ATOM 9631 CG TRP B 504 103.661 68.889 118.503 1.0035.61 B
ATOM 9632 CD2 TRP B 504 102.319 69.359 118.637 1.00 33.23 B
ATOM 9633 CE2 TRP B 504 102.059 70.206 117.536 1.00 34.44 B
ATOM 9634 CE3 TRP B 504 101.308 69.146 119.581 1.00 32.80 B
ATOM 9635 CD1 TRP B 504 104.154 69.454 117.359 1.00 35.38 B
ATOM 9636 NE1 TRP B 504 103.195 70.248 116.771 1.00 33.79 B
ATOM 9637 CZ2 TRP B 504 100.827 70.841 117.355 1.0034.83 B
ATOM 9638 CZ3 TRP B 504 100.083 69.776 119.403 1.00 33.86 B
ATOM 9639 CH2 TRP B 504 99.854 70.614 118.297 1.00 34.78 B
ATOM 9640 C TRP B 504 103.751 65.968 118.021 1.00 42.09 B
ATOM 9641 O TRP B 504 104.817 65.916 117.406 1.00 41.59 B
ATOM 9642 N LYS B 505 102.598 65.534 117.522 1.00 44.76 B
ATOM 9643 CA LYS B 505 102.529 64.946 116.196 1.0046.88 B
ATOM 9644 CB LYS B 505 102.697 66.028 115.124 1.00 48.60 B
ATOM 9645 CG LYS B 505 101.683 67.159 115.207 1.00 51.61 B
ATOM 9646 CD LYS B 505 101.850 68.133 114.044 1.00 53.57 B
ATOM 9647 CE LYS B 505 100.867 69.294 114.143 1.00 54.26 B
ATOM 9648 NZ LYS B 505 99.450 68.834 114.188 1.00 54.23 B
ATOM 9649 C LYS B 505 103.639 63.908 116.054 1.00 47.91 B
ATOM 9650 O LYS B 505 103.579 62.834 116.660 1.00 48.44 B
ATOM 9651 N ALA B 506 104.659 64.254 115.270 1.00 47.71 B
ATOM 9652 CA ALA B 506 105.786 63.366 115.015 1.00 47.56 B
ATOM 9653 CB ALA B 506 106.250 63.524 113.572 1.00 48.30 B
ATOM 9654 C ALA B 506 106.962 63.582 115.953 1.00 47.95 B
ATOM 9655 O ALA B 506 107.906 62.790 115.955 1.00 48.79 B
ATOM 9656 N LEU B 507 106.926 64.656 116.736 1.00 47.12 B
ATOM 9657 CA LEU B 507 108.014 64.925 117.668 1.00 44.79 B
ATOM 9658 CB LEU B 507 107.974 66.377 118.150 1.00 45.20 B
ATOM 9659 CG LEU B 507 108.551 67.415 117.183 1.00 45.29 B
ATOM 9660 CD1 LEU B 507 108.400 68.814 117.759 1.00 43.19 B
ATOM 9661 CD2 LEU B 507 110.018 67.105 116.932 1.00 44.60 B
ATOM 9662 C LEU B 507 107.921 63.981 118.854 1.00 43.59 B
ATOM 9663 O LEU B 507 106.837 63.741 119.386 1.00 44.29 B
ATOM 9664 N ALA B 508 109.064 63.434 119.254 1.00 41.71 B
ATOM 9665 CA ALA B 508 109.112 62.509 120.379 1.00 40.24 B
ATOM 9666 CB ALA B 508 110.446 61.773 120.394 1.00 41.00 B
ATOM 9667 C ALA B 508 108.916 63.256 121.694 1.00 37.05 B
ATOM 9668 O ALA B 508 109.390 64.381 121.854 1.00 36.02 B
ATOM 9669 N PRO B 509 108.207 62.634 122.650 1.00 34.93 B
ATOM 9670 CD PRO B 509 107.512 61.341 122.519 1.00 34.92 B
ATOM 9671 CA PRO B 509 107.943 63.233 123.962 1.00 33.18 B
ATOM 9672 CB PRO B 509 107.105 62.167 124.668 1.00 33.11 B
ATOM 9673 CG PRO B 509 106.408 61.468 123.537 1.00 34.75 B
ATOM 9674 C PRO B 509 109.233 63.520 124.722 1.00 30.98 B
ATOM 9675 O PRO B 509 110.218 62.791 124.584 1.00 30.96 B
ATOM 9676 N ILE B 510 109.231 64.593 125.506 1.00 28.53 B
ATOM 9677 CA ILE B 510 110.391 64.935 126.314 1.00 26.76 B
ATOM 9678 CB ILE B 510 110.494 66.458 126.538 1.00 27.15 B
ATOM 9679 CG2 ILE B 510 111.731 66.786 127.362 1.00 27.12 B
ATOM 9680 CG1 ILE B 510 110.561 67.174 125.188 1.00 28.18 B
ATOM 9681 CD1 ILE B 510 111.703 66.715 124.291 1.00 27.09 B
ATOM 9682 C ILE B 510 110.140 64.214 127.635 1.00 24.89 B
ATOM 9683 O ILE B 510 111.050 63.639 128.235 1.00 26.11 B
ATOM 9684 N VAL B 511 108.883 64.247 128.068 1.00 22.67 B
ATOM 9685 CA VAL B 511 108.440 63.575 129.285 1.00 22.18 B
ATOM 9686 CB VAL B 511 108.291 64.548 130.473 1.00 20.56 B
ATOM 9687 CG1 VAL B 511 109.657 65.001 130.946 1.00 20.24 B
ATOM 9688 CG2 VAL B 511 107.446 65.732 130.068 1.00 19.99 B
ATOM 9689 C VAL B 511 107.079 62.957 128.981 1.00 21.71 B
ATOM 9690 O VAL B 511 106.392 63.383 128.055 1.00 22.59 B
ATOM 9691 N PRO B 512 106.682 61.932 129.744 1.00 21.07 B
ATOM 9692 CD PRO B 512 107.531 61.151 130.658 1.00 20.51 B
ATOM 9693 CA PRO B 512 105.391 61.264 129.535 1.0021.35 B
ATOM 9694 CB PRO B 512 105.487 60.036 130.445 1.00 20.59 B
ATOM 9695 CG PRO B 512 106.956 59.765 130.512 1.00 20.52 B
ATOM 9696 C PRO B 512 104.158 62.115 129.869 1.00 22.94 B
ATOM 9697 O PRO B 512 103.139 62.035 129.185 1.00 23.66 B
ATOM 9698 N GLU B 513 104.249 62.917 130.928 1.00 21.88 B
ATOM 9699 CA GLU B 513 103.125 63.743 131.362 1.00 22.21 B
ATOM 9700 CB GLU B 513 102.455 63.113 132.588 1.00 22.52 B
ATOM 9701 CG GLU B 513 101.905 61.721 132.334 1.0025.60 B
ATOM 9702 CD GLU B 513 100.704 61.724 131.400 1.0028.58 B
ATOM 9703 OE GLU B 513 100.345 60.640 130.892 1.00 32.92 B
ATOM 9704 OE2 GLU B 513 100.111 62.800 131.179 1.0029.55 B
ATOM 9705 C GLU B 513 103.559 65.166 131.697 1.00 21.26 B
ATOM 9706 O GLU B 513 104.656 65.384 132.216 1.00 19.04 B
ATOM 9707 N PRO B 514 102.682 66.149 131.430 1.00 20.98 B
ATOM 9708 CD PRO B 514 101.265 65.941 131.081 1.00 20.30 B
ATOM 9709 CA PRO B 514 102.950 67.570 131.686 1.00 19.31 B
ATOM 9710 CB PRO B 514 101.608 68.233 131.373 1.00 18.74 B
ATOM 9711 CG PRO B 514 100.610 67.141 131.712 1.00 19.87 B
ATOM 9712 C PRO B 514 103.457 67.876 133.097 1.00 9.00 B
ATOM 9713 O PRO B 514 104.262 68.786 133.289 1.0019.35 B
ATOM 9714 N SER B 515 102.998 67.113 134.080 1.00 18.22 B
ATOM 9715 CA SER B 515 103.429 67.328 135.456 1.00 18.67 B
ATOM 9716 CB SER B 515 102.699 66.373 136.390 1.00 18.35 B
ATOM 9717 OG SER B 515 103.174 65.054 136.189 1.00 20.02 B
ATOM 9718 C SER B 515 104.933 67.111 135.631 1.00 19.60 B
ATOM 9719 O SER B 515 105.499 67.476 136.661 1.00 20.55 B
ATOM 9720 N GLN B 516 105.582 66.513 134.636 1.00 19.80 B
ATOM 9721 CA GLN B 516 107.015 66.250 134.741 1.00 19.47 B
ATOM 9722 CB GLN B 516 107.331 64.840 134.253 1.00 19.07 B
ATOM 9723 CG GLN B 516 106.557 63.756 134.967 1.0022.11 B
ATOM 9724 CD GLN B 516 106.951 62.378 134.500 1.0024.86 B
ATOM 9725 OE1 GLN B 516 108.039 61.892 134.815 1.00 28.36 B
ATOM 9726 NE2 GLN B 516 106.076 61.741 133.731 1.00 26.21 B
ATOM 9727 C GLN B 516 107.867 67.242 133.968 1.00 18.64 B
ATOM 9728 O GLN B 516 109.093 67.124 133.931 1.00 17.06 B
ATOM 9729 N SER B 517 107.220 68.215 133.343 1.00 16.85 B
ATOM 9730 CA SER B 517 107.955 69.212 132.584 1.00 16.82 B
ATOM 9731 CB SER B 517 107.327 69.393 131.198 1.00 16.29 B
ATOM 9732 OG SER B 517 108.078 70.294 130.400 1.00 17.97 B
ATOM 9733 C SER B 517 107.990 70.552 133.318 1.00 15.76 B
ATOM 9734 O SER B 517 106.962 71.213 133.470 1.00 15.91 B
ATOM 9735 N ILE B 518 109.174 70.931 133.793 1.00 13.11 B
ATOM 9736 CA ILE B 518 109.348 72.210 134.469 1.00 14.05 B
ATOM 9737 CB ILE B 518 110.522 72.171 135.475 1.00 14.00 B
ATOM 9738 CG2 ILE B 518 110.620 73.508 136.201 1.00 14.98 B
ATOM 9739 CE1 ILE B 518 110.344 71.008 136.457 1.00 9.65 B
ATOM 9740 CD1 ILE B 518 109.055 71.042 137.258 1.00 11.35 B
ATOM 9741 C ILE B 518 109.692 73.184 133.339 1.00 15.74 B
ATOM 9742 O ILE B 518 110.804 73.162 132.805 1.00 15.32 B
ATOM 9743 N ASN B 519 108.732 74.022 132.965 1.00 16.03 B
ATOM 9744 CA ASN B 519 108.928 74.973 131.874 1.00 16.42 B
ATOM 9745 CB ASN B 519 107.583 75.284 131.200 1.00 15.19 B
ATOM 9746 CG ASN B 519 106.853 74.028 130.744 1.00 17.35 B
ATOM 9747 OD1 ASN B 519 107.475 73.066 130.281 1.00 15.76 B
ATOM 9748 ND2 ASN B 519 105.526 74.036 130.863 1.00 13.82 B
ATOM 9749 C ASN B 519 109.584 76.272 132.325 1.00 16.57 B
ATOM 9750 O ASN B 519 109.101 76.932 133.249 1.00 16.87 B
ATOM 9751 N TYR B 520 110.676 76.644 131.661 1.00 15.21 B
ATOM 9752 CA TYR B 520 111.387 77.869 132.008 1.00 17.34 B
ATOM 9753 CB TYR B 520 112.271 77.640 133.241 1.00 16.11 B
ATOM 9754 CG TYR B 520 113.528 76.833 132.971 1.00 15.05 B
ATOM 9755 CD1 TYR B 520 113.461 75.561 132.400 1.00 14.16 B
ATOM 9756 CE1 TYR B 520 114.620 74.813 132.154 1.00 12.99 B
ATOM 9757 CD2 TYR B 520 114.786 77.341 133.295 1.00 16.90 B
ATOM 9758 CE2 TYR B 520 115.948 76.603 133.057 1.00 15.19 B
ATOM 9759 CZ TYR B 520 115.858 75.343 132.486 1.00 14.36 B
ATOM 9760 OH TYR B 520 117.005 74.624 132.245 1.00 14.39 B
ATOM 9761 C TYR B 520 112.259 78.389 130.874 1.00 16.75 B
ATOM 9762 O TYR B 520 112.491 77.694 129.882 1.00 16.53 B
ATOM 9763 N VAL B 521 112.728 79.625 131.033 1.00 16.03 B
ATOM 9764 CA VAL B 521 113.615 80.255 130.057 1.00 15.86 B
ATOM 9765 CB VAL B 521 112.880 81.324 129.202 1.00 14.73 B
ATOM 9766 CG1 VAL B 521 111.903 80.644 128.246 1.00 11.47 B
ATOM 9767 CG2 VAL B 521 112.152 82.310 130.109 1.00 14.53 B
ATOM 9768 C VAL B 521 114.789 80.918 130.778 1.00 16.26 B
ATOM 9769 O VAL B 521 115.748 81.350 130.140 1.00 16.23 B
ATOM 9770 N GLU B 522 114.712 80.986 132.108 1.00 15.92 B
ATOM 9771 CA GLU B 522 115.773 81.590 132.908 1.00 15.16 B
ATOM 9772 CB GLU B 522 115.536 83.103 133.054 1.00 16.14 B
ATOM 9773 CG GLU B 522 116.589 83.835 133.897 1.00 16.74 B
ATOM 9774 CD GLU B 522 116.336 85.337 133.991 1.00 18.05 B
ATOM 9775 OE1 GLU B 522 116.169 85.977 132.932 1.00 18.13 B
ATOM 9776 OE2 GLU B 522 116.312 85.882 135.119 1.00 15.26 B
ATOM 9777 C GLU B 522 115.853 80.964 134.293 1.00 16.60 B
ATOM 9778 O GLU B 522 114.837 80.565 134.866 1.00 16.67 B
ATOM 9779 N SER B 523 117.069 80.874 134.821 1.00 15.49 B
ATOM 9780 CA SER B 523 117.300 80.338 136.158 1.00 16.59 B
ATOM 9781 CB SER B 523 117.439 78.812 136.134 1.00 16.99 B
ATOM 9782 OG SER B 523 118.685 78.407 135.595 1.00 19.46 B
ATOM 9783 C SER B 523 118.591 80.974 136.663 1.00 17.12 B
ATOM 9784 O SER B 523 119.205 81.778 135.960 1.00 14.47 B
ATOM 9785 N HIS B 524 119.010 80.623 137.873 1.00 16.94 B
ATOM 9786 CA HIS B 524 120.229 81.203 138.415 1.00 16.35 B
ATOM 9787 CB HIS B 524 120.464 80.716 139.854 1.00 17.75 B
ATOM 9788 CG HIS B 524 120.903 79.289 139.948 1.00 16.64 B
ATOM 9789 CD2 HIS B 524 122.141 78.743 140.009 1.00 16.73 B
ATOM 9790 ND1 HIS B 524 120.017 78.232 139.960 1.00 16.68 B
ATOM 9791 CE1 HIS B 524 120.689 77.097 140.026 1.00 18.54 B
ATOM 9792 NE2 HIS B 524 121.980 77.378 140.057 1.00 19.36 B
ATOM 9793 C HIS B 524 121.430 80.856 137.527 1.00 17.30 B
ATOM 9794 O HIS B 524 122.420 81.586 137.500 1.00 16.75 B
ATOM 9795 N ASP B 525 121.336 79.743 136.799 1.0017.60 B
ATOM 9796 CA ASP B 525 122.412 79.312 135.903 1.00 18.36 B
ATOM 9797 CB ASP B 525 122.332 77.804 135.649 1.00 21.16 B
ATOM 9798 CG ASP B 525 122.893 76.989 136.790 1.00 24.43 B
ATOM 9799 OD1 ASP B 525 122.185 76.079 137.277 1.00 25.22 B
ATOM 9800 OD2 ASP B 525 124.048 77.253 137.189 1.00 25.88 B
ATOM 9801 C ASP B 525 122.372 80.021 134.553 1.00 17.08 B
ATOM 9802 O ASP B 525 121.295 80.312 134.034 1.00 15.84 B
ATOM 9803 N ASN B 526 123.556 80.278 133.998 1.00 16.52 B
ATOM 9804 CA ASN B 526 123.714 80.927 132.695 1.00 17.70 B
ATOM 9805 CB ASN B 526 122.941 80.146 131.625 1.00 17.52 B
ATOM 9806 CG ASN B 526 123.131 78.644 131.745 1.00 17.20 B

ATOM 9807 ODI ASN B 526 122.203 77.919 132.101 1.00 19.82 B
ATOM 9808 ND2 ASN B 526 124.336 78.172 131.460 1.00 16.25 B
ATOM 9809 C ASN B 526 123.272 82.390 132.676 1.00 18.10 B
ATOM 9810 O ASN B 526 122.847 82.931 133.694 1.00 19.88 B
ATOM 9811 N HIS B 527 123.381 83.030 131.515 1.00 17.84 B
ATOM 9812 CA HIS B 527 122.974 84.425 131.379 1.00 17.94 B
ATOM 9813 CB HIS B 527 123.198 84.914 129.946 1.0018.94 B
ATOM 9814 CG HIS B 527 124.639 85.046 129.563 1.00 22.54 B
ATOM 9815 CD2 HIS B 527 125.431 84.262 128.793 1.00 21.04 B
ATOM 9816 ND1 HIS B 527 125.431 86.091 129.990 1.00 24.08 B
ATOM 9817 CE1 HIS B 527 126.648 85.946 129.499 1.00 23.46 B
ATOM 9818 NE2 HIS B 527 126.675 84.844 128.770 1.00 25.58 B
ATOM 9819 C HIS B 527 121.492 84.570 131.705 1.00 17.24 B
ATOM 9820 O HIS B 527 120.710 83.640 131.508 1.00 17.64 B
ATOM 9821 N THR B 528 121.101 85.729 132.217 1.00 16.60 B
ATOM 9822 CA THR B 528 119.695 85.955 132.493 1.00 16.64 B
ATOM 9823 CB THR B 528 119.469 87.255 133.273 1.00 18.27 B
ATOM 9824 OG1 THR B 528 120.001 88.355132.525 1.00 17.96 B
ATOM 9825 CG2 THR B 528 120.140 87.182 134.631 1.00 16.36 B
ATOM 9826 C THR B 528 119.115 86.120 131.093 1.00 17.54 B
ATOM 9827 O THR B 528 119.859 86.383 130.142 1.00 17.88 B
ATOM 9828 N PHE B 529 117.807 85.958 130.949 1.00 16.65 B
ATOM 9829 CA PHE B 529 117.203 86.112 129.636 1.00 17.99 B
ATOM 9830 CB PHE B 529 115.680 85.995 129.722 1.00 15.48 B
ATOM 9831 CG PHE B 529 115.011 85.854 128.382 1.00 16.79 B
ATOM 9832 CD1 PHE B 529 114.969 86.925 127.488 1.00 14.60 B
ATOM 9833 CD2 PHE B 529 114.435 84.641 128.003 1.00 13.16 B
ATOM 9834 CE1 PHE B 529 114.364 86.791 126.237 1.00 15.33 B
ATOM 9835 CE2 PHE B 529 113.829 84.498 126.756 1.00 13.30 B
ATOM 9836 CZ PHE B 529 113.794 85.577 125.869 1.00 13.60 B
ATOM 9837 C PHE B 529 117.579 87.471 129.044 1.00 18.56 B
ATOM 9838 O PHE B 529 117.917 87.572 127.863 1.00 20.03 B
ATOM 9839 N TRP B 530 117.525 88.515 129.864 1.00 18.28 B
ATOM 9840 CA TRP B 530 117.849 89.850 129.386 1.00 19.74 B
ATOM 9841 CB TRP B 530 117.608 90.892 130.483 1.00 20.41 B
ATOM 9842 CG TRPB530 117.865 92.285 129.993 1.00 22.34 B
ATOM 9843 CD2 TRP B 530 116.992 93.081 129.186 1.00 22.03 B
ATOM 9844 CE2 TRP B 530 117.672 94.285 128.897 1.00 23.41 B
ATOM 9845 CE3 TRP B 530 115.699 92.893 128.675 1.00 22.41 B
ATOM 9846 CD1 TRP B 530 119.007 93.018 130.158 1.00 21.79 B
ATOM 9847 NE1 TRP B 530 118.898 94.218 129.504 1.00 20.91 B
ATOM 9848 CZ2 TRP B 530 117.104 95.302 128.115 1.00 23.30 B
ATOM 9849 CZ3 TRP B 530 115.131 93.905 127.896 1.0022.73 B
ATOM 9850 CH2 TRP B 530 115.837 95.094 127.625 1.00 22.90 B
ATOM 9851 C TRP B 530 119.278 89.991 128.867 1.00 19.95 B
ATOM 9852 O TRP B 530 119.493 90.523 127.778 1.00 18.58 B
ATOM 9853 N ASP B 531 120.253 89.520 129.641 1.00 20.69 B
ATOM 9854 CA ASP B 531 121.648 89.629 129.228 1.00 22.05 B
ATOM 9855 CB ASP B 531 122.586 89.212 130.369 1.00 21.41 B
ATOM 9856 CG ASP B 531 122.553 90.190 131.530 1.00 22.55 B
ATOM 9857 OD1 ASP B 531 121.816 91.196 131.430 1.00 21.16 B
ATOM 9858 OD2 ASP B 531 123.255 89.959 132.541 1.00 24.10 B
ATOM 9859 C ASP B 531 121.913 88.801 127.985 1.00 21.46 B
ATOM 9860 O ASP B 531 122.636 89.229 127.088 1.00 22.42 B
ATOM 9861 N LYS B 532 121.318 87.616 127.935 1.00 22.30 B
ATOM 9862 CA LYS B 532 121.463 86.726 126.789 1.00 22.88 B
ATOM 9863 CB LYS B 532 120.662 85.451 127.037 1.0020.95 B
ATOM 9864 CG LYS B 532 120.195 84.725 125.789 1.0022.85 B
ATOM 9865 CD LYS B 532 121.307 84.001 125.080 1.0023.53 B
ATOM 9866 CE LYS B 532 120.764 83.268 123.858 1.00 24.17 B
ATOM 9867 NZ LYS B 532 121.828 82.455 123.227 1.00 22.55 B
ATOM 9868 C LYS B 532 120.960 87.422 125.521 1.00 24.42 B
ATOM 9869 O LYS B 532 121.611 87.399 124.481 1.00 24.26 B
ATOM 9870 N MET B 533 119.794 88.044 125.626 1.00 25.57 B
ATOM 9871 CA MET B 533 119.183 88.741 124.504 1.00 27.18 B
ATOM 9872 CB MET B 533 117.762 89.151 124.887 1.0026.00 B
ATOM 9873 CG MET B 533 117.047 90.029 123.887 1.00 24.76 B
ATOM 9874 SD MET B 533 115.301 90.126 124.308 1.00 24.70 B
ATOM 9875 CE MET B 533 115.379 90.759 125.975 1.00 20.75 B
ATOM 9876 C MET B 533 119.994 89.963 124.085 1.00 28.51 B
ATOM 9877 O MET B 533 119.998 90.349 122.918 1.00 31.16 B
ATOM 9878 N SER B 534 120.687 90.564 125.043 1.00 29.62 B
ATOM 9879 CA SER B 534 121.496 91.744 124.774 1.00 30.50 B
ATOM 9880 CB SER B 534 122.007 92.331 126.091 1.00 29.74 B
ATOM 9881 OG SER B 534 122.552 93.624 125.905 1.00 32.18 B
ATOM 9882 C SERB 534 122.667 91.416 123.846 1.00 30.87 B
ATOM 9883 O SER B 534 123.158 92.285 123.129 1.00 32.39 B
ATOM 9884 N PHE B 535 123.105 90.160 123.852 1.00 31.04 B
ATOM 9885 CA PHE B 535 124.208 89.733 122.998 1.00 30.54 B
ATOM 9886 CB PHE B 535 125.018 88.623 123.677 1.0031.31 B
ATOM 9887 CG PHE B 535 125.839 89.092 124.846 1.00 31.76 B
ATOM 9888 CD1 PHE B 535 126.881 89.997 124.663 1.00 31.40 B
ATOM 9889 CD2 PHE B 535 125.571 88.625 126.131 1.00 31.22 B
ATOM 9890 CE1 PHE B 535 127.647 90.433 125.744 1.00 32.30 B
ATOM 9891 CE2 PHE B 535 126.329 89.053 127.217 1.00 31.88 B
ATOM 9892 CZ PHE B 535 127.370 89.960 127.023 1.00 33.49 B
ATOM 9893 C PHE B 535 123.712 89.225 121.647 1.00 31.71 B
ATOM 9894 O PHE B 535 124.348 89.458 120.620 1.00 31.61 B
ATOM 9895 N ALA B 536 122.572 88.536 121.655 1.00 31.64 B
ATOM 9896 CA ALA B 536 121.999 87.969 120.436 1.00 30.79 B
ATOM 9897 CB ALA B 536 121.005 86.869 120.801 1.00 29.20 B
ATOM 9898 C ALA B 536 121.333 88.995 119.516 1.00 30.42 B
ATOM 9899 O ALA B 536 121.299 88.812 118.300 1.00 30.16 B
ATOM 9900 N LEU B 537 120.799 90.067 120.094 1.00 30.30 B
ATOM 9901 CA LEU B 537 120.141 91.115 119.314 1.00 30.23 B
ATOM 9902 CB LEU B 537 118.622 91.079 119.542 1.00 31.70 B
ATOM 9903 CG LEU B 537 117.823 89.844 119.095 1.00 33.37 B
ATOM 9904 CD1 LEU B 537 118.219 88.642 119.920 1.00 35.97 B
ATOM 9905 CD2 LEU B 537 116.336 90.101 119.271 1.00 34.27 B
ATOM 9906 C LEU B 537 120.698 92.469 119.748 1.00 30.05 B
ATOM 9907 O LEU B 537 119.977 93.299 120.303 1.00 28.61 B
ATOM 9908 N PRO B 538 121.994 92.708 119.492 1.00 31.42 B
ATOM 9909 CD PRO B 538 122.875 91.825 118.708 1.00 32.66 B
ATOM 9910 CA PRO B 538 122.696 93.948 119.849 1.00 33.18 B
ATOM 9911 CB PRO B 538 124.139 93.647 119.457 1.0032.67 B
ATOM 9912 CG PRO B 538 123.969 92.775 118.261 1.00 33.22 B
ATOM 9913 C PRO B 538 122.181 95.230 119.200 1.0035.65 B
ATOM 9914 O PRO B 538 122.420 96.326 119.711 1.00 36.50 B
ATOM 9915 N GLN B 539 121.478 95.098 118.080 1.00 37.55 B
ATOM 9916 CA GLN B 539 120.957 96.267 117.384 1.00 38.88 B
ATOM 9917 CB GLN B 539 121.056 96.074 115.867 1.00 41.40 B
ATOM 9918 CG GLN B 539 122.294 96.698 115.241 1.00 44.16 B
ATOM 9919 CD GLN B 539 123.581 96.203 115.870 1.00 47.09 B
ATOM 9920 OE1 GLN B 539 123.922 95.022 115.770 1.00 48.31 B
ATOM 9921 NE2 GLN B 539 124.304 97.106 116.529 1.00 47.56 B
ATOM 9922 C GLN B 539 119.527 96.631 117.754 1.00 38.88 B
ATOM 9923 O GLN B 539 119.063 97.720 117.420 1.00 39.17 B
ATOM 9924 N GLU B 540 118.821 95.739 118.438 1.0037.50 B
ATOM 9925 CA GLU B 540 117.452 96.052 118.808 1.00 38.16 B
ATOM 9926 CB GLU B 540 116.633 94.771 119.007 1.00 38.87 B
ATOM 9927 CG GLU B 540 115.860 94.377 117.745 1.00 40.84 B
ATOM 9928 CD GLU B 540 114.927 93. t 96 117.946 1.00 41.13 B
ATOM 9929 OE1 GLUB540 114.161 93.206 118.931 1.00 40.25 B
ATOM 9930 OE2 GLU B 540 114.952 92.267 117.108 1.00 40.45 B
ATOM 9931 C GLU B 540 117.406 96.932 120.044 1.00 37.31 B
ATOM 9932 O GLU B 540 118.286 96.857 120.895 1.00 38.16 B
ATOM 9933 N ASN B 541 116.383 97.778 120.123 1.00 37.33 B
ATOM 9934 CA ASN B 541 116.221 98.698 121.246 1.00 39.26 B
ATOM 9935 CB ASNB541 115.373 99.902 120.824 1.00 40.06 B
ATOM 9936 CG ASN B 541 113.972 99.511 120.406 1.0041.85 B
ATOM 9937 OD1 ASN B 541 113.211 98.944 121.189 1.00 41.47 B
ATOM 9938 ND2 ASN B 541 113.621 99.818 119.164 1.00 47.19 B
ATOM 9939 C ASN B 541 115.601 98.034 122.473 1.00 38.94 B
ATOM 9940 O ASN B 541 115.060 96.929 122.392 1.00 39.14 B
ATOM 9941 N ASP B 542 115.679 98.720 123.608 1.00 37.52 B
ATOM 9942 CA ASP B 542 115.145 98.191 124.856 1.00 36.89 B
ATOM 9943 CB ASP B 542 115.475 99.143 126.016 1.00 38.51 B
ATOM 9944 CG ASP B 542 116.966 99.165 126.355 1.0039.16 B
ATOM 9945 OD1 ASP B 542 117.353 99.888 127.299 1.00 41.00 B
ATOM 9946 OD2 ASP B 542 117.751 98.461 125.682 1.00 37.99 B
ATOM 9947 C ASP B 542 113.643 97.914 124.816 1.00 35.61 B
ATOM 9948 O ASP B 542 113.172 96.951 125.423 1.00 34.00 B
ATOM 9949 N SER B 543 112.892 98.741 124.096 1.00 33.19 B
ATOM 9950 CA SER B 543 111.448 98.550 124.014 1.00 31.92 B
ATOM 9951 CB SER B 543 110.801 99.702 123.241 1.00 32.69 B
ATOM 9952 OG SER B 543 109.386 99.615 123.303 1.00 30.32 B
ATOM 9953 C SER B 543 111.078 97.218 123.355 1.00 31.99 B
ATOM 9954 O SER B 543 110.175 96.512 123.821 1.00 31.83 B
ATOM 9955 N ARG B 544 111.767 96.879 122.269 1.00 29.40 B
ATOM 9956 CA ARG B 544 111.498 95.630 121.572 1.00 29.95 B
ATOM 9957 CB ARG B 544 112.126 95.626 120.181 1.00 31.34 B
ATOM 9958 CG ARG B 544 111.283 96.284 119.116 1.00 34.69 B
ATOM 9959 CD ARG B 544 111.883 96.009 117.749 1.00 37.16 B
ATOM 9960 NE ARG B 544 111.135 96.662 116.681 1.00 38.84 B
ATOM 9961 CZ ARG B 544 111.458 96.580 115.396 1.0041.26 B
ATOM 9962 NH1 ARG B 544 112.516 95.868 115.020 1.00 40.96 B
ATOM 9963 NH2 ARG B 544 110.725 97.211 114.488 1.00 43.50 B
ATOM 9964 C ARG B 544 112.041 94.457 122.365 1.00 28.62 B
ATOM 9965 O ARG B 544 111.446 93.380 122.379 1.0027.59 B
ATOM 9966 N LYS B 45 113.186 94.662 123.008 1.0027.21 B
ATOM 9967 CA LYS B 545 113.784 93.611 123.819 1.00 25.87 B
ATOM 9968 CB LYS B 545 115.110 94.083 124.418 1.00 25.83 B
ATOM 9969 CG LYS B 545 116.260 94.163 123.424 1.0027.15 B
ATOM 9970 CD LYS B 545 117.474 94.828 124.067 1.00 28.98 B
ATOM 9971 CE LYS B 545 118.652 94.872 123.117 1.00 33.03 B
ATOM 9972 NZ LYS B 545 119.811 95.604 123.705 1.00 36.68 B
ATOM 9973 C LYS B 545 112.803 93.244 124.931 1.00 23.66 B
ATOM 9974 O LYS B 545 112.510 92.070 125.146 1.00 23.89 B
ATOM 9975 N ARG B 546 112.284 94.252 125.625 1.00 22.53 B
ATOM 9976 CA ARG B 546 111.336 94.002 126.699 1.00 22.49 B
ATOM 9977 CB ARG B 546 110.886 95.316 127.355 1.00 22.41 B
ATOM 9978 CG ARGB546 112.000 96.099 128.046 1.00 23.83 B
ATOM 9979 CD ARG B 546 111.442 97.178 128.970 1.00 23.96 B
ATOM 9980 NE ARG B 546 110.685 98.208 128.263 1.00 27.71 B
ATOM 9981 CZ ARG B 546 111.231 99.228 127.601 1.00 32.19 B
ATOM 9982 NH1 ARG B 546 112.555 99.370 127.552 1.00 29.83 B
ATOM 9983 NH2 ARG B 546 110.449 100.108 126.979 1.00 29.44 B
ATOM 9984 C ARG B 546 110.117 93.237 126.184 1.00 22.17 B
ATOM 9985 O ARG B 546 109.653 92.298 126.830 1.00 23.42 B
ATOM 9986 N SER B 547 109.598 93.622 125.022 1.00 20.78 B
ATOM 9987 CA SER B 547 108.430 92.936 124.486 1.00 20.99 B
ATOM 9988 CB SER B 547 107.913 93.649 123.226 1.00 20.41 B
ATOM 9989 OG SER B 547 108.805 93.497 122.137 1.0024.36 B
ATOM 9990 C SER B 547 108.743 91.464 124.183 1.00 19.60 B
ATOM 9991 O SERB 547 107.889 90.597 124.364 1.00 18.46 B
ATOM 9992 N ARG B 548 109.968 91.185 123.746 1.00 18.36 B
ATOM 9993 CA ARG B 548 110.370 89.816 123.433 1.00 19.98 B
ATOM 9994 CB ARG B 548 111.688 89.798 122.649 1.00 21.12 B
ATOM 9995 CG ARG B 548 111.718 90.764 121.478 1.00 25.86 B
ATOM 9996 CD ARG B 548 112.827 90.451 120.495 1.00 24.84 B
ATOM 9997 NE ARG B 548 112.418 89.363 119.622 1.00 32.73 B
ATOM 9998 CZ ARG B 548 112.219 89.474 118.312 1.00 29.18 B
ATOM 9999 NH1 ARG B 548 112.400 90.636 117.697 1.00 26.07 B
ATOM 10000 NH2 ARG B 548 111.822 88.414 117.624 1.00 30.37 B
ATOM 10001 C ARG B 548 110.517 88.972 124.701 1.00 19.41 B
ATOM 10002 O ARG B 548 110.205 87.779 124.695 1.00 19.45 B
ATOM 10003 N GLN B 549 110.998 89.585 125.782 1.00 16.10 B
ATOM 10004 CA GLN B 549 111.156 88.864 127.042 1.00 15.96 B
ATOM 10005 CB GLN B 549 112.069 89.628 128.011 1.00 14.96 B
ATOM 10006 CG GLN B 549 112.220 88.935 129.361 1.00 16.10 B
ATOM 10007 CD GLN B 549 113.511 89.287 130.087 1.00 15.06 B
ATOM 10008 OE1 GLN B 549 113.660 88.993 131.270 1.00 17.41 B
ATOM 10009 NE2 GLN B 549 114.451 89.901 129.379 1.00 15.20 B
ATOM 10010 C GLN B 549 109.781 88.645 127.663 1.00 15.11 B
ATOM 10011 O GLN B 549 109.526 87.601 128.248 1.0015.86 B
ATOM 10012 N ARG B 550 108.893 89.627 127.533 1.0014.75 B
ATOM 10013 CA ARG B 550 107.541 89.479 128.058 1.00 17.23 B
ATOM 10014 CB ARG B 550 106.719 90.762 127.859 1.00 17.19 B
ATOM 10015 CG ARG B 550 107.117 91.940 128.757 1.00 19.32 B
ATOM 10016 CD ARG B 550 105.976 92.967 128.850 1.00 19.97 B
ATOM 10017 NE ARG B 550 105.583 93.463 127.532 1.0021.18 B
ATOM 10018 CZ ARG B 550 106.244 94.396 126.856 1.0023.68 B
ATOM 10019 NH1 ARG B 550 105.819 94.777 125.659 1.00 22.37 B
ATOM 10020 NH2 ARG B 550 107.317 94.970 127.389 1.00 25.17 B
ATOM 10021 C ARG B 550 106.884 88.323 127.297 1.00 18.51 B
ATOM 10022 O ARG B 550 106.175 87.499 127.885 1.00 17.69 B
ATOM 10023 N LEU B 551 107.142 88.270 125.990 1.00 17.74 B
ATOM 10024 CA LEU B 551 106.612 87.220 125.117 1.00 17.62 B
ATOM 10025 CB LEU B 551 107.133 87.413 123.680 1.00 17.36 B
ATOM 10026 CG LEU B 551 106.768 86.327 122.662 1.00 19.33 B
ATOM 10027 CD1 LEU B 551 105.248 86.211 122.562 1.00 22.50 B
ATOM 10028 CD2 LEU B S51 107.357 86.659 121.301 1.00 21.99 B
ATOM 10029 C LEU B 551 107.041 85.841 125.630 1.00 18.00 B
ATOM 10030 O LEU B 551 106.226 84.912 125.723 1.00 16.96 B
ATOM 10031 N ALA B 552 108.327 85.718 125.951 1.00 15.74 B
ATOM 10032 CA ALA B 552 108.876 84.468 126.455 1.00 16.71 B
ATOM 10033 CB ALA B 552 110.369 84.625 126.718 1.00 15.00 B
ATOM 10034 C ALA B 552 108.149 84.057 127.738 1.00 17.17 B
ATOM 10035 O ALA B 552 107.962 82.867 128.008 1.00 16.13 B
ATOM 10036 N ALA B 553 107.743 85.051 128.522 1.00 16.35 B
ATOM 10037 CA ALA B 553 107.026 84.797 129.764 1.00 18.07 B
ATOM 10038 CB ALA B 553 106.919 86.083 130.586 1.00 16.77 B
ATOM 10039 C ALA B 553 105.635 84.259 129.444 1.00 17.34 B
ATOM 10040 O ALA B 553 105.198 83.261 130.017 1.0018.44 B
ATOM 10041 N ALA B 554 104.948 84.922 128.520 1.0017.87 B
ATOM 10042 CA ALA B 554 103.606 84.509 128.124 1.00 17.79 B
ATOM 10043 CB ALA B 554 103.055 85.460 127.063 1.00 15.92 B
ATOM 10044 C ALA B 554 103.622 83.079 127.590 1.00 18.21 B
ATOM 10045 O ALA B 554 102.745 82.271 127.913 1.0017.84 B
ATOM 10046 N ILE B 555 104.625 82.768 126.773 1.0018.10 B
ATOM 10047 CA ILE B 555 104.742 81.432 126.205 1.00 19.32 B
ATOM 10048 CB ILE B 555 105.978 81.341 125.261 1.00 19.12 B
ATOM 10049 CG2 ILE B 555 106.217 79.890 124.819 1.00 17.37 B
ATOM 10050 CG1 ILE B 555 105.745 82.232 124.034 1.00 15.75 B
ATOM 10051 CD1 ILE B 555 106.940 82.308 123.086 1.00 12.88 B
ATOM 10052 C ILE B 555 104.838 80.385 127.321 1.00 18.87 B
ATOM 10053 O ILE B 555 104.084 79.410 127.333 1.00 18.93 B
ATOM 10054 N ILE B 556 105.755 80.596 128.259 1.00 17.07 B
ATOM 10055 CA ILE B 556 105.932 79.671 129.379 1.00 17.27 B
ATOM 10056 CB ILE B 556 107.139 80.083 130.264 1.00 15.61 B
ATOM 10057 CG2 ILE B 556 107.143 79.277 131.552 1.00 20.43 B
ATOM 10058 CG1 ILE B 556 108.448 79.896 129.498 1.00 16.68 B
ATOM 10059 CD1 ILE B 556 108.761 78.466 129.122 1.00 13.55 B
ATOM 10060 C ILE B 556 104.696 79.620 130.284 1.00 17.27 B
ATOM 10061 O ILE B 556 104.230 78.543 130.658 1.00 16.68 B
ATOM 10062 N LEU B 557 104.174 80.792 130.637 1.00 17.14 B
ATOM 10063 CA LEU B 557 103.027 80.879 131.534 1.00 16.77 B
ATOM 10064 CB LEU B 557 102.869 82.318 132.039 1.00 17.15 B
ATOM 10065 CG LEU B 557 103.940 82.728 133.064 1.00 18.90 B
ATOM 10066 CD1 LEU B 557 103.844 84.210 133.372 1.00 15.10 B
ATOM 10067 CD2 LEU B 557 103.771 81.895 134.343 1.00 18.32 B
ATOM 10068 C LEU B 557 101.704 80.363 130.988 1.00 16.73 B
ATOM 10069 O LEU B 557 100.803 80.059 131.763 1.00 15.80 B
ATOM 10070 N LEU B 558 101.576 80.257 129.669 1.00 16.64 B
ATOM 10071 CA LEU B 558 100.334 79.748 129.096 1.00 18.72 B
ATOM 10072 CB LEU B 558 99.763 80.741 128.083 1.00 19.15 B
ATOM 10073 CG LEU B 558 99.526 82.160 128.622 1.00 22.18 B
ATOM 10074 CD1 LEU B 558 98.790 82.983 127.571 1.00 22.71 B
ATOM 10075 CD2 LEU B 558 98.714 82.108 129.908 1.00 21.04 B
ATOM 10076 C LEU B 558 100.536 78.379 128.450 1.00 19.25 B
ATOM 10077 O LEU B 558 99.734 77.933 127.635 1.00 21.05 B
ATOM 10078 N ALA B 559 101.615 77.710 128.839 1.00 19.37 B
ATOM 10079 CA ALA B 559 101.931 76.387 128.320 1.00 18.17 B
ATOM 10080 CB ALA B 559 103.430 76.270 128.081 1.00 16.12 B
ATOM 10081 C ALA B 559 101.492 75.306 129.299 1.00 17.93 B
ATOM 10082 O ALA B 559 101.466 75.524 130.506 1.00 18.25 B
ATOM 10083 N GLN B 560 101.143 74.136 128.780 1.00 18.44 B
ATOM 10084 CA GLN B 560 100.765 73.029 129.649 1.00 18.63 B
ATOM 10085 CB GLN B 560 100.229 71.849 128.825 1.0015.63 B
ATOM 10086 CG GLN B 560 98.991 72.18 127.986 1.00 12.31 B
ATOM 10087 CD GLN B 560 97.843 72.688 128.829 1.00 12.75 B
ATOM 10088 OE1 GLN B 560 97.562 72.152 129.906 1.00 9.73 B
ATOM 10089 NE2 GLN B 560 97.162 73.720 128.343 1.00 12.33 B
ATOM 10090 C GLN B 560 102.064 72.633 130.350 1.00 18.02 B
ATOM 10091 O GLN B 560 103.136 72.686 129.743 1.00 17.65 B
ATOM 10092 N GLY B 561 101.973 72.246 131.619 1.00 17.31 B
ATOM 10093 CA GLY B 561 103.162 71.866 132.358 1.00 15.62 B
ATOM 10094 C GLY B 561 103.321 72.684 133.629 1.00 16.13 B
ATOM 10095 O GLY B 561 102.357 73.287 134.103 1.00 14.39 B
ATOM 10096 N VAL B 562 104.540 72.716 134.166 1.00 15.75 B
ATOM 10097 CA VAL B 562 104.838 73.433 135.408 1.00 15.80 B
ATOM 10098 CB VAL B 562 105.554 72.483 136.401 1.00 16.60 B
ATOM 10099 CG1 VAL B 562 105.696 73.140 137.771 1.00 15.48 B
ATOM 10100 CG2 VAL B 562 104.769 71.175 136.518 1.00 16.59 B
ATOM 10101 C VAL B 562 105.707 74.674 135.170 1.00 15.42 B
ATOM 10102 O VAL B 562 106.907 74.558 134.917 1.00 16.75 B
ATOM 10103 N PRO B 563 105.107 75.877 135.248 1.00 14.41 B
ATOM 10104 CD PRO B 563 103.666 76.132 135.447 1.00 14.21 B
ATOM 10105 CA PRO B 563 105.836 77.132 135.036 1.00 14.71 B
ATOM 10106 CB PRO B 563 104.714 78.165 134.889 1.00 15.45 B
ATOM 10107 CG PRO B 563 103.632 77.604 135.764 1.00 15.36 B
ATOM 10108 C PRO B 563 106.824 77.476 136.151 1.00 15.44 B
ATOM 10109 O PRO B 563 106.511 77.391 137.345 1.0014.91 B
ATOM 10110 N PHE B 564 108.021 77.872 135.734 1.00 15.12 B
ATOM 10111 CA PHE B 564 109.113 78.212 136.634 1.00 13.61 B
ATOM 10112 CB PHE B 564 110.179 77.112 136.526 1.00 15.72 B
ATOM 10113 CG PHE B 564 111.431 77.358 137.327 1.00 14.70 B
ATOM 10114 CD1 PHE B 564 112.364 78.313 136.922 1.00 14.67 B
ATOM 10115 CD2 PHE B 564 111.714 76.579 138.442 1.00 15.35 B
ATOM 10116 CE1 PHE B 564 113.562 78.478 137.614 1.00 16.08 B
ATOM 10117 CE2 PHE B 564 112.909 76.737 139.143 1.00 15.23 B
ATOM 10118 CZ PHE B 564 113.836 77.688 138.725 1.00 15.56 B
ATOM 10119 C PHE B 564 109.662 79.570 136.214 1.00 13.64 B
ATOM 10120 O PHE B 564 110.007 79.779 135.051 1.00 11.73 B
ATOM 10121 N ILE B 565 109.735 80.485 137.176 1.00 12.63 B
ATOM 10122 CA ILE B 565 110.217 81.836 136.937 1.00 13.22 B
ATOM 10123 CB ILE B 565 109.095 82.849 137.222 1.00 13.93 B
ATOM 10124 CG2 ILE B 565 109.623 84.269 137.140 1.00 14.20 B
ATOM 10125 CG1 ILE B 565 107.957 82.632 136.231 1.00 14.04 B
ATOM 10126 CD1 ILE B 565 106.729 83.456 136.537 1.00 20.50 B
ATOM 10127 C ILE B 565 111.416 82.175 137.818 1.00 15.10 B
ATOM 10128 O ILE B 565 111.388 81.966 139.034 1.00 14.53 B
ATOM 10129 N HIS B 566 112.479 82.684 137.203 1.00 15.22 B
ATOM 10130 CA HIS B 566 113.660 83.071 137.959 1.00 14.83 B
ATOM 10131 CB HIS B 566 114.880 83.181 137.049 1.00 15.52 B
ATOM 10132 CG HIS B 566 116.151 83.475 137.786 1.00 17.29 B
ATOM 10133 CD2 HIS B 566 116.723 82.868 138.852 1.00 15.19 B
ATOM 10134 ND1 HIS B 566 117.000 84.502 137.429 1.00 17.99 B
ATOM 10135 CE1 HIS B 566 118.039 84.513 138.244 1.00 18.18 B
ATOM 10136 NE2 HIS B 566 117.895 83.532 139.117 1.00 17.64 B
ATOM 10137 C HIS B 566 113.376 84.438 138.577 1.00 15.90 B
ATOM 10138 O HIS B 566 112.960 85.359 137.878 1.00 15.73 B
ATOM 10139 N SER B 567 113.588 84.562 139.883 1.00 14.98 B
ATOM 10140 CA SER B 567 113.358 85.823 140.577 1.00 16.66 B
ATOM 10141 CB SER B 567 113.908 85.737 141.998 1.00 17.18 B
ATOM 10142 OG SER B 567 113.777 86.979 142.654 1.00 23.70 B
ATOM 10143 C SER B 567 114.034 86.979 139.833 1.00 17.46 B
ATOM 10144 O SER B 567 115.234 86.933 139.557 1.00 15.80 B
ATOM 10145 N GLY B 568 113.260 88.015 139.518 1.00 18.63 B
ATOM 10146 CA GLY B 568 113.807 89.156 138.803 1.00 18.62 B
ATOM 10147 C GLY B 568 113.510 89.095 137.310 1.00 19.71 B
ATOM 10148 O GLY B 568 113.474 90.122 136.632 1.00 19.95 B
ATOM 10149 N GLN B 569 113.301 87.888 136.796 1.00 19.15 B
ATOM 10150 CA GLN B 569 112.988 87.692 135.379 1.00 19.05 B
ATOM 10151 CB GLN B 569 112.562 86.237 135.142 1.00 19.18 B
ATOM 10152 CG GLN B 569 112.366 85.833 133.677 1.0018.20 B
ATOM 10153 CD GLN B 569 111.725 84.460 133.545 1.00 19.46 B
ATOM 10154 OE1 GLN B 569 112.045 83.547 134.300 1.00 18.53 B
ATOM 10155 NE2 GLN B 569 110.821 84.308 132.575 1.00 20.84 B
ATOM 10156 C GLN B 569 111.850 88.634 134.976 1.00 19.11 B
ATOM 10157 O GLN B 569 111.889 89.243 133.904 1.00 19.23 B
ATOM 10158 N GLU B 570 110.851 88.753 135.852 1.00 17.97 B
ATOM 10159 CA GLU B 570 109.682 89.607 135.619 1.00 18.25 B
ATOM 10160 CB GLU B 570 108.700 89.513 136.799 1.0017.04 B
ATOM 10161 CG GLU B 570 109.224 90.095 138.106 1.00 16.87 B
ATOM 10162 CD GLU B 570 109.952 89.077 138.970 1.00 18.70 B
ATOM 10163 OE1 GLU B 570 110.628 88.186 138.418 1.00 18.51 B
ATOM 10164 OE2 GLU B 570 109.864 89.178 140.209 1.00 17.61 B
ATOM 10165 C GLU B 570 110.034 91.079 135.362 1.00 19.40 B
ATOM 10166 O GLU B 570 109.223 91.832 134.807 1.0018.12 B
ATOM 10167 N PHE B 571 111.219 91.505 135.796 1.00 19.42 B
ATOM 10168 CA PHE B 571 111.637 92.874 135.535 1.00 19.41 B
ATOM 10169 CB PHE B 571 111.565 93.773 136.792 1.0018.43 B
ATOM 10170 CG PHE B 571 112.294 93.248 138.001 1.00 18.09 B
ATOM 10171 CD1 PHE B 571 113.682 93.262 138.065 1.00 19.18 B
ATOM 10172 CD2 PHE B 571 111.579 92.812 139.112 1.00 17.27 B
ATOM 10173 CE1 PHE B 571 114.346 92.854 139.224 1.00 17.74 B
ATOM 10174 CE2 PHE B 571 112.227 92.406 140.265 1.00 15.60 B
ATOM 10175 CZ PHE B 571 113.617 92.429 140.322 1.00 16.46 B
ATOM 10176 C PHE B 571 113.001 92.933 134.869 1.00 19.51 B
ATOM 10177 O PHE B 571 113.865 93.735 135.227 1.0017.23 B
ATOM 10178 N PHE B 572 113.169 92.040 133.892 1.00 18.27 B
ATOM 10179 CA PHE B 572 114.367 91.960 133.069 1.00 17.12 B
ATOM 10180 CB PHE B 572 114.346 93.131 132.091 1.00 17.56 B
ATOM 10181 CG PHE B 572 112.999 93.358 131.478 1.00 18.58 B
ATOM 10182 CD1 PHE B 572 112.501 92.480 130.522 1.00 18.02 B
ATOM 10183 CD2 PHE B 572 112.206 94.423 131.889 1.00 18.12 B
ATOM 10184 CE1 PHE B 572 111.231 92.661 129.986 1.00 18.37 B
ATOM 10185 CE2 PHE B 572 110.935 94.609 131.359 1.00 18.50 B
ATOM 10186 CZ PHE B 572 110.449 93.726 130.407 1.00 19.55 B
ATOM 10187 C PHE B 572 115.688 91.932 133.814 1.00 17.08 B
ATOM 10188 O PHE B 572 116.668 92.541 133.380 1.00 15.84 B
ATOM 10189 N ARG B 573 115.711 91.207 134.923 1.00 17.44 B
ATOM 10190 CA ARG B 573 116.908 91.077 135.741 1.00 17.26 B
ATOM 10191 CB ARG B 573 116.772 89.869 136.676 1.00 18.13 B
ATOM 10192 CG ARG B 573 117.959 89.673 137.592 1.00 19.12 B
ATOM 10193 CD ARG B 573 117.715 88.581 138.609 1.00 17.68 B
ATOM 10194 NE ARG B 573 118.902 88.381 139.426 1.00 17.40 B
ATOM 10195 CZ ARG B 573 118.974 87.549 140.458 1.00 18.64 B
ATOM 10196 NH1 ARG B 573 117.914 86.827 140.806 1.00 15.86 B
ATOM 10197 NH2 ARG B 573 120.109 87.448 141.149 1.00 16.19 B
ATOM 10198 C ARG B 573 118.184 90.939 134.917 1.00 16.13 B
ATOM 10199 O ARG B 573 118.258 90.147 133.977 1.00 16.97 B
ATOM 10200 N THR B 74 119.192 91.720 135.277 1.00 15.29 B
ATOM 10201 CA THR B 574 120.471 91.674 134.589 1.00 14.34 B
ATOM 10202 CB THR B 574 120.761 93.018 133.851 1.00 15.29 B
ATOM 10203 OG1 THR B 574 122.042 92.951 133.213 1.00 13.43 B
ATOM 10204 CG2 THR B 574 120.739 94.194 134.821 1.00 11.53 B
ATOM 10205 C THR B 574 121.590 91.384 135.588 1.00 15.49 B
ATOM 10206 O THR B 574 121.521 91.788 136.749 1.00 13.04 B
ATOM 10207 N LYS B 575 122.601 90.650 135.132 1.0017.03 B
ATOM 10208 CA LYS B 575 123.759 90.316 135.951 1.00 18.00 B
ATOM 10209 CB LYS B 575 123.955 88.801 136.030 1.00 16.96 B
ATOM 10210 CG LYS B 575 122.941 88.107 136.934 1.0019.52 B
ATOM 10211 CD LYS B 575 123.129 86.604 136.929 1.0017.70 B
ATOM 10212 CE LYS B 575 122.105 85.934 137.821 1.00 18.59 B
ATOM 10213 NZ LYS B 575 122.346 84.472 137.909 1.00 16.92 B
ATOM 10214 C LYS B 575 124.971 90.964 135.305 1.00 20.57 B
ATOM 10215 O LYS B 575 126.101 90.484 135.439 1.00 20.77 B
ATOM 10216 N GLN B 576 124.710 92.054 134.586 1.00 21.43 B
ATOM 10217 CA GLN B 576 125.745 92.825 133.913 1.0023.72 B
ATOM 10218 CB GLN B 576 126.658 93.493 134.949 1.00 25.45 B
ATOM 10219 CG GLN B 576 125.905 94.240 136.041 1.00 26.65 B
ATOM 10220 CD GLN B 576 126.829 94.973 136.998 1.0030.15 B
ATOM 10221 OE1 GLN B 576 128.019 94.670 137.086 1.00 30.85 B
ATOM 10222 NE2 GLN B 576 126.278 95.934 137.735 1.00 31.03 B
ATOM 10223 C GLN B 576 126.579 91.989 132.954 1.00 23.82 B
ATOM 10224 O GLN B 576 127.784 92.210 132.818 1.00 24.96 B
ATOM 10225 N GLY B 577 125.934 91.032 132.290 1.00 23.68 B
ATOM 10226 CA GLY B 577 126.631 90.187 131.333 1.00 20.76 B
ATOM 10227 C GLY B 577 127.342 88.976 131.911 1.00 20.94 B
ATOM 10228 O GLY B 577 127.906 88.180 131.162 1.0022.43 B
ATOM 10229 N VAL B 578 127.332 88.822 133.232 1.0020.20 B
ATOM 10230 CA VAL B 578 127.997 87.677 133.842 1.00 19.37 B
ATOM 10231 CB VAL B 578 128.119 87.838 135.377 1.00 20.48 B
ATOM 10232 CG1 VAL B 578 128.638 86.542 135.997 1.00 18.47 B
ATOM 10233 CG2 VAL B 578 129.076 88.984 135.702 1.00 18.76 B
ATOM 10234 C VAL B 578 127.216 86.408 133.525 1.00 19.92 B
ATOM 10235 O VAL B 578 126.013 86.336 133.759 1.00 20.06 B
ATOM 10236 N GLU B 579 127.912 85.408 132.997 1.00 20.12 B
ATOM 10237 CA GLU B 579 127.289 84.145 132.616 1.00 21.44 B
ATOM 10238 CB GLU B 579 128.126 83.476 131.528 1.0024.21 B
ATOM 10239 CG GLU B 579 127.466 82.256 130.908 1.00 31.79 B
ATOM 10240 CD GLU B 579 128.364 81.565 129.907 1.00 36.41 B
ATOM 10241 OE1 GLU B 579 129.042 82.280 129.136 1.0040.80 B
ATOM 10242 OE2 GLU B 579 128.387 80.314 129.883 1.0038.60 B
ATOM 10243 C GLU B 579 127.077 83.143 133.755 1.0020.70 B
ATOM 10244 O GLU B 579 126.053 82.470 133.815 1.00 20.48 B
ATOM 10245 N ASN B 580 128.056 83.042 134.644 1.00 18.61 B
ATOM 10246 CA ASN B 580 128.005 82.105 135.757 1.00 17.94 B
ATOM 10247 CB ASN B 580 128.978 80.969 135.468 1.00 18.48 B
ATOM 10248 CG ASN B 580 129.092 80.000 136.610 1.0021.06 B
ATOM 10249 OD1 ASN B 580 129.942 79.106 136.595 1.00 21.74 B
ATOM 10250 ND2 ASN B 580 128.234 80.161 137.610 1.00 21.83 B
ATOM 10251 C ASN B 580 128.417 82.852 137.021 1.00 17.09 B
ATOM 10252 O ASN B 580 129.582 82.828 137.416 1.00 15.60 B
ATOM 10253 N SERB 581 127.448 83.491 137.666 1.00 16.85 B
ATOM 10254 CA SER B 581 127.709 84.310 138.843 1.00 17.89 B
ATOM 10255 CB SERB 581 126.677 85.440 138.892 1.00 17.50 B
ATOM 10256 OG SER B 581 125.372 84.925 139.122 1.00 17.84 B
ATOM 10257 C SER B 581 127.756 83.633 140.212 1.00 18.27 B
ATOM 10258 O SER B 581 127.488 84.289 141.214 1.00 18.86 B
ATOM 10259 N TYR B 582 128.127 82.356 140.273 1.00 19.14 B
ATOM 10260 CA TYR B 582 128.144 81.638 141.552 1.00 19.65 B
ATOM 10261 CB TYR B 582 128.642 80.203 141.348 1.00 19.42 B
ATOM 10262 CG TYR B 582 130.124 80.080 141.103 1.0019.91 B
ATOM 10263 CD1 TYR B 582 130.672 80.346 139.847 1.00 21.20 B
ATOM 10264 CE1 TYR B 582 132.047 80.233 139.624 1.0023.45 B
ATOM 10265 CD2 TYR B 582 130.981 79.703 142.132 1.00 19.29 B
ATOM 10266 CE2 TYR B 582 132.352 79.587 141.926 1.0021.72 B
ATOM 10267 CZ TYR B 582 132.880 79.852 140.673 1.00 23.30 B
ATOM 10268 OH TYR B 582 134.234 79.734 140.476 1.0022.94 B
ATOM 10269 C TYR B 582 128.917 82.284 142.710 1.00 20.15 B
ATOM 10270 O TYR B 582 128.571 82.088 143.881 1.00 20.64 B
ATOM 10271 N GLN B 583 129.956 83.049 142.395 1.0021.09 B
ATOM 10272 CA GLN B 583 130.753 83.705 143.433 1.00 21.78 B
ATOM 10273 CB GLN B 583 132.139 83.065 143.519 1.00 21.31 B
ATOM 10274 CG GLN B 583 132.952 83.263 142.257 1.0021.68 B
ATOM 10275 CD GLN B 583 134.324 82.630 142.323 1.00 22.41 B
ATOM 10276 OE1 GLN B 583 135.034 82.572 141.320 1.00 25.11 B
ATOM 10277 NE2 GLN B 583 134.708 82.155 143.500 1.00 23.87 B
ATOM 10278 C GLN B 583 130.918 85.200 143.156 1.00 21.25 B
ATOM 10279 O GLN B 583 131.790 85.854 143.730 1.00 20.60 B
ATOM 10280 N SER B 584 130.083 85.735 142.274 1.0021.00 B
ATOM 10281 CA SER B 584 130.150 87.147 141.930 1.00 20.19 B
ATOM 10282 CB SER B 584 129.381 87.401 140.637 1.00 17.43 B
ATOM 10283 OG SER B 584 129.889 86.580 139.600 1.00 20.01 B
ATOM 10284 C SER B 584 129.579 87.991 143.057 1.00 21.18 B
ATOM 10285 O SER B 584 128.729 87.530 143.815 1.00 20.66 B
ATOM 10286 N SER B 585 130.053 89.231 143.154 1.0022.57 B
ATOM 10287 CA SER B 585 129.624 90.163 144.192 1.00 22.58 B
ATOM 10288 CB SER B 585 130.363 91.492 144.029 1.00 23.65 B
ATOM 10289 OG SER B 585 129.895 92.183 142.881 1.00 23.46 B
ATOM 10290 C SER B 585 128.120 90.438 144.182 1.00 22.56 B
ATOM 10291 O SER B 585 127.412 90.055 143.245 1.00 21.96 B
ATOM 10292 N ASP B 586 127.657 91.120 145.230 1.00 20.17 B
ATOM 10293 CA ASP B 586 126.254 91.485 145.390 1.00 22.28 B
ATOM 10294 CB ASP B 586 126.007 92.076 146.783 1.00 21.33 B
ATOM 10295 CG ASP B 586 126.210 91.066 147.896 1.00 22.43 B
ATOM 10296 OD1 ASP B 586 125.534 90.016 147.883 1.00 22.59 B
ATOM 10297 OD2 ASP B 586 127.042 91.324 148.790 1.00 24.07 B
ATOM 10298 C ASP B 586 125.799 92.499 144.337 1.00 24.02 B
ATOM 10299 O ASP B 586 124.615 92.558 143.997 1.00 25.01 B
ATOM 10300 N SER B 587 126.723 93.311 143.829 1.00 23.67 B
ATOM 10301 CA SER B 587 126.343 94.289 142.818 1.00 24.60 B
ATOM 10302 CB SER B 587 127.495 95.266 142.531 1.00 23.98 B
ATOM 10303 OG SER B 587 128.672 94.580 142.159 1.00 32.20 B
ATOM 10304 C SER B 587 125.934 93.537 141.555 1.00 22.62 B
ATOM 10305 O SERB 587 125.108 94.010 140.778 1.00 22.35 B
ATOM 10306 N ILE B 588 126.507 92.353 141.369 1.00 21.16 B
ATOM 10307 CA ILE B 588 126.184 91.512 140.223 1.00 19.94 B
ATOM 10308 CB ILE B 588 127.346 90.528 139.894 1.00 20.51 B
ATOM 10309 CG2 ILE B 588 126.890 89.517 138.842 1.00 19.71 B
ATOM 10310 CG1 ILE B 588 128.584 91.289 139.409 1.00 18.89 B
ATOM 10311 CD1 ILE B 588 128.385 91.993 138.090 1.00 21.77 B
ATOM 10312 C ILE B 588 124.928 90.662 140.490 1.00 20.72 B
ATOM 10313 O ILE B 588 124.037 90.580 139.647 1.00 20.43 B
ATOM 10314 N ASN B 589 124.860 90.051 141.675 1.00 20.65 B
ATOM 10315 CA ASN B 589 123.762 89.150 142.037 1.00 20.39 B
ATOM 10316 CB ASN B 589 124.315 87.978 142.846 1.00 21.19 B
ATOM 10317 CG ASN B 589 125.058 86.988 141.993 1.00 20.80 B
ATOM 10318 OD1 ASN B 589 124.504 86.440 141.040 1.00 21.57 B
ATOM 10319 ND2 ASN B 589 126.320 86.747 142.327 1.00 19.78 B
ATOM 10320 C ASN B 589 122.527 89.649 142.773 1.00 21.27 B
ATOM 10321 O ASN B 589 121.516 88.946 142.799 1.0021.34 B
ATOM 10322 N GLN B 590 122.588 90.829 143.377 1.00 21.25 B
ATOM 10323 CA GLN B 590 121.441 91.321 144.134 1.00 22.52 B
ATOM 10324 CB GLN B 590 121.771 92.663 144.799 1.0023.93 B
ATOM 10325 CG GLN B 590 121.695 93.874 143.884 1.0026.47 B
ATOM 10326 CD GLN B 590 122.302 95.118 144.522 1.0030.50 B
ATOM 10327 OE1 GLN B 590 122.321 95.252 145.746 1.00 30.54 B
ATOM 10328 NE2 GLN B 590 122.790 96.038 143.692 1.00 30.88 B
ATOM 10329 C GLN B 590 120.178 91.463 143.299 1.00 21.54 B
ATOM 10330 O GLN B 590 120.235 91.687 142.093 1.0022.09 B
ATOM 10331 N LEU B 591 119.036 91.294 143.953 1.00 22.67 B
ATOM 10332 CA LEU B 591 117.744 91.440 143.296 1.00 22.71 B
ATOM 10333 CB LEU B 591 116.648 90.754 144.116 1.00 20.44 B
ATOM 10334 CG LEU B 591 115.602 89.883 143.413 1.00 21.39 B
ATOM 10335 CD1 LEU B 591 114.230 90.194 144.013 1.00 17.16 B
ATOM 10336 CD2 LEU B 591 115.599 90.123 141.903 1.00 18.82 B
ATOM 10337 C LEU B 591 117.539 92.953 143.312 1.00 23.30 B
ATOM 10338 O LEU B 591 117.363 93.542 144.382 1.00 24.38 B
ATOM 10339 N ASP B 592 117.572 93.573 142.136 1.00 21.91 B
ATOM 10340 CA ASP B 592 117.440 95.023 142.008 1.00 20.56 B
ATOM 10341 CB ASP B 592 117.972 95.471 140.644 1.0020.31 B
ATOM 10342 CG ASP B 592 118.176 96.972 140.563 1.00 20.60 B
ATOM 10343 OD1 ASP B 592 117.601 97.691 141.413 1.00 17.10 B
ATOM 10344 OD2 ASP B 592 118.903 97.425 139.647 1.00 17.80 B
ATOM 10345 C ASP B 592 116.025 95.550 142.179 1.00 19.24 B
ATOM 10346 O ASP B 592 115.244 95.566 141.233 1.00 19.41 B
ATOM 10347 N TRP B 593 115.700 96.014 143.378 1.00 20.54 B
ATOM 10348 CA TRP B 593 114.367 96.529 143.624 1.00 20.98 B
ATOM 10349 CB TRP B 593 114.096 96.588 145.126 1.0021.37 B
ATOM 10350 CG TRP B 593 113.989 95.206 145.713 1.00 21.77 B
ATOM 10351 CD2 TRP B 593 112.857 94.331 145.639 1.00 20.33 B
ATOM 10352 CE2 TRP B 593 113.221 93.121 146.275 1.0020.19 B
ATOM 10353 CE3 TRP B 593 111.570 94.450 145.096 1.00 19.38 B
ATOM 10354 CD1 TRP B 593 114.966 94.511 146.370 1.00 21.21 B
ATOM 10355 NE1 TRP B 593 114.512 93.259 146.712 1.00 20.24 B
ATOM 10356 CZ2 TRP B 593 112.345 92.038 146.383 1.0020.22 B
ATOM 10357 CZ3 TRP B 593 110.696 93.373 145.203 1.00 19.62 B
ATOM 10358 CH2 TRP B 593 111.089 92.181 145.844 1.0021.50 B
ATOM 10359 C TRP B 593 114.078 97.867 142.950 1.00 21.99 B
ATOM 10360 O TRP B 593 112.916 98.253 142.813 1.00 21.97 B
ATOM 10361 N ASP B 594 115.119 98.575 142.523 1.00 22.11 B
ATOM 10362 CA ASP B 594 114.908 99.832 141.816 1.00 23.89 B
ATOM 10363 CB ASP B 594 116.221 100.592 141.637 1.00 25.61 B
ATOM 10364 CG ASP B 594 116.685 101.272 142.907 1.00 28.49 B
ATOM 10365 OD1 ASP B 594 117.851 101.712 142.934 1.00 31.18 B
ATOM 10366 OD2 ASP B 594 115.891 101.376 143.869 1.00 30.79 B
ATOM 10367 C ASP B 594 114.366 99.437 140.443 1.00 24.93 B
ATOM 10368 O ASP B 594 113.441 100.060 139.913 1.00 26.08 B
ATOM 10369 N ARG B 595 114.960 98.393 139.873 1.00 23.08 B
ATOM 10370 CA ARG B 595 114.542 97.893 138.571 1.00 24.70 B
ATOM 10371 CB ARG B 595 115.488 96.769 138.111 1.00 24.78 B
ATOM 10372 CG ARG B 595 115.089 96.108 136.805 1.00 28.62 B
ATOM 10373 CD ARG B 595 116.229 95.300 136.197 1.0028.70 B
ATOM 10374 NE ARG B 595 117.233 96.183 135.627 1.00 33.39 B
ATOM 10375 CZ ARG B 595 117.589 96.201 134.348 1.00 31.79 B
ATOM 10376 NH1 ARG B 595 117.034 95.373 133.476 1.00 32.11 B
ATOM 10377 NH2 ARG B 595 118.493 97.075 133.938 1.00 34.25 B
ATOM 10378 C ARG B 595 113.092 97.404 138.645 1.00 22.90 B
ATOM 10379 O ARG B 595 112.331 97.557 137.691 1.00 23.93 B
ATOM 10380 N ARG B 596 112.714 96.833 139.786 1.00 22.82 B
ATOM 10381 CA ARG B 596 111.352 96.351 139.984 1.00 21.49 B
ATOM 10382 CB ARG B 596 111.220 95.673 141.356 1.00 20.57 B
ATOM 10383 CG ARG B 596 109.797 95.274 141.766 1.0019.46 B
ATOM 10384 CD ARG B 596 109.092 96.413 142.499 1.00 19.03 B
ATOM 10385 NE ARG B 596 107.746 96.064 142.949 1.00 18.35 B
ATOM 10386 CZ ARG B 596 106.678 95.981 142.159 1.00 18.72 B
ATOM 10387 NH1 ARG B 596 106.782 96.223 140.857 1.00 17.02 B
ATOM 10388 NH2 ARG B 596 105.497 95.669 142.675 1.00 19.16 B
ATOM 10389 C ARG B 596 110.411 97.547 139.876 1.0023.17 B
ATOM 10390 O ARG B 596 109.312 97.435 139.331 1.00 23.59 B
ATOM 10391 N GLU B 597 110.849 98.694 140.387 1.00 23.86 B
ATOM 10392 CA GLU B 597 110.041 99.909 140.318 1.00 25.93 B
ATOM 10393 CB GLU B 597 110.658 101.037 141.146 1.00 29.36 B
ATOM 10394 CG GLU B 597 110.650 100.851 142.641 1.00 36.15 B
ATOM 10395 CD GLU B 597 110.794 102.181 143.354 1.00 40.19 B
ATOM 10396 OE1 GLU B 597 109.937 103.054 143.108 1.00 43.04 B
ATOM 10397 OE2 GLU B 597 111.747 102.360 144.147 1.00 42.46 B
ATOM 10398 C GLU B 597 109.959 100.392 138.876 1.00 24.07 B
ATOM 10399 O GLU B 597 108.881 100.532 138.308 1.00 24.25 B
ATOM 10400 N THR B 598 111.123 100.654 138.298 1.00 23.27 B
ATOM 10401 CA THR B 598 111.217 101.141 136.932 1.00 23.75 B
ATOM 10402 CB THR B 598 112.663 101.088 136.446 1.00 22.12 B
ATOM 10403 OG1 THR B 598 113.485 101.828 137.354 1.00 21.30 B
ATOM 10404 CG2 THR B 598 112.780 101.683 135.057 1.00 20.42 B
ATOM 10405 C THR B 598 110.343 100.388 135.938 1.00 24.49 B
ATOM 10406 O THR B 598 109.727 100.996 135.070 1.00 24.74 B
ATOM 10407 N PHE B 599 110.284 99.067 136.064 1.00 25.41 B
ATOM 10408 CA PHE B 599 109.478 98.274 135.149 1.00 25.23 B
ATOM 10409 CB PHE B 599 110.354 97.206 134.498 1.0023.43 B
ATOM 10410 CG PHE B 599 111.490 97.784 133.717 1.00 24.36 B
ATOM 10411 CD1 PHE B 599 111.244 98.556 132.587 1.00 23.98 B
ATOM 10412 CD2 PHE B 599 112.799 97.629 134.151 1.00 24.16 B
ATOM 10413 CE1 PHE B 599 112.285 99.171 131.905 1.00 25.49 B
ATOM 10414 CE2 PHE B 599 113.847 98.238 133.477 1.00 24.14 B
ATOM 10415 CZ PHE B 599 113.587 99.015 132.350 1.00 26.06 B
ATOM 10416 C PHE B 599 108.270 97.652 135.824 1.00 25.94 B
ATOM 10417 O PHE B 599 107.890 96.518 135.521 1.00 26.04 B
ATOM 10418 N LYS B 600 107.664 98.413 136.732 1.00 25.46 B
ATOM 10419 CA LYS B 600 106.488 97.958 137.459 1.00 27.22 B
ATOM 10420 CB LYS B 600 105.969 99.060 138.387 1.0028.89 B
ATOM 10421 CG LYS B 600 105.615 100.365 137.679 1.00 32.68 B
ATOM 10422 CD LYS B 600 104.737 101.256 138.552 1.00 35.86 B
ATOM 10423 CE LYS B 600 105.442 101.682 139.825 1.00 39.27 B
ATOM 10424 NZ LYS B 600 106.630 102.535 139.531 1.00 44.00 B
ATOM 10425 C LYS B 600 105.373 97.529 136.512 1.00 27.20 B
ATOM 10426 O LYS B 600 104.615 96.611 136.824 1.00 27.46 B
ATOM 10427 N GLU B 601 105.270 98.193 135.362 1.00 27.25 B
ATOM 10428 CA GLU B 601 104.233 97.856 134.394 1.00 27.20 B
ATOM 10429 CB GLU B 601 104.117 98.937 133.317 1.00 30.33 B
ATOM 10430 CG GLU B 601 103.694 100.293 133.866 1.00 37.63 B
ATOM 10431 CD GLU B 601 102.420 100.226 134.706 1.00 40.85 B
ATOM 10432 OE1 GLU B 601 102.075 101.249 135.336 1.00 44.99 B
ATOM 10433 OE2 GLU B 601 101.761 99.163 134.739 1.00 42.46 B
ATOM 10434 C GLU B 601 104.502 96.510 133.750 1.00 25.49 B
ATOM 10435 O GLU B 601 103.589 95.702 133.581 1.00 25.45 B
ATOM 10436 N ASP B 602 105.754 96.266 133.384 1.0024.06 B
ATOM 10437 CA ASP B 602 106.108 94.991 132.782 1.00 24.02 B
ATOM 10438 CB ASP B 602 107.579 95.003 132.380 1.0025.10 B
ATOM 10439 CG ASP B 602 107.889 96.097 131.368 1.00 27.03 B
ATOM 10440 OD 1 ASP B 602 107.492 95.958 130.192 1.00 25.58 B
ATOM 10441 OD2 ASP B 602 108.513 97.107 131.754 1.00 29.61 B
ATOM 10442 C ASP B 602 105.819 93.909 133.818 1.00 23.94 B
ATOM 10443 O ASP B 602 105.287 92.849 133.495 1.00 24.64 B
ATOM 10444 N VAL B 603 106.142 94.202 135.076 1.00 22.87 B
ATOM 10445 CA VAL B 603 105.896 93.268 136.162 1.00 21.48 B
ATOM 10446 CB VAL B 603 106.397 93.839 137.504 1.00 21.31 B
ATOM 10447 CG1 VAL B 603 105.813 93.049 138.669 1.00 20.64 B
ATOM 10448 CG2 VAL B 603 107.917 93.784 137.542 1.00 20.97 B
ATOM 10449 C VAL B 603 104.404 92.955 136.272 1.00 22.61 B
ATOM 10450 O VAL B 603 104.013 91.793 136.418 1.00 22.15 B
ATOM 10451 N HIS B 604 103.573 93.992 136.195 1.00 23.43 B
ATOM 10452 CA HIS B 604 102.125 93.823 136.286 1.00 22.84 B
ATOM 10453 CB HIS B 604 101.428 95.187 136.219 1.00 24.66 B
ATOM 10454 CG HIS B 604 99.937 95.110 136.332 1.0028.68 B
ATOM 10455 CD2 HIS B 604 99.141 94.606 137.305 1.00 30.77 B
ATOM 10456 ND1 HIS B 604 99.090 95.599 135.359 1.0031.46 B
ATOM 10457 CE1 HIS B 604 97.837 95.402 135.729 1.00 32.37 B
ATOM 10458 NE2 HIS B 604 97.840 94.801 136.906 1.0034.35 B
ATOM 10459 C HIS B 604 101.600 92.910 135.174 1.0021.71 B
ATOM 10460 O HIS B 604 100.607 92.210 135.363 1.00 19.59 B
ATOM 10461 N TYR B 605 102.269 92.922 134.020 1.00 21.40 B
ATOM 10462 CA TYR B 605 101.865 92.076 132.895 1.00 22.26 B
ATOM 10463 CB TYR B 605 102.657 92.438 131.632 1.00 20.70 B
ATOM 10464 CG TYR B 605 102.336 91.587 130.411 1.00 23.51 B
ATOM 10465 CD1 TYR B 605 103.277 90.695 129.895 1.00 23.77 B
ATOM 10466 CE1 TYRA 605 103.002 89.922 128.767 1.0026.15 B
ATOM 10467 CD2 TYR B 605 101.098 91.683 129.765 1.0022.88 B
ATOM 10468 CE2 TYR B 605 100.810 90.908 128.626 1.0023.03 B
ATOM 10469 CZ TYR B 605 101.770 90.034 128.136 1.00 24.81 B
ATOM 10470 OH TYR B 605 101.525 89.281 127.013 1.00 25.81 B
ATOM 10471 C TYR B 605 102.099 90.610 133.265 1.00 23.56 B
ATOM 10472 O TYR B 605 101.249 89.747 133.018 1.00 23.51 B
ATOM 10473 N ILE B 606 103.254 90.333 133.862 1.0023.64 B
ATOM 10474 CA ILE B 606 103.571 88.977 134.284 1.00 23.00 B
ATOM 10475 CB ILE B 606 105.001 88.889 134.873 1.0021.96 B
ATOM 10476 CG2 ILE B 606 105.113 87.685 135.780 1.00 22.05 B
ATOM 10477 CG1 ILE B 606 106.029 88.707 133.755 1.00 24.70 B
ATOM 10478 CD1 ILE B 606 105.919 89.682 132.617 1.00 28.04 B
ATOM 10479 C ILE B 606 102.552 88.555 135.343 1.00 22.27 B
ATOM 10480 O ILE B 606 102.080 87.417 135.353 1.00 21.56 B
ATOM 10481 N ARG B 607 102.198 89.483 136.223 1.00 21.03 B
ATOM 10482 CA ARG B 607 101.232 89.181 137.265 1.00 21.17 B
ATOM 10483 CB ARG B 607 101.072 90.366 138.221 1.00 21.14 B
ATOM 10484 CG ARG B 607 99.953 90.156 139.220 1.0023.77 B
ATOM 10485 CD ARG B 607 99.701 91.374 140.087 1.00 28.52 B
ATOM 10486 NE ARG B 607 98.541 91.157 140.948 1.00 32.28 B
ATOM 10487 CZ ARG B 607 98.010 92.080 141.745 1.00 35.06 B
ATOM 10488 NH1 ARG B 607 98.534 93.300 141.801 1.00 34.03 B
ATOM 10489 NH2 ARG B 607 96.944 91.783 142.479 1.00 32.41 B
ATOM 10490 C ARG B 607 99.872 88.815 136.686 1.00 21.08 B
ATOM 10491 O ARG B 607 99.189 87.943 137.214 1.00 21.31 B
ATOM 10492 N ARG B 608 99.473 89.485 135.608 1.00 22.84, B
ATOM 10493 CA ARG B 608 98.181 89.207 134.989 1.00 23.94 B
ATOM 10494 CB ARG B 608 97.781 90.350 134.054 1.00 27.74 B
ATOM 10495 CG ARG B 608 97.450 91.632 134.807 1.00 34.81 B
ATOM 10496 CD ARG B 608 96.250 92.345 134.203 1.0039.48 B
ATOM 10497 NE ARG B 608 95.104 91.446 134.060 1.00 42.84 B
ATOM 10498 CZ ARG B 608 93.925 91.815 133.571 1.00 44.03 B
ATOM 10499 NH1 ARG B 608 93.730 93.067 133.181 1.00 45.80 B
ATOM 10500 NH2 ARG B 608 92.945 90.929 133.452 1.00 44.68 B
ATOM 10501 C ARG B 608 98.178 87.875 134.246 1.00 23.28 B
ATOM 10502 O ARG B 608 97.160 87.177 134.216 1.0022.77 B
ATOM 10503 N LEU B 609 99.314 87.524 133.645 1.00 21.17 B
ATOM 10504 CA LEU B 609 99.436 86.246 132.949 1.00 19.31 B
ATOM 10505 CB LEU B 609 100.822 86.113 132.311 1.00 16.48 B
ATOM 10506 CG LEU B 609 101.073 86.923 131.037 1.00 17.30 B
ATOM 10507 CD1 LEU B 609 102.554 86.837 130.649 1.00 13.19 B
ATOM 10508 CD2 LEU B 609 100.177 86.385 129.909 1.00 12.18 B
ATOM 10509 C LEU B 609 99.234 85.131 133.982 1.00 19.21 B
ATOM 10510 O LEU B 609 98.506 84.164 133.740 1.00 20.08 B
ATOM 10511 N ILE B 610 99.877 85.285 135.137 1.00 18.38 B
ATOM 10512 CA ILE B 610 99.779 84.312 136.218 1.00 17.24 B
ATOM 10513 CB ILE B 610 100.736 84.690 137.381 1.00 17.49 B
ATOM 10514 CG2 ILE B 610 100.483 83.793 138.610 1.00 14.23 B
ATOM 10515 CG1 ILE B 610 102.188 84.557 136.900 1.00 15.77 B
ATOM 10516 CD1 ILE B 610 103.225 84.925 137.941 1.00 15.76 B
ATOM 10517 C ILE B 610 98.339 84.214 136.727 1.00 18.89 B
ATOM 10518 O ILE B 610 97.852 83.119 137.019 1.00 18.74 B
ATOM 10519 N SER B 611 97.667 85.359 136.831 1.00 18.60 B
ATOM 10520 CA SER B 611 96.278 85.403 137.282 1.00 18.22 B
ATOM 10521 CB SER B 611 95.808 86.856 137.436 1.00 20.15 B
ATOM 10522 OG SER B 611 96.433 87.491 138.533 1.00 20.47 B
ATOM 10523 C SER B 611 95.381 84.692 136.272 1.00 17.97 B
ATOM 10524 O SER B 611 94.420 84.017 136.640 1.00 17.85 B
ATOM 10525 N LEU B 612 95.699 84.858 134.993 1.00 17.55 B
ATOM 10526 CA LEU B 612 94.932 84.229 133.925 1.00 18.56 B
ATOM 10527 CB LEU B 612 95.451 84.699 132.561 1.00 19.20 B
ATOM 10528 CG LEU B 612 94.650 84.238 131.342 1.0020.81 B
ATOM 10529 CD1 LEU B 612 93.299 84.939 131.354 1.00 22.69 B
ATOM 10530 CD2 LEU B 612 95.410 84.561 130.056 1.00 19.25 B
ATOM 10531 C LEU B 612 95.054 82.709 134.029 1.00 17.76 B
ATOM 10532 O LEU B 612 94.062 81.985 133.934 1.00 20.46 B
ATOM 10533 N ARG B 613 96.279 82.233 134.222 1.00 16.57 B
ATOM 10534 CA ARG B 613 96.535 80.804 134.346 1.00 15.79 B
ATOM 10535 CB ARG B 613 98.032 80.543 134.533 1.00 14.59 B
ATOM 10536 CG ARG B 613 98.397 79.076 134.526 1.00 10.92 B
ATOM 10537 CD ARG B 613 99.860 78.844 134.846 1.00 15.67 B
ATOM 10538 NE ARG B 613 100.158 77.414 134.864 1.0014.23 B
ATOM 10539 CZ ARG B 613 100.243 76.659 133.774 1.00 15.12 B
ATOM 10540 NH1 ARG B 613 100.067 77.194 132.567 1.00 13.91 B
ATOM 10541 NH2 ARG B 613 100.464 75.359 133.892 1.00 15.74 B
ATOM 10542 C ARG B 613 95.775 80.246 135.541 1.00 17.56 B
ATOM 10543 O ARG B 613 95.141 79.196 135.456 1.00 18.29 B
ATOM 10544 N LYS B 614 95.845 80.952 136.663 1.00 17.75 B
ATOM 10545 CA LYS B 614 95.156 80.510 137.863 1.00 18.94 B
ATOM 10546 CB LYS B 614 95.451 81.463 139.023 1.00 19.41 B
ATOM 10547 CG LYS B 614 94.784 81.049 140.320 1.0020.83 B
ATOM 10548 CD LYS B 614 95.013 82.063 141.427 1.00 23.45 B
ATOM 10549 CE LYS B 614 94.414 81.562 142.737 1.0027.07 B
ATOM 10550 NZ LYS B 614 94.637 82.527 143.854 1.00 32.14 B
ATOM 10551 C LYS B 614 93.645 80.442 137.640 1.00 19.03 B
ATOM 10552 O LYS B 614 92.991 79.477 138.031 1.00 19.95 B
ATOM 10553 N ALA B 615 93.101 81.464 136.994 1.0019.31 B
ATOM 10554 CA ALA B 615 91.666 81.536 136.744 1.00 20.79 B
ATOM 10555 CB ALA B 615 91.276 82.978 136.418 1.00 18.39 B
ATOM 10556 C ALA B 615 91.134 80.602 135.656 1.0020.74 B
ATOM 10557 O ALA B 615 89.923 80.435 135.534 1.0020.89 B
ATOM 10558 N HIS B 616 92.017 79.977 134.879 1.00 20.89 B
ATOM 10559 CA HIS B 616 91.552 79.104 133.801 1.00 20.42 B
ATOM 10560 CB HIS B 616 91.679 79.850 132.474 1.0020.29 B
ATOM 10561 CG HIS B 616 90.811 81.064 132.401 1.00 20.56 B
ATOM 10562 CD2 HIS B 616 91.098 82.377 132.556 1.00 22.48 B
ATOM 10563 ND1 HIS B 616 89.445 80.987 132.243 1.00 22.80 B
ATOM 10564 CE1 HIS B 616 88.927 82.200 132.308 1.00 22.01 B
ATOM 10565 NE2 HIS B 616 89.909 83.062 132.498 1.00 22.49 B
ATOM 10566 C HIS B 616 92.212 77.734 133.701 1.00 20.53 B

ATOM 10567 O HIS B 616 93.290 77.596 133.120 1.00 20.08 B
ATOM 10568 N PRO B 617 91.548 76.695 134.241 1.00 19.03 B
ATOM 10569 CD PRO B 617 90.238 76.774 134.910 1.00 20.89 B
ATOM 10570 CA PRO B 617 92.038 75.314 134.238 1.00 19.48 B
ATOM 10571 CB PRO B 617 90.883 74.531 134.867 1.00 19.71 B
ATOM 10572 CG PRO B 617 90.257 75.532 135.788 1.00 19.99 B
ATOM 10573 C PRO B 617 92.419 74.770 132.863 1.00 18.41 B
ATOM 10574 O PRO B 617 93.076 73.737 132.768 1.00 18.74 B
ATOM 10575 N ALA B 618 91.998 75.447 131.801 1.0018.03 B
ATOM 10576 CA ALA B 618 92.332 74.999 130.451 1.00 17.83 B
ATOM 10577 CB ALA B 618 91.639 75.885 129.407 1.00 17.55 B
ATOM 10578 C ALA B 618 93.848 75.045 130.263 1.00 16.78 B
ATOM 10579 O ALA B 618 94.414 74.245 129.527 1.00 16.59 B
ATOM 10580 N PHE B 619 94.498 75.989 130.939 1.00 15.89 B
ATOM 10581 CA PHE B 619 95.948 76.132 130.856 1.00 16.52 B
ATOM 10582 CB PHE B 619 96.377 77.549 131.257 1.0015.64 B
ATOM 10583 CG PHE B 619 95.924 78.616 130.306 1.00 15.77 B
ATOM 10584 CD1 PHE B 619 96.430 78.675 129.013 1.00 17.01 B
ATOM 10585 CD2 PHE B 619 94.993 79.570 130.708 1.00 13.45 B
ATOM 10586 CE1 PHE B 619 96.014 79.676 128.129 1.00 19.03 B
ATOM 10587 CE2 PHE B 619 94.570 80.570 129.841 1.00 14.47 B
ATOM 10588 CZ PHE B 619 95.080 80.626 128.546 1.00 17.34 B
ATOM 10589 C PHE B 619 96.645 75.141 131.773 1.00 16.48 B
ATOM 10590 O PHE B 619 97.875 75.034 131.762 1.00 17.83 B
ATOM 10591 N ARG B 620 95.859 74.413 132.560 1.00 17.04 B
ATOM 10592 CA ARG B 620 96.414 73.460 133.512 1.00 17.85 B
ATOM 10593 CB ARG B 620 96.205 74.003 134.931 1.00 19.41 B
ATOM 10594 CG ARG B 620 96.867 75.372 135.128 1.00 19.38 B
ATOM 10595 CD ARG B 620 96.684 75.945 136.524 1.0018.17 B
ATOM 10596 NE ARG B 620 95.410 76.638 136.695 1.00 20.70 B
ATOM 10597 CZ ARG B 620 94.326 76.110 137.258 1.00 20.42 B
ATOM 10598 NH1 ARG B 620 94.344 74.866 137.718 1.00 19.90 B
ATOM 10599 NH2 ARG B 620 93.222 76.836 137.364 1.00 21.79 B
ATOM 10600 C ARG B 620 95.890 72.027 133.396 1.00 19.04 B
ATOM 10601 O ARG B 620 95.520 71.404 134.394 1.00 17.26 B
ATOM 10602 N LEU B 621 95.863 71.516 132.167 1.00 20.89 B
ATOM 10603 CA LEU B 621 95.427 70.148 131.894 1.00 22.47 B
ATOM 10604 CB LEU B 621 95.453 69.889 130.384 1.00 21.86 B
ATOM 10605 CG LEU B 621 94.190 70.240 129.581 1.00 24.87 B
ATOM 10606 CD1 LEU B 621 93.467 71.415 130.193 1.00 24.48 B
ATOM 10607 CD2 LEU B 621 94.572 70.531 128.132 1.00 22.44 B
ATOM 10608 C LEU B 621 96.390 69.213 132.636 1.00 23.02 B
ATOM 10609 O LEU B 621 97.606 69.422 132.619 1.00 20.87 B
ATOM 10610 N ARG B 622 95.841 68.184 133.274 1.0023.65 B
ATOM 10611 CA ARG B 622 96.639 67.265 134.081 1.00 26.46 B
ATOM 10612 CB ARG B 622 95.823 66.844 135.308 1.0027.60 B
ATOM 10613 CG ARG B 622 94.812 67.899 135.738 1.00 32.11 B
ATOM 10614 CD ARG B 622 95.122 68.552 137.077 1.00 33.56 B
ATOM 10615 NE ARG B 622 94.474 67.868 138.190 1.00 33.84 B
ATOM 10616 CZ ARG B 622 94.168 68.451 139.346 1.0035.70 B
ATOM 10617 NH1 ARG B 622 94.452 69.732 139.541 1.00 35.36 B
ATOM 10618 NH2 ARG B 622 93.575 67.756 140.308 1.00 34.75 B
ATOM 10619 C ARG B 622 97.208 66.016 133.412 1.0026.33 B
ATOM 10620 O ARG B 622 97.841 65.207 134.082 1.0028.00 B
ATOM 10621 N SER B 623 96.998 65.841 132.112 1.0024.24 B
ATOM 10622 CA SER B 623 97.532 64.655 131.442 1.00 23.52 B
ATOM 10623 CB SER B 623 96.609 63.452 131.664 1.00 25.50 B
ATOM 10624 OG SER B 623 95.418 63.573 130.902 1.00 25.34 B
ATOM 10625 C SER B 623 97.722 64.851 129.943 1.00 21.68 B
ATOM 10626 O SER B 623 97.070 65.693 129.323 1.00 19.93 B
ATOM 10627 N ALA B 624 98.618 64.058 129.369 1.00 19.59 B
ATOM 10628 CA ALA B 624 98.890 64.117 127.938 1.00 21.83 B
ATOM 10629 CB ALA B 624 100.012 63.140 127.579 1.00 20.74 B
ATOM 10630 C ALA B 624 97.621 63.779 127.148 1.00 22.12 B
ATOM 10631 O ALA B 624 97.424 64.268 126.031 1.00 20.33 B
ATOM 10632 N ALA B 625 96.760 62.948 127.735 1.0023.01 B
ATOM 10633 CA ALA B 625 95.515 62.557 127.077 1.00 23.92 B
ATOM 10634 CB ALA B 625 94.902 61.345 127.775 1.00 22.17 B
ATOM 10635 C ALA B 625 94.516 63.714 127.051 1.00 24.14 B
ATOM 10636 O ALA B 625 93.829 63.919 126.051 1.00 23.32 B
ATOM 10637 N ASP B 626 94.424 64.465 128.146 1.0024.17 B
ATOM 10638 CA ASP B 626 93.504 65.598 128.180 1.00 25.98 B
ATOM 10639 CB ASP B 626 93.415 66.226 129.575 1.00 28.66 B
ATOM 10640 CG ASP B 626 92.899 65.266 130.624 1.0031.95 B
ATOM 10641 OD1 ASP B 626 92.080 64.385 130.290 1.00 33.37 B
ATOM 10642 OD2 ASP B 626 93.306 65.408 131.795 1.0033.76 B
ATOM 10643 C ASP B 626 93.990 66.662 127.203 1.00 24.61 B
ATOM 10644 O ASP B 626 93.189 67.318 126.536 1.00 23.92 B
ATOM 10645 N ILE B 627 95.307 66.832 127.132 1.00 22.27 B
ATOM 10646 CA ILE B 627 95.894 67.816 126.236 1.00 22.77 B
ATOM 10647 CB ILE B 627 97.426 67.888 126.412 1.00 23.02 B
ATOM 10648 CG2 ILE B 627 98.026 68.816 125.363 1.00 21.88 B
ATOM 10649 CG1 ILE B 627 97.754 68.373 127.834 1.00 21.57 B
ATOM 10650 CD1 ILE B 627 99.222 68.343 128.191 1.00 18.09 B
ATOM 10651 C ILE B 627 95.559 67.454 124.800 1.00 23.00 B
ATOM 10652 O ILE B 627 95.169 68.310 124.008 1.00 24.61 B
ATOM 10653 N GLN B 628 95.697 66.177 124.469 1.00 24.78 B
ATOM 10654 CA GLN B 628 95.387 65.707 123.118 1.00 27.55 B
ATOM 10655 CB GLN B 628 95.636 64.204 123.015 1.00 29.00 B
ATOM 10656 CG GLN B 628 95.483 63.656 121.616 1.0036.97 B
ATOM 10657 CD GLN B 628 96.622 64.076 120.701 1.0042.02 B
ATOM 10658 OE1 GLN B 628 96.682 63.666 119.539 1.00 44.21 B
ATOM 10659 NE2 GLN B 628 97.535 64.896 121.222 1.00 43.27 B
ATOM 10660 C GLN B 628 93.923 65.995 122.776 1.00 26.38 B
ATOM 10661 O GLN B 628 93.571 66.218 121.622 1.0024.04 B
ATOM 10662 N ARG B 629 93.080 65.998 123.801 1.00 25.88 B
ATOM 10663 CA ARG B 629 91.653 66.232 123.645 1.0026.40 B
ATOM 10664 CB ARG B 629 90.912 65.577 124.813 1.00 28.81 B
ATOM 10665 CG ARG B 629 89.857 64.559 124.422 1.00 36.71 B
ATOM 10666 CD ARG B 629 88.442 65.141 124.443 1.0039.43 B
ATOM 10667 NE ARG B 629 88.189 66.047 123.329 1.00 43.24 B
ATOM 10668 CZ ARG B 629 87.041 66.687 123.127 1.00 43.03 B
ATOM 10669 NH1 ARG B 629 86.025 66.524 123.965 1.00 44.05 B
ATOM 10670 NH2 ARG B 629 86.917 67.499 122.090 1.00 42.08 B
ATOM 10671 C ARG B 629 91.236 67.703 123.562 1.00 25.29 B
ATOM 10672 O ARG B 629 90.314 68.038 122.826 1.00 23.04 B
ATOM 10673 N HIS B 630 91.912 68.581 124.303 1.00 24.90 B
ATOM 10674 CA HIS B 630 91.520 69.988 124.320 1.00 25.06 B
ATOM 10675 CB HIS B 630 91.107 70.375 125.735 1.0026.49 B
ATOM 10676 CG HIS B 630 90.050 69.491 126.308 1.00 27.89 B
ATOM 10677 CD2 HIS B 630 88.704 69.620 126.343 1.00 28.85 B
ATOM 10678 ND1 HIS B 630 90.336 68.281 126.901 1.00 29.79 B
ATOM 10679 CE1 HIS B 630 89.210 67.702 127.279 1.00 30.08 B
ATOM 10680 NE2 HIS B 630 88.205 68.494 126.952 1.0030.19 B
ATOM 10681 C HIS B 630 92.468 71.049 123.789 1.00 23.58 B
ATOM 10682 O HIS B 630 92.170 72.237 123.888 1.00 22.26 B
ATOM 10683 N LEU B 631 93.601 70.643 123.231 1.00 23.67 B
ATOM 10684 CA LEU B 631 94.538 71.615 122.692 1.00 20.08 B
ATOM 10685 CB LEU B 631 95.804 71.666 123.554 1.00 17.85 B
ATOM 10686 CG LEU B 631 96.942 72.580 123.072 1.00 16.78 B
ATOM 10687 CD1 LEU B 631 97.869 72.925 124.231 1.00 15.60 B
ATOM 10688 CD2 LEU B 631 97.719 71.886 121.972 1.00 14.19 B
ATOM 10689 C LEU B 631 94.899 71.287 121.251 1.00 20.28 B
ATOM 10690 O LEU B 631 95.201 70.143 120.918 1.00 18.21 B
ATOM 10691 N GLU B 632 94.862 72.294 120.388 1.0021.10 B
ATOM 10692 CA GLU B 632 95.222 72.078 118.999 1.00 21.78 B
ATOM 10693 CB GLU B 632 93.979 71.747 118.158 1.00 22.40 B
ATOM 10694 CG GLU B 632 93.083 72.924117.805 1.0025.38 B
ATOM 10695 CD GLU B 632 91.868 72.505 116.978 1.0026.50 B
ATOM 10696 OE1 GLU B 632 91.925 71.438 116.331 1.00 25.73 B
ATOM 10697 OE2 GLU B 632 90.861 73.244 116.966 1.0026.42 B
ATOM 10698 C GLU B 632 95.930 73.302 118.444 1.00 21.62 B
ATOM 10699 O GLU B 632 95.642 74.437 118.841 1.00 20.63 B
ATOM 10700 N CYS B 633 96.878 73.065 117.544 1.00 21.85 B
ATOM 10701 CA CYS B 633 97.621 74.152 116.921 1.00 23.81 B
ATOM 10702 CB CYS B 633 98.997 73.664 116.463 1.00 22.90 B
ATOM 10703 SG CYS B 633 99.987 74.926 115.642 1.0023.50 B
ATOM 10704 C CYS B 633 96.822 74.644 115.718 1.00 24.42 B
ATOM 10705 O CYS B 633 96.381 73.845 114.898 1.0026.50 B
ATOM 10706 N LEU B 634 96.635 75.956 115.620 1.0023.85 B
ATOM 10707 CA LEU B 634 95.881 76.549114.520 1.00 23.74 B
ATOM 10708 CB LEU B 634 95.030 77.707 115.046 1.00 22.62 B
ATOM 10709 CG LEU B 634 93.509 77.535 115.111 1.00 23.16 B
ATOM 10710 CD1 LEU B 634 93.105 76.077 115.151 1.00 18.07 B
ATOM 10711 CD2 LEU B 634 92.997 78.285 116.324 1.00 21.13 B
ATOM 10712 C LEU B 634 96.816 77.040 113.420 1.00 24.85 B
ATOM 10713 O LEU B 634 96.569 76.820 112.229 1.00 23.44 B
ATOM 10714 N THR B 635 97.892 77.703 113.831 1.00 25.08 B
ATOM 10715 CA THR B 635 98.880 78.224 112.898 1.00 24.46 B
ATOM 10716 CB THR B 635 98.700 79.739 112.650 1.00 25.66 B
ATOM 10717 OG1 THR B 635 97.338 80.019 112.304 1.00 26.76 B
ATOM 10718 CG2 THR B 635 99.606 80.199 111.516 1.00 24.96 B
ATOM 10719 C THR B 635 100.265 78.022 113.490 1.00 24.69 B
ATOM 10720 O THR B 635 100.462 78.169 114.697 1.00 24.46 B
ATOM 10721 N LEU B 636 101.223 77.684 112.641 1.00 24.53 B
ATOM 10722 CA LEU B 636 102.589 77.490 113.087 1.00 26.49 B
ATOM 10723 CB LEU B 636 102.858 76.011 113.359 1.00 27.05 B
ATOM 10724 CG LEU B 636 103.972 75.717 114.370 1.00 28.35 B
ATOM 10725 CD1 LEU B 636 103.988 74.229 114.688 1.00 29.50 B
ATOM 10726 CD2 LEU B 636 105.310 76.165 113.821 1.00 27.30 B
ATOM 10727 C LEU B 636 103.499 77.998 111.986 1.00 26.49 B
ATOM 10728 O LEU B 636 103.749 77.302 111.011 1.00 28.11 B
ATOM 10729 N LYS B 637 103.972 79.228 112.144 1.00 28.94 B
ATOM 10730 CA LYS B 637 104.854 79.854 111.168 1.00 29.69 B
ATOM 10731 CB LYS B 637 104.134 81.012 110.474 1.0031.75 B
ATOM 10732 CG LYS B 637 102.969 80.587 109.590 1.00 33.43 B
ATOM 10733 CD LYS B 637 102.254 81.799 109.007 1.00 36.76 B
ATOM 10734 CE LYS B 637 101.098 81.387 108.101 1.00 39.08 B
ATOM 10735 NZ LYS B 637 101.559 80.582 106.930 1.0041.61 B
ATOM 10736 C LYS B 637 106.120 80.369 111.843 1.00 31.01 B
ATOM 10737 O LYS B 637 106.244 80.326 113.068 1.00 30.80 B
ATOM 10738 N GLU B 638 107.054 80.859 111.033 1.00 31.44 B
ATOM 10739 CA GLU B 638 108.324 81.381 111.528 1.00 32.37 B
ATOM 10740 CB GLU B 638 109.034 82.179 110.431 1.00 35.28 B
ATOM 10741 CG GLU B 638 109.652 81.353 109.312 1.0041.68 B
ATOM 10742 CD GLU B 638 108.641 80.482 108.586 1.00 45.83 B
ATOM 10743 OE1 GLU B 638 107.509 80.961 108.329 1.00 45.40 B
ATOM 10744 OE2 GLU B 638 108.990 79.322 108.264 1.00 47.14 B
ATOM 10745 C GLU B 638 108.206 82.268 112.766 1.00 30.36 B
ATOM 10746 O GLU B 638 108.852 82.013 113.780 1.00 30.28 B
ATOM 10747 N HIS B 639 107.384 83.310 112.681 1.00 27.73 B
ATOM 10748 CA HIS B 639 107.233 84.241 113.794 1.00 25.81 B
ATOM 10749 CB HIS B 639 107.693 85.630 113.352 1.00 26.39 B
ATOM 10750 CG HIS B 639 108.910 85.608 112.480 1.0027.78 B
ATOM 10751 CD2 HIS B 639 109.079 85.938 111.177 1.00 26.81 B
ATOM 10752 ND1 HIS B 639 110.137 85.166 112.926 1.00 27.60 B
ATOM 10753 CE1 HIS B 639 111.010 85.223 111.935 1.0028.31 B
ATOM 10754 NE2 HIS B 639 110.392 85.688 110.863 1.0028.93 B
ATOM 10755 C HIS B 639 105.811 84.333 114.322 1.00 24.83 B
ATOM 10756 O HIS B 639 105.454 85.312 114.982 1.00 24.12 B
ATOM 10757 N LEU B 640 105.000 83.317 114.048 1.00 23.72 B
ATOM 10758 CA LEU B 640 103.613 83.335 114.496 1.00 24.36 B
ATOM 10759 CB LEU B 640 102.726 83.895 113.373 1.00 26.86 B
ATOM 10760 CG LEU B 640 101.254 84.297 113.575 1.00 29.56 B
ATOM 10761 CD1 LEU B 640 100.442 83.134 114.135 1.00 28.38 B
ATOM 10762 CD2 LEU B 640 101.179 85.495 114.493 1.00 29.20 B
ATOM 10763 C LEU B 640 103.132 81.944 114.897 1.00 23.82 B
ATOM 10764 O LEU B 640 103.220 80.996 114.116 1.00 24.09 B
ATOM 10765 N ILE B 641 102.635 81.829 116.126 1.00 22.59 B
ATOM 10766 CA ILE B 641 102.109 80.568 116.626 1.00 20.59 B
ATOM 10767 CB ILE B 641 103.004 79.935 117.718 1.00 20.96 B
ATOM 10768 CG2 ILE B 641 102.312 78.697 118.301 1.00 21.31 B
ATOM 10769 CG1 ILE B 641 104.357 79.527 117.134 1.00 21.64 B
ATOM 10770 CD1 ILE B 641 105.302 78.909 118.171 1.00 21.35 B
ATOM 10771 C ILE B 641 100.739 80.832 117.231 1.00 20.87 B
ATOM 10772 O ILE B 641 100.588 81.649 118.148 1.00 21.21 B
ATOM 10773 N ALA B 642 99.735 80.147 116.706 1.00 19.75 B
ATOM 10774 CA ALA B 642 98.380 80.304 117.201 1.00 19.77 B
ATOM 10775 CB ALA B 642 97.497 80.916 116.124 1.00 20.18 B
ATOM 10776 C ALA B 642 97.857 78.936 117.589 1.00 19.41 B
ATOM 10777 O ALA B 642 97.857 78.018 116.775 1.0021.26 B
ATOM 10778 N TYR B 643 97.447 78.789 118.842 1.00 8.20 B
ATOM 10779 CA TYR B 643 96.894 77.527 119.301 1.00 17.70 B
ATOM 10780 CB TYR B 643 97.878 76.765 120.203 1.00 16.86 B
ATOM 10781 CG TYR B 643 98.186 77.417 121.534 1.00 17.95 B
ATOM 10782 CD1 TYR B 643 99.103 78.469 121.625 1.00 16.95 B
ATOM 10783 CE1 TYR B 643 99.398 79.061 122.849 1.0017.01 B
ATOM 10784 CD2 TYR B 643 97.567 76.974 122.708 1.00 17.82 B
ATOM 10785 CE2 TYR B 643 97.855 77.561 123.942 1.00 16.65 B
ATOM 10786 CZ TYR B 643 98.774 78.604 124.002 1.00 18.75 B
ATOM 10787 OH TYR B 643 99.078 79.183 125.213 1.00 19.88 B
ATOM 10788 C TYR B 643 95.616 77.852 120.046 1.00 17.55 B
ATOM 10789 O TYR B 643 95.313 79.020 120.279 1.00 18.18 B
ATOM 10790 N ARG B 644 94.859 76.829 120.411 1.00 19.15 B
ATOM 10791 CA ARG B 644 93.606 77.064 121.109 1.00 22.41 B
ATOM 10792 CB ARG B 644 92.503 77.362 120.084 1.00 22.80 B
ATOM 10793 CG ARG B 644 92.182 76.189 119.150 1.0022.62 B
ATOM 10794 CD ARG B 644 90.932 75.448 119.602 1.00 20.87 B
ATOM 10795 NE ARG B 644 89.722 76.205 119.291 1.00 20.90 B
ATOM 10796 CZ ARG B 644 89.191 76.304 118.074 1.00 20.20 B
ATOM 10797 NH1 ARG B 644 89.757 75.686 117.044 1.00 17.87 B
ATOM 10798 NH2 ARG B 644 88.100 77.039 117.882 1.00 18.50 B
ATOM 10799 C ARG B 644 93.206 75.879 121.972 1.00 22.44 B
ATOM 10800 O ARG B 644 93.560 74.737 121.682 1.0024.54 B
ATOM 10801 N LEU B 645 92.477 76.168 123.044 1.0023.53 B
ATOM 10802 CA LEU B 645 91.989 75.147 123.966 1.00 24.02 B
ATOM 10803 CB LEU B 645 92.334 75.516 125.413 1.00 23.26 B
ATOM 10804 CG LEU B 645 93.781 75.376 125.917 1.00 25.12 B
ATOM 10805 CD1 LEU B 645 94.668 74.763 124.853 1.00 23.02 B
ATOM 10806 CD2 LEU B 645 94.306 76.740 126.345 1.00 22.39 B
ATOM 10807 C LEU B 645 90.480 75.139 123.756 1.00 24.99 B
ATOM 10808 O LEU B 645 89.833 76.188 123.850 1.00 25.07 B
ATOM 10809 N TYR B 646 89.921 73.964 123.481 1.0023.47 B
ATOM 10810 CA TYR B 646 88.498 73.865 123.180 1.00 24.56 B
ATOM 10811 CB TYR B 646 88.331 73.633 121.670 1.00 23.54 B
ATOM 10812 CG TYR B 646 88.931 72.331 121.183 1.00 23.94 B
ATOM 10813 CD1 TYR B 646 88.142 71.191 121.036 1.00 23.52 B
ATOM 10814 CE1 TYR B 646 88.692 69.982 120.619 1.0023.43 B
ATOM 10815 CD2 TYR B 646 90.295 72.231 120.897 1.0023.58 B
ATOM 10816 CE2 TYR B 646 90.857 71.025 120.476 1.00 22.76 B
ATOM 10817 CZ TYR B 646 90.048 69.903 120.342 1.0025.34 B
ATOM 10818 OH TYR B 646 90.592 68.702 119.943 1.0023.59 B
ATOM 10819 C TYR B 646 87.739 72.800 123.959 1.00 24.62 B
ATOM 10820 O TYR B 646 88.339 71.997 124.668 1.00 25.51 B
ATOM 10821 N ASP B 647 86.415 72.801 123.806 1.0024.96 B
ATOM 10822 CA ASP B 647 85.546 71.862 124.505 1.00 26.80 B
ATOM 10823 CB ASP B 647 85.714 70.454 123.933 1.00 28.21 B
ATOM 10824 CG ASP B 647 85.083 70.309 122.559 1.0030.55 B
ATOM 10825 OD1 ASP B 647 85.224 69.235 121.943 1.00 31.17 B
ATOM 10826 OD2 ASP B 647 84.437 71.274 122.096 1.00 32.62 B
ATOM 10827 C ASP B 647 85.880 71.883 125.992 1.00 27.33 B
ATOM 10828 O ASP B 647 86.055 70.842 126.621 1.00 25.75 B
ATOM 10829 N LEU B 648 85.960 73.093 126.533 1.00 29.59 B
ATOM 10830 CA LEU B 648 86.297 73.320 127.932 1.00 33.39 B
ATOM 10831 CB LEU B 648 87.156 74.583 128.043 1.00 30.67 B
ATOM 10832 CG LEU B 648 88.318 74.676 127.051 1.00 29.05 B
ATOM 10833 CD1 LEU B 648 88.947 76.065 127.104 1.00 26.79 B
ATOM 10834 CD2 LEU B 648 89.341 73.590 127.366 1.00 27.42 B
ATOM 10835 C LEU B 648 85.035 73.495 128.762 1.00 37.52 B
ATOM 10836 O LEU B 648 85.085 73.960 129.902 1.00 37.42 B
ATOM 10837 N ASP B 649 83.904 73.111 128.185 1.00 42.42 B
ATOM 10838 CA ASP B 649 82.618 73.253 128.853 1.00 46.97 B
ATOM 10839 CB ASP B 649 81.535 72.561 128.027 1.0050.28 B
ATOM 10840 CG ASP B 649 81.324 73.232 126.681 1.00 54.68 B
ATOM 10841 OD1 ASP 649 82.283 73.263 125.872 1.0055.85 B
ATOM 10842 OD2 ASP B 649 80.202 73.734 126.438 1.00 56.19 B
ATOM 10843 C ASP B 649 82.552 72.778 130.299 1.00 47.20 B
ATOM 10844 O ASP B 649 81.926 73.426 131.133 1.00 46.75 B
ATOM 10845 N GLU B 650 83.201 71.662 130.608 1.00 48.21 B
ATOM 10846 CA GLU B 650 83.159 71.143 131.970 1.0049.52 B
ATOM 10847 CB GLU B 650 82.958 69.622 131.945 1.00 52.53 B
ATOM 10848 CG GLU B 650 81.774 69.162 131.102 1.00 55.58 B
ATOM 10849 CD GLU B 650 80.496 69.900 131.451 1.00 57.16 B
ATOM 10850 OE1 GLU B 650 80.071 69.836 132.625 1.00 57.91 B
ATOM 10851 OE2 GLU B 650 79.919 70.547 130.551 1.00 58.85 B
ATOM 10852 C GLU B 650 84.396 71.460 132.804 1.00 48.60 B
ATOM 10853 O GLU B 650 84.460 71.095 133.979 1.00 49.58 B
ATOM 10854 N VAL B 651 85.371 72.145 132.217 1.00 45.84 B
ATOM 10855 CA VAL B 651 86.597 72.440 132.948 1.00 43.50 B
ATOM 10856 CB VAL B 651 87.790 71.680 132.335 1.00 42.74 B
ATOM 10857 CG1 VAL B 651 87.467 70.204 132.253 1.00 43.50 B
ATOM 10858 CG2 VAL B 651 88.113 72.230 130.958 1.00 42.71 B
ATOM 10859 C VAL B 651 86.985 73.908 133.066 1.00 42.07 B
ATOM 10860 O VAL B 651 87.768 74.272 133.946 1.00 42.48 B
ATOM 10861 N ASP B 652 86.447 74.753 132.195 1.00 39.07 B
ATOM 10862 CA ASP B 652 86.796 76.166 132.241 1.00 35.90 B
ATOM 10863 CB ASP B 652 87.777 76.492 131.108 1.00 32.79 B
ATOM 10864 CG ASP B 652 88.582 77.754 131.369 1.00 30.36 B
ATOM 10865 OD1 ASP B 652 88.019 78.864 131.263 1.00 26.29 B
ATOM 10866 OD2 ASP B 652 89.786 77.631 131.683 1.00 27.16 B
ATOM 10867 C ASP B 652 85.582 77.081 132.157 1.00 36.24 B
ATOM 10868 O ASP B 652 84.512 76.684 131.688 1.00 34.51 B
ATOM 10869 N GLU B 653 85.769 78.308 132.633 1.00 36.45 B
ATOM 10870 CA GLU B 653 84.736 79.331 132.627 1.00 37.99 B
ATOM 10871 CB GLU B 653 85.204 80.534 133.453 1.00 41.34 B
ATOM 10872 CG GLU B 653 84.307 81.758 133.365 1.00 47.61 B
ATOM 10873 CD GLU B 653 84.886 82.977 134.079 1.00 50.75 B
ATOM 10874 OE1 GLU B 653 84.224 84.042 134.066 1.00 52.51 B
ATOM 10875 OE2 GLU B 653 85.997 82.873 134.649 1.00 51.17 B
ATOM 10876 C GLU B 653 84.489 79.753 131.182 1.00 37.07 B
ATOM 10877 O GLU B 653 83.394 80.194 130.825 1.00 37.17 B
ATOM 10878 N TRP B 654 85.519 79.618 130.354 1.00 34.84 B
ATOM 10879 CA TRP B 654 85.409 79.979 128.952 1.00 33.19 B
ATOM 10880 CB TRP B 654 86.616 80.808 128.509 1.00 31.47 B
ATOM 10881 CG TRP B 654 86.679 82.144 129.190 1.0031.78 B
ATOM 10882 CD2 TRP B 654 87.837 82.972 129.359 1.00 31.25 B
ATOM 10883 CE2 TRP B 654 87.421 84.138 130.044 1.00 32.50 B
ATOM 10884 CE3 TRP B 654 89.184 82.843 129.001 1.00 30.12 B
ATOM 10885 CD1 TRP B 654 85.637 82.824 129.761 1.00 32.16 B
ATOM 10886 NE1 TRP B 654 86.075 84.020 130.276 1.00 31.65 B
ATOM 10887 CZ2 TRP B 654 88.307 85.171 130.378 1.00 32.05 B
ATOM 10888 CZ3 TRP B 654 90.066 83.870 129.334 1.0031.34 B
ATOM 10889 CH2 TRP B 654 89.622 85.019 130.017 1.00 31.21 B
ATOM 10890 C TRP B 654 85.270 78.746 128.085 1.00 33.42 B
ATOM 10891 O TRP B 654 85.914 77.725 128.321 1.0033.95 B
ATOM 10892 N LYS B 655 84.415 78.862 127.079 1.0033.98 B
ATOM 10893 CA LYS B 655 84.125 77.784 126.142 1.00 34.51 B
ATOM 10894 CB LYS B 655 82.886 78.176 125.325 1.00 37.26 B
ATOM 10895 CG LYS B 655 82.288 77.095 124.443 1.00 42.59 B
ATOM 10896 CD LYS B 655 81.040 77.628 123.730 1.0045.17 B
ATOM 10897 CE LYS B 655 80.399 76.581 122.819 1.0049.34 B
ATOM 10898 NZ LYS B 655 79.886 75.386 123.559 1.00 50.36 B
ATOM 10899 C LYS B 655 85.309 77.510 125.213 1.00 33.03 B
ATOM 10900 O LYS B 655 85.654 76.356 124.947 1.0032.16 B
ATOM 10901 N ASP B 656 85.934 78.581 124.733 1.0030.32 B
ATOM 10902 CA ASP B 656 87.054 78.469 123.807 1.00 28.15 B
ATOM 10903 CB ASP B 656 86.536 78.632 122.369 1.00 27.18 B
ATOM 10904 CG ASP B 656 87.516 78.126 121.317 1.00 28.81 B
ATOM 10905 OD1 ASPB 656 87.396 78.554 120.152 1.00 30.35 B
ATOM 10906 OD2 ASP B 656 88.393 77.295 121.635 1.00 27.91 B
ATOM 10907 C ASP B 656 88.076 79.567 124.114 1.00 27.19. B
ATOM 10908 O ASP B 656 87.702 80.688 124.467 1.00 27.89 B
ATOM 10909 N ILE B 657 89.359 79.241 123.984 1.00 23.56 B
ATOM 10910 CA ILE B 657 90.419 80.212 124.225 1.00 21.11 B
ATOM 10911 CB ILE B 657 91.168 79.966 125.555 1.0019.96 B
ATOM 10912 CG2 ILE B 657 92.162 81.095 125.784 1.00 17.49 B
ATOM 10913 CG1 ILE B 657 90.195 79.893 126.730 1.00 20.65 B
ATOM 10914 CD1 ILE B 657 90.888 79.663 128.080 1.00 19.64 B
ATOM 10915 C ILE B 657 91.454 80.118 123.113 1.00 21.48 B
ATOM 10916 O ILE B 657 91.961 79.038 122.818 1.00 21.03 B
ATOM 10917 N ILE B 658 91.771 81.253 122.502 1.00 22.21 B
ATOM 10918 CA ILE B 658 92.763 81.291 121.433 1.00 22.68 B
ATOM 10919 CB ILE B 658 92.169 81.874 120.134 1.00 22.71 B
ATOM 10920 CG2 ILE B 658 93.257 81.989 119.073 1.00 21.17 B
ATOM 10921 CG1 ILE B 658 91.016 80.994 119.648 1.00 22.78 B
ATOM 10922 CD1 ILE B 658 90.279 81.555 118.451 1.00 23.86 B
ATOM 10923 C ILE B 658 93.941 82.163 121.851 1.00 22.32 B
ATOM 10924 O ILE B 658 93.758 83.298 122.285 1.00 24.51 B
ATOM 10925 N VAL B 659 95.147 81.627 121.723 1.00 22.17 B
ATOM 10926 CA VAL B 659 96.350 82.365 122.072 1.00 21.16 B
ATOM 10927 CB VAL B 659 97.117 81.665 123.208 1.00 20.66 B
ATOM 10928 CG1 VAL B 659 98.408 82.417 123.508 1.00 21.59 B
ATOM 10929 CG2 VAL B 659 96.246 81.604 124.446 1.00 21.24 B
ATOM 10930 C VAL B 659 97.246 82.464 120.842 1.00 21.04 B
ATOM 10931 O VAL B 659 97.466 81.474 120.147 1.0022.40 B
ATOM 10932 N ILE B 660 97.753 83.663 120.574 1.00 19.62 B
ATOM 10933 CA ILE B 660 98.614 83.888 119.421 1.0019.46 B
ATOM 10934 CB ILE B 660 97.895 84.736 118.354 1.00 20.14 B
ATOM 10935 CG2 ILE B 660 98.798 84.916 117.143 1.00 18.64 B
ATOM 10936 CG1 ILE B 660 96.576 84.071 117.962 1.00 21.35 B
ATOM 10937 CD1 ILE B 660 95.550 85.034 117.411 1.00 23.17 B
ATOM 10938 C ILE B 660 99.886 84.623 119.820 1.00 19.26 B
ATOM 10939 O ILE B 660 99.833 85.763 120.260 1.00 21.02 B
ATOM 10940 N HIS B 661 101.030 83.970 119.655 1.00 19.88 B
ATOM 10941 CA HIS B 661 102.306 84.578 119.998 1.00 18.75 B
ATOM 10942 CB HIS B 661 103.196 83.567 120.726 1.0017.95 B
ATOM 10943 CG HIS B 661 102.684 83.179 122.080 1.00 18.13 B
ATOM 10944 CD2 HIS B 661 102.415 83.925 123.177 1.00 17.69 B
ATOM 10945 ND1 HIS B 661 102.382 81.876 122.420 1.00 17.88 B
ATOM 10946 CE1 HIS B 661 101.948 81.838 123.668 1.0017.91 B
ATOM 10947 NE2 HIS B 661 101.958 83.068 124.150 1.00 20.84 B
ATOM 10948 C HIS B 661 102.997 85.077 118.733 1.00 19.70 B
ATOM 10949 O HIS B 661 103.262 84.307 117.809 1.00 19.20 B
ATOM 10950 N HIS B 662 103.271 86.377 118.695 1.0020.49 B
ATOM 10951 CA HIS B 662 103.922 86.985 117.543 1.0021.64 B
ATOM 10952 CB HIS B 662 103.117 88.189 117.053 1.0021.26 B
ATOM 10953 CG HIS B 662 103.490 88.642 115.675 1.0023.63 B
ATOM 10954 CD2 HIS B 662 103.991 87.959 114.618 1.00 21.78 B
ATOM 10955 ND1 HIS B 662 103.337 89.945 115.250 1.00 22.74 B
ATOM 10956 CE1 HIS B 662 103.728 90.044 113.992 1.0022.99 B
ATOM 10957 NE2 HIS B 662 104.129 88.853 113.586 1.00 22.49 B
ATOM 10958 C HIS B 662 105.327 87.424 117.938 1.00 21.07 B
ATOM 10959 O HIS B 662 105.505 88.174 118.902 1.0021.01 B
ATOM 10960 N ALA B 663 106.319 86.957 117.189 1.00 21.48 B
ATOM 10961 CA ALA B 663 107.709 87.279 117.485 1.00 22.74 B
ATOM 10962 CB ALA B 663 108.505 85.984 117.677 1.00 21.12 B
ATOM 10963 C ALA B 663 108.391 88.163 116.439 1.00 22.50 B
ATOM 10964 O ALA B 663 109.612 88.13 116.308 1.0021.27 B
ATOM 10965 N SER B 664 107.604 88.923 115.684 1.00 22.15 B
ATOM 10966 CA SER B 664 108.161 89.823 114.684 1.00 22.55 B
ATOM 10967 CB SER B 664 107.977 89.261 113.264 1.00 20.55 B
ATOM 10968 OG SER B 664 106.622 89.281 112.858 1.0025.56 B
ATOM 10969 C SER B 664 107.451 91.166 114.855 1.00 23.24 B
ATOM 10970 O SER B 664 106.272 91.214 115.193 1.00 23.40 B
ATOM 10971 N PRO B 665 108.169 92.277 114.630 1.0024.91 B
ATOM 10972 CD PRO B 665 109.562 92.323 114.148 1.00 24.78 B
ATOM 10973 CA PRO B 665 107.625 93.631 114.768 1.00 26.58 B
ATOM 10974 CB PRO B 665 108.876 94.497 114.711 1.00 25.82 B
ATOM 10975 CG PRO B 665 109.709 93.771 113.709 1.0025.33 B
ATOM 10976 C PRO B 665 106.579 94.066 113.741 1.00 28.78 B
ATOM 10977 O PRO B 665 105.857 95.041 113.967 1.0028.42 B
ATOM 10978 N ASP B 666 106.493 93.363 112.619 1.00 30.11 B
ATOM 10979 CA ASP B 666 105.525 93.746 111.604 1.00 33.10 B
ATOM 10980 CB ASP B 666 105.760 92.972 110.299 1.00 35.61 B
ATOM 10981 CG ASP B 666 105.420 91.498 110.415 1.00 41.04 B
ATOM 10982 OD1 ASP B 666 104.257 91.174 110.739 1.00 44.03 B
ATOM 10983 OD2 ASP B 666 106.313 90.658 110.173 1.00 44.76 B
ATOM 10984 C ASP B 666 104.107 93.523 112.107 1.00 33.60 B
ATOM 10985 O ASP B 666 103.889 92.920 113.157 1.00 32.81 B
ATOM 10986 N SER B 667 103.142 94.028 111.353 1.00 33.83 B
ATOM 10987 CA SER B 667 101.744 93.892 111.714 1.00 35.00 B
ATOM 10988 CB SER B 667 101.083 95.269 111.724 1.00 36.24 B
ATOM 10989 OG SER B 667 99.702 95.166 112.000 1.00 41.48 B
ATOM 10990 C SER B 667 101.068 92.985 110.695 1.00 35.61 B
ATOM 10991 O SER B 667 101.369 93.060 109.500 1.0036.38 B
ATOM 10992 N VAL B 668 100.171 92.119 111.162 1.0034.84 B
ATOM 10993 CA VAL B 668 99.464 91.204 110.267 1.0034.94 B
ATOM 10994 CB VAL B 668 100.196 89.841 110.150 1.00 35.18 B
ATOM 10995 CG1 VAL B 668 101.580 90.037 109.550 1.00 35.15 B
ATOM 10996 CG2 VAL B 668 100.306 89.187 111.517 1.00 33.55 B
ATOM 10997 C VAL B 668 98.039 90.932 110.731 1.00 35.11 B
ATOM 10998 O VAL B 668 97.665 91.263 111.856 1.00 35.69 B
ATOM 10999 N GLU B 669 97.239 90.342 109.850 1.00 34.61 B
ATOM 11000 CA GLU B 669 95.865 89.995 110.189 1.00 35.52 B
ATOM 11001 CB GLU B 669 94.899 90.472 109.101 1.00 37.50 B
ATOM 11002 CG GLU B 669 94.651 91.979 109.102 1.0041.35 B
ATOM 11003 CD GLU B 669 93.607 92.413 110.121 1.00 43.99 B
ATOM 11004 OE1 GLU B 669 93.603 93.606 110.496 1.0044.52 B
ATOM 11005 OE2 GLU B 669 92.780 91.570 110.538 1.00 46.11 B
ATOM 11006 C GLU B 669 95.808 88.478 110.313 1.00 34.84 B
ATOM 11007 O GLU B 669 96.407 87.759 109.512 1.0036.01 B
ATOM 11008 N TRP B 670 95.106 87.990 111.327 1.00 32.61 B
ATOM 11009 CA TRP B 670 94.989 86.555 111.540 1.00 30.40 B
ATOM 11010 CB TRP B 670 95.537 86.187 112.921 1.00 28.53 B
ATOM 11011 CG TRP B 670 95.599 84.715 113.151 1.00 28.16 B
ATOM 11012 CD2 TRP B 670 94.711 83.936 113.956 1.00 26.72 B
ATOM 11013 CE2 TRP B 670 95.120 82.590 113.846 1.00 27.14 B
ATOM 11014 CE3 TRP B 670 93.606 84.244 114.760 1.00 26.36 B
ATOM 11015 CD1 TRP B 670 96.489 83.836 112.600 1.00 27.36 B
ATOM 11016 NE1 TRP B 670 96.207 82.557 113.012 1.00 27.94 B
ATOM 11017 CZ2 TRP B 670 94.462 81.551 114.511 1.00 25.40 B
ATOM 11018 CZ3 TRP B 670 92.953 83.212 115.421 1.00 26.30 B
ATOM 11019 CH2 TRP B 670 93.384 81.882 115.291 1.00 26.41 B
ATOM 11020 C TRP B 670 93.528 86.120 111.432 1.00 29.91 B
ATOM 11021 O TRP B 670 92.648 86.731 112.035 1.00 30.31 B
ATOM 11022 N ARG B 671 93.276 85.065 110.662 1.00 29.68 B
ATOM 11023 CA ARG B 671 91.921 84.551 110.479 1.00 29.99 B
ATOM 11024 CB ARG B 671 91.796 83.849 109.118 1.00 32.19 B
ATOM 11025 CG ARG B 671 91.094 84.661 108.040 1.0037.03 B
ATOM 11026 CD ARG B 671 89.685 84.137 107.735 1.00 37.27 B
ATOM 11027 NE ARG B 671 88.850 84.048 108.930 1.00 39.22 B
ATOM 11028 CZ ARG B 671 87.530 83.875 108.918 1.00 37.65 B
ATOM 11029 NH1 ARG B 671 86.875 83.774 107.770 1.00 37.83 B
ATOM 11030 NH2 ARG B 671 86.861 83.792 110.062 1.00 36.70 B
ATOM 11031 C ARG B 671 91.518 83.568 111.573 1.00 28.02 B
ATOM 11032 O ARG B 671 92.169 82.538 111.758 1.00 25.98 B
ATOM 11033 N LEU B 672 90.448 83.887 112.298 1.0027.14 B
ATOM 11034 CA LEU B 672 89.955 82.992 113.337 1.00 26.63 B
ATOM 11035 CB LEU B 672 88.857 83.670 114.162 1.00 25.52 B
ATOM 11036 CG LEU B 672 89.309 84.609 115.288 1.0027.00 B
ATOM 11037 CD1 LEU B 672 90.096 85.782 114.717 1.00 26.67 B
ATOM 11038 CD2 LEU B 672 88.089 85.103 116.052 1.00 26.92 B
ATOM 11039 C LEU B 672 89.398 81.754 112.629 1.0027.52 B
ATOM 11040 O LEU B 672 89.021 81.827 111.458 1.00 26.97 B
ATOM 11041 N PRO B 673 89.340 80.605 113.326 1.0027.03 B
ATOM 11042 CD PRO B 673 89.798 80.386 114.712 1.00 26.63 B
ATOM 11043 CA PRO B 673 88.833 79.355 112.747 1.00 27.33 B
ATOM 11044 CB PRO B 673 89.321 78.303 113.738 1.0025.83 B
ATOM 11045 CG PRO B 673 89.215 79.022 115.038 1.00 25.80 B
ATOM 11046 C PRO B 673 87.319 79.278 112.512 1.00 28.52 B
ATOM 11047 O PRO B 673 86.818 78.257 112.053 1.00 28.51 B
ATOM 11048 N ASN B 674 86.596 80.345 112.834 1.00 29.81 B
ATOM 11049 CA ASN B 674 85.151 80.378 112.628 1.00 30.67 B
ATOM 11050 CB ASN B 674 84.423 79.635 113.757 1.00 30.94 B
ATOM 11051 CG ASN B 674 84.675 80.243 115.130 1.00 33.37 B
ATOM 11052 OD1 ASN B 674 84.370 81.410115.374 1.00 31.50 B
ATOM 11053 ND2 ASN B 674 85.228 79.442 116.038 1.00 32.19 B
ATOM 11054 C ASN B 674 84.671 81.824 112.539 1.00 32.28 B
ATOM 11055 O ASN B 674 85.485 82.745 112.482 1.0034.16 B
ATOM 11056 N ASP B 675 83.358 82.031 112.522 1.0031.88 B
ATOM 11057 CA ASP B 675 82.817 83.383 112.421 1.00 32.08 B
ATOM 11058 CB ASP B 675 81.885 83.482 111.210 1.00 32.66 B
ATOM 11059 CG ASP B 675 82.612 83.242 109.892 1.00 35.42 B
ATOM 11060 OD1 ASP B 675 81.949 83.228 108.836 1.00 37.30 B
ATOM 11061 OD2 ASP B 675 83.849 83.069 109.907 1.00 36.81 B
ATOM 11062 C ASP B 675 82.090 83.835 113.683 1.00 32.09 B
ATOM 11063 O ASP B 675 81.394 84.848 113.684 1.00 32.20 B
ATOM 11064 N ILE B 676 82.258 83.087 114.763 1.00 32.22 B
ATOM 11065 CA ILE B 676 81.625 83.436 116.024 1.00 33.67 B
ATOM 11066 CB ILE B 676 81.671 82.255 117.008 1.00 33.60 B
ATOM 11067 CG2 ILE B 676 81.155 82.686 118.377 1.00 32.76 B
ATOM 11068 CG1 ILE B 676 80.843 81.096 116.452 1.00 35.07 B
ATOM 11069 CD1 ILE B 676 80.897 79.834 117.302 1.00 34.36 B
ATOM 11070 C ILE B 676 82.344 84.631 116.650 1.00 34.45 B
ATOM 11071 O ILE B 676 83.552 84.801 116.482 1.00 34.03 B
ATOM 11072 N PRO B 677 81.599 85.497 117.353 1.00 34.40 B
ATOM 11073 CD PRO B 677 80.133 85.632 117.289 1.00 34.03 B
ATOM 11074 CA PRO B 677 82.200 86.672 117.996 1.00 33.92 B
ATOM 11075 CB PRO B 677 80.983 87.529 118.353 1.0034.48 B
ATOM 11076 CG PRO B 677 79.961 87.126 117.326 1.0034.54 B
ATOM 11077 C PRO B 677 83.017 86.289 119.238 1.00 32.12 B
ATOM 11078 O PRO B 677 82.536 85.553 120.101 1.00 29.46 B
ATOM 11079 N TYR B 678 84.250 86.789 119.316 1.00 31.90 B
ATOM 11080 CA TYR B 678 85.134 86.515 120.450 1.00 31.13 B
ATOM 11081 CB TYR B 678 86.454 85.893 119.977 1.0031.37 B
ATOM 11082 CG TYR B 678 86.392 84.416 119.676 1.00 32.14 B
ATOM 11083 CD1 TYR B 678 85.720 83.940 118.551 1.00 31.36 B
ATOM 11084 CE1 TYR B 678 85.654 82.578 118.275 1.00 32.57 B
ATOM 11085 CD2 TYR B 678 87.001 83.490 120.524 1.0032.23 B
ATOM 11086 CE2 TYR B 678 86.942 82.126 120.260 1.00 33.81 B
ATOM 11087 CZ TYR B 678 86.265 81.675 119.132 1.00 33.98 B
ATOM 11088 OH TYR B 678 86.198 80.328 118.863 1.00 33.57 B
ATOM 11089 C TYR B 678 85.464 87.783 121.227 1.00 30.84 B
ATOM 11090 O TYR B 678 85.209 88.894 120.766 1.00 30.90 B
ATOM 11091 N ARG B 679 86.045 87.600 122.408 1.0031.48 B
ATOM 11092 CA ARG B 679 86.455 88.712 123.259 1.00 31.48 B
ATOM 11093 CB ARG B 679 86.081 88.433 124.716 1.00 33.78 B
ATOM 11094 CG ARG B 679 84.601 88.525 125.015 1.00 37.27 B
ATOM 11095 CD ARG B 679 84.116 89.946 124.840 1.00 40.19 B
ATOM 11096 NE ARG B 679 82.729 90.102 125.257 1.00 44.94 B
ATOM 11097 CZ ARG B 679 82.070 91.257 125.240 1.00 47.97 B
ATOM 11098 NH1 ARG B 679 80.805 91.306 125.640 1.00 48.68 B
ATOM 11099 NH2 ARG B 679 82.674 92.366 124.822 1.00 47.59 B
ATOM 11100 C ARG B 679 87.968 88.902 123.153 1.00 30.99 B
ATOM 11101 O ARG B 679 88.729 87.942 123.297 1.0029.85 B
ATOM 11102 N LEU B 680 88.399 90.133 122.885 1.00 30.56 B
ATOM 11103 CA LEU B 680 89.822 90.436 122.777 1.00 30.01 B
ATOM 11104 CB LEU B 680 90.043 91.615 121.826 1.00 31.68 B
ATOM 11105 CG LEU B 680 91.479 91.990 121.426 1.0034.12 B
ATOM 11106 CD1 LEU B 680 92.234 92.547 122.622 1.00 36.36 B
ATOM 11107 CD2 LEU B 680 92.196 90.768 120.870 1.00 33.20 B
ATOM 11108 C LEU B 680 90.301 90.787 124.180 1.00 30.17 B
ATOM 11109 O LEU B 680 90.182 91.933 124.621 1.0030.36 B
ATOM 11110 N LEU B 681 90.840 89.793 124.878 1.00 28.39 B
ATOM 11111 CA LEU B 681 91.305 89.975 126.250 1.00 26.97 B
ATOM 11112 CB LEU B 681 91.338 88.618 126.960 1.00 25.79 B
ATOM 11113 CG LEU B 681 91.364 88.471 128.488 1.0027.64 B
ATOM 11114 CD1 LEU B 681 92.463 87.483 128.837 1.00 26.56 B
ATOM 11115 CD2 LEU B 681 91.584 89.795 129.198 1.00 26.93 B
ATOM 11116 C LEU B 681 92.685 90.611 126.346 1.00 25.81 B
ATOM 11117 O LEU B 681 92.960 91.372 127.269 1.00 25.07 B
ATOM 11118 N CYS B 682 93.545 90.314 125.380 1.0025.43 B
ATOM 11119 CA CYS B 682 94.909 90.812 125.421 1.00 25.10 B
ATOM 11120 CB CYS B 682 95.707 89.882 126.346 1.00 24.64 B
ATOM 11121 SG CYS B 682 97.494 90.053 126.365 1.0027.46 B
ATOM 11122 C CYS B 682 95.556 90.886 124.039 1.00 25.59 B
ATOM 11123 O CYS B 682 95.276 90.065 123.170 1.0025.69 B
ATOM 11124 N ASP B 683 96.414 91.882 123.840 1.0025.17 B
ATOM 11125 CA ASP B 683 97.127 92.052 122.573 1.00 27.19 B
ATOM 11126 CB ASP B 683 96.390 93.065 121.666 1.00 28.75 B
ATOM 11127 CG ASP B 683 96.351 94.479 122.244 1.00 30.31 B
ATOM 11128 OD1 ASP B 683 95.545 95.292 121.747 1.00 30.60 B
ATOM 11129 OD2 ASP B 683 97.121 94.789 123.177 1.00 32.25 B
ATOM 11130 C ASP B 683 98.561 92.511 122.878 1.00 26.22 B
ATOM 11131 O ASP B 683 98.927 92.657 124.040 1.00 25.81 B
ATOM 11132 N PRO B 684 99.394 92.731 121.846 1.00 26.39 B
ATOM 11133 CD PRO B 684 99.190 92.525 120.399 1.00 26.09 B
ATOM 11134 CA PRO B 684 100.767 93.168 122.119 1.00 26.01 B
ATOM 11135 CB PRO B 684 101.293 93.512 120.733 1.00 26.71 B
ATOM 11136 CG PRO B 684 100.610 92.496 119.874 1.00 26.94 B
ATOM 11137 C PRO B 684 100.891 94.342 123.095 1.00 28.15 B
ATOM 11138 O PRO B 684 101.945 94.542 123.698 1.00 27.73 B
ATOM 11139 N SER B 685 99.811 95.106 123.249 1.00 29.75 B
ATOM 11140 CA SER B 685 99.789 96.271 124.139 1.00 31.66 B
ATOM 11141 CB SER B 685 98.668 97.237 123.727 1.00 32.22 B
ATOM 11142 OG SER B 685 98.790 97.622 122.369 1.00 40.12 B
ATOM 11143 C SER B 685 99.572 95.884 125.595 1.00 31.20 B
ATOM 11144 O SER B 685 99.849 96.669 126.503 1.00 31.08 B
ATOM 11145 N GLY B 686 99.066 94.676 125.810 1.00 30.31 B
ATOM 11146 CA GLY B 686 98.797 94.216 127.156 1.00 30.19 B
ATOM 11147 C GLY B 686 97.339 93.820 127.272 1.0030.73 B
ATOM 11148 O GLY B 686 96.619 93.801 126.274 1.0029.82 B
ATOM 11149 N PHE B 687 96.901 93.502 128.486 1.00 1.14 B
ATOM 11150 CA PHE B 687 95.520 93.105 128.716 1.00 32.76 B
ATOM 11151 CB PHE B 687 95.370 92.481 130.107 1.00 31.55 B
ATOM 11152 CG PHE B 687 95.998 91.121 130.231 1.00 31.35 B
ATOM 11153 CD1 PHE B 687 97.380 90.969 130.206 1.00 30.58 B
ATOM 11154 CD2 PHE B 687 95.203 89.983 130.347 1.00 32.32 B
ATOM 11155 CE1 PHE B 687 97.963 89.703 130.291 1.00 30.19 B
ATOM 11156 CE2 PHE B 687 95.776 88.714 130.432 1.0031.38 B
ATOM 11157 CZ PHE B 687 97.160 88.576 130.403 1.00 30.91 B
ATOM 11158 C PHE B 687 94.555 94.277 128.572 1.00 35.25 B
ATOM 11159 O PHE B 687 94.892 95.424 128.874 1.0035.69 B
ATOM 11160 N GLN B 688 93.349 93.973 128.109 1.00 37.13 B
ATOM 11161 CA GLN B 688 92.316 94.979 127.915 1.00 39.25 B
ATOM 11162 CB GLN B 688 91.481 94.630 126.689 1.00 38.70 B
ATOM 11163 CG GLN B 688 92.317 94.410 125.448 1.0039.83 B
ATOM 11164 CD GLN B 688 93.180 95.606 125.122 1.00 39.50 B
ATOM 11165 OE1 GLN B 688 92.675 96.712 124.944 1.00 41.84 B
ATOM 11166 NE2 GLN B 688 94.487 95.393 125.043 1.00 38.74 B
ATOM 11167 C GLN B 688 91.428 95.033 129.143 1.0041.20 B
ATOM 11168 O GLN B 688 90.864 94.021 129.550 1.0040.64 B
ATOM 11169 N GLU B 689 91.303 96.220 129.723 1.0045.05 B
ATOM 11170 CA GLU B 689 90.495 96.421 130.920 1.00 49.58 B
ATOM 11171 CB GLU B 689 90.499 97.903 131.292 1.00 52.73 B
ATOM 11172 CG GLU B 689 91.898 98.468 131.491 1.00 57.91 B
ATOM 11173 CD GLU B 689 91.883 99.928 131.895 1.00 61.09 B
ATOM 11174 OE1 GLU B 689 91.237 100.251 132.917 1.00 62.85 B
ATOM 11175 OE2 GLU B 689 92.517 100.748 131.194 1.00 62.04 B
ATOM 11176 C GLU B 689 89.058 95.921 130.774 1.00 50.82 B
ATOM 11177 O GLU B 689 88.424 95.516 131.753 1.00 51.38 B
ATOM 11178 N ASP B 690 88.545 95.952 129.550 1.0050.38 B
ATOM 11179 CA ASP B 690 87.189 95.495 129.282 1.00 51.54 B
ATOM 11180 CB ASP B 690 86.194 96.634 129.518 1.00 53.51 B
ATOM 11181 CG ASP B 690 86.753 97.984 129.121 1.00 56.37 B
ATOM 11182 OE1 ASP B 690 86.146 99.011 129.494 1.00 58.44 B
ATOM 11183 OD2 ASP B 690 87.799 98.020 128.436 1.00 59.01 B
ATOM 11184 C ASP B 690 87.095 94.971 127.853 1.00 50.31 B
ATOM 11185 O ASP B 690 86.742 95.701 126.924 1.00 50.58 B
ATOM 11186 N PRO B 691 87.420 93.683 127.668 1.00 48.44 B
ATOM 11187 CD PRO B 691 87.744 92.728 128.744 1.00 47.52 B
ATOM 11188 CA PRO B 691 87.397 93.005 126.374 1.00 47.21 B
ATOM 11189 CB PRO B 691 87.399 91.533 126.767 1.00 46.73 B
ATOM 11190 CG PRO B 691 88.268 91.535 127.973 1.00 47.11 B
ATOM 11191 C PRO B 691 86.215 93.368 125.480 1.00 46.36 B
ATOM 11192 O PRO B 691 85.057 93.318 125.897 1.00 45.35 B
ATOM 11193 N THR B 692 86.535 93.750 124.250 1.00 45.80 B
ATOM 11194 CA THR B 692 85.535 94.097 123.253 1.00 45.74 B
ATOM 11195 CB THR B 692 85.957 95.331 122.444 1.00 45.45 B
ATOM 11196 OG1 THR B 692 87.254 95.106 121.878 1.00 46.18 B
ATOM 11197 CG2 THR B 692 86.000 96.564 123.334 1.00 44.43 B
ATOM 11198 C THR B 692 85.464 92.891 122.323 1.00 45.79 B
ATOM 11199 O THR B 692 86.376 92.063 122.306 1.00 45.79 B
ATOM 11200 N GLU B 693 84.393 92.785 121.548 1.00 45.36 B
ATOM 11201 CA GLU B 693 84.252 91.653 120.649 1.00 44.20 B
ATOM 11202 CB GLU B 693 82.785 91.244 120.543 1.0046.63 B
ATOM 11203 CG GLU B 693 82.235 90.615 121.814 1.00 49.32 B
ATOM 11204 CD GLU B 693 80.812 90.11 121.650 1.00 51.13 B
ATOM 11205 OE1 GLU B 693 80.296 89.479 122.592 1.00 53.01 B
ATOM 11206 OE2 GLU B 693 80.211 90.366 120.581 1.00 51.60 B
ATOM 11207 C GLU B 693 84.814 91.909 119.264 1.00 42.80 B
ATOM 11208 O GLU B 693 84.769 93.029 118.761 1.00 43.04 B
ATOM 11209 N ILE B 694 85.365 90.859 118.665 1.00 40.43 B
ATOM 11210 CA ILE B 694 85.922 90.937 117.322 1.00 38.55 B
ATOM 11211 CB ILE B 694 87.473 91.022 117.352 1.00 38.40 B
ATOM 11212 CG2 ILE B 694 87.902 92.353 117.958 1.00 37.47 B
ATOM 11213 CG1 ILE B 694 88.065 89.862 118.153 1.00 36.74 B
ATOM 11214 CD1 ILE B 694 88.143 88.562 117.391 1.00 37.62 B
ATOM 11215 C ILE B 694 85.457 89.713 116.538 1.00 37.84 B
ATOM 11216 O ILE B 694 85.215 88.649 117.116 1.00 37.57 B
ATOM 11217 N LYS B 695 85.321 89.868 115.225 1.00 37.75 B
ATOM 11218 CA LYS B 695 84.848 88.782 114.376 1.00 36.83 B
ATOM 11219 CB LYS B 695 83.475 89.130 113.784 1.0038.38 B
ATOM 11220 CG LYS B 695 82.404 89.481 114.795 1.0040.60 B
ATOM 11221 CD LYS B 695 81.062 89.715 114.110 1.00 42.87 B
ATOM 11222 CE LYS B 695 79.973 90.069 115.122 1.00 44.98 B
ATOM 11223 NZ LYS B 695 78.618 90.206 114.501 1.00 45.01 B
ATOM 11224 C LYS B 695 85.774 88.439 113.224 1.00 35.26 B
ATOM 11225 O LYS B 695 86.511 89.284 112.727 1.00 34.54 B
ATOM 11226 N LYS B 696 85.709 87.178 112.811 1.00 34.87 B
ATOM 11227 CA LYS B 696 86.466 86.657 111.680 1.00 35.35 B
ATOM 11228 CB LYS B 696 85.875 87.219 110.379 1.0036.15 B
ATOM 11229 CG LYS B 696 84.361 87.056 110.274 1.0038.29 B
ATOM 11230 CD LYS B 696 83.849 87.324 108.864 1.0041.79 B
ATOM 11231 CE LYS B 696 84.104 88.756 108.426 1.0044.68 B
ATOM 11232 NZ LYS B 696 83.650 88.992 107.022 1.0046.28 B
ATOM 11233 C LYS B 696 87.976 86.860 111.679 1.00 34.96 B
ATOM 11234 O LYS B 696 88.733 85.897 111.548 1.00 35.61 B
ATOM 11235 N THR B 697 88.421 88.104 111.797 1.00 33.26 B
ATOM 11236 CA THR B 697 89.847 88.381 111.787 1.00 32.51 B
ATOM 11237 CB THR B 697 90.275 89.079 110.482 1.00 32.64 B
ATOM 11238 OG1 THR B 697 89.612 90.345 110.386 1.00 34.64 B
ATOM 11239 CG2 THR B 697 89.909 88.233 109.273 1.00 31.62 B
ATOM 11240 C THR B 697 90.262 89.266112.950 1.00 32.35 B
ATOM 11241 O THR B 697 89.436 89.932 113.574 1.00 33.23 B
ATOM 11242 N VAL B 698 91.557 89.266 113.238 1.00 31.13 B
ATOM 11243 CA VAL B 698 92.091 90.080 114.316 1.00 29.89 B
ATOM 11244 CB VAL B 698 92.175 89.280 115.638 1.00 29.30 B
ATOM 11245 CG1 VAL B 698 93.100 88.083 115.475 1.00 27.88 B
ATOM 11246 CG2 VAL B 698 92.651 90.187 116.758 1.00 29.31 B
ATOM 11247 C VAL B 698 93.475 90.568 113.915 1.00 29.26 B
ATOM 11248 O VAL B 698 94.256 89.830 113.315 1.00 28.91 B
ATOM 11249 N ALA B 699 93.769 91.821 114.229 1.00 29.53 B
ATOM 11250 CA ALA B 699 95.065 92.389 113.892 1.00 30.13 B
ATOM 11251 CB ALA B 699 94.955 93.903 113.812 1.00 29.71 B
ATOM 11252 C ALA B 699 96.124 91.994 114.921 1.00 29.69 B
ATOM 11253 O ALA B 699 95.890 92.074 116.123 1.0031.74 B
ATOM 11254 N VAL B 700 97.280 91.552 114.444 1.0029.04 B
ATOM 11255 CA VAL B 700 98.378 91.177 115.326 1.00 28.97 B
ATOM 11256 CB VAL B 700 98.854 89.731 115.068 1.00 30.28 B
ATOM 11257 CG1 VAL B 700 100.019 89.395 115.990 1.00 27.86 B
ATOM 11258 CG2 VAL B 700 97.701 88.754 115.295 1.00 27.78 B
ATOM 11259 C VAL B 700 99.509 92.148 115.013 1.00 29.91 B
ATOM 11260 O VAL B 700 100.321 91.905 114.116 1.00 28.43 B
ATOM 11261 N ASN B 701 99.547 93.250 115.759 1.00 28.79 B
ATOM 11262 CA ASN B 701 100.546 94.286 115.547 1.00 29.23 B
ATOM 11263 CB ASN B 701 99.932 95.669 115.810 1.0031.88 B
ATOM 11264 CG ASN B 701 98.606 95.878 115.084 1.0035.02 B
ATOM 11265 OD1 ASN B 701 98.508 95.683 113.871 1.00 35.18 B
ATOM 11266 ND2 ASN B 701 97.580 96.284 115.830 1.00 34.12 B
ATOM 11267 C ASN B 701 101.808 94.146 116.394 1.00 28.64 B
ATOM 11268 O ASN B 701 101.787 94.395 117.600 1.0027.32 B
ATOM 11269 N GLY B 702 102.904 93.756 115.752 1.0026.82 B
ATOM 11270 CA GLY B 702 104.171 93.635 116.451 1.00 25.66 B
ATOM 11271 C GLY B 702 104.321 92.519 117.469 1.00 26.48 B
ATOM 11272 O GLY B 702 103.434 91.680 117.643 1.00 25.51 B
ATOM 11273 N ILE B 703 105.462 92.534 118.154 1.00 23.51 B
ATOM 11274 CA ILE B 703 105.798 91.529 119.148 1.00 21.98 B
ATOM 11275 CB ILE B 703 107.248 91.697 119.600 1.00 22.80 B
ATOM 11276 CG2 ILE B 703 107.638 90.551 120.530 1.00 21.25 B
ATOM 11277 CG1 ILE B 703 108.157 91.751 118.366 1.00 23.14 B
ATOM 11278 CD1 ILE B 703 109.608 92.153 118.656 1.00 23.01 B
ATOM 11279 C ILE B 703 104.892 91.539 120.373 1.00 22.41 B
ATOM 11280 O ILE B 703 104.752 92.556 121.065 1.00 20.54 B
ATOM 11281 N GLY B 704 104.279 90.389 120.637 1.00 20.48 B
ATOM 11282 CA GLY B 704 103.390 90.277 121.771 1.00 20.02 B
ATOM 11283 C GLY B 704 102.472 89.079 121.661 1.00 21.00 B
ATOM 11284 O GLY B 704 102.652 88.210 120.804 1.0020.42 B
ATOM 11285 N THR B 705 101.473 89.041 122.531 1.00 20.55 B
ATOM 11286 CA THR B 705 100.529 87.940 122.553 1.00 19.93 B
ATOM 11287 CB THR B 705 100.681 87.113 123.858 1.00 18.53 B
ATOM 11288 OG1 THR B 705 102.010 86.584 123.941 1.00 13.74 B
ATOM 11289 CG2 THR B 705 99.672 85.972 123.897 1.00 13.96 B
ATOM 11290 C THR B 705 99.097 88.447 122.468 1.00 22.24 B
ATOM 11291 O THR B 705 98.737 89.431 123.109 1.0023.37 B
ATOM 11292 N VAL B 706 98.286 87.777 121.659 1.00 23.19 B
ATOM 11293 CA VAL B 706 96.884 88.135 121.533 1.00 23.83 B
ATOM 11294 CB VAL B 706 96.456 88.313 120.047 1.00 25.16 B
ATOM 11295 CG1 VAL B 706 94.938 88.433 119.952 1.00 22.76 B
ATOM 11296 CG2 VAL B 706 97.117 89.556 119.451 1.00 23.48 B
ATOM 11297 C VAL B 706 96.119 86.967 122.138 1.0024.42 B
ATOM 11298 O VAL B 706 96.419 85.807 121.849 1.0024.89 B
ATOM 11299 N ILE B 707 95.151 87.276 122.991 1.00 23.86 B
ATOM 11300 CA ILE B 707 94.337 86.252 123.629 1.00 23.84 B
ATOM 11301 CB ILE B 707 94.567 86.209 125.154 1.0022.96 B
ATOM 11302 CG2 ILE B 707 93.784 85.054 125.758 1.00 19.89 B
ATOM 11303 CG1 ILE B 707 96.059 86.048 125.458 1.00 20.96 B
ATOM 11304 CD1 ILE B 707 96.378 86.013 126.933 1.00 20.22 B
ATOM 11305 C ILE B 707 92.868 86.571 123.371 1.00 25.91 B
ATOM 11306 O ILE B 707 92.403 87.672 123.668 1.00 26.53 B
ATOM 11307 N LEU B 708 92.153 85.599 122.810 1.00 25.84 B
ATOM 11308 CA LEU B 708 90.737 85.741 122.491 1.00 26.92 B
ATOM 11309 CB LEU B 708 90.517 85.541 120.986 1.00 25.51 B
ATOM 11310 CG LEU B 708 91.309 86.461 120.054 1.00 25.47 B
ATOM 11311 CD1 LEU B 708 91.248 85.950 118.616 1.00 23.51 B
ATOM 11312 CD2 LEU B 708 90.742 87.865 120.156 1.00 25.37 B
ATOM 11313 C LEU B 708 89.972 84.672 123.252 1.00 27.40 B
ATOM 11314 O LEU B 708 90.515 83.604 123.528 1.00 28.11 B
ATOM 11315 N TYR B 709 88.715 84.939 123.588 1.0028.56 B
ATOM 11316 CA TYR B 709 87.942 83.938 124.308 1.0029.78 B
ATOM 11317 CB TYR B 709 88.255 84.004 125.808 1.0029.82 B
ATOM 11318 CG TYR B 709 87.677 85.196 126.535 1.0031.99 B
ATOM 11319 CD1 TYR B 709 86.328 85.235 126.891 1.00 32.25 B
ATOM 11320 CE1 TYR B 709 85.793 86.330 127.572 1.0032.30 B
ATOM 11321 CD2 TYR B 709 88.479 86.284 126.879 1.00 31.63 B
ATOM 11322 CE2 TYR B 709 87.955 87.380 127.558 1.00 31.22 B
ATOM 11323 CZ TYR B 709 86.614 87.397 127.901 1.00 30.86 B
ATOM 11324 OH TYR B 709 86.091 88.483 128.559 1.00 28.92 B
ATOM 11325 C TYR B 709 86.438 84.012 124.083 1.00 30.62 B
ATOM 11326 O TYR B 709 85.881 85.064 123.768 1.00 30.00 B

ATOM 11327 N LEU B 710 85.796 82.863 124.253 1.00 31.98 B
ATOM 11328 CA LEU B 710 84.361 82.721 124.091 1.00 33.08 B
ATOM 11329 CB LEU B 710 84.074 81.601 123.093 1.00 34.18 B
ATOM 11330 CG LEU B 710 82.972 81.796 122.059 1.00 36.16 B
ATOM 11331 CD1 LEU B 710 82.789 80.484 121.300 1.00 34.34 B
ATOM 11332 CD2 LEU B 710 81.673 82.221 122.733 1.00 35.34 B
ATOM 11333 C LEU B 710 83.830 82.338 125.466 1.00 34.29 B
ATOM 11334 O LEU B 710 84.222 81.313 126.016 1.00 34.07 B
ATOM 11335 N ALA B 711 82.945 83.159 126.020 1.00 37.51 B
ATOM 11336 CA ALA B 711 82.385 82.898 127.343 1.00 42.59 B
ATOM 11337 CB ALA B 711 81.586 84.103 127.809 1.00 41.42 B
ATOM 11338 C ALA B 711 81.519 81.640 127.413 1.00 45.88 B
ATOM 11339 O ALA B 711 81.433 80.873 126.454 1.0045.88 B
ATOM 11340 N SER B 712 80.879 81.448 128.56 1.00 50.14 B
ATOM 11341 CA SERB 712 80.00 680.300 128.831 1.00 53.27 B
ATOM 11342 CB SER B 712 79.130 80.590 130.053 1.00 3.79 B
ATOM 11343 OG SER B 712 78.238 81.665 129.790 1.0054.66 B
ATOM 11344 C SER B 712 79.097 79.908 127.667 1.00 54.86 B
ATOM 11345 O SER B 712 79.006 80.676 126.685 1.0057.08 B
ATOM 11346 OXT SER B 712 78.465 78.834 127.766 1.00 55.60 B
ATOM 11347 OH2 TIP 803 118.852 71.154 161.402 1.00 14.77
ATOM 11348 OH2 TIP 804 84.943 49.527 79.691 1.00 10.01
ATOM 11349 OH2 TIP 805 106.333 71.713 128.047 1.00 9.41
ATOM 11350 OH2 TIP 806 126.139 65.782 156.353 1.00 13.20
ATOM 11351 OH2 TIP 807 119.424 81.809 132.737 1.00 12.31
ATOM 11352 OH2 TIP 808 128.040 76.851 159.629 1.00 14.09
ATOM 11353 OH2 TIP 809 81.743 58.335 83.541 1.00 13.29
ATOM 11354 OH2 TIP 810 90.489 51.655 84.068 1.00 11.31
ATOM 11355 OH2TIP 811 101.865 69.376149.139 1.0016.42
ATOM 11356 OH2 TIP 812 70.448 55.001 117.389 1.0011.74
ATOM 11357 OH2 TIP 813 85.224 58.104 94.870 1.00 8.58
ATOM 11358 OH2 TIP 814 92.618 51.732 86.626 1.00 18.75
ATOM 11359 OH2 TIP 815 84.584 48.343 94.636 1.00 12.93
ATOM 11360 OH2 TIP 816 93.217 42.003 103.038 1.00 18.10
ATOM 11361 OH2 TIP 817 101.234 90.815 124.739 1.00 20.36
ATOM 11362 OH2 TIP 818 70.710 62.146 104.385 1.0014.78
ATOM 11363 OH2 TIP 819 129.063 56.484 157.598 1.00 13.12
ATOM 11364 OH2 TIP 820 157.523 38.184 148.981 1.00 18.37
ATOM 11365 OH2 TIP 821 118.222 85.404 143.255 1.00 12.66
ATOM 11366 OH2TIP 822 100.231 76.650 137.388 1.00 17.60
ATOM 11367 OH2 TIP 823 98.002 69.112 94.082 1.00 14.33
ATOM 11368 OH2 TIP 824 119.681 72.800 150.861 1.00 13.71
ATOM 11369 OH2 TIP 825 61.847 47.697 106.198 1.00 17.54
ATOM 11370 OH2 TIP 826 120.711 66.799 144.479 1.00 11.80
ATOM 11371 OH2 TIP 827 91.091 48.039 88.943 1.00 16.31
ATOM 11372 OH2 TIP 828 119.277 80.728 149.153 1.00 9.93
ATOM 11373 OH2 TIP 829 149.226 47.102 156.066 1.00 13.27
ATOM 11374 OH2 TIP 830 127.970 77.621 144.525 1.00 13.75
ATOM 11375 OH2 TIP 831 70.785 39.005 89.917 1.00 16.12
ATOM 11376 OH2 TIP 832 141.912 50.529 162.168 1.00 19.41
ATOM 11377 OH2 TIP 833 117.396 75.134 149.826 1.00 13.05
ATOM 111378 OH2 TIP 834 87.992 54.757 94.562 1.00 11.77
ATOM 11379 OH2 TIP 835 93.336 71.362 88.416 1.00 14.80
ATOM 11380 OH2 TIP 836 85.955 53.992 80.246 1.00 14.76
ATOM 11381 OH2 TIP 837 121.128 83.773 135.470 1.00 10.84
ATOM 11382 OH2 TIP 838 88.025 61.030 86.289 1.00 15.17
ATOM 11383 OH2 TIP 839 90.148 69.619 83.351 1.00 13.54
ATOM 11384 OH2 TIP 840 123.656 75.493 163.979 1.00 16.00
ATOM 11385 OH2 TIP 841 123.486 86.962 153.306 1.00 16.25
ATOM 11386 OH2 TIP 842 84.465 66.349 68.754 1.00 14.93
ATOM 11387 OH2 TIP 843 88.348 71.545 90.011 1.00 10.04
ATOM 11388 OH2 TIP 844 117.119 76.701 143.051 1.00 17.45
ATOM 11389 OH2 TIP 845 124.912 72.852 149.767 1.00 11.61
ATOM 11390 OH2 TIP 846 117.652 92.646 146.942 1.00 18.82
ATOM 11391 OH2 TIP 847 130.954 84.040 139.725 1.00 14.02
ATOM 11392 OH2 TIP 848 110.224 83.752 115.625 1.00 20.30
ATOM 11393 OH2 TIP 849 90.974 71.085 85.737 1.00 12.30
ATOM 11394 OH2 TIP 850 121.939 79.669 149.031 1.00 9.19
ATOM 11395 OH2 TIP 851 97.932 60.721 129.633 1.00 20.31
ATOM 11396 OH2 TIP 852 124.630 82.770 36.428 1.00 11.18
ATOM 11397 OH2 TIP 853 90.144 55.259 101.997 1.0021.63
ATOM 11398 OH2 TIP 854 109.873 88.894 131.961 1.00 18.96
ATOM 11399 OH2 TIP 855 61.290 42.316 104.087 1.00 19.31
ATOM 11400 OH2 TIP 856 99.508 74.683 87.801 1.0015.86
ATOM 11401 OH2 TIP 857 132.492 82.309 147.355 1.00 20.55
ATOM 11402 OH2 TIP 858 135.511 47.598 167.013 1.00 17.22
ATOM 11403 OH2 TIP 859 129.020 66.284 165.264 1.00 15.58
ATOM 11404 OH2 TIP 860 92.347 62.550 79.787 1.00 14.49
ATOM 11405 OH2 TIP 861 129.175 45.305 163.354 1.00 22.19
ATOM 11406 OH2 TIP 862 83.448 71.342 86.007 1.00 17.07
ATOM 11407 OH2 TIP 863 160.616 32.469 155.316 1.00 14.74
ATOM 11408 OH2 TIP 864 126.212 78.318 165.683 1.00 17.99
ATOM 11409 OH2 TIP 865 83.863 59.125 92.535 1.00 13.37
ATOM 11410 OH2 TIP 866 111.474 81.182 133.217 1.00 17.84
ATOM 11411 OH2 TIP 867 69.682 29.031 113.596 1.00 17.20
ATOM 11412 OH2 TIP 868 118.291 92.109 139.914 1.00 22.38
ATOM 11413 OH2 TIP 869 95.101 71.426 136.943 1.00 16.22
ATOM 11414 OH2 TIP 870 120.054 96.048 137.747 1.0021.34
ATOM 11415 OH2 TIP 871 117.038 75.783 139.278 1.00 14.09
ATOM 11416 OH2 TIP 872 134.546 52.603 158.514 1.00 14.64
ATOM 11417 OH2 TIP 873 121.053 82.349 129.042 1.00 20.38
ATOM 11418 OH2 TIP 874 105.559 77.105 86.849 1.0021.76
ATOM 11419 OH2 TIP 875 123.560 56.808 156.140 1.00 11.04
ATOM 11420 OH2 TIP 876 63.728 51.773 107.694 1.00 13.29
ATOM 11421 OH2 TIP 877 121.766 63.921 164.642 1.00 15.16
ATOM 11422 OH2 TIP 878 87.746 50.680 104.395 1.00 27.13
ATOM 11423 OH2TIP 879 123.017 80.161 164.073 1.00 15.95
ATOM 11424 OH2 TIP 880 140.835 44.211 168.129 1.00 21.18
ATOM 11425 OH2 TIP 881 80.303 50.827 101.047 1.00 17.44
ATOM 11426 OH2 TIP 882 84.461 60.480 100.742 1.0013.69
ATOM 11427 OH2 TIP 883 87.434 43.456 106.677 1.00 15.45
ATOM 11428 OH2 TIP 884 144.746 61.527 161.601 1.00 20.37
ATOM 11429 OH2 TIP 885 140.332 49.829 159.775 1.00 17.47
ATOM 11430 OH2 TIP 886 124.649 67.683 158.569 1.0017.68
ATOM 11431 OH2 TIP 887 74.700 45.901 110.957 1.0020.53
ATOM 11432 OH2 TIP 888 133.282 77.083 164.857 1.00 24.33
ATOM 11433 OH2 TIP 889 94.781 48.478 98.614 1.00 17.04
ATOM 11434 OH2 TIP 890 123.293 89.347 145.957 1.00 16.46
ATOM 11435 OH2 TIP 891 116.231 87.318 152.033 1.00 15.06
ATOM 11436 OH2 TIP 892 80.336 51.559 104.998 1.00 20.38
ATOM 11437 OH2 TIP 893 120.343 69.074 152.290 1.00 16.17
ATOM 11438 OH2 TIP 894 88.578 54.406 71.999 1.00 19.91
ATOM 11439 OH2 TIP 895 88.771 46.843 107.849 1.00 25.36
ATOM 11440 OH2 TIP 896 100.524 76.505 76.437 1.00 19.63
ATOM 11441 OH2 TIP 897 116.124 88.691 132.652 1.00 19.12
ATOM 11442 OH2 TIP 898 134.316 61.070 142.893 1.00 21.52
ATOM 11443 OH2 TIP 899 128.902 79.855 154.408 1.00 20.22
ATOM 11444 OH2 TIP 900 109.434 90.491 153.260 1.00 17.72
ATOM 11445 OH2 TIP 901 90.732 35.356 94.427 1.00 26.48
ATOM 11446 OH2 TIP 902 94.552 78.459 97.327 1.00 22.24
ATOM 11447 OH2 TIP 903 122.244 44.208 156.816 1.00 22.88
ATOM 11448 OH2TIP 904 133.117 85.706 153.509 1.00 15.71
ATOM 11449 OH2 TIP 905 88.155 51.110 78.245 1.00 15.51
ATOM 11450 OH2 TIP 906 70.549 35.914 110.820 1.00 21.45
ATOM 11451 OH2 TIP 907 67.452 58.032 98.344 1.00 18.70
ATOM 11452 OH2 TIP 908 111.518 75.188 129.523 1.00 18.22
ATOM 11453 OH2 TIP 909 126.614 57.366 160.328 1.00 16.33
ATOM 11454 OH2 TIP 910 94.678 41.557 88.966 1.00 19.88
ATOM 11455 OH2 TIP 911 101.880 79.491 125.416 1.00 16.68
ATOM 11456 OH2 TIP 912 87.371 43.132 90.443 1.00 15.94
ATOM 11457 OH2 TIP 913 99.405 65.195 124.649 1.00 21.92
ATOM 11458 OH2 TIP 914 134.143 43.271 165.202 1.00 26.97
ATOM 11459 OH2 TIP 915 91.360 50.469 76.511 1.00 16.72
ATOM 11460 OH2 TIP 916 82.362 44.665 110.746 1.00 15.31
ATOM 11461 OH2 TIP 917 132.515 86.197 146.171 1.00 19.88
ATOM 11462 OH2 TIP 918 70.419 65.778 102.791 1.0022.72
ATOM 11463 OH2 TIP 919 133.661 57.763 151.671 1.00 21.38
ATOM 11464 OH2 TIP 920 65.513 47.401 119.303 1.00 16.44
ATOM 11465 OH2 TIP 921 86.185 38.524 109.628 1.00 19.34
ATOM 11466 OH2 TIP 922 67.694 36.302 118.502 1.00 23.20
ATOM 11467 OH2 TIP 923 85.337 41.213 89.540 1.0017.17
ATOM 11468 OH2 TIP 924 81.081 33.753 104.609 1.00 22.62
ATOM 11469 OH2 TIP 925 90.891 61.537 86.406 1.0022.81
ATOM 11470 OH2 TIP 926 105.127 90.541 124.646 1.00 13.36
ATOM 11471 OH2 TIP 927 85.385 65.815 80.789 1.00 15.67
ATOM 11472 OH2 TIP 928 97.059 70.468 16.598 1.00 25.63
ATOM 11473 OH2 TIP 929 97.456 84.634 85.284 1.0028.10
ATOM 11474 OH2 TIP 930 83.925 43.309 114.408 1.0021.09
ATOM 11475 OH2 TIP 931 141.112 47.061 158.668 1.00 26.27
ATOM 11476 OH2 TIP 932 65.592 60.228 96.813 1.00 23.08
ATOM 11477 OH2 TIP 933 103.947 71.185 147.351 1.00 16.94
ATOM 11478 OH2 T1P 934 75.208 51.558 115.051 1.00 21.03
ATOM 11479 OH2 TIP 935 126.830 71.441 141.929 1.00 22.58
ATOM 11480 OH2 TIP 936 106.929 95.004 117.907 1.00 18.45
ATOM 11481 OH2TIP 937 119.459 93.235 137.762 1.00 19.56
ATOM 11482 OH2 TIP 938 74.864 61.259 71.209 1.00 20.32
ATOM 11483 OH2 TIP 939 120.655 84.033 139.913 1.00 20.97
ATOM 11484 OH2 TIP 940 65.761 32.385 103.313 1.00 18.85
ATOM 11485 OH2 TIP 941 103.408 68.593 97.968 1.00 21.25
ATOM 11486 OH2 TIP 942 76.417 43.373 89.684 1.00 28.22
ATOM 11487 OH2 TIP 943 121.227 90.057 139.984 1.00 12.68
ATOM 11488 OH2 TIP 944 81.339 73.863 113.366 1.0032.82
ATOM 11489 OH2 TIP 945 96.623 60.551 74.337 1.00 18.48
ATOM 11490 OH2 TIP 946 121.591 69.971 150.069 1.00 12.25
ATOM 11491 OH2TIP 947 84.224 76.127 95.395 1.00 19.79
ATOM 11492 OH2 TIP 948 90.599 71.043 88.420 1.00 14.72
ATOM 11493 OH2 TIP 949 101.020 70.270153.142 1.00 22.86
ATOM 11494 OH2 TIP 950 61.947 55.948 129.205 1.0028.79
ATOM 11495 OH2 TIP 951 119.793 62.268 164.387 1.00 22.41
ATOM 11496 OH2 TIP 952 106.572 65.962 141.440 1.00 22.98
ATOM 11497 OH2 TIP 953 98.360 65.143 102.158 1.00 17.73
ATOM 11498 OH2 TIP 954 84.245 74.206 106.948 1.0022.20
ATOM 11499 OH2 TIP 955 106.713 92.265 152.435 1.00 25.13
ATOM 11500 OH2 TIP 956 83.665 42.651 107.796 1.0015.75
ATOM 11501 OH2 TIP 957 77.239 59.651 78.317 1.00 16.75
ATOM 11502 OH2 TIP 958 130.043 76.196 157.143 1.00 18.14
ATOM 11503 OH2 TIP 959 100.891 80.503 80.015 1.0021.94
ATOM 11504 OH2 TIP 960 135.313 51.903 170.157 1.00 20.76
ATOM 11505 OH2 TIP 961 125.453 84.916 152.477 1.00 18.92
ATOM 11506 OH2 TIP 962 153.268 32.855 158.475 1.00 25.33
ATOM 11507 OH2 TIP 963 135.627 79.911 161.984 1.00 25.79
ATOM 11508 OH2 TIP 964 59.721 56.584 99.736 1.00 26.09
ATOM 11509 OH2 TIP 965 63.940 36.077 118.472 1.00 22.49
ATOM 11510 OH2 TIP 966 118.800 57.861 160.218 1.00 25.80
ATOM 11511 OH2 TIP 967 85.048 38.262 96.427 1.00 24.91
ATOM 11512 OH2 TIP 968 122.832 66.572 165.645 1.00 18.44
ATOM 11513 OH2 TIP 969 156.955 43.432 161.328 1.00 21.94
ATOM 11514 OH2 TIP 970 133.627 68.518 170.347 1.00 20.05
ATOM 11515 OH2 TIP 971 117.416 52.698 153.356 1.00 21.15
ATOM 11516 OH2 TIP 972 126.095 59.454 162.384 1.00 19.21
ATOM 11517 OH2 TIP 973 121.976 77.710 170.688 1.00 27.21
ATOM 11518 OH2 TIP 974 92.494 67.904 84.811 1.00 22.96
ATOM 11519 OH2 TIP 975 104.212 96.179 139.299 1.00 25.36
ATOM 11520 OH2 TIP 976 99.080 59.188 74.253 1.00 26.21
ATOM 11521 OH2 TIP 977 119.369 75.405 133.430 1.00 22.66
ATOM 11522 OH2 TIP 978 94.745 44.471 95.202 1.00 20.79
ATOM 11523 OH2 TIP 979 87.720 78.946 134.647 1.00 22.94
ATOM 11524 OH2TIP 980 126.331 57.273 157.539 1.00 20.23
ATOM 11525 OH2 TIP 981 131.935 66.657 182.498 1.00 27.87
ATOM 11526 OH2 TIP 982 94.030 63.268 91.216 1.0023.35
ATOM 11527 OH2 TIP 983 62.501 38.720 94.434 1.00 22.94
ATOM 11528 OH2 TIP 984 56.400 57.154 112.144 1.0020.02
ATOM 11529 OH2 TIP 985 83.034 43.013 103.408 1.00 22.09
ATOM 11530 OH2 TIP 986 67.480 51.204 113.019 1.00 18.39
ATOM 11531 OH2 TIP 987 93.281 84.411 138.921 1.00 26.75
ATOM 11532 OH2 TIP 988 79.146 35.518 95.384 1.00 17.46
ATOM 11533 OH2 TIP 989 82.063 91.077 107.479 1.0040.64
ATOM 11534 OH2 TIP 990 85.263 84.712 114.207 1.00 23.77
ATOM 11535 OH2 TIP 991 66.813 33.404 118.921 1.0034.51
ATOM 11536 OH2 TIP 992 82.808 55.250 93.726 1.00 12.99
ATOM 11537 OH2 TIP 993 81.789 47.430 94.731 1.00 16.63
ATOM 11538 OH2 TIP 994 112.751 70.410 174.157 1.00 20.21
ATOM 11539 OH2 TIP 995 101.125 77.781 147.764 1.00 18.25
ATOM 11540 OH2 TIP 996 103.600 88.353 125.477 1.0026.70
ATOM 11541 OH2 TIP 997 121.056 64.822 176.792 1.00 19.54
ATOM 11542 OH2 TIP 998 86.192 56.364 67.238 1.00 33.81
ATOM 11543 OH2 TIP 999 103.746 76.098 131.964 1.00 19.63
ATOM 11544 OH2 TIP 1000 125.469 73.044 140.267 1.00 31.54
ATOM 11545 OH2 TIP 1001 62.113 42.422 91.968 1.00 27.71
ATOM 11546 OH2 TIP 1002 141.662 57.203 159.241 1.00 26.46
ATOM 11547 OH2 TIP 1003 83.123 51.074 110.446 1.0025.36
ATOM 11548 OH2 TIP 1004 75.734 51.181 128.865 1.0023.19
ATOM 11549 OH2 TIP 1005 92.374 71.439 133.926 1.0025.37
ATOM 11550 OH2 TIP 1006 62.623 51.918 118.625 1.00 24.78
ATOM 11551 OH2 TIP 1007 82.731 69.481 84.470 1.00 21.45
ATOM 11552 OH2 TIP 1008 102.154 71.498 151.100 1.00 24.45
ATOM 11553 OH2 TIP 1009 89.668 62.232 88.764 1.00 14.45
ATOM 11554 OH2 TIP 1010 96.361 72.381 99.941 1.00 29.46
ATOM 11555 OH2 T1P 1011 118.329 88.933 160.583 1.00 16.43
ATOM 11556 OH2 TIP 1012 98.880 86.695 139.668 1.00 16.26
ATOM 11557 OH2 TIP 1013 130.900 61.119 161.031 1.0019.09
ATOM 11558 OH2 TIP 1014 101.875 61.678 100.283 1.00 26.68
ATOM 11559 OH2 TIP 1015 126.709 75.584 172.149 1.0026.25
ATOM 11560 OH2 TIP 1016 123.351 87.141 133.041 1.0027.42
ATOM 11561 OH2 TIP 1017 84.724 42.325 101.243 1.00 18.85
ATOM 11562 OH2 TIP 1018 71.733 60.620 82.388 1.00 25.54
ATOM 11563 OH2 TIP 1019 94.665 40.286 96.000 1.0024.15
ATOM 11564 OH2 TIP 1020 103.950 72.140 154.412 1.00 27.62
ATOM 11565 OH2 TIP 1021 100.272 84.888 82.296 1.00 24.68
ATOM 11566 OH2 TIP 1022 80.174 44.358 101.645 1.0026.02
ATOM 11567 OH2 TIP 1023 98.897 81.098 138.333 1.00 22.28
ATOM 11568 OH2TIP 1024 119.031 52.004 151.325 1.00 25.17
ATOM 11569 OH2 TIP 1025 80.663 58.389 112.040 1.0024.00
ATOM 11570 OH2 TIP 1026 66.478 54.656 86.467 1.0022.16
ATOM 11571 OH2 T1P 1027 70.725 61.868 107.022 1.0020.13
ATOM 11572 OH2 TIP 1028 108.514 65.003 83.819 1.0014.71
ATOM 11573 OH2 TIP 1029 120.455 81.263 116.053 1.00 25.66
ATOM 11574 OH2 TIP 1030 160.176 46.755 154.545 1.00 31.04
ATOM 11575 OH2 TIP 1031 132.231 39.544 146.928 1.0026.85
ATOM 11576 OH2 TIP 1032 96.099 90.050 138.454 1.00 34.79
ATOM 11577 OH2 TIP 1033 101.981 73.083 156.306 1.00 18.73
ATOM 11578 OH2 TIP 1034 96.226 84.974 101.370 1.0022.87
ATOM 11579 OH2 TIP 1035 77.896 41.504 91.256 1.00 17.26
ATOM 11580 OH2 TIP 1036 128.824 76.201 170.809 1.00 25.94
ATOM 11581 OH2 TIP 1037 119.332 97.450 31.541 1.0030.24
ATOM 11582 OH2 TIP 1038 129.542 78.767 167.088 1.00 22.03
ATOM 11583 OH2 TIP 1039 153.910 48.307 161.650 1.0026.46
ATOM 11584 OH2 TIP 1040 119.600 70.198 166.485 1.00 23.55
ATOM 11585 OH2 TIP 1041 104.560 64.017 138.123 1.00 30.87
ATOM 11586 OH2 TIP 1042 94.815 54.599 98.137 1.00 22.39
ATOM 11587 OH2 TIP 1043 144.683 48.886 159.763 1.00 22.03
ATOM 11588 OH2 TIP 1044 130.984 89.950 148.611 1.00 20.77
ATOM 11589 OH2 TIP 1045 95.110 73.906 101.811 1.00 27.24
ATOM 11590 OH2 TIP 1046 60.305 51.486 104.739 1.0032.47
ATOM 11591 OH2 TIP 1047 82.255 71.204 71.939 1.0035.59
ATOM 11592 OH2 TIP 1048 110.459 77.766 99.775 1.0029.55
ATOM 11593 OH2 TIP 1049 115.428 73.120 121.567 1.00 27.35
ATOM 11594 OH2 TIP 1050 103.047 92.987 126.508 1.0025.88
ATOM 11595 OH2 TIP 1051 64.073 33.064 100.714 1.0030.85
ATOM 11596 OH2 TIP 1052 103.261 69.610 85.624 1.0020.77
ATOM 11597 OH2 TIP 1053 127.594 66.849 158.577 1.00 24.46
ATOM 11598 OH2 TIP 1054 150.204 33.365 162.469 1.00 34.57
ATOM 11599 OH2 TIP 1055 89.289 94.475 123.770 1.0034.46
ATOM 11600 OH2 TIP 1056 64.344 59.918 92.863 1.00 29.95
ATOM 11601 OH2 TIP 1057 129.416 91.754 147.660 1.00 28.00
ATOM 11602 OH2 TIP 1058 86.175 62.869 79.436 1.00 20.33
ATOM 11603 OH2 TIP 1059 65.720 61.150 102.392 1.00 32.53
ATOM 11604 OH2 TIP 1060 102.628 96.221 142.434 1.00 20.30
ATOM 11605 OH2 TIP 1061 81.739 57.320 92.171 1.00 10.37
ATOM 11606 OH2 TIP 1062 140.727 44.866 159.661 1.00 23.20
ATOM 11607 OH2 TIP 1063 118.609 71.194 152.581 1.00 20.48
ATOM 11608 OH2 TIP 1064 97.372 41.125 89.371 1.00 19.56
ATOM 11609 OH2 TIP 1065 61.658 50.283 106.859 1.0025.77
ATOM 11610 OH2 TIP 1066 127.974 73.404 140.880 1.00 25.71
ATOM 11611 OH2 TIP 1067 80.091 35.279 102.604 1.0024.83
ATOM 11612 OH2 TIP 1068 93.313 74.773 105.068 1.0035.56
ATOM 11613 OH2 TIP 1069 88.614 53.799 104.377 1.0027.46
ATOM 11614 OH2 TIP 1070 125.688 83.763 149.890 1.00 23.26
ATOM 11615 OH2 TIP 1071 117.667 86.073 159.846 1.00 27.94
ATOM 11616 OH2 TIP 1072 117.547 91.885 96.670 1.00 36.13
ATOM 11617 OH2 TIP 1073 98.627 82.012 140.950 1.00 16.71
ATOM 11618 OH2 TIP 1074 131.876 77.832 144.807 1.00 19.59
ATOM 11619 OH2 TIP 1075 130.419 64.061 154.138 1.00 18.33
ATOM 11620 OH2 TIP 1076 59.061 57.342 108.881 1.0019.84
ATOM 11621 OH2 TIP 1077 114.699 78.138 90.639 1.0024.10
ATOM 11622 OH2 TIP 1078 119.731 77.921 133.023 1.00 30.89
ATOM 11623 OH2 TIP 1079 106.363 65.883 138.947 1.00 30.19
ATOM 11624 OH2 TIP 1080 122.424 84.796 141.791 1.00 19.97
ATOM 11625 OH2 TIP 1081 83.732 52.840 73.514 1.00 20.08
ATOM 11626 OH2 TIP 1082 131.754 72.037 146.172 1.00 27.24
ATOM 11627 OH2 TIP 1083 108.305 91.472 132.118 1.00 23.73
ATOM 11628 OH2 TIP 1084 78.626 38.033 110.232 1.0022.46
ATOM 11629 OH2 TIP 1085 150.298 51.460 153.864 1.00 25.88
ATOM 11630 OH2 TIP 1086 83.320 31.706 125.441 1.00 22.59
ATOM 11631 OH2 TIP 1087 93.401 73.219 140.840 1.00 28.20
ATOM 11632 OH2 TIP 1088 105.941 68.471 157.584 1.00 24.17
ATOM 11633 OH2 TIP 1089 98.077 78.448 138.184 1.00 22.91
ATOM 11634 OH2 TIP 1090 94.693 64.180 62.522 1.00 27.04
ATOM 11635 OH2 TIP 1091 152.977 29.937 158.403 I.00 24.20
ATOM 11636 OH2 TIP 1092 80.297 42.416 100.020 1.00 13.15 5
ATOM 11637 OH2 TIP 1093 92.390 69.392 142.521 1.00 33.13 3
ATOM I 1638 OH2 TIP 1094 91.643 63.989 90.167 1.00 29.16
ATOM 11639 OH2 TIP 1095 135.794 48.975 169.379 1.00 22.80
ATOM 11640 OH2 TIP 1096 106.995 76.300 84.334 1.0019.19
ATOM 11641 OH2 TIP 1097 139.694 50.637 174.063 1.00 27.59
ATOM 11642 OH2 TIP 1098 130.273 41.406 146.521 1.00 30.19
ATOM 11643 OH2 TIP 1099 94.992 67.159 84.542 1.0019.99
ATOM 11644 OH2 TIP 1100 126.336 43.053 162.157 1.00 28.39
ATOM 11645 OH2 TIP 1101 100.888 79.648 77.173 1.00 26.00
ATOM 11646 OH2 TIP 1102 89.053 65.717 101.611 1.00 23.86
ATOM 11647 OH2 TIP 1103 66.275 38.335 118.037 1.0024.86
ATOM 11648 OH2 TIP 1104 122.559 94.925 131.344 1.00 23.06
ATOM 11649 OH2 TIP 1105 79.491 56.945 73.799 1.00 22.87
ATOM 11650 OH2 TIP 1106 109.929 77.372 86.259 1.00 37.78
ATOM 11651 OH2 TIP 1107 56.917 40.229 109.773 1.0026.22
ATOM 11652 OH2 TIP 1108 128.363 60.245 160.675 1.0025.25
ATOM 11653 OH2 TIP 1109 104.336 93.057 123.785 1.00 20.93
ATOM 11654 OH2 TIP 1110 80.538 53.228 94.816 1.00 23.70
ATOM 11655 OH2 TIP 1111 1 102.684 91.190 153.736 1.00 21.58
ATOM 11656 OH2TIP 1112 88.335 83.954 90.796 1.00 27.55
ATOM 11657 OH2 TIP 1113 135.277 81.536 163.953 1.00 22.14
ATOM 11658 OH2 TIP 1114 82.503 75.685 93.295 1.00 22.76
ATOM 11659 OH2 TIP 1115 142.607 56.221 156.872 1.00 27.71
ATOM '11660 OH2 TIP 1116 79.028 43.622 83.358 1.00 23.81
ATOM 11661 OH2TIP 1117 130.599 86.238 132.156 1.00 26.72
ATOM 11662 OH2 TIP 1118 8 63.475 45.063 98.647 1.00 32.24
ATOM 11663 OH2 TIP 1119 76.088 29.837 89.716 1.00 31.87
ATOM 11664 OH2 TIP 1120 143.665 48.443 162.149 1.00 20.05
ATOM 11665 OH2 TIP 1121 1 103.630 92.189 141.954 1.00 27.30
ATOM 11666 OH2 TIP 1122 127.055 85.137 158.905 1.00 26.34
ATOM 11667 OH2 TIP 1123 97.466 84.644 140.785 1.00 18.09
ATOM 11668 OH2 TIP 1124 45.644 41.242 121.984 1.00 30.62
ATOM 11669 OH2 TIP 1125 101.069 67.540 152.665 1.00 29.09
ATOM 11670 OH2 TIP 1126 108.116 97.976 128.407 1.00 30.23
ATOM 11671 OH2 TIP 1127 81.337 31.126 104.353 1.0019.60
ATOM 11672 OH2 TIP 1128 79.280 40.840 82.131 1.0023.87
ATOM 11673 OH2 TIP 1129 83.057 48.114 110.761 1.0030.51
ATOM 11674 OH2 TIP 1130 61.317 46.911 103.535 1.0027.15
ATOM 11675 OH2 TIP 1131 87.992 43.760 93.341 1.0025.81
ATOM 11676 OH2 TIP 1132 109.802 75.325 113.261 1.00 24.98
ATOM 11677 OH2 TIP 1133 97.067 55.016 99.547 1.0025.51
ATOM 11678 OH2 TIP 1134 123.115 61.760 133.968 1.00 26.45
ATOM 11679 OH2 TIP 1135 101.164 67.656 97.328 1.00 30.44
ATOM 11680 OH2 TIP 1136 109.173 z9 1.00 44.02
ATOM 11681 OH2 TIP 1137 95.020 83.124 83.720 1.00 23.58
ATOM 11682 OH2 TIP 1138 116.179 55.799 154.680 1.00 34.56
ATOM 11683 OH2 TIP 1139 133.639 70.897 144.805 1.00 25.36
ATOM 11684 OH2 TIP 1140 134.424 45.092 167.366 1.00 31.35
ATOM 11685 OH2 TIP 1141 83.971 69.194 61.977 1.00 35.10
ATOM 11686 OH2 TIP 1142 72.369 71.134 91.499 1.00 23.84
ATOM 11687 OH2 TIP 1143 124.487 76.027 139.673 1.00 21.75
ATOM 11688 OH2 TIP 1144 64.100 51.800 97.130 1.0023.56
ATOM 11689 OH2 TIP 1145 90.828 49.278 99.111 1.00 24.72
ATOM 11690 OH2 TIP 1146 96.449 73.156 146.092 1.0029.87
ATOM 11691 OH2 TIP 1147 64.150 35.013 103.042 1.00 30.44
ATOM 11692 OH2 TIP 1148 90.777 53.652 73.105 1.00 34.41
ATOM 11693 OH2 TIP 1150 145.240 z.980 156.889 1.00 30.49
ATOM 11694 OH2 TIP 1151 83.832 78.552 96.066 1.00 29.54
ATOM 11695 OH2 TIP 1152 99.125 71.654 132.118 1.0023.77
ATOM 11696 OH2 TIP 1153 99.004 53.628 78.322 1.00 38.21
ATOM 11697 OH2 TIP 1154 123.640 81.798 128.542 1.00 31.24
ATOM 11698 OH2 TIP 1155 132.408 41.360 160.277 1.00 22.72
ATOM 11699 OH2 TIP 1156 106.014 80.819 165.111 1.00 36.29
ATOM 11700 OH2 TIP 1157 91.010 82.677 139.771 1.0035.12
ATOM 11701 OH2 TIP 1158 61.354 47.670 97.545 1.0038.89
ATOM 11702 OH2 TIP 1159 64.071 39.145 89.716 1.0023.25
ATOM 11703 OH2 TIP 1160 139.170 46.740 168.097 1.00 29.70
ATOM 11704 OH2 TIP 1161 85.818 54.275 72.103 1.00 25.74
ATOM 11705 OH2 TIP 1162 123.006 81.338 86.226 1.00 28.90
ATOM 11706 OH2 TIP 1163 135.764 85.635 152.969 1.00 25.35
ATOM 11707 OH2 TIP 1164 66.748 42.904 83.753 1.00 31.43
ATOM 11708 OH2 TIP 1165 81.231 67.532 85.484 1.00 24.29
ATOM 11709 OH2 TIP 1166 106.197 62.608 143.320 1.00 22.03
ATOM 11710 OH2 TIP 1167 130.057 94.020 133.362 1.0022.07
ATOM 11711 OH2 T1P 1168 107.014 i00.191 134.296 1.0032.80
ATOM 11712 OH2 TIP 1169 103.694 95.857 109.248 1.00 42.86
ATOM 11713 OH2 TIP 1170 101.029 91.199 149.234 1.00 28.71
ATOM 11714 OH2 TIP 1171 150.651 29.332 157.478 1.0029.98
ATOM 11715 OH2 TIP 1172 70.878 35.127 84.332 1.0024.90
ATOM 11716 OH2 TIP 1173 72.053 38.014 111.262 1.0023.68
ATOM 11717 OH2 TIP 1174 111.530 65.858 120.739 1.00 28.92
ATOM 11718 OH2 TIP 1175 102.166 73.870 74.823 1.0034.79
ATOM 11719 OH2 TIP 1176 149.932 32.038 152.028 1.0026.40
ATOM 11720 OH2 T1P 1177 95.108 72.917 111:671 1.00 30.75
ATOM 11721 OH2 TIP 1178 8 117.350 74.282 171.300 1.00 21.50
ATOM 11722 OH2 TIP 1179 75.281 63.995 82.524 1.00 35.68
ATOM 11723 OH2 TIP 1180 59.395 55.082 101.894 1.00 26.54
ATOM 11724 OH2 TIP 1181 95.083 85.669 140.908 1.00 31.62
ATOM 11725 OH2 TIP 1182 69.251 63.738 108.080 1.00 29.05
ATOM 11726 OH2 TIP 1183 77.262 45.133 77.568 1.00 27.04
ATOM 11727 OH2 TIP 1184 136.568 69.216 170.072 1.00 26.57
ATOM 11728 OH2 TIP 1185 116.536 68.122 133.287 1.00 30.78
ATOM 11729 OH2 TIP 1186 64.413 62.495 93.793 1.00 29.48
ATOM 11730 OH2 TIP 1187 141.238 53.522 153.332 1.00 25.34
ATOM 11731 OH2 TIP 1188 65.962 59.287 80.896 1.00 25.66
ATOM 11732 OH2 TIP 1189 48.439 41.993 130.207 1.00 36.75
ATOM 11733 OH2 TIP 1190 102.690 93.507 139.855 1.00 30.47
ATOM 11734 OH2 TIP 1191 99.639 87.981 152.109 t.00 32.77
ATOM 11735 OH2 TIP 1192 132.859 76.512 156.384 1.0026.21
ATOM 11736 OH2TIP 1193 115.223 61.575 158.613 1.00 25.94
ATOM 11737 OH2 TIP 1194 63.134 56.292 113.460 1.0023.90
ATOM 11738 OH2 TIP 1195 49.842 52.893 123.773 1.00 33.20
ATOM 11739 OH2 TIP 1196 59.913 44.606 103.563 1.0024.05
ATOM 11740 OH2 TIP 1197 51.046 50.282 110.845 1.00 28.27
ATOM 11741 OH2 TIP 1198 152.405 52.960 156.217 1.00 22.54
ATOM 11742 OH2 TIP 1199 97.441 93.215 117.838 1.0029.72
ATOM 11743 OH2 TIP 1200 101.437 95.068 151.121 1.00 32.60
ATOM 11744 OH2 TIP 1201 105.629 84.518 110.578 1.00 34.77
ATOM 11745 OH2 TIP 1202 93.004 67.673 132.989 1.00 28.76
ATOM 11746 OH2 TIP 1203 108.197 58.784 155.057 1.00 29.31
ATOM 11747 OH2 TIP 1204 154.652 46.470 159.775 1.00 36.77
ATOM 11748 OH2 TIP 1205 68.738 64.369 101.251 1.0028.31
ATOM 11749 OH2 TIP 1206 79.264 67.974 87.305 1.00 22.67
ATOM 11750 OH2 TIP 1207 124.279 47.197 147.625 1.00 34.38
ATOM 11751 OH2 TIP 1208 95.636 51.289 78.952 1.00 23.28
ATOM 11752 OH2 TIP 1209 97.430 47.892 81.101 1.00 28.88
ATOM 11753 OH2 TIP 1210 81.037 66.869 76.711 1.00 26.76
ATOM 11754 OH2 TIP 121 117.172 53.073 165.473 1.00 26.02
ATOM 11755 OH2 TIP 1212 108.177 96.982 125.435 1.0032.88
ATOM 11756 OH2 TIP 1213 115.401 85.552 165.742 1.00 33.60
ATOM 11757 OH2 TIP 1214 133.709 83.799139.049 1.00 24.14
ATOM 11758 OH2 TIP 1215 134.924 71.693 176.580 1.00 33.32
ATOM 11759 OH2 TIP 1216 83.137 36.942 102.783 1.0023.40
ATOM 11760 OH2 TIP 1217 68.188 27.214 107.871 1.0027.63
ATOM 11761 OH2 TIP 1218 64.947 33.596 92.664 1.00 25.77
ATOM 11762 OH2 TIP 1219 118.958 63.662 129.733 1.00 33.68
ATOM 11763 OH2 TIP 1220 74.005 41.107 127.971 1.0021.66
ATOM 11764 OH2 TIP 1221 122.466 74.210 139.153 1.00 20.26
ATOM 11765 OH2 TIP 1222 133.470 47.913 170.729 1.0035.73
ATOM 11766 OH2 TIP 1223 82.298 76.794 81.083 1.00 27.60
ATOM 11767 OH2 TIP 1224 93.815 60.483 91.278 1.00 28.49
ATOM 11768 OH2 TIP 1225 109.618 95.092 158.098 1.00 23.40
ATOM 11769 OH2 TIP 1226 67.886 36.550 132.369 1.00 34.08
ATOM 11770 OH2 TIP 1227 131.373 43.362 162.208 1.00 34.46
ATOM 11771 OH2 TIP 1228 100.580 76.482 110.172 1.0032.76
ATOM 11772 OH2TIP 1229 117.497 82.770 141.851 1.0030.36
ATOM 11773 OH2 TIP 1230 48.117 57.346 126.502 1.00 41.42
ATOM 11774 OH2 TIP 1231 63.505 66.523 117.806 1.0028.14
ATOM 11775 OH2 TIP 1232 120.018 65.017 90.047 1.0046.79
ATOM 11776 OH2 TIP 1233 3 119.467 63.651 92.236 1.0045.29
ATOM 11777 OH2 TIP 1234 114.138 69.916 87.629 1.0027.97
ATOM 11778 OH2 TIP 1235 119.592 74.237 81.634 1.00 36.40
ATOM 11779 OH2 TIP 1236 120.289 78.574 87.063 1.00 34.90
ATOM 11780 OH2 TIP 1237 127.193 79.696 85.420 1.0033.80
ATOM 11781 OH2 TIP 1238 105.016 80.615 103.168 I.00 32.31
ATOM 11782 OH2 TIP 1239 99.840 83.500 103.566 1.0035.41
ATOM 11783 OH2 TIP 1240 96.212 82.512 103.141 1.00 37.26
ATOM 11784 OH2TIP 1241 129.870 81.247 90.612 1.0043.96
ATOM 11785 OH2 TIP 1243 105.171 89.540 86.973 1.0039.92
ATOM 11786 OH2 TIP 1244 118.211 92.115 105.164 1.0034.64
ATOM 11787 OH2 TIP 1245 121.540 91.561 102.064 1.00 34.48
ATOM 11788 OH2 TIP 1246 119.854 74.995 109.820 1.0044.65
ATOM 11789 OH2 TIP 1247 124.844 77.007 105.143 1.0049.85
ATOM 11790 OH2 TIP 1248 109.498 82.169 103.178 1.00 45.04
ATOM 11791 OH2 TIP 1249 104.116 80.680 86.747 1.00 34.86
ATOM 11792 OH2 TIP 1250 106.413 79.123 88.152 1.00 34.77
ATOM 11793 OH2 TIP 1251 106.674 81.746 87.499 1.00 36.89
ATOM 11794 OH2TIP 1252 116.286 81.761 83.964 1.0038.44
ATOM 11795 OH2 TIP 1253 104.212 73.071 95.505 1.0041.85
ATOM 11796 OH2 TIP 1254 93.599 87.483 95.045 1.0039.18
ATOM 11797 OH2 TIP 1255 84.995 81.997 86.793 1.0033.99
ATOM 11798 OH2 TIP 1256 81.708 73.902 88.903 1.0028.81
ATOM 11799 OH2 TIP 1257 80.860 72.539 85.702 1.0033.47
ATOM 11800 OH2TIP 1258 83.564 67.477 81.398 1.0026.10
ATOM 11801 OH2TIP 1259 78.156 62.712 67.978 1.00 39.54
ATOM 11802 OH2 TIP 1260 100.031 59.342 71.937 1.0039.53
ATOM 11803 OH2 TIP 1261 93.718 61.609 64.041 1.00 34.87
ATOM 11804 OH2 TIP 1262 97.482 57.444 67.838 1.0030.74
ATOM 11805 OH2 TIP 1263 98.875 81.086 76.024 1.0030.76
ATOM 11806 OH2 TIP 1264 91.666 77.011 70.697 1.0035.48
ATOM 11807 OH2 TIP 1265 91.372 78.281 72.897 1.00 32.56
ATOM 11808 OH2 TIP 1266 82.010 53.676 71.667 1.0029.78
ATOM 11809 OH2 TIP 1267 81.435 55.838 66.455 1.0030.74
ATOM 11810 OH2 TIP 1268 107.016 70.111 889.125 1.00 30.21
ATOM 11811 OH2 TIP 1269 105.981 69.405 86.938 1.0024.95
ATOM 11812 OH2 TIP 1270 99.638 60.799 86.172 1.0039.21
ATOM 11813 OH2 TIP 1271 78.324 62.636 '78.039 1.0036.48
ATOM 11814 OH2 TIP 1272 79.310 57.984 77.072 1.0034.43
ATOM 1 I 815 OH2 TIP 1273 95.491 52.778 74.079 1.0035.94
ATOM 11816 OH2 TIP 1274 97.837 49.406 76.985 1.00 39.06
ATOM 11817 OH2 TIP 1275 97.041 46.745 78.750 1.0034.98
ATOM 11818 OH2 TIP 1276 99.201 45.208 78.023 1.0039.09
ATOM 11819 OH2 TIP 1277 93.322 49.146 87.435 1.0025.80
ATOM 11820 OH2 TIP 1278 95.655 48.421 89.508 1.0033.86
ATOM 11821 OH2 TIP 1279 78.431 49.875 75.270 loo 34.75
ATOM 11822 OH2 TIP 1280 80.407 50.390 73.510 1.0030.43
ATOM 11823 OH2 TIP 1281 75.767 52.714 74.102 1.00 57.75
ATOM 11824 OH2 TIP 1282 69.470 60.428 76.838 1.0035.72
ATOM 11825 OH2 TIP 1283 64.424 56.578 86.106 1.00 30.11
ATOM 11826 OH2 TIP 1285 61.664 50.951 76.742 1.0041.84
ATOM 11827 OH2 TIP 1286 61.693 50.208 83.068 1.0045.59
ATOM 11828 OH2 TIP 1287 61.399 63.424 100.286 1.0034.69
ATOM 11829 OH2 TIP 1288 68.436 63.073 103.571 1.00 27.48
ATOM 11830 OH2 TIP 1289 56.156 S8.S77109.7SS 1.0045.17
ATOM 11831 OH2 TIP 1290 77.994 41.483 98.780 1.00 35.31
ATOM 11832 OH2 TIP 1291 74.421 29.156 87.403 1.00 40.09
ATOM 11833 OH2 TIP 1292 81.536 38.999 81.352 1.0033.68
ATOM 11834 OH2 TIP 1293 82.122 30.227 86.432 1.0050.32
ATOM 11835 OH2 TIP 1294 86.127 40.793 87.147 1.0039.66
ATOM 11836 OH2 TIP 1295 86.130 38.257 87.203 1.0037.76
ATOM 11837 OH2 TIP 1296 67.851 38.456 83.160 1.0031.12
ATOM 11838 OH2 TIP 1297 67.891 37.491 90.712 1.00 18.84
ATOM 11839 OH2 TIP 1298 73.632 38.661 80.746 1.0040.69
ATOM 11840 OH2 TIP 1299 69.825 31.099 88.412 1.0026.26
ATOM 11841 OH2 TIP 1300 68.647 26.210 90.760 1.0039.96
ATOM 11842 OH2 TIP 1301 59.862 42.876 99.586 1.0033.62
ATOM 11843 OH2 TIP 1302 59.670 36.091 104.742 1.00 41.42
ATOM 11844 OH2 TIP 1303 98.135 47.959 94.103 1.0050.10
ATOM 11845 OH2 TIP 1304 97.557 44.491 91.427 1.0043.19
ATOM 11846 OH2 TIP 1306 93.140 52.459 100.911 1.0039.38
ATOM 11847 OH2 TIP 1307 85.994 52.540 105.044 1.00 29.55
ATOM 11848 OH2 TIP 1308 90.837 53.866 103.559 1.00 27.35
ATOM 11849 OH2 TIP 1309 94.341 49.839 102.992 1.0039.93
ATOM 11850 OH2 TIP 1310 90.690 50.161 103.012 1.0038.82
ATOM 11851 OH2 TIP 1311 88.428 44.642 73.154 I.00 41.41
ATOM 11852 OH2 TIP 1312 74.721 45.706 78.799 1.0036.20
ATOM 11853 OH2 TIP 1313 64.409 43.961 84.624 1.0036.76
ATOM 11854 OH2 TIP 1314 59.908 44.917 87.303 1.0037.24
ATOM 11855 OH2 TIP 1315 102.063 59.928 108.880 1.00 35.10
ATOM 11856 OH2 TIP 1316 86.938 75.485 105.783 1.00 44.29
ATOM 11857 OH2 TIP 1317 82.497 78.028 98.396 1.00 32.03
ATOM 11858 OH2 TIP 1318 78.098 77.495 92.916 1.0027.33
ATOM 11859 OH2 TIP 1319 82.003 79.429 94.793 1.00 33.37
ATOM 11860 OH2 TIP 1320 65.657 58.567 94.892 1.00 29.28
ATOM 11861 OH2 TIP 1321 84.288 76.696 111.004 1.0038.75
ATOM 11862 OH2 TIP 1322 81.920 79.688 106.141 1.0038.64
ATOM 11863 OH2 TIP 1323 76.068 76.053 101.945 1.00 42.79
ATOM 11864 OH2TIP 1324 84.177 62.881 115.834 1.0033.17
ATOM 11865 OH2 TIP 1325 94.039 67.438 113.465 1.00 36.67
ATOM 11866 OH2 TIP 1326 92.694 63.809 115.519 1.0038.16
ATOM 1 I867 OH2 TIP 1327 83.930 77.114 119.062 1.0034.66
ATOM 11868 OH2 TIP 1328 85.639 75.460 119.765 1.00 32.78
ATOM 11869 OH2 TIP 1329 72.122 73.757 115.746 1.0040.15
ATOM 11870 OH2 TIP 1330 82.629 46.168 124.646 1.0032.21
ATOM 11871 OH2TIP 1331 92.630 42.515 116.267 1.00 40.15
ATOM 11872 OH2 TIP 1332 71.897 47.944 134.670 1.0047.95
ATOM 11873 OH2 TIP 1333 70.665 43.174 136.128 1.00 29.71
ATOM 11874 OH2 TIP 1334 76.410 59.427 124.808 1.00 34.55
ATOM 11875 OH2 TIP 1335 75.246 56.567 124.526 1.0036.18
ATOM 11876 OH2TIP 1336 66.891 59.791 121.949 1.0038.73
ATOM 11877 OH2 T1P 1337 64.389 61.441 121.548 1.0032.37
ATOM 11878 OH2 TIP 1338 73.286 40.375 112.689 1.0021.57
ATOM 11879 OH2 TIP I339 86.954 43.500 114.669 1.00 31.92
ATOM 11880 OH2 TIP 1340 78.260 25.331 108.921 1.0054.82
ATOM 11881 OH2 TIP 1341 90.160 26.571 123.12 1.00 31.31
ATOM 11882 OH2 TIP 1342 81.959 23.893 122.099 1.00 34.54
ATOM 11883 OH2 TIP 1343 91.401 30.269 122.724 1.00 46.41
ATOM 11884 OH2 TIP 1344 84.556 33.589 103.570 1.00 36.61
ATOM 11885 OH2 TIP 1345 64.443 31.879 114.636 1.0043.27
ATOM 11886 OH2 TIP 1346 57.467 43.715 103.467 1.0025.77
ATOM 11887 OH2 TIP 1347 52.371 43.538 110.664 1.0031.15
ATOM 11888 OH2 TIP 1348 57.394 60.898 113.311 1.0036.92
ATOM 11889 OH2 TIP 1349 58.171 64.168 108.291 1.00 38.75
ATOM 11890 OH2 TIP 1350 57.091 61.878 129.012 1.00 33.96
ATOM 11891 OH2 TIP 1351 52.148 60.892 123.724 1.0030.17
ATOM 11892 OH2 TIP 1352 57.639 44.699 134.308 1.0039.65
ATOM 11893 OH2 TIP 1353 56.753 46.818 133.284 1.0037.52
ATOM 11894 OH2 TIP 1354 58.844 52.428 133.985 1.0032.15
ATOM 11895 OH2 TIP 1355 61.810 41.934 134.781 1.0039.41
ATOM 11896 OH2 TIP 1356 45.376 54.272 118.936 1.00 32.25
ATOM 11897 OH2 TIP 1357 46.519 44.528 108.120 1.00 42.16
ATOM 11898 OH2 TIP 1358 50.216 52.067 109.028 1.00 40.09
ATOM 11899 OH2 TIP 1359 52.217 33.025 119.470 1.00 49.41
ATOM 11900 OH2 TIP 1360 58.260 31.267 124.047 1.00 37.97
ATOM 11901 OH2 TIP 1361 54.432 33.636 127.778 1.0042.24
ATOM 11902 OH2 TIP 1362 48.950 34.386 129.332 1.00 32.23
ATOM 11903 OH2 TIP 1363 42.588 36.567 125.450 1.0045.05
ATOM 11904 OH2 TIP 1364 53.461 33.959 131.016 1.0040.22
ATOM 11905 OH2 TIP 1365 156.632 52.522 156.422 1.00 30.41
ATOM 11906 OH2 TIP 1366 155.794 49.505 153.635 1.0042.02
ATOM 11907 OH2 TIP 1367 161.864 46.029156.877 I.00 33.83
ATOM 11908 OH2 TIP 1368 157.941 47.981 161.019 1.00 46.31
ATOM 11909 OH2 TIP 1369 151.460 48.163 161.841 I .00 26.10
ATOM 11910 OH2 TIP 1370 144.506 47.436147.210 1.00 33.39
ATOM 11911 OH2TIP 1371 164.471 40.666 147.805 1.00 31.31
ATOM 11912 OH2 TIP 1372 158.710 33.454 146.917 1.00 44.38
ATOM 11913 OH2 TIP 1373 146.963 25.877 159.235 I .00 32.09
ATOM 11914 OH2 TIP 1374 152.797 31.879 163.642 1.0035.88
ATOM 11915 OH2 TIP 1375 142.127 35.890 161.720 1.0029.07
ATOM 11916 OH2 TIP 1376 138.469 37.613 160.988 1.0033.05
ATOM 11917 OH2 TIP 1377 146.069 34.686 167.396 1.0033.39
ATOM 11918 OH2 TIP 1379 149.588 30.175 143.582 1.0042.73
ATOM 11919 OH2TIP 1380 143.521 42.316 143.874 1.0046.80
ATOM 11920 OH2 TIP 1381 137.534 29.811 I59.264 1.0040.95
ATOM 11921 OH2 TIP 1382 138.744 45.884 160.701 1.0025.96
ATOM 11922 OH2 TIP 1383 143.372 46.076 160.892 1.0032.72
ATOM 11923 OH2 TIP 1384 160.995 39.026 162.240 1.0033.12
ATOM 11924 OH2 TIP 1385 146.973 39.997 166.212 1.00 39.83
ATOM 11925 OH2 TIP 1386 138.950 52.318 151.381 1.0041.68
ATOM 11926 OH2 TIP 1387 139.776 52.052 148.998 1.0043.50
ATOM 11927 OH2 TIP 1388 129.255 48.445 149.331 1.0024.79
ATOM 11928 OH2 TIP 1389 119.705 46.796 158.921 1.00 34.40
ATOM 11929 OH2 TIP 1390 112.702 51.232 154.132 1.00 4I .65
ATOM 11930 OH2 TIP 1391 112.393 57.171 156.801 1.00 43.59
ATOM 11931 OH2TIP 1392 114.710 48.335 155.178 1.0043.33
ATOM 11932 OH2 TIP 1393 116.690 49.407 152.035 1.0042.33
ATOM 11933 OH2 TIP 1394 117.910 62.573 166.260 1.00 32.08
ATOM 11934 OH2 TIP 1395 114.484 61.325 171.594 1.00 35.23
ATOM 11935 OH2 TIP 1396 112.848 64.164 176.506 1.0040.10
ATOM 11936 OH2 TIP 1397 120.087 58.273 182.185 1.0039.94
ATOM 11937 OH2 TIP 1398 124.377 53.222 173.495 1.0040.14
ATOM 11938 OH2 TIP 1400 129.646 43.259 167.033 1.0051.13
ATOM 11939 OH2 TIP 1401 125.602 44.094 170.027 1.0033.59
ATOM 11940 OH2 TIP 1402 123.181 45.581 167.544 1.0041.86
ATOM 11941 OH2 TIP 1404 123.336 70.959 178.741 1.00 28.93
ATOM 11942 OH2 TIP 1405 141.005 62.235 171.984 1.00 42.71
ATOM 11943 OH2 TIP 1406 140.743 59.881 170.719 z.25
ATOM 11944 OH2 TIP 1407 134.106 67.256 159.866 I.00 29.71 1
ATOM 11945 OH2 TIP 1408 133.092 69.051 158.310 1.0030.10
ATOM 11946 OH2 TIP 1409 117.498 62.203 160.517 1.00 25.18
ATOM 11947 OH2 TIP 1410 134.173 83.842 166.754 1.00 33.95
ATOM 11948 OH2 TIP 1411 131.043 78.405 155.980 1.0026.52
ATOM 11949 OH2 TIP 1412 116.509 72.726 168.189 1.00 35.32
ATOM 11950 OH2 TIP 1413 111.654 70.946 167.168 1.00 32.87
ATOM 11951 OH2 TIP 1415 108.508 80.852 171.607 1.00 37.38
ATOM 11952 OH2 TIP 1416 107.254 79.890 167.531 1.00 35.28
ATOM 11953 OH2 TIP 1417 102.395 79.918 163.549 1.0034.94
ATOM 11954 OH2 TIP 1418 8 109.935 71.939 164.171 1.00 36.96
ATOM 11955 OH2 TIP 1419 103.949 76.222 158.616 1.0022.93
ATOM 11956 OH2 T1P 1420 96.582 78.352 162.270 1.00 33.37
ATOM 11957 OH2 TIP 1421 62.588 34.802 91.971 1.00 38.19
ATOM 11958 OH2 TIP 1423 59.844 40.896 102.054 1.00 43.54
ATOM 11959 OH2 TIP 1424 93.993 37.525 95.799 1.0038.13
ATOM 11960 OH2 TIP 1425 68.275 44.683 82.533 1.0027.75
ATOM 11961 OH2 TIP 1427 132.050 88.379 166.026 1.00 24.18
ATOM 11962 OH2 TIP 1428 127.262 87.454 166.390 1.00 35.89
ATOM 11963 OH2 TIP 1430 106.713 82.471 162.244 1.0031.67
ATOM 11964 OH2 TIP 1431 102.639 86.992 159.284 1.00 33.08
ATOM 11965 OH2 TIP 1432 t 10.237 88.954 162.329 1.0043.25
ATOM 11966 OH2TIP 1433 99.174 82.115 146.282 1.00 34.40
ATOM 11967 OH2 TIP 1434 124.696 61.267 143.725 1.00 42.23
ATOM 11968 OH2 TIP 1435 130.814 76.227 140.283 1.00 41.20
ATOM 11969 OH2 TIP 1436 129.914 73.587 143.084 1.0030.42
ATOM 11970 OH2 TIP 1437 136.686 67.090 146.055 1.00 32.73
ATOM 11971 OH2 TIP 1438 137.858 63.988 144.847 1.0035.14
ATOM 11972 OH2 TIP 1440 121.149 5t.376 141.291 1.00 38.36
ATOM 11973 OH2 TIP 1441 114.554 50.153 146.656 1.0035.14
ATOM 11974 OH2 TIP 1442 104.054 57.049149.402 1.0037.45
ATOM 11975 OH2 TLP 1443 100.738 59.867 154.008 1.0033.28
ATOM 11976 OH2 TIP 1444 116.689 54.719 157.247 1.0029.49
ATOM 11977 OH2 TIP 1445 102.791 66.185 156.084 1.0032.45
ATOM 11978 OH2 TIP 1446 97.206 61.430 149.523 1.00 40.35
ATOM 11979 OH2 TIP 1447 127.592 78.843 133.343 1.00 41.99
ATOM 11980 OH2 TIP 1448 z20 80.208 129.223 1.0041.58
ATOM 11981 OH2 TIP 1449 126.087 67.275 122.985 1.0047.23
ATOM 11982 OH2 TIP 1450 111.774 59.603127.192 1.0031.88
ATOM 11983 OH2 TIP 1451 103.935 61.078 135.974 1.0037.13
ATOM 11984 OH2 TIP 1452 104.424 64.161 144.840 1.00 31.60
ATOM 11985 OH2 TIP 1454 129.122 80.951 122.251 1.0042.17
ATOM 11986 OH2TIP 1455 108.517 76.604 110.747 1.0039.66
ATOM 11987 OH2 TIP 1456 109.222 60.877 127.910 1.0037.69
ATOM 11988 OH2 TIP 1457 100.750 65.108 134.344 1.00 30.51
ATOM 11989 OH2 TIP 1460 112.057 89.104 114.328 1.0031.89
ATOM 11990 OH2 TIP 1461 116.599 91.536 115.089 1.0031.50
ATOM 11991 OH2 TIP 1462 110.251 86.594 130.845 1.00 12.72
ATOM 11992 OH2 TIP 1463 116.141 86.899 144.289 1.0033.54
ATOM 11993 OH2 TIP 1464 129.651 86.495 129.114 1.0036.28
ATOM 11994 OH2 TIP 1465 131.844 88.429 138.840 1.0033.71
ATOM 11995 OH2 TIP 1466 132.330 89.934 141.120 1.0028.25
ATOM 11996 OH2 TIP 1467 126.927 93.910 149.800 1.00 27.1-8
ATOM 11997 OH2 TIP 1469 113.633 103.009 139.852 1.00 31.21 1
ATOM 11998 OH2 TIP 1470 100.015 97.161 141.733 1.0038.48
ATOM 11999 OH2 TIP 1471 103.495 97.892 144.219 1.0022.72
ATOM 12000 OH2 TIP 1472 100.355 94.413 139.909 1.0044.12
ATOM 12001 OH2 TIP 1473 100.706 96.603 132.494 1.0040.28
ATOM 12002 OH2 TIP 1474 98.385 94.280 130.971 1.00 36.20
ATOM 12003 OH2 TIP 1475 94.556 88.640 134.317 1.00 31.38
ATOM 12004 OH2 TIP 1476 97.266 88.359 141.690 1.00 39.43
ATOM 12005 OH2 TIP 1477 96.909 77.273 140.340 1.0032.18
ATOM 12006 OH2 TIP 1478 91.044 69.157 132.985 1.0035.78
ATOM 12007 OH2 TIP 1479 93.321 68.179 119.850 1.0031.10
ATOM 12008 OH2 TIP 1480 97.604 68.553 120.822 1.0034.02
ATOM 12009 OH2 TIP 1481 99.047 74.402 113.320 1.0026.59
ATOM 12010 OH2 TIP 1482 02.509 79.282 111.827 1.0033.28
ATOM 12011 OH2 TIP 1483 104.809 88.255 110.878 1.00 32.89
ATOM 12012 OH2 TIP 1485 82.265 73.398 122.946 1.0035.59
ATOM 12013 OH2 TIP 1486 106.086 96.874 116.083 1.0036.70
ATOM 12014 OH2 TIP 1487 96.821 94.057 110.630 1.0039.82
ATOM 12015 OH2 TIP 1489 94.355 95.396 119.222 1.0035.87
ATOM 12016 OH2 TIP 1490 91.694 93.405 115.808 1.00 34.71 1
ATOM 12017 OH2 TIP 1491 82.292 85.420 124.950 1.00 36.33
ATOM 12018 OH2 TIP 1492 83.123 52.108 120.932 1.0045.11
ATOM 12019 OH2 TIP 1493 86.415 59.700 115.812 1.0038.64
ATOM 12020 C1 GLC 751 88.121 53.265 111.634 1.0075.07
ATOM 12021 C2 GLC 751 87.910 51.971 110.843 1.0076.10
ATOM 12022 02 GLC 751 86.568 51.527 111.000 1.00 74.55
ATOM 12023 C3 GLC 751 88.869 50.881 111.339 1.0077.20
ATOM 12024 03 GLC 751 88.796 49.754 110.478 1.0077.76
ATOM 12025 C4 GLC 751 90.328 51.367 111.410 1.0078.05
ATOM 12026 04 GLC 751 91.085 50.410 112.178 1.0079.42
ATOM 12027 C5 GLC 751 90.424 52.742 112.095 1.0078.03
ATOM 12028 05 GLC 751 1 89.479 53.669 111.513 1.00 76.52
ATOM 12029 C6 GLC 751 91.798 53.373 111.978 1.0078.94
ATOM 12030 06 GLC 751 92.445 52.982 110.775 1.0080.10
ATOM 12031 C1 GLC 752 86.561 55.303 116.255 1.0070.39
ATOM 12032 C2 GLC 752 85.432 54.585 115.520 I.00 69.58
ATOM 12033 02 GLC 752 84.571 53.962 116.461 1.0068.24
ATOM 12034 C3 GLC 752 86.033 53.527 114.596 1.0069.61
ATOM 12035 03 GLC 752 85.005 52.894 113.850 1.0068.99
ATOM 12036 C4 GLC 752 87.072 54.137 113.640 1.0070.37
ATOM 12037 04 GLC 752 87.754 53.058 112.970 1.0072.59
ATOM 12038 C5 GLC 752 88.094 54.989 114.422 1.00 69.37
ATOM 12039 05 GLC 752 87.418 55.920 115.300 1.0068.80
ATOM 12040 C6 GLC 752 89.006 55.809 113.531 1.0068.24
ATOM 12041 06 GLC 752 89.802 56.700 I 14.298 1.0066.70
ATOM 12042 C1 GLC 753 88.860 53.877 120.828 1.0078.23
ATOM 12043 C2 GLC 753 87.366 53.561 120.728 1.0077.82
ATOM 12044 02 GLC 753 87.087 52.344 121.408 1.0077.72
ATOM 12045 C3 GLC 753 86.964 53.439 119.252 1.00 76.50
ATOM 12046 03 GLC 753 85.557 53.270 119.148 1.0076.06
ATOM 12047 C4 GLC 753 87.396 54.675 118.442 1.0075.84
ATOM 12048 04 GLC 753 87.250 54.373 117.042 1.00 73.46
ATOM 12049 C5 GLC 753 88.868 55.043 118.720 1.0076.76
ATOM 12050 05 GLC 753 89.123 55.098 120.142 1.0077.52
ATOM 12051 C6 GLC 753 89.277 56.387 118.143 1.0077.21
ATOM 12052 06 GLC 753 88.144 57.178 117.809 1.0078.54
ATOM 12053 C1 GLC 754 93.350 51.076 120.582 1.0080.29
ATOM 12054 C2 GLC 754 92.276 50.434 121.460 1.0080.51
ATOM 12055 02 GLC 754 92.348 49.021 121.331 1.00 80.57
ATOM 12056 C3 GLC 754 90.895 50.919 121.009 1.00 80.31
ATOM 12057 03 GLC 754 89.895 50.416 121.881 1.0079.87
ATOM 12058 C4 GLC 754 90.814 52.451 120.968 1.0079.62
ATOM 12059 04 GLC 754 89.594 52.810 120.292 1.0078.70
ATOM 12060 C5 GLC 754 92.015 53.053 120.207 1.0079.84
ATOM 12061 05 GLC 754 93.262 52.494 120.686 1.00 79.75
ATOM 12062 C6 GLC 754 92.122 54.561 120.360 1.00 80.05
ATOM 12063 06 GLC 754 92.456 54.925 121.692 1.00 79.35
ATOM 12064 C1 GLC 755 95.307 49.867 115.829 1.0079.56
ATOM 12065 C2 GLC 755 95.184 48.666 116.764 1.0079.71
ATOM 12066 02 GLC 755 94.837 47.506 116.020 1.0080.19
ATOM 12067 C3 GLC 755 94.106 48.959 117.811 1.0079.51
ATOM 12068 03 GLC 755 94.048 47.901 118.756 1.0079.03
ATOM 12069 C4 GLC 755 94.371 50.284 118.544 1.0079.32
ATOM 12070 04 GLC 755 93.180 50.636 119.266 1.0079.47
ATOM 12071 C5 GLC 755 94.714 51.426 117.569 1.0078.95
ATOM 12072 05 GLC 755 95.697 51.000 116.597 1.00 78.88
ATOM 12073 C6 GLC 755 95.287 52.636 118.278 1.00 78.52
ATOM 12074 06 GLC 755 94.317 53.665 118.401 1.0078.12
ATOM 12075 C1 GLC 756 92.157 49.772 111.538 1.0080.10
ATOM 12076 C2 GLC 756 92.418 48.427 112.218 1.0080.43
ATOM 12077 02 GLC 756 91.259 47.607 112.138 1.0080.64
ATOM 12078 C3 GLC 756 92.785 48.673 113.685 1.0080.30
ATOM 12079 03 GLC 756 93.104 47.439 114.313 1.0079.20
ATOM 12080 C4 GLC 756 93.974 49.650 113.810 t.00 79.92
ATOM 12081 04 GLC 756 94.081 50.077 115.182 I.00 79.73
ATOM 12082 C5 GLC 756 93.780 50.902 112.929 1.0080.19
ATOM 12083 05 GLC 756 93.350 50.548 111.591 1.0080.36
ATOM 12084 C6 GLC 756 95.059 51.702 112.783 1.00 79.87
ATOM 12085 06 GLC 756 94.783 53.055 112.457 1.0080.19
ATOM 12086 C1 GLC 761 124.357 72.463 132.402 1.0077.58

ATOM 12087 C2 GLC 761 124.389 73.852 133.061 1.00 78.00
ATOM 12088 02 GLC 761 123.149 74.513 132.845 1.0076.64
ATOM 12089 C3 GLC 761 125.534 74.704 132.491 1.0078.72
ATOM 12090 03 GLC 761 125.641 75.917 133.223 1.00 78.16
ATOM 12091 C4 GLC 761 126.872 73.953 132.549 1.00 79.74
ATOM 12092 04 GLC 761 127.873 74.730 131.857 1.0080.83
ATOM 12093 C5 GLC 761 126.714 72.572 131.883 1.0079.88
ATOM 12094 05 GLC 761 125.635 71.841 132.509 1.0078.63
ATOM 12095 C6 GLC 761 127.951 1 71.696 131.979 I .00 80.26
ATOM 12096 06 GLC 761 128.520 71.748133.280 1.00 80.41
ATOM 12097 C1 GLC 762 122.479 70.014 128.161 1.0074.92
ATOM 12098 C2 GLC 762 121.441 70.977 128.729 1.0074.04
ATOM 12099 02 GLC 762 120.678 71.540 127.674 1.0073.16
ATOM 12100 C3 GLC 762 122.164 72.082 129.501 1.0074.21
ATOM 12101 03 GLC 762 121.218 72.943 130.112 1.0073.81
ATOM 12102 C4 GLC 762 123.098 71.502 30.575 1.00 74.84
ATOM 12103 04 GLC 762 123.932 72.569 131.072 I .00 76.62
ATOM 12104 C5 GLC 762 123.984 70.373130.005 1.0074.32
ATOM 12105 05 GLC 762 123.191 69.432 129.243 1.0074.81
ATOM 12106 C6 GLC 762 124.687 69.571 131.083 1.0074.51 1
ATOM 12107 06 GLC 762 125.488 68.541 130.521 1.0074.57
ATOM 12108 C GLC 763 124.895 70.682 123.480 1.0080.56
ATOM 12109 C2 GLC 763 123.606 71.493 123.633 1.00 80.15
ATOM 12110 02 GLC 763 123.749 72.749 122.984 1.0080.47
ATOM 12111 C3 GLC 763 123.313 7I.716 125.118 1.0079.31
ATOM 12112 03 GLC 763 122.057 72.363 125.269 1.0079.73
ATOM 12113 C4 GLC 763 123.314 70.396 125.911 1.00 78.42
ATOM 12114 04 GLC 763 123.340 70.720 127.312 1.0076.98
ATOM 12115 C5 GLC 763 124.559 69.553125.579 1.0078.81
ATOM I2116 05 GLC 763 124.736 69.435 124.149 1.0080.11
ATOM 12117 C6 GLC 763 124.498 68.148 126.138 1.0078.78
ATOM 12118 06 GLC 763 125.718 67.804 126.779 1.00 78.21
ATOM 12119 C GLC 764 129.582 73.151 123.165 1.00 81.2S
ATOM 12120 C2 GLC 764 128.512 73.475 122.127 1.00 81.60
ATOM 12121 02 GLC 764 128.512 74.869 121.851 1.00 81.37
ATOM 12122 C3 GLC 764 127.143 73.056 122.665 1.00 81.77
ATOM 12123 03 GLC 764 126.168 73.215 121.646 1.0082.73
ATOM 12124 C4 GLC 764 127.127 71.596 123.170 1.00 81.37
ATOM 12125 04 GLC 764 125.965 71.421 124.004 1.00 81.42
ATOM 12126 C5 GLC 764 128.377 71.249 124.010 1.0080.69
ATOM 12127 05 GLC 764 129.582 71.747 123.387 1.00 80.53
ATOM 12128 C6 GLC 764 128.561 69.756 124.195 1.0079.75
ATOM 12129 06 GLC 764 128.561 69.082 122.946 1.0078.98
ATOM 12130 C1 GLC 765 131.470 75.255 127.653 1.0081.54
ATOM 12131 C2 GLC 765 131.225 76.267 126.533 1.00 81.37
ATOM 12132 02 GLC 765 130.847 77.515 127.095 1.0081.35
ATOM 12133 C3 GLC 765 130.111 75.754 125.617 1.0081.10
ATOM 12134 03 GLC 765 129.919 76.653 124.536 1.00 81.18
ATOM 12135 C4 GLC 765 130.450 74.360 125.077 1.00 80.99
ATOM 12136 04 GLC 765 129.315 73.866 124.342 1.0080.84
ATOM 12137 C5 GLC 765 130.774 73.408 126.244 1.0080.83
ATOM 12138 05 GLC 765 131.796 73.981 127.096 1.0081.52
ATOM 12139 C6 GLC 765 131.297 72.063 125.778 1.0080.50
ATOM 12140 06 GLC 765 132.637 72.164 125.316 1.0080.05
ATOM 12141 C1 GLC 766 129.172 74.729 132.394 1.0081.34
ATOM 12142 C2 GLC 766 129.838 76.072 I32.108 1.0082.05
ATOM 12143 02 GLC 766 129.090 77.120 132.705 1.0081.68
ATOM 12144 C3 GLC 766 129.917 76.280 130.592 1.0082.30
ATOM 12145 03 GLC 766 130.639 77.470 130.306 1.0083.02
ATOM 12146 C4 GLC 766 130.594 75.091 129.890 1.00 82.04
ATOM 12147 04 GLC 766 130.343 75.198 128.478 1.0081.89
ATOM 12148 C5 GLC 766 130.051 73.735 130.386 1.0081.88
ATOM 12149 05 GLC 766 129.978 73.702 131.830 1.0081.80
ATOM 12150 C6 GLC 766 130.924 72.568 129.961 1.0081.85
ATOM 12151 06 GLC 766 132.235 72.684 130.493 1.0080.84
ATOM 12152 C ACT 731 77.359 28.803 111.594 1.00 35.21
ATOM 12153 O ACT 731 78.421 28.157 111.167 1.00 34.55
ATOM 12154 OXT ACT 731 76.938 29.935 111.180 1.00 36.25
ATOM 12155 CH3 ACT 731 76.490 28.247 112.660 1.00 33.61
ATOM 12156 C ACT 732 115.318 97.610 129.965 1.00 36.37
ATOM 12157 O ACT 732 115.136 96.417 130.472 1.00 38.15
ATOM 12158 OXT ACT 732 114.525 98.213 129.167 1.0034.62
ATOM 12159 CH3 ACT 732 116.538 98.412 130.300 1.0035.85
ATOM 12160 CA+2 CA2 801 91.040 50.153 87.703 1.00 20.01
ATOM 12161 CA+2 CA2 802 128.604 77.956 155.873 1.00 17.53
END

## Claims

1. A method for designing a mutated enzyme, wherein the activity and/or substrate specificity for hydrolyzing an α-1,6-glycosidic linkage with respect to pullulan or amylopectin is improved as compared with the enzyme to be mutated, the method comprising following steps:
(1) specifying one or two or more amino acids selected from the group shown below in an amino acid sequence of an enzyme (enzyme to be mutated) that hydrolyzes an α-1,6-glycosidic linkage, the group consisting of an amino acid corresponding to an amino acid at the 292 position, an amino acid corresponding to an amino acid at the 371 position, an amino acid corresponding to an amino acid at the 406 position, an amino acid corresponding to an amino acid at the 407 position, an amino acid corresponding to an amino acid at the 437 position, an amino acid corresponding to an amino acid at the 465 position, an amino acid corresponding to an amino acid at the 475 position, an amino acid corresponding to an amino acid at the 476 position; an amino acid corresponding to an amino acid at the 525 position, an amino acid corresponding to an amino acid at the 526 position, an amino acid corresponding to an amino acid at the 580 position and an amino acid corresponding to an amino acid at the 582 position of the amino acid sequence set forth in SEQ ID NO: 2;
(2) constructing an amino acid sequence in which the amino acid specified in the step (1) is substituted with another amino acid or deleted based on the amino acid sequence of the enzyme to be mutated;
(3) wherein the amino acid sequence of the enzyme to be mutated is an amino acid sequence set forth in any of SEQ ID NOs: 2, 13 to 16; and
(4) evaluating the activity and/or substrate specificity with respect to pullulan or amylopectin of the mutated enzyme obtained in step (2).

2. The method for designing a mutated enzyme according to claim 1, wherein in the step (1), one or two or more amino acids selected from the group consisting of an amino acid corresponding to an amino acid at the 292 position, an amino acid corresponding to an amino acid at the 371 position, an amino acid corresponding to an amino acid at the 407 position, an amino acid corresponding to an amino acid at the 475 position, an amino acid corresponding to an amino acid at the 476 position, and an amino acid corresponding to an amino acid at the 582 position of the amino acid sequence set forth in SEQ ID NO: 2 is specified.

3. The method for designing a mutated enzyme according to claim 1 or 2, wherein the specifying of an amino acid in step (1) is carried out by comparing between an amino acid sequence of the enzyme to be mutated and the amino acid sequence set forth in SEQ ID NO: 2 and/or between a three-dimensional structure of the enzyme to be mutated and a three-dimensional structure of the amino acid sequence set forth in SEQ ID NO: 2.

4. A method for preparing a mutated enzyme, the method comprising following steps:
(1) preparing a nucleic acid encoding an amino acid sequence constructed by the method described in any of claims 1 to 3;
(2) expressing the nucleic acid; and
(3) collecting expression products.

## Patentansprüche

1. Verfahren zum Entwerfen eines mutierten Enzyms, wobei die Aktivität und/oder Substratspezifität zur Hydrolysierung einer α-1,6-glykosidischen Bindung in Bezug auf Pullulan oder Amylopektin verbessert ist verglichen mit dem zu mutierenden Enzym, wobei das Verfahren die folgenden Schritte umfasst:
(1) Festlegen von einer, zwei oder mehrerer Aminosäuren, ausgewählt aus der Gruppe dargestellt unten in einer Aminosäuresequenz eines Enzyms (des zu mutierenden Enzyms), welches eine α-1,6-glykosidische Bindung hydrolysiert, die Gruppe bestehend aus einer Aminosäure entsprechend einer Aminosäure an der Position 292, einer Aminosäure, entsprechend einer Aminosäure an der Position 371, einer Aminosäure, entsprechend einer Aminosäure an der Position 406, einer Aminosäure, entsprechend einer Aminosäure an der Position 407, einer Aminosäure, entsprechend einer Aminosäure an der Position 437, einer Aminosäure, entsprechend einer Aminosäure an der Position 465, einer Aminosäure entsprechend einer Aminosäure an der Position 475, einer Aminosäure, entsprechend einer Aminosäure an der Position 476, einer Aminosäure, entsprechend einer Aminosäure an der Position 525, einer Aminosäure, entsprechend einer Aminosäure an der Position 526, einer Aminosäure, entsprechend einer Aminosäure an der Position 580 und einer Aminosäure entsprechend einer Aminosäure an der Position 582 der in SEQ ID NO: 2 dargelegten Aminosäuresequenz;
(2) Konstruktion einer Aminosäuresequenz, in welcher die Aminosäure, festgelegt in Schritt (1), durch eine andere Aminosäure substituiert oder deletiert wird, basierend auf der Aminosäuresequenz des zu mutierenden Enzyms;
(3) wobei die Aminosäuresequenz des zu mutierenden Enzyms eine Aminosäuresequenz darstellt, wie in einer der SEQ ID NOs: 2, 13 bis 16 dargelegt wird; und
(4) Bewertung der Aktivität und/oder der Substratspezifität in Bezug auf Pullulan oder Amylopektin des in Schritt (2) erhaltenen, mutierten Enzyms.

2. Verfahren zum Entwerfen eines mutierten Enzyms nach Anspruch 1, wobei in Schritt (1), eine oder zwei oder mehrere Aminosäuren festgelegt werden ausgewählt aus der Gruppe bestehend aus einer Aminosäure, entsprechend einer Aminosäure an der Position 292, einer Aminosäure, entsprechend einer Aminosäure an der Position 371, einer Aminosäure, entsprechend einer Aminosäure an der Position 407, einer Aminosäure, entsprechend einer Aminosäure an der Position 475, einer Aminosäure, entsprechend einer Aminosäure an der Position 476, und einer Aminosäure, entsprechend einer Aminosäure an der Position 582 der in SEQ ID NO: 2 dargelegten Aminosäuresequenz.

3. Verfahren zum Entwerfen eines mutierten Enzyms nach Anspruch 1 oder 2, wobei das Festlegen einer Aminosäure in Schritt (1) durchgeführt wird durch Vergleich zwischen einer Aminosäuresequenz des zu mutierenden Enzyms und der in SEQ ID NO: 2 dargelegten Aminosäuresequenz und/oder zwischen einer dreidimensionalen Struktur des zu mutierenden Enzyms und einer dreidimensionalen Struktur der in SEQ ID NO: 2 dargelegten Aminosäuresequenz.

4. Verfahren zur Herstellung eines mutierten Enzyms, wobei das Verfahren die folgenden Schritte umfasst:
(1) Herstellen einer Nukleinsäure, kodierend eine Aminosäuresequenz, die durch das Verfahren konstruiert wird, welches in einem der Ansprüche 1 bis 3 beschrieben wird;
(2) Exprimieren der Nukleinsäure; und
(3) Einsammeln von Expressionsprodukten.

## Revendications

1. Méthode pour concevoir une enzyme mutée, dans laquelle l'activité et/ou la spécificité du substrat en matière d'hydrolysation d'une liaison glucosidique α-1,6 pour ce qui concerne le pullulan ou l'amylopectine est améliorée par rapport à celle de l'enzyme à muter; méthode comprenant les étapes suivantes:
(1) spécification d'un ou deux acides aminés ou plus sélectionnés à partir du groupe figurant ci-dessous dans une séquence d'acides aminés d'une enzyme (l'enzyme à muter) qui hydrolyse une liaison glucosidique α-1,6, ce groupe consistant en un acide aminé correspondant à l'acide aminé à la position 292, un acide aminé correspondant à l'acide aminé à la position 371, un acide aminé correspondant à l'acide aminé à la position 406, un acide aminé correspondant à l'acide aminé à la position 407, un acide aminé correspondant à l'acide aminé à la position 437, un acide aminé correspondant à l'acide aminé à la position 465, un acide aminé correspondant à l'acide aminé à la position 475, un acide aminé correspondant à l'acide aminé à la position 476, un acide aminé correspondant à l'acide aminé à la position 525, un acide aminé correspondant à l'acide aminé à la position 526, un acide aminé correspondant à l'acide aminé à la position 580, un acide aminé correspondant à l'acide aminé à la position 582 de la séquence d'acides aminés décrite dans la SEQ ID NO: 2;
(2) construction d'une séquence d'acides aminés dans laquelle l'acide aminé spécifié à l'étape (1) est remplacé par un autre acide aminé ou éliminé sur la base de la séquence d'acides aminés de l'enzyme à muter;
(3) dans laquelle la séquence d'acides aminés de l'enzyme à muter est une séquence d'acides aminés décrite dans l'une quelconque des SEQ ID NO: 2, 13 à 16; et
(4) evaluation de l'activité et/ou la spécificité du substrat en fonction du pullulan ou de l'amylopectine de l'enzyme mutée obtenue en étape (2).

2. Méthode pour concevoir une enzyme mutée selon la revendication 1, dans laquelle au cours de l'étape (1) est ou sont spécifié(s) un ou deux acides aminés ou plus sélectionnés à partir du groupe consistant en un acide aminé correspondant à l'acide aminé à la position 292, un acide aminé correspondant à l'acide aminé à la position 371, un acide aminé correspondant à l'acide aminé à la position 407, un acide aminé correspondant à l'acide aminé à la position 475, un acide aminé correspondant à l'acide aminé à la position 476, et un acide aminé correspondant à l'acide aminé à la position 582 de la séquence d'acides aminés décrites dans SEQ ID NO: 2.

3. Méthode pour concevoir une enzyme mutée selon l'une quelconque des revendications 1 ou 2, dans laquelle la spécification d'un acide aminé à l'étape (1) est effectuée en comparant une séquence d'acides aminés de l'enzyme à muter avec la séquence d'acides aminés décrite dans SEQ IN NO: 2, et/ou bien en comparant une structure tridimensionnelle de l'enzyme à muter avec une structure tridimensionnelle de la séquence d'acides aminés décrites dans SEQ ID NO: 2.

4. Méthode de préparation d'une enzyme mutée, méthode comprenant les étapes suivantes:
(1) préparation d'un acide nucléique encodant une séquence d'acides aminés construite au moyen de la méthode décrite dans l'une quelconque des revendications 1 à 3;
(2) expression de l'acide nucléique; et
(3) recueil des produits d'expression.


### SEQUENCE LISTING

<110> AMANO ENZYME INC.
   MIKAMI, Bunzo
   IWAMOTO, Hiroyuki
   YAMAGUCHI, shotaro
<120> METHODS OF DESIGNING MUTANT ENZYMES, METHODS OF PREPARING THE SAME, AND MUTANT ENZYMES
<130> P06081PE
<140> 07 767 790.4
   <141> 2007-06-28
<150> JP P2006-213490
   <151> 2006-08-04
<160> 16
<170> PatentIn version 3.5
<210>
   <211> 2233
   <212> DNA
   <213> Bacillus subtilis
<400>
<210> 2
   <211> 718
   <212> PRT
   <213> Bacillus subtilis
<400> 2
<210> 3
   <211> 30
   <212> DNA
   <213> Artificial
<220> '
   <223> PCR primer
<400> 3
   ggccatggtc agcatccgcc gcagcttcga 30
<210> 4
   <211> 30
   <212> DNA
   <213> Artificial
<220>
   <223> PCR primer
<400> 4
   ggctcgagtc aagcaaaact cttaagatct 30
<210> 5
   <211> 33
   <212> DNA
   <213> Artificial
<220>
   <223> PCR primer
<400> 5
   gtaaaaggga acaccggtca ccttaaggca ata 33
<210> 6
   <211> 33
   <212> DNA
   <213> Artificial
<220>
   <223> PCR primer
<400> 6
   gtaaaaggga acacctctca ccttaaggca ata 33
<210> 7
   <211> 20
   <212> DNA
   <213> Artificial
<220>
   <223> PCR primer
<400> 7 20
   ttattgccgg agagtggcga 20
<210> 8
   <211> 20
   <212> DNA
   <213> Artificial
<220>
   <223> PCR, primer
<400> 8
   ccagactgct gacaaagtgc 20
<210> 9
   <211> 34
   <212> DNA
   <213> Artificial
<220>
   <223> PCR primer
<400> 9
   ggaattccat atggatgtcg tcgtccgctt accg 34
<210> 10
   <211> 29
   <212> DNA
   <213> Artificial
<220>
   <223> PCR primer
<400> 10
   gctctagatt atttactgct caccggcag 29
<210> 11
   <211> 19
   <212> DNA
   <213> Artificial
<220>
   <223> PCR primer.
<400> 11
   gccggccgac tccggtgac 19
<210> 12
   <211> 3664
   <212> DNA
   <213> Klebsiella pneumoniae
<400> 12
   tggcgataag cgtggcgaat cataatcagt ttgcggtttc gggtcgtcat tccctttatt      60
   gatttactcc cccggcttcc tacgcccccc gctcatttgt gggggatgat tgcgcctggg     120
   aaagcaaaaa tatctaatta gcgcgctgta aagatattca tcctttctta ccttttataa     180
   tttaataagc atattaggga ctatcgaatg ctcagatata cctgtcatgc cctatttctt     240
   ggatcgttag tattattgag tggctgtgat aacagctctt cctcttctac ctctggctca 300
   ccgggttcac caggcaatcc tggcaaccca ggcactcccg gcacgcccga cccgcaggat 360
   gtcgtcgtcc gcttaccgga cgttgccgtc ccaggcgaag cggtgcaggc ttccgccagg 420
   caggctgtca ttcatctcgt cgatatcgcc ggcatcacca gcagcacgcc ggccgactat 480
   gcgacgaaaa acctctattt atggaacaac gaaacctgtg acgcgctgag cgcgccggtg 540
   gcggactgga atgatgtcag caccaccccg accggcagcg acaaatatgg cccttattgg 600
   gtgatcccgc tgactaaaga gagcggatgc atcaacgtta tcgtccgcga tggcaccaat 660
   aagcttatcg acagcgacct gcgcgtctct ttcagtgatt tcaccgatcg gacggtatcg 720
   gtcatcgccg gcaacagcgc ggtctatgac tcccgcgccg acgccttccg cgccgccttt 780
   ggcgtggcgc tggccgatgc gcactgggtc gataagacta ctctgctgtg gccgggtggg 840
   gaaaataaac ccattgtgcg cctctattac agccacagca gtaaggtggc cgccgacagt 900
   aacggcgaat ttagcgataa atatgtcaag ctgaccccca ccaccgtcag ccagcaggta 960
   agcatgcgct tcccgcatct cgccagctat ccagccttta aactgccgga tgatgttaac 1020
   gtcgatgaat tgctgcaggg cgagacggtg gcgatagccg cggaaagcga tgggatcctg 1080
   agctcagcca cccaggtgca gaccgccggc gtgctggacg atacctatgc cgccgccgcc 1140
   gaggcgctga gctatggcgc ccagctaacc gatagcggcg tgaccttccg cgtctgggcg 1200
   cccacggcgc agcaggtcga gctggtgatc tatagcgcgg acaagaaagt gatagccagt 1260
   cacccgatga cccgcgatag cgcctccggc gcctggtcct ggcagggggg aagcgacctg 1320
   aagggcgcct tctaccgcta cgcgatgacg gtctaccacc cgcagtcgcg taaagtcgag 1380
   cagtacgaag tgaccgatcc ctacgcccac agtttgtcga ccaactcgga gtacagccag 1440
   gtggtcgatc tcaacgacag cgcgctgaag ccggaaggct gggacgggct gacgatgccg 1500
   cacgcgcaga aaaccaaagc cgatctggcg aaaatgacga ttcacgaatc gcatattcgc 1560
   gatctctccg cctgggatca aaccgtgcct gcggagctgc gcggtaagta tctggcgctc 1620
   accgcccagg aaagcaatat ggtccagcat ctgaaacagc tgtcggcctc gggcgtgacc 1680
   catattgagc tgctgccggt cttcgatctg gcgacggtca atgagttcag cgacaaggtc 1740
   gccgatattc agcagccgtt cagccgcctg tgcgaggtca atagcgcggt gaagagcagc 1800
   gagttcgcgg gctattgcga cagcggttcg acggtcgaag aggtgctgac ccagctgaag 1860
   cagaacgaca gcaaggataa cccgcaggtg caggcgttga atacgctggt ggcgcagacc 1920
   gactcctata actggggcta cgatccgttc cactacacgg taccggaagg atcctacgcc 1980
   accgatccgg aaggcacggc gcgtattaaa gagttccgca ccatgattca ggcgatcaag 2040
   caggatctgg gaatgaacgt cattatggac gtggtgtaca accacaccaa cgccgccggc 2100
   ccgaccgatc gcacctcggt actggataag atcgtcccct ggtactacca gcgtctgaac 2160
   gaaaccaccg gcagcgtgga atcggctacc tgttgctccg actcggcgcc agagcaccgg 2220
   atgttcgcca agcttatcgc cgattcactg gcggtatgga ccaccgatta taagatcgat 2280
   ggcttccgct tcgacctgat gggctaccac ccgaaagcgc agatcctctc ggcctgggaa 2340
   cgcattaaag cgctgaaccc ggatatctac ttctttggcg aaggctggga ttccaaccag 2400
   agcgatcgct ttgaaattgc ctcgcaaatc aatctcaaag gcaccgggat cggcacgttc 2460
   tccgatcgtc tgcgcgacgc cgtgcgcggc ggcgggccgt tcgactccgg tgacgcatta 2520
   cgccagaacc agggcgtggg cagcggggct ggcgttctgc cgaatgagct gaccaccctg 2580
   agcgacgatc aggcgcgtca cctcgccgat ctgacccgtc tcggcatggc cggtaacctt 2640
   gcggactttg tgctgatcga caaagacggc gcggtgaaga gaggcagcga gattgattat 2700
   aacggcgcgc caggcggcta tgcggctgat ccgacggaag tcgtgaacta tgtgtcaaaa 2760
   cacgataacc agacgctgtg ggacatgatc agctataaag ccgctcagga ggcggatctc 2820
   gatacccgcg tccggatgca ggcggtgtcg ctggcgacgg tgatgctcgg ccaagggatc 2880
   gcctttgacc agcagggctc ggagctgctg cgctctaaat cctttacccg cgattcgtat 2940
   gattccggcg actggtttaa ccgcgtggac tactccctgc aggacaacaa ctacaacgtc 3000
   ggtatgccgc gcagcagcga tgatggcagc aattatgaca ttatcgcccg ggtgaaagac 3060
   gcggtggcta ctccgggtga aacggagctc aagcagatga ccgcgtttta tcaggagctg 3120
   accgcgctgc gtaaatcgtc tccgctgttt accctcggcg acggcgcgac ggtgatgaag 3180
   cgcgtggact tccgcaatac tggcgccgat cagcagacgg gtctgctggt gatgaccatc 3240
   gatgacggga tgcaggctgg cgccagtctg gacagccgtg tcgacggcat cgtggtggcg 3300
   atcaacgccg cgccggaaag ccggacgctg caggacttcg ccggcacatc gctccagctg 3360
   agcgctattc agcaggcggc gggcgaccgg tcgctggcga gcggcgtgca ggttgccgct 3420
   gacggttcgg tcacgctgcc ggcctggtcg gttgccgttc tcgagttgcc gcagggcgag 3480
   tcgcagggcg ctggcctgcc ggtgagcagt aaataacata acgtagggcg cccgtaaggg 3540
   cgcctctggc tgatgacaaa cctgtttcgt tattgtgctc gcctttctgt cgggtggcgg 3600
   ctttcgctta cccggcctac gaaagattgc aatttcaaac agttatgcag ttatctgtag 3660
   gccc 3664
<210> 13
   <211> 1083
   <212> PRT
   <213> Klebsiella pneumoniae
<400> 13
<210> 14
   <211> 922
   <212> PRT
   <213> Bacillus sp.
<400> 14
   Asp Gly Thr Thr Thr Asn Val Ile Val His Tyr Phe Arg Pro Gly Gly
   1               5                   10                  15
   Asp Tyr Gln Ser Trp Ser Leu Trp Met Trp Pro Glu Gly Gly Asp Gly
               20                  25                  30
   Asn Asn Tyr Asn Phe Asn Gly Thr Asp Ser Tyr Gly Glu Ile Ala Asn
           35                  40                  45
   Val Ser Ile Pro Gly Ser Pro Ser Lys Val Gly Ile Ile Val Arg Thr
   50 55 60
   Gln Asp Trp Ala Lys Asp Val Ser Gln Asp Arg Tyr Ile Asp Leu Ser
   65 70 75 80
   Lys Gly His Glu Val Trp Leu Val Gln Gly Asn Ser Gln Ile Phe Tyr
   85 90 95
   Asn Glu Lys Asp Ala Glu Asp Ala Ala Glu Pro Ala Val Ser Asn Ala
   100 105 110
   Tyr Leu Asp Ala Pro Asn Lys Val Leu Val Lys Leu Ser Gln Pro Phe
   115 120 125
   Thr Leu Gly Glu Gly Ala Ser Gly Phe Thr Val His Asp Asp Thr Ala
   130 135 140
   Asn Gly Asp Ile Pro Val Thr Lys Val Lys Asn Ala Asn Lys Val Glu
   145 150 155 160
   Asp Val Thr Ala Ile Leu Ala Gly Thr Phe Gln His Ile Phe Gly Gly
   165 170 175
   Ser Asp Trp Ala Pro Asp Asn His Ser Thr Gln Leu Lys Lys Val Asn
   180 185 190
   Asp Asn Leu Tyr Gln Phe Ser Gly Glu Leu Pro Gly Gly Ser Tyr Gln
   195 200 205
   Tyr Lys Val Ala Leu Asn Asp Ser Trp Asn Ser Ser Tyr Pro Ser Asp
   210 215 220
   Asn Ile Asn Leu Thr Val Pro Asp Gly Gly Ala His Val Thr Phe Ser
   225 230 235 240
   Tyr Val Pro Ser Thr His Ala Val Tyr Asp Thr Ile Asn Asn Pro Gly
   245 250 255
   Ala Asn Leu Pro Leu Asp Gly Ser Gly Ile Lys Thr Asp Leu Val Thr
   260 265 270
   Val Thr Leu Gly Glu Asn Pro Asp Val Ser His Thr Leu Ser Ile Gln
   275 280 285
   Thr Asp Gly Phe Lys Thr Gly Arg Val Ile Pro Arg Asn Val Leu Asp
   290 295 300
   Phe Ser Gln Tyr Tyr Tyr Ser Gly Glu Asp Leu Gly Asn Thr Tyr Thr
   305 310 315 320
   Lys Lys Ala Thr Thr Phe Lys Val Trp Ala Pro Thr Ser Thr Lys Val
   325 330 335
   Asn Val Leu Leu Tyr Asn Lys Ala Ala Gly Ala Leu Thr Lys Thr Val
   340 345 350
   Pro Met Lys Ala Ser Gly His Gly Val Trp Ser Val Thr Val Pro Gln
   355 360 365
   Asn Leu Glu Asn Trp Tyr Tyr Leu Tyr Glu Val Thr Gly Gln Gly Ser
   370 375 380
   Thr Arg Thr Ala Val Asp Pro Tyr Ala Thr Ala Ile Ala Pro Asn Gly
   385 390 395 400
   Thr Arg Gly Met Val Val Asp Leu Ala Lys Thr Asn Pro Thr Gly Trp
   405 410 415
   Lys Ser Asp Lys His Met Thr Pro Lys Asn Ile Glu Asp Glu Val Ile
   420 425 430
   Tyr Glu Met His Val Arg Asp Phe Ser Ile Asp Ser Asn Ser Gly Met
   435 440 445
   Thr Asn Lys Gly Lys Tyr Leu Ala Leu Thr Glu Lys Gly Thr Lys Gly
   450 455 460
   Pro Glu Asn Val Lys Thr Gly Val Asp Ser Leu Lys Gln Leu Gly Ile
   465 470 475 480
   Thr His Val Gln Leu Gln Pro Val Phe Ala Phe Asn Ser Val Asp Glu
   485 490 495
   Thr Asp Pro Thr Gln Tyr Asn Trp Gly Tyr Asp Pro Arg Asn Tyr Asn
   500 505 510
   Val Pro Glu Gly Gln Tyr Ala Thr Asp Ala Asn Gly Thr Thr Arg Ile
   515 520 525
   Lys Glu Phe Lys Glu Met Val Leu Ser Leu His Arg Asn His Ile Gly
   530 535 540
   Val Asn Met Asp Val Val Tyr Asn His Thr Phe Ala Thr Gln Ile Ser
   545 550 555 560
   Asp Phe Asp Lys Ile Val Pro Gln Tyr Tyr Tyr Arg Thr Asp Asp Ala
   565 570 575
   Gly Asn Tyr Thr Asn Gly Ser Gly Thr Gly Asn Glu Val Ala Ala Glu
   580 585 590
   Arg Pro Met Val Gln Lys Phe Ile Ile Asp Ser Leu Lys Tyr Trp Val
   595 600 605
   Asn Glu Tyr His Ile Asp Gly Phe Arg Phe Asp Leu Met Ala Leu Leu
   610 615 620
   Gly Lys Asp Thr Met Ala Lys Ala Ala Gln Glu Leu His Ala Ile Asp
   625 630 635 640
   Pro Gly Ile Ala Leu Tyr Gly Glu Pro Trp Thr Gly Gly Thr Ser Ala
   645 650 655
   Leu Pro Thr Asp Gln Leu Leu Thr Lys Gly Val Gln Lys Gly Met Gly
   660 665 670
   Val Ala Val Phe Asn Asp Asn Leu Arg Asn Gly Leu Asp Gly Asn Val
   675 680 685
   Phe Asp Ala Ser Ser Gln Gly Phe Ala Thr Gly Ala Thr Gly Leu Thr
   690 695 700
   Asp Val Ile Lys Lys Gly Val Glu Gly Ser Ile Asn Asp Phe Thr Ser
   705 710 715 720
   Ser Pro Gly Glu Thr Ile Asn Tyr Val Thr Ser His Asp Asn Tyr Thr
   725 730 735
   Leu Trp Asp Lys Ile Ala Gln Ser Asn Pro Asn Asp Ser Glu Ala Asp
   740 745 750
   Arg Ile Lys Met Asp Glu Leu Ala Gln Ala Val Val Val Thr Ser Gln
   755 760 765
   Gly Val Pro Phe Met Gln Gly Gly Glu Glu Met Leu Arg Thr Lys Gly
   770 775 780
   Gly Asn Ser Asn Ser Tyr Asn Ala Gly Asp Ala Val Asn Glu Phe Asp
   785 790 795 800
   Trp Ser Arg Lys Ala Gln Tyr Ser Asp Val Phe Asn Tyr Tyr Ser Gly
   805 810 815
   Leu Ile His Leu Arg Leu Ala His Pro Ala Phe Arg Met Thr Thr Ala
   820 825 830
   Asn Gln Ile Lys Glu His Leu Gln Phe Ile Asp Ser Pro Asp Asn Thr
   835 840 845
   Val Ala Tyr Glu Leu Thr Asn His Ala Asn Lys Asp Lys Trp Gly Asn
   850 855 860
   Ile Val Val Ile Tyr Asn Pro Asn Lys Thr Ala Glu Thr Val Asn Leu
   865 870 875 880
   Pro Ser Gly Lys Trp Ala Ile Asn Ala Thr Asn Gly Lys Ile Gly Glu
   885 890 895
   Ser Thr Leu Ser His Ala Glu Gly His Val Gln Val Pro Gly Ile Ser
   900 905 910
   Met Met Ile Leu His Gln Glu Thr Asn Lys
   915 920
<210> 15
   <211> 928
   <212> PRT
   <213> Bacillus deramificans
<400> 15
   Asp Gly Asn Thr Thr Thr Ile Ile Val His Tyr Phe Arg Pro Ala Gly
   1 5 10 15
   Asp Tyr Gln Pro Trp Ser Leu Trp Met Trp Pro Lys Asp Gly Gly Gly
   20 25 30
   Ala Glu Tyr Asp Phe Asn Gln Pro Ala Asp Ser Phe Gly Ala Val Ala
   35 40 45
   Ser Ala Asp Ile Pro Gly Asn Pro Ser Gln Val Gly Ile Ile Val Arg
   50 55 60
   Thr Gln Asp Trp Thr Lys Asp Val Ser Ala Asp Arg Tyr Ile Asp Leu
   65 70 75 80
   Ser Lys Gly Asn Glu Val Trp Leu Val Glu Gly Asn Ser Gln Ile Phe
   85 90 95
   Tyr Asn Glu Lys Asp Ala Glu Asp Ala Ala Lys Pro Ala Val Ser Asn
   100 105 110
   Ala Tyr Leu Asp Ala Ser Asn Gln Val Leu Val Lys Leu Ser Gln Pro
   115 120 125
   Leu Thr Leu Gly Glu Gly Ala Ser Gly Phe Thr Val His Asp Asp Thr
   130 135 140
   Ala Asn Lys Asp Ile Pro Val Thr Ser Val Lys Asp Ala Ser Leu Gly
   145 150 155 160
   Gln Asp Val Thr Ala Val Leu Ala Gly Thr Phe Gln His Ile Phe Gly
   165 170 175
   Gly Ser Asp Trp Ala Pro Asp Asn His Ser Thr Leu Leu Lys Lys Val
   180 185 190
   Thr Asn Asn Leu Tyr Gln Phe Ser Gly Asp Leu Pro Glu Gly Asn Tyr
           195                 200                 205
   Gln Tyr Lys Val Ala Leu Asn Asp Ser Trp Asn Asn Pro Ser Tyr Pro
       210                 215                 220
   Ser Asp Asn Ile Asn Leu Thr Val Pro Ala Gly Gly Ala His Val Thr
   225                 230                 235                     240
   Phe Ser Tyr Ile Pro Ser Thr His Ala Val Tyr Asp Thr Ile Asn Asn
                   245                 250                 255
   Pro Asn Ala Asp Leu Gln Val Glu Ser Gly Val Lys Thr Asp Leu Val
               260                 265                 270
   Thr Val Thr Leu Gly Glu Asp Pro Asp Val Ser His Thr Leu Ser Ile
           275                 280                 285
   Gln Thr Asp Gly Tyr Gln Ala Lys Gln Val Ile Pro Arg Asn Val Leu
       290                 295                 300
   Asn Ser Ser Gln Tyr Tyr Tyr Ser Gly Asp Asp Leu Gly Asn Thr Tyr
   305                 310                 315                     320
   Thr Gln Lys Ala Thr Thr Phe Lys Val Trp Ala Pro Thr Ser Thr Gln
                   325                 330                 335
   Val Asn Val Leu Leu Tyr Asp Ser Ala Thr Gly Ser Val Thr Lys Ile
               340                 345                 350
   Val Pro Met Thr Ala Ser Gly His Gly Val Trp Glu Ala Thr Val Asn
           355                 360                 365
   Gln Asn Leu Glu Asn Trp Tyr Tyr Met Tyr Glu Val Thr Gly Gln Gly
       370                 375                 380
   Ser Thr Arg Thr Ala Val Asp Pro Tyr Ala Thr Ala Ile Ala Pro Asn
   385                 390                 395                     400
   Gly Thr Arg Gly Met Ile Val Asp Leu Ala Lys Thr Asp Pro Ala Gly
                   405                 410                 415
   Trp Asn Ser Asp Lys His Ile Thr Pro Lys Asn Ile Glu Asp Glu Val
               420                 425                 430
   Ile Tyr Glu Met Asp Val Arg Asp Phe Ser Ile Asp Pro Asn Ser Gly
           435                 440                 445
   Met Lys Asn Lys Gly Lys Tyr Leu Ala Leu Thr Glu Lys Gly Thr Lys
       450                 455                 460
   Gly Pro Asp Asn Val Lys Thr Gly Ile Asp Ser Leu Lys Gln Leu Gly
   465 470 475 480
   Ile Thr His Val Gln Leu Met Pro Val Phe Ala Ser Asn Ser Val Asp
   485 490 495
   Glu Thr Asp Pro Thr Gln Asp Asn Trp Gly Tyr Asp Pro Arg Asn Tyr
   500 505 510
   Asp Val Pro Glu Gly Gln Tyr Ala Thr Asn Ala Asn Gly Asn Ala Arg
   515 520 525
   Ile Lys Glu Phe Lys Glu Met Val Leu Ser Leu His Arg Glu His Ile
   530 535 540
   Gly Val Asn Met Asp Val Val Tyr Asn His Thr Phe Ala Thr Gln Ile
   545 550 555 560
   Ser Asp Phe Asp Lys Ile Val Pro Glu Tyr Tyr Tyr Arg Thr Asp Asp
   565 570 575
   Ala Gly Asn Tyr Thr Asn Gly Ser Gly Thr Gly Asn Glu Ile Ala Ala
   580 585 590
   Glu Arg Pro Met Val Gln Lys Phe Ile Ile Asp Ser Leu Lys Tyr Trp
   595 600 605
   Val Asn Glu Tyr His Ile Asp Gly Phe Arg Phe Asp Leu Met Ala Leu
   610 615 620
   Leu Gly Lys Asp Thr Met Ser Lys Ala Ala Ser Glu Leu His Ala Ile
   625 630 635 640
   Asn Pro Gly Ile Ala Leu Tyr Gly Glu Pro Trp Thr Gly Gly Thr Ser
   645 650 655
   Ala Leu Pro Asp Asp Gln Leu Leu Thr Lys Gly Ala Gln Lys Gly Met
   660 665 670
   Gly Val Ala Val Phe Asn Asp Asn Leu Arg Asn Ala Leu Asp Gly Asn
   675 680 685
   Val Phe Asp Ser Ser Ala Gln Gly Phe Ala Thr Gly Ala Thr Gly Leu
   690 695 700
   Thr Asp Ala Ile Lys Asn Gly Val Glu Gly Ser Ile Asn Asp Phe Thr
   705 710 715 720
   Ser Ser Pro Gly Glu Thr Ile Asn Tyr Val Thr Ser His Asp Asn Tyr
   725 730 735
   Thr Leu Trp Asp Lys Ile Ala Leu Ser Asn Pro Asn Asp Ser Glu Ala
   740 745 750
   Asp Arg Ile Lys Met Asp Glu Leu Ala Gln Ala Val Val Met Thr Ser
   755 760 765
   Gln Gly Val Pro Phe Met Gln Gly Gly Glu Glu Met Leu Arg Thr Lys
   770 775 780
   Gly Gly Asn Asp Asn Ser Tyr Asn Ala Gly Asp Ala Val Asn Glu Phe
   785 790 795 800
   Asp Trp Ser Arg Lys Ala Gln Tyr Pro Asp Val Phe Asn Tyr Tyr Ser
   805 810 815
   Gly Leu Ile His Leu Arg Leu Asp His Pro Ala Phe Arg Met Thr Thr
   820 825 830
   Ala Asn Glu Ile Asn Ser His Leu Gln Phe Leu Asn Ser Pro Glu Asn
   835 840 845
   Thr Val Ala Tyr Glu Leu Thr Asp His Val Asn Lys Asp Lys Trp Gly
   850 855 860
   Asn Ile Ile Val Val Tyr Asn Pro Asn Lys Thr Val Ala Thr Ile Asn
   865 870 875 880
   Leu Pro Ser Gly Lys Trp Ala Ile Asn Ala Thr Ser Gly Lys Val Gly
   885 890 895
   Glu Ser Thr Leu Gly Gln Ala Glu Gly Ser Val Gln Val Pro Gly Ile
   900 905 910
   Ser Met Met Ile Leu His Gln Glu Val Ser Pro Asp His Gly Lys Lys
   915 920 925
<210> 16
   <211> 750
   <212> PRT
   <213> Pseudomonas amyloderamosa
<400> 16
   Ala Ile Asn Ser Met Ser Leu Gly Ala Ser Tyr Asp Ala Gln Gln Ala
   1 5 10 15
   Asn Ile Thr Phe Arg Val Tyr Ser Ser Gln Ala Thr Arg Ile Val Leu
   20 25 30
   Tyr Leu Tyr Ser Ala Gly Tyr Gly Val Gln Glu Ser Ala Thr Tyr Thr
   35 40 45
   Leu Ser Pro Ala Gly Ser Gly Val Trp Ala Val Thr Val Pro Val Ser
   50 55 60
   Ser Ile Lys Ala Ala Gly Ile Thr Gly Ala Val Tyr Tyr Gly Tyr Arg
   65 70 75 80
   Ala Trp Gly Pro Asn Trp Pro Tyr Ala Ser Asn Trp Gly Lys Gly Ser
   85 90 95
   Gln Ala Gly Phe Val Ser Asp Val Asp Ala Asn Gly Asp Arg Phe Asn
   100 105 110
   Pro Asn Lys Leu Leu Leu Asp Pro Tyr Ala Gln Glu Val Ser Gln Asp
   115 120 125
   Pro Leu Asn Pro Ser Asn Gln Asn Gly Asn Val Phe Ala Ser Gly Ala
   130 135 140
   Ser Tyr Arg Thr Thr Asp Ser Gly Ile Tyr Ala Pro Lys Gly Val Val
   145 150 155 160
   Leu Val Pro Ser Thr Gln Ser Thr Gly Thr Lys Pro Thr Arg Ala Gln
   165 170 175
   Lys Asp Asp Val Ile Tyr Glu Val His Val Arg Gly Phe Thr Glu Gln
   180 185 190
   Asp Thr Ser Ile Pro Ala Gln Tyr Arg Gly Thr Tyr Tyr Gly Ala Gly
   195 200 205
   Leu Lys Ala Ser Tyr Leu Ala Ser Leu Gly Val Thr Ala Val Glu Phe
   210 215 220
   Leu Pro Val Gln Glu Thr Gln Asn Asp Ala Asn Asp Val Val Pro Asn
   225 230 235 240
   Ser Asp Ala Asn Gln Asn Tyr Trp Gly Tyr Met Thr Glu Asn Tyr Phe
   245 250 255
   Ser Pro Asp Arg Arg Tyr Ala Tyr Asn Lys Ala Ala Gly Gly Pro Thr
   260 265 270
   Ala Glu Phe Gln Ala Met Val Gln Ala Phe His Asn Ala Gly Ile Lys
   275 280 285
   Val Tyr Met Asp Val Val Tyr Asn His Thr Ala Glu Gly Gly Thr Trp
   290 295 300
   Thr Ser Ser Asp Pro Thr Thr Ala Thr Ile Tyr Ser Trp Arg Gly Leu
   305 310 315 320
   Asp Asn Ala Thr Tyr Tyr Glu Leu Thr Ser Gly Asn Gln Tyr Phe Tyr
   325 330 335
   Asp Asn Thr Gly Ile Gly Ala Asn Phe Asn Thr Tyr Asn Thr Val Ala
   340 345 350
   Gln Asn Leu Ile Val Asp Ser Leu Ala Tyr Trp Ala Asn Thr Met Gly
   355 360 365
   Val Asp Gly Phe Arg Phe Asp Leu Ala Ser Val Leu Gly Asn Ser Cys
   370 375 380
   Leu Asn Gly Ala Tyr Thr Ala Ser Ala Pro Asn Cys Pro Asn Gly Gly
   385 390 395 400
   Tyr Asn Phe Asp Ala Ala Asp Ser Asn Val Ala Ile Asn Arg Ile Leu
   405 410 415
   Arg Glu Phe Thr Val Arg Pro Ala Ala Gly Gly Ser Gly Leu Asp Leu
   420 425 430
   Phe Ala Glu Pro Trp Ala Ile Gly Gly Asn Ser Tyr Gln Leu Gly Gly
   435 440 445
   Phe Pro Gln Gly Trp Ser Glu Trp Asn Gly Leu Phe Arg Asp Ser Leu
   450 455 460
   Arg Gln Ala Gln Asn Glu Leu Gly Ser Met Thr Ile Tyr Val Thr Gln
   465 470 475 480
   Asp Ala Asn Asp Phe Ser Gly Ser Ser Asn Leu Phe Gln Ser Ser Gly
   485 490 495
   Arg Ser Pro Trp Asn Ser Ile Asn Phe Ile Asp Val His Asp Gly Met
   500 505 510
   Thr Leu Lys Asp Val Tyr Ser Cys Asn Gly Ala Asn Asn Ser Gln Ala
   515 520 525
   Trp Pro Tyr Gly Pro Ser Asp Gly Gly Thr Ser Thr Asn Tyr Ser Trp
   530 535 540
   Asp Gln Gly Met Ser Ala Gly Thr Gly Ala Ala Val Asp Gln Arg Arg
   545 550 555 560
   Ala Ala Arg Thr Gly Met Ala Phe Glu Met Leu Ser Ala Gly Thr Pro
   565 570 575
   Leu Met Gln Gly Gly Asp Glu Tyr Leu Arg Thr Leu Gln Cys Asn Asn
   580 585 590
   Asn Ala Tyr Asn Leu Asp Ser Ser Ala Asn Trp Leu Thr Tyr Ser Trp
   595 600 605
   Thr Thr Asp Gln Ser Asn Phe Tyr Thr Phe Ala Gln Arg Leu Ile Ala
   610 615 620
   Phe Arg Lys Ala His Pro Ala Leu Arg Pro Ser Ser Trp Tyr Ser Gly
   625 630 635 640
   Ser Gln Leu Thr Trp Tyr Gln Pro Ser Gly Ala Val Ala Asp Ser Asn
   645 650 655
   Tyr Trp Asn Asn Thr Ser Asn Tyr Ala Ile Ala Tyr Ala Ile Asn Gly
   660 665 670
   Pro Ser Leu Gly Asp Ser Asn Ser Ile Tyr Val Ala Tyr Asn Gly Trp
   675 680 685
   Ser Ser Ser Val Thr Phe Thr Leu Pro Ala Pro Pro Ser Gly Thr Gln
   690 695 700
   Trp Tyr Arg Val Thr Asp Thr Cys Asp Trp Asn Asp Gly Ala Ser Thr
   705 710 715 720
   Phe Val Ala Pro Gly Ser Glu Thr Leu Ile Gly Gly Ala Gly Thr Thr
   725 730 735
   Tyr Gly Gln Cys Gly Gln Ser Leu Leu Leu Leu Ile Ser Lys
   740 745 750